# EUROPEAN PATENT APPLICATION

(11) **EP 4 640 688 A1**
(43) Date of publication of application: **29.10.2025**
(21) Application number: 23907188.9
(22) Date of filing: 22.12.2023
(51) Int. Cl.: C07D 493/22, A61K 31/7048, A61P 31/14, C07H 17/08

(54) **AVERMECTIN DERIVATIVE**

(30) Priority: 23.12.2022 JP 2022207198
(71) Applicant: Meiji Seika Pharma Co., Ltd., Tokyo 104-8002 (JP); The Kitasato Institute, Tokyo 108-8641 (JP)
(72) Inventor: NISHIDA Keisuke, Odawara-shi, Kanagawa 250-0852 (JP); KAWANO Takara, Odawara-shi, Kanagawa 250-0852 (JP); MATSUMOTO Yuichiro, Odawara-shi, Kanagawa 250-0852 (JP); BABA Nobuyoshi, Tokyo 104-8002 (JP); SUNAZUKA Toshiaki, Tokyo 108-8641 (JP); HANAKI Hideaki, Tokyo 108-8641 (JP); KATAYAMA Kazuhiko, Tokyo 108-8641 (JP); HIROSE Tomoyasu, Tokyo 108-8641 (JP); MATSUI Hidehito, Tokyo 108-8641 (JP); HAGA Kei, Tokyo 108-8641 (JP)
(74) Representative: J A Kemp LLP
(86) International application number: PCT/JP2023/046157
(87) International publication number: WO 2024/135828

(57) **Abstract**

A compound represented by the following general formula (I), or a pharmaceutically acceptable salt thereof: 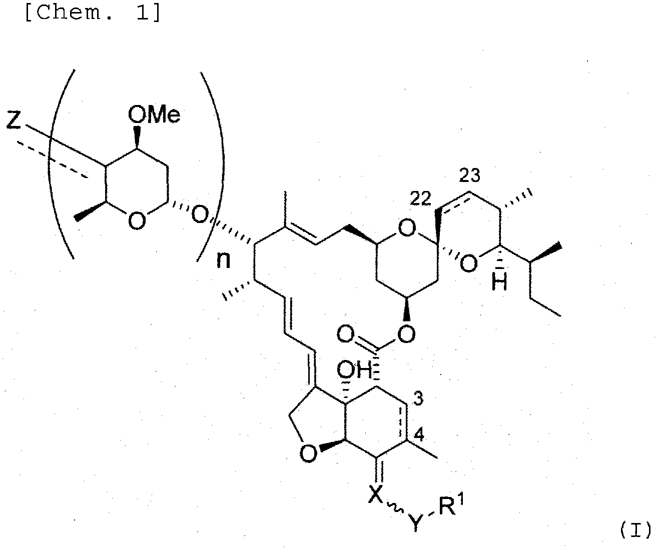 [In the formula (I), n represents an integer of 0 to 2; X represents CH, CF, CCl, N, CHCH=N, CHCH=CH, or CHCH=CF; Y represents O, NH, or a single bond; Z represents a hydroxy group or an oxo group; R¹ represents a hydrogen atom, a C1-C6 alkyl group, a 3-to 6-membered cycloalkyl group, a 4- to 8-membered heterocycloalkyl group, a 6- to 10-membered aryl group, a 5- to 10-membered heteroaryl group, a C2-C6 alkenyl group, a C2-C6 alkynyl group, a group represented by - CO-R², a group represented by -COO-R², a group represented by -CONH-R², or a group represented by - B(OR⁴)OR⁵, and these groups may be substituted; and R², R⁴, and R⁵ each represent a hydrogen atom, an alkyl group, etc.].

## Description

### [Technical Field]

The present invention relates to avermectin derivatives and compositions comprising the same, and more particularly, to novel avermectin derivatives having antiviral activity against coronaviruses, and compositions comprising the same as active ingredients.

### [Background Art]

Coronaviruses (CoVs) are enveloped viruses with a single-stranded, positive-sense RNA genome of 26 to 32 kb, the largest of the RNA viruses, and are classified in the subfamily *Orthocoronavirinae* of the family Coronaviridae of the order Nidovirales. The subfamily *Orthocoronavirinae* is further classified into four genera: Alpha- (*α*) coronavirus, Beta- (*β*) coronavirus, *Gamma- (y)* coronavirus, and Delta- (*δ*) coronavirus. Coronaviruses are characterized by a high frequency of gene recombination and a high mutation rate, resulting in diversity and the ability to easily adapt and infect a wide range of hosts, from birds to cetaceans.

At present, seven types of coronaviruses have been confirmed to infect humans. For example, human coronaviruses (HCoVs) 229E, OC43, NL63, and HKU1 account for 10 to 30% of annual upper respiratory tract infections and are characterized by causing mild respiratory illnesses such as the common cold. On the other hand, severe acute respiratory syndrome coronavirus (SARS-CoV), severe acute respiratory syndrome coronavirus 2 (novel coronavirus, SARS-CoV-2), and Middle East respiratory syndrome coronavirus (MERS-CoV) can cause respiratory diseases, and their spread can lead to high mortality rates (Non Patent Literatures 1 and 2). In particular, the novel coronavirus infection (COVID-19), which is said to have originated in Wuhan, China in December 2019, is caused by SARS-CoV-2. SARS-CoV-2 has spread and become prevalent worldwide due to its high infectivity, resulting in a cumulative total of 600 million infected people and 6.46 million deaths as of September 7, 2022 (Non Patent Literature 3), and it continues to spread, posing a major global threat.

Much research has been conducted on compounds as drugs that have antiviral activity against coronaviruses, especially SARS-CoV-2. Many of these compounds have been found to have antiviral effects through drug repositioning of existing drugs, but examples of compounds that have been newly created and already approved in Japan include small molecule compounds such as molnupiravir (product name: Lagevrio) (Patent Literatures 1 and 2) and a combination of nirmatrelvir and ritonavir (product name: Paxlovid) (Patent Literature 3) shown below. However, these compounds were granted special approval as therapeutic drugs for COVID-19 in December 2021 and February 2022, and there was limited information on their efficacy and safety at the time of approval. Furthermore, since the efficacy of these compounds against COVID-19 patients with high severity has not yet been established, further development of compounds having antiviral activity against SARS-CoV-2 is urgently needed.

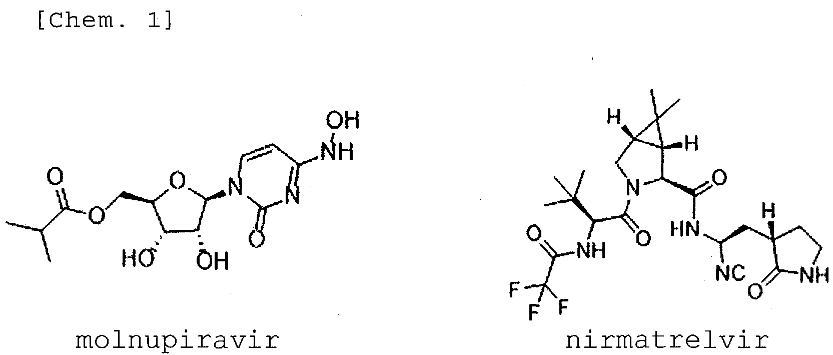

In addition, several other compounds are known to have antiviral activity against SARS-CoV-2, such as ensitrelvir (Patent Literature 4) shown below, and compounds described in Patent Literatures 5-6.

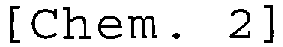

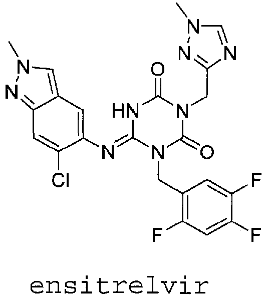

Furthermore, in addition to the above compounds, ivermectin, a compound obtained by reducing the double bond at positions 22 and 23 of avermectin, was reported to have antiviral activity against SARS-CoV-2 in 2020 (Patent Literature 7, Non Patent Literature 4).

Avermectin and derivatives thereof are a group of compounds characterized by having a 16-membered macrolide structure, a hexahydrobenzofuran skeleton, and a bispyran structure containing a spiro ring, and ivermectin, an avermectin derivative, is widely known as a therapeutic agent for river blindness and scabies. In addition to ivermectin, other avermectin derivatives, such as emamectin, eprinomectin, milbemycin oxime, lepimectin, nemadectin, and moxidectin, are widely used as anthelmintics in humans, or as agricultural chemicals and veterinary drugs.

Therefore, synthetic studies and studies on the pharmacological effects of avermectin derivatives and analogues have been actively conducted. For example, Patent Literatures 8 to 13 describe that the compounds described in these documents exhibit anthelmintic activity, and Patent Literature 14 describes that the compound represented by the general formula (I) in the document exhibits FXR regulatory activity. Furthermore, Non Patent Literature 5 describes that ivermectin binds to TELO2 and exhibits an inhibitory effect on the Wnt/β-catenin pathway.

### [Citation List]

### [Patent Literature]

[PTL 1] International Publication No. WO 2019/113462
[PTL 2] International Publication No. WO 2019/173602
[PTL 3] International Publication No. WO 2021/250648
[PTL 4] International Publication No. WO 2022/138988
[PTL 5] International Publication No. WO 2021/176010
[PTL 6] International Publication No. WO 2022/010948
[PTL 7] International Publication No. WO 2021/179050
[PTL 8] International Publication No. WO 1994/015944
[PTL 9] International Publication No. WO 1995/022552
[PTL 10] International Publication No. WO 1995/004746
[PTL 11] Japanese Patent Application Publication No. Sho 63-045281
[PTL 12] International Publication No. WO 2000/47597
[PTL 13] International Publication No. WO 2002/12248
[PTL 14] International Publication No. WO 2018/185684

### [Non Patent Literature]

[NPL 1] H. Yang and Z. Rao, Nature Reviews Microbiolo gy, 2021, 19, p. 685-700
[NPL 2] Journal of the Japanese Society for Virology, Virus, Vol. 70, No. 1, p. 29-36, 2020
[NPL 3] Weekly epidemiological update on COVID-19 - 7 September 2022, https://www.who.int/publications/m/it em/weekly-epidemiological-update-on-covid-19---7-septe mber-2022
[NPL 4] Kylie M. Wagstaff et al., Antiviral Research, 2020, 178, 104787
[NPL 5] Naoyuki Nishiya et al., iScience, 2021, 25, 3 , 103912

### [Summary of Invention]

### [Technical Problem]

The present invention has been made in view of the problems of the above-mentioned prior art, and provides a novel compound that has antiviral activity against coronaviruses and is useful for the treatment or prevention of diseases or conditions involving coronaviruses, as well as a composition comprising the same as an active ingredient for the treatment or prevention of diseases or conditions involving coronaviruses.

### [Solution to Problem]

As a result of intensive studies to achieve the above object, the present inventors have found that an avermectin derivative represented by the following general formula (I) has excellent antiviral activity against coronaviruses, and have completed the present invention. The aspects of the present invention obtained from this finding are as follows.
[1] A compound represented by the following general formula (I), or a pharmaceutically acceptable salt thereof: 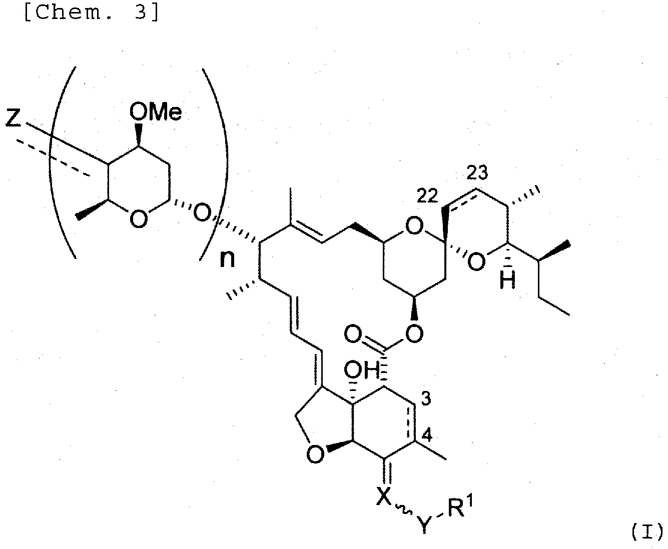 [In the above formula (I),
   n represents an integer of 0, 1, or 2,
   X represents CH, CF, CCl, N, CHCH=N, CHCH=CH, or CHCH=CF,
   Y represents O, NH, or a single bond,
   Z represents a hydroxy group or an oxo group,
   R¹ represents a hydrogen atom; a halogen atom; a C1-C6 alkyl group; a 3- to 6-membered cycloalkyl group; a 4- to 8-membered heterocycloalkyl group; a 6- to 10-membered aryl group; a 5- to 10-membered heteroaryl group; a C2-C6 alkenyl group; a C2-C6 alkynyl group; a group represented by -CO-R²; a group represented by - COO-R²; a group represented by -CONH-R²; or a group represented by -B(OR⁴)OR⁵, wherein,
   when R¹ is other than a hydrogen atom and a C1-C6 alkyl group, the R¹ may be substituted with one or more substituents selected from a halogen atom, a hydroxy group, an amino group, a cyano group, a nitro group, a formyl group, a sulfanyl group, an oxo group, a C1-C3 alkyl group, a C1-C3 hydroxyalkyl group, a C1-C6 alkoxy group, a C1-C3 aminoalkyl group, a C1-C3 haloalkyl group, a group represented by -N-R²R³, a group represented by -COO-R², a group represented by -CONH-R², a group represented by -NHCO-R², a group represented by -NHSO₂-R², and a group represented by -NHSO₂NH-R²,
   when R¹ is a C1-C6 alkyl group, the C1-C6 alkyl group may be substituted with one or more substituents selected from a halogen atom; a hydroxy group; an amino group; a nitro group; a cyano group; an oxo group; a 3-to 6-membered cycloalkyl group which may be substituted with a hydroxy group, an amino group, a group represented by -CONH-R², a group represented by -NHCO-R², or a group represented by -NHSO₂-R²; a 4- to 8-membered heterocycloalkyl group which may be substituted with an oxo group; a 6- to 10-membered aryl group which may be substituted with a halogen atom or a C1-C3 alkyl group; a 5- to 10-membered heteroaryl group; a group represented by -O-R²; a group represented by -O-CO-R²; a group represented by -O-CONH-R²; a group represented by -N-R²R³; a group represented by -S-R²; a group represented by -CONH-R²; a group represented by - NHCO-R²; a group represented by -COO-R²; a group represented by -NHCOO-R²; a group represented by - NHCONH-R²; a group represented by -NHSO₂-R²; a group represented by -CO-R²; a group represented by -SO₂-R²; and a group represented by -NHSO₂NH-R², wherein
      R² represents a hydrogen atom, a C1-C6 alkyl group, a C2-C6 alkenyl group, a C2-C6 alkynyl group, a C1-C3 haloalkyl group, a hydroxy group, a C1-C3 hydroxyalkyl group, a C1-C6 alkoxy group, a C1-C3 haloalkoxy group, a 3- to 6-membered cycloalkyl group, a 3- to 6-membered cycloalkyl C1-C3 alkyl group, a 4-to 8-membered heterocycloalkyl group, a 6- to 10-membered aryl group, a 6- to 10-membered aryl C1-C3 alkyl group, a 5- to 10-membered heteroaryl group, a 5-to 10-membered heteroaryl C1-C3 alkyl group, a C1-C3 alkoxy C1-C3 alkyl group, an amino group, a C1-C6 alkylamino group, a C1-C6 dialkylamino group, a sulfanyl group, or a C1-C6 trialkylsilyl group, wherein the 5- to 10-membered heteroaryl group represented by R² may be further substituted with one or more substituents selected from a halogen atom, a hydroxy group, an amino group, a cyano group, a C1-C3 alkyl group, a C1-C3 hydroxyalkyl group, a C1-C6 alkoxy group, a C1-C3 aminoalkyl group, a C1-C3 haloalkyl group, a group represented by -N-R²R³, and a group represented by -CONH-R², and the heteroaryl group of the 5- to 10-membered heteroaryl C1-C3 alkyl group represented by R² may be further substituted with an amino group or a C1-C3 alkyl group,
      R³ represents a hydrogen atom or a C1-C6 alkyl group, and
      R⁴ and R⁵ each represent a hydrogen atom, or independently represent a C1-C10 alkyl group which may be bonded to each other to form a ring structure, and
   numbers 3, 4, 22, and 23 indicate position numbers of carbon atoms, and a bond between positions 3 and 4 and a bond between positions 22 and 23 may each independently be a single bond or a double bond.].
[2] The compound or pharmaceutically acceptable salt thereof according to [1], wherein in the formula (I), X is N, Y is O, and the bond between positions 3 and 4 is a single bond.
[3] The compound or pharmaceutically acceptable salt thereof according to [1] or [2], wherein R¹ in the formula (I) is a C1-C6 alkyl group which may be substituted.
[4] The compound or pharmaceutically acceptable salt thereof according to any one of [1] to [3], wherein R¹ in the formula (I) is a C1-C6 alkyl group substituted with a 5- to 10-membered heteroaryl group, a group represented by -CONH-R², or a group represented by -COR².
[5] The compound or pharmaceutically acceptable salt thereof according to [1], wherein in the formula (I), X is N, Y is O, and the bond between positions 3 and 4 is a double bond.
[6] The compound or pharmaceutically acceptable salt thereof according to [1], [2], [3], or [5], wherein in the formula (I), X is N, Y is O, and R¹ is a hydrogen atom; a C2-C6 alkenyl group; a C2-C6 alkynyl group; a halogen atom; a 3- to 6-membered cycloalkyl group which may be substituted with a hydroxy group; a 4- to 8-membered heterocycloalkyl group; or a C1-C6 alkyl group substituted with one or more substituents selected from a group represented by -O-R², a group represented by - O-CONH-R², a group represented by -COO-R², a group represented by -CONH-R², a group represented by -NHCONH-R², a group represented by -CO-R², and a group represented by -NHSO₂-R²
[7] The compound or pharmaceutically acceptable salt thereof according to any one of [1] to [6], wherein the compound represented by the formula (I) is selected from compounds represented by the following formulas (1) to (94) : 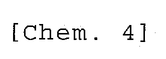 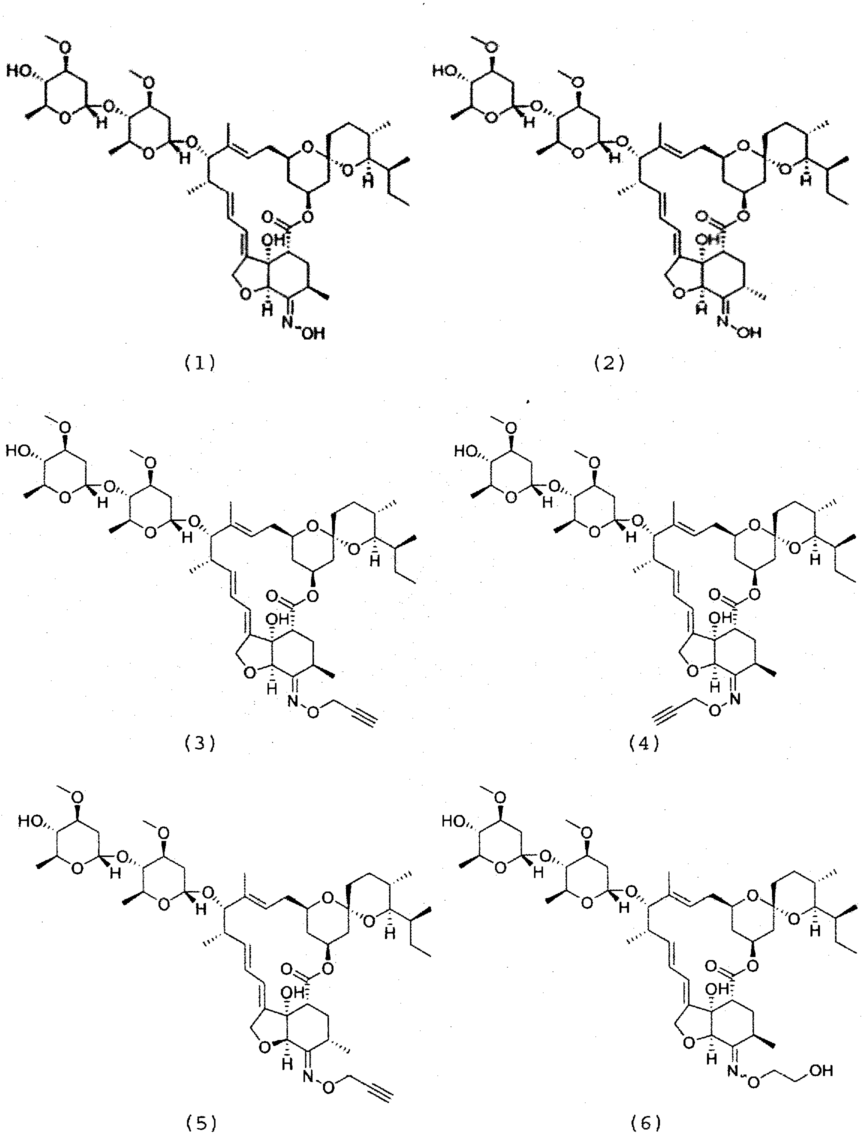 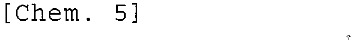 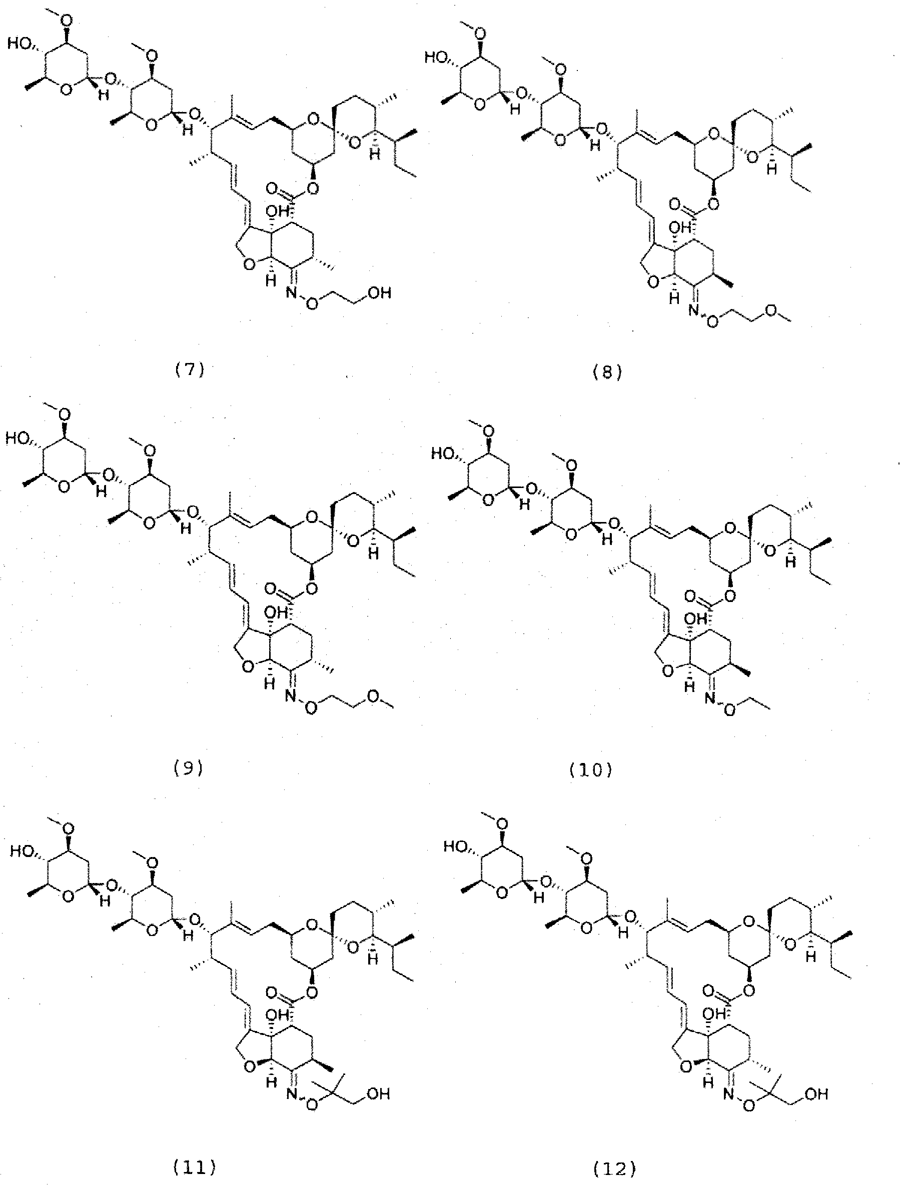 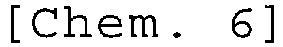 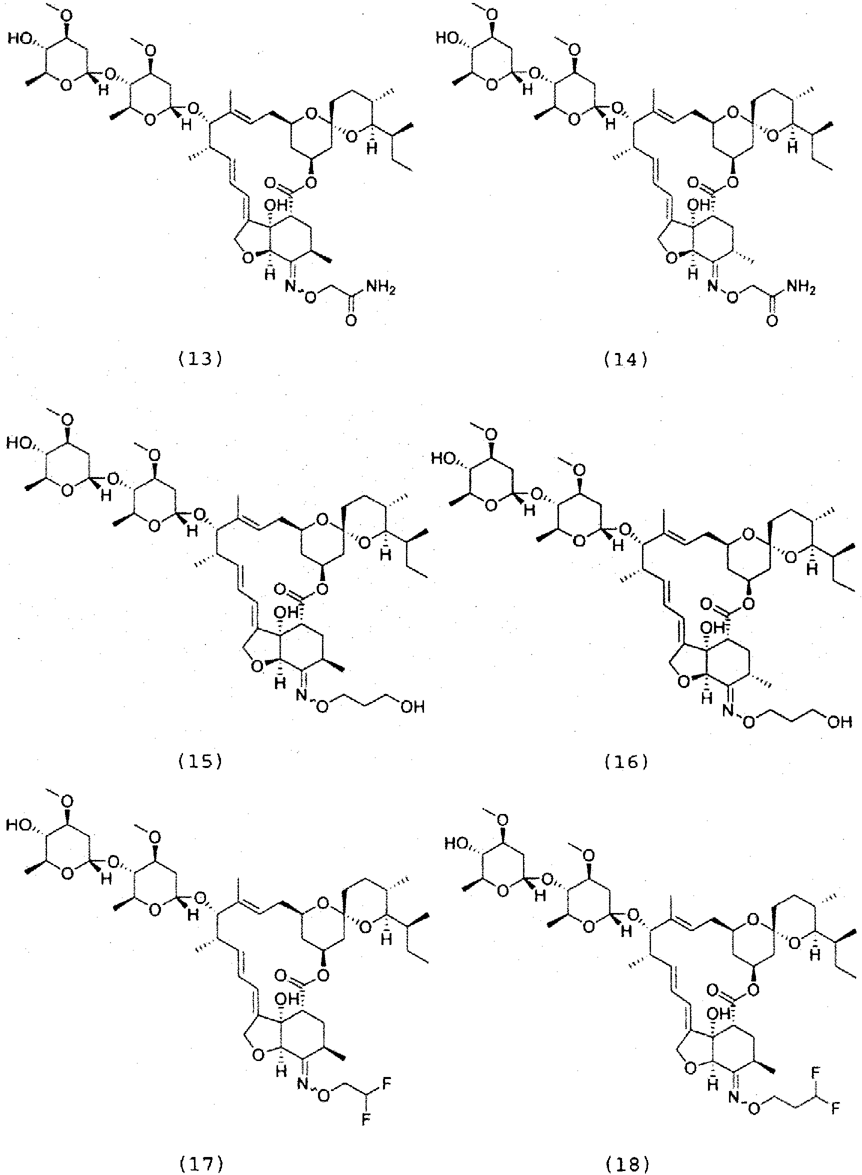 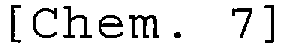 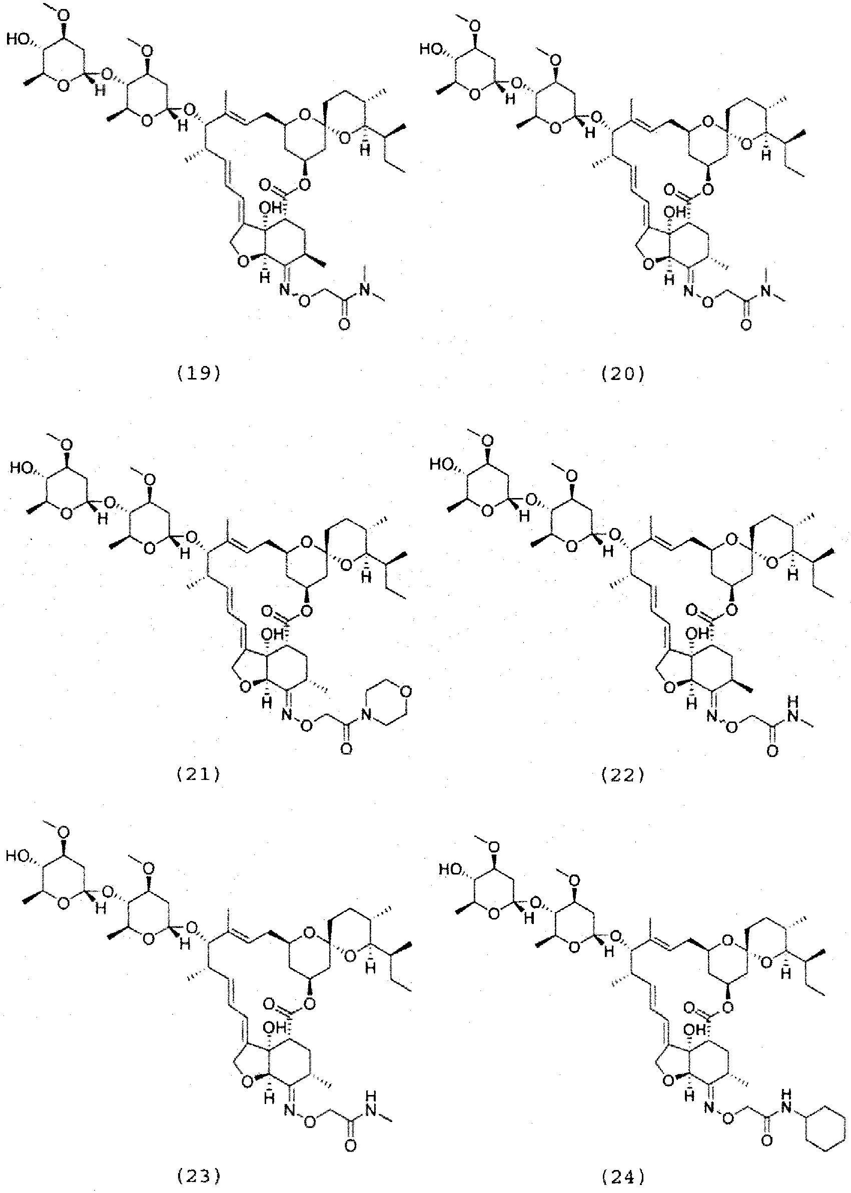 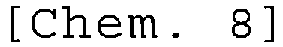 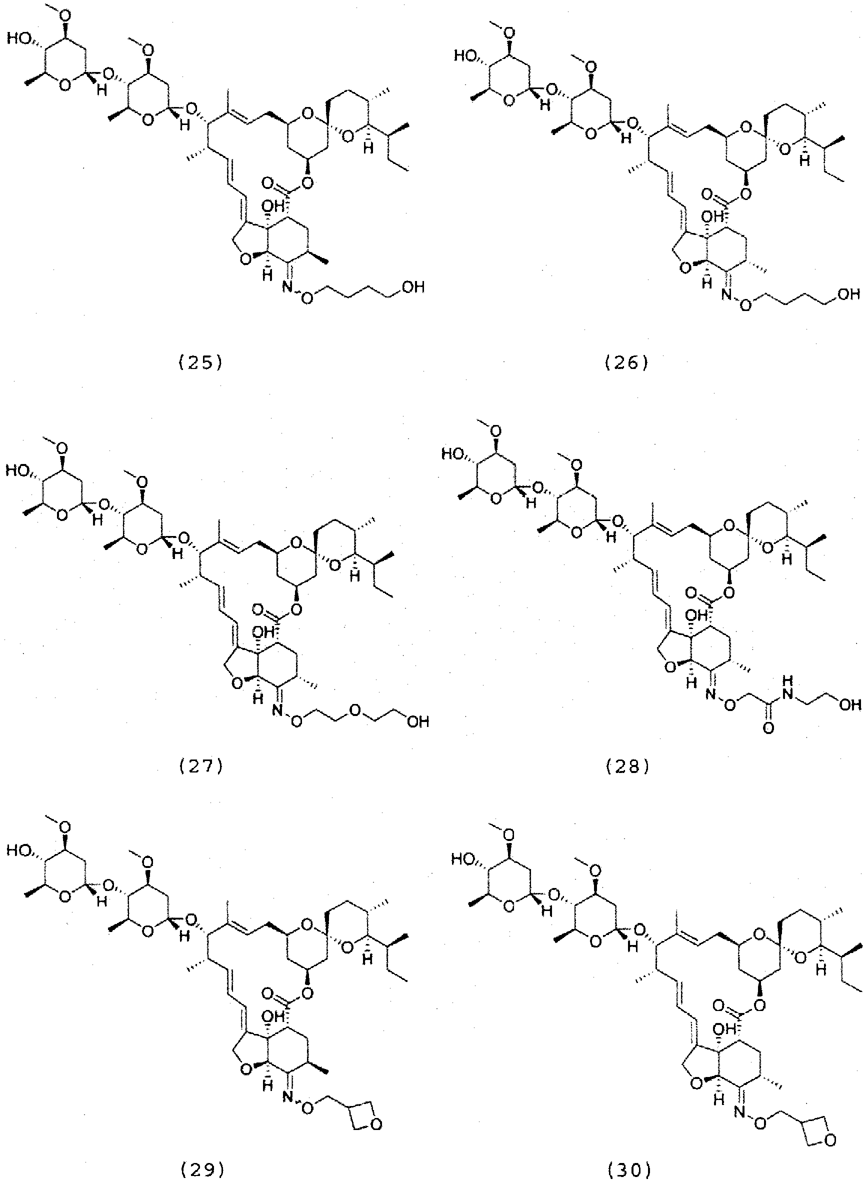 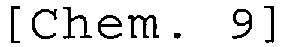 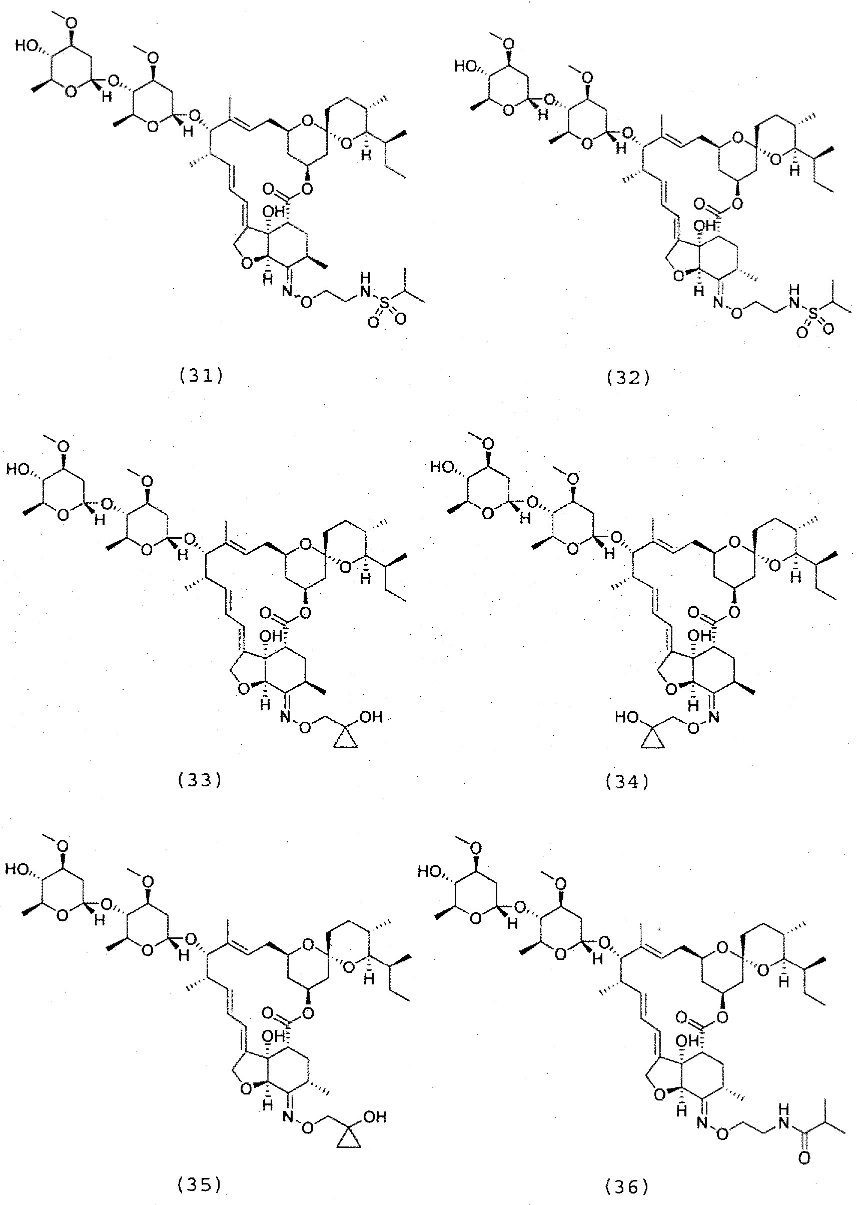 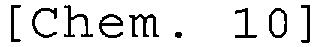 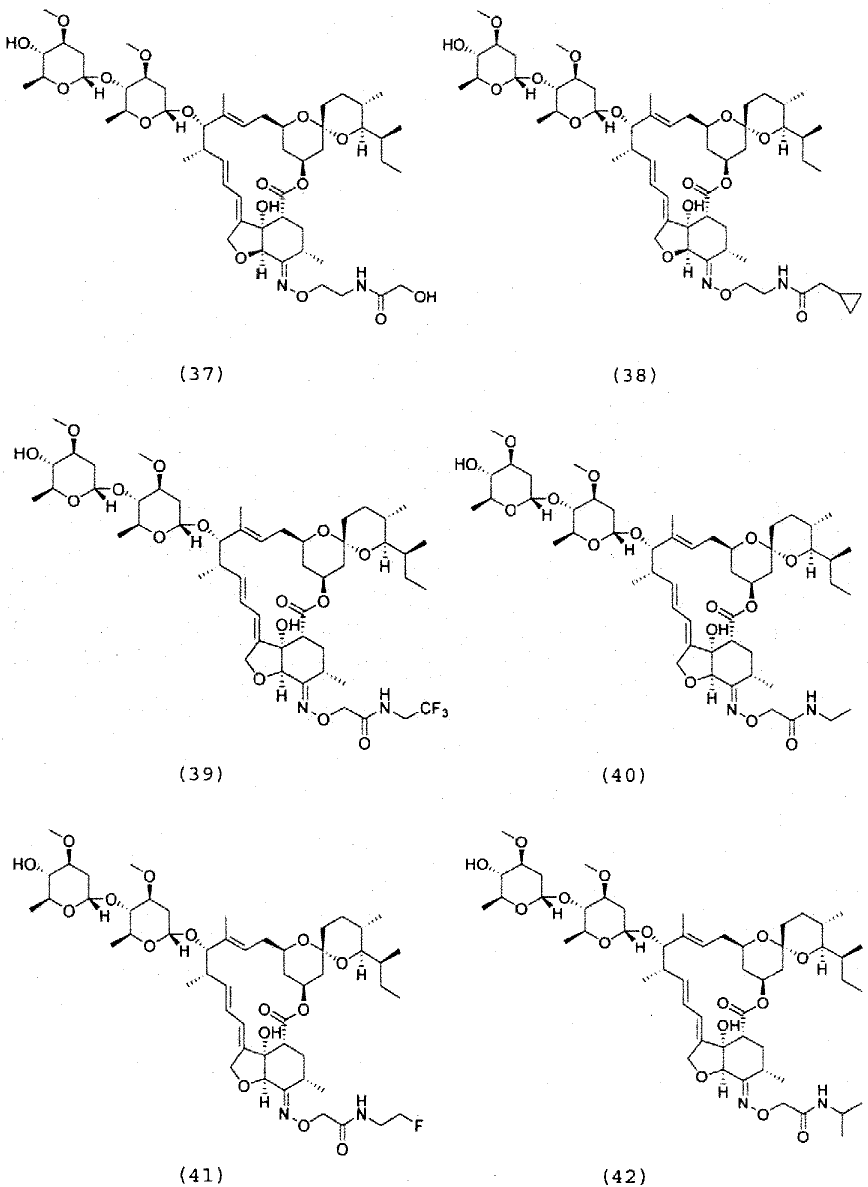 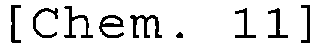 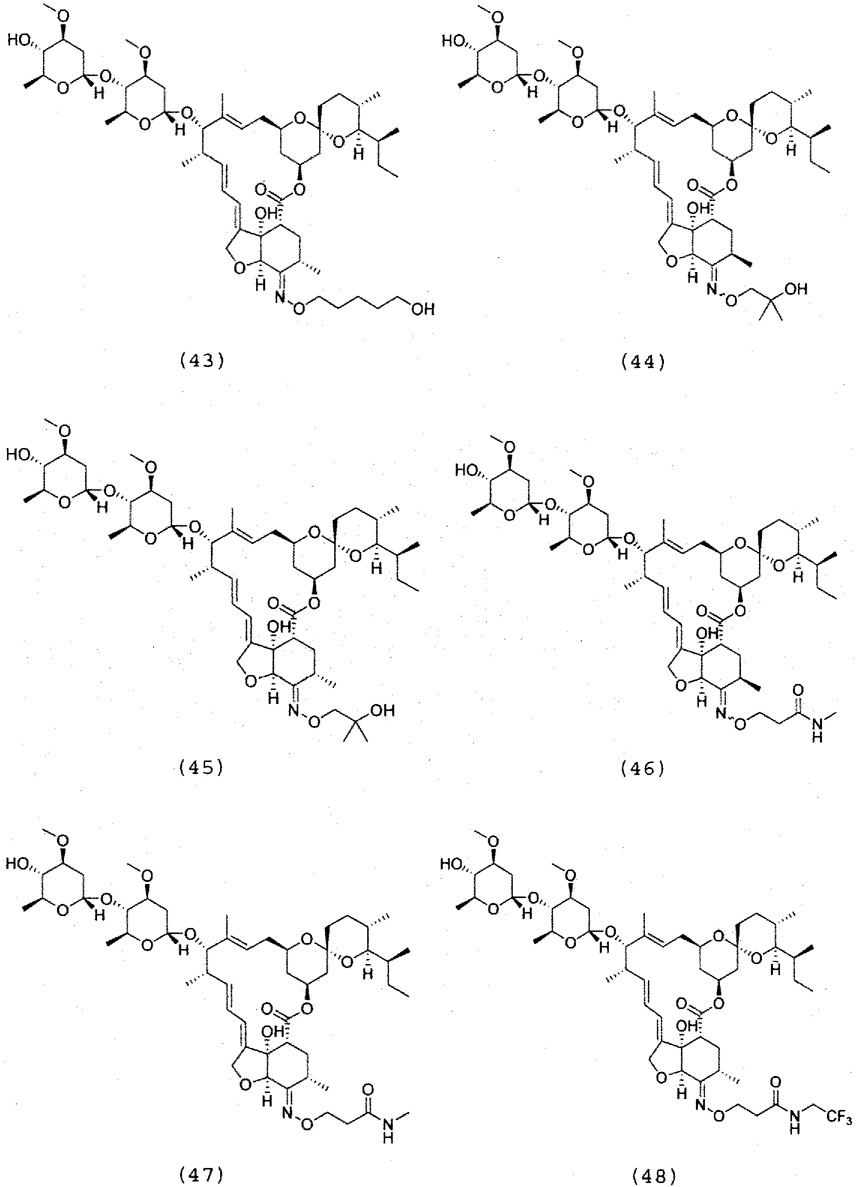 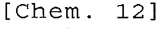 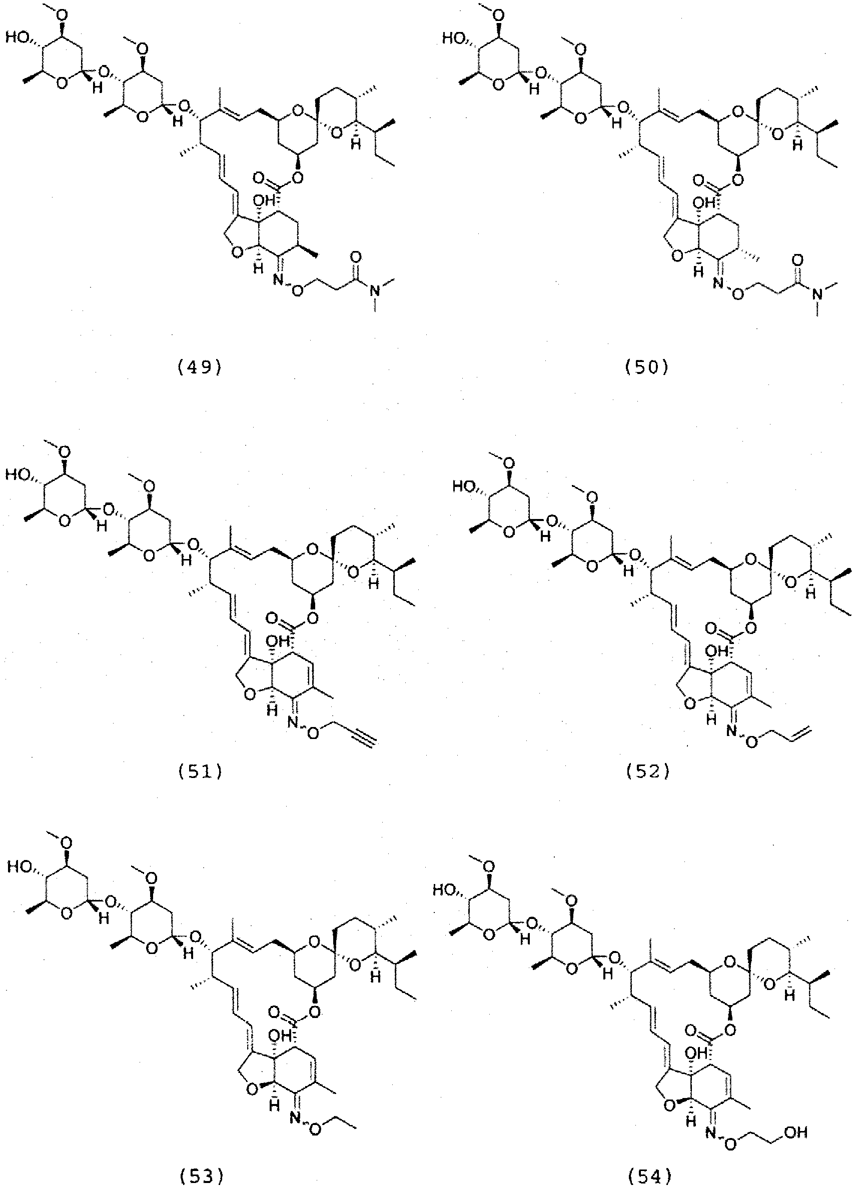 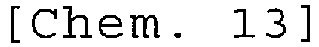 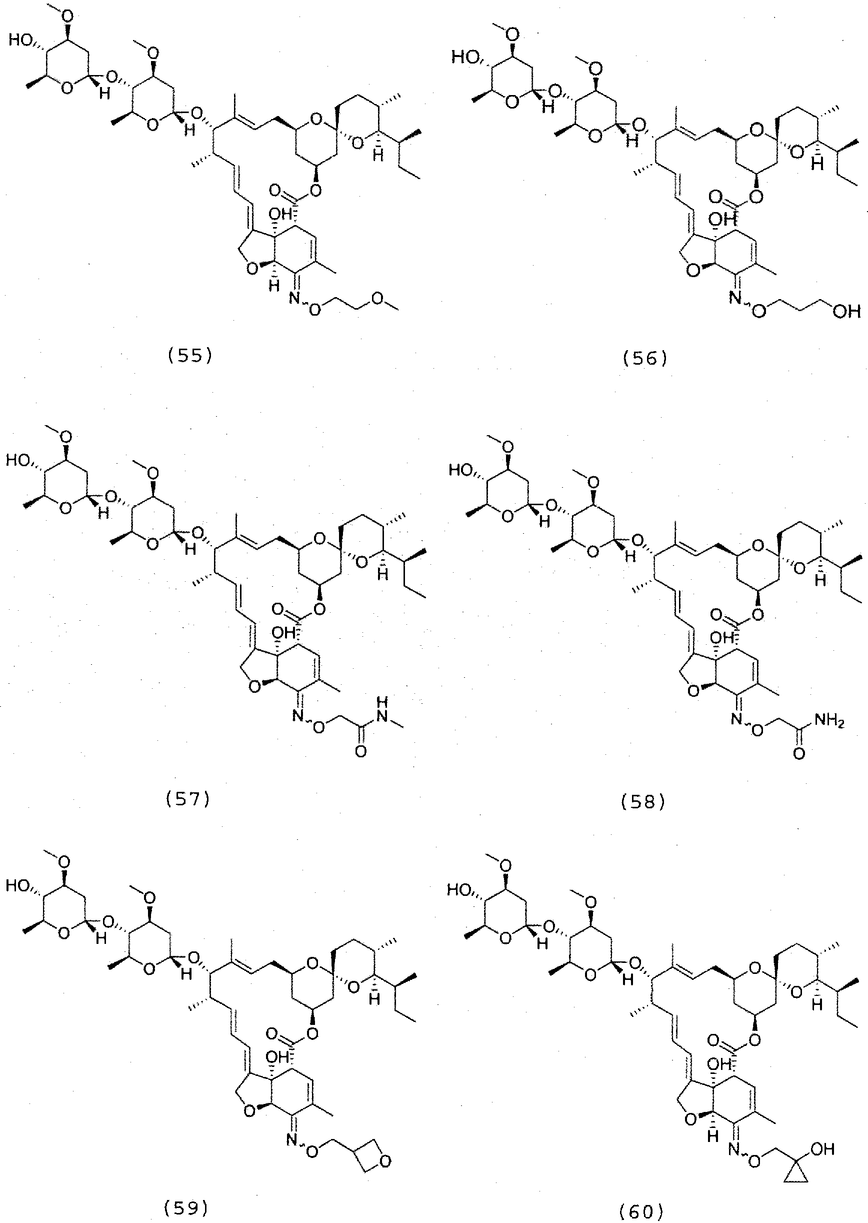 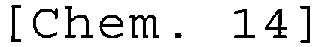 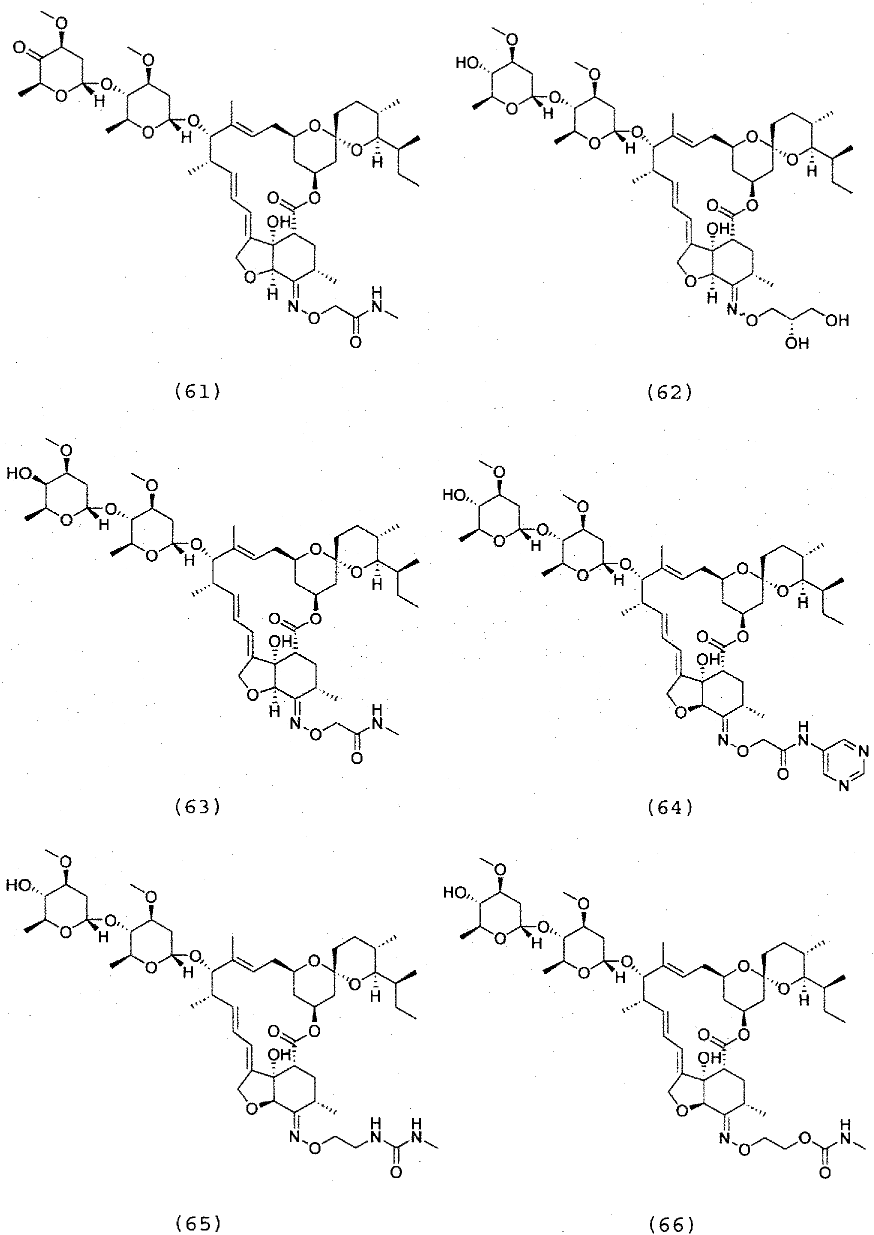 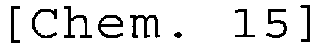 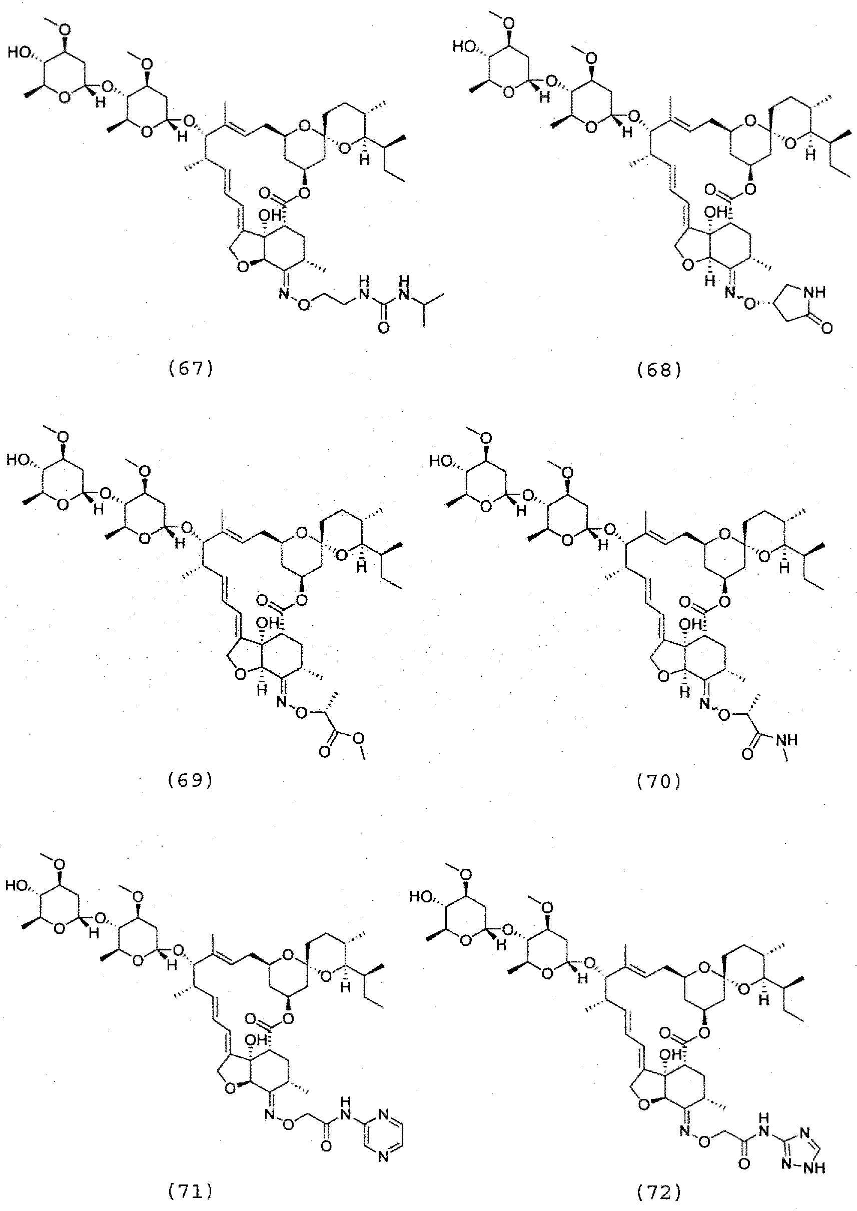 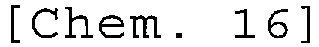 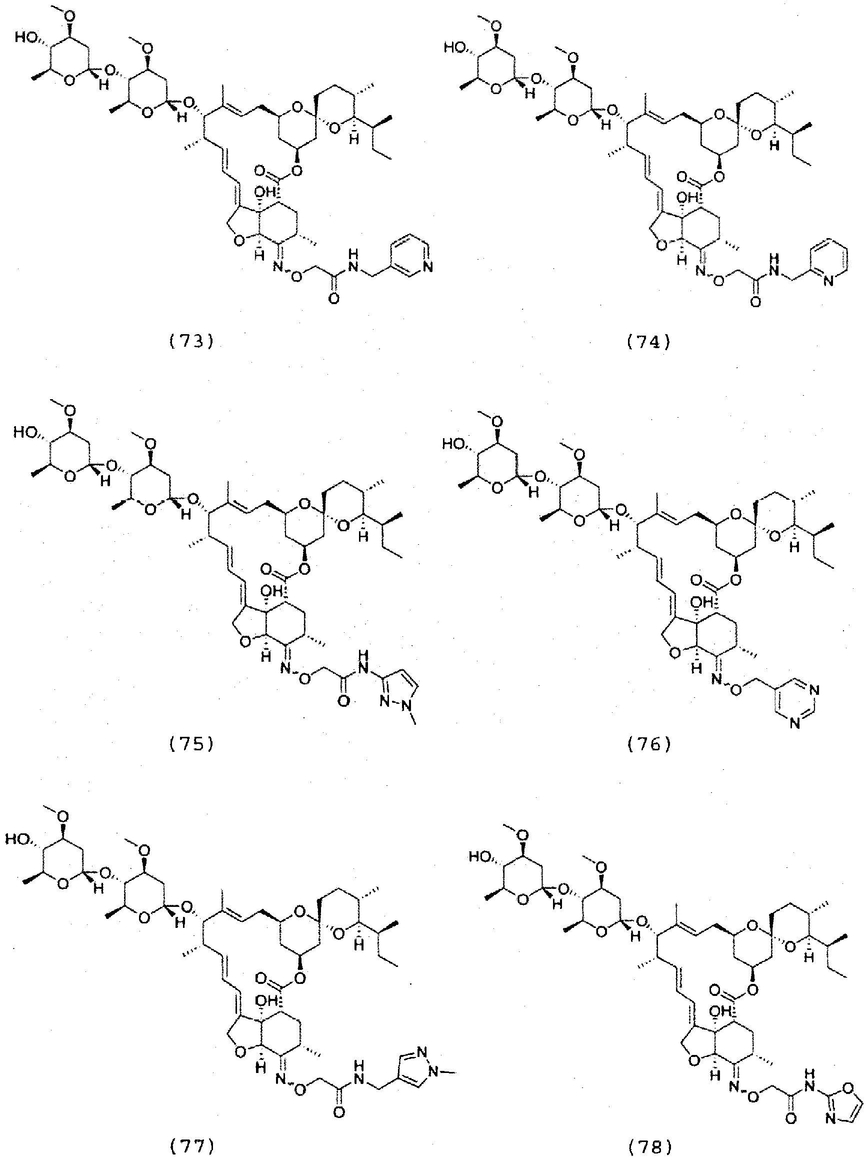 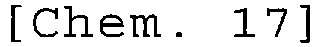 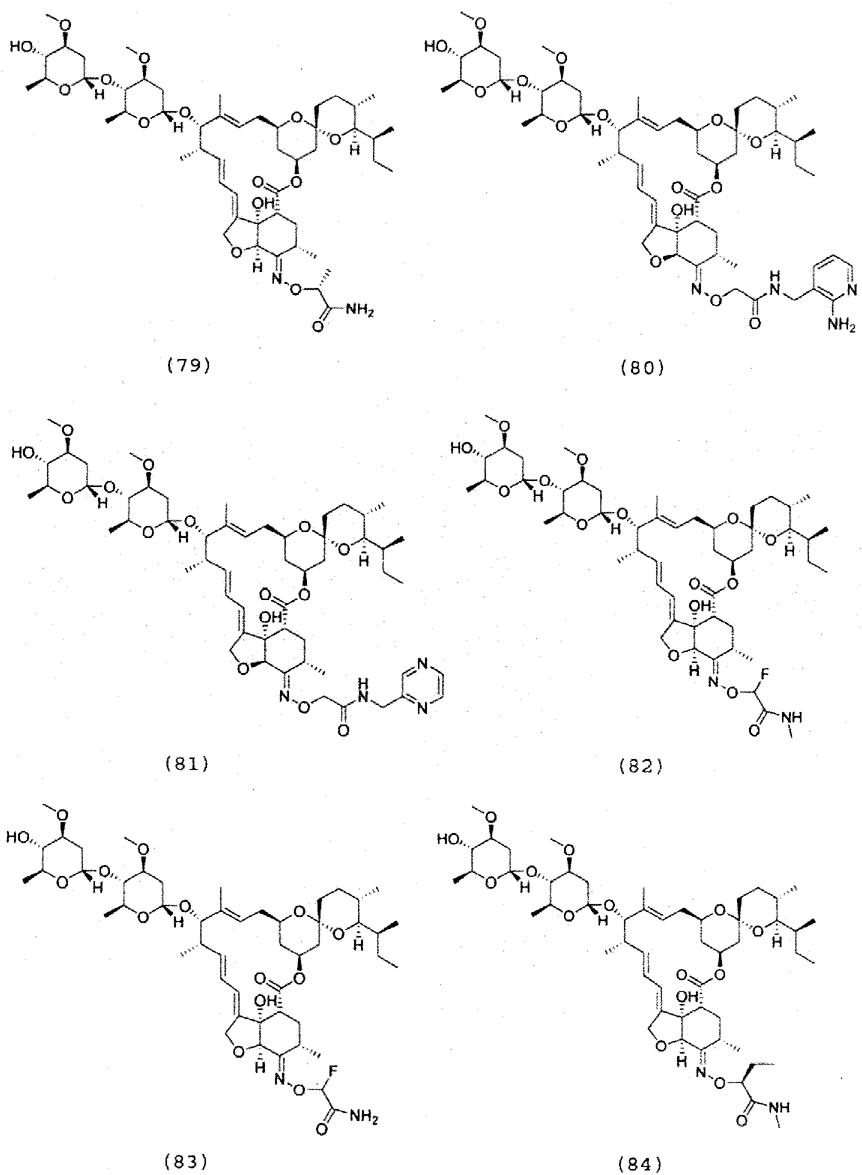 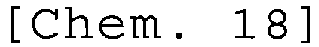 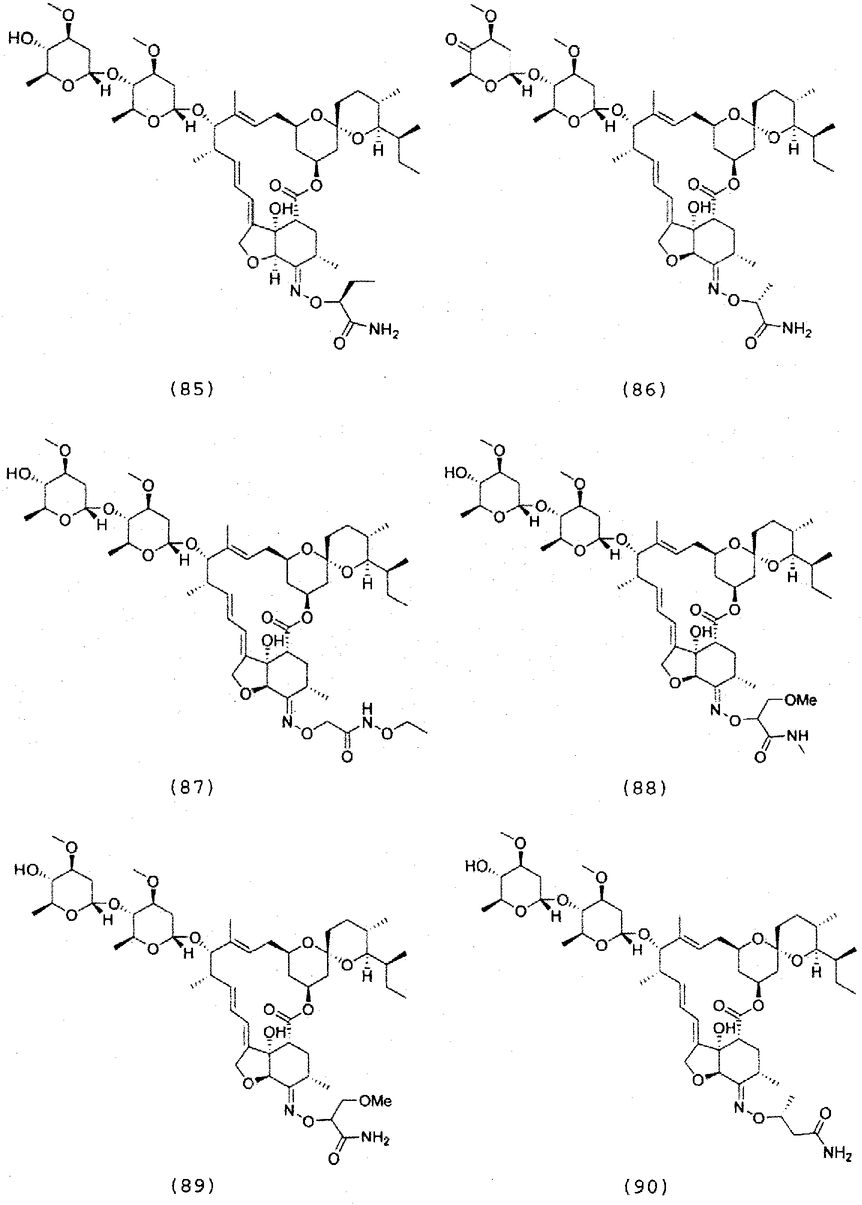 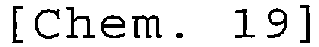 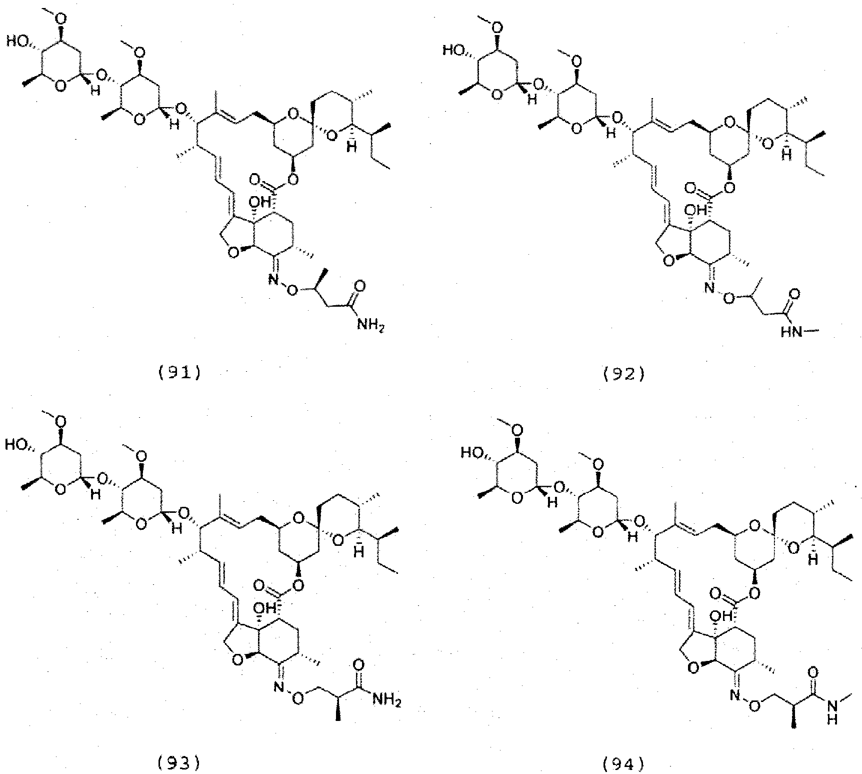
[8] The compound or pharmaceutically acceptable salt thereof according to [1], wherein X in the formula (I) is CH, CF, or CHCH=CH, and the bond between positions 3 and 4 is a single bond.
[9] The compound or pharmaceutically acceptable salt thereof according to [1], wherein X in the formula (I) is CH, CF, or CHCH=CH, and the bond between positions 3 and 4 is a double bond.
[10] The compound or pharmaceutically acceptable salt thereof according to [1], [7], or [8], wherein in the formula (I), X is CH, CF, or CHCH=CH, Y is a single bond,
   R¹ is a 4- to 8-membered heterocycloalkyl group which may be substituted with an oxo group; a group represented by -COO-R²; a 5- to 10-membered heteroaryl group which may be substituted with one or more (preferably one) substituents selected from a halogen atom, a hydroxy group, an amino group, a cyano group, a formyl group, an oxo group, a C1-C3 alkyl group, a C1-C3 hydroxyalkyl group, a C1-C6 alkoxy group, a C1-C3 haloalkyl group, a group represented by -N-R²R³, a group represented by -NHCO-R², and a group represented by -CONH-R²; or a C1-C6 alkyl group which may be substituted with one or more substituents selected from a hydroxy group, an amino group, a nitro group, an oxo group, a group represented by -CONH-R², and a group represented by -COO-R².
[11] The compound or pharmaceutically acceptable salt thereof according to [1] or [8], wherein the compound represented by the formula (I) is selected from compounds represented by the following formulas (201) to (271): 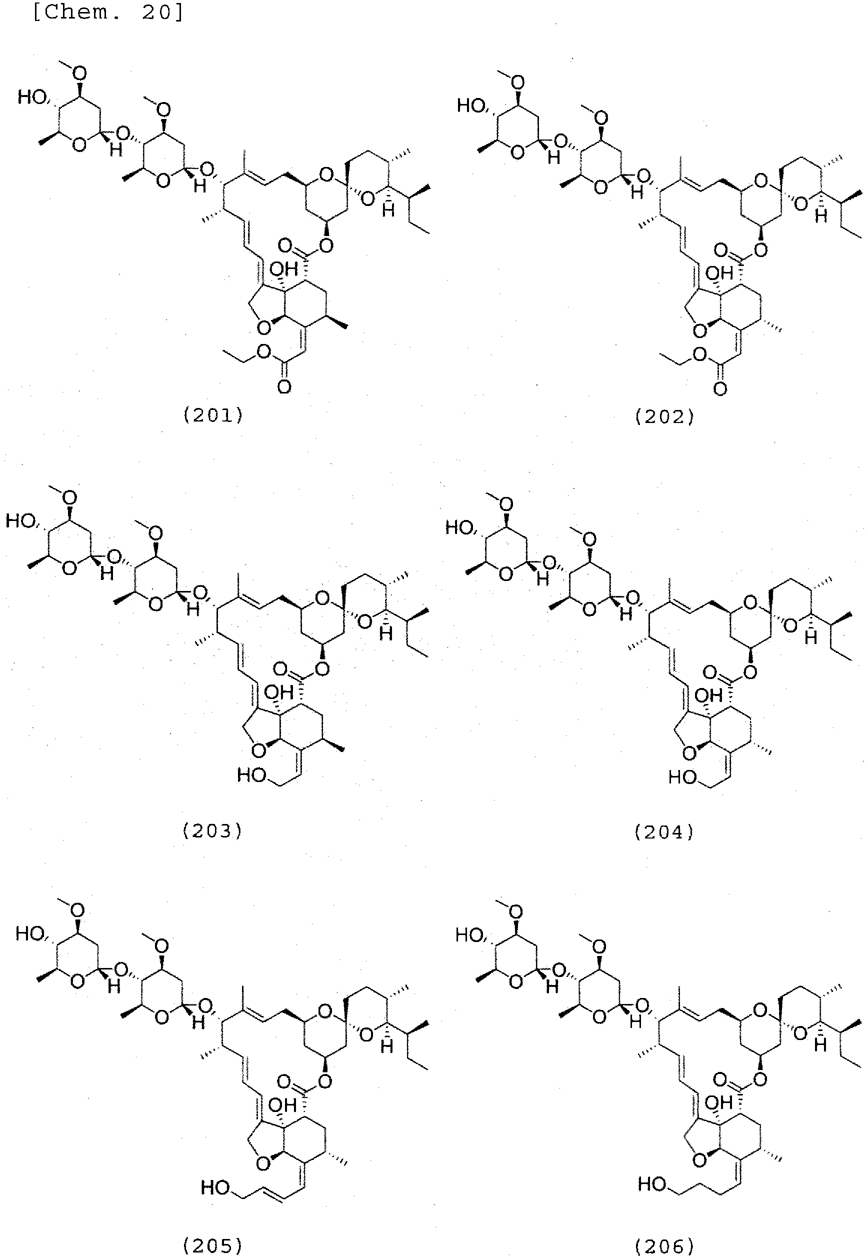 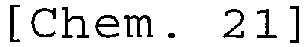 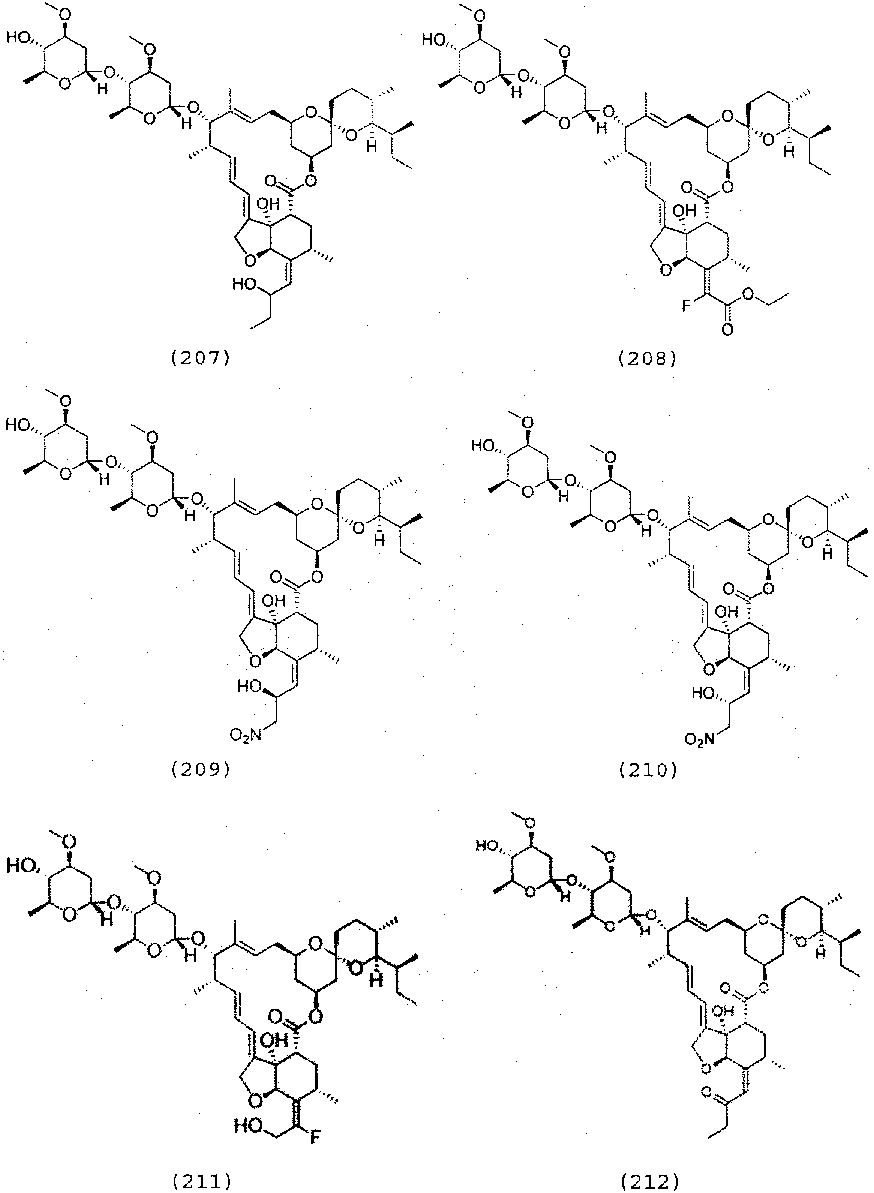 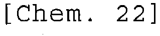 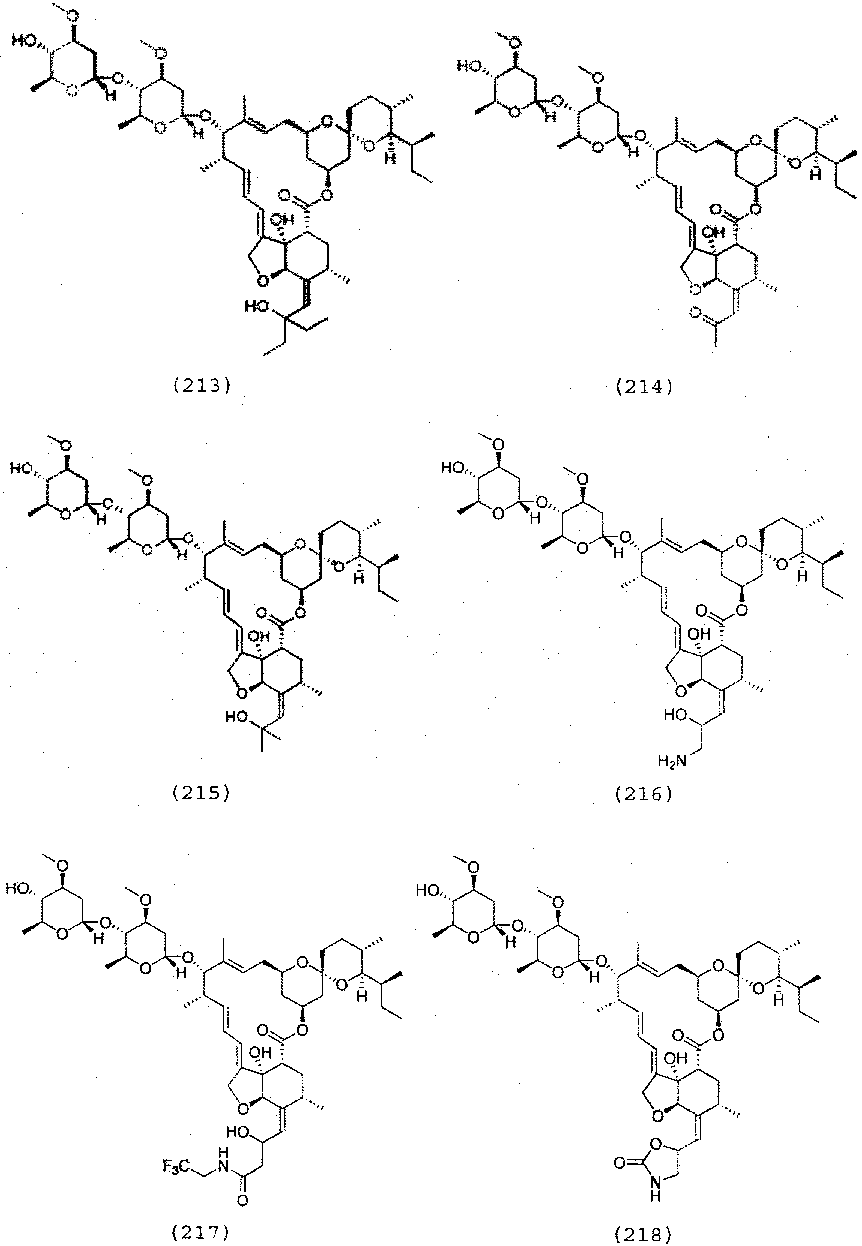 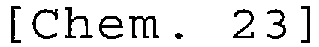 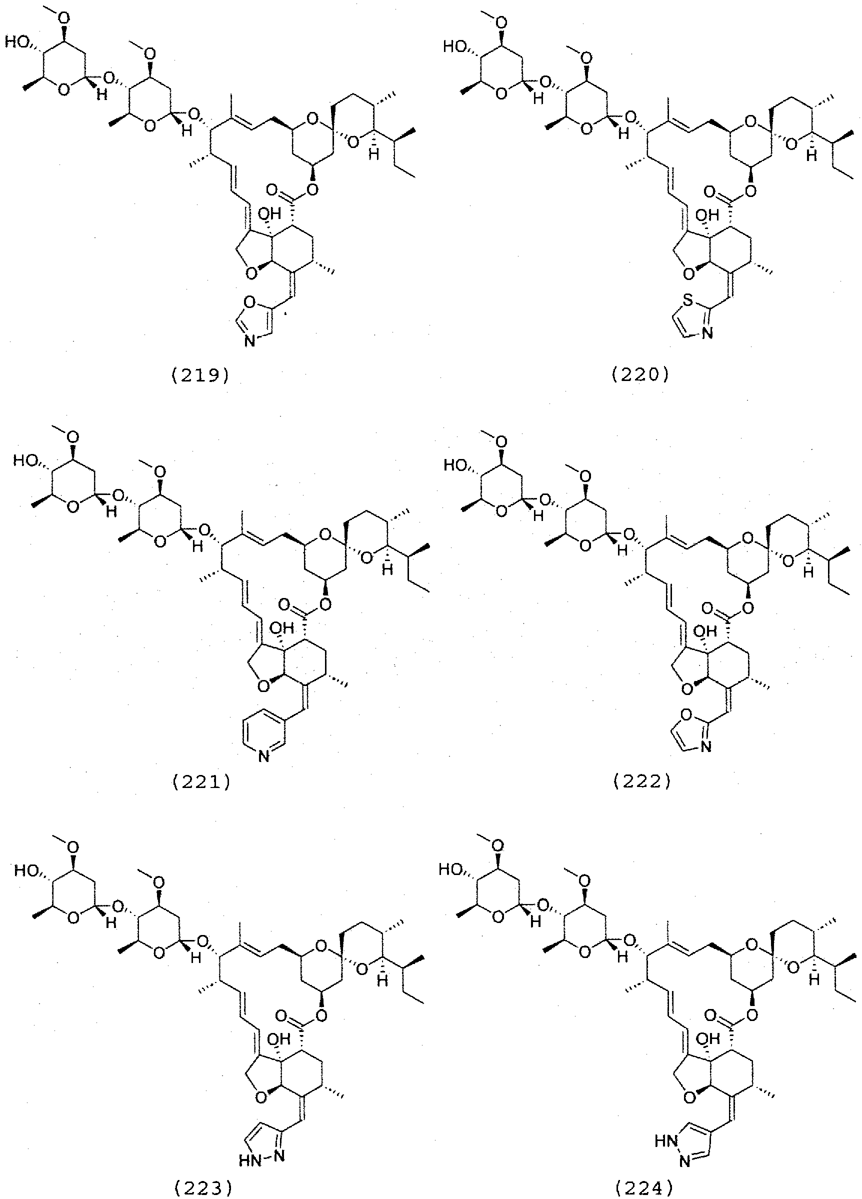 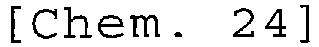 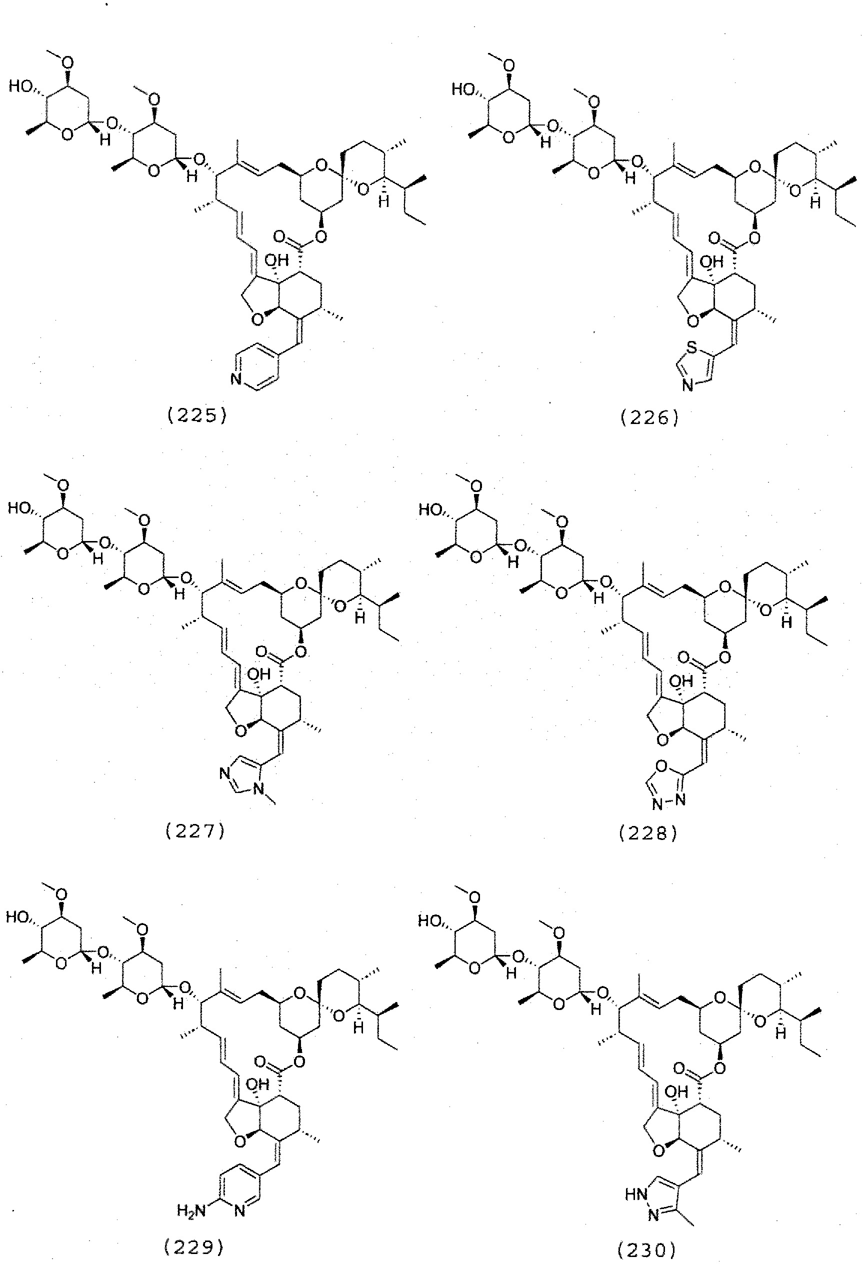 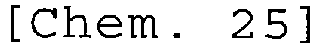 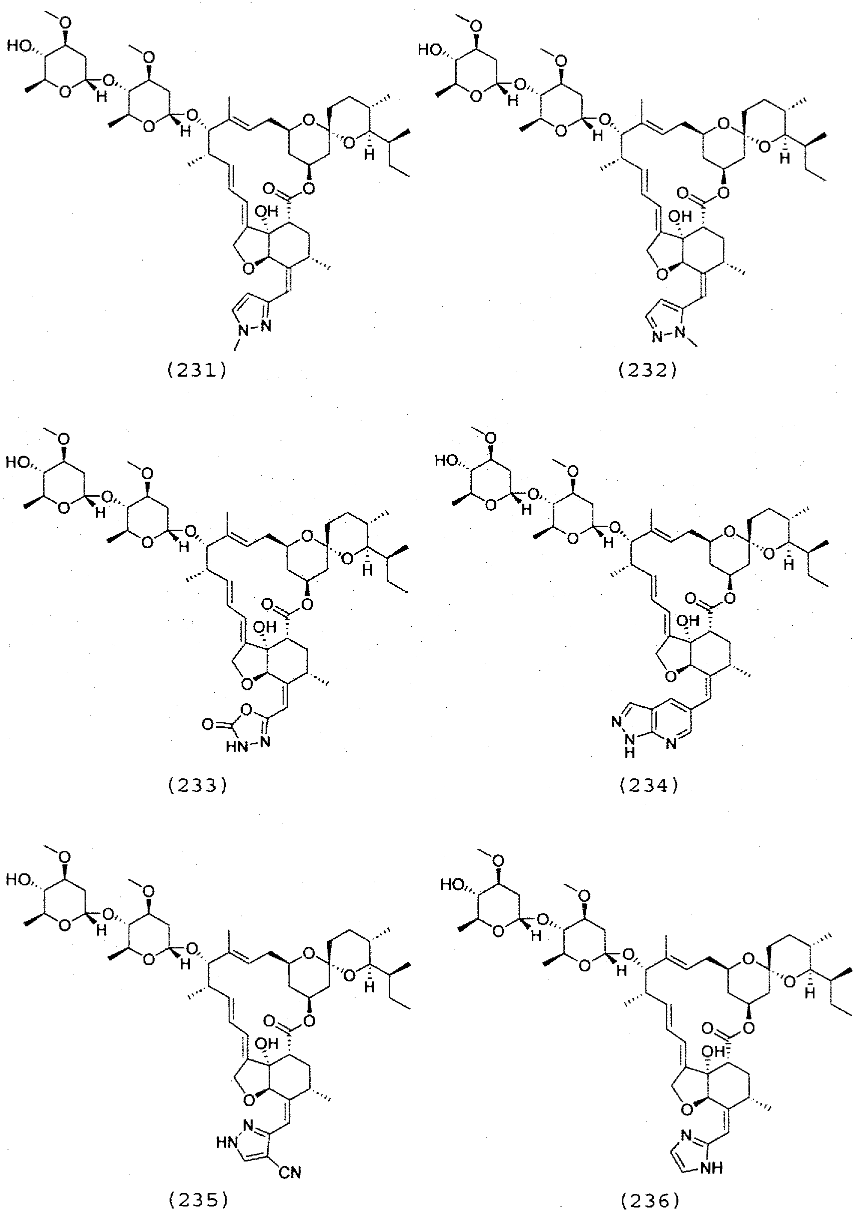 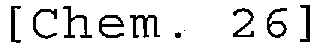 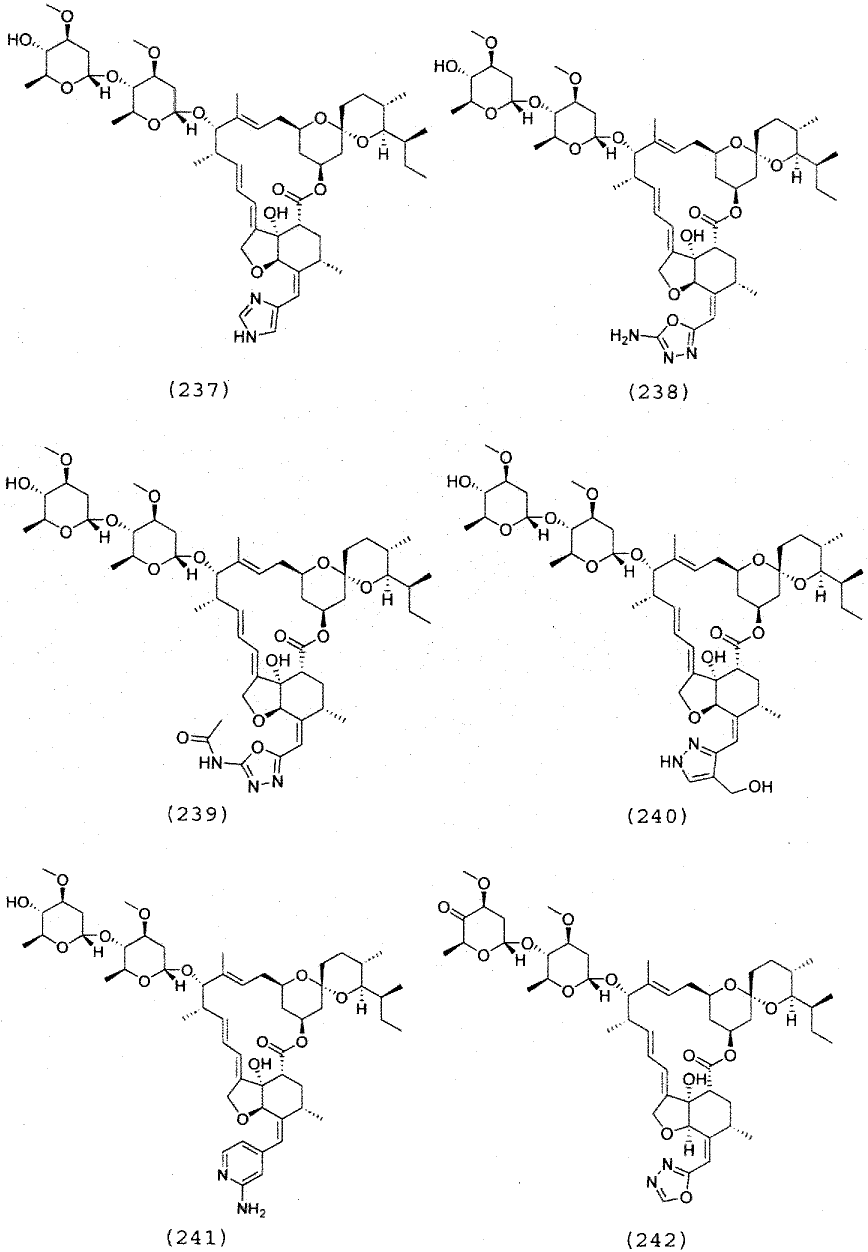 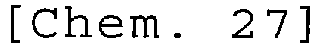 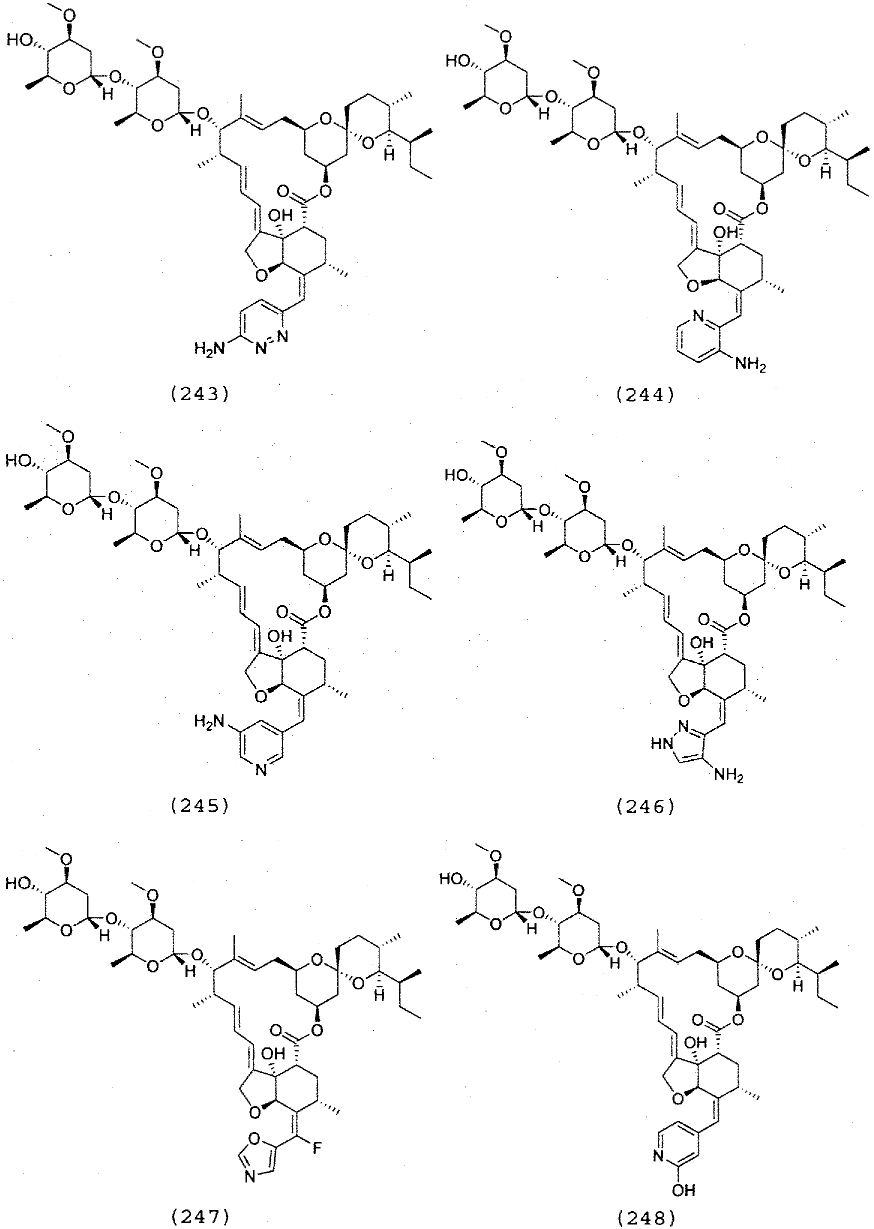 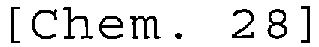 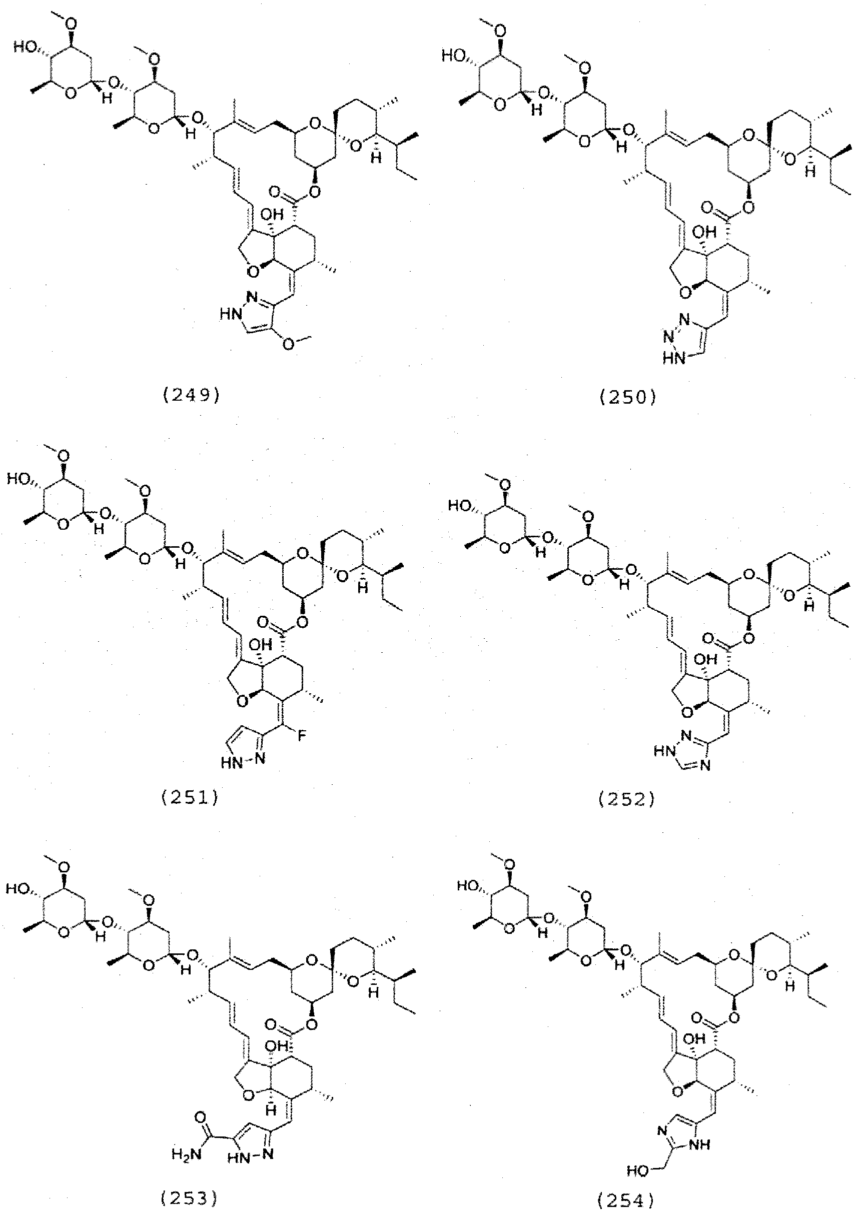 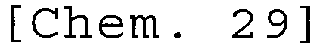 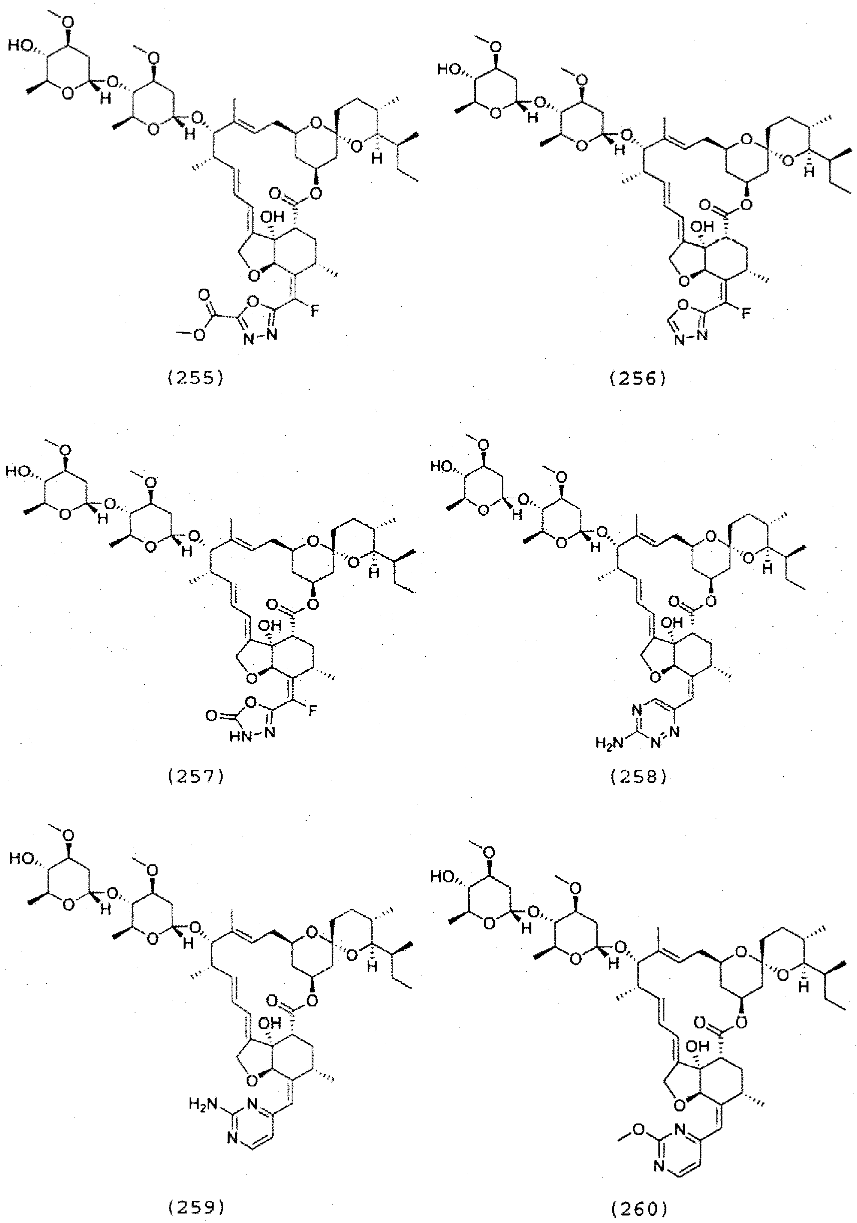 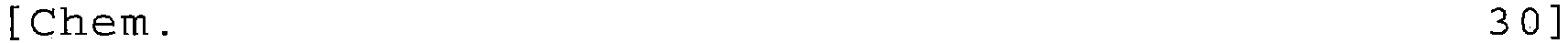 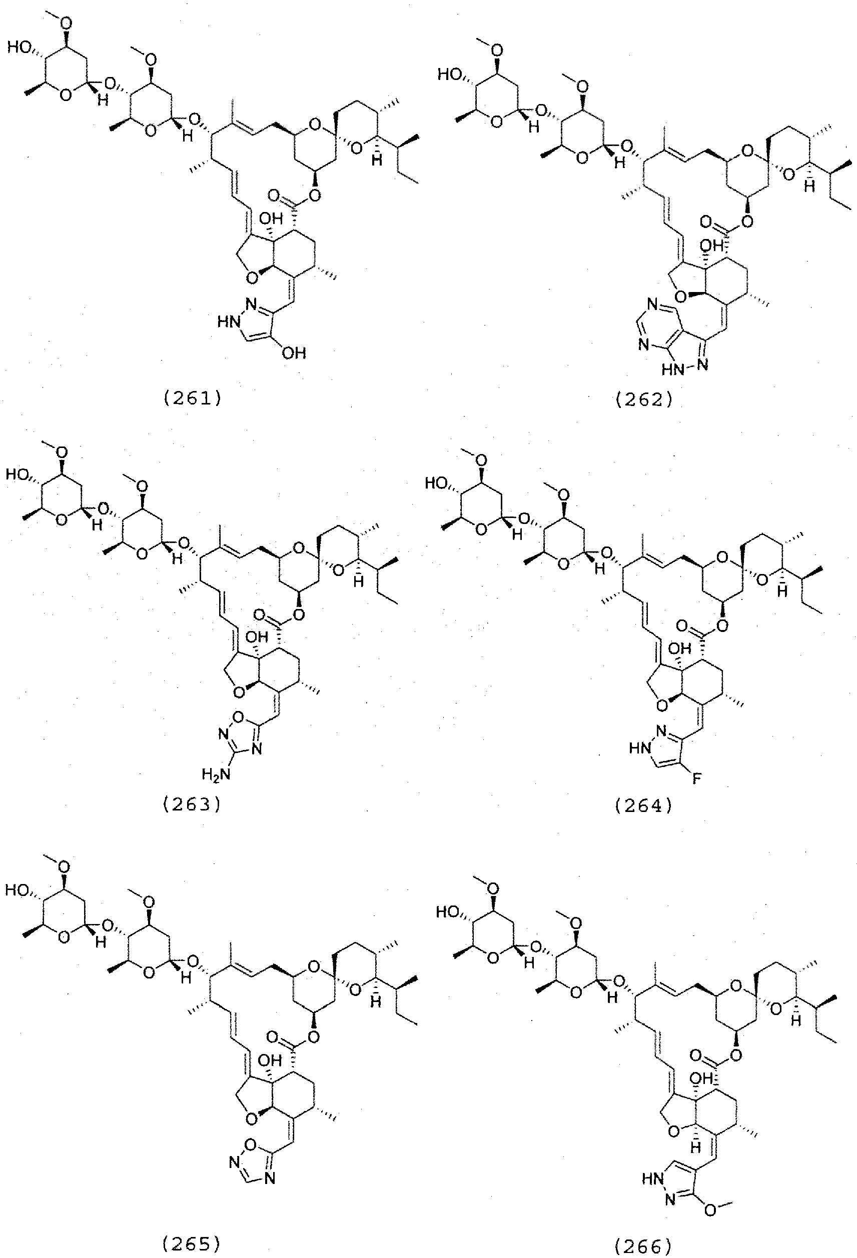 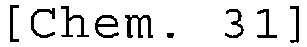 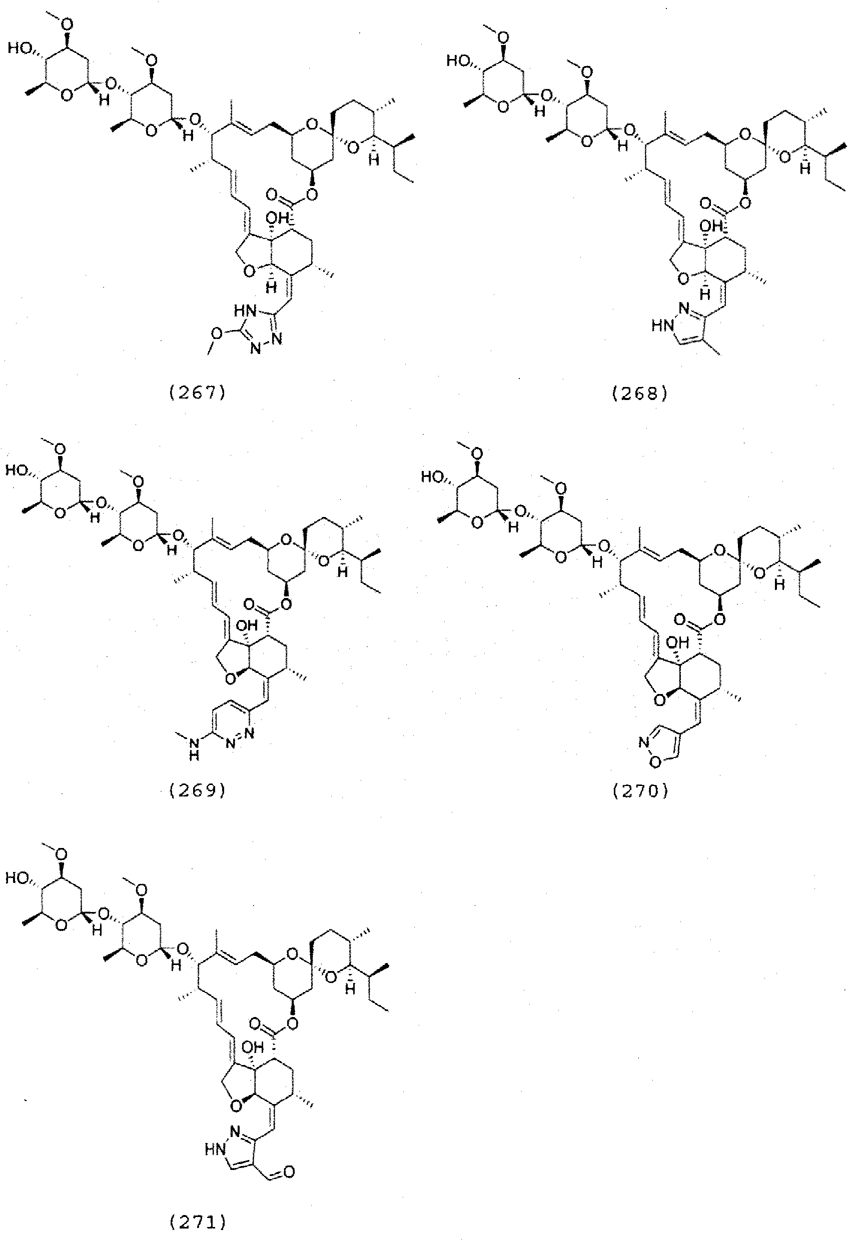
[12] The compound or pharmaceutically acceptable salt thereof according to [1], wherein in the formula (I), X is N, Y is NH, and R¹ is a group represented by -COR².
[13] The compound or pharmaceutically acceptable salt thereof according to any one of [1] to [12], which has antiviral activity against coronaviruses.
[14] The compound or pharmaceutically acceptable salt thereof according to [13], wherein the coronavirus is a coronavirus classified in the genus Betacoronavirus.
[15] The compound or pharmaceutically acceptable salt thereof according to [13] or [14], wherein the coronavirus is at least one selected from the group consisting of human coronavirus (HCoV), severe acute respiratory syndrome coronavirus (SARS-CoV), novel coronavirus (SARS-CoV-2), and Middle East respiratory syndrome coronavirus (MERS-CoV).
[16] A composition for the treatment or prevention of a disease or condition involving a coronavirus, comprising the compound or pharmaceutically acceptable salt thereof according to any one of [1] to [12] as an active ingredient.
[17] The composition according to [16], wherein the coronavirus is a coronavirus classified in the genus Betacoronavirus.
[18] The composition according to [16] or [17], wherein the coronavirus is at least one selected from the group consisting of human coronavirus (HCoV), severe acute respiratory syndrome coronavirus (SARS-CoV), novel coronavirus (SARS-CoV-2), and Middle East respiratory syndrome coronavirus (MERS-CoV).
[19] A method for treating or preventing a disease or condition involving a coronavirus, comprising the step of administering to a target the compound or pharmaceutically acceptable salt thereof according to any one of [1] to [12].
[20] Use of the compound or pharmaceutically acceptable salt thereof according to any one of [1] to [12] for the manufacture of a composition for the treatment or prevention of a disease or condition involving a coronavirus.

### [Advantageous Effects of Invention]

According to the present invention, it is possible to provide a novel compound having antiviral activity against coronaviruses and useful for the treatment or prevention of diseases or conditions involving coronaviruses, and a composition for the treatment or prevention of diseases or conditions involving coronaviruses, comprising the same as an active ingredient.

### [Description of Embodiments]

Hereinafter, the present invention will be described in detail in accordance with preferred embodiments thereof. In the following description, the same or corresponding elements are denoted by the same reference numerals, and redundant descriptions are omitted.

### <Avermectin Derivative>

The present invention provides a compound represented by the following general formula (I): 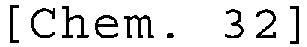 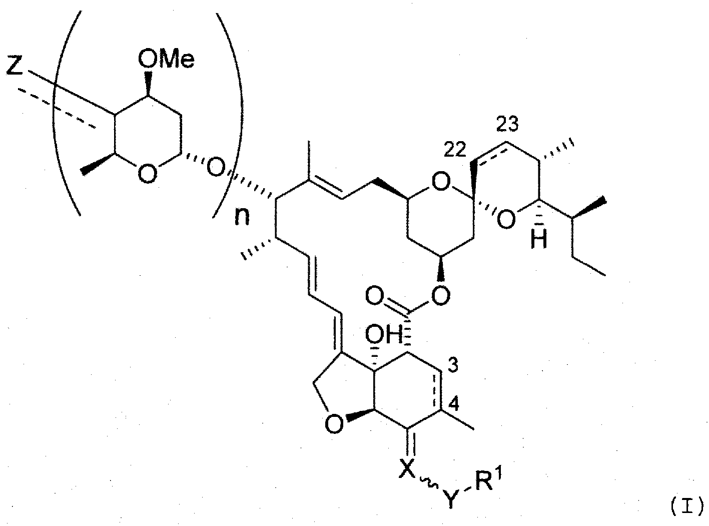 and a pharmaceutically acceptable salt thereof (hereinafter, collectively referred to as "the compound of the present invention" in some cases). The compound represented by the formula (I) is an avermectin derivative having a 16-membered macrolide structure, a hexahydrobenzofuran skeleton, and a bispyran structure containing a spiro ring, as described above. In the formula (I), numbers 3, 4, 22, and 23 indicate position numbers of carbon atoms, and a bond between positions 3 and 4 and a bond between positions 22 and 23 may each independently be a single bond or a double bond. Further, in the formula (I), Me represents a methyl group.

In the formula (I), n represents an integer of 0, 1, or 2.

In the formula (I), X represents CH, CF, CCl, N, CHCH=N, CHCH=CH, or CHCH=CF. When X represents CHCH=N, CHCH=CH, or CHCH=CF, it indicates that the structure represented by "=X~" is a structure represented by "=CHCH=N~," "=CHCH=CH~," or "=CHCH=CF~," respectively.

In the present specification, a wavy line indicating a single bond between atoms in a structural formula, for example, a wavy line (~) indicating a single bond between X and Y in the formula (I), indicates that, when geometric isomers exist, it represents either of the isomers ((E)-form, (Z)-form) shown below, and in this case, the compound represented by the formula (I) may be either of these isomers or a mixture thereof. In addition, in the present specification, a single bond indicated by a straight line (-) in a structural formula indicates that, when stereoisomers exist, it is either a "wedge bond" or a "dashed bond," and in this case, the compound represented by the formula (I) may be either of these isomers or a mixture thereof.

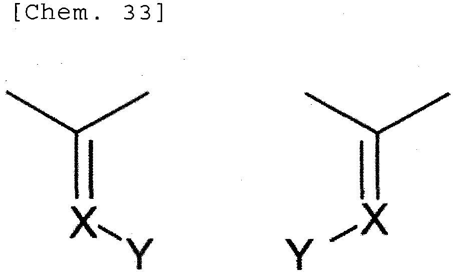

In the formula (I), Y represents O, NH, or a single bond.

In the formula (I), Z represents a hydroxy group or an oxo group. When Z is a hydroxy group, the bond between OH and the carbon atom to which it is bonded is a single bond, and when Z is an oxo group, the bond between O and the carbon atom to which it is bonded is a double bond.

In the formula (I), R¹ represents a hydrogen atom, a halogen atom, a C1-C6 alkyl group, a 3- to 6-membered cycloalkyl group, a 4- to 8-membered heterocycloalkyl group, a 6- to 10-membered aryl group, a 5- to 10-membered heteroaryl group, a C2-C6 alkenyl group, a C2-C6 alkynyl group, a group represented by -CO-R² (wherein O is bonded to C via a double bond, and R² is bonded via a single bond. The same shall apply hereinafter), a group represented by -COO-R², a group represented by -CONH-R², or a group represented by -B(OR⁴)OR⁵.

Here, when R¹ is other than a hydrogen atom and a C1-C6 alkyl group, the R¹ may be substituted with one or more substituents selected from a halogen atom, a hydroxy group, an amino group, a cyano group, a nitro group, a formyl group, a sulfanyl group, an oxo group, a C1-C3 alkyl group, a C1-C3 hydroxyalkyl group, a C1-C6 alkoxy group, a C1-C3 aminoalkyl group, a C1-C3 haloalkyl group, a group represented by -N-R²R³, a group represented by -COO-R², a group represented by -CONH-R², a group represented by -NHCO-R², a group represented by -NHSO₂-R², and a group represented by -NHSO₂NH-R². In the present specification, the phrase "each group is substituted with an oxo group" means that two hydrogen atoms bonded to a carbon atom contained in the group are substituted with one oxygen atom.

When R¹ is a C1-C6 alkyl group, the C1-C6 alkyl group may be substituted with one or more substituents selected from a halogen atom; a hydroxy group; an amino group; a nitro group; a cyano group; an oxo group; a 3-to 6-membered cycloalkyl group which may be substituted with a hydroxy group, an amino group, a group represented by -CONH-R², a group represented by -NHCO-R², or a group represented by -NHSO₂-R²; a 4- to 8-membered heterocycloalkyl group which may be substituted with an oxo group; a 6- to 10-membered aryl group which may be substituted with a halogen atom or a C1-C3 alkyl group; a 5- to 10-membered heteroaryl group; a group represented by -O-R²; a group represented by -O-CO-R²; a group represented by -O-CONH-R²; a group represented by -N-R²R³; a group represented by -S-R²; a group represented by -CONH-R²; a group represented by - NHCO-R²; a group represented by -COO-R²; a group represented by -NHCOO-R²; a group represented by - NHCONH-R²; a group represented by -NHSO₂-R²; a group represented by -CO-R²; a group represented by -SO₂-R²; and a group represented by -NHSO₂NH-R².

In the formula (I), R² in R¹ represents a hydrogen atom, a C1-C6 alkyl group, a C2-C6 alkenyl group, a C2-C6 alkynyl group, a C1-C3 haloalkyl group, a hydroxy group, a C1-C3 hydroxyalkyl group, a C1-C6 alkoxy group, a C1-C3 haloalkoxy group, a 3- to 6-membered cycloalkyl group, a 3- to 6-membered cycloalkyl C1-C3 alkyl group, a 4- to 8-membered heterocycloalkyl group, a 6- to 10-membered aryl group, a 6- to 10-membered aryl C1-C3 alkyl group, a 5- to 10-membered heteroaryl group, a 5-to 10-membered heteroaryl C1-C3 alkyl group, a C1-C3 alkoxy C1-C3 alkyl group, an amino group, a C1-C6 alkylamino group, a C1-C6 dialkylamino group, a sulfanyl group, or a C1-C6 trialkylsilyl group. Here, the 5- to 10-membered heteroaryl group represented by R² may be substituted with one or more substituents selected from a halogen atom, a hydroxy group, an amino group, a cyano group, a C1-C3 alkyl group, a C1-C3 hydroxyalkyl group, a C1-C6 alkoxy group, a C1-C3 aminoalkyl group, a C1-C3 haloalkyl group, a group represented by -N-R²R³, and a group represented by - CONH-R², and the heteroaryl group of the 5- to 10-membered heteroaryl C1-C3 alkyl group represented by R² may be further substituted with an amino group or a C1-C3 alkyl group.

Further, in the formula (I), R³ in R¹ represents a hydrogen atom or a C1-C6 alkyl group. Furthermore, in the formula (I), R⁴ and R⁵ in R¹ each represent a hydrogen atom, or independently represent a C1-C10 alkyl group which may be bonded to each other to form a ring structure.

In the present specification, the term "C1-C6 alkyl group" refers to an alkyl group having 1 to 6 carbon atoms, which may be linear or branched., Specific examples thereof include a methyl group, an ethyl group, an n-propyl group, an isopropyl group, an n-butyl group, an isobutyl group, a tert-butyl group, a sec-butyl group, an n-pentyl group, a 1-methylbutyl group, a 2-methylbutyl group, a 1,2-dimethylpropyl group, a tert-pentyl group, a 3-methylbutyl group (isopentyl group), a neopentyl group, a 1-ethylpropyl group, an n-hexyl group, an isohexyl group, a 2-methylpentyl group, a 3-methylpentyl group, a 2,3-dimethylbutyl group, a 1,1-dimethylbutyl group, a 1,2-dimethylbutyl group, a 1,3-dimethylbutyl group, a 2,2-dimethylbutyl group, a 2,3-dimethylbutyl group, a 3,3-dimethylbutyl group, and a 2-ethylbutyl group. In the present specification, the term "C1-C3 alkyl group" refers to an alkyl group having 1 to 3 carbon atoms, which may be linear or branched, and specific examples thereof include a methyl group, an ethyl group, an n-propyl group, and an isopropyl group.

In the present specification, the term "3- to 6-membered cycloalkyl group" refers to a cyclic alkyl group having 3 to 6 carbon atoms, and specific examples thereof include a cyclopropyl group, a cyclobutyl group, a cyclopentyl group, and a cyclohexyl group. In the present specification, when the range indicating the number of members of a ring structure is, for example, "3- to 6-membered," this includes any range between 3-membered or more and 6-membered or less (for example, "3- to 4-membered," "4- to 5-membered," etc.). The same applies to other ring structures.

In the present specification, "4- to 8-membered heterocycloalkyl group" means a 4- to 8-membered monocyclic or bicyclic ring structure consisting of carbon atoms and one or more heteroatoms selected from nitrogen atoms, oxygen atoms, and sulfur atoms. Specific examples include oxetanyl, azetidinyl, tetrahydrofuranyl, tetrahydropyranyl, 1,4-dioxanyl, pyrrolidinyl, piperidinyl, piperazinyl, oxadiazolidinyl (1,2,4-oxadiazolidinyl, 1,3,4-oxadiazolidinyl, etc.), morpholinyl, thiomorpholinyl, azepanyl, oxepanyl, 2,6-diazaspiro[3.3]heptyl, and octahydropyrrolo[3,4-c]pyrrolyl groups, with a bonding position at any possible carbon or nitrogen atom.

In the present specification, "6- to 10-membered aryl group" means a 6- to 10-membered monocyclic or bicyclic aromatic hydrocarbon group consisting of carbon atoms, and specific examples thereof include a phenyl group, a 1-naphthyl group, a 2-naphthyl group, and an azulenyl group.

In the present specification, "5- to 10-membered heteroaryl group" means a 5- to 10-membered monocyclic or bicyclic aromatic hydrocarbon group containing one or more heteroatoms selected from nitrogen atoms, oxygen atoms, and sulfur atoms in the ring-constituting atoms. Specific examples thereof include a pyrrolyl group, a furyl group, a thienyl group, an imidazolyl group, a pyrazolyl group, an oxazolyl group, an isoxazolyl group, a thiazolyl group, an isothiazolyl group, a triazolyl (1,2,3-triazolyl group, 1,2,4-triazolyl group, etc.), an oxadiazolyl (1,2,4-oxadiazolyl group, 1,3,4-oxadiazolyl group, etc.), a 1,3,4-thiadiazolyl group, a pyridyl group, a pyridazinyl group, a pyrimidyl group, a pyrazinyl group, a triazinyl group, a purinyl group, an indolyl group, a benzofuryl group, a benzothienyl group, a benzoimidazolyl group, an indazolyl group, a benzoxazolyl group, a benzothiazolyl group, a benzopyrazolyl group, a benzotriazolyl group, an isobenzofuryl group, an isoindolyl group, an indolizinyl group, a thienopyridinyl group, a thienopyrimidinyl group, a pyrazolopyrimidinyl group, a pyrazolopyridyl group, a pyrazolopyridazinyl group, an imidazopyridyl group, an imidazopyrimidyl group, a triazolopyridyl group, a benzofuranyl group, a benzothienyl group, a benzoisoxazolyl group, a benzoisothiazolyl group, a quinolyl group, an isoquinolyl group, a cinnolinyl group, a quinazolinyl group, a tetrazolyl group, and the like.

In the present specification, "C2-C6 alkenyl group" means an alkenyl group having 2 to 6 carbon atoms containing at least one double bond, which may be linear or branched. Specific examples include a vinyl group, an allyl group, a 2-propenyl group, an isopropenyl group, a 1-methyl-1-propenyl group, a 1-methyl-2-propenyl group, a 2-methyl-2-propenyl group, a 1-butenyl group, a 2-butenyl group, a 3-butenyl group, a 1,3-butadienyl group, a 1-pentenyl group, a 2-pentenyl group, a 3-pentenyl group, a 4-pentenyl group, a 4-methyl-3-pentenyl group, a 1-methyl-1-butenyl group, a 1-methyl-2-butenyl group, a 1-methyl-3-butenyl group, a 3-methyl-1-butenyl group, a 1,2-dimethyl-2-propenyl group, a 1,1-dimethyl-2-propenyl group, a 2-methyl-2-butenyl group, a 3-methyl-2-butenyl group, a 1,2-dimethyl-1-propenyl group, a 2-methyl-3-butenyl group, a 3-methyl-3-butenyl group, a 1,3-pentadienyl group, a 1-vinyl-2-propenyl group, a 1-hexenyl group, a 3-hexenyl group, and a 5-hexenyl group.

In the present specification, "C2-C6 alkynyl group" means an alkynyl group having 2 to 6 carbon atoms containing at least one triple bond, which may be linear or branched. Specific examples thereof include an ethynyl group, a propargyl group, a 2-propynyl group, a 1-butynyl group, a 2-butynyl group, a 3-butynyl group, a 1-pentynyl group, a 2-pentynyl group, a 3-pentynyl group, a 4-pentynyl group, a 1-hexynyl group, a 2-hexynyl group, a 3-hexynyl group, a 4-hexynyl group, a 5-hexynyl group, a 3-methyl-1-butyn-3-yl group, a prop-1-yn-1-yl group, and a 4-methyl-2-pentynyl group.

In the present specification, examples of the "halogen atom" include a fluorine atom, a chlorine atom, a bromine atom, and an iodine atom.

In the present specification, "3- to 6-membered cycloalkyl group which may be substituted with a hydroxy group, an amino group, a group represented by -CONH-R², a group represented by -NHCO-R², or a group represented by -NHSO₂-R²" refers to the 3- to 6-membered cycloalkyl group, or a group in which one or more (preferably one) hydrogen atoms of the 3- to 6-membered cycloalkyl group are substituted with a substituent selected from a hydroxy group, an amino group, a group represented by -CONH-R², a group represented by -NHCO-R², and a group represented by -NHSO₂-R². When there are multiple substituted substituents, they may be the same or different from each other. The substituent may be bonded to a carbon atom at the bonding point of the ring structure in the 3- to 6-membered cycloalkyl group substituted with the substituent.

In the present specification, "6- to 10-membered aryl group which may be substituted with a halogen atom or a C1-C3 alkyl group" refers to the 6- to 10-membered aryl group, or a group in which one or more hydrogen atoms of the 6- to 10-membered aryl group are substituted with the halogen atom and/or the C1-C3 alkyl group.

In the present specification, "C1-C3 aminoalkyl group" refers to a group in which one hydrogen atom of the C1-C3 alkyl group is substituted with an amino group. Specific examples thereof include an aminomethyl group, an aminoethyl group, and an aminopropyl group.

In the present specification, "C1-C3 haloalkyl group" refers to a group in which 1 to 6, preferably 1 to 3, of the hydrogen atoms of the C1-C3 alkyl group are substituted with the halogen atom. Specific examples thereof include a fluoromethyl group, a difluoromethyl group, a trifluoromethyl group, a chloromethyl group, a dichloromethyl group, a trichloromethyl group, a bromomethyl group, a tribromomethyl group, a chlorofluoromethyl group, a dichlorofluoromethyl group, a chlorodifluoromethyl group, a 2-fluoroethyl group, a 2,2-difluoroethyl group, a 2,2,2-trifluoroethyl group, a pentafluoroethyl group, a 2-chloroethyl group, a 2,2,2-trichloroethyl group, a 2-chloro-2-fluoroethyl group, a 2-chloro-2,2-difluoroethyl group, a 2,2-dichloro-2-fluoroethyl group, a 2-fluoropropyl group, a 3-fluoropropyl group, a 2,2-difluoropropyl group, a 3,3,3-trifluoropropyl group, and a 1,1,1,3,3,3-hexafluoro-2-propyl group.

In the present specification, "C1-C3 hydroxyalkyl group" means a group in which one hydrogen atom of the C1-C3 alkyl group is substituted with a hydroxy group. Specific examples thereof include a hydroxymethyl group, a hydroxyethyl group, a hydroxypropyl group, and a hydroxyisopropyl group.

In the present specification, "C1-C6 alkoxy group" refers to a group represented by -O-Alk, wherein Alk is the C1-C6 alkyl group. Specific examples include a methoxy group, an ethoxy group, a propoxy group, an isopropoxy group, a butoxy group, an isobutoxy group, a sec-butoxy group, a tert-butoxy group, a pentyloxy group, an isoamyloxy group, a neopentyloxy group, a 2-pentyloxy group, a 3-pentyloxy group, a 2-methylbutyloxy group, a hexyloxy group, an isohexyloxy group, a 3-methylpentyloxy group, and a 4-methylpentyloxy group. In the present specification, "C1-C3 alkoxy group" refers to a group represented by -O-Alk, wherein Alk is the C1-C3 alkyl group, and specific examples thereof include a methoxy group, an ethoxy group, a propoxy group, and an isopropoxy group.

In the present specification, "C1-C3 haloalkoxy group" refers to a group represented by -O-Alk' , wherein Alk' is the C1-C3 haloalkyl group. Specific examples thereof include a fluoromethoxy group, a difluoromethoxy group, a trifluoromethoxy group, a chloromethoxy group, a dichloromethoxy group, a trichloromethoxy group, a chlorofluoromethoxy group, a dichlorofluoromethoxy group, a chlorodifluoromethoxy group, a 2-fluoroethoxy group, a 2,2-difluoroethoxy group, a 2,2,2-trifluoroethoxy group, a pentafluoroethoxy group, a pentachloroethoxy group, a 2-chloroethoxy group, a 2-chloro-2-fluoroethoxy group, a 2-chloro-2,2-difluoroethoxy group, a 2,2,2-trichloroethoxy group, a 2,2,2-tribromoethoxy group, a 2-fluoropropoxy group, a 3-fluoropropoxy group, a 2,2-difluoropropoxy group, a 2,3-difluoropropoxy group, a 3,3,3-trifluoropropoxy group, a heptafluoropropoxy group, a 2-chloropropoxy group, a 3-chloropropoxy group, a 2,3-dichloropropoxy group, a 3,3,3-trichloropropoxy group, a heptachloropropoxy group, a 2-bromopropoxy group, a 3-bromopropoxy group, a 3,3,3-tribromopropoxy group, and a heptabromopropoxy group.

In the present specification, "3- to 6-membered cycloalkyl C1-C3 alkyl group" refers to a group in which one hydrogen atom of the C1-C3 alkyl group is substituted with the 3- to 6-membered cycloalkyl group.

In the present specification, "6- to 10-membered aryl C1-C3 alkyl group" refers to a group in which one hydrogen atom of the C1-C3 alkyl group is substituted with the 6- to 10-membered aryl group.

In the present specification, "5- to 10-membered heteroaryl C1-C3 alkyl group" refers to a group in which one hydrogen atom of the C1-C3 alkyl group is substituted with the 5- to 10-membered heteroaryl group. In the present specification, when a 6- to 10-membered aryl C1-C3 alkyl group is substituted with a substituent, it is preferable that one or two hydrogen atoms bonded to a carbon atom constituting the ring structure of the aryl group are substituted with the substituent.

In the present specification, "C1-C3 alkoxy C1-C3 alkyl group" means a group in which one hydrogen atom of the C1-C3 alkyl group is substituted with the C1-C3 alkoxy group.

In the present specification, "C1-C6 alkylamino group" refers to a group in which one hydrogen atom of an amino group is substituted with the C1-C6 alkyl group. Specific examples thereof include a methylamino group, an ethylamino group, an n-propylamino group, an isopropylamino group, an n-butylamino group, an isobutylamino group, a tert-butylamino group, a sec-butylamino group, an n-pentylamino group, a 1-methylbutylamino group, a 2-methylbutylamino group, a 1,2-dimethylpropylamino group, a tert-pentylamino group, a 3-methylbutylamino group (isopentyl group), a neopentylamino group, a 1-ethylpropylamino group, an n-hexylamino group, an isohexylamino group, a 2-methylpentylamino group, a 3-methylpentylamino group, a 2,3-dimethylbutylamino group, a 1,1-dimethylbutylamino group, a 1,2-dimethylbutylamino group, a 1,3-dimethylbutylamino group, a 2,2-dimethylbutylamino group, a 2,3-dimethylbutylamino group, a 3,3-dimethylbutylamino group, and a 2-ethylbutylamino group.

In the present specification, "C1-C6 dialkylamino group" refers to a group in which two hydrogen atoms of an amino group are substituted with the C1-C6 alkyl groups, which may be the same or different from each other. Specific examples thereof include a dimethylamino group, a diethylamino group, an N-ethyl-N-methylamino group, a di(n-propyl)amino group, an N-methyl-N-(n-propyl)amino group, an N-ethyl-N-n-propylamino group, a diisopropylamino group, an N-ethyl-N-isopropylamino group, a di(n-butyl)amino group, an N-methyl-N-sec-butylamino group, a di(n-heptyl)amino group, an N-(n-heptyl) -N-methylamino group, an N-ethyl-N-(n-heptyl)amino group, a diisoheptylamino group, an N-isoheptyl-N-methylamino group, an N-ethyl-N-isoheptylamino group, a di(n-hexyl)amino group, an N-hexyl-N-methylamino group, an N-ethyl-N-hexylamino group, a diisohexylamino group, an N-isohexyl-N-methylamino group, and an N-ethyl-N-isohexylamino group.

In the present specification, "C1-C6 trialkylsilyl group" refers to a group in which three hydrogen atoms of a silyl group are substituted with the C1-C6 alkyl groups, which may be the same or different from each other. Specific examples thereof include, for example, a trimethylsilyl group, a triethylsilyl group, a triisopropylsilyl group, a tert-butyldimethylsilyl group, and a tri(tert-butyl)silyl group.

In the present specification, the "group represented by -B(OR⁴)OR⁵" refers to a group in which - OR⁴ and -OR⁵ are each bonded to -B. The "C1-C10 alkyl group which may be bonded to each other to form a ring structure" represented by R⁴ and R⁵ means that R⁴ and R⁵ each independently represent an alkyl group having 1 to 10 carbon atoms, which may be linear or branched, and R⁴ and R⁵ may be bonded to each other to form a ring structure such as a pinacolato ester (pin) or a neopentyl glycolato ester (neo) of boronic acid.

In the present specification, the phrase "may be substituted" for each group means that the group is an unsubstituted group or a group in which one or more hydrogen atoms of the group are substituted with atoms or substituents. When there are multiple substituted atoms or substituents, they may be the same or different from each other. The number of substituted atoms or substituents is, for example, preferably 1 to 3.

When an asymmetric carbon is present in the compound represented by the formula (I), the compound may be in any form of a racemate, a diastereomeric mixture, and individual optically active forms, or a mixture thereof. When a geometric isomer is present in the compound represented by the formula (I), the compound may be in any form of (E)-form, (Z)-form, and a mixture thereof. Furthermore, in the compound represented by the formula (I), any hydrogen atom among one or more hydrogen atoms present in substituents such as an alkyl group and an alkoxy group may be replaced with a deuterium atom.

The pharmaceutically acceptable salt of the compound represented by the formula (I) is not particularly limited as long as it is pharmaceutically acceptable, and examples thereof include salts with organic acids, salts with inorganic acids, and salts with amino acids. Examples of the salts with organic acids include formate, acetate, propionate, tartrate, fumarate, maleate, succinate, lactate, citrate, malate, ascorbate, oxalate, glycolate, phenylacetate, benzoate, mandelate, butyrate, malate, methanesulfonate, and benzenesulfonate. Examples of the salts with inorganic acids include hydrochloride, hydrobromide, phosphate, sulfamate, nitrate, and sulfate. Examples of the salts with amino acids include aspartates and glutamates.

Next, preferred embodiments of the compound of the present invention will be described with examples, but the compound of the present invention is not limited to these examples.

As the compound of the present invention, n in the formula (I) is particularly preferably 2.

As the compound of the present invention, X in the formula (I) is preferably CH, CF, N, or CHCH=CH, and more preferably CH, CF, or N.

As the compound of the present invention, Y in the formula (I) is preferably O or a single bond. In the formula (I), Z is preferably either a hydroxy group or an oxo group.

As the compound of the present invention, R¹ in the formula (I) is a hydrogen atom; a 4- to 8-membered heterocycloalkyl group which may be substituted with an oxo group; a 5- to 10-membered heteroaryl group which may be substituted with one or more (preferably one) substituents selected from a halogen atom, a hydroxy group, an amino group, a cyano group, a formyl group, an oxo group, a C1-C3 alkyl group, a C1-C3 hydroxyalkyl group, a C1-C6 alkoxy group, a C1-C3 haloalkyl group, a group represented by -N-R²R³, a group represented by -COO-R², a group represented by -NHCO-R², and a group represented by -CONH-R²; a C2-C6 alkenyl group; a C2-C6 alkynyl group; a group represented by -CO-R²; a group represented by -COO-R²; or a group represented by -CONH-R²,
or a C1-C6 alkyl group (preferably a C1-C3 alkyl group) which may be substituted with one or more (preferably one) substituents selected from a halogen atom, a hydroxy group, an amino group, a nitro group, a cyano group, an oxo group, a 3- to 6-membered cycloalkyl group (which may be substituted with a hydroxy group), a 4- to 8-membered heterocycloalkyl group (which may be substituted with an oxo group), a 6- to 10-membered aryl group (which may be substituted with a halogen atom or a C1-C3 alkyl group), a 5- to 10-membered heteroaryl group, a group represented by - O-R², a group represented by -O-CONH-R², a group represented by -N-R²R³, a group represented by -CONH-R², a group represented by -NHCO-R², a group represented by -COO-R², a group represented by -NHSO₂-R², a group represented by -NHCONH-R², a group represented by -COR², and a group represented by -SO₂-R² is preferred.

In the formula (I), R¹ is more preferably a hydrogen atom; a 4- to 8-membered heterocycloalkyl group which may be substituted with an oxo group; a C2-C6 alkenyl group; a C2-C6 alkynyl group; or a group represented by -COO-R²,
or a 5- to 10-membered heteroaryl group substituted with one or more (preferably one) substituents selected from a halogen atom, a hydroxy group, an amino group, a cyano group, a formyl group, an oxo group, a C1-C3 alkyl group, a C1-C3 hydroxyalkyl group, a C1-C6 alkoxy group, a C1-C3 haloalkyl group, a group represented by -N-R²R³, a group represented by -COO-R², a group represented by -NHCO-R², and a group represented by -CONH-R²,
or a C1-C6 alkyl group (preferably a C1-C3 alkyl group) substituted with one or more (preferably one) substituents selected from a halogen atom, a hydroxy group, a nitro group, an oxo group, a 3- to 6-membered cycloalkyl group (which may be substituted with a hydroxy group), a 5- to 10-membered heteroaryl group, a 4- to 8-membered heterocycloalkyl group, a group represented by -O-R², a group represented by -CONH-R², a group represented by -NHCO-R², a group represented by -NHSO₂-R², a group represented by -NHCONH-R², a group represented by -CO-R², and a group represented by -SO₂-R².

R¹ is still more preferably a hydrogen atom, or a 5- to 10-membered heteroaryl group substituted with one or more (preferably one) substituents selected from a halogen atom, a hydroxy group, an amino group, a cyano group, an oxo group, a C1-C3 alkyl group, a C1-C3 hydroxyalkyl group, a C1-C6 alkoxy group, a group represented by -N-R²R³, and a C1-C3 haloalkyl group, or a C1-C6 alkyl group (preferably a C1-C3 alkyl group) substituted with one or more (preferably one) substituents selected from a 4- to 8-membered heterocycloalkyl group, a 5- to 10-membered heteroaryl group, a group represented by -O-R², a group represented by -CONH-R², and a group represented by -NHSO₂-R².

In the formula (I), R² is preferably a hydrogen atom, a C1-C6 alkyl group, a C2-C6 alkenyl group, a C1-C3 haloalkyl group, a C1-C3 hydroxyalkyl group, a 3- to 6-membered cycloalkyl group, a 3- to 6-membered cycloalkyl C1-C3 alkyl group, a 4- to 8-membered heterocycloalkyl group, a 6- to 10-membered aryl group, a 6- to 10-membered aryl C1-C3 alkyl group, a 5- to 10-membered heteroaryl group, a 5- to 10-membered heteroaryl C1-C3 alkyl group (which may be substituted with an amino group or a C1-C3 alkyl group), a C1-C3 alkylamino group, or a C1-C6 dialkylamino group.

R² is more preferably a hydrogen atom, a C1-C6 alkyl group, a C1-C3 haloalkyl group, a C1-C3 hydroxyalkyl group, a 3- to 6-membered cycloalkyl group, a 4- to 8-membered heterocycloalkyl group, a 3- to 6-membered cycloalkyl C1-C3 alkyl group, a 5- to 10-membered heteroaryl group, a 5- to 10-membered heteroaryl C1-C3 alkyl group (which may be substituted with an amino group or a C1-C3 alkyl group), or a C1-C6 dialkylamino group, and still more preferably a C1-C6 alkyl group, a 3- to 6-membered cycloalkyl group, a C1-C3 haloalkyl group, a 5- to 10-membered heteroaryl group, or a 5- to 10-membered heteroaryl C1-C3 alkyl group (which may be substituted with an amino group or a C1-C3 alkyl group).

Further, as the compound of the present invention, in the formula (I), the bond between positions 22 and 23 is preferably a single bond.

Preferred combinations of n, X, Y, R¹, R², and R³ in the formula (I) are not particularly limited, but include, for example, the following aspects.

### (Aspect 1)

As a preferred aspect of the compound of the present invention, in the formula (I), when n is 2 and X is N, Y is O, Aspect 1 is mentioned.

In Aspect 1, R¹ is preferably a hydrogen atom; a 4- to 8-membered heterocycloalkyl group which may be substituted with an oxo group; a C2-C6 alkenyl group; or a C2-C6 alkynyl group,
or a C1-C6 alkyl group substituted with one or more substituents selected from a halogen atom, a hydroxy group, an amino group, a 3- to 6-membered cycloalkyl group (which may be substituted with a hydroxy group), a 4- to 8-membered heterocycloalkyl group (which may be substituted with an oxo group), a 5- to 10-membered heteroaryl group, a group represented by -O-R², a group represented by -O-CONH-R², a group represented by -N-R²R³, a group represented by -CONH-R², a group represented by -NHCO-R², a group represented by -COO-R², a group represented by -NHSO₂-R², a group represented by -NHCONH-R², and a group represented by - CO-R².

R¹ is more preferably a hydrogen atom; a C2-C6 alkenyl group; or a C2-C6 alkynyl group; or a C1-C6 alkyl group which may be substituted with one or more substituents selected from a halogen atom, a hydroxy group, a 3- to 6-membered cycloalkyl group (which may be substituted with a hydroxy group), a 4- to 6-membered heterocycloalkyl group, a 5- to 10-membered heteroaryl group, a group represented by -O-R², a group represented by -CONH-R², a group represented by -NHCO-R², a group represented by -NHSO₂-R², and a group represented by - NHCONH-R².

In Aspect 1, R² is preferably a hydrogen atom, a C1-C6 alkyl group, a C1-C3 haloalkyl group, a C1-C3 hydroxyalkyl group, a 3- to 6-membered cycloalkyl group, a 3- to 6-membered cycloalkyl C1-C3 alkyl group, a 4-to 8-membered heterocycloalkyl group, a 5- to 10-membered heteroaryl group, a 5- to 10-membered heteroaryl C1-C3 alkyl group (which may be substituted with an amino group or a C1-C3 alkyl group), or a C1-C6 dialkylamino group.

R² is more preferably a hydrogen atom, a C1-C3 alkyl group, a C1-C3 haloalkyl group, a C1-C3 hydroxyalkyl group, a 4- to 6-membered cycloalkyl group, a 3- to 6-membered cycloalkyl C1-C3 alkyl group, a 4-to 6-membered heterocycloalkyl group, a 5- to 8-membered heteroaryl group, a 5- to 8-membered heteroaryl C1-C3 alkyl group (which may be substituted with an amino group or a C1-C3 alkyl group), or a C1-C6 dialkylamino group.

In Aspect 1, R³ may be either a hydrogen atom or a C1-C6 alkyl group. In Aspect 1, Z may be either a hydroxy group or an oxo group. Furthermore, in Aspect 1, the bond between positions 3 and 4 may be either a single bond or a double bond, and the bond between positions 22 and 23 is preferably a single bond.

### (Aspect 2)

As another preferred aspect of the compound of the present invention, in the formula (I), when n is 2 and X is CH, CF, CHCH=N, or CHCH=CH, Aspect 2 is mentioned where Y is a single bond; in this case, X is preferably CH, CF, or CHCH=CH, and more preferably CH or CF.

In Aspect 2, R¹ is preferably a 4- to 8-membered heterocycloalkyl group which may be substituted with an oxo group; a group represented by -COO-R²; a 5- to 10-membered heteroaryl group which may be substituted with one or more (preferably one) substituents selected from a halogen atom, a hydroxy group, an amino group, a cyano group, a formyl group, an oxo group, a C1-C3 alkyl group, a C1-C3 hydroxyalkyl group, a C1-C6 alkoxy group, a C1-C3 haloalkyl group, a group represented by -COO-R², a group represented by -N-R²R³, a group represented by - NHCO-R², and a group represented by -CONH-R²; a C2-C6 alkenyl group; a group represented by -CO-R²; or a group represented by -CONH-R²,
or a C1-C6 alkyl group which may be substituted with one or more substituents selected from a hydroxy group, an amino group, a nitro group, a cyano group, an oxo group, a 3- to 6-membered cycloalkyl group, a 6- to 10-membered aryl group (which may be substituted with a halogen atom or a C1-C3 alkyl group), a group represented by -N-R²R³, a group represented by -CONH-R², a group represented by -COO-R², a group represented by -CO-R², and a group represented by -SO₂-R².

R¹ is more preferably a 4- to 6-membered heterocycloalkyl group which may be substituted with an oxo group; a group represented by -COO-R²; a 5- to 10-membered heteroaryl group which may be substituted with one or more (preferably one) substituents selected from a halogen atom, a hydroxy group, an amino group, a cyano group, a formyl group, an oxo group, a C1-C3 alkyl group, a C1-C3 hydroxyalkyl group, a C1-C6 alkoxy group, a C1-C3 haloalkyl group, a group represented by -COO-R², a group represented by -N-R²R³, a group represented by - NHCO-R², and a group represented by -CONH-R²; or a C1-C6 alkyl group which may be substituted with one or more substituents selected from a hydroxy group, a nitro group, an oxo group, a group represented by -CONH-R², a group represented by -COO-R², a group represented by - CO-R², and a group represented by -SO₂-R².

In Aspect 2, R² is preferably a C1-C6 alkyl group, a C2-C6 alkenyl group, a C1-C3 haloalkyl group, a C1-C3 hydroxyalkyl group, a 3- to 6-membered cycloalkyl group, a 6- to 10-membered aryl group, a 6- to 10-membered aryl C1-C3 alkyl group, a 5- to 10-membered heteroaryl C1-C3 alkyl group, or a C1-C3 alkylamino group, and more preferably a C1-C6 alkyl group, a C1-C3 haloalkyl group, or a C1-C3 hydroxyalkyl group.

In Aspect 2, R³ is preferably a hydrogen atom. In Aspect 2, Z may be either a hydroxy group or an oxo group. Furthermore, in Aspect 2, the bond between positions 3 and 4 may be either a single bond or a double bond, and the bond between positions 22 and 23 is preferably a single bond.

More specific examples of preferred aspects of the compound of the present invention include the compounds represented by any one of the above formulas (1) to (94) and the compounds represented by any one of formulas (201) to (271). The compounds represented by formula (1) to formula (94) correspond to compounds I-1 to 5, 10, 11, 18, 19, 28, 49 to 55, 57, 59, 60, 62 to 64, 66, 68, 70, 72, 74, 79 to 82, 92 to 98, 101 to 106, 109 to 115, 120 to 127, 129 to 136, 139 to 148, 150 to 152, 155, 156, 159 to 162, 165, 166, 169/170, 172, 175, and 176 described in the following Examples, respectively; and the compounds represented by formula (201) to formula (271) correspond to compounds II-2 to 5, 11, 15, 22, 23, 25/26, 28, 38 to 41, 43, 47, 48, 50, 54, 55, 57, 59 to 63, 65, 68, 69, 71 to 74, 76 to 80, 84 to 92, 94 to 96, 98, 99, 102, 104 to 107, 109, 110, 112, 113, 115 to 117, and 119 to 124 described in the following Examples, respectively.

The method for producing the compound of the present invention is not particularly limited and can be carried out by combining a wide variety of synthesis methods known to those skilled in the art, and if necessary, by methods that have been modified or improved, using starting materials, precursors, reagents, solvents, etc. that are either commercially available or can be synthesized by methods recognized by those skilled in the art. For example, it can be produced by the representative methods shown in the following steps 1 to 5, but is not limited thereto.

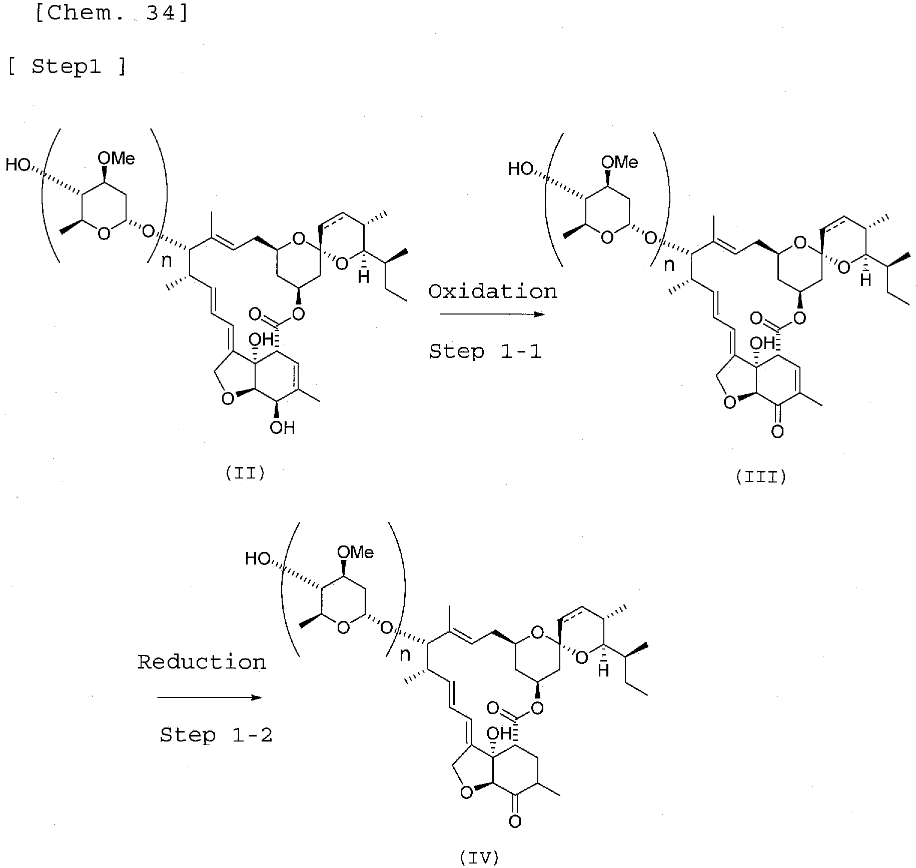

By the method shown in Step 1 above, for example, the compound represented by formula (III) and the compound represented by formula (IV) can be synthesized as intermediates for synthesizing the compound represented by the formula (I) by oxidation reaction and reduction reaction. In the formulas (III) and (IV), n has the same meaning as n in the formula (I).

### [Step 1-1]

The compound represented by the formula (III) can be synthesized by subjecting the compound represented by the formula (II) to an oxidation reaction in a solvent.

As the oxidation reaction in Step 1-1, a known method or a method equivalent thereto can be appropriately employed, and for example, it can be selected from Swern oxidation reaction; Parikh-Doering oxidation reaction; oxidation reactions using reagents such as 2-iodoxybenzoic acid, Dess-Martin Periodinane reagent, manganese dioxide, and tetrapropylammonium perruthenate. The amount of the reagent is preferably in the range of 2 to 100 equivalents based on the compound represented by the formula (II).

The solvent in Step 1-1 is not particularly limited as long as it does not significantly inhibit the reaction, and examples thereof include tetrahydrofuran (THF), 1,4-dioxane, acetonitrile, N,N-dimethylformamide (DMF), N,N-dimethylacetamide, dimethyl sulfoxide, dichloromethane, and chloroform, and one of these may be used alone or a combination of two or more may be used.

The reaction temperature in Step 1-1 is selected, for example, in the range of 0 to 100°C, and preferably in the range of 0 to 50°C. The reaction time is, for example, 1 to 72 hours, and preferably 12 to 48 hours.

### [Step 1-2]

The compound represented by the formula (IV) can be synthesized by reacting the compound represented by the formula (III) with a reducing agent in a solvent (performing a reduction reaction).

Examples of the reducing agent in Step 1-2 include sodium borohydride, lithium aluminum hydride, diisobutylaluminum hydride (DIBAL), lithium borohydride, lithium tri(sec-butyl)borohydride (L-Selectride), potassium tri(sec-butyl)borohydride (K-Selectride), and sodium triethylborohydride. The amount of the reducing agent is preferably in the range of 1 to 10 equivalents based on the compound represented by the formula (III).

The solvent in Step 1-2 is not particularly limited as long as it does not significantly inhibit the reaction, and examples thereof include tetrahydrofuran, 2-methyltetrahydrofuran, diethyl ether, 1,4-dioxane, acetonitrile, methanol, ethanol, n-hexane, heptane, and toluene, and one of these may be used alone or a combination of two or more may be used.

The reaction temperature in Step 1-2 is selected, for example, in the range of -100 to 50°C, and preferably in the range of -78 to 0°C. The reaction time is, for example, 15 minutes to 24 hours, and preferably 30 minutes to 3 hours.

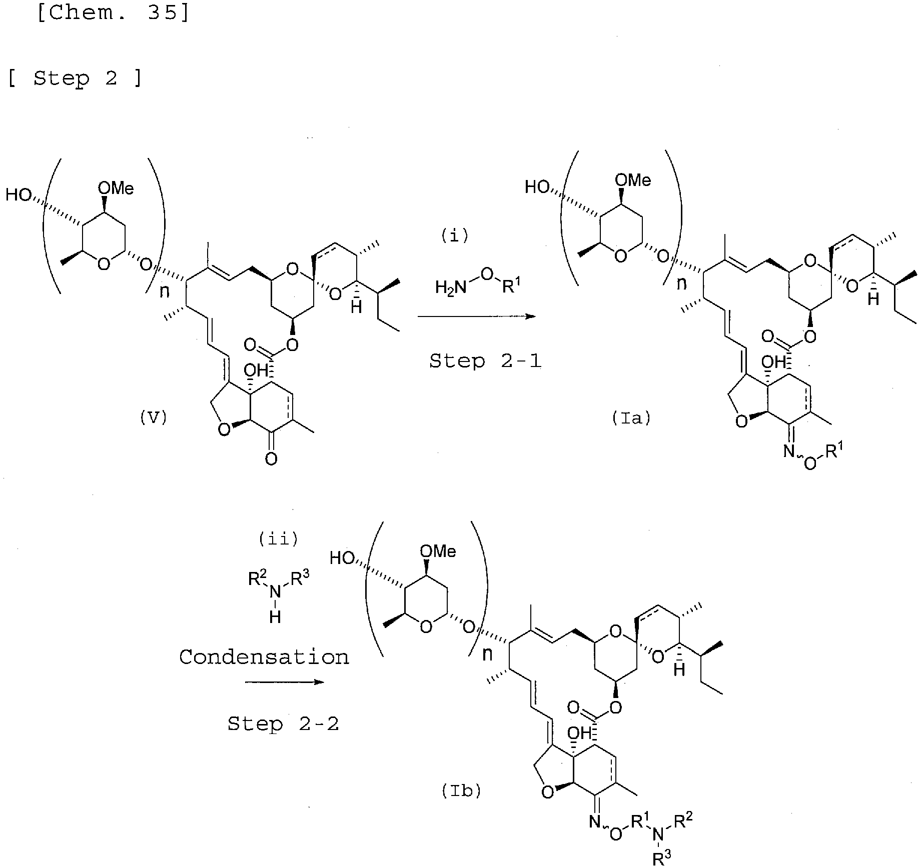

By the method shown in Step 2 above, for example, the compound represented by formula (Ia) and the compound represented by formula (Ib) among the compounds represented by the formula (I) can be synthesized. In Step 2, the compound represented by formula (V) is a compound represented by formula (III) or a compound represented by formula (IV) obtained in Step 1 above. In the formulas (V), (Ia), and (Ib), n, R¹, R², and R³ have the same meanings as n, R¹, R², and R³ in the formula (I), respectively.

### [Step 2-1]

The compound represented by the formula (Ia) can be synthesized by reacting the compound represented by the formula (V) with an appropriate hydroxylamine (compound represented by the formula (i): R¹ has the same meaning as R¹ in the formula (Ia)) in a solvent in the presence of a base.

The hydroxylamine may be a hydrochloride salt. The amount of hydroxylamine and/or its hydrochloride salt is preferably in the range of 1 to 20 equivalents (preferably 2 to 20 equivalents) based on the compound represented by the formula (V).

Examples of the base in Step 2-1 include sodium carbonate, sodium hydrogen carbonate, potassium carbonate, cesium carbonate, sodium acetate, potassium acetate, and potassium fluoride. The amount of the base is preferably in the range of 1 to 30 equivalents (preferably 2 to 30 equivalents) based on the compound represented by the formula (V).

The solvent in Step 2-1 is not particularly limited as long as it does not significantly inhibit the reaction, and examples thereof include dichloromethane, chloroform, methanol, ethanol, isopropanol, ethyl acetate, tetrahydrofuran, diethyl ether, N,N-dimethylformamide, N,N-dimethylacetamide, acetone, dimethyl sulfoxide, and toluene, and one of these may be used alone or a combination of two or more may be used.

In the reaction of Step 2-1, if necessary, a reaction reagent such as anhydrous cerium chloride, cerium chloride heptahydrate, anhydrous lanthanum chloride, or lanthanum chloride heptahydrate may be further added, and in that case, the amount of the reaction reagent is preferably in the range of 1 to 5 equivalents based on the compound represented by the formula (V).

The reaction temperature in Step 2-1 is not particularly limited, and is, for example, -20 to 80°C, and preferably 0 to 50°C. The reaction time is also not particularly limited, and is, for example, 10 minutes to 24 hours, and preferably 30 minutes to 5 hours.

### [Step 2-2]

The compound represented by the formula (Ib) can be synthesized by subjecting the compound represented by the formula (Ia) and an appropriate amine (compound represented by the formula (ii): R² and R³ have the same meanings as R² and R³ in the formula (Ib), respectively) to a condensation reaction using a condensing agent in a solvent in the presence of a base.

The amine may be a hydrochloride salt. The amount of the amine and/or its hydrochloride salt is preferably in the range of 1 to 10 equivalents (preferably 2 to 10 equivalents) based on the compound represented by the formula (Ia).

Examples of the condensing agent include N,N'-dicyclohexylcarbodiimide (DCC), 1-ethyl-3-(3-dimethylaminopropyl) carbodiimide hydrochloride (EDC), 1,1-carbonyldiimidazole (CDI), 2-chloro-1-methylpyridinium iodide, 1-propylphosphonic acid cyclic anhydride (PPA), N,N'-dicyclohexylcarbodiimide, and O-(7-azabenzotriazol-1-yl)-N,N,N',N'-tetramethyluronium hexafluorophosphate (HATU). The amount of the condensing agent is preferably in the range of 1 to 8 equivalents based on the compound represented by the formula (Ia).

Examples of the base in Step 2-2 include organic amines such as trimethylamine, triethylamine, diisopropylethylamine, tripropylamine, triisopropylamine, tributylamine, N-methylmorpholine, pyridine, N,N-dimethylpyridin-4-amine, N,N-dimethylaniline, N,N-diethylaniline, 1,5-diazabicyclo[4.3.0]non-5-ene, 1,4-diazabicyclo[2.2.2]octane (DABCO), and 1,8-diazabicyclo[5.4.0]-7-undecene (DBU). The amount of the base is preferably in the range of 1 to 30 equivalents (preferably 5 to 30 equivalents) based on the compound represented by the formula (Ia).

The solvent in Step 2-2 is not particularly limited as long as it does not significantly inhibit the reaction, and examples thereof include dichloromethane, chloroform, ethyl acetate, tetrahydrofuran, diethyl ether, N,N-dimethylformamide, N,N-dimethylacetamide, acetone, dimethyl sulfoxide, and toluene, and one of these may be used alone or a combination of two or more may be used.

In the reaction of Step 2-2, if necessary, a reaction reagent such as 1-hydroxybenzotriazole (HOBt) or N,N-dimethylpyridin-4-amine (DMAP) may be further added.

The reaction temperature in Step 2-2 is not particularly limited, and is, for example, -20 to 80°C, and preferably 0 to 50°C. The reaction time is also not particularly limited, and is, for example, 10 minutes to 48 hours, and preferably 30 minutes to 24 hours.

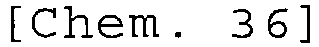

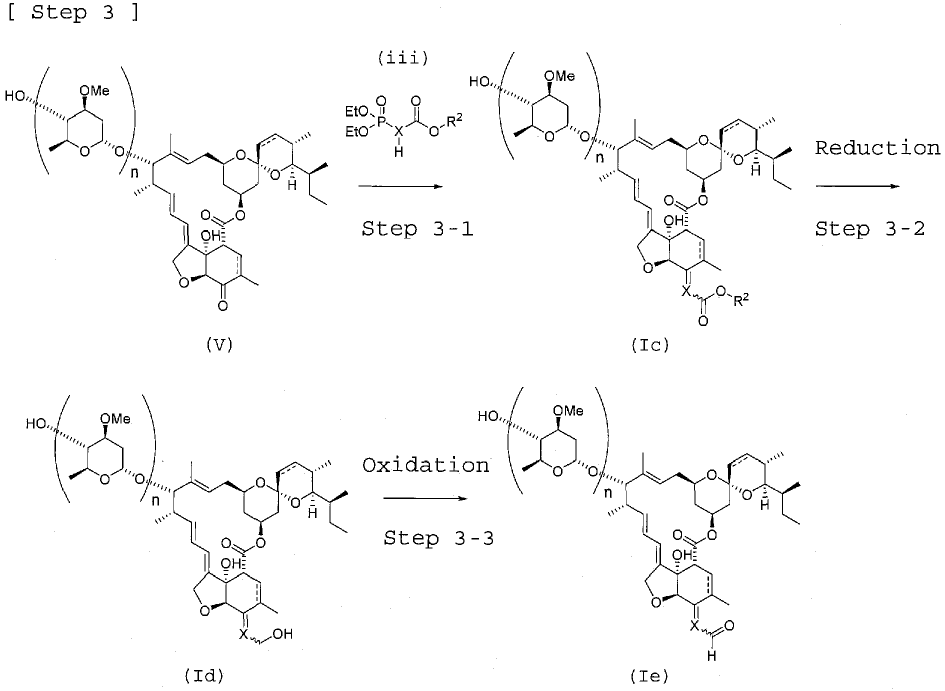

By the method shown in Step 3 above, for example, the compound represented by formula (Ic), the compound represented by formula (Id), and the compound represented by formula (Ie) among the compounds represented by the formula (I) can be synthesized. In Step 3, the compound represented by formula (V) is a compound represented by formula (III) or a compound represented by formula (IV) obtained in Step 1 above. In the formulas (V), (Ic), (Id), and (Ie), X is CH or CF, and n and R² have the same meanings as n and R² in the formula (I), respectively.

### [Step 3-1]

The compound represented by the formula (Ic) can be synthesized by reacting the compound represented by the formula (V) with the compound represented by the formula (iii) (X and R² have the same meanings as X and R² in the formula (Ic), respectively, and Et represents an ethyl group) in a solvent in the presence of a base.

As the compound represented by the formula (iii), various Horner-Wadsworth-Emmons reagents widely known to those skilled in the art, such as ethyl 2-(diethoxyphosphoryl)acetate, ethyl 2-(diphenoxyphosphoryl)acetate, and methyl 2-[bis(2,2,2-trifluoroethoxy)phosphoryl]acetate, can be used. The amount of the compound represented by the formula (iii) is preferably in the range of 1 to 10 equivalents based on the compound represented by the formula (V).

Examples of the base in Step 3-1 include sodium hydride, sodium tert-butoxide, potassium tert-butoxide, sodium hydroxide, potassium hydroxide, lithium hexamethyldisilazane, potassium hexamethyldisilazane, lithium diisopropylamide, and n-butyllithium. The amount of the base is preferably in the range of 1 to 10 equivalents based on the compound represented by the formula (V).

The solvent in Step 3-1 is not particularly limited as long as it does not significantly inhibit the reaction, and examples thereof include tetrahydrofuran, diethyl ether, tetrahydrofuran, 2-methyltetrahydrofuran, diethyl ether, 1,4-dioxane, acetonitrile, n-hexane, heptane, and toluene, and one of these may be used alone or a combination of two or more may be used.

The reaction temperature in Step 3-1 is not particularly limited, and is, for example, -100 to 60°C, and preferably -78 to 20°C. The reaction time is also not particularly limited, and is, for example, 10 minutes to 10 hours, and preferably 30 minutes to 3 hours.

### [Step 3-2]

The compound represented by the formula (Id) can be synthesized by reacting the compound represented by the formula (Ic) with a reducing agent in a solvent.

Examples of the reducing agent in Step 3-2 include sodium borohydride, lithium aluminum hydride, diisobutylaluminum hydride (DIBAL), lithium borohydride, lithium tri(sec-butyl)borohydride (L-Selectride), potassium tri(sec-butyl)borohydride (K-Selectride), and sodium triethylborohydride. The amount of the reducing agent is preferably in the range of 1 to 10 equivalents based on the compound represented by the formula (Ic).

The solvent in Step 3-2 is not particularly limited as long as it does not significantly inhibit the reaction, and examples thereof include tetrahydrofuran, 2-methyltetrahydrofuran, diethyl ether, 1,4-dioxane, acetonitrile, methanol, ethanol, n-hexane, heptane, dichloromethane, 1,2-dichloroethane, and toluene, and one of these may be used alone or a combination of two or more may be used.

The reaction temperature in Step 3-2 is selected, for example, in the range of -100 to 50°C, and preferably in the range of -78 to 0°C. The reaction time is, for example, 15 minutes to 24 hours, and preferably 30 minutes to 3 hours.

### [Step 3-3]

The compound represented by the formula (Ie) can be synthesized by subjecting the compound represented by the formula (Id) to an oxidation reaction in a solvent.

As the oxidation reaction in Step 3-3, a known method or a method equivalent thereto can be appropriately employed, and for example, it can be selected from Swern oxidation reaction; Parikh-Doering oxidation reaction; oxidation reactions using 2-iodoxybenzoic acid, Dess-Martin Periodinane reagent, manganese dioxide, tetrapropylammonium perruthenate (TPAP), and TEMPO. The amount of the reagent is preferably in the range of 2 to 100 equivalents based on the compound represented by the formula (Id).

The solvent in Step 3-3 is not particularly limited as long as it does not significantly inhibit the reaction, and examples thereof include tetrahydrofuran, 1,4-dioxane, acetonitrile, N,N-dimethylformamide, N,N-dimethylacetamide, dimethyl sulfoxide, dichloromethane, and chloroform, and one of these may be used alone or a combination of two or more may be used.

The reaction temperature in Step 3-3 is selected, for example, in the range of 0 to 100°C, and preferably in the range of 0 to 50°C. The reaction time is, for example, 1 to 24 hours, and preferably 1 to 6 hours.

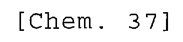

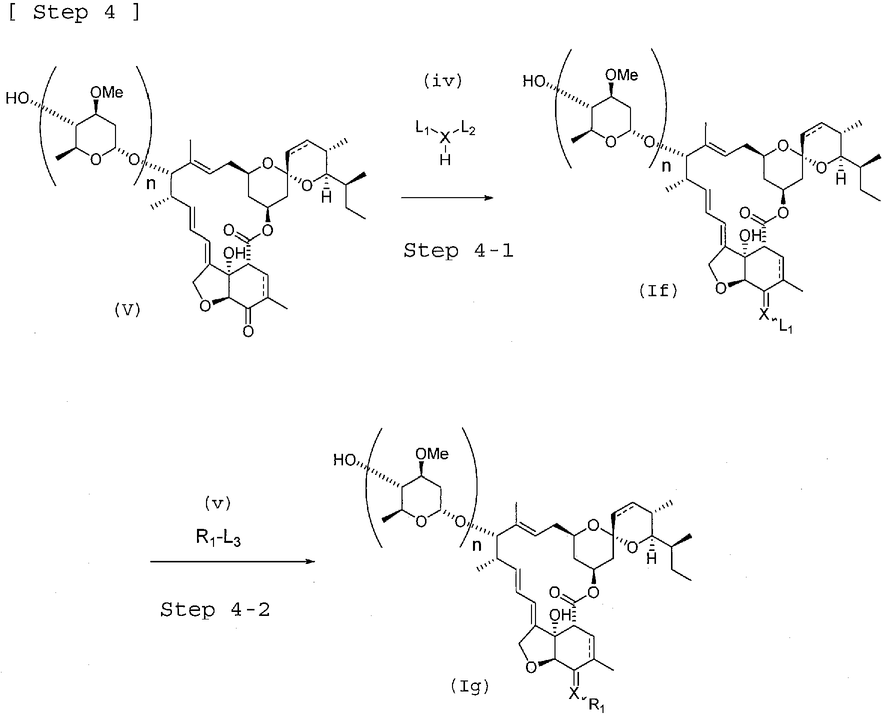

By the method shown in Step 4 above, for example, the compound represented by formula (If) and the compound represented by formula (Ig) among the compounds represented by the formula (I) can be synthesized. In Step 4, the compound represented by formula (V) is a compound represented by formula (III) or a compound represented by formula (IV) obtained in Step 1 above. In the formulas (V), (If), and (Ig), X is CH, and n and R¹ have the same meanings as n and R¹ in the formula (I), respectively. Further, L₁, L₂, and L₃ in the formulas (If), (iv), and (v) each independently represent a functional group such as Cl, Br, I, an alkylstannyl group, a dihydroxyboryl group, a 4,4,5,5-tetramethyl-1,3,2-dioxaborolan-2-yl group, a trifluoromethylsulfoxy group, a triarylphosphinyl group, or a trialkylphosphinyl group.

### [Step 4-1]

The compound represented by the formula (If) can be synthesized by reacting the compound represented by the formula (V) with the compound represented by the formula (iv) (X and L₁ have the same meanings as X and L₁ in the formula (If), respectively) in a solvent in the presence of a base.

As the compound represented by the formula (iv), various Wittig reagents widely known to those skilled in the art, such as (methoxymethyl)triphenylphosphonium chloride, (bromomethyl)triphenylphosphonium bromide, and tributyl(cyanomethyl)phosphonium chloride, and bis(4,4,5,5-tetramethyl-1,3,2-dioxaborolan-2-yl)methane can be used. The amount of the compound represented by the formula (iv) is preferably in the range of 1 to 10 equivalents based on the compound represented by the formula (V).

Examples of the base in Step 4-1 include sodium hydride, sodium tert-butoxide, potassium tert-butoxide, sodium hydroxide, potassium hydroxide, lithium hexamethyldisilazane, sodium hexamethyldisilazane, potassium hexamethyldisilazane, lithium diisopropylamide, n-butyllithium, sec-butyllithium, and lithium 2,2,6,6-tetramethylpiperidinide. The amount of the base is preferably in the range of 1 to 10 equivalents based on the compound represented by the formula (V).

The solvent in Step 4-1 is not particularly limited as long as it does not significantly inhibit the reaction, and examples thereof include tetrahydrofuran, diethyl ether, tetrahydrofuran, 2-methyltetrahydrofuran, diethyl ether, 1,4-dioxane, n-hexane, heptane, and toluene, and one of these may be used alone or a combination of two or more may be used.

The reaction temperature in Step 4-1 is not particularly limited, and is, for example, -100 to 80°C, and preferably -78 to 40°C. The reaction time is also not particularly limited, and is, for example, 10 minutes to 24 hours, and preferably 30 minutes to 6 hours.

### [Step 4-2]

The compound represented by the formula (Ig) can be synthesized by reacting the compound represented by the formula (If), a metal catalyst, and the compound represented by the formula (v) (R¹ has the same meaning as R¹ in the formula (Ig)) in a solvent in the presence of a base.

The amount of the compound represented by the formula (v) is preferably in the range of 1 to 20 equivalents based on the compound represented by the formula (If).

Examples of the metal catalyst in Step 4-2 include palladium compounds such as tetrakis(triphenylphosphine)palladium(0), palladium(II) acetate, dichlorobis(triphenylphosphine)palladium(II), dichlorobis(triethylphosphine)palladium(II), tris(dibenzylideneacetone)dipalladium(O), 1,1'-bis(diphenylphosphino)ferrocenepalladium(II) chloride, and palladium(II) acetate; nickel compounds such as tetrakis(triphenylphosphine)nickel(0); rhodium compounds such as tris(triphenylphosphine)rhodium(III) chloride; cobalt compounds; copper compounds such as copper oxide and copper(I) iodide; and platinum compounds. The amount of the metal catalyst is preferably in the range of 0.01 to 2 equivalents based on the compound represented by the formula (If).

Examples of the base in Step 4-2 include sodium carbonate, sodium hydrogen carbonate, potassium carbonate, cesium carbonate, sodium acetate, potassium acetate, sodium tert-butoxide, and potassium tert-butoxide. The amount of the base is preferably in the range of 1 to 30 equivalents based on the compound represented by the formula (If).

The solvent in Step 4-2 is not particularly limited as long as it does not significantly inhibit the reaction, and examples thereof include tetrahydrofuran, 2-methyltetrahydrofuran, diethyl ether, 1,2-dimethoxyethane, 1,4-dioxane, acetonitrile, ethanol, isopropanol, ethyl acetate, N,N-dimethylformamide, N,N-dimethylacetamide, n-hexane, heptane, and toluene, and one of these may be used alone or a combination of two or more may be used.

In the reaction of Step 4-2, if necessary, a reaction reagent such as 4,5-bis(diphenylphosphino)-9,9-dimethylxanthene, 2-dicyclohexylphosphino-2',6'-dimethoxybiphenyl, 1,2-bis(diphenylphosphino)ethane, dicyclohexyl(2',4',6'-triisopropyl-[1,1'-biphenyl]-2-yl)phosphine, or dicyclohexyl(2',6'-diisopropyl-[1,1'-biphenyl]-2-yl)phosphine may be further added, and in that case, the amount of the reaction reagent is preferably in the range of 0.01 to 4 equivalents based on the compound represented by the formula (If).

The reaction temperature in Step 4-2 is selected, for example, in the range of -78 to 200°C, and is preferably 25 to 150°C. The reaction time is, for example, 15 minutes to 24 hours, and is preferably 30 minutes to 3 hours.

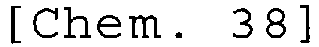

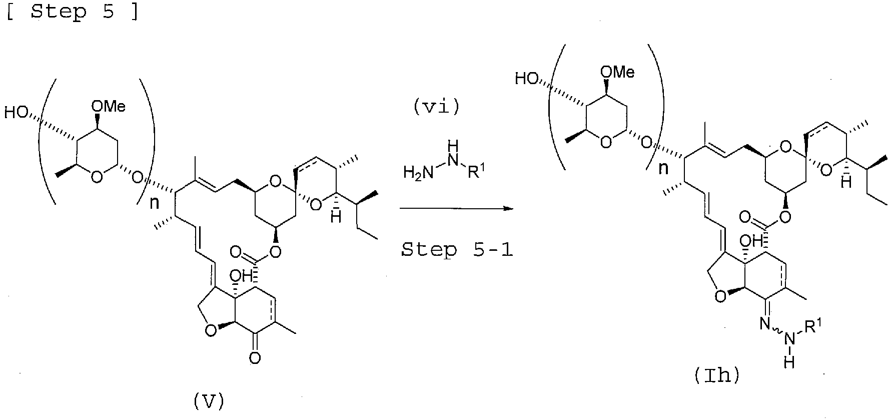

By the method shown in Step 5 above, for example, the compound represented by the formula (Ih) among the compounds represented by the formula (I) can be synthesized. In Step 5, the compound represented by formula (V) is a compound represented by formula (III) or a compound represented by formula (IV) obtained in Step 1 above. In the formulas (V) and (Ih), n and R¹ have the same meanings as n and R¹ in the formula (I), respectively.

### [Step 5-1]

The compound represented by the formula (Ih) can be synthesized by reacting the compound represented by the formula (V) with an appropriate hydrazine (compound represented by the formula (vi) : R¹ has the same meaning as R¹ in the formula (Ih)) in a solvent in the presence of a base.

The hydrazine may be a hydrochloride salt. The amount of hydrazine and/or its hydrochloride salt is preferably in the range of 2 to 20 equivalents based on the compound represented by the formula (V).

Examples of the base in Step 5-1 include sodium carbonate, sodium hydrogen carbonate, potassium carbonate, cesium carbonate, sodium acetate, potassium acetate, and potassium fluoride. The amount of the base is preferably in the range of 5 to 30 equivalents (preferably 2 to 30 equivalents) based on the compound represented by the formula (V).

The solvent in Step 5-1 is not particularly limited as long as it does not significantly inhibit the reaction, and examples thereof include dichloromethane, chloroform, methanol, ethanol, isopropanol, ethyl acetate, tetrahydrofuran, diethyl ether, N,N-dimethylformamide, N,N-dimethylacetamide, acetone, dimethyl sulfoxide, and toluene, and one of these may be used alone or a combination of two or more may be used.

The reaction temperature in Step 5-1 is not particularly limited, and is, for example, -20 to 80°C, and preferably 0 to 50°C. The reaction time is also not particularly limited, and is, for example, 10 minutes to 24 hours, and preferably 30 minutes to 5 hours.

Those skilled in the art will recognize that, at any stage in the synthesis of the compound represented by the formula (I), it is necessary or preferable to protect one or more labile groups in the molecule with a protecting group to prevent undesirable side reactions. In particular, it is necessary or preferable to protect the hydroxy group. Examples of the protecting group used in synthesizing the compound represented by the formula (I) include groups described in Greene and Wuts, PROTECTIVE GROUPS in ORGANIC SYNTHESIS THIRD EDITION, John Wiley & Sons, Inc. (the methods for removing such groups are also described).

The compound of the present invention has antiviral activity against coronaviruses. The fact that the compound has the antiviral activity can be confirmed, for example, by infecting cultured cells (e.g., VeroE6 cells) with a coronavirus (e.g., SARS-CoV-2), then adding a compound appropriately diluted stepwise to the culture medium, culturing the cells, and measuring the amount of virus in the culture supernatant, and more specifically, it can be confirmed by the method described in Test Example 1 below. Here, for example, the concentration of the compound that reduces the replication of the coronavirus to 50% is defined as the IC₅₀ value, and the value is less than 20 µM (for example, 19.9 µM or less), preferably less than 5 µM (for example, 4.9 µM or less), more preferably less than 1 µM (for example, 0.9 µM or less), and it can be judged to have excellent antiviral activity against the coronavirus.

### <Composition, Treatment Method>

Since the compound of the present invention has antiviral activity against coronaviruses as described above, a composition comprising the compound as an active ingredient, preferably a pharmaceutical composition, can be used as an antiviral agent or a therapeutic agent for the treatment or prevention of diseases or conditions involving coronaviruses. Therefore, the present invention also provides a composition comprising the compound of the present invention as an active ingredient, and the use of the compound of the present invention for the manufacture of a composition for the treatment or prevention of a disease or condition involving a coronavirus.

The present invention also provides a method for treating or preventing a disease or condition involving a coronavirus, comprising the step of administering the compound of the present invention as it is or the composition comprising the compound as an active ingredient to a target (hereinafter, sometimes simply referred to as "the method of the present invention"). The target includes humans or animals other than humans (preferably mammals).

The coronavirus is preferably, for example, one classified in the subfamily *Orthocoronavirinae* of the family Coronaviridae of the order Nidovirales, and more preferably one classified in the genus *Beta* (*β*) coronavirus. Among these, the coronavirus is preferably at least one selected from the group consisting of human coronavirus (HCoV), severe acute respiratory syndrome coronavirus (SARS-CoV), novel coronavirus (SARS-CoV-2), and Middle East respiratory syndrome coronavirus (MERS-CoV), and more preferably the novel coronavirus.

Examples of diseases and conditions involving coronaviruses include, but are not limited to, novel coronavirus infection (also called coronavirus disease 2019 or COVID-19), severe acute respiratory syndrome, and Middle East respiratory syndrome.

The compound of the present invention and the composition of the present invention may be administered to the target by either an oral or parenteral administration route, and therefore, the composition of the present invention may be a preparation in an appropriate dosage form depending on the administration route.

Specific examples of the preparation include oral preparations such as tablets, pills, capsules, granules, powders, elixirs, suspensions, emulsions, and syrups; and parenteral preparations such as injections, inhalants, rectal preparations, suppositories, lotions, sprays, ointments, creams, patches, and sustained-release preparations.

These various preparations may be produced by a conventional method using pharmaceutically acceptable additives and carriers such as excipients, disintegrants, binders, lubricants, and coloring agents, which are commonly used in the field of pharmacy, depending on the type of preparation. Therefore, the composition of the present invention may further comprise these pharmaceutically acceptable additives and/or carriers.

In the composition of the present invention, the content of the compound of the present invention (the content of the compound represented by the formula (I) or a pharmaceutically acceptable salt thereof, or the total content thereof when they are mixtures, in terms of free form) is adjusted appropriately according to the purpose of administration, the dosage form of the preparation, etc., so it cannot be generally stated, but it is usually 0.01 to 70% by mass, preferably 0.05 to 50% by mass, based on the total mass of the composition, in terms of the free form of the compound represented by the formula (I).

In the method of the present invention, the dose of the compound of the present invention (the dose of the compound represented by the formula (I) or a pharmaceutically acceptable salt thereof, or the total dose thereof when they are mixtures) is appropriately determined in each case in consideration of the species, age, weight, sex, difference in disease, degree of symptoms, etc. of the target, so it cannot be generally stated, but for example, it is 0.01 to 3000 mg, preferably 0.1 to 800 mg per day for an adult human in terms of the free form of the compound represented by the formula (I), and this can be administered once a day or in multiple divided doses.

### [Examples]

Hereinafter, the present invention will be described more specifically based on Examples, but the present invention is not limited to the following Examples.

### <Production of Compounds>

As Examples, the respective production methods of the compounds of the present invention (Compounds I-1 to III-5) and their ¹H-NMR spectra are shown below. In addition, as Reference Examples, the production methods of the respective starting compounds used in the Examples and their ¹H-NMR spectra are also shown below. However, the compounds of the present invention are not limited to these Examples, and each Reference Example is also an exemplification for specifically explaining the implementation of the present invention, and these examples do not limit the scope of the present invention, and it is self-evident that various applications, modifications, and alterations are possible without departing from the scope of the present invention.

¹H-NMR spectra were measured using deuterated chloroform (CDCl₃), deuterated dimethyl sulfoxide (DMSO-d₆), deuterated water (D₂O), or deuterated methanol (CD₃OD) as a solvent, and tetramethylsilane (TMS) as an internal standard. The measurement results of chemical shifts are shown in δ values in ppm, and the coupling constants J values are shown in Hz. The abbreviation s means singlet, d means doublet, t means triplet, q means quartet, m means multiplet, and br means broad. Mass spectra (ESI-MS) were measured by electrospray ionization.

Hereinafter, the abbreviations in the Examples and Reference Examples have the following meanings.
M: mol/L
L-Selectride: lithium tri(sec-butyl)borohydride
THF: tetrahydrofuran
DMF: N,N-dimethylformamide
DIPEA: N,N-diisopropylethylamine
DMAP: N,N-dimethylpyridin-4-amine
HATU: O-(7-azabenzotriazol-1-yl)-N,N,N',N'-tetramethyluronium hexafluorophosphate
tert: tertiary
n: normal.

### Production of 5-oxo-ivermectin B1a

Ivermectin B1a (5.00 g, 5.71 mmol) was dissolved in dichloromethane (114 mL), manganese dioxide (24.9 g, 0.286 mol) was added, and the reaction solution was stirred at room temperature overnight. The reaction solution was filtered through Celite, the solvent was distilled off from the resulting filtrate under reduced pressure, and the residue was purified by silica gel column chromatography (n-hexane/ethyl acetate system) to obtain 5-oxo-ivermectin B1a (isolated yield 3.87 g, percent yield 78%).
¹H-NMR (500 MHz, CDCl₃, δ): 6.58-6.55 (m, 1H), 5.92 (td, J = 10.9, 2.3 Hz, 1H), 5.82-5.68 (m, 2H), 5.39 (d, J = 3.4 Hz, 1H), 5.44-5.36 (m, 2H), 5.01-4.96 (m, 1H), 4.79-4.76 (m, 1H), 4.75-4.69 (m, 2H), 3.94 (br s, 1H), 3.85 (s, 1H), 3.84-3.79 (m, 1H), 3.78-3.72 (m, 1H), 3.71-3.65 (m, 1H), 3.64-3.59 (m, 1H), 3.58-3.55 (m, 1H), 3.50-3.43 (m, 2H), 3.42 (s, 3H), 3.41 (s, 3H), 3.26-3.20 (m, 2H), 3.16 (t, J = 9.2 Hz, 1H), 2.56-2.48 (m, 1H), 2.37-2.24 (m, 4H), 2.23-2.18 (m, 1H), 1.88 (d, J = 1.7 Hz, 3H), 1.90-1.85 (m, 1H), 1.79-1.75 (m, 1H), 1.67-1.64 (m, 1H), 1.60-1.34 (m, 10H), 1.29-1.23 (m, 8H), 1.16 (d, J = 6.9 Hz, 3H), 0.93 (t, J = 7.4 Hz, 3H), 0.86 (d, J = 6.3 Hz, 3H), 0.81-0.74 (m, 4H).

### Production of 3,4α-dihydro-5-oxo-ivermectin B1a and 3,4β-dihydro-5-oxo-ivermectin B1a

5-Oxo-ivermectin B1a (4.00 g, 4.58 mmol) was dissolved in THF (46 mL). Under an argon atmosphere, a 1M L-Selectride THF solution (5.95 mL, 5.95 mmol) was added at -78°C, and the mixture was stirred at the same temperature for 1 hour. An aqueous solution of ammonium chloride was added to the reaction solution to stop the reaction, and then the temperature was raised to room temperature. After extraction with ethyl acetate, the organic layer was washed with saturated brine and dried over anhydrous magnesium sulfate. After the solvent was distilled off under reduced pressure, the residue was purified by silica gel column chromatography (n-hexane/ethyl acetate system) to obtain 3,4β-dihydro-5-oxo-ivermectin B1a (isolated yield 1.33 g, percent yield 33%) and 3,4α-dihydro-5-oxo-ivermectin B1a (isolated yield 1.81 g, percent yield 45%).
(3,4β-dihydro-5-oxo-ivermectin B1a)
¹H-NMR (500 MHz, CDCl₃, δ): 5.95-5.88 (m, 1H), 5.81-5.71 (m, 1H), 5.71-5.62 (m, 1H), 5.43-5.35 (m, 2H), 4.97-4.90 (m, 1H), 4.76 (d, J = 2.9 Hz, 1H), 4.67-4.56 (m, 2H), 4.56-4.51 (m, 1H), 4.01 (s, 1H), 3.93 (br s, 1H), 3.85-3.80 (m, 1H), 3.79-3.74 (m, 1H), 3.70-3.59 (m, 2H), 3.51-3.46 (m, 1H), 3.44 (s, 3H), 3.43-3.41 (s, 3H), 3.26-3.14 (m, 3H), 3.11-3.02 (m, 1H), 2.66-2.46 (m, 3H), 2.36-2.19 (m, 5H), 1.86-1.81 (m, 1H), 1.76-1.72 (m, 1H), 1.70-1.62 (m, 3H), 1.59-1.36 (m, 10H), 1.29-1.22 (m, 8H), 1.15 (d, J = 6.9 Hz, 3H), 1.11 (d, J = 6.9 Hz, 3H), 0.92 (t, J = 7.4 Hz, 3H), 0.83 (d, J = 6.9 Hz, 3H), 0.80-0.75 (m, 4H); MSm/z 897.4962 [M+Na]⁺.
(3,4α-dihydro-5-oxo-ivermectin B1a)
¹H-NMR (500 MHz, CDCl₃, δ): 5.85-5.81 (m, 1H), 5.79-5.72 (m, 1H), 5.70-5.63 (m, 1H), 5.45-5.35 (m, 2H), 4.98-4.91 (m, 1H), 4.78-4.71 (m, 2H), 4.68-4.59 (m, 2H), 4.11 (d, J = 7.4 Hz, 1H), 3.93 (br s, 1H), 3.86-3.79 (m, 1H), 3.76 (s, 1H), 3.79-3.72 (m, 1H), 3.71-3.65 (m, 1H), 3.64-3.59 (m, 1H), 3.51-3.45 (m, 1H), 3.43 (s, 3H), 3.42 (s, 3H), 3.27-3.20 (m, 2H), 3.16 (t, J = 9.2 Hz, 1H), 3.05-3.00 (m, 1H), 2.84-2.75 (m, 1H), 2.53-2.46 (m, 1H), 2.38-2.18 (m, 5H), 2.07-2.01 (m, 2H), 1.94-1.88 (m, 1H), 1.77-1.72 (m, 1H), 1.69-1.64 (m, 1H), 1.60-1.36 (m, 10H), 1.28-1.23 (m, 8H), 1.15 (d, J = 6.9 Hz, 3H), 1.10 (d, J = 6.9 Hz, 3H), 0.92 (t, J = 7.4 Hz, 3H), 0.85-0.82 (m, 3H), 0.80-0.75 (m, 4H); MSm/z 897.4957 [M+Na]⁺.

### Production of Compound I-1

3,4β-Dihydro-5-oxo-ivermectin B1a (200 mg, 0.229 mmol) was dissolved in methanol (4.6 mL), and sodium acetate (112 mg, 1.37 mmol) and hydroxylamine hydrochloride (95 mg, 1.37 mmol) were sequentially added, followed by stirring at room temperature for 1.5 hours. A saturated aqueous sodium hydrogen carbonate solution was added to the reaction solution, and the mixture was extracted with ethyl acetate. The organic layer was washed with saturated brine and dried over anhydrous magnesium sulfate. After the solvent was distilled off under reduced pressure, the residue was purified by silica gel column chromatography (n-hexane/ethyl acetate system) to obtain Compound I-1 (isolated yield 177 mg, percent yield 87%) as a mixture of isomers that were difficult to separate. The ¹H-NMR spectrum below is the analytical data of the main isomer. ¹H-NMR (500 MHz, CDCl₃, δ): 5.88-5.83 (m, 1H), 5.76-5.65 (m, 2H), 5.45-5.38 (m, 2H), 4.95 (d, J = 10.9 Hz, 1H), 4.91-4.86 (m, 1H), 4.77 (d, J = 4.0 Hz, 1H), 4.68-4.57 (m, 3H), 3.98 (s, 1H), 3.93 (br s, 1H), 3.86-3.74 (m, 2H), 3.71-3.57 (m, 3H), 3.52-3.45 (m, 1H), 3.44 (s, 3H), 3.42 (s, 3H), 3.26-3.20 (m, 2H), 3.17 (t, J = 9.2 Hz, 1H), 2.95-2.81 (m, 1H), 2.52-2.45 (m, 1H), 2.36-2.20 (m, 5H), 1.99-1.93 (m, 1H), 1.90 (dd, J = 12.0, 5.2 Hz, 1H), 1.79-1.73 (m, 1H), 1.67 (d, J = 13.2 Hz, 1H), 1.59-1.36 (m, 13H), 1.30-1.21 (m, 10H), 1.15 (d, J = 6.9 Hz, 3H), 0.92 (t, J = 7.5 Hz, 3H), 0.86-0.80 (m, 4H), 0.78 (d, J = 6.3 Hz, 3H); MSm/z 912.5092 [M+Na]⁺.

### Production of Compound I-2

3,4α-Dihydro-5-oxo-ivermectin B1a (30.0 mg, 0.034 mmol) was dissolved in methanol (0.69 mL), and sodium acetate (16.9 mg, 0.206 mmol) and hydroxylamine hydrochloride (14.9 mg, 0.206 mmol) were sequentially added, followed by stirring at room temperature for 1 hour. A saturated aqueous sodium hydrogen carbonate solution was added to the reaction solution, and the mixture was extracted with ethyl acetate. The organic layer was washed with saturated brine and dried over anhydrous magnesium sulfate. After the solvent was distilled off under reduced pressure, the residue was purified by silica gel column chromatography (n-hexane/ethyl acetate system) to obtain Compound I-2 (isolated yield 22.5 mg, percent yield 73%).
¹H-NMR (500 MHz, CDCl₃, δ): 8.47 (br s, 1H), 5.84 (dt, J = 10.6, 2.4 Hz, 1H), 5.77-5.65 (m, 2H), 5.43-5.36 (m, 2H), 5.02 (s, 1H), 4.95 (d, J = 10.9 Hz, 1H), 4.77 (d, J = 3.4 Hz, 1H), 4.72-4.63 (m, 2H), 4.61 (s, 1H), 3.93 (br s, 1H), 3.86-3.74 (m, 2H), 3.70-3.60 (m, 2H), 3.53-3.46 (m, 1H), 3.44 (s, 3H), 3.43 (s, 4H), 3.26-3.19 (m, 2H), 3.17 (t, J = 9.2 Hz, 1H), 2.80 (dd, J = 12.9, 3.2 Hz, 1H), 2.75 (br s, 1H), 2.68-2.58 (m, 1H), 2.54-2.45 (m, 1H), 2.36-2.30 (m, 2H), 2.30-2.19 (m, 2H), 1.95-1.81 (m, 3H), 1.77-1.70 (m, 1H), 1.70-1.62 (m, 2H), 1.59-1.37 (m, 10H), 1.30-1.22 (m, 7H), 1.16 (t, J = 6.3 Hz, 6H), 0.92 (t, J = 7.5 Hz, 3H), 0.83 (d, J = 6.3 Hz, 3H), 0.82-0.75 (m, 4H); MSm/z 912.5093 [M+Na]⁺.

### Production of Compound I-3 and Compound I-4

3,4β-Dihydro-5-oxo-ivermectin B1a (250 mg, 0.286 mmol) was dissolved in methanol (5.7 mL), and sodium acetate (70.3 mg, 0.857 mmol) and O-(prop-2-yn-1-yl)hydroxylamine hydrochloride (92.0 mg, 0.857 mmol) were sequentially added, followed by stirring at room temperature for 3 hours. A saturated aqueous sodium hydrogen carbonate solution was added to the reaction solution, and the mixture was extracted with ethyl acetate. The organic layer was washed with saturated brine and dried over anhydrous magnesium sulfate. After the solvent was distilled off under reduced pressure, the residue was purified by silica gel column chromatography (n-hexane/ethyl acetate system) and high-performance liquid chromatography (acetonitrile/water = 85:15) to obtain Compound I-3 (isolated yield 8.4 mg, percent yield 3.2%) and Compound I-4 (isolated yield 2.8 mg, percent yield 1.1%), respectively.

### (Compound I-3)

¹H-NMR (500 MHz, CDCl₃, δ): 5.86 (dt, J = 10.3, 2.3 Hz, 1H), 5.78-5.65 (m, 2H), 5.46-5.35 (m, 2H), 4.95 (d, J = 11.5 Hz, 1H), 4.83 (s, 1H), 4.77 (d, J = 3.4 Hz, 1H), 4.71-4.58 (m, 5H), 3.93 (br s, 1H), 3.87-3.72 (m, 2H), 3.71-3.57 (m, 2H), 3.52-3.45 (m, 1H), 3.45 (s, 3H), 3.43 (s, 3H), 3.28-3.20 (m, 2H), 3.17 (t, J = 9.2 Hz, 1H), 2.94-2.86 (m, 2H), 2.58-2.46 (m, 2H), 2.43 (t, J = 2,6 Hz, 1H), 2.39-2.19 (m, 5H), 1.93-1.86 (m, 1H), 1.79-1.71 (m, 1H), 1.70-1.35 (m, 16H), 1.32-1.23 (m, 8H), 1.16 (d, J = 6.9 Hz, 3H), 0.92 (t, J = 7.5 Hz, 3H), 0.89-0.76 (m, 7H).

### (Compound I-4)

¹H-NMR (500 MHz, CDCl₃, δ): 5.86 (dt, J = 10.4, 2.5 Hz, 1H), 5.78-5.63 (m, 2H), 5.46-5.37 (m, 2H), 4.95 (d, J = 12.6 Hz, 1H), 4.77 (d, J = 3.4 Hz, 1H), 4.71 (dd, J = 2.3, 1.2 Hz, 2H), 4.69-4.56 (m, 3H), 4.00 (s, 1H), 3.94 (br s, 1H), 3.87-3.73 (m, 2H), 3.72-3.60 (m, 2H), 3.56-3.46 (m, 2H), 3.45 (s, 3H), 3.43 (s, 3H), 3.29-3.20 (m, 2H), 3.17 (t, J = 9.2 Hz, 1H), 2.90 (dd, J = 13.8, 2.9 Hz, 1H), 2.53-2.47 (m, 2H), 2.46-2.45 (m, 1H), 2.38-2.31 (m, 2H), 2.31-2.20 (m, 3H), 1.93-1.87 (m, 1H), 1.80-1.73 (m, 1H), 1.69-1.64 (m, 1H), 1.61-1.35 (m, 15H), 1.31-1.21 (m, 8H), 1.16 (d, J = 6.9 Hz, 3H), 0.95-0.81 (m, 7H), 0.78 (d, J = 5.7 Hz, 3H).

### Production of Compound I-5

Compound I-5 (isolated yield 41.9 mg, percent yield 83%) was obtained in a similar manner to [Production of Compound I-2] except that O-(prop-2-yn-1-yl)hydroxylamine hydrochloride was used in place of hydroxylamine hydrochloride.
¹H-NMR (500 MHz, CDCl₃, δ): 5.88-5.78 (m, 1H), 5.77-5.63 (m, 2H), 5.47-5.32 (m, 2H), 5.00-4.87 (m, 2H), 4.76 (br d, J = 3.44 Hz, 1H), 4.73-4.56 (m, 5H), 3.93 (br s, 1H), 3.87-3.71 (m, 2H), 3.70-3.56 (m, 2H), 3.43 (d, J = 9.4 Hz, 6H), 3.30-3.12 (m, 3H), 2.79 (dd, J = 12.60, 2.86 Hz, 1H), 2.68-2.58 (m, 1H), 2.55 (br s, 1H), 2.53-2.44 (m, 1H), 2.43-2.38 (m, 1H), 2.37-2.16 (m, 5H), 1.96-1.78 (m, 4H), 1.77-1.70 (m, 1H), 1.68-1.63 (m, 2H), 1.58-1.36 (m, 15H), 1.30-1.11 (m, 15H), 0.94-0.74 (m, 13H); MSm/z 950.6138 [M+Na]⁺.

### Production of Compound I-6

Compound I-6 (isolated yield 22.8 mg, percent yield 68%) was obtained as a mixture of isomers that were difficult to separate in a similar manner to [Production of Compound I-1] except that O-benzylhydroxylamine hydrochloride was used in place of hydroxylamine hydrochloride.
¹H-NMR (500 MHz, CDCl₃, δ): 7.36-7.27 (m, 5H), 5.89-5.82 (m, 1H), 5.78-5.63 (m, 2H), 5.45-5.38 (m, 2H), 5.20-5.07 (m, 2H), 4.95 (d, J = 9.2 Hz, 1H), 4.90 (s, 0.5H), 4.78-4.75 (m, 1H), 4.71-4.54 (m, 3H), 4.00 (s, 0.5H), 3.93 (br s, 1H), 3.86-3.74 (m, 2H), 3.71-3.59 (m, 2H), 3.60-3.45 (m, 2H), 3.45-3.43 (m, 3H), 3.42 (s, 3H), 3.26-3.19 (m, 2H), 3.17 (t, J = 9.2 Hz, 1H), 2.92-2.83 (m, 1H), 2.56 (br s, 1H), 2.53-2.46 (m, 1H), 2.37-2.19 (m, 5H), 1.93-1.85 (m, 1H), 1.78-1.71 (m, 1H), 1.68-1.60 (m, 2H), 1.59-1.38 (m, 12H), 1.31-1.21 (m, 10H), 1.15 (t, J = 7.2 Hz, 3H), 0.92 (t, J = 7.2 Hz, 3H), 0.85-0.80 (m, 4H), 0.78 (d, J = 5.7 Hz, 3H); MSm/z 1002.6900 [M+Na]⁺.

### Production of Compound I-7

Compound I-7 (isolated yield 25.2 mg, percent yield 75%) was obtained in a similar manner to [Production of Compound I-2] except that O-benzylhydroxylamine hydrochloride was used in place of hydroxylamine hydrochloride.
¹H-NMR (500 MHz, CDCl₃, δ): 7.37-7.24 (m, 5H), 5.83 (dt, J = 10.6, 2.4 Hz, 1H), 5.76-5.62 (m, 2H), 5.44-5.35 (m, 2H), 5.13 (s, 2H), 4.99 (s, 1H), 4.94 (d, J = 10.9 Hz, 1H), 4.76 (d, J = 3.4 Hz, 1H), 4.68-4.60 (m, 2H), 4.58 (s, 1H), 3.93 (br s, 1H), 3.86-3.74 (m, 2H), 3.70-3.60 (m, 2H), 3.52-3.45 (m, 1H), 3.44 (s, 3H), 3.43 (s, 3H), 3.26-3.19 (m, 2H), 3.17 (t, J = 9.2 Hz, 1H), 2.79 (dd, J = 12.6, 3.4 Hz, 1H), 2.64-2.56 (m, 1H), 2.55-2.45 (m, 2H), 2.36-2.19 (m, 4H), 1.94-1.80 (m, 3H), 1.78-1.70 (m, 1H), 1.69-1.61 (m, 1H), 1.59-1.37 (m, 12H), 1.29-1.24 (m, 7H), 1.18-1.13 (m, 6H), 0.92 (t, J = 7.5 Hz, 3H), 0.84 (d, J = 6.9 Hz, 3H), 0.82-0.74 (m, 4H); MSm/z 1002.5565 [M+Na]⁺.

### Reference Example 1

### (1) Production of 2-phenoxyisoindoline-1,3-dione

With reference to the synthesis method described in Journal of Medicinal Chemistry (2005), 48(5), 1576-1587, 2-phenoxyisoindoline-1,3-dione (isolated yield 1.47 g, percent yield 61%) was obtained.

### (2) Production of O-phenylhydroxylamine hydrochloride

2-Phenoxyisoindoline-1,3-dione (1.47 g, 6.14 mmol) obtained in Reference Example 1-(1) was dissolved in a mixed solvent of chloroform (27 mL) and methanol (3 mL), hydrazine monohydrate (1.14 mL, 18.4 mmol) was added, and the mixture was stirred at room temperature for 14 hours. After filtering the suspension, a 4M hydrogen chloride 1,4-dioxane solution (1.84 mL, 7.37 mmol) was added to the filtrate, and the mixture was stirred at room temperature for 30 minutes. After the solvent was distilled off under reduced pressure, ether was added and the suspension was filtered to obtain O-phenylhydroxylamine hydrochloride (isolated yield 0.819 g, percent yield 92%).
¹H-NMR (500 MHz, DMSO-d₆, δ): 7.41-7.28 (m, 2H), 7.16-7.11 (m, 2H), 7.02 (t, J = 7.5 Hz, 1H).

### Production of Compound I-8

Compound I-8 (isolated yield 30.0 mg, percent yield 91%) was obtained as a mixture of isomers that were difficult to separate in a similar manner to [Production of Compound I-1] except that O-phenylhydroxylamine hydrochloride obtained in Reference Example 1-(2) was used in place of hydroxylamine hydrochloride. The ¹H-NMR spectrum below is the analytical data of the main isomer.
¹H-NMR (500 MHz, CDCl₃, δ): 7.31-7.27 (m, 2H), 7.21-7.14 (m, 2H), 7.02 (t, J = 7.5 Hz, 1H), 5.89 (dt, J = 10.2, 2.4 Hz, 1H), 5.79-5.67 (m, 2H), 5.47-5.39 (m, 2H), 4.96 (d, J = 10.9 Hz, 1H), 4.77 (d, J = 4.0 Hz, 1H), 4.76-4.61 (m, 3H), 4.15 (s, 1H), 3.94 (br s, 1H), 3.87-3.74 (m, 3H), 3.72-3.61 (m, 2H), 3.53-3.46 (m, 1H), 3.45 (s, 3H), 3.43-3.40 (m, 3H), 3.28-3.19 (m, 2H), 3.17 (t, J = 9.2 Hz, 1H), 2.98-2.93 (m, 1H), 2.61-2.47 (m, 2H), 2.44-2.19 (m, 5H), 1.92 (dd, J = 12.0, 3.4 Hz, 1H), 1.80-1.74 (m, 1H), 1.71-1.61 (m, 4H), 1.59-1.31 (m, 14H), 1.30-1.24 (m, 7H), 1.19-1.14 (m, 3H), 0.93 (t, J = 7.5 Hz, 3H), 0.88-0.80 (m, 4H), 0.79 (d, J = 5.7 Hz, 3H); MSm/z 988.5402 [M+Na]⁺.

### Production of Compound I-9

Compound I-9 (isolated yield 22.6 mg, percent yield 68%) was obtained in a similar manner to [Production of Compound I-2] except that O-phenylhydroxylamine hydrochloride obtained in Reference Example 1-(2) was used in place of hydroxylamine hydrochloride.
¹H-NMR (500 MHz, CDCl₃, δ): 7.29-7.25 (m, 2H), 7.20-7.14 (m, 2H), 7.02-6.97 (m, 1H), 5.93-5.83 (m, 1H), 5.80-5.66 (m, 2H), 5.46-5.36 (m, 2H), 5.15 (s, 1H), 4.95 (d, J = 9.7 Hz, 1H), 4.77 (d, J = 3.4 Hz, 1H), 4.72 (d, J = 2.3 Hz, 1H), 4.65 (s, 1H), 3.94 (br s, 1H), 3.87-3.74 (m, 2H), 3.71-3.60 (m, 2H), 3.53-3.41 (m, 6H), 3.30-3.20 (m, 2H), 3.17 (t, J = 9.2 Hz, 1H), 2.86 (dd, J = 12.6, 3.4 Hz, 1H), 2.82-2.73 (m, 1H), 2.57-2.46 (m, 2H), 2.37-2.19 (m, 4H), 2.03-1.87 (m, 3H), 1.82-1.71 (m, 1H), 1.69-1.64 (m, 1H), 1.62 (br s, 1H), 1.59-1.38 (m, 13H), 1.31 (d, J = 6.3 Hz, 3H), 1.29-1.23 (m, 7H), 1.16 (d, J = 6.9 Hz, 3H), 0.93 (t, J = 7.2 Hz, 3H), 0.84 (d, J = 6.9 Hz, 3H), 0.82-0.74 (m, 4H); MSm/z 988.5415 [M+Na]⁺.

### Production of Compound I-10

Compound I-10 (isolated yield 27.8 mg, percent yield 87%) was obtained in a similar manner to [Production of Compound I-1] except that 2-(aminooxy)ethan-1-ol was used in place of hydroxylamine hydrochloride.
¹H-NMR (500 MHz, CDCl₃, δ): 5.88-5.82 (m, 1H), 5.78-5.64 (m, 2H), 5.39 (m, 2H), 4.94 (d, J = 10.9 Hz, 1H), 4.85 (s, 0.5H), 4.76 (d, J = 3.4 Hz, 1H), 4.73-4.54 (m, 3H), 4.22 (t, J = 4.6 Hz, 1H), 4.20-4.16 (m, 1H), 3.96 (s, 0.5H), 3.93 (br s, 1H), 3.89-3.73 (m, 4H), 3.65 (d, J = 4.6 Hz, 2H), 3.48 (d, J = 1.7 Hz, 2H), 3.44 (s, 3H), 3.42 (s, 3H), 3.28-3.19 (m, 2H), 3.16 (t, J = 9.2 Hz, 1H), 2.91-2.74 (m, 2H), 2.49 (d, J = 2.3 Hz, 1H), 2.38-2.16 (m, 6H), 1.93-1.87 (m, 1H), 1.79-1.71 (m, 1H), 1.70-1.62 (m, 2H), 1.59-1.45 (m, 12H), 1.30-1.20 (m, 10H), 1.15 (d, J = 6.9 Hz, 3H), 0.95-0.89 (m, 3H), 0.86-0.73 (m, 7H); MSm/z 956.6654 [M+Na]⁺.

### Production of Compound I-11

Compound I-11 (isolated yield 177 mg, percent yield 87%) was obtained in a similar manner to [Production of Compound I-2] except that 2-(aminooxy)ethan-1-ol was used in place of hydroxylamine hydrochloride.
¹H-NMR (500 MHz, CDCl₃, δ): 5.83 (dt, J = 10.7, 2.1 Hz, 1H), 5.78-5.64 (m, 2H), 5.44-5.35 (m, 2H), 4.97 (s, 1H), 4.95 (d, J = 11.5 Hz, 1H), 4.76 (d, J = 3.44 Hz, 1H), 4.72-4.61 (m, 2H), 4.20 (t, J = 4.3 Hz, 2H), 3.93 (br s, 1H), 3.88-3.74 (m, 4H), 3.70-3.60 (m, 2H), 3.51-3.45 (m, 1H), 3.44 (s, 3H), 3.42 (s, 3H), 3.26-3.19 (m, 2H), 3.16 (t, J = 9.2 Hz, 1H), 2.79 (dd, J = 12.3, 3.7 Hz, 1H), 2.63 (dt, J = 11.9, 5.8 Hz, 1H), 2.54-2.45 (m, 1H), 2.38-2.17 (m, 7H), 1.95-1.82 (m, 3H), 1.78-1.70 (m, 1H), 1.66 (d, J = 12.6 Hz, 1H), 1.59-1.36 (m, 12H), 1.29-1.23 (m, 7H), 1.16 (t, J = 6.6 Hz, 6H), 0.92 (t, J = 7.2 Hz, 3H), 0.84 (d, J = 6.3 Hz, 3H), 0.81-0.74 (m, 4H); MSm/z 956.5364 [M+Na]⁺.

### Reference Example 2

### (1) Production of 2,2,2-trichloroethyl (2-hydroxyethyl)carbamate

2-Aminoethan-1-ol (0.611 g, 10.0 mmol) and triethylamine (2.09 mL, 15.0 mmol) were dissolved in methylene chloride (50 mL) and cooled in ice to 0°C. Then, 2,2,2-trichloroethyl carbonochloridate (1.48 mL, 11.0 mmol) was added dropwise, and the mixture was stirred at the same temperature for 1 hour. The temperature was raised to room temperature, and the mixture was further stirred for 17 hours. Water was added to the reaction solution, and the mixture was extracted with chloroform. The organic layer was washed with saturated brine and dried over anhydrous magnesium sulfate. After the solvent was distilled off under reduced pressure, the residue was purified by silica gel column chromatography (n-hexane/ethyl acetate system) to obtain 2,2,2-trichloroethyl (2-hydroxyethyl)carbamate (isolated yield 1.85 g, percent yield 78%).
¹H-NMR (500 MHz, CDCl₃, δ): 4.74 (s, 2H), 3.78 (q, J = 5.2 Hz, 2H), 3.42 (q, J = 4.6 Hz, 2H), 1.81 (t, J = 5.2 Hz, 1H).

### (2) Production of 2,2,2-trichloroethyl (2-((1,3-dioxoisoindolin-2-yl)oxy)ethyl)carbamate

2-Hydroxyisoindoline-1,3-dione (1.25 g, 7.67 mmol), 2,2,2-trichloroethyl (2-hydroxyethyl) carbamate (1.85 g, 7.82 mmol) obtained in Reference Example 2-(1), and triphenylphosphine (4.10 g, 15.7 mmol) were dissolved in THF (39 mL) and cooled in ice to 0°C. Then, a 2.2M diethyl (E)-diazene-1,2-dicarboxylate toluene solution (7.1 ml, 15.7 mmol) was added dropwise, and the mixture was stirred at room temperature for 14 hours. Water was added to the reaction solution, and the mixture was extracted with ethyl acetate. The organic layer was washed with saturated brine and dried over anhydrous magnesium sulfate. After the solvent was distilled off under reduced pressure, the residue was purified by silica gel column chromatography (n-hexane/ethyl acetate system) to obtain 2,2,2-trichloroethyl (2-((1,3-dioxoisoindolin-2-yl)oxy)ethyl)carbamate (isolated yield 1.32 g, percent yield 44%).
¹H-NMR (500 MHz, CDCl₃, δ): 7.89-7.84 (m, 2H), 7.81-7.77 (m, 2H), 6.20 (br s, 1H), 4.77 (s, 2H), 4.30 (t, J = 4.6 Hz, 2H), 3.56 (q, J = 4.0 Hz, 2H).

### (3) Production of 2,2,2-trichloroethyl (2-(aminooxy)ethyl)carbamate

2,2,2-Trichloroethyl (2-((1,3-dioxoisoindolin-2-yl)oxy)ethyl)carbamate (1.32 g, 3.46 mmol) obtained in Reference Example 2-(2) was dissolved in methylene chloride (34.6 mL), hydrazine monohydrate (0.202 mL, 4.15 mmol) was added, and the mixture was stirred at room temperature for 13 hours. After filtering the suspension, the solvent was distilled off under reduced pressure. Then, the residue was purified by silica gel column chromatography (n-hexane/ethyl acetate system) to obtain 2,2,2-trichloroethyl (2-(aminooxy)ethyl)carbamate (isolated yield 1.02 g, quantitative).
¹H-NMR (500 MHz, CDCl₃, δ): 4.73 (s, 2H), 3.76 (t, J = 4.6 Hz, 2H), 3.48 (q, J = 4.6 Hz, 2H).

### Production of Compound I-12

Compound I-12 (isolated yield 37.3 mg, percent yield 74%) was obtained as a mixture of isomers that were difficult to separate in a similar manner to [Production of Compound I-1] except that 2,2,2-trichloroethyl (2-(aminooxy)ethyl)carbamate obtained in Reference Example 2-(3) was used in place of hydroxylamine hydrochloride.
¹H-NMR (500 MHz, CDCl₃, δ): 5.90-5.80 (m, 1H), 5.80-5.64 (m, 2H), 5.48-5.31 (m, 2H), 4.98-4.91 (m, 1H), 4.82 (s, 0.6H), 4.78-4.59 (m, 6H), 4.42-4.26 (m, 1H), 4.26-4.14 (m, 4H), 3.96 (s, 0.4H), 3.94-3.89 (m, 1H), 3.86-3.72 (m, 2H), 3.70-3.58 (m, 2H), 3.56-3.45 (m, 3H), 3.44 (s, 3H), 3.43-3.39 (m, 3H), 3.30-3.19 (m, 2H), 3.16 (t, J = 9.2 Hz, 1H), 2.91-2.83 (m, 1H), 2.60 (s, 1H), 2.53-2.45 (m, 1H), 2.38-2.19 (m, 4H), 1.90 (dd, J = 12.0, 5.2 Hz, 1H), 1.78-1.71 (m, 3H), 1.70-1.61 (m, 2H), 1.59-1.37 (m, 12H), 1.35-1.21 (m, 8H), 1.18-1.14 (m, 3H), 0.92 (t, J = 7.5 Hz, 3H), 0.83 (d, J = 6.9 Hz, 3H), 0.81-0.74 (m, 4H); MSm/z 1129.4558 [M+Na]⁺.

### Production of Compound I-13

Compound I-13 (isolated yield 35.3 mg, percent yield 93%) was obtained in a similar manner to [Production of Compound I-2] except that 2,2,2-trichloroethyl (2-(aminooxy)ethyl)carbamate obtained in Reference Example 2-(3) was used in place of hydroxylamine hydrochloride.
¹H-NMR (500 MHz, CDCl₃, δ): 5.87-5.62 (m, 3H), 5.48-5.30 (m, 2H), 4.97-4.91 (m, 1.5H), 4.80-4.65 (m, 5.5H), 4.31-4.15 (m, 3H), 3.96-3.88 (m, 1H), 3.86-3.72 (m, 2H), 3.70-3.55 (m, 2H), 3.54-3.44 (m, 4H), 3.44 (s, 3H), 3.42 (s, 3H), 3.29-3.19 (m, 2H), 3.16 (t, J = 9.2 Hz, 1H), 2.79 (dd, J = 12.0, 4.0 Hz, 1H), 2.68-2.59 (m, 1H), 2.57 (br s, 1H), 2.54-2.42 (m, 1H), 2.38-2.18 (m, 4H), 1.94-1.82 (m, 3H), 1.77-1.62 (m, 4H), 1.59-1.37 (m, 12H), 1.34-1.20 (m, 6H), 1.20-1.07 (m, 6H), 0.92 (t, J = 7.5 Hz, 3H), 0.89-0.74 (m, 7H); MSm/z 1129.4570 [M+Na]⁺.

### Reference Example 3

### (1) Production of 2-(3,3,3-trifluoropropoxy)isoindoline-1,3-dione

2-Hydroxyisoindoline-1,3-dione (0.500 g, 3.07 mmol), 3,3,3-trifluoropropan-1-ol (0.269 mL, 3.07 mmol), and triphenylphosphine (0.965 g, 3.68 mmol) were dissolved in THF (15.4 mL) and cooled in ice to 0°C. Thereafter, 2.2M diethyl (E)-diazine-1,2-dicarboxylate toluene solution (1.67 mL, 3.68 mmol) was added dropwise, and the mixture was stirred at room temperature for 1 hour. Water was added to the reaction solution, and extracted with ethyl acetate. The organic layer was washed with saturated brine and dried over anhydrous magnesium sulfate. After the solvent was distilled off under reduced pressure, the residue was purified by silica gel column chromatography (chloroform/methanol system) to obtain 2-(3,3,3-trifluoropropoxy)isoindoline-1,3-dione (isolated yield: 0.630 g, percent yield 79%).
¹H-NMR (500 MHz, CDCl₃, δ): 7.88-7.83 (m, 2H), 7.80-7.76 (m, 2H), 4.43 (t, J = 6.9 Hz, 2H), 2.76-2.66 (m, 2H).

### (2) Production of O-(3,3,3-trifluoropropyl)hydroxylamine hydrochloride.

2-(3,3,3-Trifluoropropoxy)isoindoline-1,3-dione (0.630 g, 2.43 mmol) obtained in Reference Example 3-(1) was dissolved in ethanol (24.3 mL), after that, hydrazine monohydrate (0.163 mL, 2.67 mmol) was added and stirred at 65°C for 1 hour. After filtering the suspension, 4M hydrogen chloride 1,4-dioxane solution (1.22 mL, 4.86 mmol) to the filtrate was added, and stirred at room temperature for 15 minutes. After distilling off the solvent under reduced pressure, O-(3,3,3-trifluoropropyl) hydroxylamine hydrochloride (isolated yield 0.339 g, percent yield 84%) was obtained. ¹H-NMR (500 MHz, DMSO-d₆, δ): 4.14 (t, J = 5.7 Hz, 2H), 2.82-2.66 (m, 2H).

### Production of Compound I-14

Compound I-14 (isolated yield 16.1 mg, percent yield 48%) was obtained as a mixture of isomers that were difficult to separate in a similar manner to [Production of Compound I-1] except that O-(3,3,3-trifluoropropyl)hydroxylamine hydrochloride obtained in Reference Example 3-(2) was used in place of hydroxylamine hydrochloride.
¹H-NMR (500 MHz, CDCl₃, δ): 5.85 (dt, J = 10.4, 2.8 Hz, 1H), 5.77-5.66 (m, 2H), 5.45-5.37 (m, 2H), 4.95 (d, J = 10.9 Hz, 1H), 4.78-4.75 (m, 1.6H), 4.71-4.55 (m, 3H), 4.33-4.23 (m, 2H), 3.96 (s, 0.4H), 3.93 (br s, 1H), 3.86-3.74 (m, 2H), 3.71-3.60 (m, 2H), 3.52-3.47 (m, 1H), 3.44 (s, 3H), 3.42 (s, 3H), 3.26-3.19 (m, 2H), 3.16 (t, J = 9.2 Hz, 1H), 2.92-2.81 (m, 2H), 2.56-2.44 (m, 4H), 2.37-2.20 (m, 5H), 1.90 (dd, J = 11.7, 4.9 Hz, 1H), 1.78-1.72 (m, 1H), 1.69-1.60 (m, 3H), 1.59-1.38 (m, 12H), 1.32-1.22 (m, 8H), 1.20 (d, J = 7.5 Hz, 1H), 1.19-1.14 (m, 3H), 0.92 (t, J = 7.2 Hz, 3H), 0.84 (d, J = 6.9 Hz, 3H), 0.82-0.76 (m, 4H); MSm/z 1008.5277 [M+Na]⁺.

### Production of Compound I-15

Compound I-15 (isolated yield 21.4 mg, percent yield 63%) was obtained in a similar manner to [Production of Compound I-2] except that O-(3,3,3-trifluoropropyl)hydroxylamine hydrochloride obtained in Reference Example 3-(2) was used in place of hydroxylamine hydrochloride.
¹H-NMR (500 MHz, CDCl₃, δ): 5.85-5.80 (m, 1H), 5.77-5.63 (m, 2H), 5.44-5.34 (m, 2H), 4.94 (d, J = 10.9 Hz, 1H), 4.88 (s, 1H), 4.76 (d, J = 2.9 Hz, 1H), 4.65 (t, J = 2.0 Hz, 2H), 4.60 (s, 1H), 4.29 (t, J = 6.9 Hz, 2H), 3.93 (br s, 1H), 3.85-3.74 (m, 2H), 3.70-3.60 (m, 2H), 3.51-3.45 (m, 1H), 3.44 (s, 3H), 3.42 (s, 3H), 3.26-3.19 (m, 2H), 3.16 (t, J = 9.2 Hz, 1H), 2.78 (dd, J = 12.6, 3.4 Hz, 1H), 2.65-2.56 (m, 1H), 2.54-2.45 (m, 4H), 2.36-2.30 (m, 2H), 2.29-2.20 (m, 2H), 1.94-1.81 (m, 3H), 1.73 (br s, 1H), 1.69-1.63 (m, 1H), 1.60 (br s, 1H), 1.59-1.38 (m, 12H), 1.30-1.22 (m, 7H), 1.16 (t, J = 6.9 Hz, 5H), 0.92 (t, J = 7.5 Hz, 3H), 0.84 (d, J = 6.9 Hz, 3H), 0.82-0.75 (m, 4H); MSm/z 1008.5268 [M+Na]⁺.

### Reference Example 4

### (1) Production of 2-(2,2,2-trifluoroethoxy)isoindoline-1,3-dione

2-Hydroxyisoindoline-1,3-dione (1.63 g, 10.0 mmol) was dissolved in methylene chloride (33.3 mL), DIPEA (1.91 mL, 11.0 mmol) and 2,2,2-trifluoroethyl trifluoromethanesulfonate (1.58 mL, 11.0 mmol) were sequentially added, and the mixture was stirred at room temperature for 16 hours. Water was added to the reaction solution, and the mixture was extracted with methylene chloride. The organic layer was washed with saturated brine and dried over anhydrous magnesium sulfate. After the solvent was distilled off under reduced pressure, the residue was purified by short-pad silica gel column chromatography to obtain 2-(2,2,2-trifluoroethoxy)isoindoline-1,3-dione (isolated yield 1.88 g, percent yield 77%).
¹H-NMR (500 MHz, CDCl₃, δ): 7.91-7.85 (m, 2H), 7.82-7.77 (m, 2H), 4.56 (q, J = 8.0 Hz, 2H).

### (2) Production of O-(2,2,2-trifluoroethyl)hydroxylamine hydrochloride

2-(2,2,2-Trifluoroethoxy)isoindoline-1,3-dione (1.88 g, 7.67 mmol) obtained in Reference Example 4-(1) was dissolved in ethanol (76.7 mL), hydrazine monohydrate (0.513 mL, 8.44 mmol) was added, and the mixture was stirred at 65°C for 30 minutes. After filtering the suspension, a 4M hydrogen chloride 1,4-dioxane solution (3.84 mL, 15.3 mmol) was added to the filtrate, and the mixture was stirred at room temperature for 15 minutes. By distilling off the solvent under reduced pressure, O-(2,2,2-trifluoroethyl) hydroxylamine hydrochloride (isolated yield 1.16 g, percent yield 99%) was obtained.
¹H-NMR (500 MHz, DMSO-d₆, δ): 4.37 (q, J = 9.2 Hz, 2H).

### Production of Compound I-16

Compound I-16 (isolated yield 11.3 mg, percent yield 34%) was obtained as a mixture of isomers that were difficult to separate in a similar manner to [Production of Compound I-1] except that O-(2,2,2-trifluoroethyl)hydroxylamine hydrochloride obtained in Reference Example 4-(2) was used in place of hydroxylamine hydrochloride.
¹H-NMR (500 MHz, CDCl₃, δ): 5.91-5.82 (m, 1H), 5.79-5.64 (m, 2H), 5.46-5.37 (m, 2H), 4.95 (d, J = 10.9 Hz, 1H), 4.81 (s, 0.6H), 4.77 (d, J = 3.4 Hz, 1H), 4.73-4.56 (m, 3H), 4.51-4.39 (m, 2H), 3.97 (s, 0.4H), 3.94 (br s, 1H), 3.87-3.73 (m, 2H), 3.71-3.57 (m, 2H), 3.52-3.45 (m, 1H), 3.45 (s, 3H), 3.43 (s, 3H), 3.27-3.20 (m, 2H), 3.17 (t, J = 9.2 Hz, 1H), 2.92-2.83 (m, 1H), 2.54-2.46 (m, 2H), 2.38-2.17 (m, 5H), 1.95-1.85 (m, 1H), 1.79-1.71 (m, 1H), 1.70-1.61 (m, 1H), 1.61-1.38 (m, 14H), 1.31-1.23 (m, 10H), 1.20-1.12 (m, 3H), 0.92 (t, J = 7.5 Hz, 3H), 0.84 (d, J = 6.9 Hz, 3H), 0.82-0.76 (m, 4H); MSm/z 994.5120 [M+Na]⁺.

### Production of Compound I-17

Compound I-17 (isolated yield 14.9 mg, percent yield 45%) was obtained in a similar manner to [Production of Compound I-2] except that O-(2,2,2-trifluoroethyl)hydroxylamine hydrochloride obtained in Reference Example 4-(2) was used in place of hydroxylamine hydrochloride.
¹H-NMR (500 MHz, CDCl₃, δ): 5.91-5.82 (m, 1H), 5.80-5.63 (m, 2H), 5.44-5.35 (m, 2H), 4.94 (d, J = 10.9 Hz, 1H), 4.92 (s, 1H), 4.77 (d, J = 3.4 Hz, 1H), 4.67 (d, J = 1.7 Hz, 2H), 4.62 (s, 1H), 4.44 (q, J = 8.6 Hz, 2H), 3.93 (br s, 1H), 3.86-3.73 (m, 3H), 3.71-3.58 (m, 2H), 3.52-3.45 (m, 1H), 3.44 (s, 3H), 3.43-3.39 (m, 4H), 3.26-3.19 (m, 2H), 3.17 (t, J = 9.2 Hz, 1H), 2.79 (dd, J = 12.3, 3.2 Hz, 1H), 2.67-2.58 (m, 1H), 2.55-2.45 (m, 2H), 2.38-2.19 (m, 4H), 1.98-1.81 (m, 3H), 1.77-1.70 (m, 1H), 1.69-1.62 (m, 1H), 1.60-1.37 (m, 8H), 1.31-1.21 (m, 9H), 1.20-1.11 (m, 6H), 0.92 (t, J = 7.5 Hz, 3H), 0.90-0.74 (m, 7H); MSm/z 994.5115 [M+Na]⁺.

### Reference Example 5

### (1) Production of 2-(2-methoxyethoxy)isoindoline-1,3-dione

2-Hydroxyisoindoline-1,3-dione (1.63 g, 10.0 mmol) was dissolved in DMF (4.35 mL), 1-bromo-2-methoxyethane (4.76 mL, 50.0 mmol) and triethylamine (5.12 mL, 36.8 mmol) were sequentially added, and the mixture was stirred at room temperature for 2 hours and further at 50°C for 2 hours. Water was added to the reaction solution, followed by filtration, and the filtrate was dissolved in ethyl acetate and washed with water and 1N hydrochloric acid. After the organic layer was dried over anhydrous magnesium sulfate, the solvent was distilled off under reduced pressure to obtain 2-(2-methoxyethoxy)isoindoline-1,3-dione (isolated yield 1.20 g, percent yield 54%).
¹H-NMR (500 MHz, CDCl₃, δ): 7.88-7.82 (m, 2H), 7.78-7.71 (m, 2H), 4.40-4.33 (m, 2H), 3.79-3.73 (m, 2H), 3.40-3.38 (m, 3H).

### (2) Production of O-(2-methoxyethyl)hydroxylamine hydrochloride

2-(2-Methoxyethoxy)isoindoline-1,3-dione (1.00 g, 4.52 mmol) obtained in Reference Example 5-(1) was dissolved in ethanol (45.2 mL), hydrazine monohydrate (0.302 mL, 4.97 mmol) was added, and the mixture was stirred at 65°C for 1 hour. After filtering the suspension, a 4M hydrogen chloride 1,4-dioxane solution (2.26 mL, 9.04 mmol) was added to the filtrate, and the mixture was stirred at room temperature for 15 minutes. By distilling off the solvent under reduced pressure, O-(2-methoxyethyl)hydroxylamine hydrochloride (isolated yield 0.338 g, percent yield 59%) was obtained. ¹H-NMR (500 MHz, DMSO-d₆, δ): 10.7 (br s, 3H), 4.12-4.07 (m, 2H), 3.57-3.53 (m, 2H), 3.26 (s, 3H).

### Production of Compound I-18

Compound I-18 (isolated yield 14.8 mg, percent yield 46%) was obtained as a mixture of isomers that were difficult to separate in a similar manner to [Production of Compound I-1] except that O-(2-methoxyethyl)hydroxylamine hydrochloride obtained in Reference Example 5-(2) was used in place of hydroxylamine hydrochloride.
¹H-NMR (500 MHz, CDCl₃, δ): 5.89-5.81 (m, 1H), 5.77-5.63 (m, 2H), 5.45-5.38 (m, 2H), 4.95 (d, J = 9.2 Hz, 1H), 4.85 (s, 0.4H), 4.77 (d, J = 4.0 Hz, 1H), 4.70-4.55 (m, 3H), 4.26 (t, J = 4.9 Hz, 1H), 4.24-4.17 (m, 1H), 3.98 (s, 0.6H), 3.93 (br s, 1H), 3.86-3.74 (m, 2H), 3.71-3.54 (m, 5H), 3.52-3.52 (m, 1H), 3.45 (s, 3H), 3.43 (s, 3H), 3.39-3.35 (m, 3H), 3.26-3.19 (m, 2H), 3.17 (t, J = 9.2 Hz, 1H), 2.92-2.82 (m, 1H), 2.52-2.46 (m, 2H), 2.37-2.20 (m, 5H), 1.93-1.87 (m, 1H), 1.78-1.73 (m, 1H), 1.70-1.64 (m, 1H), 1.40 (d, J = 7.5 Hz, 1H), 1.62-1.35 (m, 12H), 1.31-1.19 (m, 10H), 1.16 (d, J = 6.9 Hz, 3H), 0.92 (t, J = 7.5 Hz, 3H), 0.90-0.76 (m, 7H); MSm/z 970.5511 [M+Na]⁺.

### Production of Compound I-19

Compound I-19 (isolated yield 20.0 mg, percent yield 62%) was obtained in a similar manner to [Production of Compound I-2] except that O-(2-methoxyethyl) hydroxylamine hydrochloride obtained in Reference Example 5-(2) was used in place of hydroxylamine hydrochloride.
¹H-NMR (500 MHz, CDCl₃, δ): 5.86-5.79 (m, 1H), 5.75-5.65 (m, 2H), 5.43-5.34 (m, 2H), 4.96-4.92 (m, 2H), 4.76 (d, J = 3.4 Hz, 1H), 4.69-4.60 (m, 2H), 4.56 (s, 1H), 4.23 (t, J = 5.2 Hz, 2H), 3.93 (br s, 1H), 3.86-3.74 (m, 2H), 3.71-3.58 (m, 4H), 3.52-3.45 (m, 1H), 3.44 (s, 3H), 3.43 (s, 3H), 3.36 (s, 3H), 3.26-3.19 (m, 2H), 3.17 (t, J = 9.2 Hz, 1H), 2.79 (dd, J = 12.6, 3.4 Hz, 1H), 2.64-2.56 (m, 1H), 2.52-2.46 (m, 2H), 2.38-2.19 (m, 4H), 1.95-1.79 (m, 3H), 1.77-1.71 (m, 1H), 1.68-1.62 (m, 2H), 1.59-1.36 (m, 12H), 1.29-1.24 (m, 6H), 1.16 (t, J = 7.2 Hz, 6H), 0.92 (t, J = 7.5 Hz, 3H), 0.86-0.76 (m, 7H); MSm/z 970.5495 [M+Na]⁺.

### Reference Example 6

### (1) Production of 2-(2-hydroxyethoxy)isoindoline-1,3-dione

2-Hydroxyisoindoline-1,3-dione (1.63 g, 10.0 mmol) was dissolved in acetonitrile (33.3 mL), 2-bromoethan-1-ol (1.43 mL, 20.0 mmol) and triethylamine (2.78 mL, 20 mmol) were sequentially added, and the mixture was stirred at room temperature for 4 hours. Water was added to the reaction solution, and the mixture was extracted with chloroform. The organic layer was washed with saturated brine and dried over anhydrous magnesium sulfate. After the solvent was distilled off under reduced pressure, the residue was purified by silica gel column chromatography (n-hexane/ethyl acetate system) to obtain 2-(2-hydroxyethoxy)isoindoline-1,3-dione (isolated yield 1.80 g, percent yield 87%).
¹H-NMR (500 MHz, CDCl₃, δ): 7.90-7.85 (m, 2H), 7.81-7.77 (m, 2H), 4.32-4.29 (m, 2H), 3.83-3.78 (m, 2H), 3.46 (t, J = 7.2 Hz, 1H).

### (2) Production of 2-(2-((tertbutyldimethylsilyl)oxy)ethoxy)isoindoline-1,3-dione

2-(2-Hydroxyethoxy)isoindoline-1,3-dione (0.449 g, 2.17 mmol) obtained in Reference Example 6-(1) was dissolved in DMF (21.7 mL), and imidazole (0.885 g, 13.0 mmol), DMAP (26.5 mg, 0.217 mmol) and tert-butylchlorodimethylsilane (0.981 g, 6.51 mmol) were sequentially added, followed by stirring at room temperature for 1 hour. Water was added to the reaction solution, and the mixture was extracted with ether. The organic layer was washed with saturated brine and dried over anhydrous magnesium sulfate. After the solvent was distilled off under reduced pressure, the residue was purified by silica gel column chromatography (n-hexane/ethyl acetate system) to obtain 2-(2-((tertbutyldimethylsilyl)oxy)ethoxy)isoindoline-1,3-dione (isolated yield 0.704 g, percent yield 100%).
¹H-NMR (500 MHz, CDCl₃, δ): 7.85-7.80 (m, 2H), 7.75-7.71 (m, 2H), 4.32-4.29 (m, 2H), 4.01-3.98 (m, 2H), 3.46 (t, J = 7.2 Hz, 1H), 0.81 (s, 9H), 0.01 (s, 3H).

### (3) Production of O-(2-((tertbutyldimethylsilyl)oxy)ethyl)hydroxylamine hydrochloride

2-(2-((tertButyldimethylsilyl)oxy)ethoxy)isoindoline-1,3-dione (1.00 g, 3.11 mmol) obtained in Reference Example 6-(2) was dissolved in ethanol (31.1 mL), hydrazine monohydrate (0.189 mL, 3.11 mmol) was added, and the mixture was stirred at 67°C for 30 minutes. After filtering the suspension, the filtrate was washed with ether, and the solvent was distilled off under reduced pressure to obtain O-(2-((tert-butyldimethylsilyl)oxy)ethyl)hydroxylamine hydrochloride (isolated yield 0.577 g, percent yield 97%).
¹H-NMR (500 MHz, DMSO-d₆, δ): 5.95 (br s, 2H)., 3.73-3.67 (m, 2H), 3.57-3.53 (m, 2H), 0.86 (s, 9H), 0.01 (s, 3H).

### Production of Compound I-20

Compound I-20 (isolated yield 38.5 mg, percent yield 64%) was obtained as a mixture of isomers that were difficult to separate in a similar manner to [Production of Compound I-1] except that O-(2-((tertbutyldimethylsilyl)oxy)ethyl)hydroxylamine hydrochloride obtained in Reference Example 6-(3) was used in place of hydroxylamine hydrochloride.
¹H-NMR (500 MHz, CDCl₃, δ): 5.88-5.79 (m, 1H), 5.78-5.63 (m, 2H), 5.45-5.36 (m, 2H), 4.99-4.93 (m, 1H), 4.82 (s, 0.4H), 4.76 (d, J = 2.9 Hz, 1H), 4.70-4.54 (m, 2.6H), 4.54-4.51 (m, 0.4H), 4.20-4.09 (m, 2H), 3.97 (s, 0.6H), 3.93 (br s, 1H), 3.87-3.74 (m, 4H), 3.71-3.59 (m, 2H), 3.56-3.45 (m, 2H), 3.44 (s, 3H), 3.42 (s, 3H), 3.26-3.19 (m, 2H), 3.16 (t, J = 9.2 Hz, 1H), 2.91-2.81 (m, 1H), 2.58-2.45 (m, 2H), 2.36-2.27 (m, 3H), 2.27-2.19 (m, 2H), 1.92-1.85 (m, 1H), 1.79-1.72 (m, 1H), 1.66 (d, J = 13.2 Hz, 1H), 1.63-1.37 (m, 12H), 1.31-1.23 (m, 8H), 1.21 (d, J = 6.9 Hz, 2H), 1.15 (d, J = 7.5 Hz, 3H), 0.92 (t, J = 7.2 Hz, 3H), 0.89-0.86 (m, 10H), 0.86-0.80 (m, 4H), 0.78 (d, J = 6.3 Hz, 3H), 0.06-0.02 (m, 6H); MSm/z 1070.6219 [M+Na]⁺.

### Production of Compound I-21

Compound I-21 (isolated yield 51.3 mg, percent yield 86%) was obtained in a similar manner to [Production of Compound I-2] except that O-(2-((tert-butyldimethylsilyl)oxy)ethyl)hydroxylamine hydrochloride obtained in Reference Example 6-(3) was used in place of hydroxylamine hydrochloride.
¹H-NMR (500 MHz, CDCl₃, δ): 5.85-5.79 (m, 1H), 5.76-5.63 (m, 2H), 5.44-5.34 (m, 2H), 4.98-4.90 (m, 2H), 4.79-4.73 (m, 1H), 4.67-4.58 (m, 2H), 4.53 (s, 1H), 4.18-4.09 (m, 2H), 3.95-3.90 (m, 1H), 3.86-3.74 (m, 4H), 3.70-3.59 (m, 2H), 3.52-3.46 (m, 1H), 3.44 (s, 3H), 3.42 (s, 3H), 3.26-3.19 (m, 2H), 3.16 (t, 9.2 Hz, 1H), 2.81-2.73 (m, 1H), 2.65-2.54 (m, 1H), 2.55-2.45 (m, 3H), 2.36-2.18 (m, 4H), 1.95-1.78 (m, 3H), 1.77-1.71 (m, 1H), 1.69-1.63 (m, 1H), 1.62-1.36 (m, 12H), 1.31-1.23 (m, 7H), 1.18-1.14 (m, 5H), 0.95-0.89 (m, 3H), 0.89-0.86 (m, 9H), 0.84 (d, J = 6.9 Hz, 3H), 0.81-0.74 (m, 4H), 0.05 (s, 6H); MSm/z 1070.6221 [M+Na]⁺.

### Production of Compound I-22

3,4β-Dihydro-5-oxo-ivermectin B1a (30.0 mg, 0.034 mmol) was dissolved in methanol (0.69 mL), and sodium acetate (16.9 mg, 0.206 mmol) and 2-(aminooxy)ethan-1-amine dihydrochloride (10.2 mg, 0.069 mmol) were sequentially added, followed by stirring at room temperature for 3 hours. A saturated aqueous sodium hydrogen carbonate solution was added to the reaction solution, and the mixture was extracted with ethyl acetate. The organic layer was washed with saturated brine and dried over anhydrous magnesium sulfate. After the solvent was distilled off under reduced pressure, the residue was purified by silica gel column chromatography (chloroform/methanol system) to obtain Compound I-22 (isolated yield 21.3 mg, percent yield 67%) as a mixture of isomers that were difficult to separate.
¹H-NMR (500 MHz, CD₃OD, δ) : 5.92-5.84 (m, 2H), 5.78-5.68 (m, 1H) , 5.34 (d, J = 3.4 Hz, 1H), 5.28-5.18 (m, 1H), 5.13 (t, J = 7.5 Hz, 1H), 4.80 (d, J = 2.9 Hz, 1H), 4.76 (s, 0.6H), 4.71-4.58 (m, 2H), 4.31-4.18 (m, 2H), 3.99 (br s, 1H), 3.91-3.85 (m, 1H), 3.84 (s, 0.4H), 3.76-3.62 (m, 3H), 3.42 (s, 3H), 3.41 (s, 3H), 3.27 (d, J = 8.0 Hz, 1H), 3.22-3.13 (m, 3H), 3.03 (t, J = 9.2 Hz, 1H), 2.95-2.87 (m, 1H), 2.86-2.78 (m, 1H) , 2.68-2.61 (m, 1H), 2.38-2.24 (m, 5H), 2.04-1.96 (m, 1H), 1.89-1.82 (m, 1H), 1.66-1.36 (m, 15H), 1.34-1.19 (m, 12H), 1.17 (d, J = 6.9 Hz, 3H), 0.97 (t, J = 7.5 Hz, 3H), 0.91-0.78 (m, 7H); MSm/z 933.5685 [M+H]⁺.

### Production of Compound I-23

3,4α-Dihydro-5-oxo-ivermectin B1a (30.0 mg, 0.034 mmol) was dissolved in methanol (0.69 mL), and sodium acetate (16.9 mg, 0.206 mmol) and 2-(aminooxy)ethan-1-amine dihydrochloride (10.2 mg, 0.069 mmol) were sequentially added, followed by stirring at room temperature for 4 hours. A saturated aqueous sodium hydrogen carbonate solution was added to the reaction solution, and the mixture was extracted with ethyl acetate. The organic layer was washed with saturated brine and dried over anhydrous magnesium sulfate. After the solvent was distilled off under reduced pressure, the residue was purified by silica gel column chromatography (chloroform/methanol system) to obtain Compound I-23 (isolated yield 19.8 mg, percent yield 74%).
¹H-NMR (500 MHz, CDCl₃, δ): 5.89-5.81 (m, 1H), 5.77-5.64 (m, 2H), 5.39 (d, J = 3.4 Hz, 1H), 5.36-5.27 (m, 1H), 5.02-4.92 (m, 2H), 4.77 (d, J = 3.4 Hz, 1H), 4.66 (s, 2H), 4.24-4.14 (m, 2H), 3.98-3.74 (m, 5H), 3.69-3.60 (m, 2H), 3.52-3.45 (m, 1H), 3.43 (s, 3H), 3.42 (s, 3H), 3.26-3.19 (m, 2H), 3.16 (t, J = 9.2 Hz, 1H), 3.11-2.99 (m, 2H), 2.77 (dd, J = 12.6, 3.4 Hz, 1H), 2.66-2.58 (m, 1H), 2.54-2.46 (m, 1H) , 2.36-2.20 (m, 4H), 1.99-1.81 (m, 5H), 1.79-1.72 (m, 1H), 1.68-1.61 (m, 1H), 1.60-1.32 (m, 12H), 1.29-1.23 (m, 7H), 1.19-1.12 (m, 6H), 0.92 (t, J = 7.2 Hz, 3H), 0.83 (d, J = 6.9 Hz, 3H), 0.81-0.75 (m, 4H); MSm/z 933.5692 [M+H]⁺.

### Production of Compound I-24 and Compound I-25

Compound I-20 (33.0 mg, 0.031 mmol) was added to a 3% sulfuric acid methanol solution (0.63 mL) and stirred at room temperature for 2 hours. A saturated aqueous sodium hydrogen carbonate solution was added to the reaction solution, and the mixture was extracted with chloroform. The organic layer was dried over anhydrous magnesium sulfate, and the solvent was distilled off under reduced pressure. The residue was purified by preparative thin-layer chromatography (n-hexane/acetone = 2:1) to obtain Compound I-24 (isolated yield 16.2 mg, percent yield 65%) and Compound I-25 (isolated yield 4.5 mg, percent yield 22%), respectively.

### (Compound I-24)

¹H-NMR (500 MHz, CDCl₃, δ): 5.85-5.79 (m, 1H), 5.77-5.66 (m, 2H), 5.44-5.35 (m, 1H), 5.35-5.28 (m, 1H), 4.70-4.55 (m, 3H), 4.23 (t, J = 4.3 Hz, 2H), 4.00 (br s, 1H), 3.96 (s, 1H), 3.91-3.83 (m, 2H), 3.73-3.65 (m, 1H), 3.56-3.49 (m, 1H), 3.19 (dd, J = 9.2, 1.7 Hz, 1H), 2.88 (dd, J = 13.8, 2.9 Hz, 1H), 2.51 (br s, 1H), 2.35-2.16 (m, 4H), 1.94-1.84 (m, 1H), 1.77-1.70 (m, 1H), 1.70-1.63 (m, 1H), 1.63-1.39 (m, 12H), 1.30-1.19 (m, 4H), 1.17 (d, J = 6.9 Hz, 3H), 0.95 (t, J = 7.2 Hz, 3H), 0.85-0.77 (m, 7H); MSm/z 646.3950 [M+H]⁺.

### (Compound I-25)

¹H-NMR (500 MHz, CDCl₃, δ): 5.86 (dt, J = 10.5, 2.5 Hz, 1H), 5.78-5.64 (m, 2H), 5.48-5.38 (m, 1H), 4.99-4.92 (m, 1H), 4.81 (d, J = 3.4 Hz, 1H), 4.68-4.55 (m, 3H), 4.23 (t, J = 4.0 Hz, 2H), 3.96 (s, 1H), 3.95 (br s, 1H), 3.90-3.81 (m, 3H), 3.71-3.65 (m, 1H), 3.60-3.52 (m, 2H), 3.49 (s, 3H), 3.22 (dd, J = 9.2, 1.7 Hz, 1H), 3.16 (t, J = 9.2 Hz, 1H), 2.90 (dd, J = 13.8, 2.9 Hz, 1H), 2.55 (br s, 1H), 2.51-2.45 (m, 1H), 2.37-2.17 (m, 5H), 1.92-1.85 (m, 1H), 1.79-1.73 (m, 1H), 1.70-1.64 (m, 1H), 1.637-1.38 (m, 12H), 1.30-1.20 (m, 7H), 1.15 (d, J = 6.9 Hz, 3H), 0.95-0.90 (m, 3H), 0.86-0.80 (m, 4H), 0.79 (d, J = 5.7 Hz, 3H); MSm/z 790.4744 [M+H]⁺.

### Production of Compounds I-26 and 1-27

Compound I-26 (isolated yield 22.5 mg, percent yield 62%) and Compound I-27 (isolated yield 6.2 mg, percent yield 21%) were obtained in a similar manner to [Production of Compound I-24 and Compound I-25] except that Compound I-21 was used in place of Compound I-20.

### (Compound I-26)

¹H-NMR (500 MHz, CDCl₃, δ): 5.83-5.77 (m, 1H), 5.76-5.65 (m, 2H), 5.43-5.33 (m, 1H), 5.33-5.28 (m, 1H), 4.96 (s, 1H), 4.75-4.61 (m, 3H), 4.20 (t, J = 4.3 Hz, 2H), 4.00 (br s, 1H), 3.91-3.79 (m, 2H), 3.75-3.61 (m, 1H), 3.18 (dd, J = 9.2, 1.7 Hz, 1H), 2.77 (dd, J = 12.0, 3.4 Hz, 1H), 2.67-2.57 (m, 1H), 2.55-2.46 (m, 1H), 2.36-2.24 (m, 2H), 1.94-1.80 (m, 3H), 1.75-1.69 (m, 1H) , 1.68-1.45 (m, 12H), 1.44-1.37 (m, 2H), 1.20-1.13 (m, 6H), 0.95 (t, J = 7.5 Hz, 3H), 0.86-0.77 (m, 7H); MSm/z 646.3956 [M+H]⁺.

### (Compound I-27)

¹H-NMR (500 MHz, CDCl₃, δ): 5.84 (dt, J = 10.3, 2.3 Hz, 1H), 5.78-5.63 (m, 2H), 5.46-5.35 (m, 1H), 4.97 (s, 1H), 4.94 (d, J = 9.7 Hz, 1H), 4.81 (d, J = 3.4 Hz, 1H), 4.71-4.61 (m, 3H), 4.20 (t, J = 4.3 Hz, 2H), 3.95 (br s, 1H), 3.90-3.80 (m, 3H), 3.71-3.63 (m, 1H), 3.60-3.53 (m, 1H), 3.48 (s, 3H), 3.21 (d, J = 7.5 Hz, 1H), 3.16 (t, J = 9.2 Hz, 1H), 2.79 (dd, J = 12.0, 3.4 Hz, 1H), 2.67-2.54 (m, 2H), 2.52-2.46 (m, 1H) , 2.37-2.20 (m, 4H), 1.95-1.81 (m, 3H), 1.79-1.70 (m, 1H), 1.70-1.64 (m, 1H), 1.59-1.34 (m, 13H), 1.28-1.22 (m, 4H), 1.16 (d, J = 13.2 Hz, 3H), 0.95-0.89 (m, 3H), 0.86-0.75 (m, 7H); MSm/z 790.4737 [M+H]⁺.

### Production of Compound I-28

Compound I-1 (30 mg, 0.034 mmol) was dissolved in DMF (0.67 mL), and potassium carbonate (9.32 mg, 0.067 mmol) and iodoethane (10.8 µL, 0.135 mmol) were sequentially added at room temperature, followed by stirring at 70°C for 4 hours. Potassium carbonate (9.32 mg, 0.067 mmol) and iodoethane (10.8 µL, 0.135 mmol) were added to the reaction solution at room temperature, and the mixture was stirred at 70°C for 48 hours. An aqueous solution of ammonium chloride was added to the reaction solution, the mixture was extracted with ethyl acetate, and the organic layer was dried over anhydrous magnesium sulfate. After the solvent was distilled off under reduced pressure, the residue was purified by silica gel column chromatography (n-hexane/ethyl acetate system) to obtain Compound I-28 (isolated yield 10.7 mg, percent yield 35%) as a mixture of isomers that were difficult to separate. The ¹H-NMR spectrum below is the analytical data of the main isomer.
¹H-NMR (500 MHz, CDCl₃, δ): 5.86 (dt, J = 10.2, 2.4 Hz, 1H), 5.78-5.65 (m, 2H), 5.46-5.38 (m, 2H), 4.95 (d, J = 10.9 Hz, 1H), 4.77 (d, J = 3.4 Hz, 1H), 4.69-4.55 (m, 3H), 4.19-4.12 (m, 2H), 3.98 (s, 1H), 3.93 (br s, 1H), 3.86-3.74 (m, 2H), 3.72-3.59 (m, 2H), 3.54-3.45 (m, 2H), 3.44 (s, 3H), 3.43 (s, 3H), 3.27-3.19 (m, 2H), 3.17 (t, J = 9.2 Hz, 1H), 2.93-2.87 (m, 1H), 2.53-2.45 (m, 2H), 2.38-2.19 (m, 5H), 1.93-1.87 (m, 1H), 1.80-1.72 (m, 1H), 1.67 (d, J = 12.6 Hz, 1H), 1.61-1.38 (m, 12H), 1.33-1.18 (m, 14H), 1.16 (d, J = 6.9 Hz, 3H), 0.92 (t, J = 7.5 Hz, 3H), 0.85-0.76 (m, 7H); MSm/z 935.5800 [M+NH₄]⁺.

### Production of Compound I-29

Compound I-29 (isolated yield 12.2 mg, percent yield 39%) was obtained in a similar manner to [Production of Compound I-28] except that Compound I-2 was used in place of Compound I-1.
¹H-NMR (500 MHz, CDCl₃, δ): 5.84 (dt, J = 10.3, 2.3 Hz, 1H), 5.77-5.65 (m, 2H), 5.45-5.36 (m, 2H), 4.98-4.92 (m, 2H), 4.77 (d, J = 3.4 Hz, 1H), 4.66 (d, J = 2.9 Hz, 2H), 4.62 (s, 1H), 4.18-4.09 (m, 2H), 3.93 (br s, 1H), 3.87-3.72 (m, 2H), 3.72-3.60 (m, 2H), 3.53-3.45 (m, 2H), 3.44 (s, 3H), 3.44-3.41 (m, 3H), 3.27-3.19 (m, 2H), 3.17 (t, J = 9.2 Hz, 1H), 2.79 (dd, J = 12,6, 2.9 Hz, 1H), 2.65-2.55 (m, 1H), 2.55-2.45 (m, 2H), 2.37-2.18 (m, 4H), 1.95-1.79 (m, 2H), 1.78-1.71 (m, 1H), 1.69-1.64 (m, 1H), 1.62-1.37 (m, 12H), 1.30-1.22 (m, 10H), 1.20-1.14 (m, 6H), 0.92 (t, J = 7.2 Hz, 3H), 0.84 (d, J = 6.9 Hz, 3H), 0.82-0.74 (m, 4H); MSm/z 935.5800 [M+NH₄]⁺.

### Production of Compound I-30

Compound I-30 (isolated yield 7.9 mg, percent yield 25%) was obtained as a mixture of isomers that were difficult to separate in a similar manner to [Production of Compound I-28] except that 1-bromopropane was used in place of iodoethane. The ¹H-NMR spectrum below is the analytical data of the main isomer.
¹H-NMR (500 MHz, CDCl₃, δ): 5.86 (dt, J = 10.3, 2.3 Hz, 1H), 5.77-5.64 (m, 2H), 5.46-5.37 (m, 2H), 4.95 (d, J = 10.9 Hz, 1H), 4.77 (d, J = 3.4 Hz, 1H), 4.68-4.54 (m, 3H), 4.10-4.00 (m, 2H), 3.98 (s, 1H), 3.93 (br s, 1H), 3.87-3.73 (m, 2H), 3.72-3.60 (m, 2H), 3.53-3.45 (m, 1H), 3.45 (s, 3H), 3.43 (s, 3H), 3.27-3.20 (m, 2H), 3.17 (t, J = 9.2 Hz, 1H), 2.93-2.87 (m, 1H), 2.53-2.46 (m, 2H), 2.37-2.30 (m, 2H), 2.30-2.17 (m, 3H), 1.93-1.87 (m, 1H), 1.79-1.73 (m, 1H), 1.71-1.63 (m, 3H), 1.61-1.35 (m, 12H), 1.32-1.23 (m, 7H), 1.23-1.18 (m, 5H), 1.18-1.14 (m, 3H), 0.96-0.89 (m, 6H), 0.87-0.80 (m, 4H), 0.78 (d, J = 6.3 Hz, 3H); MSm/z 949.5981 [M+NH₄]⁺.

### Production of Compound I-31

Compound I-31 (isolated yield 9.6 mg, percent yield 31%) was obtained in a similar manner to [Production of Compound I-28] except that Compound I-2 was used in place of Compound I-1 and 1-bromopropane was used in place of iodoethane.
¹H-NMR (500 MHz, CDCl₃, δ): 5.86-5.80 (m, 1H), 5.76-5.64 (m, 2H), 5.44-5.36 (m, 2H), 4.98-4.93 (m, 2H), 4.77 (d, J = 3.4 Hz, 1H), 4.66 (d, J = 2.3 Hz, 2H), 4.60 (s, 1H), 4.03 (t, J = 6.9 Hz, 2H), 3.93 (br s, 1H), 3.86-3.73 (m, 2H), 3.71-3.60 (m, 2H), 3.52-3.45 (m, 1H), 3.44 (s, 3H), 3.43 (s, 3H), 3.26-3.19 (m, 2H), 3.17 (t, J = 9.2 Hz, 1H), 2.79 (dd, J = 12.6, 2.9 Hz, 1H), 2.63-2.55 (m, 1H), 2.54-2.45 (m, 2H), 2.38-2.18 (m, 4H), 1.94-1.80 (m, 3H), 1.78-1.70 (m, 1H), 1.70-1.61 (m, 3H), 1.59-1.38 (m, 12H), 1.29-1.24 (m, 6H), 1.19-1.13 (m, 6H), 0.94-0.76 (m, 14H); MSm/z 949.5981 [M+NH₄]⁺.

### Production of Compound I-32

Compound I-32 (isolated yield 14.5 mg, percent yield 50%) was obtained as a mixture of isomers that were difficult to separate in a similar manner to [Production of Compound I-1] except that O-(3-methylphenethyl)hydroxylamine was used in place of hydroxylamine hydrochloride.
¹H-NMR (500 MHz, CDCl₃, δ): 7.16 (t, J = 7.1 Hz, 1H), 7.04-6.99 (m, 3H), 5.89-5.82 (m, 1H), 5.78-5.64 (m, 2H), 5.40 (d, J = 3.4 Hz, 2H), 4.99-4.92 (m, 1H), 4.83 (s, 1H), 4.77 (d, J = 3.4 Hz, 1H), 4.70-4.59 (m, 2H), 4.57 (s, 1H), 4.24 (t, J = 7.5 Hz, 2H), 4.14-4.09 (m, 1H), 3.93 (br s, 1H), 3.88-3.72 (m, 2H), 3.71-3.61 (m, 2H), 3.51-3.40 (m, 1H), 3.45 (s, 3H), 3.43 (s, 3H), 3.27-3.19 (m, 2H), 3.17 (t, J = 9.2 Hz, 1H), 2.96-2.84 (m, 4H), 2.49 (m, 2H), 2.39-2.18 (m, 5H), 2.31 (s, 3H), 1.94-1.83 (m, 1H), 1.79-1.72 (m, 1H), 1.69-1.35 (m, 16H), 1.33-1.22 (m, 9H), 1.16 (d, J = 6.9 Hz, 3H), 0.92 (t, J = 7.2 Hz, 3H), 0.88-0.73 (m, 7H); MSm/z 1025.6306 [M+NH₄]⁺.

### Production of Compound I-33

Compound I-33 (isolated yield 21.5 mg, percent yield 56%) was obtained as a mixture of isomers that were difficult to separate in a similar manner to [Production of Compound I-1] except that O-(2-ethylbenzyl)hydroxylamine was used in place of hydroxylamine hydrochloride.
¹H-NMR (500 MHz, CDCl₃, δ): 7.35-7.30 (m, 1H) , 7.24-7.13 (m, 3H), 5.89-5.81 (m, 1H), 5.76-5.63 (m, 2H), 5.46-5.38 (m, 2H), 5.24-5.11 (m, 2H), 4.98-4.93 (m, 1H), 4.88-4.86 (m, 1H), 4.79-4.73 (m, 1H), 4.69-4.52 (m, 3H), 4.01 (s, 1H), 3.95-3.91 (m, 1H), 3.86-3.73 (m, 2H), 3.71-3.59 (m, 2H), 3.57-3.45 (m, 2H), 3.45-3.43 (m, 3H), 3.43-3.41 (m, 3H), 3.37-3.19 (m, 2H), 3.16 (t, J = 8.9 Hz, 1H), 2.93-2.82 (m, 1H) , 2.73-2.63 (m, 2H), 2.54-2.45 (m, 2H), 2.38-2.19 (m, 5H), 1.93-1.86 (m, 1H), 1.78-1.73 (m, 1H), 1.69-1.66 (m, 1H), 1.62-1.35 (m, 8H), 1.30-1.24 (m, 11H), 1.23-1.18 (m, 5H), 1.15 (dd, J = 10.3, 6.9 Hz, 3H), 0.92 (t, J = 7.5 Hz, 3H), 0.83 (dd, J = 6.9, 1.7 Hz, 3H), 0.75-0.82 (m, 4H); MSm/z 1025.6304 [M+NH₄]⁺.

### Production of Compound I-34

Compound I-34 (isolated yield 27.6 mg, percent yield 68%) was obtained in a similar manner to [Production of Compound I-2] except that O-(3-fluorophenethyl)hydroxylamine was used in place of hydroxylamine hydrochloride.
¹H-NMR (500 MHz, CDCl₃, δ): 7.21 (dt, J = 7.9, 6.0 Hz, 1H), 6.96 (d, J = 7.4 Hz, 1H), 6.93-6.85 (m, 2H), 5.83 (dt, J = 10.3, 2.3 Hz, 1H) , 5.76-5.65 (m, 2H), 5.43-5.36 (m, 2H), 4.95 (d, J = 9.7 Hz, 1H) , 4.91 (s, 1H), 4.76 (d, J = 3.4 Hz, 1H) , 4.69-4.61 (m, 2H), 4.58 (s, 1H), 4.27 (t, J = 6.9 Hz, 2H), 4.14-4.11 (m, 1H), 4.11-4.09 (m, 1H), 3.93 (br s, 1H), 3.86-3.80 (m, 1H), 3.80-3.73 (m, 1H), 3.70-3.60 (m, 2H), 3.51-3.45 (m, 1H), 3.44 (s, 3H), 3.42 (s, 3H), 3.45-3.41 (m, 1H), 3.26-3.19 (m, 2H), 3.16 (t, J = 9.2 Hz, 1H), 2.97-2.92 (m, 2H), 2.78 (dd, J = 12.6, 3.4 Hz, 1H), 2.64-2.56 (m, 1H), 2.54-2.45 (m, 2H), 2.37-2.19 (m, 5H), 1.91-1.79 (m, 1H), 1.77-1.72 (m, 1H), 1.68-1.59 (m, 1H), 1.58-1.36 (m, 9H), 1.29-1.23 (m, 8H), 1.16 (dd, J = 6.6, 1.4 Hz, 6H), 0.92 (t, J = 7.4 Hz, 3H), 0.83 (d, J = 6.3 Hz, 3H), 0.82-0.74 (m, 4H); MSm/z 1029.6066 [M+NH₄]⁺.

### Production of Compound I-35

Compound I-35 (isolated yield 26.1 mg, percent yield 66%) was obtained as a mixture of isomers that were difficult to separate in a similar manner to [Production of Compound I-1] except that O-(3-methylbenzyl) hydroxylamine was used in place of hydroxylamine hydrochloride.
¹H-NMR (500 MHz, CDCl₃, δ): 7.25-7.18 (m, 1H), 7.16-7.05 (m, 3H), 5.88-5.82 (m, 1H) , 5.77-5.64 (m, 2H), 5.46-5.37 (m, 2H), 5.15-5.04 (m, 2H), 4.98-4.93 (m, 1H), 4.90 (s, 0.5H), 4.78-4.75 (m, 1H), 4.70-462 (m, 1H), 4.61-4.56 (m, 1H), 4.00 (s, 0.5H), 3.94-3.91 (m, 1H), 3.86-3.79 (m, 1H), 3.79-3.73 (m, 1H), 3.72-3.52 (m, 2H), 3.52-3.45 (m, 1H), 3.44 (s, 3H), 3.42 (s, 3H), 3.26-3.19 (m, 2H), 3.16 (t, J = 9.2 Hz, 1H) , 2.93-2.84 (m, 1H), 2.53-2.45 (m, 1H) , 2.34 (m, 5H) , 2.30-2.19 (m, 3H), 1.93-1.87 (m, 1H), 1.80-1.72 (m, 1H), 1.70-1.64 (m, 1H), 1.64-1.35 (m, 12H), 1.33-1.20 (m, 12H), 1.15 (t, J = 7.4 Hz, 3H), 0.92 (t, J = 7.2 Hz, 3H), 0.90-0.75 (m, 9H) ; MSm/z 1011.6149 [M+NH₄]⁺.

### Production of Compound I-36

Compound I-36 (isolated yield 14.0 mg, percent yield 35%) was obtained in a similar manner to [Production of Compound I-2] except that O-(3-methylbenzyl)hydroxylamine was used in place of hydroxylamine hydrochloride.
¹H-NMR (500 MHz, CDCl₃, δ): 7.20 (t, J = 7.2 Hz, 1H), 7.16-7.11 (m, 2H), 7.08 (d, J = 7.4 Hz, 1H), 5.84-5.79 (m, 1H), 5.75-5.63 (m, 2H), 5.40 (d, J = 4.0 Hz, 2H), 5.09 (s, 2H), 4.99 (s, 1H) , 4.97-4.91 (m, 1H), 4.78-4.73 (m, 1H), 4.64 (br s, 2H), 4.58 (s, 1H), 3.92 (br s, 1H), 3.87-3.73 (m, 2H), 3.71-3.58 (m, 2H), 3.51-3.45 (m, 1H), 3.44 (s, 3H), 3.42 (s, 3H), 3.27-3.19 (m, 2H), 3.16 (t, J = 9.2 Hz, 1H), 2.79 (dd, J = 12.9, 3.2 Hz, 1H), 2.66-2.56 (m, 1H), 2.55-2.43 (m, 2H), 2.36-2.33 (m, 2H), 2.33 (s, 3H), 2.27-2.23 (m, 2H), 1.94-1.79 (m, 3H), 1.77-1.70 (m, 1H), 1.68-1.62 (m, 1H), 1.61-1.34 (m, 7H), 1.32-1.20 (m, 9H), 1.16 (dd, J = 14.6, 6.6 Hz, 6H), 0.92 (t, J = 7.4 Hz, 3H), 0.89-0.75 (m, 8H); MSm/z 1011.6145 [M+NH₄]⁺.

### Production of Compound I-37

Compound I-37 (isolated yield 20.5 mg, percent yield 60%) was obtained as a mixture of isomers that were difficult to separate in a similar manner to [Production of Compound I-1] except that O-(3-fluorobenzyl)hydroxylamine was used in place of hydroxylamine hydrochloride.
¹H-NMR (500 MHz, CDCl₃, δ): 7.32-7.26 (m, 1H), 7.13-7.01 (m, 2H), 7.00-6.91 (m, 1H), 5.85 (s, 1H), 5.71 (m, 2H), 5.40 (m, 2H), 5.13 (d, J = 5.7 Hz, 1H) , 5.10 (d, J = 8.0 Hz, 1H), 4.99-4.92 (m, 1H), 4.89 (s, 0.5H), 4.79-4.74 (m, 1H), 4.71-4.63 (m, 1H), 4.62-4.55 (m, 1H), 3.99 (s, 0.5H), 3.94-3.92 (m, 1H), 3.87-3.73 (m, 2H), 3.71-3.53 (m, 2H), 3.51-3.45 (m, 1H), 3.44 (s, 3H), 3.42 (s, 3H), 3.27-3.19 (m, 2H), 3.16 (t, J = 9.2 Hz, 1H), 2.93-2.88 (m, 1H), 2.87-2.75 (m, 1H), 2.53-2.45 (m, 2H), 2.37-2.19 (m, 5H), 1.93-1.79 (m, 2H), 1.79-1.71 (m, 1H) , 1.70-1.34 (m, 15H), 1.32-1.17 (m, 11H), 1.17-1.13 (m, 3H), 0.92 (t, J = 7.2 Hz, 3H), 0.88 (d, J = 14.3 Hz, 1H), 0.85-0.74 (m, 8H); MSm/z 1015.5895 [M+NH₄]⁺.

### Production of Compound I-38

Compound I-38 (isolated yield 17.5 mg, percent yield 51%) was obtained in a similar manner to [Production of Compound I-2] except that O-(3-fluorobenzyl)hydroxylamine was used in place of hydroxylamine hydrochloride.
¹H-NMR (500 MHz, CDCl₃, δ): 7.30-7.23 (m, 1H) , 7.09 (d, J = 8.0 Hz, 1H), 7.07-7.03 (m, 1H), 6.95 (dt, J = 8.3, 2.3 Hz, 1H), 5.83 (td, J = 10.2, 2.4 Hz, 1H), 5.70 (dd, J = 13.7, 9.7 Hz, 2H), 5.40 (d, J = 2.9 Hz, 2H), 5.11 (s, 2H), 4.98 (s, 1H), 4.97-4.91 (m, 1H), 4.78-4.75 (m, 1H), 4.65 (s, 2H), 4.59 (s, 1H), 3.93 (br s, 1H), 3.86-3.73 (m, 2H), 3.71-3.60 (m, 2H), 3.51-3.45 (m, 1H), 3.44 (s, 3H), 3.42 (s, 3H), 3.23 (d, J = 9.2 Hz, 2H), 3.16 (t, J = 9.5 Hz, 1H), 2.79 (dd, J = 12.6, 3.4 Hz, 1H), 2.65-2.57 (m, 1H), 2.48 (br s, 2H), 2.32 (br s, 2H), 2.29-2.19 (m, 2H), 1.89 (d, J = 12.6 Hz, 3H), 1.77-1.72 (m, 1H), 1.69-1.63 (m, 1H), 1.60-1.34 (m, 11H), 1.30-1.23 (m, 7H), 1.15 (dd, J = 6.9, 4.0 Hz, 6H), 0.92 (t, J = 7.4 Hz, 3H), 0.84 (m, 4H), 0.78 (d, J = 5.7 Hz, 4H); MSm/z 1015.5908 [M+NH₄]⁺.

### Production of Compound I-39

Compound I-39 (isolated yield 20.0 mg, percent yield 53%) was obtained in a similar manner to [Production of Compound I-2] except that O-(2-ethylbenzyl) hydroxylamine was used in place of hydroxylamine hydrochloride.
¹H-NMR (500 MHz, CDCl₃, δ): 7.32 (dd, J = 1.1, 7.4 Hz, 1H), 7.23 (dd, J = 7.2, 1.4 Hz, 1H), 7.20-7.17 (m, 1H), 7.17-7.13 (m, 1H), 5.81 (td, J = 10.3, 2.3 Hz, 1H), 5.75-5.63 (m, J = 13.2, 9.7 Hz, 2H), 5.39 (d, J = 4.0 Hz, 2H), 5.21-5.12 (m, 2H), 4.96 (s, 1H) , 4.95 (d, J = 4.0 Hz, 1H), 4.76 (d, J = 3.4 Hz, 1H) , 4.67-4.57 (m, 2H), 4.52 (s, 1H), 3.92 (br s, 1H), 3.86-3.73 (m, 2H), 3.70-3.59 (m, 2H), 3.52-3.45 (m, 1H), 3.44 (s, 3H), 3.42 (s, 3H), 3.26-3.19 (m, 2H), 3.16 (t, J = 9.2 Hz, 1H), 2.78 (dd, J = 12.6, 2.6 Hz, 1H), 2.69 (q, J = 7.4 Hz, 2H), 2.64-2.57 (m, 1H), 2.52-2.43 (m, 2H), 2.37-2.30 (m, 2H), 2.29-2.18 (m, 2H), 1.94-1.85 (m, 2H), 1.84-1.78 (m, 1H), 1.77-1.72 (m, 1H), 1.68-1.62 (m, 1H) , 1.58-1.37 (m, 15H), 1.22-1.22 (m, 1H), 1.31-1.12 (m, 19H), 0.92 (t, J = 7.4 Hz, 3H), 0.90-0.86 (m, 3H), 0.83 (d, J = 6.3 Hz, 3H), 0.82-0.74 (m, 4H); MSm/z 1025.6302 [M+NH₄]⁺.

### Production of Compound I-40

Compound I-40 (isolated yield 23.2 mg, percent yield 75%) was obtained in a similar manner to [Production of Compound I-2] except that O-(3-methylphenethyl) hydroxylamine was used in place of hydroxylamine hydrochloride.
¹H-NMR (500 MHz, CDCl₃, δ): 7.15 (t, J = 7.4 Hz, 1H), 7.05-6.95 (m, 3H), 5.83 (td, J = 10.3, 2.3 Hz, 1H), 4.94 (s, 2H), 5.40 (d, J = 2.9 Hz, 2H), 4.95 (d, J = 10.8 Hz, 1H), 4.94 (s, 1H) , 4.77 (d, J = 3.4 Hz, 1H), 4.69-4.62 (m, 2H), 4.56 (s, 1H), 4.28-4.24 (m, 2H), 4.11 (q, J = 6.9 Hz, 1H), 3.93 (br s, 1H), 3.87-3.73 (m, 2H), 3.71-3.60 (m, 2H), 3.52-3.46 (m, 1H), 3.44 (s, 3H), 3.42 (s, 3H), 3.27-3.19 (m, 2H), 3.16 (t, J = 9.2 Hz, 1H) , 2.96-2.90 (m, 3H), 2.82-2.77 (m, 1H) , 2.65-2.57 (m, 1H), 2.49 (br s, 2H), 2.37-2.19 (m, 5H), 2.31 (s, 3H), 1.95-1.87 (m, 2H), 1.87-1.80 (m, 2H), 1.77-1.72 (m, 1H), 1.69-1.64 (m, 1H), 1.60-1.35 (m, 15H), 1.30-1.24 (m, 9H), 1.17 (dd, J = 9.6, 6.9 Hz, 6H), 0.92 (t, J = 7.4 Hz, 3H), 0.89-0.74 (m, 8H); MSm/z 1025.6306 [M+NH₄]⁺.

### Production of Compound I-41 and I-42

Compound I-41 (isolated yield 8.7 mg, percent yield 23%) and Compound I-42 (isolated yield 8.2 mg, percent yield 22%) were obtained in a similar manner to [Production of Compound I-1] except that O-(3-fluorophenethyl)hydroxylamine was used in place of hydroxylamine hydrochloride.

### (Compound I-41)

¹H-NMR (500 MHz, CDCl₃, δ): 7.24-7.18 (m, 1H), 6.96 (d, J = 8.0 Hz, 1H), 6.94-6.90 (m, 1H), 6.90-6.85 (m, 1H), 5.88-5.83 (m, 1H), 5.78-5.65 (m, 2H), 5.40 (m, 2H), 4.98-4.93 (m, 1H), 4.80 (s, 1H), 4.77 (d, J = 4.0 Hz, 1H), 4.71-4.59 (m, 2H), 4.57 (s, 1H), 4.25 (t, J = 7.2 Hz, 2H), 3.93 (br s, 1H), 3.87-3.74 (m, 2H), 3.72-3.60 (m, 2H), 3.51-3.45 (m, 1H), 3.45 (s, 3H), 3.42 (s, 4H), 3.26-3.20 (m, 2H), 3.16 (t, J = 9.2 Hz, 1H), 2.95 (t, J = 7.2 Hz, 2H), 2.90 (dd, J = 13.2, 2.9 Hz, 1H), 2.87-2.82 (m, 1H), 2.53-2.45 (m, 2H), 2.38-2.19 (m, 5H), 1.93-1.87 (m, 1H), 1.79-1.73 (m, 1H), 1.69-1.60 (m, 2H), 1.49 (s, 8H), 1.30-1.23 (m, 11H), 1.16 (d, J = 6.9 Hz, 4H), 0.92 (t, J = 7.4 Hz, 3H), 0.85-0.75 (m, 8H); MSm/z 1029.6057 [M+NH₄]⁺.

### (Compound I-42)

¹H-NMR (500 MHz, CDCl₃, δ) : 7.23 (dt, J = 7.7, 6.3 Hz, 1H), 6.97 (d, J = 7.4 Hz, 1H), 6.93-6.86 (m, 2H), 5.86 (td, J = 10.3, 2.6 Hz, 1H), 5.77-5.65 (m, 2H), 5.46-5.38 (m, 2H), 4.95 (d, J = 10.9 Hz, 1H), 4.77 (d, J = 2.9 Hz, 1H), 4.68-4.55 (m, 2H), 4.61 (s, 1H), 4.34-4.23 (m, 2H), 3.99 (s, 1H), 3.93 (br s, 1H), 3.87-3.72 (m, 2H), 3.71-3.60 (m, 2H), 3.52-3.45 (m, 1H), 3.45-3.40 (m, 2H), 3.44 (s, 3H), 3.43 (s, 3H), 3.27-3.20 (m, 2H), 3.16 (t, J = 9.2 Hz, 1H), 2.97 (t, J = 6.9 Hz, 2H), 2.89 (dd, J = 13.7, 2.9 Hz, 1H), 2.52-2.45 (m, 2H), 2.37-2.18 (m, 5H), 1.92-1.86 (m, 1H), 1.79-1.73 (m, 1H), 1.70-1.64 (m, 1H), 1.60-1.35 (m, 10H), 1.30-1.23 (m, 8H), 1.16 (d, J = 7.4 Hz, 6H), 0.92 (t, J = 7.4 Hz, 3H), 0.84 (d, J = 6.3 Hz, 3H), 0.82-0.75 (m, 5H); MSm/z 1029.6050 [M+NH₄]⁺.

### Production of Compound I-43 and I-44

Compound I-43 (isolated yield 10.7mg, percent yield 26%) and Compound I-44 (isolated yield 10.2 mg, percent yield 25%) were obtained in a similar manner, except O-(3-bromophenethyl) hydroxylamine (28.5 mg, 0.132 mmol) was used in place of hydroxylamine hydrochloride in [Production of Compound I-1].

### (Compound I-43)

¹H-NMR (500 MHz, CDCl₃, δ): 7.36 (m, 1H), 7.34-7.30 (m, 1H), 7.14-7.10 (m, 2H), 5.86 (td, J = 10.3, 2.6 Hz, 1H), 5.79-5.65 (m, 2H), 5.46-5.37 (m, 2H), 4.99-4.92 (m, 1H) , 4.79 (s, 1H) , 4.77 (d, J = 3.4 Hz, 1H), 4.71-4.61 (m, 2H), 4.55 (s, 1H), 4.29-4.21 (m, 2H), 3.94 (br s, 1H), 3.87-3.73 (m, 2H), 3.71-3.60 (m, 2H), 3.52-3.46 (m, 1H), 3.45 (s, 3H), 3.43 (s, 3H), 3.27-3.19 (m, 2H), 3.16 (t, J = 9.2 Hz, 1H), 2.96-2.81 (m, 4H), 2.53-2.45 (m, 2H), 2.39-2.19 (m, 5H), 1.94-1.87 (m, 1H), 1.79-1.73 (m, 1H), 1.69-1.61 (m, 2H), 1.60-1.36 (m, 10H), 1.33-1.23 (m, 11H), 1.16 (d, J = 6.9 Hz, 3H), 0.92 (t, J = 7.4 Hz, 3H), 0.89-0.86 (m, 1H), 0.84 (d, J = 6.9 Hz, 3H), 0.82-0.75 (m, 4H); MSm/z 1089.5251 [M+NH₄]⁺.

### (Compound I-44)

¹H-NMR (500 MHz, CDCl₃, δ): 7.37-7.35 (m, 1H), 7.33 (td, J = 7.0, 2.2 Hz, 1H), 7.13 (s, 2H), 5.88-5.84 (m, 1H), 5.78-5.66 (m, 2H), 5.40 (m, 2H), 4.99-4.91 (m, 1H), 4.77 (d, J = 3.4 Hz, 1H), 4.68-4.54 (m, 2H), 4.61 (s, 1H), 4.33-4.23 (m, 2H), 3.99 (s, 1H), 3.93 (br s, 1H), 3.86-3.74 (m, 2H), 3.72-3.59 (m, 2H), 3.52-3.41 (m, 2H), 3.44 (s, 3H), 3.42 (s, 3H), 3.27-3.19 (m, 2H), 3.16 (t, J = 9.5 Hz, 1H), 2.94 (dt, J = 6.7, 2.0 Hz, 2H), 2.89 (dd, J = 13.5, 2.6 Hz, 1H), 2.52-2.44 (m, 2H), 2.37-2.19 (m, 6H), 1.93-1.86 (m, 1H), 1.79-1.73 (m, 1H), 1.69-1.63 (m, 1H), 1.59-1.35 (m, 9H), 1.29-1.23 (m, 9H), 1.16 (dd, J = 6.9, 2.9 Hz, 6H), 0.92 (t, J = 7.4 Hz, 3H), 0.90-0.85 (m, 1H), 0.84 (d, J = 6.3 Hz, 3H), 0.82-0.75 (m, 4H); MSm/z 1089.5254 [M+NH₄]⁺.

### Production of Compound I-45

Compound I-45 (isolated yield 28.8 mg, percent yield 71%) was obtained in a similar manner to [Production of Compound I-2] except that O-(3-bromophenethyl)hydroxylamine was used in place of hydroxylamine hydrochloride.
¹H-NMR (500 MHz, CDCl₃, δ): 7.38-7.35 (m, 1H), 7.34-7.29 (m, 1H), 7.15-7.11 (m, 2H), 5.83 (td, J = 10.3, 2.6 Hz, 1H), 5.76-5.65 (m, 2H), 5.44-5.37 (m, 2H), 4.97-4.92 (m, 1H), 4.91 (s, 1H), 4.77 (d, J = 3.4 Hz, 1H) , 4.66 (d, J = 2.3 Hz, 2H), 4.56 (s, 1H), 4.26 (t, J = 6.9 Hz, 2H), 4.19 (t, J = 6.9 Hz, 1H), 3.93 (br s, 1H) , 3.87-3.74 (m, 2H), 3.71-3.60 (m, 2H), 3.52-3.46 (m, 1H), 3.44 (s, 3H), 3.42 (s, 3H), 3.43-3.39 (m, 1H), 3.26-3.19 (m, 2H), 3.16 (t, J = 9.2 Hz, 1H), 2.94-2.90 (m, 3H), 2.79 (dd, J = 12.6, 3.4 Hz, 1H), 2.65-2.57 (m, 1H), 2.48 (m, 2H), 2.37-2.19 (m, 5H), 1.92-1.80 (m, 1H), 1.77-1.72 (m, 1H), 1.68-1.64 (m, 1H), 1.59-1.36 (m, 7H), 1.30-1.24 (m, 9H), 1.15 (dd, J = 6.9, 1.7 Hz, 6H), 0.92 (t, J = 7.2 Hz, 3H), 0.89-0.86 (m, 1H), 0.84 (d, J = 6.9 Hz, 3H), 0.82-0.74 (m, 4H); MSm/z 1089.5244 [M+NH₄]⁺.

### Production of Compound I-46 and Compound I-47

Compound I-46 (isolated yield 11.5 mg, percent yield 30%) and Compound I-47 (isolated yield 15.5 mg, percent yield 41%) were obtained in a similar manner to [Production of Compound I-1] except that 2-(aminooxy) acetic acid 1/2 hydrochloride was used in place of hydroxylamine hydrochloride.

### (Compound I-46)

¹H-NMR (500 MHz, CDCl₃, δ): 5.89-5.82 (m, 1H), 5.77-5.62 (m, 2H), 5.41-5.33 (m, 2H), 4.99-4.91 (m, 1H) , 4.79-4.75 (m, 1H) , 4.68-4.52 (m, 4H), 3.94 (d, J = 9.7 Hz, 2H), 3.86-3.72 (m, 3H), 3.71-3.60 (m, 3H), 3.56 (m, 1H) , 3.51-3.46 (m, 1H), 3.44 (s, 3H), 3.42 (s , 3H), 3.43-3.37 (m, 1H), 3.26-3.19 (m, 2H), 3.16 (t, J = 8.9 Hz, 1H) , 2.89-2.84 (m, 1H), 2.54-2.45 (m, 1H) , 2.38-2.19 (m, 5H), 1.95-1.87 (m, 1H), 1.79-1.71 (m, 1H), 1.69-1.63 (m, 1H), 1.60-1.35 (m, 9H), 1.30-1.19 (m, 11H), 1.15 (d, J = 6.9 Hz, 4H), 0.92 (t, J = 7.2 Hz, 3H), 0.89-0.86 (m, 1H), 0.83 (d, J = 6.9 Hz, 3H), 0.82-0.74 (m, 4H); MSm/z 965.5586 [M+NH₄]⁺.

### (Compound I-47)

¹H-NMR (500 MHz, CDCl₃, δ): 5.89-5.80 (m, 1H) , 5.78-5.64 (m, 2H), 5.42-5.30 (m, 2H), 5.01-4.93 (m, 1H), 4.85-4.74 (m, 2H), 4.68-4.42 (m, 4H), 3.93 (br s, 1H), 3.86-3.72 (m, 2H), 3.69-3.60 (m, 2H), 3.58-3.46 (m, 2H), 3.44 (s, 3H), 3.41 (s, 3H), 3.27-3.19 (m, 2H), 3.16 (t, J = 8.9 Hz, 1H), 2.90-2.75 (m, 1H), 2.54-2.46 (m, 1H), 2.37-2.19 (m, 5H), 2.00 (br s, 1H), 1.95-1.89 (m, 1H), 1.79-1.73 (m, 1H), 1.68-1.61 (m, 1H), 1.60-1.34 (m, 12H), 1.30-1.24 (m, 8H), 1.22-1.18 (m, 3H), 1.17-1.12 (m, 3H), 0.92 (t, J = 7.2 Hz, 3H), 0.89-0.85 (m, 1H), 0.83 (d, J = 6.3 Hz, 3H), 0.80-0.74 (m, 4H); MSm/z 965.5583 [M+NH₄]⁺.

### Production of Compound I-48

Compound I-48 (isolated yield 24.7 mg, percent yield 65%) was obtained in a similar manner to [Production of Compound I-2] except that 2-(aminooxy)acetic acid 1/2 hydrochloride was used in place of hydroxylamine hydrochloride.
¹H-NMR (500 MHz, CDCl₃, δ): 5.88-5.81 (m, 1H), 5.79-5.64 (m, 2H), 5.38 (d, J = 3.4 Hz, 1H), 5.35-5.27 (m, 1H), 5.00-4.92 (m, 2H), 4.77 (d, J = 3.4 Hz, 1H) , 4.64 (br s, 2H), 4.51 (br s, 2H), 3.93 (br s, 1H), 3.86-3.72 (m, 2H), 3.70-3.59 (m, 2H), 3.52-3.45 (m, 1H), 3.43 (s, 3H), 3.41 (s, 1H), 3.40-3.35 (m, 1H), 3.26-3.19 (m, 2H), 3.16 (t, J = 9.2 Hz, 1H), 2.73-2.66 (m, 1H), 2.63-2.47 (m, 2H), 2.37-2.19 (m, 4H), 1.95-1.86 (m, 2H), 1.82-1.71 (m, 2H), 1.67-1.61 (m, 1H), 1.59-1.37 (m, 12H), 1.30-1.23 (m, 8H), 1.15 (d, J = 6.9 Hz, 6H), 0.93 (t, J = 7.2 Hz, 3H), 0.88 (s, 1H), 0.84 (d, J = 6.9 Hz, 3H), 0.82-0.71 (m, 5H); MSm/z 965.5586 [M+NH₄]⁺.

### Reference Example 7

### Production of 2-(aminooxy)-2-methylpropan-1-ol hydrochloride

With reference to the literature of US20120238599A1, 2-(aminooxy)-2-methylpropan-1-ol hydrochloride (isolated yield 898 mg, quantitative) was obtained by a three-step conversion using tert-butyl hydroxycarbamate and ethyl 2-bromo-2-methylpropionate as raw materials.
¹H-NMR (500 MHz, CDCl₃, δ): 3.39 (s, 1H), 3.38 (s, 1H), 1.71-1.66 (m, 1H), 1.19 (s, 6H).

### Production of Compound I-49

Compound I-49 (isolated yield 27.4 mg, percent yield 71%) was obtained as a mixture of isomers that were difficult to separate in a similar manner to [Production of Compound I-1] except that 2-(aminooxy)-2-methylpropan-1-ol hydrochloride obtained in Reference Example 7 was used in place of hydroxylamine hydrochloride.
¹H-NMR (500 MHz, CDCl₃, δ): 5.87-5.84 (m, 1H), 5.77-5.64 (m, 2H), 5.46-5.37 (m, 2H), 4.97-4.91 (m, 1H), 4.85 (s, 1H), 4.76 (d, J = 3.4 Hz, 1H), 4.70-4.55 (m, 3H), 3.94-3.91 (m, 1H), 3.87-3.72 (m, 2H), 3.71-3.58 (m, 4H), 3.45 (s, 2H), 3.44 (s, 3H), 3.42 (s, 3H), 3.27-3.19 (m, 2H), 3.16 (t, J = 9.2 Hz, 1H), 2.89 (dd, J = 3.2,13.5 Hz, 1H), 2.87-2.81 (m, 1H), 2.53-2.45 (m, 2H), 2.37-2.18 (m, 5H), 1.93-1.87 (m, 1H), 1.79-1.73 (m, 1H), 1.69-1.35 (m, 10H), 1.31-1.22 (m, 17H), 1.20 (d, J = 7.4 Hz, 1H), 1.17-1.11 (m, 3H), 0.92 (t, J = 7.2 Hz, 3H), 0.89-0.85 (m, 2H), 0.84 (d, J = 6.3 Hz, 3H), 0.82-0.75 (m, 4H); MSm/z 962.5841 [M+H]⁺.

### Production of Compound I-50

Compound I-50 (isolated yield 24.7 mg, percent yield 64%) was obtained in a similar manner to [Production of Compound I-2] except that 2-(aminooxy)-2-methylpropan-1-ol hydrochloride obtained in Reference Example 7 was used in place of hydroxylamine hydrochloride.
¹H-NMR (500 MHz, CDCl₃, δ): 5.83 (td, J = 10.5, 2.5 Hz, 1H), 5.76-5.64 (m, 2H), 5.43-5.35 (m, 2H), 4.96 (s, 1H), 4.94 (d, J = 9.7 Hz, 1H), 4.76 (d, J = 3.4 Hz, 1H), 4.66 (s, 2H), 3.93 (br s, 1H), 3.86-3.73 (m, 2H), 3.70-3.57 (m, 4H), 3.51-3.46 (m, 1H), 3.44 (s, 3H), 3.42 (s, 3H), 3.26-3.19 (m, 2H), 3.16 (t, J = 9.2 Hz, 1H), 2.79 (dd, J = 11.7, 4.3 Hz, 1H), 2.67-2.58 (m, 1H), 2.54-2.45 (m, 2H), 2.37-2.30 (m, 2H), 2.29-2.19 (m, 2H), 1.92-1.82 (m, 3H), 1.78-1.71 (m, 1H), 1.69-1.63 (m, 1H), 1.59-1.32 (m, 11H), 1.29-1.22 (m, 14H), 1.15 (t, J = 7.2 Hz, 6H), 0.92 (t, J = 7.4 Hz, 3H), 0.89-0.85 (m, 1H), 0.83 (d, J = 6.9 Hz, 3H), 0.81-0.73 (m, 4H); MSm/z 962.5833 [M+H]⁺.

### Production of Compound I-51

To a solution of Compound I-1 (21 mg, 0.024 mmol) obtained in [Production of Compound I-1] in DMF (0.24 mL) were added potassium carbonate (13 mg, 0.094 mmol) and 2-bromoacetamide (7.1 µL, 0.094 mmol), and the mixture was stirred at 50°C overnight. A saturated aqueous sodium bicarbonate solution was added to the reaction solution to stop the reaction. After extraction with ethyl acetate, the organic layer was washed with saturated brine and dried over anhydrous magnesium sulfate. After the solvent was distilled off under reduced pressure, the residue was purified by preparative thin-layer silica gel column chromatography (ethyl acetate) to obtain Compound I-51 (isolated yield 14.5 mg, percent yield 65%) as a mixture of isomers that were difficult to separate. The ¹H-NMR spectrum below is the analytical data of the main isomer.
¹H-NMR (500 MHz, CDCl₃, δ): 6.08 (br s, 1H), 5.86 (td, J = 10.5, 2.5 Hz, 1H), 5.78-5.63 (m, 3H), 5.46-5.40 (m, 1H), 5.39 (d, J = 4.0 Hz, 1H), 4.98-4.92 (m, 1H), 4.76 (d, J = 3.4 Hz, 1H), 4.68-4.63 (m, 1H), 4.60 (d, J = 2.3 Hz, 1H), 4.60-4.55 (m, 2H), 3.97 (s, 1H), 3.93 (br s, 1H), 3.85-3.79 (m, 1H), 3.78-3.72 (m, 1H), 3.70-3.59 (m, 2H), 3.58-3.52 (m, 1H), 3.50-3.45 (m, 1H), 3.44 (s, 3H), 3.41 (s, 3H), 3.27-3.19 (m, 2H), 3.16 (t, J = 9.2 Hz, 1H), 2.90 (dd, J = 13.7, 2.9 Hz, 1H), 2.53-2.45 (m, 1H), 2.37-2.19 (m, 5H), 1.93-1.88 (m, 1H), 1.79-1.72 (m, 1H), 1.69-1.63 (m, 1H), 1.61-1.35 (m, 15H), 1.29-1.23 (m, 10H), 1.15 (d, J = 6.9 Hz, 3H), 0.92 (t, J = 7.4 Hz, 3H), 0.83 (d, J = 6.9 Hz, 3H), 0.82-0.74 (m, 4H); MSm/z 964.5757 [M+NH₄]⁺.

### Production of Compound I-52

Compound I-52 (isolated yield 14.6 mg, percent yield 68%) was obtained in a similar manner to [Production of Compound I-51] except that Compound I-2 was used in place of Compound I-1.
¹H-NMR (500 MHz, CDCl₃, δ): 6.40 (br s, 1H) , 5.86-5.80 (m, 1H), 5.78-5.71 (m, 1H), 5.70-5.60 (m, 2H), 5.43-5.35 (m, 2H), 4.97 (s, 1H), 4.95 (d, J = 10.9 Hz, 1H), 4.76 (d, J = 3.4 Hz, 1H), 4.71-4.63 (m, 2H), 4.58 (d, J = 1.7 Hz, 2H), 3.93 (br s, 1H) , 3.86-3.79 (m, 1H), 3.78-3.71 (m, 1H), 3.70-3.58 (m, 2H), 3.51-3.46 (m, 1H), 3.43 (s, 3H), 3.41 (s, 3H), 3.27-3.19 (m, 2H), 3.16 (t, J = 9.2 Hz, 1H), 2.80-2.73 (m, 1H), 2.68-2.60 (m, 1H), 2.52-2.46 (m, 1H) , 2.37-2.30 (m, 2H), 2.29-2.17 (m, 2H) , 1.93-1.83 (m, 3H), 1.75-1.69 (m, 1H), 1.68-1.62 (m, 1H), 1.59-1.36 (m, 13H), 1.27 (d, J = 6.3 Hz, 3H), 1.26-1.23 (m, 4H), 1.15 (d, J = 6.9 Hz, 6H), 0.92 (t, J = 7.4 Hz, 3H), 0.83 (d, J = 6.9 Hz, 3H), 0.78 (d, J = 5.7 Hz, 4H); MSm/z 964.5746 [M+NH₄]⁺.

### Reference Example 8

### Production of 3-(aminooxy)propan-1-ol hydrochloride

With reference to European Journal of Organic Chemistry (2018), 2018(29), 3920-3927, the target 3-(aminooxy)propan-1-ol hydrochloride (isolated.yield 447 mg, percent yield 87%) was obtained using 2-hydroxyisoindoline-1,3-dione (891 mg, 4.03 mmol) as a raw material.
¹H-NMR (500 MHz, D₂O, δ): 4.06 (t, J = 6.0 Hz, 2H), 3.59 (t, J = 6.3 Hz, 2H), 3.48 (t, J = 6.6 Hz, 2H), 1.83-1.79 (m, 2H).

### Production of Compound I-53

Compound I-53 (isolated yield 9.5 mg, percent yield 25%) was obtained in a similar manner to [Production of Compound I-1] except that 3-(aminooxy)propan-1-ol hydrochloride obtained in Reference Example 8 was used in place of hydroxylamine hydrochloride.
¹H-NMR (500 MHz, CDCl₃, δ): 5.87-5.84 (dt, J = 10.7, 2.4 Hz, 1H), 5.76-5.65 (m, 2H), 5.45-5.40 (m, 2H), 4.95 (d, J = 10.9 Hz, 1H), 4.77-4.76 (m, 2H), 4.69-4.60 (m, 3H), 4.27-4.19 (m, 2H), 3.93 (br s, 1H), 3.86-3.61 (m, 6H), 3.51-3.42 (m, 7H), 3.26-3.15 (m, 3H), 2.88-2.85 (m, 2H), 2.50-2.49 (m, 2H), 2.36-2.20 (m, 4H), 1.94-1.84 (m, 3H), 1.76-1.73 (m, 1H), 1.68-1.36 (m, 17H), 1.28-1.25 (m, 9H), 1.16-1.14 (m, 2H), 0.935-0.756 (m, 10H); MSm/z 970.5552 [M+Na]⁺.

### Production of Compound I-54

Compound I-54 (isolated yield 19.4 mg, percent yield 51%) was obtained in a similar manner to [Production of Compound I-2] except that 3-(aminooxy)propan-1-ol hydrochloride obtained in Reference Example 8 was used in place of hydroxylamine hydrochloride.
¹H-NMR (500 MHz, CDCl₃, δ): 5.84-5.81 (dt, J = 10.3, 2.3 Hz, 1H), 5.75-5.64 (m, 2H), 5.43-5.36 (m, 2H), 4.95 (d, J = 10.9 Hz, 1H), 4.91 (s, 1H), 4.76 (d, J = 2.9 Hz, 1H), 4.68-4.61 (m, 3H), 4.25 (t, J = 5.7 Hz, 2H), 4.18 (t, J = 5.7 Hz, 1H), 3.93 (br s, 1H), 3.85-3.60 (m, 7H), 3.50-3.42 (m, 7H), 3.25-3.14 (m, 3H), 2.79 (dd, J = 12.6, 3.4 Hz, 1H), 2.63-2.57 (m, 1H), 2.51-2.47 (m, 2H), 2.35-2.20 (m, 4H), 1.93-1.81 (m, 9H), 1.74-1.73 (m, 1H), 1.67-1.65 (m, 1H), 1.58-1.36 (m, 12H), 1.28-1.24 (m, 6H), 1.18-1.14 (m, 6H), 0.931-0.739 (m, 10H); MSm/z 970.5623 [M+Na]⁺.

### Reference Example 9

### (1) Production of 2-(2,2-difluoroethoxy)isoindoline-1,3-dione

2-(2,2-Difluoroethoxy)isoindoline-1,3-dione (isolated yield 1.94 g, percent yield 85%) was obtained in a similar manner to the production of Reference Example 1-(2), except that 2,2-difluoroethan-1-ol was used in place of cyclopropylmethanol in the production of Reference Example 1-(2).
¹H-NMR (500 MHz, CDCl₃, δ): 7.89-7.85 (m, 2H), 7.82-7.76 (m, 2H), 6.23 (tt, J = 55.0, 4.3 Hz, 1H), 4.38 (td, J = 12.7, 4.3 Hz, 2H).

### (2) Production of O-(2,2-difluoroethyl)hydroxylamine hydrochloride

2-(2,2-Difluoroethoxy)isoindoline-1,3-dione (1.94 g, 8.54 mmol) obtained in Reference Example 9-(1) was dissolved in methylene chloride (34.2 mL), hydrazine monohydrate (0.415 mL, 8.54 mmol) was added, and the mixture was stirred at room temperature for 14 hours. After filtering the suspension, a 4M hydrogen chloride 1,4-dioxane solution (2.24 mL, 8.97 mmol) was added to the filtrate, and the mixture was stirred at room temperature for 15 minutes. After the solvent was distilled off under reduced pressure, ether was added and the suspension was filtered to obtain O-(2,2-difluoroethyl) hydroxylamine hydrochloride (isolated yield 1.55 g, percent yield 95%).
¹H-NMR (500 MHz, DMSO-d₆, δ): 6.51-6.19 (m, 1H), 4.33-4.16 (m, 2H).

### Production of Compound I-55

Compound I-55 (isolated yield 20.4 mg, percent yield 62%) was obtained as a mixture of isomers that were difficult to separate in a similar manner to [Production of Compound I-1] except that O-(2,2-difluoroethyl)hydroxylamine hydrochloride obtained in Reference Example 9-(2) was used in place of hydroxylamine hydrochloride. The ¹H-NMR spectrum below is the analytical data of the main isomer.
¹H-NMR (500 MHz, CDCl₃, δ): 6.10-5.94 (m, 1H), 5.89-5.82 (m, 1H), 5.79-5.63 (m, 2H), 5.46-5.38 (m, 2H), 4.95 (d, J = 10.9 Hz, 1H), 4.81 (s, 1H), 4.77 (d, J = 3.4 Hz, 1H), 4.71-4.56 (m, 3H), 4.30-4.19 (m, 2H), 3.93 (br s, 1H), 3.86-3.74 (m, 2H), 3.71-3.60 (m, 2H), 3.52-3.45 (m, 1H), 3.44 (s, 3H), 3.43 (s, 3H), 3.26-3.19 (m, 2H), 3.17 (t, J = 9.2 Hz, 1H), 2.92-2.81 (m, 2H), 2.54-2.46 (m, 2H), 2.37-2.20 (m, 5H), 1.90 (dd, J = 12.0, 4.6 Hz, 1H), 1.79-1.71 (m, 1H), 1.69-1.63 (m, 1H), 1.63-1.38 (m, 12H), 1.32-1.21 (m, 11H), 1.16 (d, J = 6.9 Hz, 3H), 0.92 (t, J = 7.2 Hz, 3H), 0.86-0.76 (m, 7H); MSm/z 971.5892 [M+NH₄]⁺.

### Production of Compound I-56

Compound I-56 (isolated yield 30.9 mg, percent yield 94%) was obtained in a similar manner to [Production of Compound I-2] except that O-(2,2-difluoroethyl)hydroxylamine hydrochloride obtained in Reference Example 9-(2) was used in place of hydroxylamine hydrochloride.
¹H-NMR (500 MHz, CDCl₃, δ): 6.12-5.85 (tt, J = 55.0, 4.3 Hz, 1H), 5.83 (dt, J = 10.5, 2.5 Hz, 1H), 5.77-5.65 (m, 2H), 5.44-5.36 (m, 2H), 4.94 (d, J = 11.5 Hz, 1H), 4.91 (s, 1H), 4.76 (d, J = 2.9 Hz, 1H), 4.66 (s, 2H), 4.62 (s, 1H), 4.24 (td, J = 13.3, 4.3 Hz, 2H), 3.93 (br s, 1H), 3.87-3.73 (m, 2H), 3.71-3.59 (m, 2H), 3.52-3.45 (m, 1H), 3.44 (s, 3H), 3.42 (s, 3H), 3.28-3.19 (m, 2H), 3.16 (t, J = 8.9 Hz, 1H) , 2.79 (dd, J = 12.6, 2.9 Hz, 1H), 2.65-2.56 (m, 1H) , 2.56-2.45 (m, 2H), 2.37-2.19 (m, 4H), 1.95-1.81 (m, 3H), 1.78-1.70 (m, 1H), 1.68-1.64 (m, 1H), 1.59-1.37 (m, 12H), 1.29-1.24 (m, 7H), 1.19-1.12 (m, 6H), 0.92 (t, J = 7.5 Hz, 3H), 0.85-0.76 (m, 7H); MSm/z 971.5892 [M+NH₄]⁺.

### Reference Example 10

### (1) Production of 2-(3,3-difluoropropyl)isoindoline-1,3-dione

2-(3,3-Difluoropropyl)isoindoline-1,3-dione (isolated yield 418 mg, percent yield 94%) was obtained in a similar manner to the production of Reference Example 1-(2), except that 3,3-difluoropropan-1-ol was used in place of cyclopropylmethanol in the production of Reference Example 1-(2).
¹H-NMR (500 MHz, CDCl₃, δ): 7.88-7.82 (m, 2H), 7.78-7.73 (m, 2H), 6.27 (tt, J = 55.0, 4.9 Hz1H), 4.38 (t, J = 6.3 Hz, 2H), 2.39-2.27 (m, 2H).

### (2) Production of O-(3,3-difluoropropyl)hydroxylamine hydrochloride

In place of 2-(2,2-difluoroethoxy)isoindoline-1,3-dione in the production of Reference Example 10-(2), 2-(3,3-difluoropropyl)isoindoline-1,3-dione obtained in Reference Example 5-(1) was used, and O-(3,3-difluoropropyl)hydroxylamine hydrochloride (isolated yield 254 mg, percent yield 69%) was obtained in a similar manner to the production of Reference Example 4-(2).
¹H-NMR (500 MHz, DMSO-d₆, δ): 6.16 (tt, J = 55.0, 4.6 Hz1H), 4.17-4.04 (m, 2H), 2.31-2.14 (m, 2H).

### Production of Compound I-57

Compound I-57 (isolated yield 23.1 mg, percent yield 70%) was obtained as a mixture of isomers that were difficult to separate in a similar manner to [Production of Compound I-1] except that O-(3,3-difluoropropyl)hydroxylamine hydrochloride obtained in Reference Example 10-(2) was used in place of hydroxylamine hydrochloride. The ¹H-NMR spectrum below is the analytical data of the main isomer.
¹H-NMR (500 MHz, CDCl₃, δ): 5.93 (t, J = 55.0, 4.9 Hz, 1H), 5.89-5.83 (m, 1H), 5.78-5.64 (m, 2H), 5.47-5.37 (m, 2H), 4.96 (d, J = 11.5 Hz, 1H), 4.78 (s, 1H), 4.77 (d, J = 3.4 Hz, 1H) , 4.71-4.55 (m, 3H), 4.30-4.17 (m, 2H), 3.94 (br s, 1H), 3.87-3.73 (m, 2H), 3.71-3.58 (m, 2H), 3.52-3.45 (m, 1H), 3.44 (s, 3H), 3.42 (s, 3H), 3.27-3.20 (m, 2H), 3.16 (t, J = 9.2 Hz, 1H), 2.92-2.77 (m, 2H), 2.55-2.44 (m, 2H), 2.38-2.13 (m, 7H), 1.90 (dd, J = 12.0, 4.6 Hz, 1H), 1.80-1.71 (m, 1H), 1.71-1.38 (m, 16H), 1.31-1.24 (m, 7H), 1.21 (d, J = 7.5 Hz, 1H), 1.19-1.14 (m, 3H), 0.93 (t, J = 7.2 Hz, 3H), 0.84 (d, J = 6.9 Hz, 3H), 0.82-0.75 (m, 4H); MSm/z 985.6105 [M+NH₄]⁺.

### Production of Compound I-58

Compound I-58 (isolated yield 31.0 mg, percent yield 93%) was obtained in a similar manner to [Production of Compound I-2] except that O-(3,3-difluoropropyl)hydroxylamine hydrochloride obtained in Reference Example 10-(2) was used in place of hydroxylamine hydrochloride.
¹H-NMR (500 MHz, CDCl₃, δ): 5.95 (tt, J = 55.0, 4.9 Hz, 1H), 5.86-5.80 (m, 1H), 5.77-5.63 (m, 2H), 5.45-5.34 (m, 2H), 4.95 (d, J = 10.9 Hz, 1H), 4.89 (s, 1H), 4.77 (d, J = 3.4 Hz, 1H), 4.70-4.62 (m, 2H), 4.61 (s, 1H), 4.23 (t, J = 6.3 Hz, 2H), 3.93 (br s, 1H), 3.87-3.72 (m, 2H), 3.71-3.59 (m, 2H), 3.52-3.44 (m, 1H), 3.44 (s, 3H), 3.42 (s, 3H), 3.26-3.19 (m, 2H), 3.16 (t, J = 9.2 Hz, 1H), 2.79 (dd, J = 12.3, 3.7 Hz, 1H), 2.65-2.56 (m, 1H), 2.54-2.46 (m, 2H), 2.37-2.13 (m, 6H), 1.93-1.81 (m, 3H), 1.77-1.71 (m, 1H), 1.69-1.63 (m, 1H) , 1.61-1.36 (m, 12H), 1.30-1.23 (m, 7H), 1.16 (t, J = 6.3 Hz, 6H), 0.92 (t, J = 7.5 Hz, 3H), 0.84 (d, J = 6.3 Hz, 3H), 0.80-0.74 (m, 4H); MSm/z 985.6105 [M+NH₄]⁺.

### Production of Compound I-59

To a solution of a mixture of Compound I-46 and Compound I-47 (30 mg, 0.0352 mmol) obtained in [Production of Compound I-46 and Compound I-47] in dichloromethane (0.32 mL), DIPEA (0.022 mL, 0.141 mmol), 2M dimethylamine THF solution (63 µL, 0.141 mmol) and HATU (24.0 mg, 0.070 mmol) were added, followed by stirring at room temperature overnight. A saturated aqueous sodium bicarbonate solution was added to the reaction solution to stop the reaction. After extraction with ethyl acetate, the organic layer was washed with saturated brine and dried over anhydrous magnesium sulfate. After the solvent was distilled off under reduced pressure, the residue was purified by preparative thin-layer silica gel column chromatography (n-hexane/ethyl acetate system) to obtain Compound I-59 (isolated yield 24.0 mg, percent yield 78%). The ¹H-NMR spectrum below is the analytical data of the main isomer.
¹H-NMR (500 MHz, CDCl₃, δ): 5.88-5.82 (m, 1H), 5.77-5.64 (m, 2H), 5.46-5.31 (m, 2H), 4.95 (d, J = 10.3 Hz, 1H), 4.82-4.72 (m, 3H), 4.67-4.57 (m, 2H), 3.97-3.95 (m, 1H) , 3.93 (br s, 1H), 3.86-3.73 (m, 2H), 3.71-3.58 (m, 3H), 3.52-3.46 (m, 1H), 3.44 (s, 3H), 3.42 (s, 3H), 3.27-3.13 (m, 3H), 2.97 (dd, J = 17.2, 3.2 Hz, 6H), 2.53-2.44 (m, 2H), 2.37-2.18 (m, 5H), 1.96-1.80 (m, 2H), 1.78-1.72 (m, 1H), 1.70-1.33 (m, 16H), 1.32-1.21 (m, 9H), 1.18-1.11 (m, 3H), 0.96-0.72 (m, 11H); MSm/z 992.6049 [M+NH₄]⁺.

### Production of Compound I-60

To a solution of Compound I-48 (30 mg, 0.0352 mmol) obtained in [Production of Compound I-48] in dichloromethane (0.32 mL), DIPEA (0.016 mL, 0.106 mmol), 2M dimethylamine THF solution (0.048 mL, 0.106 mmol) and HATU (15.6 mg, 0.0458 mmol) were added, followed by stirring at room temperature overnight. A saturated aqueous sodium bicarbonate solution was added to the reaction solution to stop the reaction. After extraction with ethyl acetate, the organic layer was washed with saturated brine and dried over anhydrous magnesium sulfate. After the solvent was distilled off under reduced pressure, the residue was purified by preparative thin-layer silica gel column chromatography (ethyl acetate) to obtain Compound I-60 (isolated yield 26.0 mg, percent yield 84%).
¹H-NMR (500 MHz, CDCl₃, δ): 5.87-5.82 (m, 1H), 5.75-5.64 (m, 2H), 5.41-5.30 (m, 2H), 5.03 (d, J = 1.7 Hz, 1H), 4.98-4.92 (m, 1H), 4.79-4.71 (m, 3H), 4.65 (br s, 2H), 3.92 (br s, 1H), 3.86-3.79 (m, 1H), 3.78-3.72 (m, 1H), 3.70-3.59 (m, 2H), 3.51-3.45 (m, 1H), 3.43 (s, 3H), 3.42 (s, 3H), 3.41-3.38 (m, 1H), 3.26-3.13 (m, 3H), 2.98 (d, J = 2.3 Hz, 3H), 2.92 (d, J = 1.7 Hz, 3H), 2.80-2.73 (m, 1H), 2.65-2.57 (m, 1H), 2.55-2.45 (m, 2H), 2.37-2.18 (m, 5H), 1.96-1.79 (m, 2H), 1.77-1.71 (m, 1H), 1.68-1.60 (m, 1H), 1.59-1.33 (m, 9H), 1.30-1.21 (m, 8H), 1.18-1.10 (m, 6H), 0.94-0.86 (m, 4H), 0.85-0.71 (m, 8H); MSm/z 975.5793 [M+H]⁺.

### Production of Compound I-61

Compound I-61 (isolated yield 28.1 mg, percent yield 87%) was obtained as a mixture of isomers that were difficult to separate in a similar manner to [Production of Compound I-59] except that morpholine was used in place of dimethylamine.
¹H-NMR (500 MHz, CDCl₃, δ): 5.88-5.82 (m, 1H), 5.78-5.63 (m, 2H), 5.46-5.33 (m, 2H), 4.98-4.90 (m, 0.5H), 4.78-4.75 (m, 2H), 4.84-4.52 (m, 5H), 3.97-3.94 (m, 0.5H), 3.92 (br s, 1H), 3.86-3.80 (m, 1H), 3.79-3.73 (m, 1H), 3.71-3.54 (m, 9H), 3.53-3.46 (m, 3H), 3.45-3.43 (m, 3H), 3.43-3.41 (m, 3H), 3.27-3.14 (m, 3H), 2.92-2.86 (m, 1H) , 2.81-2.75 (m, 1H), 2.54-2.44 (m, 2H), 2.37-2.20 (m, 5H), 1.94-1.80 (m, 2H), 1.78-1.73 (m, 1H), 1.68-1.64 (m, 1H), 1.62-1.35 (m, 10H), 1.30-1.22 (m, 11H), 1.18-1.12 (m, 4H), 0.96-0.74 (m, 8H); MSm/z 1034.6150 [M+NH₄]⁺.

### Production of Compound I-62

Compound I-62 (isolated yield 26.9 mg, percent yield 84%) was obtained in a similar manner to [Production of Compound I-60] except that morpholine was used in place of dimethylamine.
¹H-NMR (500 MHz, CDCl₃, δ): 5.86-5.81 (m, 1H), 5.77-5.63 (m, 2H), 5.42-5.33 (m, 2H), 4.98-4.91 (m, 2H), 4.79-4.71 (m, 3H), 4.71-4.59 (m, 3H), 3.93 (br s, 1H), 3.86-3.80 (m, 1H), 3.79-3.72 (m, 1H), 3.70-3.56 (m, 9H), 3.54-3.45 (m, 3H), 3.43 (s, 3H), 3.42 (s, 3H), 3.27-3.12 (m, 3H), 2.81-2.74 (m, 1H), 2.66-2.58 (m, 1H), 2.56-2.43 (m, 2H), 2.37-2.30 (m, 2H), 2.29-2.18 (m, 2H), 1.95-1.81 (m, 3H), 1.78-1.68 (m, 2H), 1.68-1.62 (m, 1H), 1.60-1.34 (m, 9H), 1.30-1.22 (m, 7H), 1.18-1.11 (m, 6H), 0.91 (t, J = 7.2 Hz, 3H), 0.89-0.73 (m, 8H) ; MSm/z 1017.5884 [M+H]⁺.

### Production of Compound I-63

Compound I-63 (isolated yield 13.2 mg, percent yield 43%) was obtained as a mixture of isomers that were difficult to separate in a similar manner to [Production of Compound I-59] except that a 2M methylamine THF solution was used in place of dimethylamine.
¹H-NMR (500 MHz, CDCl₃, δ): 6.12-6.07 (m, 1H), 5.89-5.83 (m, 1H), 5.79-5.64 (m, 2H), 5.46-5.37 (m, 2H), 4.97-4.91 (m, 0.5H), 4.76 (d, J = 3.4 Hz, 1H), 4.74-4.54 (m, 4H), 3.97 (s, 0.5H), 3.93 (br s, 1H) , 3.86-3.79 (m, 1H), 3.79-3.72 (m, 1H), 3.71-3.59 (m, 2H), 3.58-3.51 (m, 1H), 3.44 (s, 3H), 3.42 (s, 3H), 3.27-3.19 (m, 2H), 3.16 (t, J = 9.2 Hz, 1H), 2.93-2.88 (m, 1H), 2.86 (d, J = 5.2 Hz, 2H), 2.81 (d, J = 4.6 Hz, 1H) , 2.55-2.45 (m, 2H), 2.37-2.18 (m, 4H), 1.93-1.85 (m, 1H), 1.78-1.71 (m, 1H), 1.69-1.35 (m, 16H), 1.32-1.22 (m, 10H), 1.17-1.14 (m, 3H), 0.92 (t, J = 7.2 Hz, 3H), 0.89-0.85 (m, 1H), 0.83 (d, J = 6.3 Hz, 3H), 0.82-0.74 (m, 4H); MSm/z 978.5889 [M+NH₄]⁺.

### Production of Compound I-64

Compound I-64 (isolated yield 23.1 mg, percent yield 76%) was obtained in a similar manner to [Production of Compound I-60] except that a 2M methylamine THF solution was used in place of dimethylamine.
¹H-NMR (500 MHz, CDCl₃, δ): 6.44-6.38 (m, 1H), 5.87-5.81 (m, 1H), 5.79-5.64 (m, 2H), 5.45-5.36 (m, 2H), 4.99 (s, 1H), 4.95 (d, J = 10.3 Hz, 1H), 4.82 (s, 1H), 4.76 (d, J = 3.4 Hz, 1H), 4.73-4.65 (m, 2H), 4.64-4.54 (m, 2H), 3.93 (br s, 1H), 3.86-3.79 (m, 1H), 3.79-3.72 (m, 1H), 3.71-3.59 (m, 2H), 3.51-3.45 (m, 1H), 3.44 (s, 3H), 3.42 (s, 3H), 3.23 (m, 2H), 3.16 (t, J = 8.9 Hz, 1H), 2.81 (d, J = 4.6 Hz, 3H), 2.80-2.76 (m, 1H), 2.69-2.61 (m, 1H), 2.54-2.46 (m, 2H), 2.37-2.31 (m, 2H), 2.30-2.18 (m, 2H), 1.92-1.82 (m, 3H), 1.77-1.70 (m, 1H), 1.69-1.35 (m, 11H), 1.30-1.24 (m, 8H), 1.15 (t, J = 6.6 Hz, 6H), 0.92 (t, J = 7.4 Hz, 3H), 0.87 (t, J = 6.6 Hz, 1H), 0.84 (d, J = 6.9 Hz, 3H), 0.81-0.74 (m, 4H); MSm/z 978.5902 [M+NH₄]⁺.

### Production of Compound I-65

Compound I-65 (isolated yield 28.0 mg, percent yield 86%) was obtained in a similar manner to [Production of Compound I-59] except that cyclohexylamine was used in place of dimethylamine. The ¹H-NMR spectrum below is the analytical data of the main isomer.
¹H-NMR (500 MHz, CDCl₃, δ): 5.95 (d, J = 8.0 Hz, 1H), 5.89-5.83 (m, 1H) , 5.79-5.64 (m, 2H), 5.48-5.37 (m, 2H), 4.97-4.92 (m, 1H), 4.79-4.71 (m, 1H), 4.70-4.63 (m, 1H), 4.61-4.51 (m, 3H), 3.97 (s, 1H), 3.95-3.91 (m, 1H), 3.87-3.73 (m, 3H), 3.72-3.59 (m, 2H), 3.58-3.52 (m, 1H), 3.51-3.46 (m, 1H), 3.44 (s, 3H), 3.42 (s, 3H), 3.27-3.20 (m, 2H), 3.16 (t, J = 9.2 Hz, 1H) , 2.94-2.88 (m, 1H), 2.53-2.44 (m, 2H), 2.38-2.17 (m, 4H), 1.93-1.81 (m, 3H), 1.74 (d, J = 4.6 Hz, 1H), 1.79-1.32 (m, 19H), 1.30-1.21 (m, 10H), 1.21-1.11 (m, 7H), 0.92 (t, J = 7.2 Hz, 3H), 0.89-0.86 (m, 1H), 0.84 (d, J = 6.3 Hz, 3H), 0.81-0.74 (m, 4H); MSm/z 1046.6510 [M+NH₄]⁺.

### Production of Compound I-66

Compound I-66 (isolated yield 25.5 mg, percent yield 78%) was obtained in a similar manner to [Production of Compound I-60] except that cyclohexylamine was used in place of dimethylamine.
¹H-NMR (500 MHz, CDCl₃, δ): 6.28 (d, J = 8.0 Hz, 1H), 5.88-5.83 (m, 1H), 5.80-5.65 (m, 2H), 5.46-5.37 (m, 2H), 4.99 (s, 1H), 4.96-4.92 (m, 1H), 4.76 (d, J = 3.4 Hz, 1H), 4.74 (s, 1H), 4.73-4.64 (m, 2H), 4.60-4.51 (m, 2H), 3.93 (br s, 1H), 3.86-3.79 (m, 2H), 3.78-3.73 (m, 1H), 3.71-3.60 (m, 2H), 3.52-3.45 (m, 1H), 3.44 (s, 3H), 3.42 (s, 3H), 3.28-3.19 (m, 2H), 3.16 (t, J = 9.2 Hz, 1H), 2.79 (dd, J = 11.5, 4.6 Hz, 1H), 2.68-2.60 (m, 1H), 2.53-2.45 (m, 2H), 2.36-2.30 (m, 2H), 2.30-2.19 (m, 2H), 1.92-1.81 (m, 5H), 1.68 (m, 5H), 1.63-1.30 (m, 10H), 1.29-1.23 (m, 9H), 1.21-1.09 (m, 10H), 0.92 (t, J = 7.2 Hz, 3H), 0.87 (t, J = 6.9 Hz, 1H) , 0.85-0.81 (m, 3H), 0.82-0.74 (m, 4H); MSm/z 1029.6263 [M+H]⁺.

### Reference Example 11

O-(4-((tertButyldimethylsilyl)oxy)butyl)hydroxylamine (isolated yield 966 mg, three-step percent yield 41%) was obtained in a similar manner to the three steps of Reference Examples 6-(1), (2) and (3), except that 4-bromobutan-1-ol was used in place of 2-bromoethan-1-ol in Reference Example 6-(1).
¹H-NMR (500 MHz, DMSO-d₆, δ): 5.84 (br s, 1H), 3.55 (t, J = 6.0 Hz, 2H), 3.48 (t, J = 6.6 Hz, 2H), 1.50-1.38 (m, 4H), 0.823 (s, 9H), 0.823 (s, 6H).

### Production of Compound I-67

Compound I-67 (isolated yield 30.6 mg, percent yield 71%) was obtained in a similar manner to [Production of Compound I-1] except that O-(4-((tertbutyldimethylsilyl)oxy)butyl)hydroxylamine obtained in Reference Example 11-(3) was used in place of hydroxylamine hydrochloride.
¹H-NMR (500 MHz, CDCl₃, δ): 5.86-5.84 (m, 1H), 5.75-5.65 (m, 2H), 5.44-5.38 (m, 2H), 4.95 (d, J = 10.9 Hz, 1H), 4.76 (d, J = 3.4 Hz, 1H), 4.69-4.55 (m, 3H), 4.13-3.93 (m, 4H), 3.85-3.73 (m, 2H), 3.69-3.58 (m, 5H), 3.52-3.42 (m, 8H), 3.25-3.14 (m, 3H), 2.91 (dt, J = 13.6, 3.0 Hz, 1H), 2.53-2.47 (m, 2H), 2.35-2.20 (m, 5H), 1.91-1.88 (m, 1H) , 1.76-1.36 (m, 24H), 1.28-1.14 (m, 13H), 0.930-0.742 (m, 25H), 0.034-0.022 (m, 8H); MSm/z 1076.6895 [M+H] ⁺.

### Production of Compound I-68

Compound I-67 (30.6 mg, 0.028 mmol) obtained in [Production of Compound I-67] was dissolved in THF (1.12 mL), a hydrogen fluoride pyridine solution (0.378 mL) was added, and the mixture was stirred at room temperature for 10 minutes. A saturated aqueous sodium hydrogen carbonate solution was added to the reaction solution, and the mixture was extracted with ethyl acetate. The organic layer was washed with saturated brine and dried over anhydrous magnesium sulfate. After the solvent was distilled off under reduced pressure, the residue was purified by preparative thin-layer chromatography (n-hexane/ethyl acetate = 1:2) to obtain Compound I-68 (isolated yield 13.1 mg, percent yield 49%).
¹H-NMR (500 MHz, CDCl₃, δ) : 5.86-5.84 (m, 1H) , 5.75-5.65 (m, 2H), 5.44-5.39 (m, 2H), 4.95 (d, J = 10.9 Hz, 1H) , 4.76 (d, J = 3.4 Hz, 1H), 4.66-4.56 (m, 3H), 4.16-4.09 (m, 2H), 3.97 (s, 1H) , 3.93 (br s, 1H) , 3.85-3.73 (m, 2H), 3.69-3.60 (m, 4H), 3.50-3.42 (m, 8H), 3.25-3.14 (m, 3H), 2.90 (dd, J = 13.5, 2.6 Hz, 1H), 2.53-2.47 (m, 2H), 2.35-2.20 (m, 4H), 1.91 (dd, J = 12.3, 4.9 Hz, 1H), 1.77-1.36 (m, 24H), 1.28-1.14 (m, 13H), 0.931-0.771 (m, 11H); MSm/z 962.5991 [M+H]⁺.

### Production of Compound I-69

Compound I-69 (isolated yield 25.2 mg, percent yield 59%) was obtained in a similar manner to [Production of Compound I-2] except that O-(4-((tertbutyldimethylsilyl)oxy)butyl)hydroxylamine obtained in Reference Example 11-(3) was used in place of hydroxylamine hydrochloride.
¹H-NMR (500 MHz, CDCl₃, δ): 5.84-5.81 (m, 1H) , 5.74-5.65 (m, 2H), 5.42-5.36 (m, 2H), 4.94-4.93 (m, 2H), 4.76 (d, J = 3.4 Hz, 1H), 4.65 (d, J = 1.1 Hz, 1H), 4.59 (s, 1H), 4.09 (t, J = 6.6 Hz, 2H), 3.92 (br s, 1H), 3.85-3.74 (m, 2H), 3.68-3.59 (m, 4H), 3.50-3.42 (m, 7H), 3.25-3.14 (m, 3H), 2.80 (dd, J = 12.6, 3.4 Hz, 1H) , 2.62-2.55 (m, 1H), 2.51-2.47 (m, 2H), 2.36-2.20 (m, 4H), 1.92-1.79 (m, 3H), 1.75-1.34 (m, 21H), 1.28-1.14 (m, 12H), 0.929-0.769 (m, 21H), 0.026 (s, 6H); MSm/z 1093.7158 [M+NH₄].

### Production of Compound I-70

Compound I-69 (25.2 mg, 0.023 mmol) obtained in [Production of Compound I-69] was dissolved in THF (0.920 mL), a hydrogen fluoride pyridine solution (0.311 mL) was added, and the mixture was stirred at room temperature for 10 minutes. A saturated aqueous sodium hydrogen carbonate solution was added to the reaction solution, and the mixture was extracted with ethyl acetate. The organic layer was washed with saturated brine and dried over anhydrous magnesium sulfate. After the solvent was distilled off under reduced pressure, the residue was purified by preparative thin-layer chromatography (n-hexane/ethyl acetate = 1:2) to obtain Compound I-70 (isolated yield 15.8 mg, percent yield 71%).
¹H-NMR (500 MHz, CDCl₃, δ): 5.84 (dd, J = 8.0, 2.3 Hz, 1H), 5.76-5.65 (m, 2H), 5.43-5.36 (m, 2H), 4.95-4.93 (m, 2H), 4.77 (d, J = 3.4 Hz, 1H), 4.66-4.62 (m, 3H), 4.17-4.10 (m, 2H), 3.93 (br s, 1H), 3.86-3.75 (m, 2H), 3.69-3.61 (m, 4H), 3.51-3.43 (m, 7H), 3.28-3.15 (m, 3H), 2.80 (dd, J = 12.3, 3.2 Hz, 1H), 2.62-2.57 (m, 1H), 2.51-2.48 (m, 2H), 2.36-2.20 (m, 4H), 1.93-1.80 (m, 3H), 1.77-1.36 (m, 22H), 1.28-1.15 (m, 12H), 0.935-0.776 (m, 10H); MSm/z 984.5617 [M+Na]⁺.

### Reference Example 12

### (1) Production of 2-(2-(2-((tertbutyldimethylsilyl)oxy)ethoxy)ethoxy)isoindoline-1,3-dione

According to the method described in International Publication No. WO 2013/186632, 2-(2-(2-((tertbutyldimethylsilyl)oxy)ethoxy)ethoxy)isoindoline-1,3-dione (isolated yield 340 mg, percent yield 36%) was obtained by a three-step conversion using 2,2'-oxybis(ethan-1-ol) (1.73 mL, 18.3 mmol) as a raw material.
¹H-NMR (500 MHz, CDCl₃, δ): 7.85-7.82 (m, 2H), 7.76-7.73 (m, 2H), 4.37-4.36 (m, 2H), 3.88-3.86 (m, 2H), 3.68 (t, J = 5.2 Hz, 2H), 3.56 (t, J = 5.2 Hz, 2H), 0.85 (s, 9H), 0.01 (s, 6H).

### (2) Production of 2-(2-((tertbutyldimethylsilyl)oxy)ethoxy)ethan-1-ol

2-(2-(2-((tertButyldimethylsilyl)oxy)ethoxy)ethoxy)isoindoline-1,3-dione (292 mg, 1.16 mmol) obtained in Reference Example 12-(1) was dissolved in ethanol (11.6 mL, 11.6 mmol). To the solution, hydrazine monohydrate (0.062 mL, 1.28 mmol) was added, followed by heating under reflux, and stirring for 0.5 hours. After completion of the reaction, the mixture was filtered, and the solvent was distilled off under reduced pressure, and then 2-(2-((tert-butyldimethylsilyl)oxy) ethoxy) ethan-1-ol(isolated yield 185 mg, percent yield 68%) was obtained.
¹H-NMR (500 MHz, DMSO-d₆, δ): 5.97 (s, 2H), 3.69 (t, J = 5.2 Hz, 2H), 3.63-3.61 (m, 2H), 3.54-3.53 (m, 2H), 3.45 (t, J = 5.2 Hz, 2H), 0.86 (s, 9H), 0.04 (s, 6H).

### Production of Compound I-71

Compound I-71 (isolated yield 4.1 mg, percent yield 14%) was obtained in a similar manner to [Production of Compound I-67] and [Production of Compound I-68], except that 2-(2-((tertbutyldimethylsilyl)oxy)ethoxy)ethan-1-ol obtained in Reference Example 12-(2) was used in place of O-(4-((tert-butyldimethylsilyl)oxy)butyl)hydroxylamine in [Production of Compound I-67].
¹H-NMR (500 MHz, CDCl₃, δ): 5.87-5.85 (m, 1H), 5.76-5.66 (m, 2H), 5.45-5.39 (m, 2H), 4.96 (d, J = 9.2 Hz, 1H), 4.77 (d, J = 4.0 Hz, 1H), 4.68-4.57 (m, 3H), 4.31-4.24 (m, 2H), 3.98 (s, 1H) , 3.93 (br s, 1H), 3.86-3.58 (m, 19H), 3.26-3.15 (m, 3H), 2.91 (dd, J = 13.2, 2.9 Hz, 1H), 2.51-2.49 (m, 2H), 2.36-2.18 (m, 5H), 1.92 (dd, J = 11.7, 3.7 Hz, 1H), 1.77-1.74 (m, 1H), 1.68-1.66 (m, 1H), 1.58-1.37 (m, 21H), 1.29-1.15 (m, 12H), 0.94 (t, J = 7.2 Hz, 3H), 0.85-0.80 (m, 7H); MSm/z 978.5771 [M+H]⁺.

### Production of Compound I-72

Compound I-72 (isolated yield 12.0 mg, percent yield 42%) was obtained in a similar manner to [Production of Compound I-69] and [Production of Compound I-70], except that 2-(2-((tertbutyldimethylsilyl)oxy)ethoxy)ethan-1-ol obtained in Reference Example 12-(2) was used in place of O-(4-((tert-butyldimethylsilyl)oxy)butyl)hydroxylamine in [Production of Compound I-69].
¹H-NMR (500 MHz, CDCl₃, δ): 5.84 (dd, J = 8.0, 2.3 Hz, 1H), 5.76-5.65 (m, 2H), 5.41-5.34 (m, 2H), 4.97-4.93 (m, 2H), 4.77 (d, J = 3.4 Hz, 1H), 4.68-4.65 (m, 3H), 4.26-4.24 (m, 2H), 3.93 (br s, 1H), 3.86-3.54 (m, 11H), 3.51-3.43 (m, 8H), 3.26-3.15 (m, 3H), 2.80 (dd, J = 12.6, 3.4 Hz, 1H), 2.64-2.47 (m, 4H), 2.36-2.20 (m, 4H), 1.94-1.82 (m, 3H), 1.75-1.73 (m, 1H), 1.66 (s, 3H), 1.58-1.34 (m, 13H), 1.28-1.15 (m, 13H), 0.93-0.78 (m, 11H); MSm/z 995.6039 [M+NH₄]⁺.

### Production of Compound I-73

Compound I-73 (isolated yield 11.7 mg, percent yield 37%) was obtained in a similar manner to [Production of Compound I-59] except that 2-aminoethan-1-ol was used in place of dimethylamine.
¹H-NMR (500 MHz, CDCl₃, δ): 6.52 (t, J = 5.7 Hz, 1H), 5.90-5.81 (m, 1H), 5.79-5.62 (m, 2H), 5.45-5.35 (m, 2H), 4.99-4.90 (m, 1H), 4.86-4.74 (m, 2H), 4.71-4.62 (m, 2H), 4.62-4.56 (m, 2H), 3.97 (s, 1H), 3.93 (br s, 1H), 3.86-3.79 (m, 1H), 3.79-3.73 (m, 1H), 3.72-3.59 (m, 4H), 3.59-3.54 (m, 1H), 3.52-3.45 (m, 2H), 3.44 (s, 3H), 3.42 (s, 3H), 3.27-3.19 (m, 2H), 3.16 (t, J = 8.9 Hz, 1H), 2.92-2.84 (m, 2H), 2.57-2.46 (m, 1H), 2.37-2.30 (m, 2H), 2.30-2.17 (m, 2H), 1.93-1.87 (m, 1H), 1.78-1.69 (m, 1H), 1.68-1.64 (m, 1H), 1.63-1.34 (m, 14H), 1.33-1.21 (m, 11H), 1.15 (d, J = 6.9 Hz, 3H), 0.92 (t, J = 7.2 Hz, 3H), 0.89-0.85 (m, 1H), 0.83 (d, J = 6.9 Hz, 3H), 0.82-0.74 (m, 3H); MSm/z 1008.6011 [M+NH₄] ⁺.

### Production of Compound I-74

Compound I-74 (isolated yield 13.5 mg, percent yield 43%) was obtained in a similar manner to [Production of Compound I-60] except that 2-aminoethan-1-ol was used in place of dimethylamine.
¹H-NMR (500 MHz, CDCl₃, δ): 6.82 (t, J = 5.4 Hz, 1H), 5.85 (d, J = 9.7 Hz, 1H), 5.79-5.64 (m, 2H), 5.43-5.34 (m, 2H), 5.00 (s, 1H), 4.99-4.90 (m, 2H), 4.76 (d, J = 4.0 Hz, 1H), 4.73-4.54 (m, 4H), 3.93 (br s, 1H), 3.86-3.79 (m, 1H), 3.79-3.74 (m, 1H), 3.72-3.60 (m, 4H), 3.52-3.34 (m, 3H), 3.43 (s, 3H), 3.41 (s, 3H), 3.26-3.19 (m, 2H), 3.16 (t, J = 8.9 Hz, 1H), 2.87-2.85 (m, 1H), 2.82-2.75 (m, 3H), 2.70-2.63 (m, 1H), 2.54-2.45 (m, 1H), 2.37-2.30 (m, 2H), 2.29-2.18 (m, 2H), 1.93-1.82 (m, 3H), 1.76-1.69 (m, 1H), 1.68-1.62 (m, 1H), 1.59-1.35 (m, 5H), 1.33-1.21 (m, 10H), 1.18-1.11 (m, 6H), 0.92 (t, J = 7.2 Hz, 3H), 0.87 (t, J = 6.3 Hz, 2H), 0.83 (d, J = 6.3 Hz, 3H), 0.80-0.74 (m, 4H); MSm/z 991.5743 [M+H]⁺.

### Production of Compound I-75

To a solution of Compound I-22 (30 mg, 0.0321 mmol) obtained in [Production of Compound I-22] in dichloromethane (0.32 mL), 37% formalin (23.8 µL, 0.32 mmol) and sodium cyanoborohydride (16.2 mg, 0.252 mmol) were sequentially added, followed by stirring at room temperature overnight. A saturated aqueous sodium bicarbonate solution was added to the reaction solution to stop the reaction. After extraction with ethyl acetate, the organic layer was washed with saturated brine and dried over anhydrous magnesium sulfate. After the solvent was distilled off under reduced pressure, the residue was purified by preparative thin-layer chromatography (chloroform:methanol = 10:1) to obtain Compound I-75 (isolated yield 6.5 mg, percent yield 21%) as a mixture of isomers that were difficult to separate.
¹H-NMR (500 MHz, CDCl₃, δ): 5.88-5.82 (m, 1H), 5.76-5.64 (m, 2H), 5.44-5.36 (m, 2H), 4.97-4.92 (m, 1H), 4.83 (s, 0.67H), 4.78-4.75 (m, 1H), 4.70-4.54 (m, 2H), 4.26-4.15 (m, 2H), 3.97 (s, 0.33H), 3.93 (br s, 1H), 3.86-3.79 (m, 1H), 3.79-3.73 (m, 1H), 3.70-3.60 (m, 2H), 3.53-3.46 (m, 1H), 3.45 (s, 3H), 3.42 (s, 3H), 3.26-3.19 (m, 2H), 3.16 (t, J = 8.9 Hz, 1H), 2.91-2.81 (m, 2H), 2.71-2.59 (m, 2H), 2.52-2.45 (m, 1H), 2.30 (s, 6H), 2.38-2.18 (m, 6H), 1.94-1.87 (m, 1H), 1.78-1.73 (m, 1H), 1.69-1.60 (m, 2H), 1.59-1.35 (m, 12H), 1.27 (d, J = 6.9 Hz, 5H), 1.25 (d, J = 6.3 Hz, 4H), 1.20 (d, J = 6.9 Hz, 1H), 1.15 (d, J = 6.9 Hz, 3H), 0.92 (t, J = 7.4 Hz, 3H), 0.89-0.85 (m, 1H), 0.83 (d, J = 6.3 Hz, 3H), 0.81-0.75 (m, 4H); MSm/z 961.6001 [M+H]⁺ .

### Production of Compound I-76

Compound I-76 (isolated yield 101 mg, percent yield 33%) was obtained in a similar manner to [Production of Compound I-75] except that Compound I-23 was used in place of Compound I-22.
¹H-NMR (500 MHz, CDCl₃, δ): 5.86-5.81 (m, 1H), 5.76-5.64 (m, 2H), 5.40 (d, J = 2.9 Hz, 1H), 5.39-5.32 (m, 1H), 4.98-4.93 (m, 1H), 4.93 (s, 1H), 4.76 (d, J = 3.4 Hz, 1H), 4.68-4.60 (m, 2H), 4.21 (t, J = 6.0 Hz, 2H), 3.92 (br s, 1H), 3.86-3.80 (m, 1H), 3.80-3.72 (m, 1H), 3.70-3.59 (m, 2H), 3.52-3.46 (m, 1H), 3.44 (s, 3H), 3.42 (s, 3H), 3.26-3.18 (m, 2H), 3.16 (t, J = 9.2 Hz, 1H), 2.77 (dd, J = 3.4, 12.6 Hz, 1H), 2.69-2.56 (m, 3H), 2.52-2.45 (m, 1H), 2.37-2.18 (m, 4H), 2.28 (s, 6H), 1.94-1.85 (m, 2H), 1.85-1.79 (m, 1H), 1.77-1.70 (m, 1H), 1.68-1.61 (m, 1H), 1.59-1.35 (m, 13H), 1.30-1.23 (m, 7H), 1.15 (t, J = 6.9 Hz, 6H), 0.91 (t, J = 7.4 Hz, 3H), 0.87 (t, J = 6.9 Hz, 1H), 0.83 (d, J = 6.3 Hz, 3H), 0.80-0.73 (m, 4H); MSm/z 961.6001 [M+H]⁺ .

### Production of Compound I-77

To a solution of Compound I-22 (25 mg, 0.0267 mmol) obtained in [Production of Compound I-22] in dichloromethane (0.27 mL), triethylamine (11.1 µL, 0.0803 mmol) was added, and further phenylsulfonyl chloride (4.11 µL, 0.0321 mmol) was added, followed by stirring at room temperature for 1.5 hours. A saturated aqueous sodium bicarbonate solution was added to the reaction solution to stop the reaction. After extraction with ethyl acetate, the organic layer was washed with saturated brine and dried over anhydrous magnesium sulfate. After the solvent was distilled off under reduced pressure, the residue was purified by preparative thin-layer silica gel column chromatography (n-hexane/ethyl acetate system) to obtain Compound I-77 (isolated yield 18.9 mg, percent yield 66%). The ¹H-NMR spectrum below is the analytical data of the main isomer.
¹H-NMR (500 MHz, CDCl₃, δ): 7.87-7.82 (m, 2H), 7.58-7.52 (m, 1H), 7.52-7.44 (m, 2H), 5.90-5.83 (m, 1H), 5.79-5.66 (m, 2H), 5.53 (t, J = 6.0 Hz, 1H), 5.46-5.36 (m, 2H), 4.99-4.92 (m, 1H), 4.76 (d, J = 3.4 Hz, 1H), 4.72 (s, 1H), 4.70-4.65 (m, 1H), 4.64-4.54 (m, 1H), 4.14-4.02 (m, 2H), 3.95-3.91 (m, 1H), 3.87-3.80 (m, 1H), 3.80-3.73 (m, 1H), 3.71-3.59 (m, 2H), 3.52-3.46 (m, 1H), 3.44 (s, 3H), 3.42 (s, 3H), 3.29-3.13 (m, 5H), 2.84-2.76 (m, 1H), 2.56-2.45 (m, 2H), 2.38-2.17 (m, 5H), 1.94-1.86 (m, 1H), 1.79-1.72 (m, 1H), 1.70-1.62 (m, 3H), 1.61-1.35 (m, 12H), 1.29-1.24 (m, 8H), 1.20-1.13 (m, 6H), 0.92 (t, J = 7.4 Hz, 3H), 0.87 (t, J = 6.9 Hz, 2H), 0.84 (d, J = 6.9 Hz, 3H), 0.82-0.74 (m, 4H); MSm/z 1090.5881 [M+NH₄] ⁺.

### Production of Compound I-78

Compound I-78 (isolated yield 19.2 mg, percent yield 67%) was obtained in a similar manner to [Production of Compound I-77] except that Compound I-23 was used in place of Compound I-22.
¹H-NMR (500 MHz, CDCl₃, δ): 7.86-7.83 (m, 2H), 7.57-7.53 (m, 1H), 7.51-7.46 (m, 2H), 5.84 (td, J = 2.4,10.7 Hz, 1H), 5.79-5.64 (m, 2H), 5.46-5.36 (m, 3H), 4.97-4.92 (m, 1H), 4.85 (s, 1H), 4.76 (d, J = 3.4 Hz, 1H), 4.72 (s, 1H), 4.71-4.62 (m, 2H), 4.12-4.04 (m, 2H), 3.93 (br s, 1H), 3.86-3.80 (m, 1H), 3.79-3.73 (m, 1H), 3.71-3.60 (m, 2H), 3.51-3.46 (m, 1H), 3.44 (s, 3H), 3.41 (s, 3H), 3.22 (s, 4H), 3.16 (t, J = 9.2 Hz, 1H), 2.76 (dd, J = 4.0,12.0 Hz, 1H), 2.61-2.46 (m, 3H), 2.37-2.30 (m, 2H), 2.30-2.18 (m, 2H), 1.93-1.87 (m, 1H), 1.86-1.78 (m, 2H), 1.76-1.71 (m, 1H), 1.70-1.64 (m, 3H), 1.59-1.35 (m, 7H), 1.30-1.22 (m, 9H), 1.16 (d, J = 6.9 Hz, 3H), 1.08 (d, J = 6.9 Hz, 3H), 0.92 (t, J = 7.4 Hz, 3H), 0.89-0.85 (m, 2H), 0.84 (d, J = 6.9 Hz, 3H), 0.80-0.75 (m, 4H); MSm/z 1090.5879 [M+NH₄]⁺.

### Reference Example 13

### (1) Production of O-(oxetan-3-ylmethyl)hydroxylamine

Oxetan-3-ylmethanol (0.75 mL, 9.28 mmol) was dissolved in THF (93 mL), and bis(2-methoxyethyl) (E)-diazene-1,2-dicarboxylate toluene solution (2.28 mL, 10.6 mmol), 2-hydroxyisoindoline-1,3-dione (1.44 g, 8.84 mmol) and triphenylphosphine (2.78 g, 10.6 mmol) were sequentially added, followed by stirring at room temperature for 1 hour. Water was added to the reaction solution, and the mixture was extracted with ethyl acetate. The organic layer was washed with saturated brine and dried over anhydrous magnesium sulfate. After the solvent was distilled off under reduced pressure, the residue was purified by silica gel column chromatography (n-hexane/ethyl acetate system) to obtain 2-(oxetan-3-ylmethoxy)isoindoline-1,3-dione (isolated yield 1.96 g, percent yield 95%).
¹H-NMR (500 MHz, CDCl₃, δ): 7.86-7.81 (m, 2H), 7.78-7.73 (m, 2H), 4.89-4.84 (m, 2H), 4.59 (dt, J = 4.0, 6.0 Hz, 2H), 4.47 (dd, J = 3.7, 7.2 Hz, 2H), 3.49-3.38 (m, 1H).

### (2) O-(Oxetan-3-ylmethyl)hydroxylamine

2-(Oxetan-3-ylmethoxy)isoindoline-1,3-dione (400 mg, 1.71 mmol) obtained in Reference Example 13-(1) was dissolved in ethanol (17 mL). To the solution, hydrazine monohydrate (91.7 µL, 1.89 mmol) was added, and the mixture was stirred at 60°C for 30 minutes. The reaction solution was cooled to 0°C, and the resulting precipitate was removed by filtration. The solvent was distilled off from the filtrate under reduced pressure to obtain O-(oxetan-3-ylmethyl)hydroxylamine (isolated yield 166 mg, percent yield 93%).
MSm/z 104.0706 [M+H]⁺ C4H10NO2 (FAB)
¹H-NMR (500 MHz, DMSO-d₆, δ): 4.58 (d, J = 6.3 Hz, 1H), 4.57 (d, J = 6.3 Hz, 1H), 4.25 (t, J = 6.3 Hz, 2H), 3.71 (d, J = 6.3 Hz, 2H), 3.18-3.09 (m, 1H).

### Production of Compound I-79

Compound I-79 (isolated yield 255 mg, percent yield 77%) was obtained as a mixture of isomers that were difficult to separate in a similar manner to [Production of Compound I-1] except that O-(oxetan-3-ylmethyl)hydroxylamine (14.1 mg, 0.141 mmol) obtained in Reference Example 13-(2) was used in place of hydroxylamine hydrochloride.
¹H-NMR (500 MHz, CDCl₃, δ): 5.88-5.83 (m, 1H), 5.78-5.64 (m, 2H), 5.46-5.37 (m, 2H), 4.94 (d, J = 10.9 Hz, 1H), 4.80-4.75 (m, 3H), 4.70-4.56 (m, 3H), 4.53-4.46 (m, 2H), 4.35-4.27 (m, 2H), 4.02-4.00 (m, 1H), 3.95 (s, 1H), 3.93 (br s, 1H), 3.86-3.80 (m, 1H), 3.79-3.73 (m, 1H), 3.71-3.60 (m, 2H), 3.52-3.45 (m, 2H), 3.44 (s, 3H), 3.42 (s, 3H), 3.38-3.29 (m, 1H), 3.27-3.19 (m, 2H), 3.16 (t, J = 9.2 Hz, 1H), 2.88 (dd, J = 2.9,13.7 Hz, 1H), 2.85-2.78 (m, 1H), 2.53-2.45 (m, 2H), 2.37-2.28 (m, 3H), 2.28-2.18 (m, 2H), 1.93-1.87 (m, 1H), 1.78-1.72 (m, 1H), 1.69-1.35 (m, 22H), 1.29-1.23 (m, 10H), 1.20 (d, J = 7.4 Hz, 1H), 1.17-1.12 (m, 4H), 0.92 (t, J = 7.4 Hz, 3H), 0.89-0.85 (m, 1H), 0.83 (d, J = 6.3 Hz, 3H), 0.81-0.75 (m, 5H); MSm/z 960.5689 [M+H]⁺ .

### Production of Compound I-80

Compound I-80 (isolated yield 25.5 mg, percent yield 77%) was obtained in a similar manner to [Production of Compound I-2] except that O-(oxetan-3-ylmethyl)hydroxylamine obtained in Reference Example 13-(2) was used in place of hydroxylamine hydrochloride. ¹H-NMR (500 MHz, CDCl₃, δ): 5.83 (td, J = 2.3, 10.3 Hz, 1H), 5.76-5.63 (m, 2H), 5.43-5.35 (m, 2H), 4.93 (d, J = 11.5 Hz, 1H), 4.88 (s, 1H), 4.81-4.74 (m, 3H), 4.70-4.60 (m, 3H), 4.50 (dt, J = 1.4, 6.2 Hz, 2H), 4.32 (d, J = 6.9 Hz, 2H), 3.92 (br s, 1H), 3.86-3.79 (m, 1H), 3.79-3.73 (m, 1H), 3.70-3.59 (m, 2H), 3.51-3.46 (m, 1H), 3.44 (s, 3H), 3.42 (s, 3H), 3.37-3.29 (m, 1H), 3.26-3.19 (m, 2H), 3.16 (t, J = 9.2 Hz, 1H), 2.77 (dd, J = 3.4, 12.0 Hz, 1H), 2.62-2.54 (m, 1H), 2.53-2.44 (m, 2H), 2.37-2.30 (m, 2H), 2.29-2.18 (m, 2H), 1.93-1.80 (m, 3H), 1.77-1.70 (m, 1H), 1.68-1.60 (m, 3H), 1.59-1.34 (m, 10H), 1.31-1.23 (m, 7H), 1.14 (dd, J = 2.6, 6.6 Hz, 6H), 0.91 (t, J = 7.4 Hz, 3H), 0.83 (d, J = 6.9 Hz, 3H), 0.81-0.71 (m, 4H); MSm/z 977.5950 [M+NH₄] ⁺.

### Production of Compound 1-81

Compound I-81 (isolated yield 3.1 mg, percent yield 11%) was obtained as a mixture of isomers that were difficult to separate in a similar manner to [Production of Compound I-77] except that isopropylsulfonium chloride was used in place of phenylsulfonyl chloride. The ¹H-NMR spectrum below is the analytical data of the main isomer.
¹H-NMR (500 MHz, CDCl₃, δ): 5.86 (td, J = 10.5, 2.5 Hz, 1H), 5.78-5.65 (m, 2H), 5.45-5.37 (m, 2H), 5.01-4.97 (m, 1H), 4.94 (d, J = 10.3 Hz, 1H), 4.79 (s, 1H), 4.76 (d, J = 3.4 Hz, 1H), 4.71-4.55 (m, 3H), 4.24-4.16 (m, 2H), 3.93 (br s, 1H), 3.86-3.80 (m, 1H), 3.80-3.73 (m, 1H), 3.71-3.60 (m, 2H), 3.52-3.45 (m, 2H), 3.44 (s, 3H), 3.42 (s, 3H), 3.41-3.35 (m, 2H), 3.27-3.19 (m, 2H), 3.19-3.11 (m, 2H), 2.91-2.83 (m, 2H), 2.54-2.45 (m, 1H), 2.37-2.28 (m, 3H), 2.28-2.18 (m, 2H), 1.93-1.88 (m, 1H), 1.78-1.72 (m, 1H), 1.70-1.33 (m, 12H), 1.31-1.23 (m, 11H), 1.16 (d, J = 6.9 Hz, 3H), 0.92 (t, J = 7.2 Hz, 3H), 0.89-0.86 (m, 2H), 0.84 (d, J = 6.9 Hz, 3H), 0.82-0.74 (m, 4H); MSm/z 1056.6042 [M+NH₄] ⁺.

### Production of Compound I-82

Compound I-82 (isolated yield 6.3 mg, percent yield 23%) was obtained in a similar manner to [Production of Compound I-78] except that isopropylsulfonium chloride was used in place of phenylsulfonyl chloride.
¹H-NMR (500 MHz, CDCl₃, δ): 5.83 (td, J = 2.5,10.5 Hz, 1H), 5.78-5.63 (m, 2H), 5.44-5.35 (m, 2H), 4.98-4.91 (m, 3H), 4.76 (d, J = 3.4 Hz, 1H), 4.72-4.59 (m, 3H), 4.24-4.16 (m, 2H), 3.93 (br s, 1H), 3.87-3.80 (m, 1H), 3.80-3.73 (m, 1H), 3.71-3.59 (m, 2H), 3.51-3.45 (m, 1H), 3.44 (s, 3H), 3.42 (s, 3H), 3.41-3.37 (m, 2H), 3.27-3.19 (m, 2H), 3.19-3.10 (m, 2H), 2.81-2.75 (m, 1H), 2.66-2.59 (m, 1H), 2.53-2.45 (m, 2H), 2.37-2.31 (m, 2H), 2.30-2.18 (m, 2H), 1.93-1.83 (m, 3H), 1.76-1.70 (m, 1H), 1.69-1.38 (m, 8H), 1.35 (d, J = 6.9 Hz, 6H), 1.29-1.23 (m, 7H), 1.18-1.14 (m, 6H), 0.92 (t, J = 7.4 Hz, 3H), 0.83 (d, J = 6.3 Hz, 3H), 0.78 (d, J = 5.7 Hz, 4H); MSm/z 1056.6042 [M+NH₄] ⁺.

### Reference Example 14

### (1) Production of 2-((1-((tertbutyldimethylsilyl)oxy)cyclopropyl)methoxy)isoindoline -1,3-dione

According to the method described in International Publication No. WO 2020/260857, 2-((1-((tertbutyldimethylsilyl)oxy)cyclopropyl)methoxy)isoindoline -1,3-dione (isolated yield 1.17 g, percent yield 42%) was obtained using methyl 1-hydroxycyclopropane-1-carboxylate (3 g, 25.8 mmol) as a raw material.
¹H-NMR (500 MHz, CDCl₃, δ): 7.81 (dd, J = 5.5, 3.0 Hz, 2H), 7.73 (dd, J = 5.5, 3.0 Hz, 2H), 4.18 (s, 2H), 0.90-0.87 (m, 2H), 0.83 (s, 9H), 0.85-0.82 (m, 2H), 0.17 (s, 6H).

### (2) Production of 2-(2-((tertbutyldimethylsilyl)oxy)ethoxy)ethan-1-ol

2-((1-((tertButyldimethylsilyl)oxy)cyclopropyl)methoxy)isoindoline -1,3-dione (500 mg, 1.43 mmol) obtained in Reference Example 14-(1) was dissolved in ethanol (14 mL). To the solution, hydrazine monohydrate (76.9 µL, 1.52 mmol) was added, and the mixture was stirred at 60°C for 30 minutes. After completion of the reaction, the reaction solution was cooled to 0°C, and the resulting precipitate was removed by filtration. The solvent was distilled off from the filtrate under reduced pressure to obtain 2-(2-((tertbutyldimethylsilyl)oxy)ethoxy)ethan-1-ol (isolated yield 261 mg, percent yield 83%).
¹H-NMR (500 MHz, DMSO-d₆, δ): 3.52 (s, 2H), 0.77 (d, J = 1.1 Hz, 9H), 0.55 (s, 4H), 0.05 (d, J = 1.1 Hz, 6H).

### Production of Compound I-83 and Compound I-84

In place of hydroxylamine hydrochloride in [Production of Compound I-1], O-((1-((tertbutyldimethylsilyl)oxy)cyclopropyl)methyl)hydroxylamin e obtained in Reference Example 14-(2) was used, and purification was performed by preparative thin-layer silica gel column chromatography (n-hexane/ethyl acetate system) in place of silica gel chromatography, to obtain Compound I-83 (isolated yield 12.7 mg, percent yield 34%) and Compound I-84 (isolated yield 16.7 mg, percent yield 45%) in a similar manner to [Production of Compound I-1].

### (Compound I-83)

¹H-NMR (500 MHz, CDCl₃, δ): 5.87-5.81 (m, 1H), 5.77-5.66 (m, 2H), 5.45-5.37 (m, 2H), 4.98-4.93 (m, 1H), 4.83 (s, 1H), 4.77 (d, J = 3.4 Hz, 1H), 4.69-4.49 (m, 3H), 4.25-4.18 (m, 1H), 4.14-4.00 (m, 1H), 3.93 (br s, 1H), 3.86-3.80 (m, 1H), 3.79-3.73 (m, 1H), 3.71-3.60 (m, 2H), 3.52-3.45 (m, 1H), 3.44 (s, 3H), 3.42 (s, 3H), 3.26-3.20 (m, 2H), 3.16 (t, J = 9.2 Hz, 1H), 2.93-2.86 (m, 1H), 2.86-2.75 (m, 1H), 2.53-2.45 (m, 2H), 2.37-2.30 (m, 3H), 2.30-2.19 (m, 2H), 1.93-1.87 (m, 1H), 1.79-1.73 (m, 1H), 1.69-1.64 (m, 1H), 1.63-1.36 (m, 12H), 1.30-1.23 (m, 10H), 1.18-1.13 (m, 3H), 0.92 (t, J = 7.4 Hz, 3H), 0.86-0.80 (m, 14H), 0.78 (d, J = 6.3 Hz, 3H), 0.74-0.61 (m, 4H), 0.10 (s, 3H), 0.10 (s, 3H); MSm/z 1091.6815 [M+NH₄] ⁺.

### (Compound I-84)

¹H-NMR (500 MHz, CDCl₃, δ): 5.85 (td, J = 2.3,10.3 Hz, 1H), 5.76-5.64 (m, 2H), 5.46-5.38 (m, 2H), 4.95 (d, J = 9.7 Hz, 1H), 4.76 (d, J = 3.4 Hz, 1H), 4.60 (s, 1H), 4.67-4.55 (m, 2H), 4.23 (d, J = 11.5 Hz, 1H), 4.06 (d, J = 11.5 Hz, 1H), 3.96 (s, 1H), 3.93 (br s, 1H), 3.86-3.80 (m, 1H), 3.79-3.73 (m, 1H), 3.71-3.60 (m, 2H), 3.60-3.53 (m, 1H), 3.51-3.46 (m, 1H), 3.44 (s, 3H), 3.42 (s, 3H), 3.27-3.20 (m, 2H), 3.16 (t, J = 9.2 Hz, 1H), 2.90 (dd, J = 2.9, 13.7 Hz, 1H), 2.49 (d, J = 2.3 Hz, 2H), 2.34 (dd, J = 4.3, 12.3 Hz, 2H), 2.23 (d, J = 5.7 Hz, 3H), 1.93-1.87 (m, 1H), 1.79-1.72 (m, 1H), 1.69-1.64 (m, 1H), 1.61-1.36 (m, 10H), 1.26 (dd, J = 6.3, 12.0 Hz, 8H), 1.22 (d, J = 6.9 Hz, 3H), 1.15 (d, J = 6.9 Hz, 3H), 0.92 (t, J = 7.4 Hz, 3H), 0.89-0.85 (m, 1H), 0.85-0.81 (m, 13H), 0.80-0.77 (m, 4H), 0.74-0.62 (m, 3H), 0.10 (s, 3H), 0.10 (s, 3H); MSm/z 1091.6815 [M+NH₄] ⁺.

### Production of Compound I-85 and Compound I-86

Compound I-83 (12.7 mg, 0.0118 mmol) obtained in [Production of Compound I-83 and Compound I-84] was dissolved in THF (0.24 mL) and then cooled in ice to 0°C. There, a hydrogen fluoride-pyridine solution (0.108 mL, 1.18 mmol) was added, and the mixture was stirred at room temperature for 20 minutes. A saturated aqueous sodium hydrogen carbonate solution was added to the reaction solution, and the mixture was extracted with ethyl acetate. The organic layer was washed with saturated brine and dried over anhydrous magnesium sulfate. After the solvent was distilled off under reduced pressure, the residue was purified by preparative thin-layer silica gel column chromatography (n-hexane/ethyl acetate system) to obtain Compound I-85 (isolated yield 4.4 mg, percent yield 55%) and Compound I-86 (isolated yield 1.8 mg, percent yield 19%) .

### (Compound I-85)

¹H-NMR (500 MHz, CDCl₃, δ): 5.85-5.79 (m, 1H), 5.77-5.67 (m, 2H), 5.43-5.35 (m, 1H), 5.33-5.27 (m, 1H), 4.69-4.54 (m, 3H), 4.21-4.18 (m, 1H), 4.17-4.04 (m, 2H), 3.99 (br s, 1H), 3.96 (s, 1H), 3.72-3.64 (m, 1H), 3.59-3.52 (m, 1H), 3.20-3.16 (m, 1H), 2.88 (dd, J = 2.9, 13.7 Hz, 1H), 2.55-2.46 (m, 1H), 2.36-2.21 (m, 3H), 1.92-1.83 (m, 1H), 1.76-1.71 (m, 1H), 1.69-1.46 (m, 15H), 1.46-1.37 (m, 4H), 1.28-1.21 (m, 5H), 1.17 (d, J = 6.9 Hz, 4H), 0.95 (t, J = 7.4 Hz, 3H), 0.85-0.77 (m, 10H), 0.67-0.60 (m, 2H); MSm/z 672.4112 [M+H]⁺ .

### (Compound I-86)

¹H-NMR (500 MHz, CDCl₃, δ): 5.89-5.84 (m, 1H), 5.77-5.64 (m, 2H), 5.47-5.37 (m, 1H), 4.97-4.92 (m, 1H), 4.82-4.79 (m, 1H), 4.69-4.54 (m, 3H), 4.21-4.17 (m, 1H), 4.13-4.09 (m, 1H), 3.97 (s, 1H), 3.96-3.93 (m, 1H), 3.89-3.83 (m, 1H), 3.73-3.64 (m, 1H), 3.60-3.53 (m, 2H), 3.49 (s, 3H), 3.42-3.32 (m, 1H), 3.24-3.19 (m, 1H), 3.18-3.14 (m, 1H), 2.93-2.88 (m, 1H), 2.53-2.45 (m, 2H), 2.37-2.22 (m, 5H), 1.93-1.86 (m, 2H), 1.80-1.74 (m, 2H), 1.71-1.34 (m, 9H), 1.32-1.22 (m, 8H), 1.14 (d, J = 6.9 Hz, 3H), 0.92 (t, J = 7.4 Hz, 3H), 0.86-0.83 (m, 5H), 0.78 (d, J = 6.3 Hz, 4H), 0.67-0.59 (m, 2H); MSm/z 816.4898 [M+H]⁺ .

### Production of Compound I-87 and Compound I-88

Compound I-87 (isolated yield 5.9 mg, percent yield 56%) and Compound I-88 (isolated yield 3.3 mg, percent yield 26%) were obtained in a similar manner to [Production of Compound I-85 and Compound I-86] except that Compound I-84 was used in place of Compound I-83.

### (Compound I-87)

¹H-NMR (500 MHz, CDCl₃, δ): 5.85-5.80 (m, 1H), 5.76-5.67 (m, 2H), 5.43-5.35 (m, 1H), 5.32-5.27 (m, 1H), 4.68-4.56 (m, 3H), 4.22-4.17 (m, 1H), 4.16-4.04 (m, 2H), 4.00 (br s, 1H), 3.96 (s, 1H), 3.72-3.64 (m, 1H), 3.59-3.51 (m, 1H), 3.21-3.17 (m, 1H), 2.88 (dd, J = 2.9, 13.7 Hz, 1H), 2.54-2.45 (m, 1H), 2.37-2.21 (m, 3H), 1.91-1.86 (m, 1H), 1.77-1.70 (m, 1H), 1.69-1.63 (m, 2H), 1.61-1.44 (m, 9H), 1.44-1.37 (m, 3H), 1.30-1.21 (m, 7H), 1.17 (d, J = 6.9 Hz, 3H), 0.95 (t, J = 7.2 Hz, 3H), 0.87 (s, 1H), 0.85-0.74 (m, 10H), 0.67-0.58 (m, 2H); MSm/z 672.4112 [M+H]⁺ .

### (Compound I-88)

¹H-NMR (500 MHz, CDCl₃, δ): 5.86 (td, J = 2.4, 10.2 Hz, 1H), 5.77-5.64 (m, 2H), 5.47-5.38 (m, 1H), 4.96-4.91 (m, 1H), 4.81 (d, J = 3.4 Hz, 1H), 4.68-4.61 (m, 2H), 4.60-4.55 (m, 1H), 4.21-4.17 (m, 1H), 4.15-4.08 (m, 1H), 3.97 (s, 1H), 3.95 (br s, 1H), 3.90-3.83 (m, 1H), 3.72-3.65 (m, 1H), 3.60-3.54 (m, 2H), 3.49 (s, 3H), 3.42-3.30 (m, 1H), 3.24-3.19 (m, 1H), 3.16 (t, J = 9.2 Hz, 1H), 2.90 (dd, J = 2.9, 13.2 Hz, 1H), 2.56-2.44 (m, 2H), 2.38-2.22 (m, 4H), 1.93-1.87 (m, 1H), 1.79-1.73 (m, 1H), 1.70-1.64 (m, 1H), 1.62-1.35 (m, 10H), 1.27 (d, J = 6.3 Hz, 4H), 1.26-1.22 (m, 4H), 1.14 (d, J = 6.9 Hz, 3H), 0.92 (t, J = 7.4 Hz, 3H), 0.87-0.76 (m, 10H), 0.68-0.58 (m, 2H); MSm/z 816.4898 [M+H]⁺.

### Production of Compound I-89

Compound I-89 (isolated yield 17.5 mg, percent yield 48%) was obtained in a similar manner to [Production of Compound I-2] except that O-((1-((tertbutyldimethylsilyl)oxy)cyclopropyl)methyl)hydroxylamin e obtained in Reference Example 14-(2) was used in place of hydroxylamine hydrochloride.
¹H-NMR (500 MHz, CDCl₃, δ): 5.85-5.79 (m, 1H), 5.76-5.64 (m, 2H), 5.43-5.35 (m, 2H), 4.97-4.92 (m, 1H), 4.94 (s, 1H), 4.76 (d, J = 3.4 Hz, 1H), 4.68-4.56 (m, 2H), 4.51 (s, 1H), 4.20 (d, J = 11.5 Hz, 1H), 4.07 (d, J = 11.5 Hz, 1H), 3.93 (br s, 1H), 3.86-3.80 (m, 1H), 3.80-3.73 (m, 1H), 3.70-3.60 (m, 2H), 3.51-3.45 (m, 1H), 3.51-3.45 (m, 1H), 3.44 (s, 3H), 3.42 (s, 3H), 3.26-3.19 (m, 2H), 3.16 (t, J = 9.2 Hz, 1H), 2.78 (dd, J = 2.9,12.6 Hz, 1H), 2.64-2.55 (m, 1H), 2.53-2.45 (m, 2H), 2.37-2.19 (m, 4H), 1.93-1.86 (m, 2H), 1.85-1.78 (m, 1H), 1.77-1.72 (m, 1H), 1.68-1.63 (m, 1H), 1.57-1.35 (m, 6H), 1.27 (d, J = 6.3 Hz, 4H), 1.25 (d, J = 6.3 Hz, 4H), 1.16 (d, J = 3.4 Hz, 3H), 1.15 (d, J = 2.9 Hz, 3H), 0.92 (t, J = 7.2 Hz, 3H), 0.88-0.82 (m, 8H), 0.82-0.76 (m, 12H), 0.73-0.64 (m, 4H), 0.09 (d, J = 5.2 Hz, 6H); MSm/z 1091.6815 [M+NH₄] ⁺.

### Production of Compound I-90 and Compound I-91

Compound I-90 (isolated yield 6.5 mg, percent yield 59%) and Compound I-91 (isolated yield 2.6 mg, percent yield 20%) were obtained in a similar manner to [Production of Compound I-85 and Compound I-86] except that Compound I-89 was used in place of Compound I-83.

### (Compound I-90)

¹H-NMR (500 MHz, CDCl₃, δ): 5.82-5.77 (m, 1H), 5.73-5.69 (m, 2H), 5.40-5.33 (m, 1H), 5.32-5.27 (m, 1H), 5.03 (s, 1H), 4.76 (s, 1H), 4.72-4.63 (m, 2H), 4.18-4.14 (m, 1H), 4.08-4.04 (m, 1H), 3.99 (br s, 1H), 3.71-3.64 (m, 1H), 3.20-3.16 (m, 1H), 2.77 (dd, J = 4.0,12.0 Hz, 1H), 2.68-2.61 (m, 1H), 2.54-2.47 (m, 1H), 2.36-2.23 (m, 2H), 2.17 (s, 1H), 1.91-1.83 (m, 3H), 1.74-1.69 (m, 1H), 1.69-1.46 (m, 7H), 1.44-1.36 (m, 4H), 1.27-1.23 (m, 2H), 1.17 (dd, J = 1.7, 6.9 Hz, 6H), 0.95 (t, J = 7.4 Hz, 3H), 0.83 (d, J = 6.9 Hz, 4H), 0.80-0.76 (m, 6H), 0.66-0.56 (m, 2H); MSm/z 672.4112 [M+H]⁺ .

### (Compound I-91)

¹H-NMR (500 MHz, CDCl₃, δ) : 5.84 (td, J = 2.4,10.6 Hz, 1H), 5.77-5.64 (m, 2H), 5.44-5.36 (m, 1H), 5.04 (s, 1H), 4.96-4.91 (m, 1H), 4.81 (d, J = 4.0 Hz, 1H), 4.67 (s, 2H), 4.18-4.14 (m, 1H), 4.10-4.05 (m, 1H), 3.95 (br s, 1H), 3.90-3.83 (m, 1H), 3.72-3.65 (m, 1H), 3.60-3.54 (m, 1H), 3.49 (s, 3H), 3.23-3.19 (m, 1H), 3.16 (t, J = 9.2 Hz, 1H), 2.80 (dd, J = 4.6,11.5 Hz, 1H), 2.69-2.62 (m, 1H), 2.54-2.47 (m, 2H), 2.37-2.31 (m, 2H), 2.29-2.22 (m, 2H), 1.92-1.86 (m, 4H), 1.77-1.72 (m, 2H), 1.68-1.65 (m, 3H), 1.59-1.36 (m, 10H), 1.29-1.24 (m, 5H), 1.19-1.11 (m, 3H), 0.94-0.91 (m, 3H), 0.86-0.76 (m, 6H), 0.67-0.54 (m, 2H); MSm/z 816.4907 [M+H]⁺.

### Production of Compound I-92

Compound I-83 (15.0 mg, 0.0140 mmol) obtained in [Production of Compound I-83 and Compound I-84] was dissolved in THF (0.23 mL), and then the reaction solution was cooled to -10°C. Pyridine (0.75 mL) and a hydrogen fluoride-pyridine solution (0.125 mL) were added, and the mixture was stirred at the same temperature for 30 minutes. Further, a hydrogen fluoride-pyridine solution (62.5 µL) was added, and the mixture was stirred at the same temperature for 30 minutes. Then, a hydrogen fluoride-pyridine solution (0.11 mL) was added again, the temperature was raised to 0°C, and the mixture was stirred for 1 hour. A saturated aqueous sodium hydrogen carbonate solution was added to the reaction solution, and the mixture was extracted with ethyl acetate. The organic layer was washed with saturated brine and dried over anhydrous magnesium sulfate. After the solvent was distilled off under reduced pressure, the residue was purified by preparative thin-layer silica gel column chromatography (n-hexane/ethyl acetate system) to obtain Compound I-92 (isolated yield 10.8 mg, percent yield 81%).
¹H-NMR (500 MHz, CDCl₃, δ): 5.88-5.83 (m, 1H), 5.78-5.65 (m, 2H), 5.44-5.37 (m, 2H), 4.97-4.92 (m, 1H), 4.91 (s, 1H), 4.76 (d, J = 3.4 Hz, 1H), 4.71-4.62 (m, 3H), 4.16-4.13 (m, 1H), 4.11 (d, J = 6.9 Hz, 1H), 4.08-4.04 (m, 1H), 3.93 (br s, 1H), 3.86-3.80 (m, 1H), 3.79-3.73 (m, 1H), 3.71-3.59 (m, 2H), 3.51-3.46 (m, 1H), 3.44 (s, 3H), 3.42 (s, 3H), 3.26-3.19 (m, 2H), 3.16 (t, J = 9.2 Hz, 1H), 2.91-2.85 (m, 2H), 2.57-2.46 (m, 2H), 2.39-2.19 (m, 5H), 2.13 (s, 2H), 1.92-1.85 (m, 2H), 1.79-1.71 (m, 2H), 1.71-1.35 (m, 9H), 1.30-1.23 (m, 12H), 1.20-1.13 (m, 4H), 0.92 (t, J = 7.4 Hz, 3H), 0.84 (d, J = 6.9 Hz, 2H), 0.82-0.75 (m, 6H), 0.65-0.57 (m, 2H); MSm/z 960.5684 [M+H]⁺ .

### Production of Compound I-93

Compound I-93 (isolated yield 14.1 mg, percent yield 95%) was obtained in a similar manner to [Production of Compound I-92] except that Compound I-84 was used in place of Compound I-83.
¹H-NMR (500 MHz, CDCl₃, δ): 5.88-5.83 (m, 1H), 5.77-5.65 (m, 2H), 5.47-5.38 (m, 2H), 4.97-4.92 (m, 1H), 4.76 (d, J = 3.4 Hz, 1H), 4.64 (s, 1H), 4.68-4.62 (m, 1H), 4.61-4.56 (m, 1H), 4.21-4.17 (m, 1H), 4.13-4.09 (m, 1H), 3.97 (s, 1H), 3.93 (br s, 1H), 3.87-3.80 (m, 1H), 3.80-3.73 (m, 1H), 3.71-3.54 (m, 3H), 3.51-3.46 (m, 1H), 3.44 (s, 3H), 3.42 (s, 3H), 3.26-3.19 (m, 2H), 3.16 (t, J = 9.2 Hz, 1H), 2.90 (dd, J = 13.7, 2.9 Hz, 1H), 2.58-2.45 (m, 2H), 2.36-2.19 (m, 5H), 2.13 (s, 1H), 1.93-1.86 (m, 1H), 1.79-1.72 (m, 1H), 1.70-1.35 (m, 13H), 1.27 (d, J = 6.3 Hz, 4H), 1.24 (dd, J = 6.9, 4.6 Hz, 6H), 1.16 (d, J = 6.9 Hz, 3H), 0.92 (t, J = 7.4 Hz, 3H), 0.88-0.76 (m, 10H), 0.67-0.59 (m, 2H); MSm/z 960.5684 [M+H]⁺.

### Production of Compound I-94

Compound I-94 (isolated yield 14.8 mg, percent yield 85%) was obtained in a similar manner to [Production of Compound I-92] except that Compound I-89 was used in place of Compound I-83.
¹H-NMR (500 MHz, CDCl₃, δ): 5.83 (td, J = 10.6, 2.4 Hz, 1H), 5.77-5.64 (m, 2H), 5.44-5.36 (m, 2H), 5.04 (s, 1H), 4.97-4.92 (m, 1H), 4.76 (d, J = 3.4 Hz, 1H), 4.75 (s, 1H), 4.69-4.66 (m, 2H), 4.18-4.13 (m, 1H), 4.09-4.04 (m, 1H), 3.93 (br s, 1H), 3.86-3.79 (m, 1H), 3.79-3.73 (m, 1H), 3.70-3.59 (m, 3H), 3.44 (s, 3H), 3.42 (s, 3H), 3.51-3.38 (m, 2H), 3.27-3.19 (m, 2H), 3.16 (t, J = 9.2 Hz, 1H), 2.80 (dd, J = 11.7, 4.3 Hz, 1H), 2.69-2.61 (m, 1H), 2.54-2.45 (m, 2H), 2.37-2.18 (m, 5H), 2.13 (s, 1H), 1.92-1.84 (m, 3H), 1.76-1.71 (m, 1H), 1.68-1.35 (m, 10H), 1.27 (d, J = 5.7 Hz, 4H), 1.25 (d, J = 6.3 Hz, 4H), 1.16 (dd, J = 10.0, 7.2 Hz, 3H), 0.92 (t, J = 7.4 Hz, 4H), 0.83 (d, J = 6.9 Hz, 4H), 0.85-0.76 (m, 6H), 0.67-0.56 (m, 2H); MSm/z 960.5684 [M+H]⁺.

### Production of Compound I-95

To a solution of Compound I-23 (20 mg, 0.0214 mmol) obtained in [Production of Compound I-23] in DMF (0.21 mL), DIPEA (22.4 µL, 0.129 mmol), isobutyric acid (11.9 µL, 0.129 mmol) and HATU (16.3 mg, 0.0428 mmol) were added, followed by stirring at room temperature overnight. A saturated aqueous sodium bicarbonate solution was added to the reaction solution to stop the reaction. After extraction with ethyl acetate, the organic layer was washed with saturated brine and dried over anhydrous magnesium sulfate. After the solvent was distilled off under reduced pressure, the residue was purified by preparative thin-layer silica gel column chromatography (n-hexane/ethyl acetate system) to obtain Compound I-95 (isolated yield 17.7 mg, percent yield 82%).
¹H-NMR (500 MHz, CDCl₃, δ): 6.14-6.09 (m, 1H), 5.84 (td, J = 10.7, 2.4 Hz, 1H), 5.77-5.65 (m, 2H), 5.44-5.35 (m, 2H), 4.95 (s, 1H), 4.95-4.91 (m, 1H), 4.76 (d, J = 2.9 Hz, 1H), 4.71-4.62 (m, 2H), 4.20-4.11 (m, 2H), 3.93 (br s, 1H), 3.86-3.79 (m, 1H), 3.79-3.73 (m, 1H), 3.70-3.60 (m, 2H), 3.56-3.44 (m, 4H), 3.44 (s, 3H), 3.42 (s, 3H), 3.26-3.19 (m, 2H), 3.16 (t, J = 9.2 Hz, 1H), 2.82-2.77 (m, 1H), 2.67-2.59 (m, 1H), 2.53-2.45 (m, 1H), 2.36-2.19 (m, 6H), 1.92-1.84 (m, 4H), 1.76-1.71 (m, 1H), 1.59-1.34 (m, 7H), 1.27 (d, J = 6.3 Hz, 5H), 1.25 (d, J = 6.3 Hz, 5H), 1.16 (t, J = 6.9 Hz, 7H), 1.13 (dd, J = 6.9, 1.1 Hz, 7H), 0.92 (t, J = 7.4 Hz, 3H), 0.89-0.85 (m, 1H), 0.83 (d, J = 6.9 Hz, 3H), 0.82-0.76 (m, 4H); MSm/z 1003.6106 [M+H]⁺.

### Production of Compound I-96

Compound I-96 (isolated yield 17.1 mg, percent yield 80%) was obtained in a similar manner to [Production of Compound I-95] except that glycolic acid was used in place of isobutyric acid.
¹H-NMR (500 MHz, CDCl₃, δ): 5.83 (td, J = 10.9, 2.3 Hz, 1H), 5.78-5.63 (m, 2H), 5.44-5.36 (m, 2H), 4.94 (s, 1H), 4.97-4.91 (m, 1H), 4.76 (d, J = 3.4 Hz, 1H), 4.70-4.62 (m, 2H), 4.28-4.15 (m, 2H), 4.04 (s, 2H), 3.93 (br s, 1H), 3.87-3.79 (m, 1H), 3.79-3.72 (m, 1H), 3.71-3.59 (m, 2H), 3.56 (q, J = 4.8 Hz, 2H), 3.51-3.46 (m, 1H), 3.44 (s, 3H), 3.42 (s, 3H), 3.27-3.19 (m, 2H), 3.16 (t, J = 9.2 Hz, 1H), 2.79 (dd, J = 4.9, 11.2 Hz, 1H), 2.66-2.58 (m, 1H), 2.53-2.45 (m, 1H), 2.36-2.31 (m, 2H), 2.30-2.19 (m, 3H), 1.93-1.81 (m, 3H), 1.75-1.69 (m, 1H), 1.69-1.63 (m, 1H), 1.59-1.34 (m, 12H), 1.27 (d, J = 6.3 Hz, 4H), 1.25 (d, J = 6.3 Hz, 4H), 1.16 (dd, J = 3.7, 6.6 Hz, 6H), 0.92 (t, J = 7.2 Hz, 3H), 0.87 (t, J = 6.9 Hz, 1H), 0.83 (d, J = 6.9 Hz, 3H), 0.82-0.74 (m, 4H); MSm/z 991.5743 [M+H]⁺.

### Production of Compound I-97

Compound I-97 (isolated yield 16.3 mg, percent yield 75%) was obtained in a similar manner to [Production of Compound I-95] except that cyclopropylacetic acid was used in place of isobutyric acid.
¹H-NMR (500 MHz, CDCl₃, δ): 5.83 (td, J = 10.7, 2.4 Hz, 1H), 5.77-5.65 (m, 2H), 5.43-5.35 (m, 2H), 4.94 (s, 1H), 4.98-4.91 (m, 1H), 4.76 (d, J = 3.4 Hz, 1H), 4.70-4.61 (m, 2H), 4.23-4.14 (m, 2H), 3.93 (br s, 1H), 3.87-3.79 (m, 1H), 3.79-3.73 (m, 1H), 3.70-3.45 (m, 6H), 3.44 (s, 3H), 3.42 (s, 3H), 3.27-3.19 (m, 2H), 3.16 (t, J = 9.2 Hz, 1H), 2.81-2.76 (m, 1H), 2.67-2.57 (m, 1H), 2.53-2.45 (m, 1H), 2.36-2.33 (m, 1H), 2.32 (d, J = 3.4 Hz, 1H), 2.29-2.18 (m, 2H), 2.12 (d, J = 6.9 Hz, 2H), 1.92-1.83 (m, 3H), 1.76-1.71 (m, 1H), 1.66 (d, J = 12.6 Hz, 1H), 1.60-1.33 (m, 10H), 1.27 (d, J = 5.7 Hz, 4H), 1.25 (d, J = 6.3 Hz, 4H), 1.16 (dd, J = 3.7, 6.6 Hz, 6H), 0.99-0.94 (m, 1H), 0.92 (t, J = 7.2 Hz, 3H), 0.89-0.85 (m, 2H), 0.83 (d, J = 6.9 Hz, 3H), 0.81-0.73 (m, 5H), 0.60-0.53 (m, 2H), 0.16 (q, J = 5.2 Hz, 2H); MSm/z 1015.6102 [M+H]⁺.

### Production of Compound I-98

Compound I-98 (isolated yield 36.3 mg, percent yield 67%) was obtained in a similar manner to [Production of Compound I-60] except that 2,2,2-trifluoroethylamine hydrochloride was used in place of dimethylamine.
¹H-NMR (500 MHz, CDCl₃, δ): 7.04 (t, J = 6.3 Hz, 1H), 5.86 (dt, J = 10.9, 2.6 Hz, 1H), 5.78-5.65 (m, 2H), 5.44-5.37 (m, 2H), 5.00 (s, 1H), 4.95-4.84 (m, 2H), 4.76 (d, J = 3.4 Hz, 1H), 4.72-4.60 (m, 4H), 4.13-4.02 (m, 1H), 3.93 (br s, 1H), 3.85-3.60 (m, 5H), 3.50-3.40 (m, 7H), 3.25-3.14 (m, 3H), 2.78 (dd, J = 12.3, 3.7 Hz, 1H), 2.70-2.63 (m, 1H), 2.52-2.47 (m, 1H), 2.34-2.19 (m, 4H), 1.91-1.80 (m, 3H), 1.74-1.71 (m, 1H), 1.67-1.64 (m, 1H), 1.58-1.35 (m, 12H), 1.27-1.13 (m, 12H), 0.93-0.74 (m, 10H); MSm/z 1046.5906 [M+NH₄]⁺.

### Reference Example 15

### Production of O-(azetidin-3-yl)hydroxylamine dihydrochloride

With reference to Journal of Medicinal Chemistry (2008), 51(15), 4601-4608, a crude product of O-(azetidin-3-yl)hydroxylamine dihydrochloride (isolated yield 648 mg) was obtained using tert-butyl 3-hydroxyazetidine-1-carboxylate (1.34 g, 7.72 mmol) as a raw material.
¹H-NMR (500 MHz, DMSO-d₆, δ): 9.68-9.16 (m, 2H), 4.66 (br s, 1H), 4.22-3.82 (m, 4H); MSm/z 89.0723 [M+H]⁺.

### Production of Compound I-99

Compound I-99 (isolated yield 4.2 mg, percent yield 13%) was obtained as a mixture of isomers that were difficult to separate in a similar manner to [Production of Compound I-1] except that the crude product of O-(azetidin-3-yl)hydroxylamine dihydrochloride obtained in Reference Example 15 was used in place of hydroxylamine hydrochloride.
¹H-NMR (500 MHz, CDCl₃, δ): 5.89-5.83 (m, 1H), 5.78-5.65 (m, 2H), 5.43-5.36 (m, 2H), 5.12-5.06 (m, 1H), 5.12-5.00 (m, 1H), 4.98-4.92 (m, 1H), 4.81 (s, 0.5H), 4.76 (d, J = 3.4 Hz, 1H), 4.70-4.56 (m, 2H), 4.33-4.23 (m, 2H), 4.21-4.13 (m, 2H), 3.98 (s, 0.5H), 3.93 (br s, 1H), 3.86-3.72 (m, 3H), 3.70-3.59 (m, 3H), 3.44 (s, 3H), 3.42 (s, 3H), 3.53-3.40 (m, 1H), 3.27-3.19 (m, 2H), 3.16 (t, J = 9.2 Hz, 2H), 2.94-2.86 (m, 2H), 2.53-2.46 (m, 2H), 2.37-2.19 (m, 5H), 1.95-1.87 (m, 2H), 1.78-1.73 (m, 1H), 1.69-1.63 (m, 2H), 1.60-1.33 (m, 9H), 1.30-1.21 (m, 10H), 1.16 (d, J = 6.9 Hz, 4H), 0.92 (s, 3H), 0.89-0.76 (m, 8H); MSm/z 945.5688 [M+H]⁺.

### Production of Compound I-100

Compound I-100 (isolated yield 10.7 mg, percent yield 33%) was obtained as a mixture of isomers that were difficult to separate in a similar manner to [Production of Compound I-2] except that the crude product of O-(azetidin-3-yl)hydroxylamine dihydrochloride obtained in Reference Example 15 was used in place of hydroxylamine hydrochloride.
¹H-NMR (500 MHz, CDCl₃, δ): 5.84 (td, J = 10.7, 2.4 Hz, 1H), 5.78-5.64 (m, 2H), 5.39 (br s, 1H), 5.38-5.30 (m, 1H), 5.10-5.03 (m, 1H), 4.98-4.93 (m, 1H), 4.92 (s, 1H), 4.78-4.75 (m, 1H), 4.66 (s, 2H), 4.28-4.13 (m, 4H), 3.93 (br s, 1H), 3.86-3.73 (m, 3H), 3.69-3.61 (m, 2H), 3.43 (s, 3H), 3.41 (s, 3H), 3.51-3.38 (m, 2H), 3.26-3.18 (m, 2H), 3.18-3.13 (m, 1H), 2.80-2.75 (m, 1H), 2.67-2.60 (m, 1H), 2.53-2.45 (m, 1H), 2.36-2.30 (m, 2H), 2.28-2.19 (m, 2H), 1.97-1.83 (m, 3H), 1.77-1.71 (m, 1H), 1.67-1.62 (m, 1H), 1.58-1.34 (m, 12H), 1.28-1.23 (m, 8H), 1.16 (dd, J = 6.6,10.0 Hz, 6H), 0.91 (t, J = 7.4 Hz, 3H), 0.89-0.85 (m, 1H), 0.82 (d, J = 6.9 Hz, 3H), 0.80-0.75 (m, 4H); MSm/z 945.5688 [M+H]⁺.

### Production of Compound I-101

Compound I-101 (isolated yield 21.8 mg, percent yield 70%) was obtained in a similar manner to [Production of Compound I-60] except that ethylamine hydrochloride was used in place of dimethylamine.
¹H-NMR (500 MHz, CDCl₃, δ): 6.45 (t, J = 5.7 Hz, 1H), 5.85 (dd, J = 8.6, 2.3 Hz, 1H), 5.44-5.33 (m, 2H), 4.99 (s, 1H), 4.95 (d, J = 10.9 Hz, 1H), 4.76-4.53 (m, 6H), 3.93 (br s, 1H), 3.85-3.72 (m, 2H), 3.69-3.60 (m, 2H), 3.50-3.41 (m, 8H), 3.38-3.14 (m, 3H), 2.80 (dd, J = 11.7, 3.7 Hz, 1H), 2.68-2.61 (m, 1H), 2.51-2.47 (m, 1H), 2.34-2.19 (m, 4H), 1.90-1.81 (m, 3H), 1.74-1.72 (m, 1H), 1.67-1.65 (m, 1H), 1.58-1.35 (m, 13H), 1.27-1.10 (m, 17H), 0.93-0.75 (m, 11H); MSm/z 992.6066 [M+NH₄]⁺.

### Production of Compound I-102

Compound I-102 (isolated yield 23.1 mg, percent yield 73%) was obtained in a similar manner to [Production of Compound I-60] except that 2-fluoroethylamine hydrochloride was used in place of dimethylamine.
¹H-NMR (500 MHz, CDCl₃, δ): 6.98 (t, J = 6.0 Hz, 1H), 5.86 (dt, J = 10.9, 2.3 Hz, 1H), 5.78-5.65 (m, 2H), 5.44-5.36 (m, 2H), 5.00 (s, 1H), 4.95 (d, J = 10.9 Hz, 1H), 4.76-4.50 (m, 6H), 4.46-4.40 (m, 1H), 3.93 (br s, 1H), 3.85-3.60 (m, 5H), 3.54-3.42 (m, 8H), 3.25-3.14 (m, 3H), 2.81-2.77 (m, 1H), 2.70-2.63 (m, 1H), 2.51-2.46 (m, 1H), 2.35-2.19 (m, 4H), 1.92-1.34 (m, 19H), 1.28-1.13 (m, 12H), 0.93-0.742 (m, 10H); MSm/z 1010.6110 [M+NH₄]⁺.

### Production of Compound I-103

Compound I-103 (isolated yield 24.2 mg, percent yield 76%) was obtained in a similar manner to [Production of Compound I-60] except that isopropylamine was used in place of dimethylamine.
¹H-NMR (500 MHz, CDCl₃, δ): 6.29 (d, J = 8.6 Hz, 1H), 5.85 (dd, J = 8.6, 2.3 Hz, 1H), 5.78-5.65 (m, 2H), 5.44-5.38 (m, 2H), 4.99 (s, 1H), 4.95 (d, J = 10.9 Hz, 1H), 4.76-4.65 (m, 4H), 4.58 (dd, J = 25.8, 16.0 Hz, 2H), 4.17-4.09 (m, 1H), 3.93 (br s, 1H), 3.85-3.72 (m, 2H), 3.69-3.61 (m, 2H), 3.50-3.41 (m, 7H), 3.25-3.14 (m, 3H), 2.80 (dd, J = 12.0, 4.0 Hz, 1H), 2.68-2.61 (m, 1H), 2.51-2.46 (m, 1H), 2.34-2.19 (m, 4H), 1.99-1.65 (m, 6H), 1.58-1.34 (m, 13H), 1.27-1.10 (m, 19H), 0.93-0.75 (m, 10H); MSm/z 1006.6337 [M+NH₄] ⁺.

### Reference Example 16

### Production of 2-((5-hydroxypentyl)oxy)isoindoline-1,3-dione (MM10-18)

5-(Aminooxy)pentan-1-ol hydrochloride (isolated yield 368 mg, percent yield 88%) was obtained in a similar manner to Reference Examples 5-(1) and (2), except that 5-bromopentan-1-ol was used in place of 1-bromo-2-methoxyethane in Reference Example 5-(1).
¹H-NMR (500 MHz, DMSO-d₆, δ): 5.84 (br s, 1H), 3.55 (t, J = 6.0 Hz, 2H), 3.48 (t, J = 6.6 Hz, 2H), 1.50-1.38 (m, 4H), 0.823 (s, 9H), 0.823 (s, 6H).

### Production of Compound I-104

Compound I-104 (isolated yield 9.3 mg, percent yield 28%) was obtained in a similar manner to [Production of Compound I-2] except that 5-(aminooxy)pentan-1-ol hydrochloride obtained in Reference Example 16 was used in place of hydroxylamine hydrochloride.
¹H-NMR (500 MHz, CDCl₃, δ): 5.84-5.82 (m, 1H), 5.75-5.64 (m, 2H), 5.42-5.36 (m, 2H), 4.94-4.93 (m, 2H), 4.76 (d, J = 6.9 Hz, 1H), 4.654-4.650 (m, 2H), 4.12-4.06 (m, 2H), 3.93 (br s, 1H), 3.86-3.74 (m, 2H), 3.68-3.60 (m, 4H), 3.52-3.41 (m, 8H), 3.26-3.15 (m, 3H), 2.80 (dd, J = 12.6, 3.4 Hz, 1H), 2.62-2.57 (m, 1H), 2.50-2.47 (m, 1H), 2.36-2.20 (m, 4H), 1.92-1.14 (m, 51H), 0.93-0.77 (m, 10H); MSm/z 998.5876 [M+Na]⁺.

### Production of Compound I-105

Compound I-105 (isolated yield 17.4 mg, percent yield 62%) was obtained in a similar manner to [Production of Compound I-1], except that 1-(aminooxy) - 2-methylpropan-2-ol was used in place of hydroxylamine hydrochloride in [Production of Compound I-1].
¹H-NMR (500 MHz, CDCl₃, δ): 5.87-5.84 (m, 1H), 5.77-5.65 (m, 2H), 5.45-5.39 (m, 2H), 4.97-4.93 (m, 1H), 4.76 (d, J = 3.4 Hz, 1H), 4.70-4.56 (m, 2H), 4.03-3.93 (m, 4H), 3.85-3.74 (m, 2H), 3.69-3.60 (m, 2H), 3.50-3.41 (m, 7H), 3.25-3.14 (m, 3H), 2.91-2.84 (m, 1H), 2.51-2.47 (m, 1H), 2.35-2.20 (m, 5H), 1.93-1.89 (m, 1H), 1.76-1.74 (m, 1H), 1.68-1.34 (m, 15H), 1.28-1.14 (m, 20H), 0.93-0.77 (m, 10H); MSm/z 962.5615 [M+H]⁺.

### Production of Compound I-106

Compound I-106 (isolated yield 19.2 mg, percent yield 69%) was obtained in a similar manner to [Production of Compound I-2] except that 1-(aminooxy)-2-methylpropan-2-ol was used in place of hydroxylamine hydrochloride.
¹H-NMR (500 MHz, CDCl₃, δ): 5.85-5.83 (m, 1H), 5.76-5.65 (m, 2H), 5.43-5.36 (m, 2H), 4.97-4.93 (m, 2H), 4.76 (d, J = 3.4 Hz, 1H), 4.69-4.63 (m, 2H), 4.01-3.95 (m, 2H), 3.92 (br s, 1H), 3.85-3.73 (m, 2H), 3.69-3.60 (m, 2H), 3.50-3.41 (m, 7H), 3.25-3.14 (m, 3H), 2.80-2.77 (dd, J = 12.6, 3.4 Hz, 1H), 2.65-2.60 (m, 1H), 2.50-2.47 (m, 1H), 2.35-2.20 (m, 5H), 1.93-1.82 (m, 3H), 1.74-1.73 (m, 1H), 1.67-1.65 (m, 1H), 1.58-1.36 (m, 13H), 1.28-1.14 (m, 20H), 0.93-0.77 (m, 10H); MSm/z 979.5811 [M+NH₄]⁺.

### Production of Compound I-107

Compound I-107 (isolated yield 94.6 mg, percent yield 86%) was obtained as a mixture of isomers that were difficult to separate in a similar manner to [Production of Compound I-1] except that 3-(aminooxy)propanoic acid hydrochloride was used in place of hydroxylamine hydrochloride.
¹H-NMR (500 MHz, CDCl₃, δ): 5.88-5.83 (m, 1H), 5.77-5.65 (m, 2H), 5.40 (d, J = 3.4 Hz, 1H), 5.45-5.38 (m, 1H), 4.95 (d, J = 10.9 Hz, 1H), 4.77 (s, 1H), 4.76 (br s,0.6H), 4.70-4.56 (m, 3H), 4.30 (t, J = 5.7 Hz, 2H), 3.96 (s, 0.4H), 3.94-3.92 (br s, 1H), 3.87-3.80 (m, 1H), 3.80-3.73 (m, 1H), 3.71-3.65 (m, 1H), 3.65-3.60 (m, 1H), 3.52-3.46 (m, 2H), 3.44 (s, 3H), 3.42 (s, 3H), 3.26-3.20 (m, 2H), 3.17 (t, J = 9.2 Hz, 1H), 2.91-2.82 (m, 2H), 2.77-2.73 (m, 2H), 2.52-2.46 (m, 1H), 2.37-2.20 (m, 5H), 1.92-1.87 (m, 3H), 1.78-1.72 (m, 1H), 1.69-1.65 (m, 1H), 1.59-1.36 (m, 14H), 1.29-1.24 (m, 6H), 1.20-1.18 (m, 1H), 1.15 (dd, J = 1.4, 7.2 Hz, 3H), 0.92 (t, J = 7.4 Hz, 3H), 0.84 (d, J = 6.9 Hz, 3H), 0.82-0.75 (m, 5H); MSm/z 979.5597 [M+NH₄]⁺.

### Production of Compound I-108

Compound I-108 (isolated yield 85.5 mg, percent yield 78%) was obtained in a similar manner to [Production of Compound I-2] except that 3-(aminooxy)propanoic acid hydrochloride was used in place of hydroxylamine hydrochloride.
¹H-NMR (500 MHz, CDCl₃, δ): 6.14 (br s, 1H), 5.84-5.81 (m, 1H), 5.78-5.64 (m, 2H), 5.44-5.36 (m, 2H), 4.94 (brd, J = 9.7 Hz, 1H), 4.89 (s, 1H), 4.78-4.74 (m, 1H), 4.70-4.60 (m, 1H), 4.35 (t, J = 5.7 Hz, 2H), 3.93 (br s, 1H), 3.86-3.79 (m, 1H), 3.79-3.73 (m, 1H), 3.70-3.59 (m, 2H), 3.51-3.45 (m, 1H), 3.45-3.43 (m, 3H), 3.42-3.41 (m, 3H), 3.27-3.19 (m, 2H), 3.16 (t, J = 9.2 Hz, 1H), 2.80-2.74 (m, 4H), 2.62 (d, J = 8.6 Hz, 1H), 2.55-2.45 (m, 3H), 2.36-2.18 (m, 5H), 1.92-1.83 (m, 3H), 1.76-1.70 (m, 1H), 1.68-1.64 (m, 1H), 1.60-1.34 (m, 10H), 1.28-1.23 (m, 6H), 1.16 (t, J = 5.7 Hz, 6H), 0.92 (t, J = 7.2 Hz, 3H), 0.83 (d, J = 6.3 Hz, 3H), 0.80-0.74 (m, 4H); MSm/z 979.5597 [M+NH₄]⁺.

### Production of Compound I-109

To a solution of Compound I-107 (25 mg, 0.026 mmol) obtained in [Production of Compound I-107] in DMF (0.26 mL), DIPEA (45 µL, 0.260 mmol), 2M methylamine THF solution (0.065 mL, 0.065 mmol) and HATU (29.7 mg, 0.078 mmol) were added, and the mixture was stirred at room temperature for 1 hour. A saturated aqueous sodium bicarbonate solution was added to the reaction solution to stop the reaction. After extraction with ethyl acetate, the organic layer was washed with saturated brine and dried over anhydrous magnesium sulfate. After the solvent was distilled off under reduced pressure, the residue was purified by preparative thin-layer silica gel column chromatography (ethyl acetate) to obtain Compound I-109 (isolated yield 16.2 mg, percent yield 64%) as a mixture of isomers that were difficult to separate.
¹H-NMR (500 MHz, CDCl₃, δ): 6.16 (br s,0.4H), 5.88-5.83 (m, 1H), 5.82 (br s,0.6H), 5.78-5.64 (m, 2H), 5.45-5.37 (m, 2H), 4.94 (brd, J = 10.9 Hz, 1H), 4.77 (s, 0.6H), 4.76 (s, 1H), 4.71-4.55 (m, 2H), 4.39-4.29 (m, 2H), 3.96 (s, 0.4H), 3.93 (br s, 1H), 3.86-3.80 (m, 1H), 3.79-3.73 (m, 1H), 3.71-3.59 (m, 2H), 3.50-3.46 (m, 1H), 3.44 (s, 3H), 3.42-3.41 (m, 3H), 3.26-3.19 (m, 2H), 3.16 (t, J = 9.2 Hz, 1H), 2.91-2.81 (m, 2H), 2.78 (d, J = 5.2 Hz, 1.2H), 2.76 (d, J = 5.2 Hz, 1.8H), 2.57 (t, J = 6.0 Hz, 1H), 2.53-2.47 (m, 2H), 2.37-2.16 (m, 6H), 1.92-1.88 (m, 1H), 1.77-1.72 (m, 1H), 1.68-1.64 (m, 1H), 1.59-1.34 (m, 15H), 1.28-1.23 (m, 8H), 1.19-1.17 (m, 1H), 1.16 (d, J = 6.9 Hz, 3H), 0.92 (t, J = 7.2 Hz, 3H), 0.85-0.82 (m, 3H), 0.80-0.75 (m, 4H); MSm/z 975.5660 [M+H]⁺.

### Production of Compound I-110

To a solution of Compound I-108 (25 mg, 0.026 mmol) obtained in [Production of Compound I-108] in DMF (0.26 mL), DIPEA (45 µL, 0.260 mmol), 2M methylamine THF solution (0.065 mL, 0.065 mmol) and HATU (29.7 mg, 0.078 mmol) were added, and the mixture was stirred at room temperature for 1 hour. A saturated aqueous sodium bicarbonate solution was added to the reaction solution to stop the reaction. After extraction with ethyl acetate, the organic layer was washed with saturated brine and dried over anhydrous magnesium sulfate. After the solvent was distilled off under reduced pressure, the residue was purified by preparative thin-layer silica gel column chromatography (ethyl acetate) to obtain Compound I-110 (isolated yield 16.2 mg, percent yield 64%).
¹H-NMR (500 MHz, CDCl₃, δ): 6.14 (br s, 1H), 5.85-5.81 (m, 1H), 5.77-5.63 (m, 2H), 5.44-5.35 (m, 2H), 4.94 (brd, J = 9.7 Hz, 1H), 4.90 (s, 1H), 4.78-4.75 (m, 1H), 4.70-4.59 (m, 2H), 4.35 (t, J = 5.7 Hz, 2H), 3.93 (br s, 1H), 3.86-3.79 (m, 1H), 3.79-3.72 (m, 1H), 3.70-3.59 (m, 2H), 3.50-3.45 (m, 1H), 3.45-3.43 (m, 3H), 3.42-3.41 (m, 3H), 3.26-3.19 (m, 2H), 3.16 (t, J = 9.2 Hz, 1H), 2.80-2.77 (m, 1H), 2.76-2.74 (m, 3H), 2.65-2.59 (m, 1H), 2.55-2.47 (m, 3H), 2.36-2.18 (m, 5H), 1.92-1.84 (m, 3H), 1.76-1.70 (m, 1H), 1.68-1.63 (m, 1H), 1.59-1.36 (m, 13H), 1.28-1.23 (m, 7H), 1.16 (t, J = 5.7 Hz, 6H), 0.92 (t, J = 7.2 Hz, 3H), 0.83 (d, J = 6.3 Hz, 3H), 0.80-0.76 (m, 4H); MSm/z 1046.5776 [M+NH₄]⁺.

### Production of Compound I-111

Compound I-111 (isolated yield 22.5 mg, percent yield 83%) was obtained in a similar manner to [Production of Compound I-110] except that 2,2,2-trifluoroethylamine hydrochloride was used in place of methylamine.
¹H-NMR (500 MHz, CDCl₃, δ): 6.47 (t, J = 6.3 Hz, 1H), 5.82 (td, J = 2.6,10.9 Hz, 1H), 5.78-5.63 (m, 2H), 5.44-5.37 (m, 1H), 5.40 (d, J = 4.6 Hz, 1H), 4.94 (brd, J = 11.5 Hz, 1H), 4.88 (s, 1H), 4.76 (d, J = 3.4 Hz, 1H), 4.64 (dq, J = 2.3, 14.1 Hz, 2H), 4.38 (t, J = 5.4 Hz, 2H), 4.04-3.96 (m, 1H), 3.93 (br s, 1H), 3.87-3.80 (m, 1H), 3.80-3.71 (m, 2H), 3.70-3.60 (m, 2H), 3.50-3.46 (m, 1H), 3.44 (s, 3H), 3.42 (s, 3H), 3.27-3.20 (m, 2H), 3.16 (t, J = 8.9 Hz, 1H), 2.80-2.75 (m, 1H), 2.68-2.62 (m, 1H), 2.60-2.56 (m, 2H), 2.53-2.47 (m, 1H), 2.36-2.31 (m, 2H), 2.29-2.20 (m, 2H), 1.93-1.84 (m, 4H), 1.76-1.71 (m, 2H), 1.68-1.64 (m, 1H), 1.60-1.36 (m, 12H), 1.28-1.24 (m, 7H), 1.17 (dd, J = 4.9, 6.6 Hz, 6H), 0.92 (t, J = 7.4 Hz, 3H), 0.83 (d, J = 6.3 Hz, 3H), 0.80-0.76 (m, 4H); MSm/z 1010.6110 [M+NH₄]⁺.

### Production of Compound I-112

Compound I-112 (isolated yield 14.0 mg, percent yield 67%) was obtained as a mixture of isomers that were difficult to separate in a similar manner to [Production of Compound I-109] except that dimethylamine was used in place of methylamine.
¹H-NMR (500 MHz, CDCl₃, δ): 5.87-5.83 (m, 1H), 5.77-5.65 (m, 2H), 5.40 (d, J = 4.0 Hz, 1H), 5.46-5.37 (m, 1H), 4.94 (brd, J = 1.7 Hz, 1H), 4.78 (s, 0.6H), 4.76 (d, J = 3.4 Hz, 1H), 4.69-4.55 (m, 2H), 4.44-4.36 (m, 2H), 3.96 (s, 0.4H) 3.93 (br s, 1H), 3.85-3.80 (m, 1H), 3.79-3.73 (m, 1H), 3.71-3.60 (m, 2H), 3.51-3.45 (m, 2H), 3.44 (s, 3H), 3.42 (s, 3H), 3.27-3.20 (m, 2H), 3.16 (t, J = 9.2 Hz, 1H), 3.01-2.92 (m, 6H), 2.91-2.86 (m, 1H), 2.75-2.70 (m, 2H), 2.52-2.46 (m, 1H), 2.36-2.19 (m, 6H), 1.93-1.87 (m, 1H), 1.78-1.74 (m, 1H), 1.68-1.60 (m, 2H), 1.59-1.35 (m, 12H), 1.29-1.23 (m, 10H), 1.19 (d, J = 7.4 Hz, 2H), 1.15 (d, J = 6.9 Hz, 3H), 0.92 (t, J = 7.4 Hz, 3H), 0.83 (d, J = 6.3 Hz, 3H), 0.82-0.75 (m, 4H); MSm/z 989.6359 [M+H]⁺.

### Production of Compound I-113

Compound I-113 (isolated yield 17.5 mg, percent yield 85%) was obtained in a similar manner to [Production of Compound I-110] except that dimethylamine hydrochloride was used in place of methylamine hydrochloride.
¹H-NMR (500 MHz, CDCl₃, δ): 5.85-5.80 (m, 1H), 5.76-5.64 (m, 2H), 5.43-5.35 (m, 2H), 4.94 (brd, J = 10.9 Hz, 1H), 4.89 (s, 1H), 4.78-4.74 (m, 1H), 4.68-4.59 (m, 2H), 4.40 (t, J = 7.2 Hz, 2H), 3.93 (br s, 1H), 3.86-3.80 (m, 1H), 3.79-3.73 (m, 1H), 3.70-3.59 (m, 2H), 3.51-3.45 (m, 1H), 3.44 (s, 3H), 3.42 (s, 3H), 3.26-3.19 (m, 2H), 3.16 (t, J = 9.2 Hz, 1H), 2.99 (br s, 3H), 2.93 (br s, 3H), 2.80-2.75 (m, 1H), 2.72 (q, J = 6.9 Hz, 2H), 2.63-2.54 (m, 1H), 2.52-2.45 (m, 1H), 2.36-2.18 (m, 5H), 1.93-1.86 (m, 2H), 1.85-1.79 (m, 1H), 1.76-1.72 (m, 1H), 1.68-1.63 (m, 1H), 1.59-1.36 (m, 13H), 1.27 (d, J = 6.3 Hz, 3H), 1.25 (d, J = 6.3 Hz, 4H), 1.15 (dd, J = 2.6, 7.2 Hz, 6H), 0.92 (t, J = 7.4 Hz, 3H), 0.83 (d, J = 6.9 Hz, 3H), 0.79-0.75 (m, 4H); MSm/z 989.6359 [M+H]⁺.

### Production of Compound I-114

5-Oxo-ivermectin B1a (50.0 mg, 0.057 mmol) was dissolved in methanol (0.57 mL), and sodium acetate (28.2 mg, 0.344 mmol) and O-(prop-2-yn-1-yl)hydroxylamine hydrochloride (37.0 mg, 0.344 mmol) were sequentially added, followed by stirring at room temperature for 24 hours. A saturated aqueous sodium hydrogen carbonate solution was added to the reaction solution, and the mixture was extracted with ethyl acetate. The organic layer was washed with saturated brine and dried over anhydrous magnesium sulfate. After the solvent was distilled off under reduced pressure, the residue was purified by silica gel column chromatography (n-hexane/ethyl acetate system) to obtain Compound I-114 (isolated yield 45.4 mg, percent yield 86%).
¹H-NMR (500 MHz, CDCl₃, δ): 5.94-5.90 (m, 1H), 5.83-5.81 (m, 1H), 5.79-5.70 (m, 2H), 5.42-5.36 (m, 2H), 4.96 (d, J = 10.9 Hz, 1H), 4.78 (d, J = 2.9 Hz, 2H), 4.77-4.64 (m, 3H), 4.58 (s, 1H), 4.00-3.90 (m, 2H), 3.85-3.73 (m, 2H), 3.69-3.59 (m, 2H), 3.50-3.46 (m, 1H), 3.43 (s, 3H), 3.42 (s, 3H), 3.38 (t, J = 2.3 Hz, 1H), 3.26-3.19 (m, 2H), 3.16 (t, J = 9.17 Hz, 1H), 2.55-2.47 (m, 1H), 2.45-2.40 (m, 1H), 2.35-2.18 (m, 4H), 1.99-1.91 (m, 4H), 1.79-1.73 (m, 1H), 1.68-1.62 (m, 1H), 1.59-1.34 (m, 13H), 1.29-1.22 (m, 7H), 1.16 (d, J = 6.9 Hz, 3H), 0.92 (t, J = 7.5 Hz, 3H), 0.86-0.81 (m, 4H), 0.78 (d, J = 5.2 Hz, 3H); MSm/z 948.5490 [M+Na]⁺.

### Production of Compound I-115

5-Oxo-ivermectin B1a (50.0 mg, 0.057 mmol) was dissolved in methanol (1.14 mL), and sodium acetate (28.2 mg, 0.344 mmol), cesium chloride heptahydrate (25.6 mg, 0.069 mmol) and O-allylhydroxylamine hydrochloride (37.0 mg, 0.344 mmol) were sequentially added at 0°C, followed by stirring at the same temperature for 3 hours. A saturated aqueous sodium hydrogen carbonate solution was added to the reaction solution, and the mixture was extracted with ethyl acetate. The organic layer was washed with saturated brine and dried over anhydrous magnesium sulfate. After the solvent was distilled off under reduced pressure, the residue was purified by silica gel column chromatography (n-hexane/ethyl acetate system) to obtain Compound I-115 (isolated yield 34.0 mg, percent yield 64%).
¹H-NMR (500 MHz, CD₃OD, δ): 6.04-5.96 (m, 1H), 5.96-5.87 (m, 3H), 5.80-5.69 (m, 1H), 5.34 (d, J = 2.9 Hz, 1H), 5.32-5.27 (m, 1H), 5.21-5.08 (m, 3H), 4.81 (d, J = 2.9 Hz, 1H), 4.71-4.59 (m, 4H), 4.51 (s, 1H), 4.01-3.96 (m, 1H), 3.91-3.85 (m, 1H), 3.75-3.60 (m, 3H), 3.44-3.41 (m, 4H), 3.40 (s, 3H), 3.34 (t, J = 2.3 Hz, 1H), 3.27 (d, J = 8.0 Hz, 1H), 3.19 (t, J = 8.9 Hz, 1H), 3.03 (t, J = 9.2 Hz, 1H), 2.70-2.61 (m, 1H), 2.34-2.26 (m, 4H), 2.15 (dd, J = 12.6 Hz, 2.9 Hz, 1H), 1.94-1.84 (m, 4H), 1.69-1.37 (m, 14H), 1.30-1.20 (m, 8H), 1.17 (d, J = 6.9 Hz, 3H), 0.97 (t, J = 7.5 Hz, 3H), 0.89 (d, J = 6.9 Hz, 3H), 0.87-0.78 (m, 4H); MSm/z 950.5767 [M+Na]⁺.

### Reference Example 17

### (1) Production of 2-(cyclopropylmethoxy)isoindoline-1,3-dione

2-Hydroxyisoindoline-1,3-dione (3.26 g, 20.0 mmol), cyclopropylmethanol (1.44 g, 20.0 mmol) and triphenylphosphine (6.30 g, 24.0 mmol) were dissolved in THF (100 mL) and cooled in ice to 0°C. Then, a 2.2M diethyl (E)-diazene-1,2-dicarboxylate toluene solution (10.9 ml, 24.0 mmol) was added dropwise, and the mixture was stirred at room temperature for 14 hours. Water was added to the reaction solution, and the mixture was extracted with ethyl acetate. The organic layer was washed with saturated brine and dried over anhydrous magnesium sulfate. After the solvent was distilled off under reduced pressure, the residue was purified by silica gel column chromatography (n-hexane/ethyl acetate system) to obtain 2-(cyclopropylmethoxy)isoindoline-1,3-dione (isolated yield 3.82 g, percent yield 88%).
¹H-NMR (500 MHz, CDCl₃, δ): 7.86-7.82 (m, 2H), 7.77-7.73 (m, 2H), 4.04 (d, J = 7.5 Hz, 2H), 1.33-1.24 (m, 1H), 0.65-0.61 (m, 2H), 0.34-0.31 (m, 2H).

### (2) Production of O-(cyclopropylmethyl)hydroxylamine hydrochloride

2-(Cyclopropylmethoxy)isoindoline-1,3-dione (3.82 g, 17.6 mmol) obtained in Reference Example 17-(1) was dissolved in methylene chloride (35.2 mL), methylhydrazine (1.11 mL, 21.1 mmol) was added, and the mixture was stirred at room temperature for 2 hours. After filtering the suspension, the organic layer was washed with a saturated aqueous sodium hydrogen carbonate solution and saturated brine, and dried over anhydrous magnesium sulfate. After the solvent was distilled off under reduced pressure, ether and a 4M hydrogen chloride 1,4-dioxane solution (5.28 mL, 21.1 mmol) were added, and the mixture was stirred at room temperature for 15 minutes. After filtering the suspension, the obtained solid was washed with ether to obtain O-(cyclopropylmethyl)hydroxylamine hydrochloride (isolated yield 0.992 g, percent yield 46%).
¹H-NMR (500 MHz, DMSO-d₆, δ): 10.9-10.6 (m, 3H), 3.81-3.78 (m, 2H), 1.10-1.02 (m, 1H), 0.58-0.55 (m, 2H), 0.31-0.27 (m, 2H).

### Production of Compound I-116

Compound I-116 (isolated yield 45.0 mg, percent yield 83%) was obtained in a similar manner to [Production of Compound I-115] except that O-(cyclopropylmethyl)hydroxylamine hydrochloride obtained in Reference Example 17-(2) was used in place of O-allylhydroxylamine hydrochloride.
¹H-NMR (500 MHz, CD₃OD, δ): 5.97-5.86 (m, 3H), 5.78-5.70 (m, 1H), 5.34 (d, J = 2.86 Hz, 1H), 5.21-5.06 (m, 2H), 4.81 (d, J = 3.4 Hz, 1H), 4.74-4.58 (m, 2H), 4.52 (s, 1H), 4.01-3.98 (m, 1H), 3.98-3.94 (m, 2H), 3.93-3.85 (m, 1H), 3.75-3.63 (m, 3H), 3.45-3.41 (m, 4H), 3.40 (s, 3H), 3.35-3.32 (m, 1H), 3.27 (d, J = 8.6 Hz, 1H), 3.19 (t, J = 8.9 Hz, 1H), 3.03 (t, J = 9.2 Hz, 1H), 2.70-2.63 (m, 1H), 2.34-2.23 (m, 4H), 2.18-2.11 (m, 1H), 1.94-1.88 (m, 4H), 1.96-1.86 (m, 1H), 1.68-1.38 (m, 14H), 1.31-1.13 (m, 11H), 0.97 (t, J = 7.5 Hz, 3H), 0.89 (d, J = 6.9 Hz, 3H), 0.87-0.79 (m, 4H), 0.56-0.49 (m, 2H), 0.30 (q, J = 5.5 Hz, 2H); MSm/z 964.5845 [M+Na]⁺.

### Production of Compound I-117

5-Oxo-ivermectin B1a (50.0 mg, 0.057 mmol) was dissolved in methanol (1.14 mL), and sodium acetate (28.2 mg, 0.344 mmol), lanthanum chloride heptahydrate (25.5 mg, 0.069 mmol) and O-isobutylhydroxylamine hydrochloride (43.2 mg, 0.344 mmol) were sequentially added at 0°C, followed by stirring at room temperature for 4 hours. A saturated aqueous sodium hydrogen carbonate solution was added to the reaction solution, and the mixture was extracted with ethyl acetate. The organic layer was washed with saturated brine and dried over anhydrous magnesium sulfate. After the solvent was distilled off under reduced pressure, the residue was purified by silica gel column chromatography (n-hexane/ethyl acetate system) and preparative thin-layer chromatography (n-hexane/acetone = 2:1) to obtain Compound I-117 (isolated yield 45.0 mg, percent yield 83%).
¹H-NMR (500 MHz, CD₃OD, δ): 5.95-5.88 (m, 3H), 5.78-5.69 (m, 1H), 5.35 (d, J = 3.44 Hz, 1H), 5.19-5.07 (m, 2H), 4.81 (d, J = 2.9 Hz, 1H), 4.66 (q, J = 13.9 Hz, 2H), 4.50 (s, 1H), 4.00 (br s, 1H), 3.91 (d, J = 6.9 Hz, 2H), 3.90-3.84 (m, 1H), 3.75-3.62 (m, 3H), 3.46-3.41 (m, 4H), 3.41-3.37 (m, 3H), 3.33 (t, J = 2.3 Hz, 1H), 3.27 (d, J = 8.0 Hz, 1H), 3.19 (t, J = 8.9 Hz, 1H), 3.03 (t, J = 9.2 Hz, 1H), 2.71-2.61 (m, 1H), 2.34-2.25 (m, 4H), 2.18-2.11 (m, 1H), 2.05-1.96 (m, 1H), 1.94-1.87 (m, 4H), 1.68-1.38 (m, 14H), 1.30-1.20 (m, 7H), 1.17 (d, J = 6.9 Hz, 3H), 0.99-0.83 (m, 13H), 0.81 (d, J = 5.7 Hz, 4H); MSm/z 966.6112 [M+Na]⁺.

### Production of Compound I-118 and Compound I-119

Compound I-114 (80.0 mg, 0.086 mmol) was added to a 3% sulfuric acid methanol aqueous solution (1.7 mL) and stirred at room temperature for 3 hours. A saturated aqueous sodium hydrogen carbonate solution was added to the reaction solution, and the mixture was extracted with chloroform. The organic layer was dried over anhydrous magnesium sulfate, and the solvent was distilled off under reduced pressure. The residue was purified by silica gel column chromatography (n-hexane/ethyl acetate system) and preparative thin-layer chromatography (chloroform/ethyl acetate = 2:1) to obtain Compound I-118 (isolated yield 17.0 mg, percent yield 31%) and Compound I-119 (isolated yield 33.4 mg, percent yield 49%), respectively.

### (Compound I-118)

¹H-NMR (500 MHz, CDCl₃, δ): 5.90-5.85 (m, 1H), 5.82-5.81 (m, 1H), 5.77-5.68 (m, 2H), 5.39-5.29 (m, 2H), 4.78 (d, J = 2.3 Hz, 2H), 4.75-4.70 (m, 1H), 4.68 (d, J = 2.3 Hz, 1H), 4.58 (s, 1H), 4.01 (br s, 1H), 3.91 (s, 1H), 3.70-3.64 (m, 1H), 3.36 (q, J = 2.4 Hz, 1H), 3.19 (dd, J = 9.2, 1.7 Hz, 1H), 2.52 (ddd, J = 9.3, 7.0, 2.6 Hz, 1H), 2.45-2.44 (m, 1H), 2.34-2.26 (m, 2H), 1.99-1.93 (m, 4H), 1.77-1.73 (m, 1H), 1.67-1.59 (m, 4H), 1.57-1.40 (m, 8H), 1.34 (t, J = 11.7 Hz, 1H), 1.17 (d, J = 6.9 Hz, 3H), 0.96 (t, J = 7.5 Hz, 3H), 0.87-0.78 (m, 7H); MSm/z 660.4483 [M+Na]⁺.

### (Compound I-119)

¹H-NMR (500 MHz, CDCl₃, δ): 5.93-5.89 (m, 1H), 5.82 (dd, J = 2.3, 1.2 Hz, 1H), 5.79-5.68 (m, 2H), 5.38 (t, J = 4.9 Hz, 1H), 4.96 (dt, J = 10.9, 1.7 Hz, 1H), 4.81 (d, J = 3.4 Hz, 1H), 4.78 (d, J = 2.3 Hz, 2H), 4.76-4.63 (m, 2H), 4.58 (s, 1H), 3.95 (br s, 1H), 3.92 (s, 1H), 3.86 (dd, J = 9.2, 6.3 Hz, 1H), 3.70-3.63 (m, 1H), 3.59-3.51 (m, 1H), 3.48 (s, 3H), 3.38 (t, J = 2.3 Hz, 1H), 3.22 (d, J = 7.5 Hz, 1H), 3.16 (t, J = 9.2 Hz, 1H), 2.60 (br s, 1H), 2.56-2.48 (m, 1H), 2.45-2.43 (m, 1H), 2.36-2.20 (m, 3H), 2.12 (s, 1H), 2.03-1.92 (m, 4H), 1.80-1.74 (m, 1H), 1.70-1.61 (m, 1H), 1.57-1.36 (m, 12H), 1.27 (d, J = 6.3 Hz, 3H), 1.15 (d, J = 6.9 Hz, 3H), 0.93 (t, J = 7.2 Hz, 3H), 0.87-0.80 (m, 4H), 0.78 (d, J = 5.7 Hz, 3H); MSm/z 804.5444 [M+Na]⁺.

### Production of Compound I-120

Compound I-120 (isolated yield 11.7 mg, percent yield 20%) was obtained in a similar manner to [Production of Compound I-117] except that O-ethylhydroxylamine was used in place of O-isobutylhydroxylamine hydrochloride.
¹H-NMR (500 MHz, CDCl₃, δ): 5.93-5.91 (dd, J = 8.0, 2.3 Hz, 1H), 5.831-5.827 (m, 1H), 5.79-5.69 (m, 2H), 5.42-5.36 (m, 2H), 4.98 (d, J = 11.5 Hz, 1H), 4.77-4.65 (m, 3H), 4.64 (s, 1H), 4.31 (t, J = 2.3 Hz, 2H), 3.99 (s, 1H), 3.94 (br s, 1H), 3.87-3.74 (m, 4H), 3.69-3.59 (m, 2H), 3.50-3.42 (m, 7H), 3.67-3.36 (m, 1H), 3.26-3.14 (m, 3H), 2.53-2.94 (m, 2H), 2.35-2.20 (m, 4H), 1.98-1.94 (m, 4H), 1.77-1.75 (m, 1H), 1.67-1.35 (m, 16H), 1.27-1.25 (m, 7H), 1.17-1.15 (m, 2H), 0.94-0.78 (m, 11H); MSm/z 949.5826 [M+NH₄]⁺.

### Production of Compound I-121

Compound I-121 (isolated yield 21.9 mg, percent yield 58%) was obtained in a similar manner to [Production of Compound I-117] except that O-(2-hydroxyethyl)hydroxylamine hydrochloride obtained in Reference Example 5-(2) was used in place of O-isobutylhydroxylamine hydrochloride.
¹H-NMR (500 MHz, CDCl₃, δ): 5.93-5.88 (m, 1H), 5.79-5.77 (m, 1H), 5.76-5.70 (m, 2H), 5.42-5.35 (m, 2H), 5.00-4.94 (m, 1H), 4.77 (d, J = 3.4 Hz, 1H), 4.74-4.63 (m, 2H), 4.60 (s, 1H), 4.39-4.29 (m, 2H), 3.94 (br s, 1H), 3.91 (s, 1H), 3.86-3.79 (m, 1H), 3.79-3.73 (m, 1H), 3.70-3.59 (m, 4H), 3.51-3.45 (m, 1H), 3.43 (s, 3H), 3.42 (s, 3H), 3.39-3.37 (m, 1H), 3.36 (s, 3H), 3.26-3.20 (m, 2H), 3.16 (t, J = 9.2 Hz, 1H), 2.55-2.46 (m, 2H), 2.36-2.30 (m, 2H), 2.30-2.18 (m, 2H), 2.00-1.95 (m, 1H), 1.93 (d, J = 2.3 Hz, 3H), 1.80-1.73 (m, 1H), 1.67-1.63 (m, 1H), 1.68-1.33 (m, 7H), 1.32-1.23 (m, 9H), 1.16 (d, J = 6.9 Hz, 3H), 0.93 (t, J = 7.2 Hz, 3H), 0.90-0.83 (m, 6H), 0.82-0.75 (m, 4H); MSm/z 946.5528 [M+H] ⁺.

### Production of Compound I-122

Compound I-122 (isolated yield 21.9 mg, percent yield 58%) was obtained in a similar manner to [Production of Compound I-117] except that O-(2-methoxyethyl)hydroxylamine hydrochloride obtained in Reference Example 5-(2) was used in place of O-isobutylhydroxylamine hydrochloride.
¹H-NMR (500 MHz, CDCl₃, δ): 5.93-5.88 (m, 1H), 5.79-5.77 (m, 1H), 5.76-5.70 (m, 2H), 5.42-5.35 (m, 2H), 5.00-4.94 (m, 1H), 4.77 (d, J = 3.4 Hz, 1H), 4.74-4.63 (m, 2H), 4.60 (s, 1H), 4.39-4.29 (m, 2H), 3.94 (br s, 1H), 3.91 (s, 1H), 3.86-3.79 (m, 1H), 3.79-3.73 (m, 1H), 3.70-3.59 (m, 4H), 3.51-3.45 (m, 1H), 3.43 (s, 3H), 3.42 (s, 3H), 3.39-3.37 (m, 1H), 3.36 (s, 3H), 3.26-3.20 (m, 2H), 3.16 (t, J = 9.2 Hz, 1H), 2.55-2.46 (m, 2H), 2.36-2.30 (m, 2H), 2.30-2.18 (m, 2H), 2.00-1.95 (m, 1H), 1.93 (d, J = 2.3 Hz, 3H), 1.80-1.73 (m, 1H), 1.67-1.63 (m, 1H), 1.68-1.33 (m, 7H), 1.32-1.23 (m, 9H), 1.16 (d, J = 6.9 Hz, 3H), 0.93 (t, J = 7.2 Hz, 3H), 0.90-0.83 (m, 6H), 0.82-0.75 (m, 4H); MSm/z 946.5528 [M+H] ⁺.

### Production of Compound I-123

Compound I-123 (isolated yield 15.9 mg, percent yield 49%) was obtained in a similar manner to [Production of Compound I-117] except that 3-(aminooxy)propan-1-ol hydrochloride obtained in Reference Example 8 was used in place of O-isobutylhydroxylamine hydrochloride.
¹H-NMR (500 MHz, CDCl₃, δ): 5.93-5.88 (m, 1H), 5.81-5.78 (m, 1H), 5.77-5.66 (m, 2H), 5.42-5.34 (m, 2H), 4.99-4.92 (m, 1H), 4.76 (d, J = 2.9 Hz, 1H), 4.73-4.68 (m, 1H), 4.66-4.61 (m, 1H), 4.54 (s, 1H), 4.36 (t, J = 5.7 Hz, 2H), 3.98-3.94 (m, 1H), 3.94-3.91 (m, 1H), 3.86-3.79 (m, 1H), 3.78-3.72 (m, 3H), 3.70-3.59 (m, 2H), 3.51-3.44 (m, 1H), 3.42 (s, 3H), 3.41 (s, 3H), 3.34 (t, J = 2.3 Hz, 1H), 3.26-3.19 (m, 2H), 3.15 (t, J = 9.2 Hz, 1H), 2.59 (br s, 1H), 2.54-2.47 (m, 1H), 2.36-2.18 (m, 4H), 2.00-1.91 (m, 6H), 1.77-1.72 (m, 2H), 1.67-1.62 (m, 1H), 1.58-1.33 (m, 11H), 1.29-1.23 (m, 7H), 1.15 (d, J = 6.9 Hz, 3H), 0.92 (t, J = 7.2 Hz, 3H), 0.86-0.83 (m, 1H), 0.84 (d, J = 6.9 Hz, 3H), 0.81-0.75 (m, 4H); MSm/z 968.5347 [M+Na]⁺.

### Production of 5-[(carboxymethoxy)imino]-ivermectin B1a

5-[(Carboxymethoxy)imino]-ivermectin B1a (isolated yield 108 mg, percent yield 83%) was obtained in a similar manner to [Production of Compound I-117] except that 2-(aminooxy)acetic acid 1/2 hydrochloride was used in place of O-isobutylhydroxylamine hydrochloride.
¹H-NMR (500 MHz, CD₃OD, δ): 6.27-6.22 (m, 1H), 6.23-6.16 (m, 1H), 6.01-5.85 (m, 2H), 5.78-5.66 (m, 1H), 5.33 (d, J = 3.4 Hz, 1H), 5.29-5.21 (m, 1H), 5.20-4.98 (m, 2H), 4.81-4.74 (m, 1H), 4.71-4.54 (m, 4H), 4.00-3.96 (m, 1H), 3.95-3.92 (m, 1H), 3.92-3.82 (m, 1H), 3.75-3.60 (m, 3H), 3.43-3.41 (m, 1H), 3.40 (s, 3H), 3.38 (s, 3H), 3.34-3.31 (m, 1H), 3.30-3.27 (m, 2H), 3.26-3.21 (m, 1H), 3.17 (t, J = 8.9 Hz, 1H), 3.01 (t, J = 8.9 Hz, 1H), 2.69-2.62 (m, 1H), 2.60-2.50 (m, 1H), 2.33-2.23 (m, 4H), 2.16-2.09 (m, 1H), 1.92-1.87 (m, 3H), 1.85-1.80 (m, 1H), 1.68-1.36 (m, 9H), 1.35-1.26 (m, 1H), 1.26-1.22 (m, 4H), 1.21 (d, J = 6.3 Hz, 4H), 1.16 (d, J = 6.9 Hz, 3H), 0.98-0.91 (m, 3H), 0.91-0.76 (m, 7H).

### Production of Compound I-124

5-[(Carboxymethoxy)imino]-ivermectin B1a (24 mg, 0.025 mmol) was dissolved in dichloromethane (0.25 mL), and DIPEA (22 µL, 0.127 mmol) and HATU (14.5 mg, 0.038 mmol) were added, followed by stirring at room temperature for 15 minutes. Then, a 2M methylamine THF solution (38 µL, 0.076 mmol) was added, followed by stirring at room temperature overnight. A saturated aqueous sodium bicarbonate solution was added to the reaction solution to stop the reaction. After extraction with ethyl acetate, the organic layer was washed with saturated brine and dried over anhydrous magnesium sulfate. After the solvent was distilled off under reduced pressure, the residue was purified by preparative thin-layer silica gel column chromatography (chloroform/methanol system) to obtain Compound I-124 (isolated yield 6.2 mg, percent yield 25%).
¹H-NMR (500 MHz, CDCl₃, δ): 7.05 (br s, 1H), 5.89-5.83 (m, 1H), 5.82-5.66 (m, 2H), 5.46-5.35 (m, 2H), 5.00-4.93 (m, 1H), 4.80-4.70 (m, 4H), 4.68-4.59 (m, 2H), 4.16-4.02 (m, 1H), 3.94 (br s, 1H), 3.88-3.79 (m, 1H), 3.79-3.72 (m, 1H), 3.70-3.58 (m, 2H), 3.50-3.45 (m, 1H), 3.44-3.42 (m, 3H), 3.42-3.41 (m, 3H), 3.38-3.32 (m, 1H), 3.28-3.19 (m, 2H), 3.16 (t, J = 9.2 Hz, 1H), 2.83-2.76 (m, 3H), 2.59-2.48 (m, 2H), 2.38-2.18 (m, 4H), 2.01-1.95 (m, 1H), 1.92 (br s, 3H), 1.80-1.72 (m, 2H), 1.71-1.34 (m, 8H), 1.29-1.21 (m, 9H), 1.16 (d, J = 5.7 Hz, 4H), 0.95-0.91 (m, 3H), 0.87-0.76 (m, 9H); MSm/z 976.5746 [M+NH₄]⁺.

### Production of Compound I-125

Compound I-125 (isolated yield 3.3 mg, percent yield 12%) was obtained in a similar manner to [Production of Compound I-124] except that ammonium chloride was used in place of methylamine.
¹H-NMR (500 MHz, CDCl₃, δ): 7.04 (q, J = 4.6 Hz, 1H), 5.97-5.92 (m, 1H), 5.87-5.84 (m, 1H), 5.81-5.68 (m, 2H), 5.43-5.34 (m, 2H), 5.00-4.93 (m, 1H), 4.79-4.60 (m, 6H), 4.13-4.06 (m, 1H), 4.10 (d, J = 6.9 Hz, 1H), 3.93 (br s, 1H), 3.85-3.78 (m, 1H), 3.77-3.71 (m, 1H), 3.70-3.57 (m, 2H), 3.49-3.43 (m, 1H), 3.42-3.41 (m, 3H), 3.41-3.39 (m, 3H), 3.35-3.32 (m, 1H), 3.25-3.18 (m, 2H), 3.17-3.11 (m, 1H), 2.55-2.47 (m, 1H), 2.36-2.17 (m, 5H), 2.03-2.01 (m, 2H), 2.00-1.94 (m, 1H), 1.91 (s, 3H), 1.79-1.73 (m, 1H), 1.67-1.62 (m, 1H), 1.59-1.31 (m, 12H), 1.28-1.21 (m, 9H), 1.15 (d, J = 6.9 Hz, 3H), 0.92 (t, J = 7.4 Hz, 2H), 0.84 (d, J = 6.9 Hz, 3H), 0.82-0.76 (m, 4H); MSm/z 962.5589 [M+H]⁺.

### Production of Compound I-126

Compound I-126 (isolated yield 5.5 mg, percent yield 13%) was obtained in a similar manner to [Production of Compound I-117] except that O-(oxetan-3-ylmethyl)hydroxylamine obtained in Reference Example 13-(2) was used in place of O-isobutylhydroxylamine hydrochloride.
¹H-NMR (500 MHz, CDCl₃, δ): 5.95-5.86 (m, 1H), 5.81-5.77 (m, 1H), 5.77-5.69 (m, 2H), 5.43-5.34 (m, 2H), 5.00-4.93 (m, 1H), 4.80-4.75 (m, 3H), 4.74-4.63 (m, 2H), 4.57-4.49 (m, 3H), 4.43 (d, J = 6.9 Hz, 2H), 4.01 (s, 1H), 3.94 (br s, 1H), 3.86-3.79 (m, 1H), 3.78-3.73 (m, 1H), 3.69-3.59 (m, 2H), 3.51-3.46 (m, 1H), 3.43 (s, 3H), 3.42 (s, 3H), 3.40-3.34 (m, 2H), 3.27-3.19 (m, 2H), 3.16 (t, J = 9.2 Hz, 1H), 2.55-2.47 (m, 2H), 2.37-2.31 (m, 2H), 2.29-2.18 (m, 2H), 1.99-1.94 (m, 1H), 1.90 (s, 3H), 1.79-1.74 (m, 1H), 1.68-1.40 (m, 7H), 1.40-1.33 (m, 2H), 1.27 (d, J = 6.3 Hz, 4H), 1.26-1.24 (m, 5H), 1.16 (d, J = 6.9 Hz, 3H), 0.93 (t, J = 7.4 Hz, 3H), 0.85 (d, J = 6.9 Hz, 4H), 0.83-0.76 (m, 5H); MSm/z 958.5528 [M+H]⁺.

### Production of Compound I-127

Compound I-127 (isolated yield 4.0 mg, percent yield 15%) was obtained in a similar manner to [Production of Compound I-89] and [Production of Compound I-92], except that 5-oxo-ivermectin B1a was used in place of 3,4α-dihydro-5-oxo-ivermectin B1a in [Production of Compound I-89].
¹H-NMR (500 MHz, CDCl₃, δ): 5.93 (td, J = 2.4, 10.6 Hz, 1H), 5.84-5.82 (m, 1H), 5.80-5.68 (m, 2H), 5.44-5.36 (m, 2H), 4.97 (brd, J = 9.7 Hz, 1H), 4.79-4.73 (m, 2H), 4.69 (s, 1H), 4.68-4.64 (m, 1H), 4.37-4.33 (m, 1H), 4.10 (d, J = 12.0 Hz, 1H), 3.94 (br s, 1H), 3.86-3.80 (m, 1H), 3.80-3.74 (m, 1H), 3.71-3.59 (m, 2H), 3.51-3.44 (m, 2H), 3.43 (s, 3H), 3.42 (s, 3H), 3.40-3.36 (m, 1H), 3.27-3.20 (m, 2H), 3.16 (t, J = 8.9 Hz, 1H), 2.56-2.49 (m, 1H), 2.37-2.19 (m, 5H), 2.00-1.93 (m, 5H), 1.79-1.74 (m, 1H), 1.68-1.64 (m, 1H), 1.60-1.34 (m, 9H), 1.27 (d, J = 6.3 Hz, 4H), 1.25 (d, J = 6.3 Hz, 5H), 1.16 (d, J = 6.9 Hz, 3H), 1.19-1.14 (m, 1H), 0.93 (t, J = 7.4 Hz, 3H), 0.88-0.84 (m, 4H), 0.84-0.75 (m, 7H), 0.63-0.60 (m, 2H); MSm/z 1089.6658 [M+NH₄]⁺.

### Production of Compound I-128

Compound I-128 (isolated yield 9.7 mg, percent yield 34%) was obtained in the same manner as in [Production of Compound I-78] except that trifluoromethylsulfonic anhydride was used in place of phenylsulfonyl chloride in [Production of Compound I-78].
¹H-NMR (500 MHz, CDCl₃, δ): 6.61 (t, J = 5.4 Hz, 1H), 5.86 (d, J = 10.9 Hz, 1H), 5.81-5.74 (m, 1H), 5.72-5.65 (m, 1H), 5.46-5.38 (m, 2H), 5.00 (s, 1H), 4.96 (br d, J = 11.5 Hz, 1H), 4.78 (d, J = 3.4 Hz, 1H), 4.74-4.65 (m, 2H), 4.32-4.19 (m, 2H), 3.95 (br s, 1H), 3.88-3.81 (m, 1H), 3.81-3.75 (m, 1H), 3.72-3.61 (m, 2H), 3.56-3.47 (m, 3H), 3.46 (s, 3H), 3.44 (s, 3H), 3.28-3.20 (m, 2H), 3.18 (t, J = 9.2 Hz, 1H), 2.80 (dd, J = 12.0, 3.4 Hz, 1H), 2.73-2.64 (m, 1H), 2.55-2.47 (m, 1H), 2.35 (dd, J = 12.3, 4.3 Hz, 2H), 2.32-2.20 (m, 2H), 1.96-1.82 (m, 4H), 1.77-1.71 (m, 1H), 1.68 (d, J = 11.5 Hz, 1H), 1.60-1.39 (m, 13H), 1.29 (d, J = 5.7 Hz, 3H), 1.28-1.25 (m, 4H), 1.18 (t, J = 6.0 Hz, 6H), 0.94 (t, J = 7.2 Hz, 3H), 0.85 (d, J = 6.9 Hz, 3H), 0.82-0.77 (m, 4H); MSm/z 1082.5459 [M+NH₄]⁺.

### Production of Compound I-129

Compound I-64 (0.180 g, 0.187 mmol) obtained in [Production of Compound I-64] was dissolved in dichloromethane (3.7 mL), and dimethyl sulfoxide (1.33 mL, 18.7 mmol) and triethylamine (0.470 mL, 3.37 mmol) were sequentially added. Then, sulfur trioxide pyridine complex (0.268 g, 1.69 mmol) was added, and the mixture was stirred at room temperature for 19 hours. Water was added to the reaction solution, the mixture was extracted with chloroform, and the organic layer was dried over anhydrous magnesium sulfate. After the solvent was distilled off under reduced pressure, the residue was purified by silica gel column chromatography (n-hexane/ethyl acetate system) to obtain Compound I-129 (isolated yield 0.167 g, percent yield 93%).
¹H-NMR (500 MHz, CDCl₃, δ): 6.49-6.39 (m, 1H), 5.97-5.83 (m, 1H), 5.82-5.67 (m, 2H), 5.54 (s, 1H), 5.48-5.37 (m, 1H), 5.00 (s, 1H), 4.97 (d, J = 9.7 Hz, 1H), 4.80 (d, J = 3.4 Hz, 1H), 4.76-4.66 (m, 2H), 4.68-4.57 (m, 2H), 4.43 (q, J = 6.3 Hz, 1H), 4.21 (dd, J = 11.5, 6.3 Hz, 1H), 3.96 (br s, 1H), 3.94-3.86 (m, 1H), 3.78-3.64 (m, 2H), 3.53 (s, 3H), 3.47 (s, 3H), 3.35 (t, J = 9.2 Hz, 1H), 3.23 (d, J = 7.5 Hz, 1H), 2.88-2.77 (m, 4H), 2.74-2.64 (m, 1H), 2.64-2.55 (m, 1H), 2.52 (m, 1H), 2.40-2.32 (m, 1H), 2.32-2.22 (m, 2H), 2.19-2.10 (m, 1H), 1.98-1.81 (m, 3H), 1.80-1.72 (m, 1H), 1.68 (d, J = 12.6 Hz, 1H), 1.62-1.39 (m, 13H), 1.34-1.23 (m, 6H), 1.20-1.14 (m, 6H), 0.94 (t, J = 7.5 Hz, 3H), 0.85 (d, J = 6.9 Hz, 3H), 0.83-0.77 (m, 4H) ; MSm/z 976.5746 [M+NH₄]⁺.

### Production of Compound I-130

Compound I-2 (50.0 mg, 0.056 mmol) obtained in [Production of Compound I-2] was dissolved in DMF (1.12 mL), and potassium carbonate (93.0 mg, 0.674 mmol), (S)-3-chloropropane-1,2-diol (0.0375 mL, 0.449 mmol) and sodium iodide (0.842 mg, 5.62 µmol) were sequentially added at room temperature, followed by stirring at 100°C for 12 days. An aqueous solution of ammonium chloride was added to the reaction solution, the mixture was extracted with ethyl acetate, and the organic layer was dried over anhydrous sodium sulfate. After the solvent was distilled off under reduced pressure, the residue was purified by preparative thin-layer chromatography (chloroform:acetone = 2:1) to obtain Compound I-130 (isolated yield 4.60 mg, percent yield 5%).
¹H-NMR (500 MHz, CDCl₃, δ): 5.87-5.83 (m, 1H), 5.79-5.66 (m, 2H), 5.46-5.38 (m, 2H), 4.99-4.92 (m, 2H), 4.78 (d, J = 3.4 Hz, 1H), 4.72-4.63 (m, 2H), 4.24-4.14 (m, 2H), 4.04-3.92 (m, 1H), 3.95 (br s, 1H), 3.88-3.81 (m, 1H), 3.78 (dq, J = 9.5, 6.2 Hz, 1H), 3.73-3.60 (m, 4H), 3.53-3.47 (m, 1H), 3.46 (s, 3H), 3.44 (s, 3H), 3.28-3.21 (m, 2H), 3.18 (t, J = 9.2 Hz, 1H), 2.83-2.77 (m, 1H), 2.69-2.61 (m, 1H), 2.55-2.45 (m, 2H), 2.38-2.20 (m, 5H), 2.15-2.10 (m, 1H), 1.94-1.84 (m, 3H), 1.77-1.73 (m, 1H), 1.70-1.37 (m, 15H), 1.31-1.25 (m, 6H), 1.21-1.14 (m, 6H), 0.94 (t, J = 7.4 Hz, 3H), 0.85 (d, J = 6.9 Hz, 3H), 0.81-0.78 (m, 4H); MSm/z 986.5457 [M+Na]⁺.

### Production of Compound I-131

Compound I-129 (0.500 g, 0.521 mmol) obtained in [Production of Compound I-129] was dissolved in methanol (10 mL), sodium borohydride (0.0592 g, 1.56 mmol) was added, and the mixture was stirred at room temperature for 30 minutes. An aqueous solution of ammonium chloride was added to the reaction solution, the mixture was extracted with ethyl acetate, and the organic layer was dried over anhydrous magnesium sulfate. After the solvent was distilled off under reduced pressure, the residue was purified by silica gel column chromatography (n-hexane/ethyl acetate system) to obtain Compound I-131 (isolated yield 0.435 g, percent yield 89%).
¹H-NMR (500 MHz, CDCl₃, δ): 6.42 (q, J = 4.6 Hz, 1H), 5.90-5.82 (m, 1H), 5.79-5.66 (m, 2H), 5.46-5.37 (m, 2H), 4.99 (s, 1H), 4.96 (d, J = 10.9 Hz, 1H), 4.77 (d, J = 3.4 Hz, 1H), 4.73-4.65 (m, 2H), 4.65-4.56 (m, 2H), 3.99-3.90 (m, 2H), 3.87-3.79 (m, 2H), 3.72-3.64 (m, 1H), 3.65-3.56 (m, 2H), 3.44 (s, 3H), 3.42 (s, 3H), 3.25 (t, J = 9.2 Hz, 1H), 3.22 (d, J = 9.2 Hz, 1H), 2.89-2.77 (m, 4H), 2.70-2.62 (m, 1H), 2.54-2.46 (m, 1H), 2.38-2.31 (m, 1H), 2.30-2.16 (m, 2H), 2.01-1.94 (m, 1H), 1.93-1.82 (m, 4H), 1.78-1.70 (m, 1H), 1.67 (d, J = 12.6 Hz, 1H), 1.62-1.34 (m, 13H), 1.31-1.21 (m, 7H), 1.17-1.11 (m, 6H), 0.93 (t, J = 7.2 Hz, 3H), 0.84 (d, J = 6.3 Hz, 3H), 0.82-0.74 (m, 4H); MSm/z 978.5912 [M+NH₄]⁺.

### Production of Compound I-132

Compound I-48 (25 mg, 0.026 mmol) obtained in [Production of Compound I-48] was dissolved in DMF (0.53 mL), and DIPEA (0.037 mL, 0.211 mmol), 5-aminopyrimidine (10 mg, 0.105 mmol), and a 50% solution of propylphosphonic anhydride in ethyl acetate (0.062 mL, 0.105 mmol) were added, followed by stirring at room temperature overnight. A saturated aqueous sodium bicarbonate solution was added to the reaction solution to stop the reaction. After extraction with a mixed solution of ethyl acetate/hexane, the organic layer was washed with saturated brine and dried over anhydrous sodium sulfate. After the solvent was distilled off under reduced pressure, the residue was purified by preparative thin-layer silica gel column chromatography (n-hexane:ethyl acetate = 1:2) to obtain Compound I-132 (isolated yield 19.5 mg, percent yield 72%).
¹H-NMR (500 MHz, CD₃OD, δ): 9.07 (s, 2H), 8.88 (s, 1H), 5.93-5.86 (m, 2H), 5.79-5.67 (m, 1H), 5.34-5.31 (m, 1H), 5.19-5.10 (m, 2H), 4.96 (s, 1H), 4.80-4.77 (m, 1H), 4.71 (d, J = 5.2 Hz, 4H), 3.98 (br s, 1H), 3.91-3.83 (m, 1H), 3.74-3.61 (m, 3H), 3.40 (s, 3H), 3.39 (s, 3H), 3.44-3.37 (m, 1H), 3.25 (br d, J = 8.6 Hz, 1H), 3.17 (t, J = 8.9 Hz, 1H), 3.01 (t, J = 9.2 Hz, 1H), 2.85-2.80 (m, 1H), 2.69-2.59 (m, 2H), 2.32-2.25 (m, 4H), 2.04-2.00 (m, 1H), 1.94-1.79 (m, 3H), 1.63-1.44 (m, 12H), 1.28-1.19 (m, 8H), 1.15 (d, J = 6.9 Hz, 3H), 1.12 (d, J = 6.3 Hz, 3H), 0.95 (t, J = 7.5 Hz, 3H), 0.86 (d, J = 6.9 Hz, 3H), 0.83-0.77 (m, 4H); MSm/z 1025.5698 [M+H]⁺.

### Production of Compound I-133

Compound I-23 (20 mg, 0.021 mmol) obtained in [Production of Compound I-23] was dissolved in dichloromethane (0.45 mL), and DIPEA (0.011 mL, 0.064 mmol) and 2,5-dioxopyrrolidin-1-yl methylcarbamate (4.4 mg, 0.026 mmol) were added, followed by stirring at room temperature for 4 hours. A saturated aqueous sodium bicarbonate solution was added to the reaction solution to stop the reaction. After extraction with chloroform, the organic layer was washed with saturated brine and dried over anhydrous sodium sulfate. After the solvent was distilled off under reduced pressure, the residue was purified by silica gel column chromatography (chloroform/methanol system) to obtain Compound I-133 (isolated yield 16.1 mg, percent yield 76%).
¹H-NMR (500 MHz, CDCl₃, δ): 5.83 (dt, J = 10.3, 2.3 Hz, 1H), 5.77-5.65 (m, 2H), 5.44-5.33 (m, 2H), 4.98-4.91 (m, 2H), 4.76 (br d, J = 3.4 Hz, 1H), 4.72-4.57 (m, 2H), 4.23-4.09 (m, 2H), 3.93 (br s, 1H), 3.86-3.79 (m, 1H), 3.79-3.72 (m, 1H), 3.71-3.57 (m, 2H), 3.43 (s, 3H), 3.41 (s, 3H), 3.52-3.43 (m, 2H), 3.29-3.18 (m, 2H), 3.16 (t, J = 9.2 Hz, 1H), 2.85-2.77 (m, 1H), 2.73 (s, 3H), 2.68-2.56 (m, 1H), 2.56-2.42 (m, 1H), 2.37-2.30 (m, 2H), 2.30-2.16 (m, 2H), 1.96-1.76 (m, 3H), 1.78-1.69 (m, 1H), 1.66 (d, J = 12.60 Hz, 1H), 1.59-1.35 (m, 13H), 1.31-1.23 (m, 8H), 1.16 (d, J = 6.87 Hz, 5H), 0.91 (t, J = 7.45 Hz, 3H), 0.83 (d, J = 6.87 Hz, 3H), 0.81-0.74 (m, 4H); MSm/z 990.5902 [M+H]⁺.

### Production of Compound I-134

Compound I-134 (isolated yield 4.6 mg, percent yield 17%) was obtained in a similar manner to [Production of Compound I-133], except that Compound I-11 obtained in [Production of Compound I-11] was used in place of Compound I-23 in [Production of Compound I-133].
¹H-NMR (500 MHz, CDCl₃, δ): 5.88-5.80 (m, 1H), 5.76-5.63 (m, 2H), 5.43-5.33 (m, 2H), 4.98-4.89 (m, 2H), 4.78-4.60 (m, 4H), 4.34-4.20 (m, 4H), 3.93 (br s, 1H), 3.86-3.79 (m, 1H), 3.79-3.72 (m, 1H), 3.71-3.58 (m, 2H), 3.52-3.38 (m, 8H), 3.27-3.13 (m, 3H), 2.81-2.73 (m, 4H), 2.64-2.54 (m, 1H), 2.53-2.46 (m, 1H), 2.36-2.30 (m, 2H), 2.28-2.19 (m, 2H), 1.95-1.78 (m, 4H), 1.78-1.71 (m, 1H), 1.69-1.63 (m, 1H), 1.59-1.34 (m, 7H), 1.31-1.23 (m, 8H), 1.19-1.10 (m, 7H), 0.96-0.88 (m, 3H), 0.87-0.72 (m, 8H); MSm/z 991.5743 [M+H]⁺.

### Production of Compound I-135

Compound I-135 (isolated yield 19.7 mg, percent yield 90%) was obtained in a similar manner to [Production of Compound I-133], except that 2-isocyanatopropane was used in place of 2,5-dioxopyrrolidin-1-yl methylcarbamate in [Production of Compound I-133].
¹H-NMR (500 MHz, CDCl₃, δ): 5.84 (dd, J = 10.31, 2.29 Hz, 1H), 5.76-5.64 (m, 2H), 5.41-5.35 (m, 2H), 4.98-4.90 (m, 2H), 4.76 (br s, 1H), 4.69-4.60 (m, 2H), 4.18-4.09 (m, 2H), 3.93 (br s, 1H), 3.84-3.72 (m, 3H), 3.69-3.59 (m, 2H), 3.49-3.38 (m, 8H), 3.38-3.32 (m, 1H), 3.26-3.12 (m, 3H), 2.84-2.77 (m, 1H), 2.66-2.57 (m, 1H), 2.53-2.46 (m, 1H), 2.35-2.18 (m, 4H), 1.94-1.81 (m, 3H), 1.73 (d, J = 10.31 Hz, 1H), 1.65 (d, J = 10.31 Hz, 1H), 1.58-1.35 (m, 11H), 1.28-1.22 (m, 7H), 1.18-1.12 (m, 6H), 1.12-1.07 (m, 6H), 0.96-0.89 (m, 3H), 0.89-0.80 (m, 4H), 0.80-0.72 (m, 4H); MSm/z 1018.6223 [M+NH₄]⁺.

### Reference Example 18

### (1) Production of (S)-2-((5-oxopyrrolidin-3-yl)oxy)isoindoline-1,3-dione

2-Hydroxyisoindoline-1,3-dione (1.63 g, 10.0 mmol), (R)-4-hydroxypyrrolidin-2-one (1.01 g, 10.0 mmol) and triphenylphosphine (3.66 g, 21.0 mmol) were dissolved in THF (100 mL) and cooled in ice to 0°C. Then, a 2.2M diethyl (E)-diazene-1,2-dicarboxylate toluene solution (9.55 mL, 21.0 mmol) was added dropwise, and the mixture was stirred at room temperature for 4 hours. Water was added to the reaction solution, and the mixture was extracted with ethyl acetate. The organic layer was washed with saturated brine and dried over anhydrous sodium sulfate. After the solvent was distilled off under reduced pressure, the residue was purified by silica gel column chromatography (n-hexane/ethyl acetate system) to obtain (S)-2-((5-oxopyrrolidin-3-yl)oxy)isoindoline-1,3-dione (isolated yield 0.300 g, percent yield 12%).
¹H-NMR (500 MHz, CDCl₃, δ): 7.88-7.85 (m, 2H), 7.80-7.78 (m, 2H), 5.12-5.08 (m, 1H), 3.83-3.81 (m, 1H), 3.74-3.70 (m, 1H), 2.80-2.69 (m, 2H).

### (2) Production of (S)-4-(aminooxy)pyrrolidin-2-one

(S)-2-((5-Oxopyrrolidin-3-yl)oxy)isoindoline-1,3-dione (isolated yield 0.300 g, 1.22 mmol) obtained in Reference Example 18-(1) was dissolved in dichloromethane (12.2 mL), methylhydrazine (0.0710 mL, 1.46 mmol) was added, and the mixture was stirred at room temperature for 2 hours. After filtering the suspension, the solvent was distilled off under reduced pressure to obtain (S)-4-(aminooxy)pyrrolidin-2-one (isolated yield 0.120 g, percent yield 85%).
¹H-NMR (500 MHz, CDCl₃, δ): 4.43-4.41 (m, 1H), 3.56-3.49 (m, 2H), 2.55-2.50 (m, 1H), 2.41-2.37 (m, 1H).

### Production of Compound I-136

Compound I-136 (isolated yield 12.8 mg, percent yield 38%) was obtained in a similar manner to [Production of Compound I-2] except that (S)-4-(aminooxy)pyrrolidin-2-one obtained in Reference Example 18-(2) was used in place of hydroxylamine hydrochloride.
¹H-NMR (500 MHz, CDCl₃, δ): 5.90-5.80 (m, 1H), 5.79-5.65 (m, 3H), 5.45-5.37 (m, 2H), 4.99-4.93 (m, 2H), 4.90-4.86 (m, 1H), 4.80-4.75 (m, 1H), 4.70-4.58 (m, 2H), 3.94 (br s, 1H), 3.87-3.81 (m, 1H), 3.81-3.73 (m, 1H), 3.71-3.60 (m, 3H), 3.58-3.46 (m, 2H), 3.45 (s, 3H), 3.43 (s, 3H), 3.28-3.20 (m, 2H), 3.17 (t, J = 9.2 Hz, 1H), 2.93-2.76 (m, 1H), 2.69-2.59 (m, 2H), 2.57-2.46 (m, 2H), 2.38-2.19 (m, 5H), 1.95-1.82 (m, 4H), 1.79-1.72 (m, 1H), 1.69-1.65 (m, 1H), 1.60-1.37 (m, 12H), 1.31-1.24 (m, 8H), 1.21-1.14 (m, 5H), 0.93 (t, J = 7.4 Hz, 3H), 0.85 (d, J = 6.9 Hz, 3H), 0.81-0.75 (m, 4H); MSm/z 973.5626 [M+H]⁺.

### Reference Example 19

### Production of O-amino-D-serine dihydrochloride

(R)-4-Aminoisoxazolidin-3-one (0.102 g, 1.00 mmol) and a 4N hydrogen chloride 1,4-dioxane solution (2.50 mL, 10.0 mmol) were dissolved in water (1 mL) and stirred at 60°C for 19 hours. By distilling off the solvent under reduced pressure, O-amino-D-serine dihydrochloride (isolated yield 0.222 g, quantitative) was obtained.
¹H-NMR (500 MHz, CDCl₃, δ): 4.60-4.51 (m, 2H), 4.49-4.47 (m, 1H).

### Production of Compound I-137

Compound I-137 (isolated yield 48.2 mg, percent yield 86%) was obtained in a similar manner to [Production of Compound I-2] except that O-amino-D-serine dihydrochloride obtained in Reference Example 19 was used in place of hydroxylamine hydrochloride.
¹H-NMR (500 MHz, CD3OD, δ): 5.94-5.82 (m, 2H), 5.78-5.69 (m, 1H), 5.34 (d, J = 2.9 Hz, 1H), 5.20-5.10 (m, 2H), 4.85 (s, 1H), 4.80 (d, J = 2.9 Hz, 1H), 4.67 (s, 2H), 4.51 (dd, J = 12.0, 3.4 Hz, 1H), 4.34 (dd, J = 12.0, 7.5 Hz, 1H), 3.99 (br s, 1H), 3.93-3.83 (m, 2H), 3.79-3.62 (m, 3H), 3.45-3.42 (m, 4H), 3.46-3.41 (m, 1H), 3.42 (m, 3H), 3.40 (s, 3H), 3.32-3.25 (m, 3H), 3.19 (t, J = 8.9 Hz, 1H), 3.03 (t, J = 9.2 Hz, 1H), 2.81 (dd, J = 12.9, 3.2 Hz, 1H), 2.70-2.56 (m, 2H), 2.34-2.25 (m, 4H), 2.06-2.01 (m, 1H), 1.95-1.79 (m, 3H), 1.43-1.40 (m, 1H), 1.71-1.37 (m, 13H), 1.33-1.20 (m, 9H), 1.20-1.13 (m, 6H), 0.96 (t, J = 7.5 Hz, 3H), 0.88 (d, J = 6.9 Hz, 3H), 0.81 (d, J = 5.7 Hz, 4H); MSm/z 977.5604

### [M+H]⁺.

### Production of Compound I-138

Compound I-138 (isolated yield 2.46 mg, percent yield 13%) was obtained in a similar manner to [Production of Compound I-132], except that in [Production of Compound I-132], Compound I-137 obtained in [Production of Compound I-137] was used in place of Compound I-48, and methylamine hydrochloride was used in place of 5-aminopyrimidine.
¹H-NMR (500 MHz, CD₃OD, δ): 5.92-5.82 (m, 2H), 5.76-5.69 (m, 1H), 5.35 (d, J = 3.4 Hz, 1H), 5.20-5.11 (m, 2H), 4.81-4.79 (m, 1H), 4.67-4.63 (m, 1H), 4.34-4.17 (m, 2H), 3.99 (br s, 1H), 3.93-3.84 (m, 1H), 3.75-3.61 (m, 4H), 3.46-3.43 (m, 1H), 3.42 (s, 3H), 3.40 (s, 3H), 3.28-3.24 (m, 1H), 3.19 (t, J = 8.9 Hz, 1H), 3.03 (t, J = 9.2 Hz, 1H), 2.83-2.78 (m, 1H), 2.77-2.72 (m, 3H), 2.69-2.52 (m, 2H), 2.34-2.26 (m, 4H), 2.07-2.01 (m, 1H), 1.92-1.75 (m, 3H), 1.67-1.38 (m, 12H), 1.31-1.24 (m, 7H), 1.22 (d, J = 6.3 Hz, 3H), 1.17 (d, J = 6.9 Hz, 3H), 1.14 (d, J = 6.3 Hz, 3H), 0.96 (t, J = 7.4 Hz, 3H), 0.88 (d, J = 6.9 Hz, 3H), 0.83-0.69 (m, 4H); MSm/z 990.5907 [M+H]⁺.

### Reference Example 20

### (1) Production of methyl (R)-2-((1,3-dioxoisoindolin-2-yl)oxy)propanoate

Methyl (R)-2-((1,3-dioxoisoindolin-2-yl)oxy)propanoate (isolated yield 1.91 g, percent yield 77%) was obtained in a similar manner to Reference Example 18-(1), except that methyl (S)-2-hydroxypropanoate was used in place of (R)-4-hydroxypyrrolidin-2-one in Reference Example 18-(1).
¹H-NMR (500 MHz, CDCl₃, δ): 7.86-7.83 (m, 2H), 7.77-7.74 (m, 2H), 4.87 (q, J = 6.9 Hz, 1H), 3.79 (s, 3H), 1.65 (d, J = 6.9 Hz, 3H).

### (2) Production of methyl (R)-2-(aminooxy)propanoate

Methyl (R)-2-(aminooxy)propanoate (isolated yield 0.892 g, percent yield 98%) was obtained in a similar manner to Reference Example 18-(2), except that methyl (R)-2-((1,3-dioxoisoindolin-2-yl)oxy)propanoate obtained in Reference Example 20-(2) was used in place of (S)-2-((5-oxopyrrolidin-3-yl)oxy)isoindoline-1,3-dione in Reference Example 18-(2).
¹H-NMR (500 MHz, CDCl₃, δ): 4.45 (q, J = 6.9 Hz, 1H), 3.80 (s, 3H), 1.47 (d, J = 7.4 Hz, 3H).

### Production of Compound I-139

Compound I-139 (isolated yield: 25.8 mg, percent yield: 46%) was obtained by a method similar to that of [Production of Compound I-2], except that methyl (R)-2-(aminooxy)propanoate obtained in Reference Example 20-(2) was used in place of hydroxylamine hydrochloride. ¹H-NMR (500 MHz, CDCl₃, δ): 5.91-5.80 (m, 1H), 5.77-5.66 (m, 2H), 5.46-5.36 (m, 2H), 5.01 (s, 1H), 4.98-4.93 (m, 1H), 4.80-4.53 (m, 5H), 3.94 (br s, 1H), 3.88-3.75 (m, 2H), 3.73 (s, 3H), 3.71-3.61 (m, 2H), 3.53-3.46 (m, 2H), 3.45 (s, 3H), 3.43 (s, 3H), 3.27-3.20 (m, 2H), 3.17 (t, J = 9.2 Hz, 1H), 2.84-2.78 (m, 1H), 2.68-2.57 (m, 1H), 2.50 (br s, 1H), 2.39-2.20 (m, 5H), 1.98-1.81 (m, 3H)1.76 (br s, 1H), 1.71-1.62 (m, 1H), 1.59-1.39 (m, 16H), 1.32-1.22 (m, 12H), 1.19-1.14 (m, 3H), 1.13-1.10 (m, 2H), 0.93 (t, J = 7.2 Hz, 3H), 0.90-0.75 (m, 9H); MSm/z 993.5910 [M+NH₄]⁺.

### Production of Compound I-140

Compound I-139 (13.8 mg, 0.0141 mmol) obtained in [Production of Compound I-139] and methylamine hydrochloride (5.73 mg, 0.0849 mmol) were dissolved in dichloromethane (0.47 mL), and then DIPEA (0.030 mL, 0.170 mmol) and 1.4M trimethylaluminum hexane solution (0.061 mL, 0.0849 mmol) were sequentially added and stirred at room temperature for 3 hours. A saturated aqueous sodium hydrogen carbonate solution was added to the reaction solution, and the mixture was extracted with ethyl acetate. The organic layer was washed with saturated brine and dried over anhydrous sodium sulfate. After the solvent was distilled off under reduced pressure, the residue was purified by preparative thin-layer chromatography (hexane:ethyl acetate = 1:2) to give Compound I-140 (isolated yield 7.35 mg, percent yield 53%).
¹H-NMR (500 MHz, CDCl₃, δ): 6.52-6.37 (m, 1H), 5.71 (s, 2H), 5.40 (d, J = 3.4 Hz, 2H), 4.94 (s, 2H), 4.73-4.60 (m, 4H), 3.93 (br s, 1H), 3.86-3.73 (m, 2H), 3.71-3.60 (m, 2H), 3.51-3.45 (m, 1H), 3.44 (s, 3H), 3.42 (s, 3H), 3.28-3.19 (m, 2H), 3.17 (t, J = 9.2 Hz, 1H), 2.79 (d, J = 5.2 Hz, 2H), 2.85-2.78 (m, 3H), 2.77-2.72 (m, 1H), 2.65-2.56 (m, 1H), 2.52-2.46 (m, 1H), 2.36-2.19 (m, 4H), 1.91 (s, 4H), 1.81-1.36 (m, 15H), 1.26 (dd, J = 10.9, 6.3 Hz, 7H), 1.16 (dd, J = 6.9, 4.6 Hz, 6H), 0.92 (t, J = 7.4 Hz, 3H), 0.84 (d, J = 6.9 Hz, 3H), 0.81-0.73 (m, 4H); MSm/z 992.6062 [M+NH₄]⁺.

### Production of Compound I-141

Compound I-48 (25 mg, 0.026 mmol) obtained in [Production of Compound I-48] was dissolved in THF (0.53 mL), after which DIPEA (0.033 mL, 0.190 mmol), 2-aminopyrazine (12 mg, 0.127 mmol), DMAP (11.6 mg, 0.095 mmol) and 1-[3-(dimethylamino)propyl]-3-ethylcarbodiimide hydrochloride (18.20 mg, 0.095 mmol) were added, followed by stirring at room temperature overnight. A saturated aqueous sodium bicarbonate solution was added to the reaction solution to stop the reaction. After extraction with a mixed solution of ethyl acetate/hexane, the organic layer was washed with saturated brine and dried over anhydrous sodium sulfate. After the solvent was distilled off under reduced pressure, the residue was purified by silica gel column chromatography (n-hexane/ethyl acetate system) to obtain Compound I-141 (isolated yield 6.1 mg, percent yield 19%).
¹H-NMR (500 MHz, CDCl₃, δ): 9.55 (d, J = 2.86 Hz, 1H), 9.04-8.99 (m, 1H), 8.38-8.33 (m, 1H), 8.32-8.26 (m, 1H), 5.93-5.85 (m, 1H), 5.80-5.68 (m, 2H), 5.43-5.35 (m, 2H), 5.10-5.02 (m, 2H), 4.98-4.92 (m, 1H), 4.80-4.73 (m, 4H), 3.95 (br s, 1H), 3.85-3.71 (m, 2H), 3.69-3.60 (m, 2H), 3.56-3.55 (m, 1H), 3.49-3.38 (m, 7H), 3.27-3.19 (m, 2H), 3.18-3.12 (m, 1H), 2.85-2.79 (m, 1H), 2.78-2.67 (m, 1H), 2.54-2.46 (m, 2H), 2.38-2.17 (m, 5H), 1.94-1.82 (m, 3H), 1.79-1.71 (m, 1H), 1.69-1.37 (m, 6H), 1.30-1.21 (m, 9H), 1.20-1.09 (m, 7H), 0.98-0.73 (m, 12H); MSm/z 1025.5698 [M+H]⁺.

### Production of Compound I-142

Compound I-142 (isolated yield 4.0 mg, percent yield 6%) was obtained as a mixture of isomers that were difficult to separate, in a similar manner to [Production of Compound I-132], except that 3-amino-1,2,4-triazole was used in place of 5-aminopyrimidine in [Production of Compound I-132].
¹H-NMR (500 MHz, CDCl₃, δ): 7.73-7.80 (m, 0.5H), 7.43-7.49 (m, 0.5H), 6.37-6.55 (m, 1H), 5.81-5.93 (m, 1H), 5.66-5.79 (m, 2H), 5.34-5.46 (m, 2H), 5.27-5.34 (m, 1H), 5.08 (d, J = 5.7 Hz, 1H), 4.89-5.02 (m, 1H), 4.81-4.85 (m, 1H), 4.74-4.81 (m, 2H), 4.59-4.74 (m, 2H), 3.89-3.98 (m, 1H), 3.74-3.85 (m, 2H), 3.60-3.71 (m, 2H), 3.47-3.52 (m, 1H), 3.43-3.47 (m, 3H), 3.40-3.42 (m, 3H), 3.19-3.28 (m, 2H), 3.12-3.19 (m, 1H), 2.69-2.84 (m, 2H), 2.59-2.68 (m, 1H), 2.46-2.53 (m, 1H), 2.19-2.36 (m, 4H), 2.03-2.08 (m, 1H), 1.80-1.96 (m, 2H), 1.71-1.79 (m, 1H), 1.61-1.68 (m, 1H), 1.36-1.59 (m, 10H), 1.23-1.29 (m9,H), 1.11-1.18 (m, 6H), 0.89-0.95 (m, 3H), 0.84 (dd, J = 6.9, 1.7 Hz, 3H), 0.76-0.80 (m, 4H); MSm/z 1014.5651 [M+H]⁺.

### Production of Compound I-143

Compound I-143 (isolated yield 31.0 mg, percent yield 94%) was obtained in a similar manner to [Production of Compound I-132], except that pyridin-3-yl-methylamine was used in place of 5-aminopyrimidine in [Production of Compound I-132].
¹H-NMR (500 MHz, CDCl₃, δ): 8.72 (br s, 1H), 8.64 (d, J = 4.6 Hz, 1H), 7.95 (d, J = 7.4 Hz, 1H), 7.48 (dd, J = 6.6 Hz, 1H), 7.30 (br s, 1H), 5.85-5.80 (m, 1H), 5.77-5.63 (m, 2H), 5.38 (d, J = 3.4 Hz, 1H), 5.35-5.29 (m, 1H), 5.00 (s, 1H), 4.98-4.86 (m, 2H), 4.76 (d, J = 2.9 Hz, 1H), 4.67-4.59 (m, 5H), 4.46 (dd, J = 15.2, 5.4 Hz, 1H), 3.93 (br s, 1H), 3.84-3.74 (m, 2H), 3.68-3.59 (m, 2H), 3.50-3.44 (m, 1H), 3.42 (s, 3H), 3.41 (s, 3H), 3.26-3.19 (m, 2H), 3.16 (t, J = 9.2 Hz, 1H), 2.73 (dd, J = 12.9, 3.2 Hz, 1H), 2.66-2.55 (m, 2H), 2.55-2.42 (m, 2H), 2.35-2.18 (m, 4H), 1.94-1.85 (m, 1H), 1.88-1.70 (m, 2H), 1.66 (d, J = 12.6 Hz, 1H), 1.57-1.35 (m, 9H), 1.29-1.21 (m, 10H), 1.14 (d, J = 6.9 Hz, 3H), 1.12-1.09 (m, 3H), 0.91 (t, J = 7.2 Hz, 3H), 0.84 (d, J = 6.9 Hz, 3H), 0.79-0.76 (m, 4H); MSm/z 1038.589 [M+H]⁺.

### Production of Compound I-144

Compound I-144 (isolated yield 303 mg, percent yield 92%) was obtained in a similar manner to [Production of Compound I-132] except that pyridin-2-yl-methylamine (0.558 mL, 0.949 mmol) was used in place of 5-aminopyrimidine in [Production of Compound I-132]. ¹H-NMR (500 MHz, CDCl₃, δ): 8.69-8.65 (m, 1H), 7.98-7.86 (m, 1H), 7.76-7.64 (m, 1H), 7.63-7.53 (m, 1H), 7.49-7.36 (m, 1H), 5.97-5.91 (m, 1H), 5.81-5.68 (m, 2H), 5.43-5.39 (m, 1H), 5.36-5.27 (m, 1H), 5.10 (s, 1H), 4.97 (d, J = 10.6 Hz, 1H), 4.87-4.80 (m, 1H), 4.78 (d, J = 3.5 Hz, 1H), 4.76-4.58 (m, 4H), 3.94 (br s, 1H), 3.88-3.81 (m, 1H), 3.80-3.74 (m, 1H), 3.72-3.60 (m, 2H), 3.54-3.47 (m, 1H), 3.46-3.44 (m, 3H), 3.43 (s, 3H), 3.30-3.21 (m, 2H), 3.18 (t, J = 9.2 Hz, 1H), 2.81 (dd, J = 12.6, 3.4 Hz, 1H), 2.70-2.61 (m, 1H), 2.56-2.48 (m, 2H), 2.38-2.17 (m, 5H), 2.06-2.04 (m, 1H), 2.02-1.95 (m, 1H), 1.94-1.71 (m, 2H), 1.68-1.65 (m, 1H), 1.59-1.31 (m, 11H), 1.31-1.24 (m, 9H), 1.17 (d, J = 6.9 Hz, 3H), 1.07 (d, J = 6.9 Hz, 3H), 0.95-0.91 (m, 3H), 0.87-0.84 (m, 3H), 0.82-0.77 (m, 4H); MSm/z 1038.5884 [M+H]⁺.

### Production of Compound I-145

Compound I-145 (isolated yield 294 mg, percent yield 91%) was obtained in a similar manner to [Production of Compound I-132] except that 1-methyl-1H-pyrazol-3-amine (0.929 mL, 1.58 mmol) was used in place of 5-aminopyrimidine in [Production of Compound I-132]. ¹H-NMR (500 MHz, CDCl₃, δ): 8.95 (br s, 1H), 7.28-7.27 (m, 1H), 6.72 (d, J = 1.7 Hz, 1H), 5.91 (dt, J = 10.3, 2.3 Hz, 1H), 5.81-5.68 (m, 2H), 5.43-5.34 (m, 2H), 5.08 (s, 1H), 4.99 (br d, J = 11.0 Hz, 1H), 4.80-4.67 (m, 5H), 3.95 (br s, 1H), 3.88-3.85 (m, 3H), 3.85-3.76 (m, 2H), 3.73-3.60 (m, 2H), 3.53-3.46 (m, 1H), 3.45 (s, 3H), 3.43 (s, 3H), 3.28-3.20 (m, 2H), 3.18 (t, J = 8.9 Hz, 1H), 2.85-2.80 (m, 1H), 2.75-2.67 (m, 1H), 2.57-2.48 (m, 2H), 2.38-2.20 (m, 5H), 2.05 (s, 1H), 1.97-1.86 (m, 3H), 1.81-1.74 (m, 1H), 1.70-1.65 (m, 1H), 1.61-1.36 (m, 10H), 1.31-1.24 (m, 8H), 1.17 (d, J = 6.9 Hz, 3H), 1.15 (d, J = 6.9 Hz, 3H), 0.94 (t, J = 7.2 Hz, 3H), 0.85 (d, J = 6.9 Hz, 3H), 0.83-0.77 (m, 4H); MSm/z 1027.5848 [M+H]⁺.

### Reference Example 21

### Production of 5-(chloromethyl)pyrimidine

To a solution of pyrimidin-5-yl-methanol (11 mg, 0.101 mmol) in dichloromethane (0.10 mL), thionyl chloride (0.011 mL, 0.152 mmol) was added, and the mixture was stirred at 0°C for 30 minutes. The solvent of the reaction solution was distilled off under reduced pressure to obtain 5-(chloromethyl)pyrimidine as a crude product.

### Production of Compound I-146

Compound I-2 (30 mg, 0.034 mmol) obtained in [Production of Compound I-2] was dissolved in DMF (0.67 mL), and then 5-(chloromethyl)pyrimidine obtained in Reference Example 21 as well as potassium carbonate (56 mg, 0.034 mmol) and sodium iodide (0.50 mg, 0.003 mmol) were added, followed by stirring at 50°C for 2 hours. The temperature was further raised to 70°C and stirred for 1 hour. A saturated aqueous sodium bicarbonate solution was added to the reaction solution to stop the reaction. After extraction with ethyl acetate, the organic layer was washed with saturated brine and dried over anhydrous sodium sulfate. After the solvent was distilled off under reduced pressure, the residue was purified by silica gel column chromatography (ethyl acetate/methanol system) to obtain Compound I-146 (isolated yield 15.0 mg, percent yield 45%).
¹H-NMR (500 MHz, CDCl₃, δ): 9.17 (s, 1H), 8.77 (s, 2H), 5.83 (dt, J = 10.5, 2.5 Hz, 1H), 5.76-5.61 (m, 2H), 5.42-5.36 (m, 2H), 5.16-5.11 (m, 2H), 4.95-4.90 (m, 2H), 4.76 (d, J = 2.9 Hz, 1H), 4.71-4.59 (m, 3H), 3.92 (br s, 1H), 3.82 (dd, J = 9.7, 6.3 Hz, 1H), 3.80-3.73 (m, 1H), 3.69-3.58 (m, 2H), 3.51-3.45 (m, 1H), 3.43 (s, 3H), 3.42 (s, 3H), 3.28-3.18 (m, 2H), 3.16 (t, J = 9.2 Hz, 1H), 2.77 (dd, J = 11.7, 3.7 Hz, 1H), 2.64-2.56 (m, 1H), 2.48 (ddd, J = 9.6, 7.0, 2.3 Hz, 1H), 2.36-2.18 (m, 6H), 1.92-1.82 (m, 3H), 1.77-1.69 (m, 1H), 1.68-1.61 (m, 1H), 1.58-1.35 (m, 9H), 1.29-1.23 (m, 8H), 1.14 (t, J = 6.9 Hz, 7H), 0.92 (t, J = 7.4 Hz, 3H), 0.83 (d, J = 6.9 Hz, 3H), 0.79-0.73 (m, 4H); MSm/z 982.5637 [M+H]⁺.

### Production of Compound I-147

Compound I-147 (isolated yield 27.7 mg, percent yield 88%) was obtained in the same manner as in [Production of Compound I-132] except that (1-methyl-1H-pyrazol-4-yl)methanamine (0.015 mL, 0.158 mmol) was used in place of 5-aminopyrimidine in [Production of Compound I-132].
¹H-NMR (500 MHz, CDCl₃, δ): 7.44 (s, 1H), 7.37 (s, 1H), 6.74 (t, J = 5.7 Hz, 1H), 5.81 (dt, J = 11.0, 2.5 Hz, 1H), 5.79-5.72 (m, 1H), 5.69-5.62 (m, 1H), 5.43-5.36 (m, 2H), 4.97-4.93 (m, 2H), 4.77 (d, J = 3.4 Hz, 1H), 4.71-4.55 (m, 5H), 4.40 (dd, J = 14.9, 6.3 Hz, 1H), 4.21 (dd, J = 14.9, 5.2 Hz, 1H), 3.93 (br s, 1H), 3.90-3.88 (m, 3H), 3.84-3.75 (m, 2H), 3.70-3.60 (m, 2H), 3.51-3.46 (m, 1H), 3.44 (s, 3H), 3.42 (s, 3H), 3.26-3.19 (m, 2H), 3.16 (t, J = 9.2 Hz, 1H), 2.74 (dd, J = 13.2, 2.9 Hz, 1H), 2.65-2.58 (m, 1H), 2.53-2.46 (m, 1H), 2.36-2.31 (m, 2H), 2.28-2.19 (m, 2H), 1.90-1.82 (m, 3H), 1.79-1.67 (m, 3H), 1.59-1.36 (m, 12H), 1.26 (dd, J = 10.9, 6.3 Hz, 7H), 1.17-1.14 (m, 3H), 1.10 (d, J = 6.9 Hz, 3H), 0.93 (t, J = 7.4 Hz, 3H), 0.84 (d, J = 6.9 Hz, 3H), 0.79-0.75 (m, 4H); MSm/z 1041.5997 [M+H]⁺.

### Production of Compound I-148

Compound I-148 (isolated yield 5.5 mg, percent yield 17%) was obtained in a similar manner to [Production of Compound I-132], except that oxazol-2-amine (13 mg, 0.158 mmol) was used in place of 5-aminopyrimidine in [Production of Compound I-132].
¹H-NMR (500 MHz, CDCl₃, δ): 9.61 (br s, 1H), 7.45 (d, J = 1.0 Hz, 1H), 7.03 (d, J = 1.1 Hz, 1H), 5.90 (dt, J = 10.9, 2.4 Hz, 1H), 5.82-5.68 (m, 2H), 5.47-5.38 (m, 2H), 5.11-5.03 (m, 2H), 4.96 (br d, J = 10.3 Hz, 1H), 4.81-4.72 (m, 4H), 3.95 (br s, 1H), 3.87-3.80 (m, 1H), 3.80-3.74 (m, 1H), 3.73-3.60 (m, 2H), 3.52-3.47 (m, 1H), 3.46 (s, 3H), 3.43 (s, 3H), 3.29-3.20 (m, 2H), 3.18 (t, J = 9.2 Hz, 1H), 2.83-2.78 (m, 1H), 2.77-2.69 (m, 1H), 2.55-2.47 (m, 2H), 2.38-2.20 (m, 4H), 1.97-1.84 (m, 3H), 1.79-1.73 (m, 1H), 1.70-1.38 (m, 11H), 1.32-1.28 (m, 4H), 1.28-1.23 (m, 5H), 1.17 (t, J = 6.9 Hz, 6H), 0.94 (t, J = 7.4 Hz, 3H), 0.90-0.75 (m, 8H); MSm/z 1014.5522 [M+H]⁺.

### Production of Compound I-149

Compound I-139 (0.728 g, 0.746 mmol) obtained in [Production of Compound I-139] was dissolved in THF (7.5 mL), 1M aqueous potassium hydroxide solution (2.24 mL, 2.24 mmol) was added, and the mixture was stirred at 0°C for 15 minutes. Then, the temperature was raised to room temperature, and the mixture was stirred for 2 hours and 45 minutes. An aqueous solution of ammonium chloride was added to the reaction solution, the mixture was extracted with ethyl acetate, and the organic layer was dried over anhydrous magnesium sulfate. After the solvent was distilled off under reduced pressure, the residue was purified by silica gel column chromatography (n-hexane/ethyl acetate system) to obtain Compound I-149 (isolated yield 0.495 g, percent yield 69%).
¹H-NMR (500 MHz, CDCl₃, δ): 5.94-5.80 (m, 1H), 5.78-5.62 (m, 2H), 5.47-5.33 (m, 2H), 4.94 (s, 1H), 4.94 (d, J = 9.0 Hz, 1H), 4.85-4.50 (m, 5H), 3.93 (br s, 1H), 3.86-3.72 (m, 2H), 3.71-3.56 (m, 2H), 3.53-3.45 (m, 1H), 3.45 (s, 3H), 3.42 (s, 3H), 3.32-3.19 (m, 2H), 3.17 (t, J = 9.5 Hz, 1H)2.96-2.85 (m, 1H), 2.81-2.69 (m, 1H), 2.68-2.57 (m, 1H), 2.55-2.42 (m, 1H), 2.39-2.17 (m, 4H), 1.97-1.81 (m, 2H), 1.78-1.70 (m, 1H), 1.70-1.61 (m, 1H), 1.59-1.34 (m, 12H), 1.30-1.20 (m, 10H), 1.18-1.10 (m, 5H), 0.92 (t, J = 7.5 Hz, 3H), 0.83 (d, J = 7.0 Hz, 3H), 0.80-0.72 (m, 4H).

### Production of Compound I-150

Compound I-150 (isolated yield 0.230 g, percent yield 58%) was obtained in a similar manner to [Production of Compound I-132], except that in [Production of Compound I-132], Compound I-149 was used in place of Compound I-48, and ammonium chloride was used in place of 5-aminopyrimidine.
¹H-NMR (500 MHz, CDCl₃, δ): 6.40 (d, J = 2.9 Hz, 1H), 5.93-5.79 (m, 1H), 5.78-5.64 (m, 2H), 5.48-5.37 (m, 3H), 4.94 (s, 1H), 4.97-4.92 (m, 2H), 4.76 (1br d, J = 3.4 Hz, 1H), 4.71-4.63 (m, 4H), 3.93 (br s, 1H), 3.85-3.70 (m, 2H), 3.70-3.58 (m, 2H), 3.51-3.46 (m, 1H), 3.44 (s, 3H), 3.42 (s, 3H), 3.26-3.19 (m, 2H), 3.16 (t, J = 9.2 Hz, 1H), 2.73 (dd, J = 12.6, 2.9 Hz, 1H), 2.64-2.53 (m, 2H), 2.53-2.46 (m, 1H), 2.36-2.29 (m, 2H), 2.29-2.19 (m, 2H), 1.97-1.81 (m, 3H), 1.77-1.71 (m, 1H), 1.69-1.63 (m, 2H), 1.58-1.37 (1m, 6H), 1.28-1.23 (m, 7H), 1.17 (d, J = 6.9 Hz, 3H), 1.15 (d, J = 6.9 Hz, 3H), 0.92 (t, J = 7.2 Hz, 3H), 0.83 (d, J = 6.9 Hz, 3H), 0.81-0.74 (m, 4H); MSm/z 978.592 [M+NH₄]⁺.

### Production of Compound I-151

Compound I-151 (isolated yield 38.2 mg, percent yield 65%) was obtained in a similar manner to [Production of Compound I-132], except that 2-(aminomethyl)aniline was used in place of 5-aminopyrimidine in [Production of Compound I-132].
¹H-NMR (500 MHz, CDCl₃, δ): 8.09 (d, J = 6.2 Hz, 1H), 7.41 (d, J = 7.4 Hz, 1H), 6.96 (t, J = 6.7 Hz, 1H), 6.53 (t, J = 6.8 Hz, 1H), 5.83-5.73 (m, 2H), 5.68-5.62 (m, 1H), 5.42-5.35 (m, 2H), 4.97-4.93 (m, 2H), 4.88-4.83 (m, 1H), 4.76 (d, J = 4.0 Hz, 1H), 4.69-4.58 (m, 4H), 4.47 (dd, J = 14.9, 7.4 Hz, 1H), 4.25 (dd, J = 14.9, 5.7 Hz, 1H), 3.93 (br s, 1H), 3.85-3.73 (m, 2H), 3.70-3.59 (m, 2H), 3.51-3.46 (m, 1H), 3.44 (s, 3H), 3.42 (s, 3H), 3.27-3.19 (m, 2H), 3.16 (t, J = 9.2 Hz, 1H), 2.75-2.70 (m, 1H), 2.63-2.46 (m, 3H), 2.36-2.14 (m, 4H), 1.92-1.86 (m, 1H), 1.84-1.79 (m, 1H), 1.74-1.64 (m, 2H), 1.60-1.38 (1m, 2H), 1.26 (dd, J = 10.3, 6.3 Hz, 7H), 1.15 (d, J = 6.9 Hz, 3H), 1.07 (d, J = 6.3 Hz, 3H), 0.93 (t, J = 7.4 Hz, 3H), 0.85 (d, J = 6.9 Hz, 3H), 0.80-0.74 (m, 4H); MSm/z 1053.6022 [M+H]⁺.

### Production of Compound I-152

Compound I-152 (isolated yield 29.8mg, percent yield 56%) was obtained in a similar manner to [Production of compound I-132] except that pyrazin-2-yl-methanamine was used in place of 5-aminopyrimidine in [Production of compound I-132].
¹H-NMR (500 MHz, CDCl₃, δ): 8.61 (s, 1H), 8.56 (s, 1H), 8.50 (d, J = 2.9 Hz, 1H), 7.31 (t, J = 6.0 Hz, 1H), 5.84 (dt, J = 10.9, 2.3 Hz, 1H), 5.76-5.67 (m, 2H), 5.41-5.34 (m, 2H), 5.03 (s, 1H), 4.96-4.92 (m, 2H), 4.81-4.75 (m, 2H), 4.71-4.61 (m, 4H), 4.47 (dd, J = 15.8, 5.4 Hz, 1H), 3.93 (br s, 1H), 3.85-3.73 (m, 2H), 3.69-3.59 (m, 2H), 3.51-3.44 (m, 1H), 3.43 (s, 3H), 3.41 (s, 3H), 3.26-3.19 (m, 2H), 3.16 (t, J = 9.2 Hz, 1H), 2.80 (dd, J = 13.2, 2.9 Hz, 1H), 2.69-2.62 (m, 1H), 2.56-2.46 (m, 2H), 2.35-2.30 (m, 2H), 2.30-2.18 (m, 2H), 1.93-1.85 (m, 2H), 1.79-1.69 (m, 2H), 1.69-1.61 (m, 1H), 1.58-1.34 (m, 1H), 1.28-1.23 (m, 7H), 1.15 (d, J = 6.9 Hz, 3H), 1.11 (d, J = 6.9 Hz, 3H), 0.91 (t, J = 7.4 Hz, 3H), 0.84 (d, J = 6.9 Hz, 3H), 0.80-0.74 (m, 4H); MSm/z 1039.5867 [M+H]⁺.

### Production of Compound I-153

Compound I-2 (200 mg, 0.224 mmol) obtained in [Production of Compound I-2] was dissolved in DMF (2.2 mL), and cesium carbonate (370 mg, 1.12 mmol) and ethyl 2-bromo-2-fluoroacetate (0.13 mL, 1.12 mmol) were added, followed by stirring at room temperature for 1 hour. Water was added to the reaction solution to stop the reaction. After extraction with a mixed solvent of ethyl acetate and hexane, the organic layer was washed with saturated brine and dried over anhydrous sodium sulfate. After the solvent was distilled off under reduced pressure, the residue was purified by silica gel column chromatography (n-hexane/ethyl acetate system) to obtain Compound I-153 (isolated yield 81.1 mg, percent yield 36%) as a mixture of isomers that were difficult to separate.
¹H-NMR (500 MHz, CDCl₃, δ): 6.13-6.08 (m, 0.5H), 6.03-5.94 (m, 0.5H), 5.88-5.82 (m, 1H), 5.78-5.66 (m, 2H), 5.44-5.37 (m, 2H), 4.96 (d, J = 12.0 Hz, 1H), 4.92 (s, 1H), 4.78 (d, J = 3.4 Hz, 1H), 4.68 (s, 2H), 4.66 (s, 0.5H), 4.56 (s, 0.5H), 4.38-4.24 (m, 2H), 3.94 (br s, 1H), 3.89-3.74 (m, 2H), 3.72-3.61 (m, 2H), 3.53-3.47 (m, 1H), 3.45 (s, 3H), 3.44 (s, 3H), 3.28-3.21 (m, 2H), 3.18 (t, J = 9.2 Hz, 1H), 2.84-2.77 (m, 1H), 2.73-2.62 (m, 1H), 2.53-2.49 (m, 2H), 2.40-2.20 (m, 4H), 1.99-1.82 (m, 3H), 1.79-1.50 (m, 10H), 1.47-1.38 (m, 3H), 1.38-1.32 (m, 3H), 1.31-1.25 (m, 8H), 1.22-1.13 (m, 6H), 0.93 (t, J = 7.4 Hz, 3H), 0.85 (d, J = 6.3 Hz, 3H), 0.81-0.76 (m, 4H); MSm/z 1011.5790 [M+NH₄]⁺.

### Production of Compound I-154

Compound I-153 (77 mg, 0.078 mmol) obtained in [Production of Compound I-153] was dissolved in THF (3.4 mL), 1M aqueous potassium hydroxide solution (0.78 mL, 0.78 mmol) was added, and the mixture was stirred at room temperature for 30 minutes. A saturated aqueous solution of ammonium chloride was added to the reaction solution to stop the reaction. After extraction with ethyl acetate, the organic layer was washed with saturated brine and dried over anhydrous sodium sulfate. After the solvent was distilled off under reduced pressure, the residue was purified by silica gel column chromatography (ethyl acetate/methanol system) to obtain Compound I-154 (isolated yield 66.6 mg, percent yield 89%) as a mixture of isomers that were difficult to separate. The ¹H-NMR spectrum below is the analytical data of the main isomer.
¹H-NMR (500 MHz, CDCl₃, δ): 5.98-5.90 (m, 1H), 5.89-5.79 (m, 2H), 5.76-5.64 (m, 2H), 5.41-5.22 (m, 3H), 5.01-4.90 (m, 2H), 4.86-4.80 (m, 1H), 4.76 (br s, 2H), 4.74-4.67 (m, 1H), 4.65-4.58 (m, 3H), 3.96-3.90 (m, 2H), 3.89-3.80 (m, 2H), 3.80-3.73 (m, 2H), 3.71-3.62 (m, 4H), 3.54-3.46 (m, 4H), 3.46-3.36 (m, 3H), 3.26-3.09 (m, 3H), 2.80-2.56 (m, 3H), 2.55-2.45 (m, 2H), 2.38-2.18 (m, 3H), 2.12-1.60 (m, 4H), 1.58-1.34 (m, 6H), 1.33-1.21 (m, 3H), 1.21-1.08 (m, 4H), 0.97-0.88 (m, 6H), 0.85-0.70 (m, 9H).

### Production of Compound I-155

Compound I-155 (isolated yield 7.1 mg, percent yield 21%) was obtained in a similar manner to [Production of Compound I-132], except that in [Production of Compound I-132], Compound I-154 obtained in [Production of Compound I-154] was used in place of Compound I-48, and methylamine hydrochloride was used in place of 5-aminopyrimidine.
¹H-NMR (500 MHz, CDCl₃, δ): 6.48-6.43 (m, 1H), 6.12-6.00 (m, 1H), 5.85 (dt, J = 10.7, 2.4 Hz, 1H), 5.78-5.65 (m, 2H), 5.43-5.36 (m, 2H), 4.96 (d, J = 10.9 Hz, 1H), 4.91 (s, 1H), 4.78 (d, J = 3.4 Hz, 1H), 4.71-4.63 (m, 2H), 3.94 (br s, 1H), 3.87-3.75 (m, 2H), 3.72-3.62 (m, 2H), 3.53-3.47 (m, 1H), 3.45 (s, 3H), 3.43 (s, 3H), 3.29-3.20 (m, 2H), 3.18 (t, J = 9.2 Hz, 1H), 2.89 (d, J = 5.2 Hz, 3H), 2.81 (dd, J = 12.6, 3.4 Hz, 1H), 2.75-2.62 (m, 1H), 2.55-2.47 (m, 1H), 2.38-2.32 (m, 2H), 2.31-2.20 (m, 2H), 2.16-1.98 (m, 2H), 1.96-1.87 (m, 4H), 1.78-1.71 (m, 1H), 1.67 (d, J = 12.6 Hz, 1H), 1.60-1.38 (m, 11H), 1.32-1.23 (m, 7H), 1.21-1.14 (m, 6H), 0.93 (t, J = 7.2 Hz, 3H), 0.88-0.82 (m, 3H), 0.82-0.75 (m, 4H) ; MSm/z 996.5801 [M+NH₄]⁺.

### Production of Compound I-156

Compound I-156 (isolated yield 7.0 mg, percent yield 20%) was obtained in a similar manner to [Production of Compound I-132], except that in [Production of Compound I-132], Compound I-154 obtained in [Production of Compound I-154] was used in place of Compound I-48, and ammonium chloride was used in place of 5-aminopyrimidine.
¹H-NMR (500 MHz, CDCl₃, δ): 6.40 (br S,1H), 6.11-5.99 (m, 1H), 5.88-5.82 (m, 1H), 5.82-5.66 (m, 3H), 5.45-5.36 (m, 2H), 4.96 (d, J = 10.9 Hz, 1H), 4.91 (s, 1H), 4.78 (d, J = 3.4 Hz, 1H), 4.73-4.63 (m, 2H), 3.94 (br s, 1H), 3.87-3.74 (m, 2H), 3.71-3.57 (m, 2H), 3.53-3.46 (m, 1H), 3.45 (s, 3H), 3.43 (s, 3H), 3.31-3.20 (m, 2H), 3.18 (t, J = 9.2 Hz, 1H), 2.81 (dd, J = 12.6, 3.4 Hz, 1H), 2.75-2.61 (m, 1H), 2.59-2.43 (m, 1H), 2.37-2.18 (m, 4H), 2.07-1.78 (m, 6H), 1.77-1.70 (m, 2H), 1.67 (d, J = 12.6 Hz, 2H), 1.60-1.38 (m, 10H), 1.31-1.24 (m, 6H), 1.23-1.12 (m, 6H), 0.93 (t, J = 7.4 Hz, 3H), 0.89-0.82 (m, 3H), 0.82-0.74 (m, 4H); MSm/z 982.5658 [M+NH₄]⁺.

### Reference Example 22

### Production of methyl (S)-2-(p-toluenesulfonyloxy)butanoate

Methyl (S)-2-hydroxybutanoate (300 µL, 2.67 mmol) was dissolved in acetonitrile (5.3 mL), and triethylamine (0.74 mL, 5.33 mmol), methylamine hydrochloride (25.5 mg, 0.267 mmol) and p-toluenesulfonyl chloride (760 mg, 4.00 mmol) were sequentially added, followed by stirring at room temperature for 1 hour and 30 minutes. Water was added to the reaction solution to stop the reaction. After extraction with ethyl acetate, the organic layer was washed with saturated brine and dried over anhydrous sodium sulfate. After the solvent was distilled off under reduced pressure, the residue was purified by silica gel column chromatography (n-hexane:ethyl acetate system) to obtain methyl (S)-2-(p-toluenesulfonyloxy)butanoate (isolated yield 712 mg, percent yield 98%).
¹H-NMR (500 MHz, CDCl₃, δ): 7.86-7.79 (m, 2H), 7.35 (d, J = 8.0 Hz, 2H), 4.80 (dd, J = 7.4, 5.2 Hz, 1H), 3.67 (s, 3H), 2.46 (s, 3H), 1.92-1.79 (m, 2H), 0.91 (t, J = 7.4 Hz, 3H).

### Production of Compound I-157

Compound 1-157 (isolated yield 91.0 mg, percent yield 82%) was obtained as a mixture of isomers that were difficult to separate in a similar manner to [Production of Compound I-153], except that methyl (S)-2-(p-toluenesulfonyloxy)butanoate obtained in Reference Example 22 was used in place of ethyl 2-bromo-2-fluoroacetate in [Production of Compound 1-153]. The ¹H-NMR spectrum below is the analytical data of the main isomer.
¹H-NMR (500 MHz, CDCl₃, δ): 5.89-5.79 (m, 1H), 5.79-5.64 (m, 2H), 5.44-5.32 (m, 2H), 5.01 (s, 1H), 4.96 (d, J = 11.5 Hz, 1H), 4.78 (d, J = 2.9 Hz, 1H), 4.71-4.65 (m, 2H), 4.64 (s, 1H), 4.57 (t, J = 6.6 Hz, 1H), 3.95 (br s, 1H), 3.88-3.76 (m, 2H), 3.74 (s, 3H), 3.72-3.61 (m, 2H), 3.53-3.47 (m, 1H), 3.45 (s, 3H), 3.44 (s, 3H), 3.29-3.14 (m, 3H), 2.85-2.75 (m, 1H), 2.68-2.58 (m, 2H), 2.55-2.47 (m, 2H), 2.39-2.20 (m, 5H), 1.93-1.81 (m, 5H), 1.77-1.59 (m, 13H), 1.57-1.48 (m, 6H), 1.48-1.40 (m, 1H), 1.31-1.25 (m, 6H), 1.19-1.09 (m, 4H), 0.98 (t, J = 7.4 Hz, 2H), 0.96-0.91 (m, 2H), 0.89-0.77 (m, 4H).

### Production of Compound 1-158

Compound 1-158 (isolated yield 65.6 mg, percent yield 77%) was obtained as a mixture of isomers that were difficult to separate in a similar manner to [Production of Compound I-154], except that Compound I-157 obtained in [Production of Compound I-157] was used in place of Compound I-153 in [Production of Compound I-154]. The ¹H-NMR spectrum below is the analytical data of the main isomer.
¹H-NMR (500 MHz, CD₃OD, δ): 5.93-5.80 (m, 2H), 5.76-5.69 (m, 1H), 5.35 (d, J = 3.4 Hz, 1H), 5.17-5.11 (m, 2H), 4.90 (s, 1H), 4.80 (d, J = 3.4 Hz, 1H), 4.65 (d, J = 1.7 Hz, 2H), 4.47 (dd, J = 6.9, 5.7 Hz, 1H), 3.99 (s, 1H), 3.92-3.84 (m, 1H), 3.75-3.63 (m, 3H), 3.42 (s, 3H), 3.41 (s, 3H), 3.46-3.38 (m, 7H), 3.28-3.24 (m, 1H), 3.19 (t, J = 8.9 Hz, 1H), 3.03 (t, J = 9.2 Hz, 1H), 2.82 (dd, J = 12.9, 3.2 Hz, 1H), 2.69-2.55 (m, 2H), 2.34-2.26 (m, 4H), 2.06-2.01 (m, 1H), 1.92-1.77 (m, 4H), 1.68-1.40 (m, 10H), 1.30-1.20 (m, 8H), 1.19-1.15 (m, 3H), 1.11 (d, J = 6.9 Hz, 3H), 1.02-0.93 (m, 6H), 0.88 (d, J = 6.9 Hz, 3H), 0.85-0.77 (m, 4H).

### Production of Compound I-159

Compound 1-159 (isolated yield 36.8 mg, percent yield 56%) was obtained in a similar manner to [Production of Compound 1-132], except that in [Production of Compound 1-132], Compound I-158 obtained in [Production of Compound I-158] was used in place of Compound I-48, and methylamine hydrochloride was used in place of 5-aminopyrimidine.
¹H-NMR (500 MHz, CDCl₃, δ): 6.52 (q, J = 5.2 Hz, 1H), 5.88 (dt, J = 10.7, 2.4 Hz, 1H), 5.79-5.67 (m, 2H), 5.45-5.39 (m, 2H), 4.98-4.93 (m, 2H), 4.77 (d, J = 3.4 Hz, 1H), 4.71 (d, J = 2.3 Hz, 2H), 4.69 (s, 1H), 4.56 (dd, J = 8.0, 4.0 Hz, 1H), 3.95 (br s, 1H), 3.86-3.75 (m, 2H), 3.72-3.61 (m, 2H), 3.53-3.46 (m, 1H), 3.46-3.44 (m, 3H), 3.44-3.42 (m, 3H), 3.27-3.20 (m, 2H), 3.18 (t, J = 9.2 Hz, 1H), 2.80 (d, J = 5.2 Hz, 3H), 2.75 (dd, J = 12.6, 2.9 Hz, 1H), 2.64-2.55 (m, 2H), 2.54-2.47 (m, 1H), 2.38-2.21 (m, 4H), 1.99-1.83 (m, 4H), 1.81-1.71 (m, 2H), 1.71-1.64 (m, 4H), 1.60-1.38 (m, 7H), 1.31-1.23 (m, 8H), 1.20-1.14 (m, 6H), 0.98-0.91 (m, 6H), 0.90-0.77 (m, 8H); MSm/z 989.5950 [M+H]⁺.

### Production of Compound 1-160

Compound 1-160 (isolated yield 38.8 mg, percent yield 53%) was obtained in a similar manner to [Production of Compound I-132], except that in [Production of Compound I-132], Compound I-158 obtained in [Production of Compound I-158] was used in place of Compound I-48, and ammonium chloride was used in place of 5-aminopyrimidine.
¹H-NMR (500 MHz, CDCl₃, δ): 8.04 (br s, 2H), 6.80 (d, J = 2.9 Hz, 1H), 6.72 (d, J = 2.9 Hz, 1H), 5.88 (dt, J = 10.7, 2.4 Hz, 1H), 5.80-5.65 (m, 2H), 5.47-5.35 (m, 2H), 4.98-4.91 (m, 2H), 4.78 (d, J = 3.4 Hz, 1H), 4.71-4.64 (m, 2H), 4.57 (dd, J = 7.7, 4.3 Hz, 1H), 3.94 (br s, 1H), 3.87-3.74 (m, 2H), 3.72-3.59 (m, 2H), 3.53-3.47 (m, 2H), 3.45 (s, 3H), 3.43 (s, 3H), 3.28-3.20 (m, 2H), 3.18 (t, J = 9.2 Hz, 1H), 2.73 (dd, J = 12.6, 2.9 Hz, 1H), 2.64-2.57 (m, 1H), 2.55-2.46 (m, 1H), 2.37-2.21 (m, 4H), 1.99-1.79 (m, 5H), 1.78-1.73 (m, 1H), 1.67 (d, J = 13.2 Hz, 1H), 1.60-1.38 (m, 15H), 1.30-1.24 (m, 6H), 1.22-1.13 (m, 3H), 1.00 (t, J = 7.4 Hz, 3H), 0.93 (t, J = 7.2 Hz, 3H), 0.84 (d, J = 6.3 Hz, 3H), 0.82-0.75 (m, 4H); MSm/z 992.6063 [M+NH₄]⁺.

### Production of Compound 1-161

Compound 1-150 (15.0 mg, 0.016 mmol) obtained in [Production of Compound I-150] was dissolved in dichloromethane (0.32 mL), and sodium hydrogen carbonate (7.87 mg, 0.094 mmol) and Dess-Martin Periodinane reagent (19.9 mg, 0.047 mmol) were sequentially added, followed by stirring at room temperature for 2 hours. Water was added to the reaction solution, the mixture was extracted with chloroform, and the organic layer was dried over anhydrous magnesium sulfate. After the solvent was distilled off under reduced pressure, the residue was purified by thin-layer preparative silica gel column chromatography (n-hexane:ethyl acetate = 1:2) to obtain Compound I-161 (isolated yield 9.0 mg, percent yield 60%).
¹H-NMR (500 MHz, CDCl₃, δ): 6.45 (d, J = 2.3 Hz, 1H), 5.92-5.82 (m, 1H), 5.80-5.65 (m, 3H), 5.57-5.49 (m, 1H), 5.48-5.36 (m, 1H), 4.99-4.92 (m, 2H), 4.84-4.74 (m, 1H), 4.74-4.60 (m, 3H), 4.41 (q, J = 6.3 Hz, 1H), 4.20 (dd, J = 12.0, 6.3 Hz, 1H), 3.94 (br s, 1H), 3.89 (dq, J = 9.8, 6.3 Hz, 1H), 3.74-3.63 (m, 2H), 3.50 (s, 3H), 3.46 (s, 3H), 3.39-3.30 (m, 1H), 3.21 (d, J = 7.4 Hz, 1H), 2.73 (dd, J = 12.6, 2.9 Hz, 1H), 2.67-2.55 (m, 2H), 2.55-2.45 (m, 1H), 2.38-2.31 (m, 1H), 2.31-2.22 (m, 2H), 2.13 (td, J = 12.3, 4.0 Hz, 1H), 2.07 (br s, 1H), 1.98-1.83 (m, 3H), 1.77-1.70 (m, 1H), 1.68-1.63 (m, 1H), 1.58-1.37 (m, 14H), 1.29-1.23 (m, 7H), 1.19-1.14 (m, 5H), 0.92 (t, J = 7.4 Hz, 3H), 0.85-0.82 (m, 3H), 0.81-0.73 (m, 4H); MSm/z 959.5489 [M+H]⁺.

### Production of Compound 1-162

Compound 1-162 (isolated yield 28.0 mg, percent yield 67%) was obtained in a similar manner to [Production of Compound 1-132], except that O-ethylhydroxylamine hydrochloride was used in place of 5-aminopyrimidine in [Production of Compound I-132].
¹H-NMR (500 MHz, CDCl₃, δ): 9.20 (s, 1H), 5.87-5.81 (m, 1H), 5.79-5.64 (m, 2H), 5.44-5.36 (m, 2H), 4.96-4.90 (m, 2H), 4.88 (s, 1H), 4.76 (br s, 1H), 4.74-4.61 (m, 4H), 3.99-3.89 (m, 3H), 3.85-3.79 (m, 1H), 3.79-3.72 (m, 1H), 3.70-3.59 (m, 2H), 3.52-3.45 (m, 1H), 3.43 (s, 3H), 3.41 (s, 3H), 3.26-3.18 (m, 2H), 3.16 (t, J = 9.2 Hz, 1H), 2.77 (d, J = 11.5 Hz, 1H), 2.69-2.55 (m, 2H), 2.54-2.44 (m, 1H), 2.36-2.18 (m, 4H), 1.96-1.78 (m, 1H), 1.78-1.71 (m, 2H), 1.66 (d, J = 10.9 Hz, 1H), 1.58-1.35 (m, 12H), 1.30-1.21 (m, 11H), 1.20-1.10 (m, 6H), 0.95-0.89 (m, 3H), 0.85-0.81 (m, 3H), 0.80-0.74 (m, 4H):MSm/z 1008.6008 [M+NH₄]⁺.

### Production of Compound 1-163

Compound 1-163 (isolated yield 167 mg, percent yield 74%) was obtained as a mixture of isomers that were difficult to separate in a similar manner to [Production of Compound I-153], except that methyl 2-bromo-3-methoxypropanoate was used in place of ethyl 2-bromo-2-fluoroacetate in [Production of Compound I-153].
¹H-NMR (500 MHz, CDCl₃, δ): 5.93-5.81 (m, 1H), 5.78-5.66 (m, 2H), 5.47-5.30 (m, 2H), 5.06 (s, 0.5H), 5.02 (s, 0.5H), 5.00-4.91 (m, 1H), 4.89-4.86 (m, 0.5H), 4.85-4.82 (m, 0.5H), 4.78 (d, J = 4.0 Hz, 1H), 4.67 (br s, 2H), 4.62 (s, 0.5H), 4.51 (s, 0.5H), 3.94 (br s, 1H), 3.87-3.79 (m, 3H), 3.79-3.74 (m, 4H), 3.73-3.61 (m, 2H), 3.53-3.48 (m, 1H), 3.47-3.42 (m, 6H), 3.42-3.36 (m, 3H), 3.29-3.14 (m, 3H), 2.85-2.76 (m, 1H), 2.69-2.57 (m, 1H), 2.54-2.44 (m, 2H), 2.37-2.19 (m, 4H), 1.96-1.81 (m, 3H), 1.79-1.72 (m, 1H), 1.69-1.59 (m, 2H), 1.57-1.35 (m, 11H), 1.31-1.24 (m, 8H), 1.19-1.15 (m, 3H), 1.15-1.10 (m, 3H), 0.93 (t, J = 7.4 Hz, 3H), 0.85 (d, J = 6.9 Hz, 3H), 0.81-0.75 (m, 3H).

### Production of Compound I-164

Compound 1-164 (isolated yield 57.9 mg, percent yield 71%) was obtained as a mixture of isomers that were difficult to separate, in a similar manner to [Production of Compound I-154], except that Compound I-163 obtained in [Production of Compound I-163] was used in place of Compound I-153 in [Production of Compound I-154].
¹H-NMR (500 MHz, CD₃OD, δ): 5.91-5.79 (m, 1H), 5.79-5.58 (m, 2H), 5.43-5.26 (m, 2H), 5.03-4.88 (m, 1H), 4.84-4.56 (m, 4H), 3.94 (br s, 1H), 3.87-3.70 (m, 4H), 3.69-3.58 (m, 2H), 3.52-3.32 (m, 10H), 3.27-3.12 (m, 3H), 2.70-2.60 (m, 1H), 2.56-2.45 (m, 2H), 2.40-2.18 (m, 3H), 1.92-1.34 (m, 30H), 1.30-1.23 (m, 8H), 1.22-1.11 (m, 7H), 0.92 (t, J = 7.4 Hz, 3H), 0.86-0.73 (m, 7H).

### Production of Compound 1-165

Compound I-165 (isolated yield 7.8 mg, percent yield 29%) was obtained in a similar manner to [Production of Compound I-132], except that in [Production of Compound I-132], Compound I-164 obtained in [Production of Compound I-164] was used in place of Compound I-48, and methylamine hydrochloride was used in place of 5-aminopyrimidine.
¹H-NMR (500 MHz, CDCl₃, δ): 6.64 (q, J = 4.6 Hz, 1H), 5.88 (dt, J = 10.7, 2.4 Hz, 1H), 5.79-5.65 (m, 2H), 5.44-5.38 (m, 2H), 5.00 (s, 1H), 4.96 (d, J = 10.9 Hz, 1H), 4.79-4.76 (m, 2H), 4.74-4.67 (m, 2H), 4.65 (s, 1H), 3.95 (br s, 1H), 3.87-3.74 (m, 4H), 3.72-3.59 (m, 2H), 3.53-3.46 (m, 1H), 3.45 (s, 3H), 3.43 (s, 3H), 3.36 (s, 3H), 3.28-3.20 (m, 2H), 3.18 (t, J = 9.2 Hz, 1H), 2.82 (d, J = 5.2 Hz, 3H), 2.75 (dd, J = 12.6, 2.9 Hz, 1H), 2.67-2.58 (m, 1H), 2.58-2.47 (m, 2H), 2.38-2.20 (m, 4H), 2.00-1.81 (m, 3H), 1.78-1.63 (m, 6H), 1.60-1.39 (m, 8H), 1.32-1.23 (m, 7H), 1.17 (t, J = 6.6 Hz, 6H), 0.93 (t, J = 7.2 Hz, 3H), 0.87-0.76 (m, 7H); MSm/z 1005.5908

### [M+H]⁺.

### Production of Compound I-166

Compound 1-166 (isolated yield 7.3 mg, percent yield 24%) was obtained in a similar manner to [Production of Compound I-132], except that in [Production of Compound I-132], Compound I-164 obtained in [Production of Compound I-164] was used in place of Compound I-48, and ammonium chloride (8.2 mg, 0.157 mmol) was used in place of 5-aminopyrimidine.
¹H-NMR (500 MHz, CDCl₃, δ) : 6.63 (d, J = 2.9 Hz, 1H), 5.89-5.85 (m, 1H), 5.79-5.66 (m, 2H), 5.53 (d, J = 2.9 Hz, 1H), 5.46-5.36 (m, 1H), 5.00 (s, 1H), 4.96 (d, J = 11.5 Hz, 1H), 4.81-4.76 (m, 2H), 4.71-4.62 (m, 3H), 3.95 (br s, 1H), 3.87-3.75 (m, 4H), 3.71-3.59 (m, 2H), 3.53-3.46 (m, 1H), 3.45 (s, 3H), 3.43 (s, 3H), 3.38 (s, 3H), 3.29-3.20 (m, 2H), 3.18 (t, J = 9.2 Hz, 1H), 2.74 (dd, J = 12.6, 2.9 Hz, 1H), 2.67-2.58 (m, 1H), 2.55-2.46 (m, 1H), 2.39-2.31 (m, 2H), 2.31-2.18 (m, 2H), 2.03-1.81 (m, 4H), 1.79-1.63 (m, 8H), 1.60-1.39 (m, 14H), 1.31-1.24 (m, 3H), 1.23-1.12 (m, 3H), 0.93 (t, J = 7.4 Hz, 3H), 0.89-0.82 (m, 3H), 0.82-0.75 (m, 4H); MSm/z 991.5753 [M+H]⁺.

### Production of Compound I-167

Compound I-2 (200 mg, 0.225 mmol) obtained in [Production of Compound I-2] was dissolved in THF (2.30 mL), and sodium hydride (9.00 mg, 0.225 mmol) and methyl (E)-2-butenoate (23.7 µL, 0.225 mmol) were sequentially added at 0°C, followed by stirring at 40°C overnight. A saturated aqueous sodium chloride solution was added to the reaction solution, the mixture was extracted with ethyl acetate, and the organic layer was dried over anhydrous sodium sulfate. After the solvent was distilled off under reduced pressure, the residue was purified by silica gel column chromatography (n-hexane/ethyl acetate system) to obtain Compound I-167 (isolated yield 41.3 mg, percent yield 19%) as a mixture of isomers that were difficult to separate.
¹H-NMR (500 MHz, CDCl₃, δ): 5.87-5.80 (m, 1H), 5.77-5.64 (m, 2H), 5.45-5.35 (m, 2H), 4.96 (d, J = 9.7 Hz, 1H), 4.91 (dd, J = 8.9, 2.0 Hz, 1H), 4.77 (d, J = 3.4 Hz, 1H), 4.68-4.59 (m, 3.5H), 4.54 (d, J = 6.3 Hz, 0.5H), 3.94 (br s, 1H), 3.87-3.75 (m, 2H), 3.73-3.60 (m, 4H), 3.53-3.46 (m, 1H), 3.45 (s, 3H), 3.44 (s, 3H), 3.27-3.20 (m, 2H), 3.18 (t, J = 9.5 Hz, 1H), 2.84-2.75 (m, 2H), 2.64-2.55 (m, 1H), 2.53-2.47 (m, 2H), 2.45-2.40 (m, 1H), 2.38-2.21 (m, 4H), 1.93-1.81 (m, 3H), 1.78-1.73 (m, 1H), 1.67 (d, J = 12.0 Hz, 1H), 1.62-1.46 (m, 12H), 1.46-1.36 (m, 3H), 1.33-1.23 (m, 8H), 1.16 (d, J = 6.3 Hz, 6H), 0.93 (t, J = 7.4 Hz, 3H), 0.86-0.82 (m, 3H), 0.82-0.75 (m, 4H).

### Production of Compound 1-168

Compound 1-168 (isolated yield 25.7 mg, percent yield 63%) was obtained as a mixture of isomers that were difficult to separate in a similar manner to [Production of Compound 1-154], except that Compound I-167 obtained in [Production of Compound I-167] was used in place of Compound I-153 in [Production of Compound I-154]. The ¹H-NMR spectrum below is the analytical data of the main isomer.
¹H-NMR (500 MHz, CDCl₃, δ): 5.89-5.79 (m, 1H), 5.79-5.65 (m, 2H), 5.46-5.33 (m, 2H), 5.00-4.93 (m, 1H), 4.92 (d, J = 2.3 Hz, 1H), 4.78 (d, J = 2.9 Hz, 1H), 4.67 (d, J = 1.1 Hz, 3H), 3.98-3.92 (m, 1H), 3.88-3.76 (m, 2H), 3.73-3.61 (m, 2H), 3.53-3.47 (m, 1H), 3.45 (s, 3H), 3.44 (s, 3H), 3.28-3.21 (m, 2H), 3.18 (t, J = 9.2 Hz, 1H), 2.88-2.75 (m, 2H), 2.68-2.47 (m, 4H), 2.37-2.20 (m, 4H), 1.94-1.83 (m, 3H), 1.76-1.43 (m, 14H), 1.36-1.33 (m, 3H), 1.30-1.25 (m, 8H), 1.21-1.14 (m, 6H), 0.93 (t, J = 7.4 Hz, 3H), 0.85 (d, J = 6.3 Hz, 3H), 0.81-0.78 (m, 4H).

### Production of Compound I-169 and Compound I-170

Compound 1-169 (isolated yield 7.4 mg, percent yield 29%) and Compound I-170 (isolated yield 7.3 mg, percent yield 28%) were obtained in a similar manner to [Production of Compound I-132], except that in [Production of Compound 1-132], Compound I-168 obtained in [Production of Compound 1-168] was used in place of Compound I-48, and ammonium chloride was used in place of 5-aminopyrimidine.

### (Compound I-169)

¹H-NMR (500 MHz, CDCl₃, δ): 6.24 (br s, 1H), 5.86 (dt, J = 10.5, 2.5 Hz, 1H), 5.79-5.64 (m, 3H), 5.46-5.36 (m, 2H), 4.96 (d, J = 10.9 Hz, 1H), 4.90 (s, 1H), 4.78 (d, J = 3.4 Hz, 1H), 4.70-4.61 (m, 2H), 4.61-4.54 (m, 1H), 3.94 (br s, 1H), 3.86-3.75 (m, 2H), 3.71-3.61 (m, 2H), 3.52-3.46 (m, 1H), 3.45 (s, 3H), 3.43 (s, 3H), 3.27-3.20 (m, 2H), 3.18 (t, J = 9.2 Hz, 1H), 2.76 (dd, J = 12.9, 3.2 Hz, 1H), 2.66-2.58 (m, 1H), 2.56-2.53 (m, 2H), 2.52-2.47 (m, 1H), 2.38-2.19 (m, 5H), 1.97-1.82 (m, 3H), 1.78-1.72 (m, 1H), 1.67 (d, J = 12.6 Hz, 1H), 1.60-1.38 (m, 13H), 1.33 (d, J = 6.9 Hz, 3H), 1.31-1.24 (m, 7H), 1.20 (d, J = 6.9 Hz, 3H), 1.16 (d, J = 7.4 Hz, 3H), 0.93 (t, J = 7.4 Hz, 3H), 0.85 (d, J = 6.3 Hz, 3H), 0.82-0.76 (m, 4H); MSm/z 997.5613 [M+Na]⁺.

### (Compound 1-170)

¹H-NMR (500 MHz, CDCl₃, δ): 6.03 (br s, 1H), 5.87-5.80 (m, 1H), 5.78-5.65 (m, 2H), 5.47 (br s, 1H), 5.44-5.37 (m, 2H), 4.98-4.91 (m, 2H), 4.78 (d, J = 3.4 Hz, 1H), 4.70-4.59 (m, 3H), 3.94 (br s, 1H), 3.87-3.75 (m, 2H), 3.71-3.60 (m, 2H), 3.54-3.47 (m, 1H), 3.45 (s, 3H), 3.44 (s, 3H), 3.27-3.20 (m, 2H), 3.18 (t, J = 9.2 Hz, 1H), 2.86-2.75 (m, 1H), 2.70-2.62 (m, 1H), 2.62-2.45 (m, 4H), 2.38-2.18 (m, 5H), 1.96-1.79 (m, 4H), 1.79-1.71 (m, 2H), 1.67 (d, J = 12.6 Hz, 2H), 1.60-1.38 (m, 9H), 1.35-1.31 (m, 3H), 1.31-1.24 (m, 7H), 1.17 (t, J = 6.6 Hz, 6H), 0.93 (t, J = 7.4 Hz, 3H), 0.85 (d, J = 6.9 Hz, 3H), 0.82-0.75 (m, 4H); MSm/z 997.5616 [M+Na]⁺.

### Production of Compound 1-171 and Compound 1-172

Compound 1-171 (isolated yield 1.7 mg, percent yield 16%) and Compound I-172 (isolated yield 1.5 mg, percent yield 14%) were obtained in a similar manner to [Production of Compound 1-132], except that in [Production of Compound 1-132], Compound I-168 obtained in [Production of Compound I-168] was used in place of Compound I-48, and methylamine hydrochloride was used in place of 5-aminopyrimidine.

### (Compound 1-171)

¹H-NMR (500 MHz, CDCl₃, δ): 6.28-6.18 (m, 1H), 5.88-5.82 (m, 1H), 5.76-5.65 (m, 2H), 5.44-5.37 (m, 2H), 4.95 (br d, J = 11.5 Hz, 1H), 4.89 (s, 1H), 4.76 (d, J = 3.4 Hz, 1H), 4.68-4.61 (m, 3H), 4.57-4.50 (m, 1H), 3.93 (br s, 1H), 3.86-3.73 (m, 2H), 3.69-3.60 (m, 2H), 3.51-3.45 (m, 1H), 3.44 (s, 3H), 3.42 (s, 3H), 3.26-3.19 (m, 2H), 3.16 (t, J = 9.2 Hz, 1H), 2.76-2.74 (m, 3H), 2.77-2.72 (m, 1H), 2.60 (dd, J = 12.3, 6.6 Hz, 1H), 2.51-2.47 (m, 3H), 2.36-2.19 (m, 4H), 1.96-1.84 (m, 3H), 1.77-1.72 (m, 1H), 1.70-1.39 (m, 13H), 1.33-1.23 (m, 11H), 1.19 (d, J = 6.9 Hz, 3H), 1.15 (d, J = 6.9 Hz, 3H), 0.92 (t, J = 7.4 Hz, 3H), 0.84 (d, J = 6.9 Hz, 3H), 0.81-0.75 (m, 4H); MSm/z 989.5947 [M+H]⁺.

### (Compound 1-172)

¹H-NMR (500 MHz, CDCl₃, δ): 6.03-5.98 (m, 1H), 5.83 (td, J = 2.3,10.3 Hz, 1H), 5.77-5.65 (m, 2H), 5.45-5.36 (m, 2H), 4.99-4.92 (m, 2H), 4.80-4.70 (m, 2H), 4.70-4.63 (m, 2H), 4.61-4.54 (m, 1H), 3.93 (br s, 1H), 3.87-3.73 (m, 2H), 3.70-3.61 (m, 2H), 3.52-3.46 (m, 1H), 3.44 (s, 3H), 3.42 (s, 3H), 3.27-3.19 (m, 2H), 3.16 (t, J = 9.2 Hz, 1H), 2.83-2.78 (m, 1H), 2.74 (d, J = 4.6 Hz, 3H), 2.69-2.61 (m, 1H), 2.54-2.41 (m, 3H), 2.36-2.19 (m, 4H), 1.92-1.82 (m, 3H), 1.75-1.71 (m, 1H), 1.68-1.36 (m, 13H), 1.31-1.23 (m, 10H), 1.16 (d, J = 6.9 Hz, 6H), 1.17-1.14 (m, 6H), 0.92 (t, J = 7.4 Hz, 3H), 0.84 (d, J = 6.3 Hz, 3H), 0.82-0.75 (m, 4H); MSm/z 989.5945 [M+H]⁺.

### Reference Example 23

### Production of methyl (S)-3-aminooxy-2-methylpropionate

With reference to European Journal of Medicinal Chemistry (2001), 36, 799-807, methyl (S)-3-aminooxy-2-methylpropionate (isolated yield 435 mg, percent yield 79%) was obtained using methyl (S)-3-hydroxy-2-methylpropionate (1.50 g, 12.7 mmol) as a raw material. ¹H-NMR (500 MHz, D₂O, δ): 3.74-3.68 (m, 1H), 3.68-3.61 (m, 1H), 3.61-3.56 (m, 3H), 2.81-2.73 (m, 1H), 1.01-0.97 (m, 3H).

### Production of Compound 1-173

Compound 1-173 (isolated yield 443 mg, percent yield 78%) was obtained in a similar manner to [Production of Compound 1-2], except that methyl (S)-3-aminooxy-2-methylpropionate obtained in Reference Example 23 was used in place of hydroxylamine hydrochloride.
¹H-NMR (500 MHz, CDCl₃, δ): 5.87-5.80 (m, 1H), 5.76-5.64 (m, 2H), 5.43-5.35 (m, 2H), 4.95 (br d, J = 9.2 Hz, 1H), 4.88 (s, 1H), 4.76 (br d, J = 3.4 Hz, 1H), 4.70-4.60 (m, 2H), 4.60-4.51 (m, 1H), 4.33 (dd, J = 11.2, 6.6 Hz, 1H), 4.08 (dd, J = 11.2, 6.6 Hz, 1H), 3.93 (br s, 1H), 3.86-3.72 (m, 2H), 3.67 (s, 3H), 3.71-3.59 (m, 2H), 3.52-3.45 (m, 1H), 3.44 (s, 3H), 3.42 (s, 3H), 3.26-3.19 (m, 2H), 3.16 (t, J = 9.2 Hz, 1H), 2.92-2.82 (m, 1H), 2.79-2.75 (m, 1H), 2.61-2.46 (m, 2H), 2.34 (dd, J = 4.6, 12.6 Hz, 2H), 2.30-2.16 (m, 2H), 1.92-1.85 (m, 2H), 1.85-1.79 (m, 1H), 1.76-1.71 (m, 1H), 1.69-1.36 (m, 12H), 1.29-1.23 (m, 8H), 1.16 (t, J = 6.6 Hz, 8H), 0.92 (t, J = 7.4 Hz, 3H), 0.88 (t, J = 6.9 Hz, 1H), 0.83 (d, J = 6.9 Hz, 3H), 0.82-0.74 (m, 4H); MSm/z 1007.5834 [M+NH₄]⁺.

### Production of Compound I-174

Compound 1-174 (isolated yield 195 mg, percent yield 90%) was obtained by a method similar to that of [Production of Compound I-154], except that Compound I-173 obtained in [Production of Compound I-173] was used in place of Compound I-153 in [Production of Compound I-154].
¹H-NMR (500 MHz, CDCl₃, δ): 5.86-5.80 (m, 1H), 5.76-5.64 (m, 2H), 5.44-5.34 (m, 2H), 4.94 (br d, J = 10.3 Hz, 1H), 4.89 (s, 1H), 4.76 (br d, J = 2.9 Hz, 1H), 4.65 (br s, 2H), 4.36-4.30 (m, 1H), 4.16-4.11 (m, 1H), 3.93 (br s, 1H), 3.86-3.72 (m, 2H), 3.70-3.56 (m, 2H), 3.48 (ddd, J = 4.6, 9.2, 11.5 Hz, 1H), 3.44 (s, 3H), 3.42 (s, 3H), 3.26-3.19 (m, 2H), 3.17 (t, J = 9.2 Hz, 1H), 2.93-2.87 (m, 1H), 2.77 (dd, J = 12.3, 3.2 Hz, 1H), 2.64-2.56 (m, 1H), 2.52-2.46 (m, 1H), 2.36-2.19 (m, 2H), 1.96-1.80 (m, 3H), 1.77-1.71 (m, 1H), 1.68-1.63 (m, 1H), 1.59-1.36 (m, 12H), 1.29-1.24 (m, 9H), 1.20 (d, J = 7.4 Hz, 3H), 1.15 (dd, J = 6.6, 2.6 Hz, 6H), 0.92 (t, J = 7.4 Hz, 3H), 0.88 (s, 1H), 0.85-0.82 (m, 3H), 0.80-0.74 (m, 4H); MSm/z 998.5208 [M+Na]⁺.

### Production of Compound 1-175

Compound 1-175 (isolated yield 15.7 mg, percent yield 39%) was obtained in a similar manner to [Production of Compound I-132], except that in [Production of Compound I-132], Compound I-174 obtained in [Production of Compound I-174] was used in place of Compound I-48, and ammonium chloride was used in place of 5-aminopyrimidine.
¹H-NMR (500 MHz, CDCl₃, δ): 7.25 (s, 1H), 5.97 (br s, 1H), 5.86-5.80 (m, 1H), 5.76-5.64 (m, 2H), 5.49 (br s, 1H), 5.45-5.36 (m, 2H), 4.94 (br d, J = 10.3 Hz, 1H), 4.91-4.88 (m, 1H), 4.76 (d, J = 3.4 Hz, 1H), 4.70-4.59 (m, 3H), 4.25-4.18 (m, 1H), 4.16-4.09 (m, 1H), 3.92 (br s, 1H), 3.85-3.72 (m, 2H), 3.70-3.57 (m, 2H), 3.51-3.46 (m, 1H), 3.44 (s, 3H), 3.42 (s, 3H), 3.27-3.18 (m, 2H), 3.16 (t, J = 9.2 Hz, 1H), 2.79-2.67 (m, 2H), 2.65-2.46 (m, 2H), 2.36-2.19 (m, 4H), 1.93-1.80 (m, 2H), 1.75-1.71 (m, 1H), 1.66 (d, J = 12.6 Hz, 1H), 1.58-1.35 (m, 12H), 1.28-1.23 (m, 9H), 1.19-1.11 (m, 8H), 0.92 (t, J = 7.2 Hz, 3H), 0.83 (d, J = 6.9 Hz, 3H), 0.81-0.74 (m, 4H); MSm/z 997.5370 [M+Na]⁺.

### Production of Compound I-176

Compound 1-176 (isolated yield 24.0 mg, percent yield 59%) was obtained in a similar manner to [Production of Compound I-132], except that in [Production of Compound 1-132], Compound I-174 obtained in [Production of Compound 1-174] was used in place of Compound I-48, and methylamine hydrochloride was used in place of 5-aminopyrimidine.
¹H-NMR (500 MHz, CDCl₃, δ): 5.93 (br s, 1H), 5.83 (d, J = 10.3 Hz, 1H), 5.77-5.64 (m, 2H), 5.43-5.36 (m, 2H), 4.94 (d, J = 11.5 Hz, 1H), 4.89 (s, 1H), 4.76 (d, J = 4.0 Hz, 1H), 4.73-4.57 (m, 3H), 4.22-4.09 (m, 2H), 3.93 (br s, 1H), 3.85-3.73 (m, 2H), 3.71-3.58 (m, 2H), 3.51-3.46 (m, 1H), 3.44 (s, 3H), 3.42 (s, 3H), 3.26-3.19 (m, 2H), 3.16 (t, J = 8.9 Hz, 1H), 2.78-2.73 (m, 3H), 2.65-2.57 (m, 2H), 2.53-2.45 (m, 1H), 2.33 (dd, J = 12.6, 4.0 Hz, 2H), 2.27-2.19 (m, 2H), 1.92-1.81 (m, 2H), 1.76-1.69 (m, 1H), 1.69-1.61 (m, 1H), 1.59-1.37 (m, 13H), 1.26 (dd, J = 10.9, 6.3 Hz, 8H), 1.18-1.11 (m, 8H), 0.92 (t, J = 7.4 Hz, 3H), 0.83 (d, J = 6.9 Hz, 3H), 0.80-0.74 (m, 4H); MSm/z 1011.5554 [M+Na]⁺.

### Production of Compound II-1 and Compound II-2

Ethyl 2-(diethoxyphosphoryl)acetate (37.7 µL, 0.189 mmol) was dissolved in THF and cooled to 0°C. After that, 1M lithium bis(trimethylsilyl)amide THF solution (0.189 ml, 0.189 mmol) was added dropwise, and the mixture was stirred at the same temperature for 30 minutes. 3,4β-Dihydro-5-oxo-ivermectin B1a (50.0 mg, 0.0570 mmol) dissolved in THF (0.457 mL) was slowly added dropwise thereto, and the mixture was further stirred at the same temperature for 2 hours. A saturated aqueous solution of ammonium chloride was added to the reaction solution, the temperature was raised to room temperature, and then extracted with ethyl acetate. The organic layer was washed with saturated brine and dried over anhydrous magnesium sulfate. After the solvent was distilled off under reduced pressure, the residue was purified by preparative thin-layer chromatography (n-hexane:ethyl acetate = 3:2) to give Compound II-1 (isolated yield 5.2 mg, percent yield 10%) and Compound II-2 (isolated yield 19.4 mg, percent yield 36%).

### (Compound II-1)

¹H-NMR (500 MHz, CDCl₃, δ): 5.93 (s, 1H), 5.88-5.81 (m, 1H), 5.76-5.66 (m, 2H), 5.46-5.38 (m, 2H), 4.95 (d, J = 10.88 Hz, 1H), 5.78-5.65 (m, 3H), 4.60-4.55 (m, 1H), 4.19-4.10 (m, 2H), 4.08-4.01 (m, 1H), 3.93 (br s, 1H), 3.86-3.74 (m, 3H), 3.72-3.60 (m, 2H), 3.51-3.40 (m, 7H), 3.28-3.13 (m, 3H), 2.93 (d, J = 13.75 Hz, 1H), 2.52-2.46 (m, 2H), 2.36-2.15 (m, 5H), 1.94-1.86 (m, 1H), 1.79-1.70 (m, 1H), 1.69-1.64 (m, 1H), 1.59-1.38 (m, 17H), 1.31-1.23 (m, 13H), 1.15 (d, J = 6.87 Hz, 3H), 0.94-0.74 (m, 10H); MSm/z 962.5745 [M+NH₄]⁺.

### (Compound II-2)

¹H-NMR (500 MHz, CDCl₃, δ): 6.01 (s, 1H), 5.88-5.76 (m, 1H), 5.75-5.66 (m, 2H), 5.45-5.35 (m, 2H), 5.17 (s, 1H), 4.94 (d, J = 10.31 Hz, 1H), 4.76 (d, J = 3.44 Hz, 1H), 4.72-4.62 (m, 2H), 4.52 (s, 1H), 4.17-4.09 (m, 3H), 3.93 (br s, 1H), 3.87-3.71 (m, 2H), 3.70-3.59 (m, 2H), 3.52-3.35 (m, 7H), 3.27-3.11 (m, 3H), 2.95-2.88 (m, 1H), 2.75-2.67 (m, 1H), 2.55-2.46 (m, 2H), 2.36-2.19 (m, 5H), 1.93-1.84 (m, 1H), 1.83-1.71 (m, 1H), 1.67-1.60 (m, 3H), 1.58-1.37 (m, 13H), 1.30-1.22 (m, 13H), 1.19-1.11 (m, 3H), 0.91 (t, J = 7.16 Hz, 3H), 0.86-0.74 (m, 7H); MSm/z 962.5745 [M+NH₄]⁺.

### Production of Compound II-3

Compound II-3 (isolated yield 29.6 mg, percent yield 55%) was obtained in a similar manner to [Production of Compound II-1 and Compound II-2] except that 3,4α-dihydro-5-oxo-ivermectin B1a was used in place of 3,4β-dihydro-5-oxo-ivermectin B1a.
¹H-NMR (500 MHz, CDCl₃, δ): 5.95 (d, J = 1.7 Hz, 1H), 5.84-5.77 (m, 1H), 5.74-5.66 (m, 2H), 5.41-5.33 (m, 3H), 4.96-4.91 (m, 1H), 4.76 (d, J = 3.4 Hz, 1H), 4.67 (d, J = 2.3 Hz, 2H), 4.46 (s, 1H), 4.17-4.11 (m, 2H), 3.92 (br s, 1H), 3.85-3.73 (m, 2H), 3.69-3.59 (m, 2H), 3.50-3.46 (m, 1H), 3.46-3.40 (m, 6H), 3.25-3.13 (m, 3H), 2.89-2.83 (m, 1H), 2.67-2.60 (m, 1H), 2.56 (br s, 1H), 2.49 (t, J = 6.6 Hz, 1H), 2.36-2.19 (m, 4H), 1.92-1.85 (m, 1H), 1.82-1.61 (m, 6H), 1.57-1.36 (m, 14H), 1.29-1.22 (m, 10H), 1.13 (d, J = 9.16 Hz, 3H), 0.91 (t, J = 7.5 Hz, 3H), 0.85-0.74 (m, 7H); MSm/z 962.5745 [M+NH₄]⁺.

### Production of Compound II-4

Compound II-2 (18.0 mg, 19.0 µmol) obtained in [Production of Compound II-1 and Compound II-2] was dissolved in dichloromethane (224 µL) and cooled to - 78°C. Then, a 1M diisobutylaluminum hydride dichloromethane solution (66.7 µL, 66.7 µmol) was added dropwise, and the mixture was stirred at the same temperature for 4 hours. Methanol and a saturated aqueous Rochelle salt solution were added dropwise thereto, and the mixture was stirred at room temperature for 30 minutes, followed by extraction of the reaction solution with chloroform. After the solvent was distilled off under reduced pressure, the residue was purified by preparative thin-layer chromatography (n-hexane:ethyl acetate = 1:3) to obtain Compound II-4 (isolated yield 13.2 mg, percent yield 77%).
¹H-NMR (500 MHz, CDCl₃, δ): 5.97-5.89 (m, 1H), 5.88-5.81 (m, 1H), 5.77-5.63 (m, 3H), 5.40 (d, J = 3.4 Hz, 2H), 4.98-4.90 (m, 1H), 4.78-4.74 (m, 1H), 4.70 (s, 1H), 4.67-4.54 (m, 2H), 4.40-4.36 (m, 1H), 4.32 (s, 1H), 4.25-4.14 (m, 2H), 3.94-3.89 (m, 1H), 3.86-3.71 (m, 2H), 3.70-3.60 (m, 2H), 3.52-3.38 (m, 7H), 3.26-3.14 (m, 3H), 2.87-2.76 (m, 1H), 2.72-2.62 (m, 1H), 2.58-2.53 (m.1H), 2.49 (t, J = 6.87 Hz, 1H), 2.38-2.30 (m, 2H), 2.30-2.16 (m, 3H), 1.94-1.85 (m, 2H), 1.79-1.71 (m, 2H), 1.71-1.62 (m, 4H), 1.58-1.37 (m, 14H), 1.30-1.19 (m, 9H), 0.92 (t, J = 7.5 Hz, 3H), 0.86-0.75 (m, 7H); MSm/z 920.5573 [M+NH₄]⁺.

### Production of Compound II-5

Compound II-5 (isolated yield 20.3 mg, percent yield 76%) was obtained in a similar manner to [Production of Compound II-4] except that Compound II-3 was used in place of Compound II-2.
¹H-NMR (500 MHz, CDCl₃, δ): 5.90 (td, J = 7.0, 2.0 Hz, 1H), 5.86-5.78 (m, 1H), 5.76-5.63 (m, 2H), 5.41-5.35 (m, 2H), 4.97-4.91 (m, 1H), 4.79-4.73 (m, 2H), 4.63 (dd, J = 6.9, 2.3 Hz, 2H), 4.38 (s, 1H), 4.31-4.26 (m, 1H), 4.23-4.17 (m, 1H), 3.92 (br s, 1H), 3.86-3.71 (m, 2H), 3.69-3.60 (m, 2H), 3.52-3.38 (m, 7H), 3.25-3.13 (m, 3H), 2.78 (dd, J = 12.9, 3.15 Hz, 1H), 2.60 (s, 1H), 2.52-2.45 (m, 2H), 2.35-2.19 (m, 4H), 1.93-1.80 (m, 2H), 1.77-1.70 (m, 4H), 1.68-1.60 (m, 2H), 1.58-1.37 (m, 14H), 1.28-1.22 (m, 6H), 1.13 (d, J = 12.0 Hz, 3H), 0.91 (t, J = 7.5 Hz, 3H), 0.84-0.74 (m, 7H); MSm/z 920.5573 [M+NH₄]⁺.

### Production of Compound II-6

Compound II-5 (98.0 mg, 0.109 mmol) obtained in [Production of Compound II-5] was dissolved in dichloromethane (2.17 mL), manganese dioxide (385 mg, 4.34 mmol) was added, and the mixture was stirred at room temperature for 14 hours. After filtering the reaction solution using Celite, the solvent was distilled off under reduced pressure to obtain a crude product. The crude product was purified by silica gel column chromatography (n-hexane/ethyl acetate system) to obtain Compound II-6 (isolated yield 67.7 mg, percent yield 69%).
¹H-NMR (500 MHz, CDCl₃, δ): 10.13 (d, J = 7.5 Hz, 1H), 6.13 (dd, J = 8.0, 1.7 Hz, 1H), 5.85 (s, 1H), 5.73 (dd, J = 17.2, 9.7 Hz, 2H), 5.40 (d, J = 4.0 Hz, 2H), 4.98 (s, 2H), 4.79-4.67 (m, 6H), 3.94 (br s, 1H), 3.86-3.73 (m, 2H), 3.71-3.58 (m, 2H), 3.43 (d, J = 9.7 Hz, 7H), 3.23 (d, J = 9.2 Hz, 2H), 3.17 (s, 1H), 2.85 (s, 1H), 2.76-2.66 (m, 1H), 2.50 (d, J = 1.2 Hz, 2H), 2.33 (br s, 4H), 1.82 (s, 4H), 1.65 (br s, 1H), 1.59-1.49 (m, 16H), 1.41 (t, J = 12.0 Hz, 3H), 1.31-1.22 (m, 7H), 1.16 (d, J = 6.3 Hz, 6H), 0.92 (t, J = 7.5 Hz, 3H), 0.86-0.75 (m, 7H); MSm/z 920.5573 [M+NH₄]⁺.

### Production of Compound II-7

Compound II-7 (isolated yield 26.8 mg, percent yield 71%) was obtained in a similar manner to [Production of Compound II-1 and Compound II-2] except that Compound II-6 was used in place of 3,4β-dihydro-5-oxo-ivermectin B1a.
¹H-NMR (500 MHz, CDCl₃, δ): 7.67 (dd, J = 15.2,11.7 Hz, 1H), 6.32 (d, J = 11.5 Hz, 1H), 5.95 (d, J = 14.9 Hz, 1H), 5.88-5.80 (m, 1H), 5.76-5.67 (m, 2H), 5.43-5.35 (m, 2H), 4.94 (d, J = 10.9 Hz, 1H), 4.76 (d, J = 3.4 Hz, 1H), 4.70-4.60 (m, 4H), 4.21-4.15 (m, 2H), 3.93 (br s, 1H), 3.86-3.72 (m, 2H), 3.72-3.57 (m, 2H), 3.48-3.38 (m, 7H), 3.27-3.13 (m, 3H), 2.83 (dd, J = 12.6, 3.44 Hz, 1H), 2.64-2.54 (m, 2H), 2.54-2.46 (m, 1H), 2.37-2.18 (m, 4H), 1.89 (dd, J = 11.5, 4.6 Hz, 1H), 1.80-1.61 (m, 6H), 1.58-1.36 (m, 14H), 1.31-1.14 (m, 17H), 0.93-0.74 (m, 11H); MSm/z 988.6065 [M+NH₄]⁺.

### Production of Compound II-8

Compound II-6 (15.0 mg, 17.0 µmol) obtained in [Production of Compound II-6] was dissolved in methanol (200 µL), and 2-(aminooxy)ethan-1-ol (5.13 mg, 67.0 µmol) and sodium acetate (5.46 mg, 67.0 µmol) were added, followed by stirring at room temperature for 14 hours. A saturated aqueous sodium hydrogen carbonate solution was added to the reaction solution, and the mixture was extracted with chloroform. After the solvent was distilled off under reduced pressure, the residue was purified by preparative thin-layer chromatography (n-hexane:acetone = 2:1) to obtain Compound II-8 (isolated yield 12.6 mg, percent yield 79%) as a mixture of isomers that were difficult to separate.
¹H-NMR (500 MHz, CDCl₃, δ): 8.20 (d, J = 10.3 Hz, 1H), 7.45 (d, J = 9.7 Hz, 1H), 6.27 (dd, J = 10.3, 1.7 Hz, 1H), 5.87-5.80 (m, 1H), 5.71 (t, J = 9.7 Hz, 2H), 5.45-5.33 (m, 2H), 4.94 (d, J = 10.9 Hz, 1H), 4.76 (d, J = 3.4 Hz, 1H), 4.70-4.60 (m, 3H), 4.51 (s, 0.3H), 4.43 (s, 0.7H), 4.24-4.14 (m, 2H), 3.93 (br s, 1H), 3.89-3.71 (m, 4H), 3.72-3.60 (m, 2H), 3.52-3.38 (m, 7H), 3.26-3.13 (m, 3H), 2.80 (dd, J = 12.6, 3.4 Hz, 1H), 2.63-2.53 (m, 2H), 2.53-2.43 (m, 1H), 2.36-2.16 (m, 5H), 1.89 (dd, J = 12.6, 4.6 Hz, 1H), 1.82-1.69 (m, 3H), 1.68-1.62 (m, 3H), 1.58-1.37 (m, 13H), 1.29-1.14 (m, 12H), 0.91 (t, J = 7.5 Hz, 3H), 0.85-0.74 (m, 7H); MSm/z 982.5557 [M+Na]⁺.

### Production of Compound II-9

Compound II-6 (15.0 mg, 17.0 µmol) obtained in [Production of Compound II-6] was dissolved in THF (333 µL), and 2-aminoethan-1-ol (3.02 µL, 50.0 µmol), acetic acid (9.53 µL, 166 µmol), zinc chloride (3.40 mg, 25.0 µmol) and sodium cyanoborohydride (5.23 mg, 83.0 µmol) were added, followed by stirring at room temperature for 14 hours. A saturated aqueous sodium hydrogen carbonate solution was added to the reaction solution, and the mixture was extracted with chloroform. After the solvent was distilled off under reduced pressure, the residue was purified by preparative thin-layer chromatography (chloroform:methanol:ammonium aqueous solution = 90:10:1) to obtain Compound II-9 (isolated yield 8.5 mg, percent yield 54%).
¹H-NMR (500 MHz, CDCl₃, δ): 5.87-5.81 (m, 1H), 5.79-5.68 (m, 3H), 5.40-5.31 (m, 2H), 4.98-4.91 (m, 1H), 4.76 (d, J = 3.4 Hz, 1H), 4.63 (dd, J = 4.6, 2.3 Hz, 2H), 4.38 (s, 1H), 3.93 (br s, 1H), 3.85-3.73 (m, 2H), 3.69-3.60 (m, 4H), 3.52-3.41 (m, 8H), 3.38-3.33 (m, 1H), 3.26-3.14 (m, 3H), 2.83-2.73 (m, 3H), 2.61-2.38 (m, 7H), 2.38-2.17 (m, 6H), 1.94-1.88 (m, 1H), 1.77-1.61 (m, 4H), 1.58-1.35 (m, 14H), 1.35-1.21 (m, 8H), 1.13 (dd, J = 15.2, 6.6 Hz, 7H), 0.91 (t, J = 7.5 Hz, 3H), 0.87-0.73 (m, 8H); MSm/z 946.5779 [M+H]⁺.

### Production of Compound II-10

Compound II-7 (19.3 mg, 20.0 µmol) obtained in [Production of Compound II-7] was dissolved in methanol (397 µL) and then cooled to 0°C. Then, nickel chloride hexahydrate (0.95 mg, 3.97 µmol) and sodium borohydride (3.76 mg, 99.0 µmol) were added, and the mixture was stirred at the same temperature for 3 hours. A saturated aqueous solution of ammonium chloride was added to the reaction solution, the temperature was raised to room temperature, and the mixture was extracted with chloroform. After the solvent was distilled off under reduced pressure, the residue was purified by preparative thin-layer chromatography (n-hexane:ethyl acetate = 1:1) to obtain Compound II-10 (isolated yield 5.2 mg, percent yield 10%) as a mixture of isomers that were difficult to separate. The ¹H-NMR spectrum below is the analytical data of the main isomer. ¹H-NMR (500 MHz, CDCl₃, δ): 5.86-5.75 (m, 1H), 5.73-5.50 (m, 3H), 5.41-5.32 (m, 2H), 4.94 (d, J = 11.5 Hz, 1H), 4.76 (d, J = 2.3 Hz, 1H), 4.62 (t, J = 2.7, 2.7 Hz, 1H), 4.59-4.46 (m, 2H), 4.14-4.08 (m, 2H), 3.92 (br s, 1H), 3.84-3.60 (m, 5H), 3.50-3.37 (m, 7H), 3.25-3.14 (m, 3H), 3.07-3.06 (m, 1H), 2.77 (dd, J = 12.9, 3.2 Hz, 1H), 2.58-2.40 (m, 4H), 2.38-2.20 (m, 5H), 1.92-1.85 (m, 1H), 1.79-1.70 (m, 2H), 1.68-1.36 (m, 15H), 1.27-1.21 (m, 10H), 1.14 (d, J = 6.9 Hz, 3H), 1.08-1.03 (m, 2H), 0.94-0.77 (m, 13H); MSm/z 990.6157 [M+NH₄]⁺.

### Production of Compound II-11

Compound II-7 (7.00 mg, 7.21 µmol) obtained in [Production of Compound II-7] was dissolved in dichloromethane (290 µL) and then cooled to -78°C. Then, a 1M diisobutylaluminum hydride dichloromethane solution (36.0 µL, 36.0 µmol) was added dropwise, and the mixture was stirred at the same temperature for 4 hours. Methanol and a saturated aqueous Rochelle salt solution were added dropwise thereto, and the mixture was stirred at room temperature for 30 minutes, followed by extraction of the reaction solution with chloroform/methanol (9:1). After the solvent was distilled off under reduced pressure, the residue was purified by preparative thin-layer chromatography (chloroform:methanol = 9:1) to obtain Compound II-11 (isolated yield 5.30 mg, percent yield 79%).
¹H-NMR (500 MHz, CDCl₃, δ): 6.66 (dd, J = 14.9,10.9 Hz, 1H), 6.25 (dd, J = 10.9, 1.7 Hz, 1H), 5.94 (d, J = 14.9 Hz, 1H), 5.87-5.78 (m, 1H), 5.76-5.66 (m, 2H), 5.41-5.35 (m, 2H), 4.98-4.89 (m, 1H), 4.76 (d, J = 2.9 Hz, 1H), 4.70-4.59 (m, 3H), 4.57 (s, 1H), 4.18 (d, J = 5.2 Hz, 2H), 3.93 (br s, 1H), 3.88-3.58 (m, 5H), 3.52-3.41 (m, 7H), 3.28-3.12 (m, 3H), 2.80 (s, 1H), 2.51 (br s, 3H), 2.36-2.30 (m, 2H), 2.27 (s, 2H), 1.93-1.83 (m, 1H), 1.77-1.62 (m, 5H), 1.61-1.40 (m, 19H), 1.38 (s, 2H), 1.30-1.22 (m, 8H), 1.15 (d, J = 6.9 Hz, 6H), 0.91 (t, J = 7.5 Hz, 3H), 0.84-0.75 (m, 7H); MSm/z 946.5919 [M+NH₄]⁺.

### Production of Compound II-12

Compound II-5 (30.6 mg, 34.0 µmol) obtained in [Production of Compound II-5] was dissolved in dichloromethane (339 µL) and cooled in ice to 0°C. Then, pyridine (5.48 µL, 68.0 µmol), 4-methylbenzoyl chloride (5.82 µL, 44.0 µmol) and DMAP (0.414 mg, 3.39 µmol) were added, the temperature was raised to room temperature, and the mixture was stirred at the same temperature for 24 hours. Then, the reagents were added again, and the mixture was stirred at room temperature for another 24 hours. A saturated aqueous sodium hydrogen carbonate solution was added to the reaction solution, and the mixture was extracted with chloroform. After the solvent was distilled off under reduced pressure, the residue was purified by preparative thin-layer chromatography (n-hexane:ethyl acetate = 1:1) to obtain Compound II-12 (isolated yield 5.00 mg, percent yield 15%).
¹H-NMR (500 MHz, CDCl₃, δ): 7.93 (d, J = 7.6 Hz, 2H), 7.21 (d, J = 8.0 Hz, 2H), 5.89-5.82 (m, 2H), 5.75-5.68 (m, 2H), 5.42-5.33 (m, 2H), 5.02-4.92 (m, 3H), 4.77 (d, J = 3.4 Hz, 1H), 4.65 (dd, J = 5.2, 2.3 Hz, 2H), 4.53 (s, 1H), 4.47 (s, 1H), 3.93 (br s, 1H), 3.87-3.72 (m, 2H), 3.71-3.58 (m, 2H), 3.51-3.36 (m, 7H), 3.27-3.14 (m, 3H), 2.83-2.78 (m, 1H), 2.50 (br s, 3H), 2.39 (s, 3H), 2.37-2.30 (m, 2H), 2.30-.2.17 (m, 2H), 1.95-1.83 (m, 1H), 1.79-1.62 (m, 5H), 1.60-1.39 (m,21H), 1.31-1.21 (m, 7H), 1.14 (d, J = 3.4 Hz, 3H), 0.92 (t, J = 7.2 Hz, 3H), 0.86-0.76 (m, 7H); MSm/z 1038.6484 [M+NH₄]⁺.

### Production of Compound 11-13

Compound II-13 (isolated yield 91.3 mg, percent yield 84%) was obtained in a similar manner to [Production of Compound II-3] except that allyl 2-(diethoxyphosphoryl)acetate was used in place of ethyl 2-(diethoxyphosphoryl)acetate.
¹H-NMR (500 MHz, CDCl₃, δ) : 5.98 (d, J = 1.7 Hz, 1H), 5.95-5.87 (m, 1H), 5.82-5.79 (m, 1H), 5.74-5.63 (m, 2H), 5.40-5.27 (m, 4H), 5.24-5.18 (m, 1H), 4.93 (d, J = 10.9 Hz, 1H), 4.75 (d, J = 3.4 Hz, 1H), 4.67 (d, J = 2.3 Hz, 2H), 4.63-4.55 (m, 2H), 4.46 (s, 1H), 3.92 (br s, 1H), 3.85-3.73 (m, 2H), 3.69-3.60 (m, 2H), 3.51-3.39 (m, 7H), 3.25-3.13 (m, 3H), 2.88-2.84 (m, 1H), 2.65 (dd, J = 5.2, 2.9 Hz, 1H), 2.60 (br s, 1H), 2.48 (br s, 1H), 2.35-2.19 (m, 4H), 1.92-1.87 (m, 1H), 1.82-1.70 (m, 4H), 1.64 (d, J = 12.6 Hz, 1H), 1.57-1.35 (m,14H), 1.35-1.21 (m, 8H), 1.14 (dd, J = 8.6, 6.9 Hz, 6H), 0.91 (t, J = 7.5 Hz, 3H), 0.86-0.74 (m, 7H); MSm/z 974.6138 [M+NH₄]⁺.

### Production of Compound II-14

Compound II-13 (15.0 mg, 16.0 µL) obtained in [Production of Compound II-13] was dissolved in dichloromethane (313 µL) and cooled in ice to 0°C. Then, morpholine (13.7 µL, 157 µmol) and tetrakis(triphenylphosphine)palladium (1.81 mg, 1.57 µmol) were added, and the mixture was stirred at the same temperature for 30 minutes. Next, the temperature was raised to room temperature and stirred for 30 minutes. The reaction solution was diluted with chloroform and washed with 1N hydrochloric acid. After the solvent was distilled off under reduced pressure, the residue was purified by preparative thin-layer chromatography (n-hexane:ethyl acetate = 1:3) to obtain Compound II-14 (isolated yield 11.6 mg, percent yield 81%).
¹H-NMR (500 MHz, CDCl₃, δ): 7.69-7.63 (m, 1H), 7.53 (dd, J = 7.2, 1.4 Hz, 1H), 7.50-7.41 (m, 1H), 5.98 (s, 1H), 5.85-5.78 (m, 1H), 5.75-5.66 (m, 2H), 5.42-5.33 (m, 3H), 4.95 (d, J = 9.74 Hz, 1H), 4.76 (d, J = 3.4 Hz, 1H), 4.68 (br s, 2H), 3.93 (br s, 1H), 3.86-3.72 (m, 2H), 3.72-3.57 (m, 2H), 3.52-.3.40 (m, 7H), 3.29-3.11 (m, 3H), 2.94-2.82 (m, 1H), 2.72-2.62 (m, 1H), 2.54-2.43 (m, 1H), 2.38-2.16 (m, 5H), 1.90 (dd, J = 12.0, 4.0 Hz, 1H), 1.84-1.71 (m, 3H), 1.65 (d, J = 12.6 Hz, 1H), 1.58-1.36 (m, 14H), 1.35-1.21 (m, 7H), 1.14 (dd, J = 6.3, 3.4 Hz, 6H), 0.91 (t, J = 7.5 Hz, 3H), 0.87-0.73 (m, 7H); MSm/z 934.5815 [M+NH₄]⁺.

### Production of Compound 11-15

Compound II-10 (13.4 mg, 14.0 µmol) obtained in [Production of Compound II-10] was dissolved in THF (275 µL) and cooled in ice to 0°C. Then, lithium triethylborohydride (62.0 µL, 62.0 µmol) was added, and the mixture was stirred at the same temperature for 1 hour. Methanol and a saturated aqueous sodium hydrogen carbonate solution were added to the reaction solution, and the mixture was extracted with ethyl acetate. The organic layer was washed with saturated brine and dried over anhydrous sodium sulfate. After the solvent was distilled off under reduced pressure, the residue was purified by preparative thin-layer chromatography (n-hexane:ethyl acetate = 1:2) to obtain Compound II-15 (isolated yield 7.90 mg, percent yield 62%) as a mixture of isomers that were difficult to separate. The ¹H-NMR spectrum below is the analytical data of the main isomer. ¹H-NMR (500 MHz, CDCl₃, δ): 5.83-5.76 (m, 1H), 5.74-5.66 (m, 2H), 5.52-5.49 (m, 1H), 5.40-5.34 (m, 2H), 4.95-4.93 (m, 1H), 4.76 (d, J = 2.3 Hz, 1H), 4.63-4.61 (m, 1H), 4.58-4.46 (m, 2H), 3.92 (br s, 1H), 3.85-3.74 (m, 2H), 3.69-3.56 (m, 4H), 3.50-3.40 (m, 7H), 3.25-3.14 (m, 3H), 2.59-2.44 (m, 3H), 2.35-2.20 (m, 5H), 1.89-1.85 (m, 1H), 1.75-1.36 (m,25H), 1.26 (dd, J = 12.3Hz, 6.0 Hz, 7H), 1.16-1.10 (m, 4H), 1.07-1.02 (m, 1H), 0.95-0.88 (m, 6H), 0.83 (d, J = 6.3 Hz, 3H), 0.79-0.76 (m, 4H); MSm/z 948.5929 [M+NH₄]⁺.

### Production of Compound II-16

Compound II-14 (11.6 mg, 13.0 µmol) obtained in [Production of Compound II-14] was dissolved in a dichloromethane solution (506 µL) and cooled in ice to 0°C. Then, aniline (2.31 µL, 25.0 µmol), 3-(((ethylimino)methylene)amino)-N,N-dimethylpropan-1-amine hydrochloride (4.85 mg, 25.0 µmol) and DMAP (3.09 mg, 25.0 µmol) were added, and the mixture was stirred at room temperature for 16 hours. A saturated aqueous sodium hydrogen carbonate solution was added to the reaction solution, and the mixture was extracted with chloroform. After the solvent was distilled off under reduced pressure, the residue was purified by preparative thin-layer chromatography (n-hexane:ethyl acetate = 1:1) to obtain Compound II-16 (isolated yield 9.80 mg, percent yield 78%).
¹H-NMR (500 MHz, CDCl₃, δ): 8.10 (s, 1H), 7.55 (d, J = 8.0 Hz, 2H), 7.30 (t, J = 7.7 Hz, 2H), 7.08 (s, 1H), 6.11 (d, J = 1.7 Hz, 1H), 5.82 (d, J = 10.3 Hz, 1H), 5.76-5.61 (m, 2H), 5.39 (d, J = 3.4 Hz, 2H), 4.95 (d, J = 9.7 Hz, 1H), 4.80 (s, 1H), 4.74 (s, 2H), 4.71-4.59 (m, 2H), 3.92 (br s, 1H), 3.86-3.72 (m, 2H), 3.71-3.58 (m, 2H), 3.42 (d, J = 10.3 Hz, 7H), 3.23 (s, 3H), 2.87-2.78 (m, 1H), 2.69-2.58 (m, 1H), 2.58-2.53 (m, 1H), 2.51-2.43 (m, 1H), 2.37-2.18 (m, 1H), 1.95-1.87 (m, 1H), 1.87-1.71 (m, 3H), 1.66 (s, 7H), 1.58-1.37 (m,13H), 1.25 (dd, J = 12.6, 6.3 Hz, 7H), 1.15 (dd, J = 18.9, 6.9 Hz, 6H), 0.92 (t, J = 7.5 Hz, 3H), 0.86-0.76 (m, 7H); MSm/z 1009.6273 [M+NH₄]⁺.

### Production of Compound II-17

Compound II-17 (isolated yield 10.6 mg, percent yield 77%) was obtained in a similar manner to [Production of Compound II-16] except that ethylamine was used in place of aniline.
¹H-NMR (500 MHz, CDCl₃, δ): 6.09 (t, J = 5.7 Hz, 1H), 5.96 (d, J = 2.3 Hz, 1H), 5.87-5.79 (m, 1H), 5.76-5.62 (m, 2H), 5.39 (d, J = 4.0 Hz, 2H), 4.98-4.91 (m, 1H), 4.77-4.72 (m, 2H), 4.68-4.57 (m, 3H), 3.93 (br s, 1H), 3.87-3.72 (m, 2H), 3.71-3.58 (m, 2H), 3.51-3.39 (m, 7H), 3.36-3.14 (m, 5H), 2.79 (dd, J = 12.3, 3.7 Hz, 1H), 2.59-2.52 (m, 2H), 2.51-2.45 (m, 1H), 2.36-2.29 (m, 2H), 2.29-2.19 (m, 2H), 1.94-1.86 (m, 1H), 1.82-1.62 (m, 9H), 1.58-1.36 (m,13H), 1.27 (d, J = 6.3 Hz, 7H), 1.17-1.10 (m, 9H), 0.91 (t, J = 7.5 Hz, 3H), 0.85-0.74 (m, 7H); MSm/z 961.6087 [M+NH₄]⁺.

### Production of Compound II-18

Compound II-18 (isolated yield 18.9 mg, percent yield 57%) was obtained in a similar manner to [Production of Compound II-9] except that thiophen-2-ylmethanamine was used in place of 2-aminoethan-1-ol. ¹H-NMR (500 MHz, CDCl₃, δ): 7.19 (dd, J = 4.9, 1.4 Hz, 1H), 6.95-6.91 (m, 2H), 5.86-5.80 (m, 1H), 5.77 (t,1H), 5.74-5.66 (m, 2H), 5.45-5.29 (m, 2H), 4.95 (d, J = 10.9 Hz, 1H), 4.78-4.75 (m, 1H), 4.66-4.57 (m, 2H), 4.34 (s, 1H), 3.98 (s, 2H), 3.93 (br s, 1H), 3.86-3.73 (m, 2H), 3.70-3.60 (m, 2H), 3.50-3.35 (m, 9H), 3.27-3.14 (m, 3H), 2.77 (dd, J = 12.9, 3.2 Hz, 1H), 2.52-2.42 (m, 3H), 2.40-2.17 (m, 8H), 1,91 (dd, J = 11.7, 4.3 Hz, 1H), 1.77-1.69 (m, 2H), 1.68-1.61 (m, 2H), 1.60-1.35 (m,13H), 1.32-1.21 (m, 7H), 1.21-1.05 (m, 6H), 0.94-0.75

### (m,10H); MSm/z 998.6152 [M+H]⁺.

### Production of Compound 11-19

Compound II-19 (isolated yield 23.1 mg, percent yield 72%) was obtained in a similar manner to [Production of Compound II-9] except that aniline was used in place of 2-aminoethan-1-ol.
¹H-NMR (500 MHz, CDCl₃, δ): 7.16 (t, J = 6.8 Hz, 2H), 6.70 (t, J = 7.2 Hz, 1H), 6.61 (d, J = 7.5 Hz, 2H), 5.87-5.79 (m, 2H), 5.74-5.65 (m, 2H), 5.43-5.36 (m, 2H), 4.94 (d, J = 10.9 Hz, 1H), 4.79-4.72 (m, 1H), 4.68 (s, 1H), 4.67-4.58 (m, 2H), 4.42 (s, 1H), 3.92 (br s, 1H), 3.87-3.73 (m, 5H), 3.73-3.60 (m, 2H), 3.43 (d, J = 10.9 Hz, 7H), 3.26-3.14 (m, 3H), 2.81 (dd, J = 12.9, 3.15 Hz, 1H), 2.53 (s, 1H), 2.51-2.44 (m, 2H), 2.36-2.19 (m, 4H), 1.90 (dd, J = 12.0, 3.4 Hz, 1H), 1.77-1.70 (m, 2H), 1.69-1.60 (m, 4H), 1.59-1.38 (m,14H), 1.31-1.21 (m, 7H), 1.13 (dd, J = 10.6, 6.6 Hz, 6H), 0.92 (s, 3H), 0.83 (d, J = 6.3 Hz, 3H), 0.74-0.81 (m, 4H); MSm/z 978.6411 [M+H]⁺.

### Production of Compound II-20

Triphenyl(propyl)phosphonium bromide (30.8 mg, 80.0 µmol) was dissolved in THF (228 µL) and cooled to 0°C. Then, a 1M potassium tert-butoxide THF solution (80.0 µL, 80.0 µmol) was added dropwise, and the mixture was stirred at the same temperature for 1 hour. 3,4α-Dihydro-5-oxo-ivermectin B1a (20.0 mg, 23.0 µmol) dissolved in THF (228 µL) was slowly added dropwise thereto, and the mixture was further stirred at the same temperature for 20 hours. A saturated aqueous solution of ammonium chloride was added to the reaction solution, the temperature was raised to room temperature, and the mixture was extracted with ethyl acetate. The organic layer was washed with saturated brine and dried over anhydrous sodium sulfate. After the solvent was distilled off under reduced pressure, the residue was purified by preparative thin-layer chromatography (n-hexane:ethyl acetate = 1:1) to obtain Compound II-20 (isolated yield 2.6 mg, percent yield 13%) as a mixture of isomers that were difficult to separate. The ¹H-NMR spectrum below is the analytical data of the main isomer.
¹H-NMR (500 MHz, CDCl₃, δ): 5.82 (d, J = 2.3 Hz, 1H), 5.77-5.66 (m, 2H), 5.57 (s, 1H), 5.44-5.34 (m, 2H), 4.98-4.91 (m, 1H), 4.76 (d, J = 3.4 Hz, 1H), 4.65 (s, 1H), 4.60-4.50 (m, 2H), 3.97 (s, 1H), 3.92 (br s, 1H), 3.87-3.73 (m, 2H), 3.70-3.59 (m, 2H), 3.39-3.52 (m, 7H), 3.24-3.14 (m, 3H), 2.75-2.67 (m, 1H), 2.63 (dd, J = 12.9, 3.7 Hz, 1H), 2.53-2.39 (m, 2H), 2.38-2.14 (m, 7H), 1.94-1.83 (m, 1H), 1.56-1.45 (m,13H), 1.82-1.33 (m,37H), 1.32-1.21 (m,12H), 1.21-1.08 (m,6),1.07-0.96 (m, 4H), 0.92 (t, J = 7.5 Hz, 3H), 0.83 (d, J = 6.8 Hz, 4H), 0.78 (d, J = 5.7 Hz, 6H); MSm/z 918.6136 [M+NH₄]⁺.

### Production of Compound 11-21 and Compound II-22

Compound II-6 (20.0 mg, 22.0 µmol) obtained in [Production of Compound II-6] was dissolved in THF (0.440 mL) and cooled to 0°C. Then, a 3M ethylmagnesium bromide THF solution (66.0 µL, 66.0 µmol) was added dropwise, and the mixture was stirred at the same temperature for 30 minutes. A saturated aqueous solution of ammonium chloride was added to the reaction solution, and the mixture was extracted with ethyl acetate. The organic layer was washed with saturated brine and dried over anhydrous sodium sulfate. After the solvent was distilled off under reduced pressure, the residue was purified by preparative thin-layer chromatography (n-hexane:ethyl acetate = 1:1) to obtain Compound II-21 (isolated yield 6.3 mg, percent yield 31%) and Compound II-22 (isolated yield 6.6 mg, percent yield 32%).

### (Compound II-21)

¹H-NMR (500 MHz, CDCl₃, δ): 5.83-5.81 (m, 1H), 5.75-5.69 (m, 2H), 5.63 (dd, J = 8.3, 1.4 Hz, 1H), 5.40-5.35 (m, 2H), 4.95 (d, J = 10.9 Hz, 1H), 4.79 (s, 1H), 4.76 (d, J = 3.4 Hz, 1H), 4.64 (dd, J = 7.2, 2.6 Hz, 2H), 4.43 (s, 1H), 4.38 (d, J = 8.0 Hz, 1H), 3.93 (br s, 1H), 3.85-3.75 (m, 2H), 3.69-3.60 (m, 2H), 3.50-3.42 (m, 7H), 3.26-3.15 (m, 3H), 2.80 (dd, J = 12.9, 3.2 Hz, 1H), 2.51-2.48 (m, 3H), 2.36-2.20 (m, 4H), 1.91-1.87 (m, 1H), 1.78-1.72 (m, 2H), 1.68-1.46 (m,25H), 1.45-1.37 (m, 4H), 1.26 (dd, J = 11.5, 6.3 Hz, 7H), 1.15 (d, J = 6.9 Hz, 3H), 1.10 (d, J = 6.3 Hz, 3H), 0.91 (td, J = 7.3,7.3, 2.6 Hz, 7H), 0.83 (d, J = 6.3 Hz, 3H), 0.78 (m, 4H); MSm/z 948.6349 [M+NH₄]⁺.

### (Compound II-22)

¹H-NMR (500 MHz, CDCl₃, δ): 5.84-5.82 (m, 1H), 5.74-5.68 (m, 2H), 5.63)dd, J = 8.1, 2.0 Hz, 1H), 5.40-5.35 (m, 2H), 4.96-4.94 (m, 1H), 4.76 (, J = 3.44 Hz, 1H), 4.66-4.60 (m, 2H), 4.58 (s, 1H), 4.48-4.44 (m, 1H), 4.41 (s, 1H), 3.93 (br s, 1H), 3.84-3.75 (m, 2H), 3.69-3.60 (m, 2H), 3.51-3.42 (m, 7H), 3.26-3.15 (m, 3H), 2.76 (dd, J = 12.6, 2.9 Hz, 1H), 2.50-2.43 (m, 3H), 2.36-2.20 (m, 4H), 1.91-1.88 (m, 1H), 1.79-1.70 (m, 3H), 1.67-1.64 (m, 2H), 1.62-1.49 (m,25H), 1.45-1.36 (m, 4H), 1.28-1.24 (m, 8H), 1.13 (dd, J = 12.0, 6.9 Hz, 6H), 0.92 (td, J = 7.5,7.5, 2.9 Hz, 6H), 0.84-0.77 (m, 7H); MSm/z 948.6349 [M+NH₄]⁺.

### Production of Compound II-23 and Compound II-24

Compound II-23 (isolated yield 8.4 mg, percent yield 8%) and Compound II-24 (isolated yield 78.1 mg, percent yield 71%) were obtained in a similar manner to [Production of Compound II-3] except that ethyl 2-(diethoxyphosphoryl)-2-fluoroacetate was used in place of ethyl 2-(diethoxyphosphoryl)acetate.

### (Compound II-23)

¹H-NMR (500 MHz, CDCl₃, δ): 5.88 (d, J = 10.9 Hz, 1H), 5.75-5.63 (m, 2H), 5.41-5.35 (m, 2H), 4.91 (d, J = 10.9 Hz, 1H), 4.76 (d, J = 3.4 Hz, 1H), 4.68-4.53 (m, 3H), 4.31-4.23 (m, 2H), 4.20 (s, 1H), 3.93 (br s, 1H), 3.87-3.74 (m, 2H), 3.71-3.55 (m, 3H), 3.51-3.38 (m, 7H), 3.27-3.14 (m, 3H), 2.58 (dd, J = 13.8, 5.2 Hz, 1H), 2.53-2.43 (m, 2H), 2.37-2.19 (m, 4H), 2.13-2.03 (m, 1H), 1.88-1.70 (m, 3H), 1.65-1.38 (m,20H), 1.38-1.23 (m,13H), 1.16 (d, J = 6.9 Hz, 3H), 0.91 (t, J = 7.5 Hz, 3H), 0.86-0.69 (m, 7H); MSm/z 980.5739 [M+NH₄]⁺.

### (Compound II-24)

¹H-NMR (500 MHz, CDCl₃, δ): 5.83 (d, J = 10.3 Hz, 1H), 5.74-5.62 (m, 2H), 5.41-5.34 (m, 2H), 5.00 (s, 1H), 4.93 (d, J = 10.9 Hz, 1H), 4.76 (d, J = 3.4 Hz, 1H), 4.61 (d, J = 2.3 Hz, 2H), 4.46 (br s, 1H), 4.27-4.23 (m, 2H), 3.92 (br s, 1H), 3.85-3.73 (m, 2H), 3.69-3.60 (m, 2H), 3.50-3.39 (m, 7H), 3.25-3.13 (m, 3H), 2.83-2.76 (m, 1H), 2.73 (dd, J = 12.9, 3.15 Hz, 1H), 2.48 (t, J = 6.9 Hz, 1H), 2.35-2.19 (m, 4H), 1.97-1.85 (m, 2H), 1.79-1.70 (m, 2H), 1.70-1.61 (m, 2H), 1.57-1.34 (m,17H), 1.34-1.22 (m, 11H), 1.15 (d, J = 7.5 Hz, 4H), 0.91 (t, J = 7.5 Hz, 3H), 0.72-0.87 (m, 8H); MSm/z 980.5739 [M+NH₄]⁺.

### Production of Compound II-25 and Compound II-26

Compound II-6 (20.0 mg, 22.0 µmol) obtained in [Production of Compound II-6] was dissolved in nitromethane (59.1 µL, 1.10 mmol), triethylamine (46.0 µL, 330 µmol) was added, and the mixture was stirred at room temperature for 1 hour. After the reaction solution was distilled off under reduced pressure, the residue was roughly purified by preparative thin-layer chromatography (n-hexane:ethyl acetate = 3:2), and further repurified by high-performance liquid chromatography to obtain Compound II-25 (isolated yield 1.1 mg, percent yield 5%) and Compound II-26 (isolated yield 1.0 mg, percent yield 5%).

### (Compound II-25)

¹H-NMR (500 MHz, CDCl₃, δ): 5.84-5.80 (m, 1H), 5.76-5.66 (m, 2H), 5.53 (dd, J = 8.0, 1.7 Hz, 1H), 5.41-5.37 (m, 2H), 5.31-5.28 (m, 1H), 4.95 (dd, J = 10.6, 1.4 Hz, 1H), 4.76 (d, J = 3.4 Hz, 1H), 4.66 (br s, 1H), 4.50-4.40 (m, 2H), 4.30 (s, 1H), 3.93 (br s, 1H), 3.85-3.75 (m, 2H), 3.69-3.60 (m, 2H), 3.50-3.41 (m, 7H), 3.26-3.14 (m, 3H), 2.77 (dd, J = 13.2, 3.4 Hz, 1H), 2.53-2.49 (m, 2H), 2.38-2.30 (m, 2H), 2.29-2.19 (m, 2H), 1.90-1.87 (m, 1H), 1.77-1.37 (m,20H), 1.28-1.24 (m, 7H), 1.17-1.10 (m,13H), 0.92 (t, J = 7.5 Hz, 3H), 0.86-0.77 (m, 2H); MSm/z 979.6013 [M+NH₄]⁺.

### (Compound II-26)

¹H-NMR (500 MHz, CDCl₃, δ): 5.85-5.82 (m, 1H), 5.77-5.68 (m, 2H), 5.59 (dd, J = 8.3, 2.0 Hz, 1H), 5.43-5.38 (m, 2H), 5.30-5.26 (m, 1H), 4.98-4.94 (m, 1H), 4.77 (d, J = 3.4 Hz, 1H), 4.65 (br s, 1H), 4.50-4.38 (m, 2H), 4.38 (s, 1H), 3.93 (br s, 1H), 3.86-3.82 (m, 1H), 3.79-3.75 (m, 1H), 3.71-3.62 (m, 2H), 3.51-3.42 (m, 7H), 3.26-3.15 (m, 3H), 2.79-2.77 (m, 1H), 2.53-2.48 (m, 2H), 2.36-2.18 (m, 4H), 1.90-1.87 (m, 1H), 1.77-1.37 (m,20H), 1.28-1.24 (m, 7H), 1.17-1.10 (m,13H), 0.92 (t, J = 7.5 Hz, 3H), 0.86-0.77 (m, 2H); MSm/z 979.6013 [M+NH₄]⁺.

### Production of Compound II-27 and Compound II-28

Compound II-27 (isolated yield 4.5 mg, percent yield 29%) and Compound II-28 (isolated yield 8.5 mg, percent yield 54%) were obtained in a similar manner to [Production of Compound II-4] except that Compound II-24 was used in place of Compound II-2, and a 1.5M diisobutylaluminum hydride toluene solution was used in place of a 1M diisobutylaluminum hydride dichloromethane solution.

### (Compound II-27)

¹H-NMR (500 MHz, CDCl₃, δ): 9.79 (d, J = 17.8 Hz, 1H), 5.90 (dt, J = 10.9, 2.6, 2.6 Hz, 1H), 5.80-5.66 (m, 2H), 5.43-5.36 (m, 2H), 4.95-4.93 (m, 1H), 4.76 (d, J = 3.4 Hz, 1H), 4.75 (s, 1H), 4.67-4.60 (m, 2H), 3.94 (br s, 1H), 3.86-3.74 (m, 2H), 3.70-3.61 (m, 2H), 3.51-3.47 (m, 1H), 3.45 (s, 3H), 3.42 (s, 3H), 3.24 (t, J = 8.9 Hz, 1H), 3.22-3.20 (m, 1H), 3.17 (t, 9.2 Hz, 1H), 3.04-2.99 (m, 1H), 2.67 (t, 8.6 Hz, 1H), 2.51-2.48 (m, 1H), 2.34 (dd, J = 13.2, 4.0 Hz, 2H), 2.29-2.20 (m, 2H), 1.96-1.93 (m, 2H), 1.89-1.86 (m, 2H), 1.76-1.73 (m, 1H), 1.67-1.49 (m, 8H), 1.45-1.40 (m, 3H), 1.39-1.36 (m, 4H), 1.31-1.24 (m, 8H), 1.16 (d, J = 6.9 Hz, 3H), 0.92 (t, J = 7.4 Hz, 3H), 0.84 (d, J = 6.9 Hz, 3H), 0.81-0.73 (m, 5H); MSm/z 982.6067 [M+NH₄]⁺
* Observed as a 2MeOH adduct during MS measurement.

### (Compound II-28)

¹H-NMR (500 MHz, CDCl₃, δ): 5.86 (dt, J = 10.7, 2.4 Hz, 1H), 5.76-5.64 (m, 2H), 5.43-5.35 (m, 2H), 4.95-4.92 (m, 1H), 4.76 (d, J = 2.9 Hz, 1H), 4.59 (d, J = 2.3 Hz, 2H), 4.27 (s, 1H), 4.25-4.12 (m, 2H), 3.93 (br s, 1H), 3.86-3.74 (m, 2H), 3.69-3.61 (m, 2H), 3.51-3.46 (m, 1H), 3.45 (s, 3H), 3.42 (s, 3H), 3.24 (t, J = 9.2 Hz, 1H), 3.21-3.19 (m, 1H), 3.16 (t, J = 9.2 Hz, 1H), 2.77-2.71 (m, 1H), 2.63 (dd, J = 12.0, 4.6 Hz, 1H), 2.52-2.44 (m, 1H), 2.35-2.32 (m, 2H), 2.28-2.20 (m, 2H), 1.89-1.84 (m, 2H), 1.82-1.72 (m, 5H), 1.67-1.64 (m, 2H), 1.58-1.48 (m, 8H), 1.45-1.36 (m, 4H), 1.31 (dd, J = 6.9, 4.6 Hz, 3H), 1.28-1.24 (m, 7H), 1.15 (d, J = 6.9 Hz, 3H), 0.92 (t, J = 7.4 Hz, 3H), 0.83 (d, J = 6.9 Hz, 3H), 0.80-0.72 (m, 5H); MSm/z 938.5821 [M+NH₄]⁺.

### Production of Compound II-29

Compound II-29 (isolated yield 1.1 mg, percent yield 17%) was obtained in a similar manner to [Production of Compound II-27 and Compound II-28] except that Compound II-23 was used in place of Compound II-24.
¹H-NMR (500 MHz, CDCl₃, δ): 5.84 (dt, J = 10.3, 2.3 Hz, 1H), 5.75-5.66 (m, 2H), 5.42-5.35 (m, 2H), 4.95-4.92 (m, 1H), 4.76 (d, J = 3.4 Hz, 1H), 4.65 (s, 1H), 4.62 (d, J = 1.7 Hz, 2H), 4.42-4.31 (m, 2H), 3.93 (br s, 1H), 3.86-3.74 (m, 2H), 3.69-3.59 (m, 2H), 3.51-3.47 (m, 1H), 3.45 (s, 3H), 3.42 (s, 3H), 3.26-3.20 (m, 2H), 3.16 (t, J = 9.2 Hz, 1H), 2.72-2.68 (m, 2H), 2.51-2.45 (m, 1H), 2.36-2.32 (m, 2H), 2.29-2.20 (m, 2H), 1.90-1.49 (m,11H), 1.45-1.37 (m, 4H), 1.31 (d, J = 6.9 Hz, 3H), 1.28-1.25 (m, 9H), 1.16 (d, J = 6.9 Hz, 3H), 0.92 (t, J = 7.2 Hz, 3H), 0.84-0.76 (m, 8H); MSm/z 938.5751 [M+NH₄]⁺.

### Production of Compound II-30 and Compound 11-31

Compound II-30 (isolated yield 16.4 mg, percent yield 75%) and Compound II-31 (isolated yield 3.4 mg, percent yield 15%) were obtained in a similar manner to [Production of Compound II-23 and Compound II-24] except that Compound II-6 was used in place of 3,4α-dihydro-5-oxo-ivermectin B1a.

### (Compound II-30)

¹H-NMR (500 MHz, CDCl₃, δ): 7.26-7.23 (m, 1H)m6.89-6.83 (m, 1H), 5.84 (dt, J = 10.3, 2.3 Hz, 1H), 5.76-5.67 (m, 2H), 5.42-5.36 (m, 2H), 4.95-4.93 (m, 1H), 4.76 (d, J = 3.4 Hz, 1H), 4.69-4.62 (m, 3H), 4.44 (s, 1H), 4.34-4.28 (m, 2H), 3.93 (br s, 1H), 3.86-3.74 (m, 2H), 3.69-3.62 (m, 2H), 3.50-3.45 (m, 1H), 3.44 (s, 3H), 3.42 (s, 3H), 3.25-3.20 (m, 2H), 3.16 (t, J = 9.2 Hz, 1H), 2.82 (d, J = 12.6, 3.4 Hz, 1H), 2.63-2.58 (m, 1H), 2.52-2.47 (m, 1H), 2.35-2.20 (m, 4H), 1.90-1.87 (m, 1H), 1.80-1.71 (m, 3H), 1.69-1.64 (m, 2H), 1.58-1.46 (m, 9H), 1.45-1.38 (m, 3H), 1.35 (t, J = 7.2 Hz, 3H), 1.28-1.20 (m,10H), 1.16 (d, J = 7.4 Hz, 3H), 0.91 (t, J = 7.2 Hz, 3H), 0.83 (d, J = 6.3 Hz, 3H), 0.80-0.75 (m, 4H); MSm/z 1006.5724 [M+NH₄]⁺.

### (Compound II-31)

¹H-NMR (500 MHz, CDCl₃, δ): 7.01-6.93 (m, 1H), 6.56 (d, J = 11.6 Hz, 1H), 5.87-5.83 (m, 1H), 5.76-5.68 (m, 2H), 5.43-5.38 (m, 2H), 4.95 (d, J = 10.9 Hz, 1H), 4.76 (d, J = 4.0 Hz, 1H), 4.71-4.66 (m, 3H), 4.51 (s, 1H), 4.30-4.26 (m, 2H), 3.93 (br s, 1H), 3.85-3.75 (m, 2H), 3.70-3.61 (m, 2H), 3.51-3.46 (m, 1H), 3.44 (s, 3H), 3.42 (s, 3H), 3.26-3.20 (m, 2H), 3.16 (t, J = 8.9 Hz, 1H), 2.84 (dd, J = 12.6, 3.4 Hz, 1H), 2.62-2.60 (m, 1H), 2.51-2.48 (m, 1H), 2.36-2.20 (m, 4H), 1.91-1.88 (m, 1H), 1.81-1.72 (m, 3H), 1.67-1.49 (m, 9H), 1.45-1.31 (m, 7H), 1.26 (dd, J = 10.6, 6.0 Hz, 7H), 1.21 (d, J = 6.3 Hz, 3H), 1.16 (d, J = 6.9 Hz, 3H), 0.92 (t, J = 7.4 Hz, 3H), 0.83 (d, J = 6.3 Hz, 3H), 0.80-0.76 (m, 4H); MSm/z 1006.5724 [M+NH₄]⁺.

### Production of Compound II-32 and Compound II-33

Compound II-32 (isolated yield 2.4 mg, percent yield 18%) and Compound II-33 (isolated yield 6.2 mg, percent yield 46%) were obtained in a similar manner to [Production of Compound II-27 and Compound II-28] except that Compound II-30 was used in place of Compound II-24.

### (Compound II-32)

¹H-NMR (500 MHz, CDCl₃, δ): 9.85 (d, J = 12.6 Hz, 1H), 7.04-7.00 (m, 2H), 5.87-5.84 (m, 1H), 5.78-5.67 (m, 2H), 5.43-5.37 (m, 2H), 4.96-4.94 (m, 1H), 4.77 (d, J = 3.4 Hz, 1H), 4.71-4.64 (m, 2H), 4.46 (s, 1H), 3.94 (br s, 1H), 3.36-3.74 (m, 2H), 3.69-3.60 (m, 2H), 3.51-3.46 (m, 1H), 3.44 (s, 3H), 3.42 (s, 3H), 3.26-3.20 (m, 2H), 3.17 (t, J = 9.2HZ,1H), 2.85-2.82 (m, 1H), 2.66-2.61 (m, 1H), 2.52-2.48 (m, 1H), 2.35-2.20 (m, 4H), 19.1-1.88 (m, 1H), 1.84-1.79 (m, 1H), 1.77-1.72 (m, 2H), 1.67-1.49 (m,10H), 1.45-1.36 (m, 5H), 1.31-1.30 (m, 1H), 1.28-1.22 (m, 9H), 1.16 (d, J = 6.9 Hz, 3H), 0.93 (t, J = 7.4 Hz, 3H), 0.84-0.76 (m, 7H); MSm/z 1008.6177 [M+NH₄]⁺

* Observed as a 2MeOH adduct during MS measurement.

### (Compound II-33)

¹H-NMR (500 MHz, CDCl₃, δ): 6.20-6.15 (m, 2H), 5.85-5.82 (m, 1H), 5.75-5.66 (m, 2H), 5.42-5.36 (m, 2H), 5.29 (s, 1H), 4.95-4.93 (m, 1H), 4.76 (d, J = 3.4 Hz, 1H), 4.69 (br s, 1H), 4.68-4.61 (m, 2H), 4.41 (s, 1H), 4.34 (s, 1H), 4.29 (s, 1H), 3.93 (br s, 1H), 3.85-.375 (m, 2H), 3.69-3.61 (m, 2H), 3.51-3.46 (m, 1H), 3.44 (s, 3H), 3.42 (s, 3H), 3.26-3.20 (m, 2H), 3.16 (t, J = 9.2 Hz, 1H), 2.80 (dd, J = 12.6, 3.4 Hz, 1H), 2.53-2.47 (m, 2H), 2.36-2.20 (m, 4H), 1.91-1.87 (m, 1H), 1.76-1.72 (m, 2H), 1.70-1.65 (m, 2H), 1.60-1.49 (m, 8H), 1.45-1.36 (m, 4H), 1.28-1.24 (m, 8H), 1.16 (dd, J = 6.6, 2.6 Hz, 6H), 0.92 (t, J = 7.4 Hz, 3H), 0.83 (d, J = 6.3 Hz, 3H), 0.80-0.75 (m, 4H); MSm/z 964.5808 [M+NH₄]⁺.

### Production of Compound II-34

Compound II-34 (isolated yield 6.5 mg, percent yield 62%) was obtained as a mixture of isomers that were difficult to separate in a similar manner to [Production of Compound II-21 and Compound II-22] except that a 0.7M allylmagnesium bromide diethyl ether solution was used in place of a 3M ethylmagnesium bromide THF solution.
¹H-NMR (500 MHz, CDCl₃, δ): 5.92-5.78 (m, 2H), 5.77-5.63 (m, 3H), 5.44-5.33 (m, 2H), 5.19-5.08 (m, 2H), 4.96 (d, J = 9.7 Hz, 1H), 4.77 (d, J = 3.4 Hz, 1H), 4.68-4.52 (m, 3H), 4.43 (s, 0.5H), 4.41 (s, 0.5H), 3.94 (br s, 1H), 3.86-3.73 (m, 2H), 3.71-3.60 (m, 2H), 3.53-3.45 (m, 1H), 3.44 (s, 3H), 3.43 (s, 3H), 3.27-3.20 (m, 2H), 3.17 (t, J = 8.9 Hz, 1H), 2.81-2.74 (m, 1H), 2.53-2.43 (m, 2H), 2.38-2.17 (m, 6H), 1.90 (dd, J = 12.0, 4.6 Hz, 1H), 1.78-1.60 (m, 8H), 1.59-1.38 (m,12H), 1.30-1.23 (m, 6H), 1.15 (dd, J = 6.6, 3.2 Hz, 3H), 1.11 (t, J = 6.6 Hz, 3H), 0.92 (t, J = 7.5 Hz, 3H), 0.84 (d, J = 6.9 Hz, 3H), 0.82-0.75 (m, 4H); MSm/z 960.6162 [M+NH₄]⁺.

### Production of Compound II-35

Compound II-35 (isolated yield 6.5 mg, percent yield 62%) was obtained in a similar manner to [Production of Compound II-21 and Compound II-22] except that a 0.9M benzylmagnesium bromide THF solution was used in place of a 3M ethylmagnesium bromide THF solution.
¹H-NMR (500 MHz, CDCl₃, δ): 7.33-7.21 (m, 5H), 5.87-5.81 (m, 1H), 5.77-5.66 (m, 2H), 5.44-5.34 (m, 2H), 4.96 (d, J = 9.7 Hz, 1H), 4.84-4.73 (m, 2H), 4.67-4.57 (m, 2H), 4.43 (s, 1H), 3.94 (br s, 1H), 3.87-3.73 (m, 2H), 3.71-3.60 (m, 2H), 3.52-3.46 (m, 1H), 3.44 (s, 3H), 3.43 (s, 3H), 3.28-3.20 (m, 2H), 3.17 (t, J = 9.2 Hz, 1H), 2.95-2.74 (m, 2H), 2.56-2.43 (m, 2H), 2.38-2.20 (m, 4H), 1.93-1.87 (m, 1H), 1.80-1.70 (m, 2H), 1.68-1.46 (m,14H), 1.46-1.36 (m, 3H), 1.33-1.21 (m, 8H), 1.16 (d, J = 7.5 Hz, 3H), 1.11 (d, J = 6.3 Hz, 3H), 0.95-0.81 (m, 7H), 0.81-0.75 (m, 4H); MSm/z 1010.6326 [M+NH₄]⁺.

### Production of Compound II-36

Compound II-36 (isolated yield 2.8 mg, percent yield 27%) was obtained as a mixture of isomers that were difficult to separate in a similar manner to [Production of Compound II-21 and Compound II-22] except that a 0.7M cyclopropylmagnesium bromide THF solution was used in place of a 3M ethylmagnesium bromide THF solution.
¹H-NMR (500 MHz, CDCl₃, δ): 5.85-5.76 (m, 1H), 5.75-5.66 (m, 2H), 5.45-5.34 (m, 2H), 4.95 (d, J = 10.9 Hz, 1H), 4.77 (d, J = 3.4 Hz, 1H), 4.72 (br s,0.4H), 4.68-4.57 (m, 2H), 4.55 (s, 0.6H), 4.36 (s, 0.4H), 4.34 (s, 0.6H), 4.03-3.98 (m,0.4H), 3.93 (br s, 1H), 3.86-3.74 (m,2.6H), 3.72-3.58 (m, 2H), 3.52-3.46 (m, 1H), 3.44 (s, 3H), 3.43-3.42 (m, 3H), 3.28-3.20 (m, 2H), 3.17 (t, J = 9.2 Hz, 1H), 2.82-2.74 (m, 1H), 2.57-2.43 (m, 3H), 2.39-2.19 (m, 4H), 1.94-1.86 (m, 1H), 1.79-1.70 (m, 2H), 1.68-1.47 (m,17H), 1.46-1.35 (m, 3H), 1.30-1.23 (m, 6H), 1.19-1.10 (m, 5H), 1.08-0.99 (m, 1H), 0.96-0.89 (m, 3H), 0.86-0.82 (m, 3H), 0.81-0.75 (m, 3H), 0.61-0.36 (m, 2H), 0.34-0.17 (m, 1H); MSm/z 965.5605 [M+Na]⁺.

### Production of Compound II-37

Compound II-37 (isolated yield 5.1 mg, percent yield 46%) was obtained as a mixture of isomers that were difficult to separate in a similar manner to [Production of Compound II-21 and Compound II-22] except that a 1M (4-fluorophenyl)magnesium bromide THF solution was used in place of a 3M ethylmagnesium bromide THF solution.
¹H-NMR (500 MHz, CDCl₃, δ): 7.44-7.35 (m, 2H), 7.06-6.98 (m, 2H), 5.89-5.82 (m, 1H), 5.77-5.57 (m, 4H), 5.43-5.36 (m, 2H), 4.96 (d, J = 11.5 Hz, 1H), 4.77 (d, J = 3.4 Hz, 1H), 4.72-4.64 (m, 2H), 4.63-4.57 (m, 2H), 3.94 (br s, 1H), 3.86-3.74 (m, 2H), 3.71-3.59 (m, 2H), 3.51-3.46 (m, 1H), 3.45 (s, 3H), 3.43 (s, 3H), 3.28-3.19 (m, 2H), 3.17 (t, J = 9.2 Hz, 1H), 2.83-2.78 (m, 1H), 2.56-2.45 (m, 3H), 2.37-2.18 (m, 5H), 1.93-1.85 (m, 1H), 1.80-1.71 (m, 2H), 1.65-1.46 (m,12H), 1.46-1.36 (m, 3H), 1.30-1.24 (m, 6H), 1.16 (t, J = 6.9 Hz, 3H), 1.09 (dd, J = 9.7, 6.9 Hz, 3H), 0.93 (t, J = 7.5 Hz, 3H), 0.86-0.75 (m, 7H); MSm/z 1019.5527 [M+Na]⁺.

### Production of Compound II-38 and Compound II-39

Compound II-3 (20.0 mg, 21.0 µmol) obtained in [Production of Compound II-3] was dissolved in THF (0.423 mL) and cooled to 0°C. Then, a 3M ethylmagnesium bromide THF solution (35.3 µL, 106.0 µmol) was added dropwise, and the mixture was stirred at the same temperature for 5 hours. A saturated aqueous solution of ammonium chloride was added to the reaction solution, and the mixture was extracted with ethyl acetate. The organic layer was washed with saturated brine and dried over anhydrous sodium sulfate. After the solvent was distilled off under reduced pressure, the residue was purified by preparative thin-layer chromatography (n-hexane:ethyl acetate = 1:1) and further repurified by preparative thin-layer chromatography (chloroform:diethyl ether = 1:1) to obtain Compound II-38 (isolated yield 1.0 mg, percent yield 5%) and Compound II-39 (isolated yield 3.6 mg, percent yield 18%).

### (Compound II-38)

¹H-NMR (500 MHz, CDCl₃, δ): 6.30-6.26 (m, 1H), 5.85-5.77 (m, 1H), 5.74-5.67 (m, 2H), 5.47-5.34 (m, 2H), 5.04 (s, 1H), 4.99-4.89 (m, 1H), 4.80-4.74 (m, 1H), 4.66 (s, 2H), 3.94 (br s, 1H), 3.88-3.73 (m, 2H), 3.70-3.59 (m, 2H), 3.52-3.46 (m, 1H), 3.44 (s, 3H), 3.43 (s, 3H), 3.27-3.20 (m, 2H), 3.17 (t, J = 9.2 Hz, 1H), 2.92-2.82 (m, 1H), 2.71-2.61 (m, 1H), 2.57-2.44 (m, 3H), 2.39-2.19 (m, 4H), 1.95-1.88 (m, 1H), 1.84-1.72 (m, 4H), 1.68-1.38 (m,12H), 1.30-1.24 (m, 6H), 1.19-1.12 (m, 6H), 1.10-1.02 (m, 5H), 0.98-0.87 (m, 4H), 0.87-0.75 (m, 7H); MSm/z 946.5859 [M+NH₄]⁺.

### (Compound II-39)

¹H-NMR (500 MHz, CDCl₃, δ): 5.84-5.78 (m, 1H), 5.73-5.66 (m, 2H), 5.59-5.53 (m, 1H), 5.43-5.32 (m, 2H), 5.17 (s, 1H), 4.96 (d, J = 9.7 Hz, 1H), 4.77 (d, J = 3.4 Hz, 1H), 4.62 (s, 2H), 3.93 (br s, 1H), 3.87-3.74 (m, 2H), 3.71-3.60 (m, 2H), 3.51-3.45 (m, 1H), 3.44 (s, 3H), 3.43 (s, 3H), 3.27-3.20 (m, 2H), 3.17 (t, J = 9.2 Hz, 1H), 2.77 (dd, J = 12.6, 4.0 Hz, 1H), 2.53-2.44 (m, 2H), 2.39-2.19 (m, 4H), 1.95-1.86 (m, 1H), 1.80-1.37 (m,21H), 1.31-1.24 (m, 6H), 1.15 (d, J = 6.9 Hz, 3H), 1.08 (d, J = 6.3 Hz, 3H), 0.95-0.86 (m,10H), 0.86-0.75 (m, 9H); MSm/z 976.6240 [M+NH₄]⁺.

### Production of Compound II-40 and Compound II-41

Compound II-40 (isolated yield 1.9 mg, percent yield 10%) and Compound II-41 (isolated yield 2.9 mg, percent yield 15%) were obtained in a similar manner to [Production of Compound II-38 and Compound 11-39] except that a 1M methylmagnesium bromide dibutyl diethyl ether solution was used in place of a 3M ethylmagnesium bromide THF solution.

### (Compound II-40)

¹H-NMR (500 MHz, CDCl₃, δ): 6.28 (s, 1H), 5.83-5.79 (m, 1H), 5.72-5.68 (m, 2H), 5.44-5.32 (m, 2H), 5.03 (s, 1H), 4.95 (br d, J = 10.3 Hz, 1H), 4.76 (d, J = 4.0 Hz, 1H), 4.67-4.64 (m, 2H), 3.93 (br s, 1H), 3.86-3.80 (m, 1H), 3.80-3.73 (m, 1H), 3.71-3.60 (m, 2H), 3.51-3.46 (m, 1H), 3.44 (s, 3H), 3.42 (s, 3H), 3.27-3.19 (m, 2H), 3.16 (t, J = 9.2 Hz, 1H), 2.88-2.83 (m, 1H), 2.67-2.60 (m, 1H), 2.52-2.45 (m, 1H), 2.37-2.30 (m, 2H), 2.26 (s, 3H), 2.27-2.19 (m, 2H), 1.93-1.88 (m, 1H), 1.82-1.79 (m, 1H), 1.78-1.73 (m, 2H), 1.69-1.35 (m,15H), 1.27 (d, J = 6.3 Hz, 3H), 1.29-1.23 (m, 1H), 1.25 (d, J = 6.3 Hz, 3H), 1.15 (d, J = 6.9 Hz, 3H), 1.13 (d, J = 6.9 Hz, 3H), 0.92 (t, J = 7.4 Hz, 3H), 0.83 (d, J = 6.9 Hz, 3H), 0.81-0.79 (m, 1H), 0.78 (d, J = 6.3 Hz, 3H); MSm/z 932.5686 [M+NH₄]⁺.

### (Compound 11-41)

¹H-NMR (500 MHz, CDCl₃, δ): 5.84-5.80 (m, 1H), 5.76-5.74 (m, 1H), 5.71-5.68 (m, 2H), 5.40 (d, J = 2.9 Hz, 1H), 5.39-5.33 (m, 1H), 5.15 (s, 1H), 4.95 (br d, J = 12.6 Hz, 1H), 4.76 (d, J = 3.4 Hz, 1H), 4.67-4.59 (m, 2H), 3.93 (br s, 1H), 3.86-3.80 (m, 1H), 3.80-3.74 (m, 1H), 3.71-3.58 (m, 2H), 3.52-3.45 (m, 2H), 3.44 (s, 3H), 3.42 (s, 3H), 3.26-3.19 (m, 2H), 3.16 (t, J = 9.2 Hz, 1H), 2.80-2.76 (m, 1H), 2.52-2.42 (m, 2H), 2.37-2.19 (m, 5H), 1.92-1.88 (m, 1H), 1.78-1.63 (m, 3H), 1.59-1.42 (m,12H), 1.41 (s, 3H), 1.40 (s, 3H), 1.27 (d, J = 6.3 Hz, 4H), 1.25 (d, J = 6.3 Hz, 4H), 1.29-1.23 (m, 2H), 1.14 (d, J = 6.9 Hz, 3H), 1.07 (d, J = 6.3 Hz, 3H), 0.92 (t, J = 7.4 Hz, 3H), 0.83 (d, J = 6.9 Hz, 3H), 0.80-0.76 (m, 4H); MSm/z 948.5939 [M+NH₄]⁺.

### Production of Compound II-42

Acetonitrile (3.48 µL, 0.067 mmol) was added to THF (111 µL), and then a 2.0M lithium diisopropylamide THF solution (27.7 µL, 0.055 mmol) was added dropwise at -78°C, and the mixture was stirred at the same temperature for 30 minutes. Then, Compound II-6 (10 mg, 0.011 mmol) obtained in [Production of Compound II-6] dissolved in THF (111 µL) was added, and the mixture was stirred at the same temperature for 4 hours. A saturated aqueous sodium chloride solution was added to the reaction solution, and the mixture was extracted with ethyl acetate. The organic layer was washed with saturated brine and dried over anhydrous sodium sulfate. After the solvent was distilled off under reduced pressure, the residue was purified by preparative thin-layer chromatography (n-hexane:ethyl acetate = 1:1) to obtain Compound II-42 (isolated yield 3.6 mg, percent yield 34%) as a mixture of isomers that were difficult to separate.
¹H-NMR (500 MHz, CDCl₃, δ): 5.87-5.81 (m, 1H), 5.78-5.67 (m, 3H), 5.44-5.36 (m, 2H), 4.96 (d, J = 11.5 Hz, 1H), 4.84-4.79 (m, 1H), 4.78 (d, J = 2.9 Hz, 1H), 4.71-4.60 (m, 2H), 4.38 (s, 0.65H), 4.32-4.29 (m,0.35H), 3.95 (br s, 1H), 3.87-3.81 (m, 1H), 3.81-3.74 (m, 1H), 3.72-3.60 (m, 2H), 3.53-3.47 (m, 1H), 3.45 (s, 3H), 3.44 (s, 3H), 3.28-3.20 (m, 2H), 3.18 (t, J = 9.2 Hz, 1H), 2.83-2.75 (m, 1H), 2.71-2.47 (m, 4H), 2.39-2.18 (m, 4H), 1.93-1.86 (m, 1H), 1.84-1.71 (m, 2H), 1.70-1.62 (m, 2H), 1.61-1.36 (m,11H), 1.29 (d, J = 5.7 Hz, 3H), 1.27 (d, J = 6.3 Hz, 4H), 1.20-1.11 (m, 6H), 0.93 (t, J = 7.4 Hz, 3H), 0.85 (d, J = 6.9 Hz, 3H), 0.83-0.75 (m, 5H); MSm/z 959.5852 [M+NH₄]⁺.

### Production of Compound II-43

Compound II-25 (6 mg, 6.24 µmol) obtained in [Production of Compound II-25 and Compound II-26] was dissolved in a mixed solution of ethanol (125 µL) and water (25 µL), and ammonium chloride (3.34 mg, 0.062 mmol) and iron powder (3.48 mg, 0.062 mmol) were added, followed by stirring at 80°C overnight. A saturated aqueous sodium hydrogen carbonate solution was added to the reaction solution, and the mixture was extracted with a mixed solution of chloroform and methanol. The organic layer was washed with saturated brine and dried over anhydrous sodium sulfate. After the solvent was distilled off under reduced pressure, the residue was purified by preparative thin-layer chromatography (chloroform:methanol = 9:1) to obtain Compound II-43 (isolated yield 1.2 mg, percent yield 21%).
¹H-NMR (500 MHz, CDCl₃, δ): 5.89-5.79 (m, 1H), 5.76-5.66 (m, 2H), 5.61-5.52 (m,0.5H), 5.42-5.25 (m, 2H), 5.01-4.94 (m, 1H), 4.80-4.76 (m, 1H), 4.75-4.57 (m, 2H), 4.42-4.38 (m,0.5H), 3.94 (br s, 1H), 3.88-3.73 (m, 2H), 3.73-3.60 (m, 2H), 3.54-3.46 (m, 1H), 3.46-3.41 (m, 6H), 3.28-3.20 (m, 2H), 3.17 (t, J = 8.9 Hz, 1H), 2.97-2.87 (m, 1H), 2.84-2.72 (m, 1H), 2.54-2.40 (m, 2H), 2.39-2.21 (m, 5H), 1.98-1.84 (m, 1H), 1.80-1.63 (m, 3H), 1.61-1.34 (m,14H), 1.31-1.24 (m, 7H), 1.20-1.08 (m, 6H), 0.93 (t, J = 7.2 Hz, 3H), 0.84 (d, J = 6.9 Hz, 4H), 0.81-0.75 (m, 4H); MSm/z 932.5745 [M+H]⁺.

### Production of Compound II-44

Compound II-44 (isolated yield 6.0 mg, percent yield 36%) was obtained as a mixture of isomers that were difficult to separate in a similar manner to [Production of Compound 11-42], except that dimethyl sulfone was used in place of acetonitrile in [Production of Compound II-42].
¹H-NMR (500 MHz, CDCl₃, δ): 5.87-5.61 (m, 4H), 5.43-5.36 (m, 2H), 5.22-5.13 (m, 1H), 4.96 (br d, J = 10.3 Hz, 2H), 4.78 (d, J = 3.4 Hz, 1H), 4.68-4.61 (m, 2H), 4.43 (s, 0.8H), 4.33 (s, 0.2H), 3.95 (br s, 1H), 3.87-3.81 (m, 1H), 3.81-3.75 (m, 1H), 3.71-3.62 (m, 2H), 3.53-3.47 (m, 1H), 3.45 (s, 3H), 3.44 (s, 3H), 3.38-3.29 (m, 1H), 3.28-3.20 (m, 2H), 3.18 (t, J = 9.2 Hz, 1H), 3.12-3.07 (m, 1H), 3.05 (s, 2.4H), 3.04-3.02 (m,0.6H), 2.81-2.74 (m, 1H), 2.57-2.48 (m, 2H), 2.38-2.20 (m, 4H), 1.93-1.85 (m, 1H), 1.68-1.65 (m, 3H), 1.82-1.35 (m,16H), 1.28 (d, J = 6.3 Hz, 2H), 1.27 (d, J = 6.3 Hz, 2H), 1.18 (d, J = 6.3 Hz, 3H), 1.11 (d, J = 6.3 Hz, 3H), 0.93 (t, J = 7.4 Hz, 3H), 0.84 (d, J = 6.9 Hz, 3H), 0.81-0.75 (m, 4H); MSm/z 1012.5662 [M+H]⁺.

### Production of Compound II-45

Compound II-45 (isolated yield 14.0 mg, percent yield 63%) was obtained as a mixture of isomers that were difficult to separate in a similar manner to [Production of Compound II-42], except that allyl acetate was used in place of acetonitrile in [Production of Compound II-42].
¹H-NMR (500 MHz, CDCl₃, δ): 5.96-5.88 (m, 1H), 5.86-5.82 (m, 1H), 5.77-5.63 (m, 3H), 5.42-5.30 (m, 2H), 5.27-5.23 (m, 1H), 5.03-4.93 (m, 2H), 4.80-4.67 (m, 2H), 4.66-4.51 (m, 4H), 4.43 (s, 0.75H), 4.37 (s, 0.25H), 3.94 (br s, 1H), 3.87-3.75 (m, 2H), 3.71-3.61 (m, 2H), 3.53-3.46 (m, 1H), 3.45 (s, 3H), 3.44 (s, 3H), 3.27-3.20 (m, 2H), 3.18 (t, J = 8.6 Hz, 1H), 2.81-2.76 (m, 1H), 2.68-2.44 (m, 5H), 2.38-2.20 (m, 5H), 1.93-1.87 (m, 1H), 1.79-1.64 (m, 5H), 1.61-1.37 (m,12H), 1.31-1.24 (m, 7H), 1.19-1.15 (m, 3H), 1.12 (d, J = 6.9 Hz, 3H), 0.93 (t, J = 7.4 Hz, 3H), 0.84 (d, J = 6.9 Hz, 3H), 0.80-0.75 (m, 4H); MSm/z 1018.6096 [M+NH₄]⁺.

### Production of Compound II-46

Compound II-45 (50.3 mg, 0.050 mmol) obtained in [Production of Compound II-45] was dissolved in dichloromethane (1.0 mL), and morpholine (43.8 µL, 0.502 mmol) and tetrakis(triphenylphosphine)palladium (5.81 mg, 5.02 µmol) were sequentially added, followed by stirring at room temperature for 1 hour. 1M hydrochloric acid was added to the reaction solution, and the mixture was extracted with chloroform. After the solvent was distilled off under reduced pressure, the residue was purified by silica gel column chromatography (chloroform/methanol system) to obtain Compound II-46 (isolated yield 41.8 mg, percent yield 87%) as a mixture of isomers that were difficult to separate.
¹H-NMR (500 MHz, CDCl₃, δ): 5.84 (d, J = 10.3 Hz, 1H), 5.78-5.63 (m, 3H), 5.43-5.35 (m, 2H), 5.03-4.93 (m, 2H), 4.81-4.75 (m, 1H), 4.72-4.58 (m, 2H), 4.44 (s, 0.75H), 4.38 (s, 0.25H), 3.95 (br s, 1H), 3.87-3.82 (m, 1H), 3.81-3.75 (m, 1H), 3.71-3.62 (m, 2H), 3.53-3.47 (m, 1H), 3.45 (s, 3H), 3.44 (s, 3H), 3.29-3.09 (m, 4H), 2.82-2.75 (m, 1H), 2.66-2.59 (m, 2H), 2.55-2.46 (m, 2H), 2.38-2.18 (m, 5H), 1.93-1.88 (m, 1H), 1.80-1.73 (m, 2H), 1.69-1.37 (m,15H), 1.30-1.25 (m, 8H), 1.19-1.10 (m, 6H), 0.93 (t, J = 7.4 Hz, 3H), 0.85 (d, J = 6.9 Hz, 3H), 0.81-0.76 (m, 4H); MSm/z 978.5805 [M+NH₄]⁺.

### Production of Compound II-47

Compound II-46 (11 mg, 0.011 mmol) obtained in [Production of Compound II-46] was dissolved in DMF (229 µL), and 2,2,2-trifluoroethane-1-amine hydrochloride (7.75 mg, 0.057 mmol), DIPEA (20.0 µL, 0.114 mmol) and HATU (13.1 mg, 0.034 mmol) were sequentially added, followed by stirring at room temperature for 30 minutes. A saturated aqueous sodium hydrogen carbonate solution was added to the reaction solution, and the mixture was extracted with diethyl ether. The organic layer was washed with water and saturated brine sequentially, and dried over anhydrous sodium sulfate. After the solvent was distilled off under reduced pressure, the residue was purified by preparative thin-layer chromatography (n-hexane:ethyl acetate = 1:2) to obtain Compound II-47 (isolated yield 9.2 mg, percent yield 77%) as a mixture of isomers that were difficult to separate.
¹H-NMR (500 MHz, CDCl₃, δ): 7.01 (t, J = 6.6Hz,0.8H), 6.68 (t, J = 6.3Hz,0.2H), 5.86-5.82 (m, 1H), 5.78-5.63 (m, 3H), 5.43-5.63 (m, 2H), 4.99-4.94 (m, 2H), 4.92-4.83 (m, 1H), 4.78 (d, J = 3.4 Hz, 1H), 4.65 (dd, J = 7.4, 2.3 Hz, 2H), 4.41 (s, 0.8H), 4.34-4.28 (m,0.2H), 4.01-3.92 (m, 2H), 3.91-3.74 (m, 3H), 3.72-3.61 (m, 2H), 3.53-3.46 (m, 1H), 3.45 (s, 3H), 3.43 (s, 3H), 3.28-3.20 (m, 2H), 3.17 (t, J = 9.2 Hz, 1H), 2.81 (s, 1H), 2.61-2.46 (m, 5H), 2.38-2.20 (m, 4H), 1.93-1.87 (m, 1H), 1.81-1.70 (m, 4H), 1.69-1.65 (m, 1H), 1.62-1.37 (m,13H), 1.28 (d, J = 6.3 Hz, 3H), 1.26 (d, J = 6.3 Hz, 3H), 1.17 (d, J = 6.9 Hz, 3H), 1.10 (d, J = 6.9 Hz, 3H), 0.93 (t, J = 7.4 Hz, 3H), 0.85 (d, J = 6.9 Hz, 3H), 0.82-0.75 (m, 4H); MSm/z 1059.598 [M+NH₄]⁺.

### Production of Compound II-48

Compound II-43 (6.0 mg, 6.44 µmol) obtained in [Production of Compound II-43] was dissolved in THF (257 µL), and di(1H-imidazol-1-yl)methanone (1.36 mg, 8.37 µmol) and DMAP (0.079 mg, 0.644 µmol) were sequentially added, followed by stirring under heating and refluxing for 2.5 hours. A saturated aqueous solution of ammonium chloride was added to the reaction solution, and the mixture was extracted with chloroform. After the solvent was distilled off under reduced pressure, the residue was purified by preparative thin-layer chromatography (n-hexane:ethyl acetate = 1:5) to obtain Compound II-48 (isolated yield 1.9 mg, percent yield 31%).
¹H-NMR (500 MHz, CDCl₃, δ): 5.86-5.79 (m, 1H), 5.77-5.67 (m, 3H), 5.51 (q, J = 8.0 Hz, 1H), 5.44-5.35 (m, 2H), 4.97 (br d, J = 10.9 Hz, 1H), 4.84 (br s, 1H), 4.78 (d, J = 3.4 Hz, 1H), 4.71-4.60 (m, 2H), 4.18-4.15 (m, 1H), 3.95 (br s, 1H), 3.87-3.81 (m, 1H), 3.81-3.73 (m, 2H), 3.72-3.61 (m, 2H), 3.53-3.46 (m, 1H), 3.45 (s, 3H), 3.44 (s, 3H), 3.36-3.32 (m, 2H), 3.30-3.21 (m, 2H), 3.18 (t, J = 8.9 Hz, 1H), 2.81 (dd, J = 13.2, 2.9 Hz, 1H), 2.60-2.48 (m, 2H), 2.38-2.20 (m, 4H), 1.94-1.87 (m, 1H), 1.81-1.72 (m, 2H), 1.69-1.65 (m, 1H), 1.62-1.36 (m,11H), 1.29 (d, J = 6.3 Hz, 3H), 1.28-1.22 (m, 4H), 1.17 (d, J = 6.9 Hz, 3H), 1.15-1.11 (m, 3H), 0.94 (t, J = 7.4 Hz, 3H), 0.85 (d, J = 6.3 Hz, 3H), 0.83-0.76 (m, 5H); MSm/z 975.5797 [M+NH₄]⁺.

### Production of Compound II-49

Compound II-43 (6.0 mg, 6.44 µmol) obtained in [Production of Compound II-43] was dissolved in THF (257 µL), and triethylamine (1.17 µL, 8.37 µmol) and 2-chloroacetyl chloride (0.567 µL, 7.08 µmol) were sequentially added at 0°C, followed by stirring at the same temperature for 30 minutes. A saturated aqueous sodium hydrogen carbonate solution was added to the reaction solution, and the mixture was extracted with chloroform. After the solvent was distilled off under reduced pressure, the residue was dissolved in THF (257 µL), sodium hydride (0.843 mg, 0.019 mmol) was added at 0°C, and the mixture was stirred at the same temperature for 30 minutes. Then, the mixture was stirred under heating and refluxing for 1 hour. A saturated aqueous solution of ammonium chloride was added to the reaction solution, and the mixture was extracted with chloroform. After the solvent was distilled off under reduced pressure, the residue was purified by preparative thin-layer chromatography (n-hexane:acetone = 1:1) to obtain Compound II-49 (isolated yield 1.1 mg, percent yield 18%).
¹H-NMR (500 MHz, CDCl₃, δ): 6.44-6.35 (m, 1H), 5.80-5.74 (m, 2H), 5.66-5.60 (m, 1H), 5.49-5.44 (m, 1H), 5.43-5.38 (m, 2H), 5.14-5.01 (m, 2H), 4.81 (d, J = 3.4 Hz, 1H), 4.69 (dd, J = 14.3, 2.3 Hz, 1H), 4.65-4.59 (m, 1H), 4.57-4.51 (m, 1H), 4.22-4.08 (m, 3H), 3.99 (br s, 1H), 3.87-3.81 (m, 1H), 3.78 (dd, J = 9.5, 6.0 Hz, 1H), 3.72-3.60 (m, 2H), 3.53-3.46 (m, 2H), 3.43 (s, 3H), 3.42 (s, 3H), 3.40 (s, 1H), 3.28-3.20 (m, 2H), 3.18 (t, J = 9.2 Hz, 1H), 2.92 (dd, J = 13.2, 3.4 Hz, 1H), 2.63-2.54 (m, 2H), 2.38-2.23 (m, 4H), 2.17-2.11 (m, 1H), 1.89-1.69 (m, 4H), 1.69-1.39 (m, 7H), 1.33-1.24 (m,10H), 1.22-1.10 (m, 8H), 0.93 (t, J = 7.4 Hz, 3H), 0.86-0.77 (m, 8H); MSm/z 994.5518 [M+Na]⁺.

### Production of Compound II-50

Compound II-6 (15 mg, 0.017 mmol) obtained in [Production of Compound II-6] was dissolved in methanol (333 µL), and potassium carbonate (3.45 mg, 0.025 mmol) and 1-((isocyanomethyl)sulfonyl)-4-methylbenzene (3.90 mg, 0.020 mmol) were sequentially added, followed by stirring at 60°C for 2 hours. After the reaction solution was distilled off under reduced pressure, the residue was dissolved in chloroform and washed with a saturated aqueous solution of ammonium chloride. After the solvent was distilled off under reduced pressure, the residue was purified by preparative thin-layer chromatography (n-hexane:ethyl acetate = 1:3) to obtain Compound II-50 (isolated yield 10.8 mg, percent yield 69%).
¹H-NMR (500 MHz, CDCl₃, δ): 7.88 (s, 1H), 7.12 (s, 1H), 7.04-6.82 (m, 1H), 6.45 (s, 1H), 5.91-5.81 (m, 1H), 5.79-5.69 (m, 1H), 5.51-5.38 (m, 2H), 4.97 (br d, J = 10.3 Hz, 1H), 4.83-4.76 (m, 1H), 4.76-4.66 (m, 3H), 3.95 (br s, 1H), 3.89-3.81 (m, 1H), 3.81-3.73 (m, 2H), 3.73-3.60 (m, 3H), 3.54-3.47 (m, 1H), 3.45 (s, 3H), 3.43 (s, 3H), 3.31-3.21 (m, 2H), 3.17 (t,1H), 2.91-2.83 (m, 1H), 2.71-2.61 (m, 1H), 2.57-2.47 (m, 1H), 2.40-2.19 (m, 5H), 1.95-1.88 (m, 1H), 1.87-1.80 (m, 2H), 1.80-1.73 (m, 1H), 1.68 (d, J = 12.6 Hz, 1H), 1.63-1.33 (m,12H), 1.32-1.22 (m, 9H), 1.17 (d, J = 6.9 Hz, 3H), 0.93 (t, J = 7.4 Hz, 3H), 0.89-0.82 (m, 4H), 0.80 (d, J = 5.2 Hz, 3H); MSm/z 940.5425 [M+H]⁺.

### Production of Compound II-51

Compound II-51 (isolated yield: 19.4 mg, percent yield: 90%) was obtained as a mixture of isomers that were difficult to separate in a similar method to [Production of Compound II-47], except that aniline was used in place of 2,2,2-trifluoroethane 1-amine hydrochloride in [Production of Compound II-47].
¹H-NMR (500 MHz, CD₃OD, δ): 7.90 (s, 1H), 7.57-7.53 (m, 2H), 7.32-7.27 (m, 2H), 7.11-7.06 (m, 1H), 5.94-5.81 (m, 2H), 5.74-5.59 (m, 2H), 5.35 (d, J = 4.0 Hz, 1H), 5.19-5.09 (m, 2H), 5.06-4.98 (m, 1H), 4.80 (br d, J = 3.4 Hz, 1H), 4.69-4.55 (m, 2H), 4.41 (s, 0.35H), 4.40-4.37 (m,0.65H), 3.99 (br s, 1H), 3.88 (dq, J = 9.2, 6.3 Hz, 1H), 3.76-3.63 (m, 3H), 3.46-3.39 (m, 7H), 3.28-3.24 (m, 1H), 3.19 (t, J = 9.2 Hz, 1H), 3.05-2.99 (m, 1H), 2.81-2.75 (m, 1H), 2.68-2.56 (m, 2H), 2.51-2.38 (m, 2H), 2.34-2.26 (m, 4H), 2.08-2.01 (m, 1H), 1.90-1.85 (m, 1H), 1.75-1.39 (m,16H), 1.32-1.28 (m, 1H), 1.25 (d, J = 6.3 Hz, 3H), 1.23 (d, J = 6.3 Hz, 3H), 1.17 (d, J = 6.9 Hz, 3H), 1.15-1.10 (m, 3H), 0.97 (t, J = 7.4 Hz, 3H), 0.88 (d, J = 6.9 Hz, 3H), 0.83-0.78 (m, 4H); MSm/z 1036.6001 [M+H]⁺.

### Production of Compound II-52 and Compound II-53

(Bromomethyl)triphenylphosphonium bromide (10.5 g, 24 mmol) was dissolved in toluene (91.4 mL) and cooled to 0°C. Then, a 1M potassium tert-butoxide THF solution (22.6 mL, 22.6 mmol) was added dropwise, and the mixture was stirred at the same temperature for 1 hour. Then, the mixture was cooled to -20°C, 3,4α-dihydro-5-oxo-ivermectin B1a (6.00 g, 6.86 mmol) dissolved in toluene (45.7 mL) was slowly added dropwise, and the temperature was raised to 0°C. After stirring at the same temperature for 2 hours, a saturated aqueous solution of ammonium chloride was added to the reaction solution, the temperature was raised to room temperature, and the mixture was extracted with ethyl acetate. The organic layer was washed with saturated brine and dried over anhydrous sodium sulfate. After the solvent was distilled off under reduced pressure, the residue was purified by silica gel column chromatography (n-hexane/ethyl acetate system) and high-performance liquid chromatography to obtain Compound II-52 (isolated yield 1.01 g, percent yield 16%) and Compound II-53 (isolated yield 2.03 g, percent yield 31%).

### (Compound II-52)

¹H-NMR (500 MHz, CDCl₃, δ): 6.41-6.34 (m, 1H), 5.87-5.80 (m, 1H), 5.75-5.66 (m, 2H), 5.43-5.34 (m, 2H), 5.30-5.28 (m, 1H), 4.97-4.91 (m, 1H), 4.78-4.74 (m, 1H), 4.72-4.69 (m, 1H), 4.68-4.63 (m, 2H), 4.62-4.55 (m, 1H), 3.92 (br s, 1H), 3.86-3.72 (m, 2H), 3.69-3.60 (m, 2H), 3.51-3.45 (m, 1H), 3.43 (s, 3H), 3.42 (s, 3H), 3.26-3.13 (m, 3H), 2.82-2.76 (m, 1H), 2.53-2.45 (m, 2H), 2.36-2.17 (m, 4H), 2.03 (s, 1H), 1.91-1.86 (m, 1H), 1.77-1.61 (m, 4H), 1.58-1.34 (m,11H), 1.29-1.22 (m, 7H), 1.15 (d, J = 6.9 Hz, 6H), 0.91 (s, 3H), 0.84-0.81 (m, 3H), 0.80-0.74 (m, 4H).

### (Compound II-53)

¹H-NMR (500 MHz, CDCl₃, δ): 6.38-6.39 (m, 1H), 5.86-5.90 (m, 1H), 5.69-5.78 (m, 2H), 5.36-5.43 (m, 2H), 4.95 (br d, J = 11.5 Hz, 1H), 4.76 (d, J = 3.4 Hz, 1H), 4.62 (dd, J = 13.7, 1.7 Hz, 1H), 4.53 (s, 1H), 4.44 (dd, J = 13.7, 2.3 Hz, 1H), 4.36 (s, 1H), 3.93 (br s, 1H), 3.79-3.85 (m, 1H), 3.74-3.79 (m, 1H), 3.59-3.70 (m, 2H), 3.46-3.52 (m, 1H), 3.44 (s, 3H), 3.42 (s, 3H), 3.19-3.26 (m, 2H), 3.08-3.19 (m, 2H), 2.44-2.58 (m, 2H), 2.40 (dd, J = 13.2, 4.0 Hz, 1H), 2.30-2.37 (m, 2H), 2.20-2.28 (m, 2H), 2.17 (s, 1H), 1.96-2.04 (m, 1H), 1.82-1.90 (m, 2H), 1.74-1.79 (m, 1H), 1.45-1.67 (m, 9H), 1.42-1.44 (m, 1H), 1.37-1.41 (m, 2H), 1.23-1.29 (m,10H), 1.15 (d, J = 6.9 Hz, 3H), 0.92 (t, J = 7.2 Hz, 3H), 0.83 (d, J = 6.9 Hz, 3H), 0.72-0.80 (m, 4H).

### Production of Compound II-54

A mixture of Compound II-52 and Compound II-53 (30 mg, 0.032 mmol) obtained in [Production of Compound II-52 and Compound II-53] was dissolved in toluene (640 µL), and 2-(tributylstannyl)thiazole (14.7 µL, 0.048 mmol) and tetrakis(triphenylphosphine)palladium (3.70 mg, 0.0032 mmol) were added, and the mixture was stirred at 120°C for 3 hours using a microwave reactor. A saturated aqueous sodium bicarbonate solution was added to the reaction solution to stop the reaction. After extraction with ethyl acetate, the organic layer was washed with saturated brine and dried over anhydrous sodium sulfate. After the solvent was distilled off under reduced pressure, the residue was purified by preparative thin-layer silica gel column chromatography (n-hexane:ethyl acetate = 1:1) to obtain Compound II-54 (isolated yield 4.4 mg, percent yield 13%).
¹H-NMR (500 MHz, CDCl₃, δ): 7.83 (d, J = 3.4 Hz, 1H), 7.31 (d, J = 3.4 Hz, 1H), 6.98 (s, 1H), 5.95-5.92 (m, 1H), 5.79-5.74 (m, 2H), 5.44-5.38 (m, 2H), 5.00-4.96 (m, 1H), 4.78 (d, J = 2.9 Hz, 1H), 4.73 (dd, J = 13.2, 1.7 Hz, 1H), 4.66 (s, 1H), 4.60 (s, 1H), 4.51 (dd, J = 13.5, 2.0 Hz, 1H), 4.08-4.00 (m, 1H), 3.95 (br s, 1H), 3.87-3.80 (m, 1H), 3.80-3.76 (m, 1H), 3.72-3.61 (m, 2H), 3.54-3.48 (m, 1H), 3.46 (s, 3H), 3.44 (s, 3H), 3.27-3.20 (m, 2H), 3.18 (t, J = 9.2 Hz, 1H), 2.57-2.47 (m, 2H), 2.42 (dd, J = 13.2, 3.4 Hz, 1H), 2.38-2.31 (m, 2H), 2.31-2.21 (m, 2H), 2.14-2.06 (m, 1H), 2.00-1.91 (m, 1H), 1.88-1.82 (m, 1H), 1.82-1.76 (m, 1H), 1.68-1.62 (m, 2H), 1.60-1.35 (m, 9H), 1.34-1.31 (m, 3H), 1.31-1.24 (m, 8H), 1.18 (d, J = 6.9 Hz, 3H), 0.96-0.91 (m, 4H), 0.85 (d, J = 6.9 Hz, 3H), 0.81-0.71 (m, 4H); MSm/z 956.5192 [M+H]⁺.

### Production of Compound II-55

A mixture of Compound II-52 and Compound II-53 (30 mg, 0.032 mmol) obtained in [Production of Compound II-52 and Compound II-53] was dissolved in 1,4-dioxane (640 µL) and water (64 µL), and pyridin-3-ylboronic acid (7.76 mg, 0.063 mmol), potassium carbonate (6.55 mg, 0.047 mmol) and tetrakis(triphenylphosphine)palladium (3.65 mg, 3.16 µmol) were added, and the mixture was stirred at 120°C for 1.5 hours using a microwave reactor. A saturated aqueous sodium bicarbonate solution was added to the reaction solution to stop the reaction. After extraction with chloroform, the organic layer was washed with saturated brine and dried over anhydrous sodium sulfate. After the solvent was distilled off under reduced pressure, the residue was purified by preparative thin-layer silica gel column chromatography (chloroform:methanol = 9:1) to obtain Compound II-55 (isolated yield 4.0 mg, percent yield 13%).
¹H-NMR (500 MHz, CDCl₃, δ): 8.60-8.57 (m, 1H), 8.48-8.45 (m, 1H), 7.64 (m, J = 7.5 Hz, 1H), 7.23 (dd, J = 8.0, 5.2 Hz, 1H), 6.69-6.67 (m, 1H), 5.83-5.77 (m, 1H), 5.74-5.66 (m, 2H), 5.45-5.38 (m, 2H), 4.95 (br d, J = 10.8 Hz, 1H), 4.77 (br d, J = 3.4 Hz, 1H), 4.71-4.61 (m, 2H), 4.60 (s, 1H), 4.27 (s, 1H), 3.94 (br s, 1H), 3.86-3.74 (m, 2H), 3.73-3.59 (m, 2H), 3.51-3.46 (m, 1H), 3.44 (s, 3H), 3.42 (s, 3H), 3.27-3.20 (m, 2H), 3.17 (t, J = 8.9 Hz, 1H), 2.88 (dd, J = 8.9, 6.6 Hz, 1H), 2.69-2.61 (m, 1H), 2.54-2.45 (m, 2H), 2.37-2.18 (m, 4H), 1.95-1.87 (m, 1H), 1.87-1.80 (m, 2H), 1.79-1.72 (m, 1H), 1.71-1.65 (m, 1H), 1.63-1.36 (m,13H), 1.28 (d, J = 6.3 Hz, 6H), 1.26 (d, J = 6.3 Hz, 3H), 1.14 (d, J = 6.9 Hz, 3H), 0.93 (t, J = 7.4 Hz, 3H), 0.85 (d, J = 6.9 Hz, 3H), 0.79 (d, J = 5.7 Hz, 3H); MSm/z 950.5649 [M+H]⁺.

### Production of Compound II-56 and Compound II-57

Compound II-56 (isolated yield 6.9 mg, percent yield 23%) and Compound II-57 (isolated yield 4.4 mg, percent yield 15%) were obtained in a similar manner to [Production of Compound II-54], except that 2-(tributylstannyl)oxazole was used in place of 2-(tributylstannyl)thiazole in [Production of Compound II-54].

### (Compound II-56)

¹H-NMR (500 MHz, CDCl₃, δ): 7.61-7.54 (m, 1H), 7.17-7.15 (m, 1H), 6.62-6.60 (m, 1H), 5.94-5.90 (m, 1H), 5.78-5.74 (m, 2H), 5.40 (d, J = 4.0 Hz, 2H), 4.97 (d, J = 10.9 Hz, 1H), 4.77 (d, J = 3.4 Hz, 1H), 4.73-4.68 (m, 1H), 4.64 (br s, 1H), 4.60 (s, 1H), 4.48-4.42 (m, 1H), 4.18-4.09 (m, 1H), 3.95-3.92 (m, 1H), 3.93 (br s, 1H), 3.85-3.74 (m, 2H), 3.71-3.61 (m, 2H), 3.52-3.47 (m, 1H), 3.45 (s, 3H), 3.42 (s, 3H), 3.26-3.13 (m, 3H), 2.55-2.46 (m, 2H), 2.36 (d, J = 3.4 Hz, 3H), 2.28-2.19 (m, 2H), 2.12-2.06 (m, 1H), 1.97-1.87 (m, 1H), 1.87-1.81 (m, 1H), 1.80-1.75 (m, 1H), 1.66-1.61 (m, 1H), 1.54-1.37 (m, 9H), 1.32-1.23 (m,12H), 1.16 (d, J = 6.9 Hz, 3H), 0.92 (t, J = 7.4 Hz, 3H), 0.83 (d, J = 6.9 Hz, 3H), 0.79-0.73 (m, 4H); MSm/z 940.543 [M+H]⁺.

### (Compound II-57)

¹H-NMR (500 MHz, CDCl₃, δ): 7.57 (s, 1H), 7.14 (s, 1H), 6.47 (d, J = 2.3 Hz, 1H), 5.89-5.83 (m, 1H), 5.78-5.70 (m, 2H), 5.53 (s, 1H), 5.44-5.37 (m, 2H), 4.99-4.95 (m, 1H), 4.79-4.70 (m, 3H), 4.60 (s, 1H), 3.95 (br s, 1H), 3.89-3.75 (m, 2H), 3.73-3.61 (m, 2H), 3.53-3.46 (m, 1H), 3.45 (s, 3H), 3.44 (s, 3H), 3.28-3.21 (m, 2H), 3.18 (t, J = 9.2 Hz, 1H), 2.95-2.89 (m, 1H), 2.76-2.69 (m, 1H), 2.55-2.46 (m, 2H), 2.39-2.20 (m, 4H), 2.18 (s, 1H), 1.95-1.89 (m, 1H), 1.87-1.73 (m, 3H), 1.70-1.37 (m,10H), 1.32-1.22 (m,11H), 1.17 (d, J = 6.9 Hz, 3H), 0.93 (t, J = 7.4 Hz, 3H), 0.91-0.77 (m, 8H); MSm/z 940.5418 [M+H]⁺.

### Production of Compound II-58 and Compound II-59

Compound II-58 (isolated yield 6.3 mg, percent yield 21%) and Compound II-59 (isolated yield 4.1 mg, percent yield 14%) were obtained in a similar manner to [Production of Compound II-55], except that 3-(4,4,5,5-tetramethyl-1,3,2-dioxaborolan-2-yl)-1H-pyrazole was used in place of pyridin-3-ylboronic acid in [Production of Compound II-55].

### (Compound II-58)

¹H-NMR (500 MHz, CDCl₃, δ): 7.56 (d, J = 1.7 Hz, 1H), 6.63 (d, J = 1.7 Hz, 1H), 6.31 (d, J = 1.7 Hz, 1H), 5.95-5.90 (m, 1H), 5.81-5.73 (m, 2H), 5.45-5.37 (m, 2H), 4.98 (br d, J = 11.4 Hz, 1H), 4.78 (d, J = 3.4 Hz, 1H), 4.70 (dd, J = 13.5, 2.0 Hz, 1H), 4.62-4.48 (m, 3H), 3.95 (br s, 1H), 3.88-3.81 (m, 1H), 3.81-3.75 (m, 1H), 3.73-3.61 (m, 2H), 3.54-3.48 (m, 1H), 3.47 (s, 3H), 3.44 (s, 3H), 3.44-3.38 (m, 1H), 3.28-3.20 (m, 2H), 3.18 (t, J = 9.2 Hz, 1H), 2.56-2.48 (m, 1H), 2.46 (dd, J = 13.2, 4.0 Hz, 1H), 2.39-2.31 (m, 2H), 2.30-2.21 (m, 2H), 2.05-1.99 (m, 1H), 1.95-1.83 (m, 2H), 1.82-1.76 (m, 1H), 1.67-1.63 (m, 1H), 1.60-1.35 (m,12H), 1.29 (d, J = 6.3 Hz, 3H), 1.28-1.24 (m, 8H), 1.18 (d, J = 7.4 Hz, 3H), 0.93 (t, J = 7.4 Hz, 3H), 0.85 (d, J = 6.9 Hz, 3H), 0.81-0.71 (m, 4H); MSm/z 961.5399 [M+Na]⁺.

### (Compound II-59)

¹H-NMR (500 MHz, CDCl₃, δ): 7.52 (d, J = 2.3 Hz, 1H), 6.58 (d, J = 1.7 Hz, 1H), 6.30 (d, J = 1.7 Hz, 1H), 5.88-5.83 (m, 1H), 5.77-5.68 (m, 2H), 5.45-5.38 (m, 2H), 4.96 (br d, J = 10.9 Hz, 1H), 4.78 (d, J = 3.4 Hz, 1H), 4.71 (d, J = 2.3 Hz, 2H), 4.55 (s, 1H), 3.94 (br s, 1H), 3.84 (dd, J = 9.7, 6.3 Hz, 1H), 3.78 (dd, J = 9.5, 6.0 Hz, 1H), 3.72-3.61 (m, 2H), 3.52-3.46 (m, 1H), 3.45 (s, 3H), 3.43 (s, 3H), 3.29-3.20 (m, 2H), 3.17 (t, J = 9.2 Hz, 1H), 2.87-2.82 (m, 1H), 2.67-2.59 (m, 1H), 2.54-2.47 (m, 1H), 2.38-2.19 (m, 4H), 1.94-1.88 (m, 1H), 1.88-1.74 (m, 3H), 1.71-1.64 (m, 1H), 1.59-1.39 (m,13H), 1.30-1.24 (m, 9H), 1.16 (d, J = 6.9 Hz, 3H), 0.93 (t, J = 7.4 Hz, 3H), 0.85 (d, J = 6.3 Hz, 3H), 0.83-0.77 (m, 4H); MSm/z 939.5592 [M+H]⁺.

### Production of Compound II-60

Compound II-60 (isolated yield 1.5 mg, percent yield 5%) was obtained in a similar manner to [Production of Compound II-55], except that 4-(4,4,5,5-tetramethyl-1,3,2-dioxaborolan-2-yl)-1H-pyrazole was used in place of pyridin-3-ylboronic acid in [Production of Compound II-55].
¹H-NMR (500 MHz, CD₃OD, δ): 7.60 (br s, 2H), 6.54 (d, J = 2.3 Hz, 1H), 5.95-5.85 (m, 1H), 5.81 (dt, J = 11.3, 2.3 Hz, 1H), 5.69 (dd, J = 14.9, 10.3 Hz, 1H), 5.34 (br d, J = 3.4 Hz, 1H), 5.23-5.04 (m, 2H), 4.80 (br d, J = 3.4 Hz, 1H), 4.72-4.63 (m, 2H), 4.43 (s, 1H), 3.99 (br s, 1H), 3.91-3.84 (m, 1H), 3.76-3.61 (m, 3H), 3.42 (s, 3H), 3.40 (s, 3H), 3.45-3.38 (m, 1H), 3.28-3.25 (m, 1H), 3.19 (t, J = 9.2 Hz, 1H), 3.02 (t, J = 9.2 Hz, 1H), 2.81 (dd, J = 12.6, 2.9 Hz, 1H), 2.68-2.60 (m, 1H), 2.56-2.48 (m, 1H), 2.34-2.26 (m, 4H), 2.05-1.99 (m, 1H), 1.90-1.84 (m, 1H), 1.80 (q, J = 12.6 Hz, 1H), 1.73-1.67 (m, 1H), 1.66-1.37 (m,13H), 1.32-1.27 (m, 1H), 1.23 (dd, J = 13.2, 6.3 Hz, 9H), 1.16 (d, J = 6.9 Hz, 3H), 0.96 (t, J = 7.4 Hz, 3H), 0.88 (d, J = 6.9 Hz, 3H), 0.86-0.77 (m, 4H); MSm/z 939.5585 [M+H]⁺.

### Production of Compound II-61

Compound II-61 (isolated yield 4.8 mg, percent yield 16%) was obtained in a similar manner to [Production of Compound II-55], except that pyridin-4-ylboronic acid was used in place of pyridin-3-ylboronic acid in [Production of Compound II-55].
¹H-NMR (500 MHz, CDCl₃, δ): 8.53 (d, J = 4.6 Hz, 2H), 7.25 (d, J = 4.6 Hz, 2H), 6.64 (d, J = 1.7 Hz, 1H), 5.85-5.77 (m, 1H), 5.75-5.66 (m, 2H), 5.46-5.36 (m, 2H), 4.95 (d, J = 11.2 Hz, 1H), 4.77 (d, J = 3.4 Hz, 1H), 4.73-4.62 (m, 3H), 4.30 (s, 1H), 3.94 (br s, 1H), 3.86-3.73 (m, 2H), 3.72-3.60 (m, 2H), 3.51-3.45 (m, 1H), 3.44 (s, 3H), 3.42 (s, 3H), 3.28-3.20 (m, 2H), 3.17 (t, J = 9.2 Hz, 1H), 2.91-2.84 (m, 1H), 2.69-2.61 (m, 1H), 2.54-2.46 (m, 2H), 2.37-2.19 (m, 4H), 1.95-1.89 (m, 1H), 1.86-1.72 (m, 4H), 1.71-1.36 (m, 8H), 1.30-1.23 (m,12H), 1.14 (d, J = 6.9 Hz, 3H), 0.96-0.91 (m, 3H), 0.91-0.76 (m, 9H); MSm/z 950.5635 [M+H]⁺.

### Production of Compound II-62

Compound II-62 (isolated yield 6.5 mg, percent yield 21%) was obtained in a similar manner to [Production of Compound II-54], except that 5-(tributylstannyl)thiazole was used in place of 2-(tributylstannyl)thiazole in [Production of Compound II-54].
¹H-NMR (500 MHz, CDCl₃, δ): 8.73 (s, 1H), 7.85 (s, 1H), 6.72 (d, J = 1.7 Hz, 1H), 5.87-5.82 (m, 1H), 5.77-5.70 (m, 2H), 5.46-5.38 (m, 2H), 4.96 (br d, J = 11.5 Hz, 1H), 4.78 (d, J = 4.0 Hz, 1H), 4.74-4.66 (m, 2H), 4.62 (s, 1H), 4.53 (s, 1H), 3.95 (br s, 1H), 3.87-3.75 (m, 2H), 3.72-3.61 (m, 2H), 3.52-3.46 (m, 1H), 3.45 (s, 3H), 3.43 (s, 3H), 3.29-3.21 (m, 2H), 3.17 (t, J = 8.6 Hz, 1H), 2.90-2.84 (m, 1H), 2.70-2.61 (m, 1H), 2.55-2.47 (m, 2H), 2.39-2.20 (m, 4H), 1.95-1.88 (m, 1H), 1.87-1.80 (m, 2H), 1.80-1.74 (m, 1H), 1.71-1.65 (m, 1H), 1.64-1.37 (m,10H), 1.31-1.24 (m,11H), 1.16 (d, J = 6.9 Hz, 3H), 0.93 (t, J = 7.4 Hz, 3H), 0.87-0.82 (m, 4H), 0.80 (d, J = 6.3 Hz, 3H); MSm/z 956.52 [M+H]⁺.

### Production of Compound II-63

Compound II-63 (isolated yield 5.5 mg, percent yield 18%) was obtained in a similar manner to [Production of Compound II-54], except that 1-methyl-5-(tributylstannyl)-1H-imidazole was used in place of 2-(tributylstannyl)thiazole in [Production of Compound II-54] .
¹H-NMR (500 MHz, CDCl₃, δ): 7.43 (s, 1H), 7.14 (s, 1H), 6.34 (d, J = 2.3 Hz, 1H), 5.84-5.80 (m, 1H), 5.74-5.67 (m, 2H), 5.45-5.38 (m, 2H), 5.31 (s, 1H), 4.96 (br d, J = 10.9 Hz, 1H), 4.78 (d, J = 3.4 Hz, 1H), 4.68 (d, J = 2.3 Hz, 2H), 4.56-4.52 (m, 1H), 4.50 (s, 1H), 3.94 (br s, 1H), 3.87-3.81 (m, 1H), 3.77 (s, 1H), 3.72-3.61 (m, 3H), 3.58 (s, 3H), 3.51-3.46 (m, 1H), 3.45 (s, 3H), 3.43 (s, 3H), 3.27-3.20 (m, 2H), 3.17 (t, J = 9.2 Hz, 1H), 2.88-2.84 (m, 1H), 2.68-2.61 (m, 1H), 2.55-2.47 (m, 1H), 2.37-2.20 (m, 4H), 1.94-1.89 (m, 1H), 1.87-1.62 (m, 4H), 1.59-1.37 (m, 8H), 1.33-1.21 (m,11H), 1.19-1.14 (m, 4H), 0.93 (t, J = 7.4 Hz, 3H), 0.90-0.82 (m, 4H), 0.80 (d, J = 6.3 Hz, 4H); MSm/z 953.5753 [M+H]⁺.

### Production of Compound II-64

Compound II-14 (605 mg, 0.660 mmol) obtained in [Production of Compound II-14] was dissolved in DMF (6.60 mL) and then cooled in ice to 0°C. Then HATU (752 mg, 1.98 mmol), DIPEA (575 µL, 3.30 mmol) and hydrazine monohydrate (160 µL, 3.30 mmol) were sequentially added, and the mixture was stirred at room temperature for 30 minutes. Water was added to the reaction solution, and extracted with diethyl ether. The organic layer was washed with water and saturated brine sequentially, and dried over anhydrous sodium sulfate. After the solvent was distilled off under reduced pressure, the residue was purified by silica gel column chromatography (chloroform/methanol system) to obtain Compound II-64 (isolated yield 517 mg, percent yield 84%).
¹H-NMR (500 MHz, CDCl₃, δ): 7.33 (s, 1H), 5.93 (d, J = 1.7 Hz, 1H), 5.85-5.82 (m, 1H), 5.76-5.66 (m, 2H), 5.41 (d, J = 3.7 Hz, 1H), 5.40-5.35 (m, 1H), 4.96-4.95 (m, 1H), 4.77 (d, J = 2.9 Hz, 1H), 4.72 (s, 1H), 4.66 (br s, 2H), 4.63 (s, 1H), 3.94 (br s, 3H), 3.86-3.74 (m, 2H), 3.69-3.61 (m, 2H), 3.51-3.46 (m, 1H), 3.44 (s, 3H), 3.43 (s, 3H), 3.26-3.15 (m, 3H), 2.81 (dd, J = 12.6, 3.4 Hz, 1H), 2.62-2.57 (m, 1H), 2.53 (d, J = 1.7 Hz, 1H), 2.53-2.47 (m, 1H), 2.36-2.20 (m, 4H), 1.92-1.88 (m, 1H), 1.82-1.72 (m, 3H), 1.69-1.36 (m,13H), 1.27 (dd, J = 11.5, 6.3 Hz, 7H), 1.15 (dd, J = 11.5, 6.9 Hz, 6H), 0.92 (t, J = 7.4 Hz, 3H), 0.84 (d, J = 6.3 Hz, 3H), 0.80-0.75 (m, 4H); MSm/z 931.5549 [M+H]⁺.

### Production of Compound II-65

Compound II-64 (430 mg, 0.462 mmol) obtained in [Production of Compound II-64] was dissolved in toluene (4.62 mL), and triethoxymethane (131 µL, 9.24 mmol) was added, followed by stirring at 120°C overnight. After distilling off the reaction solution under reduced pressure, the residue was roughly purified by silica gel column chromatography (n-hexane/ethyl acetate system) and repurified by high-performance liquid chromatography to obtain Compound II-65 (isolated yield 240 mg, percent yield 55%).
¹H-NMR (500 MHz, CDCl₃, δ): 8.35 (s, 1H), 6.53 (d, J = 2.3 Hz, 1H), 5.91-5.81 (m, 1H), 5.78-5.70 (m, 2H), 5.45-5.36 (m, 2H), 5.29 (s, 1H), 5.01-4.94 (m, 1H), 4.78 (d, J = 4.0 Hz, 1H), 4.77-4.71 (m, 2H), 4.57 (s, 1H), 3.95 (br s, 1H), 3.88-3.81 (m, 1H), 3.81-3.74 (m, 1H), 3.72-3.62 (m, 2H), 3.53-3.46 (m, 1H), 3.45 (s, 3H), 3.43 (s, 3H), 3.31-3.20 (m, 2H), 3.18 (t, J = 9.2 Hz, 1H), 2.97-2.89 (m, 1H), 2.82-2.72 (m, 1H), 2.58-2.47 (m, 2H), 2.38-2.32 (m, 2H), 2.32-2.20 (m, 2H), 1.95-1.90 (m, 1H), 1.90-1.82 (m, 2H), 1.79-1.73 (m, 1H), 1.68 (br d, J = 12.6 Hz, 1H), 1.65-1.37 (m,12H), 1.30-1.25 (m,10H), 1.17 (d, J = 6.9 Hz, 3H), 0.93 (t, J = 7.2 Hz, 3H), 0.85 (d, J = 6.9 Hz, 3H), 0.81-0.76 (m, 4H); MSm/z 958.5627 [M+NH₄]⁺.

### Production of Compound II-66

Compound II-66 (isolated yield 19.2 mg, percent yield 19%) was obtained in a similar manner to [Production of Compound II-55], except that cyclopent-1-en-1-ylboronic acid was used in place of pyridin-3-ylboronic acid in [Production of Compound II-55].
¹H-NMR (500 MHz, CDCl₃, δ): 6.20 (br s, 1H), 5.88-5.80 (m, 1H), 5.77 (br s, 1H), 5.73-5.68 (m, 2H), 5.44-5.36 (m, 2H), 4.96 (br d, J = 11.2 Hz, 1H), 4.77 (d, J = 3.4 Hz, 1H), 4.68-4.58 (m, 4H), 3.94 (br s, 1H), 3.84 (dq, J = 9.4, 6.2 Hz, 1H), 3.81-3.75 (m, 1H), 3.72-3.61 (m, 2H), 3.53-3.47 (m, 1H), 3.45 (s, 3H), 3.44 (s, 3H), 3.28-3.20 (m, 2H), 3.18 (t, J = 9.2 Hz, 1H), 2.86-2.79 (m, 1H), 2.56-2.41 (m, 5H), 2.39-2.20 (m, 6H), 1.95-1.82 (m, 3H), 1.81-1.71 (m, 3H), 1.70-1.65 (m, 1H), 1.64-1.37 (m,12H), 1.29 (d, J = 6.3 Hz, 3H), 1.27-1.22 (m, 4H), 1.19-1.13 (m, 6H), 0.93 (t, J = 7.4 Hz, 3H), 0.84 (d, J = 6.9 Hz, 3H), 0.82-0.76 (m, 4H); MSm/z 956.6096 [M+NH₄]⁺.

### Production of Compound II-67

Compound II-67 (isolated yield 13.2 mg, percent yield 36%) was obtained in a similar manner to [Production of Compound 11-65], except that 1,1,1-triethoxyethane was used in place of triethoxymethane in [Production of Compound II-65].
¹H-NMR (500 MHz, CDCl₃, δ): 6.46 (d, J = 2.3 Hz, 1H), 5.88-5.81 (m, 1H), 5.79-5.69 (m, 2H), 5.43-5.36 (m, 2H), 5.22 (s, 1H), 4.96 (br d, J = 11.0 Hz, 1H), 4.78 (d, J = 3.4 Hz, 1H), 4.75-4.70 (m, 2H), 4.59 (s, 1H), 3.95 (br s, 1H), 3.87-3.81 (m, 1H), 3.81-3.74 (m, 1H), 372-3.61 (m, 2H), 3.52-3.46 (m, 1H), 3.45 (s, 3H), 3.43 (s, 3H), 3.28-3.20 (m, 2H), 3.17 (t, J = 9.2 Hz, 1H), 2.93-2.89 (m, 1H), 2.77-2.71 (m, 1H), 2.53 (s, 3H), 2.53-2.49 (m, 1H), 2.38-2.20 (m, 4H), 1.95-1.90 (m, 1H), 1.88-1.81 (m, 2H), 1.80-1.74 (m, 1H), 1.71-1.62 (m, 3H), 1.60-1.37 (m,11H), 1.28 (d, J = 6.3 Hz, 3H), 1.27-1.23 (m, 7H), 1.17 (d, J = 6.9 Hz, 3H), 0.93 (t, J = 7.4 Hz, 3H), 0.84 (d, J = 6.9 Hz, 3H), 0.83-0.77 (m, 4H); MSm/z 977.5351 [M+Na]⁺.

### Production of Compound II-68

Compound II-68 (isolated yield 10.6 mg, percent yield 11%) was obtained in a similar manner to [Production of Compound II-55], except that 5-(4,4,5,5-tetramethyl-1,3,2-dioxaborolan-2-yl)pyridin-2-amine was used in place of pyridin-3-ylboronic acid in [Production of Compound II-55].
¹H-NMR (500 MHz, CDCl₃, δ): 8.03 (s, 1H), 7.41 (dd, J = 8.6, 2.3 Hz, 1H), 6.55 (d, J = 1.7 Hz, 1H), 6.43 (d, J = 8.6 Hz, 1H), 5.82-5.76 (m, 1H), 5.70-5.66 (m, 2H), 5.43-5.35 (m, 2H), 4.96-4.90 (m, 1H), 4.78-4.74 (m, 1H), 4.69-4.60 (m, 2H), 4.58-4.48 (m, 3H), 3.92 (br s, 1H), 3.85-3.72 (m, 2H), 3.70-3.59 (m, 2H), 3.50-3.44 (m, 2H), 3.42 (s, 3H), 3.41 (s, 3H), 3.26-3.19 (m, 2H), 3.15 (t, J = 9.2 Hz, 1H), 2.87-2.82 (m, 1H), 2.62-2.55 (m, 1H), 2.50-2.45 (m, 1H), 2.35-2.18 (m, 5H), 1.99-1.71 (m, 3H), 1.68-1.64 (m, 1H), 1.62-1.33 (m,11H), 1.29-1.20 (m, 9H), 1.14-1.09 (m, 3H), 1.14-1.09 (m, 3H), 0.91 (t, J = 7.4 Hz, 3H), 0.83 (d, J = 6.3 Hz, 3H), 0.81-0.75 (m, 4H); MSm/z 965.5734 [M+H]⁺.

### Production of Compound II-69

Compound II-69 (isolated yield 16.0 mg, percent yield 16%) was obtained in a similar manner to [Production of Compound II-55], except that 3-methyl-4-(4,4,5,5-tetramethyl-1,3,2-dioxaborolan-2-yl)-1H-pyrazole was used in place of pyridin-3-ylboronic acid in [Production of Compound II-55].
¹H-NMR (500 MHz, CDCl₃, δ): 7.57 (s, 1H), 6.40 (d, J = 1.7 Hz, 1H), 5.86-5.82 (m, 1H), 5.75-5.68 (m, 2H), 5.44-5.38 (m, 2H), 4.96 (br d, J = 11.0 Hz, 1H), 4.77 (d, J = 3.4 Hz, 1H), 4.72-4.61 (m, 2H), 4.45 (s, 1H), 3.94 (br s, 1H), 3.87-3.73 (m, 2H), 3.72-3.60 (m, 2H), 3.53-3.46 (m, 1H), 3.45 (s, 3H), 3.43 (s, 3H), 3.27-3.20 (m, 2H), 3.17 (t, J = 9.2 Hz, 1H), 2.88-2.82 (m, 1H), 2.63-2.55 (m, 1H), 2.54-2.46 (m, 1H), 2.38-2.28 (m, 3H), 2.27 (s, 3H), 2.25-2.19 (m, 1H), 1.94-1.89 (m, 1H), 1.84-1.74 (m, 3H), 1.71-1.63 (m, 1H), 1.60-1.35 (m,13H), 1.32-1.21 (m,12H), 1.15 (d, J = 6.9 Hz, 3H), 0.93 (t, J = 7.4 Hz, 3H), 0.85 (d, J = 6.3 Hz, 3H), 0.83-0.75 (m, 4H); MSm/z 953.5731 [M+H]⁺.

### Production of Compound II-70

Compound II-70 (isolated yield 11.7 mg, percent yield 11%) was obtained in a similar manner to [Production of Compound II-55], except that 4-(4,4,5,5-tetramethyl-1,3,2-dioxaborolan-2-yl)-3-(trifluoromethyl) -1H-pyrazole was used in place of pyridin-3-ylboronic acid in [Production of Compound II-55].
¹H-NMR (500 MHz, CDCl₃, δ): 7.72 (s, 1H), 6.53 (s, 1H), 5.85 (dt, J = 10.3, 2.3 Hz, 1H), 5.78-5.67 (m, 2H), 5.45-5.37 (m, 2H), 4.96 (br d, J = 11.5 Hz, 1H), 4.77 (d, J = 3.4 Hz, 1H), 4.73-4.60 (m, 3H), 4.36 (s, 1H), 3.94 (br s, 1H), 3.87-3.81 (m, 1H), 3.81-3.74 (m, 1H), 3.73-3.61 (m, 2H), 3.53-3.46 (m, 1H), 3.44 (s, 3H), 3.43 (s, 3H), 3.27-3.21 (m, 2H), 3.18 (t, J = 9.2 Hz, 1H), 2.86-2.82 (m, 1H), 2.65-2.57 (m, 1H), 2.54-2.47 (m, 1H), 2.38-2.20 (m, 4H), 1.94-1.90 (m, 1H), 1.84-1.73 (m, 3H), 1.70-1.62 (m, 3H), 1.60-1.37 (m,11H), 1.28 (d, J = 6.3 Hz, 3H), 1.27-1.23 (m, 7H), 1.15 (d, J = 6.9 Hz, 3H), 0.93 (t, J = 7.4 Hz, 3H), 0.85 (d, J = 6.9 Hz, 3H), 0.83-0.77 (m, 4H); MSm/z 1024.571 [M+NH₄]⁺.

### Production of Compound II-71

Compound II-71 (isolated yield 17.2 mg, percent yield 16%) was obtained in a similar manner to [Production of Compound II-55], except that 1-methyl-3-(4,4,5,5-tetramethyl-1,3,2-dioxaborolan-2-yl)-1H-pyrazole was used in place of pyridin-3-ylboronic acid in [Production of Compound II-55].
¹H-NMR (500 MHz, CDCl₃, δ): 7.25 (d, J = 2.3 Hz, 1H), 6.62 (d, J = 1.7 Hz, 1H), 6.30 (d, J = 1.7 Hz, 1H), 5.87-5.83 (m, 1H), 5.77-5.68 (m, 2H), 5.43-5.36 (m, 2H), 4.96 (br d, J = 10.9 Hz, 1H), 4.85 (s, 1H), 4.77 (d, J = 3.4 Hz, 1H), 4.73-4.65 (m, 2H), 4.56 (br s, 1H), 3.94 (br s, 1H), 3.86 (s, 3H), 3.86-3.81 (m, 1H), 3.81-3.74 (m, 1H), 3.71-3.60 (m, 2H), 3.52-3.45 (m, 1H), 3.45 (s, 3H), 3.43 (s, 3H), 3.27-3.20 (m, 2H), 3.17 (t, J = 8.9 Hz, 1H), 2.86 (dd, J = 12.0, 4.0 Hz, 1H), 2.65-2.57 (m, 1H), 2.54-2.47 (m, 1H), 2.38-2.20 (m, 4H), 1.95-1.90 (m, 1H), 1.85-1.74 (m, 3H), 1.67 (d, J = 13.2 Hz, 1H), 1.60-1.36 (m,13H), 1.28 (d, J = 6.3 Hz, 3H), 1.27-1.22 (m, 7H), 1.15 (d, J = 6.9 Hz, 3H), 0.92 (t, J = 7.2 Hz, 3H), 0.84 (d, J = 6.9 Hz, 3H), 0.83-0.77 (m, 4H); MSm/z 953.5738 [M+H]⁺.

### Production of Compound II-72

Compound II-72 (isolated yield 15.4 mg, percent yield 13%) was obtained in a similar manner to [Production of Compound II-55], except that 1-methyl-5-(4,4,5,5-tetramethyl-1,3,2-dioxaborolan-2-yl)-1H-pyrazole was used in place of pyridin-3-ylboronic acid in [Production of Compound II-55].
¹H-NMR (500 MHz, CDCl₃, δ): 7.38 (d, J = 1.7 Hz, 1H), 6.41 (d, J = 2.3 Hz, 1H), 6.31 (d, J = 1.7 Hz, 1H), 5.84-5.80 (m, 1H), 5.75-5.67 (m, 2H), 5.44-5.37 (m, 2H), 4.95 (br d, J = 10.9 Hz, 1H), 4.77 (d, J = 3.4 Hz, 1H), 4.71-4.61 (m, 2H), 4.56 (s, 1H), 4.47 (s, 1H), 3.94 (br s, 1H), 3.86-3.82 (m, 1H), 3.81 (s, 3H), 3.81-3.74 (m, 1H), 3.72-3.61 (m, 2H), 3.51-3.45 (m, 1H), 3.44 (s, 3H), 3.43 (s, 3H), 3.27-3.20 (m, 2H), 3.17 (t, J = 9.2 Hz, 1H), 2.89-2.83 (m, 1H), 2.68-2.61 (m, 1H), 2.54 (br s, 1H), 2.52-2.46 (m, 1H), 2.37-2.20 (m, 4H), 1.94-1.89 (m, 1H), 1.85-1.79 (m, 2H), 1.79-1.74 (m, 1H), 1.70-1.62 (m, 4H), 1.60-1.37 (m,10H), 1.31-1.23 (m, 9H), 1.15 (d, J = 7.4 Hz, 3H), 0.93 (t, J = 7.4 Hz, 3H), 0.85 (d, J = 6.9 Hz, 3H), 0.83-0.77 (m, 4H); MSm/z 953.5743 [M+Na]⁺.

### Production of Compound II-73

Compound II-64 (41 mg, 0.044 mmol) obtained in [Production of Compound II-64] was dissolved in THF (440 µL), di(1H-imidazol-1-yl)methanone (7.85 mg, 0.048 mmol) was added, and the mixture was stirred under heating and refluxing overnight. Water was added to the reaction solution, and the mixture was extracted with ethyl acetate. The organic layer was washed with saturated brine and dried over anhydrous sodium sulfate. After the solvent was distilled off under reduced pressure, the residue was purified by silica gel column chromatography (n-hexane/ethyl acetate system) to obtain Compound II-73 (isolated yield 27 mg, percent yield 64%).
¹H-NMR (500 MHz, CDCl₃, δ): 9.28 (s, 1H), 7.25 (s, 1H), 6.07 (d, J = 2.3 Hz, 1H), 5.86-5.81 (m, 1H), 5.77-5.67 (m, 2H), 5.42-5.35 (m, 2H), 5.05 (s, 1H), 4.97-4.92 (m, 1H), 4.76 (d, J = 3.4 Hz, 1H), 4.69 (d, J = 5.2 Hz, 3H), 3.93 (br s, 1H), 3.86-3.73 (m, 2H), 3.70-3.60 (m, 2H), 3.52-3.46 (m, 1H), 3.47-3.45 (m, 1H), 3.44 (s, 3H), 3.42 (s, 3H), 3.26-3.19 (m, 2H), 3.16 (t, J = 9.2 Hz, 1H), 2.87 (dd, J = 11.7, 4.3 Hz, 1H), 2.72-2.65 (m, 1H), 2.63 (s, 1H), 2.53-2.46 (m, 1H), 2.36-2.16 (m, 4H), 1.90 (dd, J = 12.0, 4.0 Hz, 1H), 1.85-1.70 (m, 3H), 1.58-1.37 (m,11H), 1.29-1.24 (m, 7H), 1.19 (d, J = 6.3 Hz, 3H), 1.16 (d, J = 6.9 Hz, 3H), 0.92 (t, J = 7.2 Hz, 3H), 0.83 (d, J = 6.9 Hz, 3H), 0.81-0.75 (m, 4H); MSm/z 974.5579 [M+NH₄]⁺.

### Reference Example 24

### Production of 5-(4,4,5,5-tetramethyl-1,3,2-dioxaborolan-2-yl)-1H-pyrazolo[3,4-b]pyridine

5-Bromo-1H-pyrazolo[3,4-b]pyridine (100 mg, 0.505 mmol), 4,4,4',4',5,5,5',5'-octamethyl-2,2'-bis(1,3,2-dioxaborolane) (154 mg, 0.606 mmol), and potassium acetate (198 mg, 2.02 mmol) were dissolved in 1,4-dioxane (5.05 mL), and (1,1'-bis(diphenylphosphino)ferrocene)-dichloropalladium(II) (41.2 mg, 0.050 mmol) was added, followed by stirring at 100°C overnight. The reaction solution was filtered through Celite, the filtrate was distilled off under reduced pressure, and 5-(4,4,5,5-tetramethyl-1,3,2-dioxaborolan-2-yl)-1H-pyrazolo[3,4-b]pyridine was obtained as a crude product (isolated yield 356 mg, quantitative) .

### Production of Compound II-74

Compound II-74 (isolated yield 3.8 mg, percent yield 18%) was obtained in a similar manner to [Production of Compound II-55], except that 5-(4,4,5,5-tetramethyl-1,3,2-dioxaborolan-2-yl)-1H-pyrazolo[3,4-b]pyridine obtained in Reference Example 24 was used in place of pyridin-3-ylboronic acid in [Production of Compound II-55].
¹H-NMR (500 MHz, CDCl₃, δ): 8.54 (d, J = 1.7 Hz, 1H), 8.07 (d, J = 1.1 Hz, 1H), 8.06 (s, 1H), 6.87-6.79 (m, 1H), 5.83-5.78 (m, 1H), 5.74-5.65 (m, 2H), 5.45-5.38 (m, 2H), 4.95 (br d, J = 10.4 Hz, 1H), 4.77 (d, J = 3.4 Hz, 1H), 4.75-4.64 (m, 2H), 4.63 (s, 1H), 4.31 (s, 1H), 3.93 (br s, 1H), 3.85-3.79 (m, 1H), 3.79-3.74 (m, 1H), 3.72-3.59 (m, 2H), 3.51-3.45 (m, 1H), 3.44 (s, 3H), 3.42 (s, 3H), 3.26-3.20 (m, 2H), 3.17 (t, J = 9.2 Hz, 1H), 2.93-2.87 (m, 1H), 2.72-2.64 (m, 1H), 2.64-2.59 (m, 1H), 2.52-2.45 (m, 1H), 2.38-2.19 (m, 4H), 2.18 (s, 1H), 1.94-1.89 (m, 1H), 1.88-1.82 (m, 2H), 1.79-1.72 (m, 1H), 1.71-1.66 (m, 1H), 1.64-1.37 (m,10H), 1.32 (d, J = 6.3 Hz, 3H), 1.30-1.20 (m,10H), 1.13 (d, J = 6.9 Hz, 3H), 0.93 (t, J = 7.4 Hz, 3H), 0.90-0.78 (m, 7H); MSm/z 990.5693 [M+H]⁺.

### Production of Compound II-75

2,2,6,6-Tetramethylpiperidine (2.7 mL, 15.76 mmol) was dissolved in THF (54.0 mL) and cooled to - 30°C. Then, a 1.6M n-butyllithium hexane solution (10.1 mL, 15.80 mmol) was added dropwise, and the mixture was stirred at the same temperature for 30 minutes. Then, the mixture was cooled to -78°C, and bis(4,4,5,5-tetramethyl-1,3,2-dioxaborolan-2-yl)methane (4.25 g, 15.85 mmol) dissolved in THF (18.0 mL) was slowly added dropwise thereto, followed by stirring at the same temperature for 30 minutes. Furthermore, 3,4α-dihydro-5-oxo-ivermectin B1a (3.96 g, 4.53 mmol) dissolved in THF (18.0 mL) was slowly added dropwise, the temperature was raised to 0°C, and the mixture was stirred at the same temperature for 30 minutes. Then, the temperature was raised to room temperature, and the mixture was stirred at the same temperature for 1.5 hours. After cooling the reaction solution to 0°C, a saturated aqueous solution of ammonium chloride was added to the reaction solution, and the mixture was extracted with ethyl acetate. The organic layer was washed with a saturated aqueous sodium hydrogen carbonate solution and saturated brine, respectively, and dried over anhydrous sodium sulfate. After the solvent was distilled off under reduced pressure, the residue was purified by silica gel column chromatography (n-hexane/ethyl acetate system) and high-performance liquid chromatography to obtain Compound II-75 (isolated yield 1.47 g, percent yield 32%).
¹H-NMR (500 MHz, CDCl₃, δ): 5.87-5.78 (m, 1H), 5.75-5.68 (m, 2H), 5.59 (d, J = 1.7 Hz, 1H), 5.41-5.34 (m, 2H), 4.96 (br d, J = 9.7 Hz, 1H), 4.82 (s, 1H), 4.77 (d, J = 2.9 Hz, 1H), 4.68-4.59 (m, 2H), 4.53 (s, 1H), 3.93 (br s, 1H), 3.86-3.79 (m, 1H), 3.79-3.74 (m, 1H), 3.70-3.57 (m, 2H), 3.52-3.45 (m, 1H), 3.43 (s, 3H), 3.42 (s, 3H), 3.26-3.19 (m, 2H), 3.16 (t, J = 9.2 Hz, 1H), 2.84 (dd, J = 12.3, 3.7 Hz, 1H), 2.58-2.45 (m, 3H), 2.34 (dd, J = 12.0, 4.6 Hz, 2H), 2.30-2.20 (m, 2H), 1.93-1.84 (m, 1H), 1.78-1.68 (m, 3H), 1.67-1.45 (m,12H), 1.42-1.36 (m, 2H), 1.27 (d, J = 6.3 Hz, 3H), 1.26-1.23 (m,15H), 1.16 (d, J = 6.9 Hz, 3H), 1.10 (d, J = 6.3 Hz, 3H), 0.92 (t, J = 7.2 Hz, 3H), 0.83 (d, J = 6.9 Hz, 3H), 0.81-0.75 (m, 4H).

### Production of Compound II-76

Compound II-75 (200 mg, 0.201 mmol) obtained in [Production of Compound II-75] was dissolved in 1,4-dioxane (4.0 mL) and water (0.4 mL), and 3-iodo-1H-pyrazole-4-carbonitrile (88.0 mg, 0.402 mmol), potassium carbonate (41.7 mg, 0.302 mmol) and tetrakis(triphenylphosphine)palladium (23.2 mg, 0.020 mmol) were added, and the mixture was stirred at 120°C for 1.5 hours using a microwave reactor. A saturated aqueous sodium bicarbonate solution was added to the reaction solution to stop the reaction. After extraction with chloroform, the organic layer was washed with saturated brine and dried over anhydrous sodium sulfate. After the solvent was distilled off under reduced pressure, the residue was purified by high-performance liquid chromatography to obtain Compound II-76 (isolated yield 45.6 mg, percent yield 24%).
¹H-NMR (500 MHz, CDCl₃, δ): 7.83 (s, 1H), 6.57 (d, J = 2.3 Hz, 1H), 5.88 (dt, J = 10.7, 2.4 Hz, 1H), 5.80-5.67 (m, 2H), 5.48-5.38 (m, 2H), 4.96 (br d, J = 10.9 Hz, 1H), 4.78 (d, J = 3.4 Hz, 1H), 4.72 (s, 2H), 4.41 (s, 1H), 3.95 (br s, 1H), 3.88-3.81 (m, 1H), 3.81-3.73 (m, 1H), 3.72-3.58 (m, 2H), 3.52-3.46 (m, 1H), 3.45 (s, 3H), 3.42 (s, 3H), 3.31-3.20 (m, 2H), 3.17 (t, J = 9.2 Hz, 1H), 2.83 (dd, J = 11.5, 4.6 Hz, 1H), 2.76-2.64 (m, 1H), 2.51 (ddd, J = 9.5, 6.9, 2.6 Hz, 1H), 2.40-2.32 (m, 2H), 2.32-2.19 (m, 2H), 1.94-1.88 (m, 1H), 1.88-1.79 (m, 2H), 1.79-1.72 (m, 1H), 1.68 (d, J = 12.6 Hz, 1H), 1.64-1.34 (1m, 3H), 1.30 (d, J = 6.9 Hz, 3H), 1.28 (d, J = 6.3 Hz, 3H), 1.26 (d, J = 6.3 Hz, 3H), 1.19-1.12 (m, 3H), 0.93 (t, J = 7.4 Hz, 3H), 0.85 (d, J = 6.9 Hz, 3H), 0.83-0.75 (m, 4H); MSm/z 964.5543 [M+H]⁺.

### Production of Compound II-77

Compound II-77 (isolated yield 30.8 mg, percent yield 42%) was obtained in a similar manner to [Production of Compound II-76], except that 2-bromo-1H-imidazole was used in place of 3-iodo-1H-pyrazole-4-carbonitrile in [Production of Compound II-76].
¹H-NMR (500 MHz, CDCl₃, δ): 7.07 (s, 2H), 6.65 (d, J = 2.3 Hz, 1H), 5.90 (dt, J = 10.2, 2.4 Hz, 1H), 5.81-5.68 (m, 2H), 5.46-5.35 (m, 2H), 4.97 (br d, J = 10.5 Hz, 1H), 4.78 (d, J = 3.4 Hz, 1H), 4.73 (d, J = 2.3 Hz, 2H), 4.58 (s, 1H), 3.95 (br s, 1H), 3.87-3.81 (m, 1H), 3.78 (dq, J = 9.2, 6.3 Hz, 1H), 3.74-3.61 (m, 2H), 3.52-3.46 (m, 1H), 3.45 (s, 3H), 3.43 (s, 3H), 3.28-3.21 (m, 2H), 3.18 (t, J = 9.2 Hz, 1H), 2.84-2.79 (m, 1H), 2.70-2.62 (m, 1H), 2.55-2.48 (m, 1H), 2.38-2.20 (m, 4H), 1.96-1.91 (m, 1H), 1.87-1.81 (m, 2H), 1.79-1.74 (m, 1H), 1.69-1.65 (m, 1H), 1.61-1.36 (m,13H), 1.29 (d, J = 6.3 Hz, 3H), 1.27-1.24 (m, 6H), 1.16 (d, J = 7.4 Hz, 3H), 0.93 (t, J = 7.4 Hz, 3H), 0.85 (d, J = 6.9 Hz, 3H), 0.84-0.77 (m, 4H); MSm/z 939.5591 [M+H]⁺.

### Production of Compound II-78

Compound II-78 (isolated yield 5.8 mg, percent yield 8%) was obtained in a similar manner to [Production of Compound II-76], except that 4-bromo-1H-imidazole was used in place of 3-iodo-1H-pyrazole-4-carbonitrile in [Production of Compound II-76].
¹H-NMR (500 MHz, CDCl₃, δ): 7.76-7.62 (m, 1H), 7.17-7.02 (m, 1H), 6.55 (br s, 1H), 5.93-5.83 (m, 1H), 5.79-5.68 (m, 2H), 5.46-5.36 (m, 2H), 4.97 (br d, J = 11.5 Hz, 1H), 4.78 (d, J = 3.4 Hz, 1H), 4.69 (br s, 2H), 4.55 (br s, 1H), 3.95 (br s, 1H), 3.87-3.81 (m, 1H), 3.81-3.75 (m, 1H), 3.73-3.61 (m, 2H), 3.52-3.46 (m, 1H), 3.45 (s, 3H), 3.43 (s, 3H), 3.30-3.20 (m, 3H), 3.17 (t, J = 9.2 Hz, 1H), 2.86-2.80 (m, 1H), 2.68-2.57 (m, 1H), 2.57-2.47 (m, 1H), 2.40-2.20 (m, 4H), 1.96-1.72 (m, 4H), 1.70-1.65 (m, 1H), 1.62-1.37 (m,12H), 1.31-1.23 (m,12H), 1.17 (d, J = 7.4 Hz, 3H), 0.93 (t, J = 7.4 Hz, 3H), 0.85 (d, J = 6.9 Hz, 3H), 0.83-0.76 (m, 4H); MSm/z 939.5582 [M+H]⁺.

### Production of Compound II-79

Compound II-64 (50 mg, 0.054 mmol) obtained in [Production of Compound II-64] was dissolved in THF (537 µL), bis(1H-benzo[d][1,2,3]triazol-1-yl)methanimine (14.1 mg, 0.054 mmol) was added, and the mixture was stirred under heating and refluxing for 6 hours. A saturated aqueous sodium hydrogen carbonate solution was added to the reaction solution, and the mixture was extracted with ethyl acetate. The organic layer was washed with saturated brine and dried over anhydrous sodium sulfate. After the solvent was distilled off under reduced pressure, the residue was purified by silica gel column chromatography (n-hexane/ethyl acetate system and chloroform/methanol system) to obtain Compound II-79 (isolated yield 20.3 mg, percent yield 40%).
¹H-NMR (500 MHz, CDCl₃, δ): 6.32 (d, J = 1.7 Hz, 1H), 5.88-5.82 (m, 1H), 5.78-5.69 (m, 2H), 5.43-5.35 (m, 2H), 5.28 (br s, 2H), 5.15 (s, 1H), 4.96 (br d, J = 10.9 Hz, 1H), 4.78 (d, J = 2.9 Hz, 1H), 4.75-4.62 (m, 3H), 3.95 (br s, 1H), 3.88-3.81 (m, 1H), 3.81-3.74 (m, 1H), 3.71-3.61 (m, 2H), 3.53-3.46 (m, 1H), 3.45 (s, 3H), 3.43 (s, 3H), 3.27-3.20 (m, 2H), 3.17 (t, J = 9.2 Hz, 1H), 2.91-2.86 (m, 1H), 2.83-2.65 (m, 1H), 2.55-2.48 (m, 1H), 2.37-2.20 (m, 4H), 1.95-1.89 (m, 1H), 1.88-1.74 (m, 3H), 1.69-1.64 (m, 1H), 1.61-1.36 (m,11H), 1.28 (d, J = 6.3 Hz, 3H), 1.27-1.20 (m, 7H), 1.17 (d, J = 6.9 Hz, 3H), 0.93 (t, J = 7.2 Hz, 3H), 0.84 (d, J = 6.9 Hz, 3H), 0.82-0.77 (m, 4H); MSm/z 956.5491 [M+H]⁺.

### Production of Compound II-80

Compound II-79 (10.2 mg, 10.7 µmol) obtained in [Production of Compound II-79] was dissolved in dichloromethane (213 µL) and then cooled in ice to 0°C. Then, triethylamine (2.97 µL, 0.021 mmol) and acetyl chloride (0.83 µL, 0.012 mmol) were added, and the mixture was stirred at the same temperature for 3 hours. A saturated aqueous sodium hydrogen carbonate solution was added to the reaction solution, and the mixture was extracted with chloroform. The organic layer was washed with saturated brine and dried over anhydrous sodium sulfate. After the solvent was distilled off under reduced pressure, the residue was purified by preparative thin-layer chromatography (n-hexane:ethyl acetate = 1:4) to obtain Compound II-80 (isolated yield 2.8 mg, percent yield 26%).
¹H-NMR (500 MHz, CDCl₃, δ): 7.82-7.86 (m, 1H), 6.06-6.11 (m, 1H), 5.86-5.86 (m, 1H), 5.79-5.93 (m, 1H), 5.66-5.78 (m, 2H), 5.31-5.47 (m, 2H), 5.00-5.03 (m, 1H), 4.90-4.99 (m, 1H), 4.80-4.87 (m, 1H), 4.77 (d, J = 4.0 Hz, 1H), 4.65-4.73 (m, 2H), 4.52-4.57 (m, 1H), 3.94 (br s, 1H), 3.72-3.86 (m, 2H), 3.56-3.70 (m, 2H), 3.45-3.51 (m, 1H), 3.43 (s, 3H), 3.42 (s, 3H), 3.19-3.29 (m, 2H), 3.16 (t, J = 9.2 Hz, 1H), 2.84-2.90 (m, 1H), 2.66-2.77 (m, 1H), 2.45-2.56 (m, 2H), 2.43 (s, 2H), 2.19-2.36 (m, 4H), 1.88-1.93 (m, 1H), 1.71-1.85 (m, 3H), 1.35-1.69 (1m, 2H), 1.26 (dd, J = 10.3, 6.3 Hz, 6H), 1.19-1.23 (m, 3H), 1.14-1.18 (m, 3H), 0.92 (t, J = 7.2 Hz, 3H), 0.83 (d, J = 6.9 Hz, 3H), 0.75-0.81 (m, 4H); MSm/z 998.5598 [M+H]⁺.

### Production of Compound II-81

Compound II-64 (50 mg, 0.054 mmol) obtained in [Production of Compound II-64] was dissolved in dichloromethane (537 µL) and then cooled in ice to 0°C. Then, triethylamine (24.7 µL, 0.177 mmol) and methyl 2-chloro-2-oxoacetate (5.42 µL, 0.059 mmol) were sequentially added, and the mixture was stirred at the same temperature for 1 hour. 4-Methylbenzenesulfonyl chloride (11.3 mg, 0.059 mmol) was added to the reaction solution, followed by stirring at room temperature overnight. A saturated aqueous sodium hydrogen carbonate solution was added to the reaction solution, and the mixture was extracted with chloroform. After the solvent was distilled off under reduced pressure, the residue was purified by silica gel column chromatography (n-hexane/ethyl acetate system) to obtain Compound II-81 (isolated yield 44.8 mg, percent yield 84%).
¹H-NMR (500 MHz, CDCl₃, δ): 6.53 (d, J = 2.3 Hz, 1H), 5.87-5.83 (m, 1H), 5.78-5.69 (m, 2H), 5.44-5.36 (m, 2H), 5.35 (s, 1H), 4.97 (br d, J = 11.1 Hz, 1H), 4.80-4.71 (m, 3H), 4.56 (s, 1H), 4.05 (s, 2H), 3.95 (br s, 1H), 3.88-3.81 (m, 1H), 3.81-3.74 (m, 1H), 3.72-3.61 (m, 2H), 3.53-3.43 (m, 1H), 3.45 (s, 3H), 3.43 (s, 3H), 3.27-3.20 (m, 2H), 3.17 (t, J = 9.2 Hz, 1H), 2.95-2.90 (m, 1H), 2.82-2.75 (m, 1H), 2.56-2.47 (m, 2H), 2.37-2.20 (m, 4H), 1.95-1.83 (m, 3H), 1.79-1.74 (m, 1H), 1.70-1.62 (m, 6H), 1.60-1.37 (m,11H), 1.28 (d, J = 6.3 Hz, 3H), 1.27-1.24 (m, 6H), 1.18 (d, J = 6.9 Hz, 3H), 0.93 (t, J = 7.2 Hz, 3H), 0.84 (d, J = 6.3 Hz, 3H), 0.83-0.77 (m, 4H); MSm/z 1016.5693 [M+NH₄]⁺.

### Production of Compound II-82

Compound II-81 (33.3 mg, 0.033 mmol) obtained in [Production of Compound II-81] was dissolved in THF (333 µL) and then cooled in ice to 0°C. Then, a 1M lithium triethylhydroborate THF solution (333 µL, 0.333 mmol) was added, and the mixture was stirred at the same temperature for 1 hour. Methanol was added to the reaction solution, and the mixture was stirred at the same temperature for 5 minutes. Then, a saturated aqueous sodium hydrogen carbonate solution was added, and the mixture was extracted with ethyl acetate. The organic layer was washed with saturated brine and dried over anhydrous sodium sulfate. After the solvent was distilled off under reduced pressure, the residue was roughly purified by silica gel column chromatography (n-hexane/ethyl acetate system) and further purified by preparative thin-layer chromatography (n-hexane:ethyl acetate = 1:4) to obtain Compound II-82 (isolated yield 12.6 mg, percent yield 39%).
¹H-NMR (500 MHz, CDCl₃, δ): 6.48 (d, J = 2.3 Hz, 1H), 5.88-5.83 (m, 1H), 5.79-5.67 (m, 2H), 5.44-5.36 (m, 2H), 5.25 (s, 1H), 4.97 (br d, J = 10.9 Hz, 1H), 4.84 (br s, 2H), 4.78 (d, J = 3.4 Hz, 1H), 4.77-4.70 (m, 2H), 4.64 (s, 1H), 3.95 (br s, 1H), 3.84 (dq, J = 9.5, 6.2 Hz, 1H), 3.81-3.75 (m, 1H), 3.72-3.61 (m, 2H), 3.52-3.46 (m, 1H), 3.45 (s, 3H), 3.43 (s, 3H), 3.27-3.20 (m, 2H), 3.17 (t, J = 9.2 Hz, 1H), 3.02-2.95 (m, 1H), 2.94-2.89 (m, 1H), 2.79-2.72 (m, 1H), 2.58 (br s, 1H), 2.55-2.48 (m, 1H), 2.38-2.20 (m, 4H), 1.9674-1.74 (m, 4H), 1.71-1.65 (m, 3H), 1.61-1.38 (m,11H), 1.28 (d, J = 6.3 Hz, 3H), 1.28-1.23 (m, 6H), 1.18 (d, J = 6.9 Hz, 3H), 0.93 (t, J = 7.2 Hz, 3H), 0.85 (d, J = 6.9 Hz, 3H), 0.83-0.77 (m, 4H); MSm/z 971.5479 [M+H]⁺.

### Production of Compound II-83

Compound II-83 (isolated yield 7.9 mg, percent yield 7%) was obtained in a similar manner to [Production of Compound II-76], except that ethyl 4-bromo-1H-pyrazole-5-carboxylate was used in place of 3-iodo-1H-pyrazole-4-carbonitrile in [Production of Compound II-76].
¹H-NMR (500 MHz, CDCl₃, δ): 7.96 (s, 1H), 6.96 (d, J = 2.3 Hz, 1H), 5.90-5.85 (m, 1H), 5.80-5.67 (m, 2H), 5.47-5.35 (m, 2H), 4.96 (br d, J = 10.9 Hz, 1H), 4.80 (s, 1H), 4.78 (d, J = 4.0 Hz, 1H), 4.72 (d, J = 1.7 Hz, 2H), 4.35 (s, 1H), 4.33-4.25 (m, 2H), 3.95 (br s, 1H), 3.87-3.81 (m, 1H), 3.81-3.74 (m, 1H), 3.72-3.62 (m, 2H), 3.52-3.46 (m, 1H), 3.45 (s, 3H), 3.43 (s, 3H), 3.28-3.20 (m, 2H), 3.17 (t, J = 9.2 Hz, 1H), 2.84-2.79 (m, 1H), 2.71-2.64 (m, 1H), 2.55-2.48 (m, 2H), 2.39-2.20 (m, 4H), 1.94-1.82 (m, 3H), 1.80-1.72 (m, 1H), 1.71-1.65 (m, 1H), 1.64-1.39 (m, 9H), 1.36 (t, J = 7.2 Hz, 3H), 1.32 (d, J = 6.3 Hz, 3H), 1.30-1.25 (m, 9H), 1.17 (d, J = 7.1 Hz, 3H), 0.93 (t, J = 7.4 Hz, 3H), 0.85 (d, J = 6.9 Hz, 3H), 0.83-0.77 (m, 4H); MSm/z 1011.58 [M+H]⁺.

### Production of Compound II-84

Compound II-84 (isolated yield 4.5 mg, percent yield 59%) was obtained in a similar manner to [Production of Compound II-82], except that Compound II-83 was used in place of Compound II-81 in [Production of Compound II-82].
¹H-NMR (500 MHz, CDCl₃, δ): 7.57 (s, 1H), 6.53 (d, J = 2.3 Hz, 1H), 5.83 (dt, J = 10.3, 2.4 Hz, 1H), 5.77-5.64 (m, 2H), 5.45-5.33 (m, 2H), 4.96 (br d, J = 11.5 Hz, 1H), 4.82 (br s, 1H), 4.79-4.76 (m, 1H), 4.72-4.60 (m, 2H), 4.56-4.49 (m, 2H), 4.30 (s, 1H), 3.94 (br s, 1H), 3.87-3.81 (m, 1H), 3.81-3.74 (m, 1H), 3.72-3.61 (m, 2H), 3.52-3.46 (m, 1H), 3.45 (s, 3H), 3.43 (s, 3H), 3.27-3.21 (m, 2H), 3.17 (t, J = 9.2 Hz, 1H), 2.86-2.81 (m, 1H), 2.69-2.61 (m, 1H), 2.54-2.46 (m, 1H), 2.38-2.19 (m, 4H), 1.95-1.90 (m, 1H), 1.87-1.79 (m, 2H), 1.78-1.72 (m, 1H), 1.70-1.64 (m, 1H), 1.61-1.37 (m,11H), 1.30-1.24 (m,13H), 1.15 (d, J = 6.9 Hz, 3H), 0.93 (t, J = 7.4 Hz, 3H), 0.90-0.87 (m, 1H), 0.85 (d, J = 6.9 Hz, 3H), 0.81-0.77 (m, 4H); MSm/z 969.5695 [M+H]⁺.

### Production of Compound II-85

Compound II-85 (isolated yield 22.4 mg, percent yield 42%) was obtained in a similar manner to [Production of Compound II-55], except that 4-(4,4,5,5-tetramethyl-1,3,2-dioxaborolan-2-yl)pyridin-2-amine was used in place of pyridin-3-ylboronic acid in [Production of Compound II-55].
¹H-NMR (500 MHz, CDCl₃, δ): 7.86 (d, J = 5.2 Hz, 1H), 6.56 (d, J = 4.6 Hz, 1H), 6.50 (d, J = 1.7 Hz, 1H), 6.41 (s, 1H), 5.78-5.83 (m, 1H), 5.65-5.73 (m, 2H), 5.33-5.40 (m, 2H), 4.94 (br d, J = 10.9 Hz, 1H), 4.71-4.80 (m, 2H), 4.64 (qd, J = 14.6, 2.6 Hz, 2H), 4.32 (s, 1H), 3.92 (br s, 1H), 3.71-3.84 (m, 2H), 3.58-3.70 (m, 2H), 3.47 (ddd, J = 11.5, 8.9, 4.9 Hz, 1H), 3.41 (s, 3H), 3.40 (s, 3H), 3.18-3.25 (m, 2H), 3.15 (t, J = 9.2 Hz, 1H), 2.80-2.88 (m, 1H), 2.55-2.63 (m, 1H), 2.44-2.51 (m, 1H), 2.17-2.35 (m, 4H), 1.89-1.94 (m, 1H), 1.72-1.84 (m, 3H), 1.65 (d, J = 12.6 Hz, 1H), 1.36-1.57 (1m, 3H), 1.22-1.28 (m, 6H), 1.21 (d, J = 6.3 Hz, 3H), 1.12 (d, J = 6.9 Hz, 3H), 0.91 (t, J = 7.4 Hz, 3H), 0.87-0.88 (m, 1H), 0.82 (d, J = 6.9 Hz, 3H), 0.75-0.81 (m, 4H); MSm/z 965.575 [M+H]⁺.

### Production of Compound II-86

Compound II-86 (isolated yield 31.9 mg, percent yield 64%) was obtained in a similar manner to [Production of Compound I-129], except that Compound II-65 obtained in [Production of Compound II-65] was used in place of Compound I-64 in [Production of Compound I-129].
¹H-NMR (500 MHz, CDCl₃, δ): 8.35 (s, 1H), 6.52 (d, J = 2.3 Hz, 1H), 5.88-5.83 (m, 1H), 5.77-5.70 (m, 2H), 5.55-5.51 (m, 1H), 5.43-5.36 (m, 1H), 5.29 (s, 1H), 4.96 (d, J = 9.7 Hz, 1H), 4.79 (d, J = 3.4 Hz, 1H), 4.77-4.69 (m, 2H), 4.57 (br s, 1H), 4.42 (q, J = 6.7 Hz, 1H), 4.21 (dd, J = 11.5, 6.3 Hz, 1H), 3.95 (br s, 1H), 3.93-3.88 (m, 1H), 3.73-3.63 (m, 2H), 3.51 (s, 3H), 3.46 (s, 3H), 3.33 (t, J = 8.9 Hz, 1H), 3.22 (d, J = 9.2 Hz, 1H), 2.94-2.89 (m, 1H), 2.80-2.72 (m, 1H), 2.60 (ddd, J = 13.0, 6.4, 1.7 Hz, 1H), 2.55-2.49 (m, 1H), 2.38-2.23 (m, 3H), 2.16-2.08 (m, 1H), 1.96-1.72 (m, 5H), 1.67 (d, J = 12.6 Hz, 1H), 1.60-1.38 (m,10H), 1.32-1.22 (m,10H), 1.17 (d, J = 6.9 Hz, 3H), 0.93 (t, J = 7.5 Hz, 3H), 0.87-0.75 (m, 7H); MSm/z 956.5491 [M+NH₄]⁺.

### Production of Compound II-87

Compound II-87 (isolated yield 23.1 mg, percent yield 32%) was obtained in a similar manner to [Production of Compound II-76], except that 6-bromopyridazin-3-amine was used in place of 3-iodo-1H-pyrazole-4-carbonitrile in [Production of Compound II-76].
¹H-NMR (500 MHz, CDCl₃, δ): 7.31 (d, J = 9.2 Hz, 1H), 6.76-6.70 (m, 2H), 5.89-5.77 (m, 1H), 5.73-5.65 (m, 2H), 5.42-5.31 (m, 2H), 5.11 (br s, 2H), 4.98-4.90 (m, 1H), 4.75 (d, J = 3.4 Hz, 1H), 4.65 (qd, J = 14.3, 2.3 Hz, 2H), 4.49 (s, 1H), 3.91 (br s, 1H), 3.84-3.78 (m, 1H), 3.78-3.72 (m, 1H), 3.71-3.64 (m, 1H), 3.64-3.59 (m, 1H), 3.50-3.44 (m, 1H), 3.41 (s, 3H), 3.40 (s, 3H), 3.25-3.18 (m, 2H), 3.15 (t, J = 9.2 Hz, 1H), 2.89-2.81 (m, 1H), 2.71-2.60 (m, 1H), 2.51-2.43 (m, 1H), 2.35-2.18 (m, 5H), 1.96-1.89 (m, 1H), 1.86-1.77 (m, 2H), 1.77-1.72 (m, 1H), 1.65 (d, J = 12.6 Hz, 1H), 1.57-1.35 (1m, 3H), 1.27-1.21 (m,10H), 1.13 (d, J = 6.9 Hz, 3H), 0.90 (t, J = 7.4 Hz, 3H), 0.82 (d, J = 6.9 Hz, 3H), 0.81-0.73 (m, 4H); MSm/z 966.5699 [M+H]⁺.

### Production of Compound II-88

Compound II-88 (isolated yield 35.1 mg, percent yield 72%) was obtained in a similar manner to [Production of Compound II-76], except that 2-bromopyridin-3-amine was used in place of 3-iodo-1H-pyrazole-4-carbonitrile in [Production of Compound II-76].
¹H-NMR (500 MHz, CDCl₃, δ): 7.95 (dd, J = 4.3, 1.4 Hz, 1H), 6.97-6.91 (m, 2H), 6.50 (d, J = 1.7 Hz, 1H), 5.88-5.84 (m, 1H), 5.72-5.65 (m, 2H), 5.45 (1H,br s),5.38 (d, J = 3.4 Hz, 1H), 5.30-5.23 (m, 1H), 4.97 (br d, J = 9.7 Hz, 1H), 4.76 (br d, J = 3.4 Hz, 1H), 4.63 (dd, J = 14.3, 2.3 Hz, 1H), 4.52 (dd, J = 14.3, 2.3 Hz, 1H), 4.44 (s, 1H), 3.91 (br s, 1H), 3.86-3.78 (m, 3H), 3.78-3.72 (m, 1H), 3.71-3.64 (m, 1H), 3.64-3.59 (m, 1H), 3.50-3.45 (m, 1H), 3.42 (s, 3H), 3.41 (s, 3H), 3.24-3.18 (m, 2H), 3.15 (t, J = 9.2 Hz, 1H), 2.86-2.82 (m, 1H), 2.77-2.70 (m, 1H), 2.56 (br s, 1H), 2.52-2.4 (m,5H), 2.35-2.27 (m, 2H), 2.27-2.19 (m, 2H), 2.04-1.99 (m, 1H), 1.86-1.75 (m, 4H), 1.66-1.60 (m, 1H), 1.57-1.38 (1m, 2H), 1.31 (t, J = 11.7 Hz, 1H), 1.27-1.22 (m, 9H), 1.12 (d, J = 6.9 Hz, 3H), 0.91 (t, J = 7.4 Hz, 3H), 0.82 (d, J = 6.9 Hz, 3H), 0.81-0.75 (m, 4H); MSm/z 965.5739 [M+H]⁺.

### Production of Compound II-89

Compound II-89 (isolated yield 33.8 mg, percent yield 68%) was obtained in a similar manner to [Production of Compound II-76], except that 5-bromopyridin-3-amine was used in place of 3-iodo-1H-pyrazole-4-carbonitrile in [Production of Compound II-76] .
¹H-NMR (500 MHz, CDCl₃, δ): 7.90 (d, J = 18.9 Hz, 1H), 6.89 (s, 1H), 6.56 (s, 1H), 5.82-5.74 (m, 1H), 5.71-5.64 (m, 2H), 5.40-5.33 (m, 2H), 4.92 (br d, J = 10.3 Hz, 1H), 4.74 (d, J = 3.4 Hz, 1H), 4.69-4.57 (m, 2H), 4.28 (s, 1H), 3.90 (br s, 1H), 3.82-3.71 (m, 2H), 3.69-3.58 (m, 3H), 3.49-3.43 (m, 1H), 3.41 (s, 3H), 3.39 (s, 3H), 3.27-3.17 (m, 2H), 3.14 (t, J = 9.2 Hz, 1H), 2.88-2.80 (m, 1H), 2.79-2.76 (m, 1H), 2.63-2.55 (m, 1H), 2.50-2.44 (m, 1H), 2.34-2.27 (m, 2H), 2.27-2.17 (m, 2H), 1.93-1.86 (m, 1H), 1.83-1.70 (m, 3H), 1.67-1.60 (m, 1H), 1.56-1.36 (1m, 3H), 1.24 (dd, J = 12.6, 6.3 Hz, 9H), 1.11 (d, J = 6.9 Hz, 3H), 0.90 (t, J = 7.4 Hz, 3H), 0.82 (d, J = 6.9 Hz, 3H), 0.80-0.74 (m, 4H); MSm/z 965.5742 [M+H]⁺.

### Production of Compound II-90

Compound II-90 (isolated yield 0.8 mg, percent yield 2%) was obtained in a similar manner to [Production of Compound II-76], except that 5-bromo-1H-pyrazol-4-amine was used in place of 3-iodo-1H-pyrazole-4-carbonitrile in [Production of Compound II-76].
¹H-NMR (500 MHz, CDCl₃, δ): 7.17 (s, 1H), 6.39-6.35 (m, 1H), 5.85-5.80 (m, 1H), 5.73-5.66 (m, 2H), 5.42-5.36 (m, 2H), 4.98-4.92 (m, 1H), 4.79-4.74 (m, 1H), 4.72-4.61 (m, 3H), 4.39 (s, 1H), 3.93 (br s, 1H), 3.85-3.79 (m, 1H), 3.78-3.72 (m, 1H), 3.68-3.59 (m, 2H), 3.49 (s, 1H), 3.44 (s, 3H), 3.42 (s, 3H), 3.26-3.19 (m, 2H), 3.16 (t, J = 9.2 Hz, 1H), 2.85-2.80 (m, 1H), 2.66-2.59 (m, 1H), 2.53-2.47 (m, 1H), 2.36-2.31 (m, 2H), 2.28-2.19 (m, 2H), 1.93-1.89 (m, 2H), 1.84-1.33 (1m, 3H), 1.28-1.23 (1m, 1H), 1.14 (d, J = 6.9 Hz, 4H), 0.92 (t, J = 7.2 Hz, 1H), 0.84 (d, J = 6.3 Hz, 3H), 0.80-0.77 (m, 4H); MSm/z 954.5703 [M+H]⁺.

### Production of Compound II-91

Compound II-91 (isolated yield 64.7 mg, percent yield 62%) was obtained in a similar manner to [Production of Compound II-50], except that Compound II-27 obtained in [Production of Compound II-27] was used in place of Compound II-6 in [Production of Compound II-50].
¹H-NMR (500 MHz, CDCl₃, δ): 7.90 (s, 1H), 7.33 (s, 1H), 5.85-5.81 (m, 1H), 5.75-5.65 (m, 2H), 5.43-5.36 (m, 2H), 4.94 (br d, J = 9.7 Hz, 1H), 4.75 (d, J = 4.0 Hz, 1H), 4.67-4.58 (m, 2H), 4.28-4.26 (m, 1H), 3.92 (br s, 1H), 3.85-3.73 (m, 2H), 3.69-3.57 (m, 2H), 3.51-3.44 (m, 1H), 3.44-3.42 (m, 3H), 3.42-3.40 (m, 3H), 3.27-3.18 (m, 2H), 3.15 (t, J = 9.2 Hz, 1H), 2.75 (dd, J = 13.2, 2.9 Hz, 2H), 2.52-2.45 (m, 1H), 2.36-2.19 (m, 4H), 1.99-1.92 (m, 1H), 1.92-1.85 (m, 1H), 1.77-1.70 (m, 2H), 1.66 (br d, J = 12.6 Hz, 1H), 1.58-1.37 (1m, 6H), 1.28-1.23 (m, 6H), 1.14 (d, J = 6.9 Hz, 3H), 0.93-0.89 (m, 3H), 0.83 (d, J = 6.9 Hz, 3H), 0.80-0.74 (m, 4H); MSm/z 980.5164 [M+Na]⁺.

### Production of Compound II-92

Compound II-92 (isolated yield 30.9 mg, percent yield 70%) was obtained in a similar manner to [Production of Compound II-76], except that 4-bromopyridin-2-ol was used in place of 3-iodo-1H-pyrazole-4-carbonitrile in [Production of Compound II-76] .
¹H-NMR (500 MHz, CDCl₃, δ): 7.24 (d, J = 6.3 Hz, 1H), 6.54 (s, 1H), 6.44 (br s, 1H), 6.23 (dd, J = 6.9, 1.7 Hz, 1H), 5.81-5.77 (m, 1H), 5.71-5.64 (m, 2H), 5.42-5.35 (m, 2H), 4.93 (br d, J = 10.9 Hz, 1H), 4.75 (br d, J = 3.4 Hz, 1H), 4.68-4.62 (m, 2H), 4.56 (br s, 1H), 4.34 (s, 1H), 3.92 (br s, 1H), 3.85-3.72 (m, 2H), 3.71-3.59 (m, 2H), 3.50-3.44 (m, 1H), 3.43 (s, 3H), 3.41 (s, 3H), 3.26-3.19 (m, 2H), 3.16 (t, J = 9.2 Hz, 1H), 2.87-2.81 (m, 1H), 2.64-2.56 (m, 1H), 2.51-2.44 (m, 1H), 2.35-2.30 (m, 2H), 2.30-2.18 (m, 2H), 1.92-1.87 (m, 1H), 1.86-1.64 (m, 5H), 1.57-1.37 (1m, 2H), 1.27 (d, J = 6.3 Hz, 3H), 1.24 (d, J = 5.7 Hz, 4H), 1.21 (d, J = 6.3 Hz, 3H), 1.13 (d, J = 6.9 Hz, 3H), 0.93-0.89 (m, 3H), 0.83 (d, J = 6.9 Hz, 3H), 0.81-0.74 (m, 5H); MSm/z 966.5586 [M+H]⁺.

### Production of Compound II-93

Compound II-93 (isolated yield 9.8 mg, percent yield 20%) was obtained in a similar manner to [Production of Compound II-55], except that 5-(4,4,5,5-tetramethyl-1,3,2-dioxaborolan-2-yl)-pyrimidine was used in place of pyridin-3-ylboronic acid in [Production of Compound II-55].
¹H-NMR (500 MHz, CDCl₃, δ): 9.06 (s, 1H), 8.70 (s, 2H), 6.58 (d, J = 1.7 Hz, 1H), 5.81-5.78 (m, 1H), 5.72-5.66 (m, 2H), 5.43-5.37 (m, 2H), 4.93 (br d, J = 9.7 Hz, 1H), 4.75 (d, J = 2.9 Hz, 1H), 4.71-4.64 (m, 2H), 4.63 (s, 1H), 4.16 (s, 1H), 3.92 (br s, 1H), 3.84-3.72 (m, 2H), 3.69-3.59 (m, 2H), 3.50-3.44 (m, 1H), 3.42 (s, 3H), 3.41 (s, 3H), 3.26-3.19 (m, 2H), 3.15 (t, J = 9.2 Hz, 1H), 2.89-2.84 (m, 1H), 2.69-2.62 (m, 1H), 2.57-2.44 (m, 2H), 2.32 (dd, J = 12.0, 5.2 Hz, 2H), 2.29-2.18 (m, 2H), 1.92-1.87 (m, 1H), 1.85-1.80 (m, 2H), 1.76-1.59 (m, 4H), 1.57-1.34 (1m, 1H), 1.30-1.22 (m, 9H), 1.13 (d, J = 6.9 Hz, 3H), 0.91 (t, J = 7.4 Hz, 3H), 0.83 (d, J = 6.9 Hz, 3H), 0.80-0.75 (m, 4H); MSm/z 951.5570 [M+H]⁺.

### Production of Compound II-94

Compound II-94 (isolated yield 22.7 mg, percent yield 47%) was obtained in a similar manner to [Production of Compound II-76], except that 3-iodo-4-methoxy-1H-pyrazole was used in place of 3-iodo-1H-pyrazole-4-carbonitrile in [Production of Compound II-76] .
¹H-NMR (500 MHz, CDCl₃, δ): 7.25 (s, 1H), 6.45 (d, J = 2.3 Hz, 1H), 5.87-5.81 (m, 1H), 5.75-5.68 (m, 2H), 5.42-5.36 (m, 2H), 4.95 (br d, J = 10.9 Hz, 1H), 4.76 (d, J = 3.4 Hz, 1H), 4.69 (d, J = 2.3 Hz, 2H), 4.62 (br s, 1H), 4.55 (s, 1H), 3.93 (br s, 1H), 3.83-3.79 (m, 1H), 3.78 (s, 3H), 3.77-3.73 (m, 1H), 3.70-3.60 (m, 2H), 3.51-3.45 (m, 1H), 3.44 (s, 3H), 3.41 (s, 3H), 3.26-3.19 (m, 2H), 3.16 (t, J = 8.9 Hz, 1H), 2.81 (dd, J = 12.0, 4.0 Hz, 1H), 2.65-2.45 (m, 3H), 2.36-2.19 (m, 4H), 1.93-1.88 (m, 1H), 1.84-1.72 (m, 3H), 1.68-1.61 (m, 1H), 1.59-1.36 (1m, 3H), 1.29-1.22 (m,10H), 1.15 (d, J = 6.9 Hz, 3H), 0.91 (t, J = 7.4 Hz, 3H), 0.83 (d, J = 6.9 Hz, 3H), 0.81-0.75 (m, 4H); MSm/z 969.5672 [M+H]⁺.

### Production of Compound II-95

Compound II-95 (isolated yield 5.9 mg, percent yield 13%) was obtained in a similar manner to [Production of Compound II-76], except that 4-bromo-1H-1,2,3-triazole was used in place of 3-iodo-1H-pyrazole-4-carbonitrile in [Production of Compound II-76].
¹H-NMR (500 MHz, CDCl₃, δ): 7.67 (s, 1H), 6.61 (d, J = 2.3 Hz, 1H), 5.88-5.80 (m, 1H), 5.76-5.67 (m, 2H), 5.44-5.36 (m, 2H), 4.95 (br d, J = 11.5 Hz, 1H), 4.76 (d, J = 3.4 Hz, 1H), 4.75-4.65 (m, 3H), 4.62 (s, 1H), 3.93 (br s, 1H), 3.86-3.73 (m, 2H), 3.71-3.60 (m, 2H), 3.52-3.45 (m, 1H), 3.43 (s, 3H), 3.42 (s, 3H), 3.29-3.19 (m, 2H), 3.16 (t, J = 9.2 Hz, 1H), 2.88-2.83 (m, 1H), 2.69-2.62 (m, 1H), 2.53-2.47 (m, 1H), 2.36-2.31 (m, 2H), 2.31-2.19 (m, 3H), 1.94-1.87 (m, 2H), 1879-1.79 (m, 2H), 1.79-1.73 (m, 2H), 1.69-1.65 (m, 1H), 1.59-1.36 (1m, 2H), 1.29-1.24 (m, 9H), 1.15 (d, J = 7.4 Hz, 3H), 0.92 (t, J = 7.4 Hz, 3H), 0.83 (d, J = 6.9 Hz, 3H), 0.82-0.75 (m, 4H); MSm/z 940.5524 [M+H]⁺.

### Reference Example 25

### Production of diethyl (E)-(2-(2-tosylhydrazinylidene)ethyl)phosphonate

With reference to Tetrahedron 39, (1998) 3287-3290, diethyl (E)-(2-(2-tosylhydrazinylidene)ethyl)phosphonate (isolated yield 2.8 g, percent yield 82%) was obtained using 4-methylbenzenesulfonohydrazine (1.50 g, 8.05 mmol) and diethyl (2,2-diethoxyethyl)phosphonate (2.15 g, 8.46 mmol) as raw materials.
¹H-NMR (500 MHz, CDCl₃, δ): 7.74 (d, J = 8.6 Hz, 2H), 7.36 (d, J = 8.0 Hz, 2H), 7.12 (td, J = 6.2, 4.9 Hz, 1H), 4.00-3.93 (m, 4H), 2.85 (d, J = 6.3 Hz, 1H), 2.81 (d, J = 5.7 Hz, 1H), 2.40 (s, 3H), 1.19 (t, J = 7.2 Hz, 6H).

### Production of Compound II-96

Diethyl (E)-(2-(2-tosylhydrazinylidene)ethyl)phosphonate (133 mg, 0.381 mmol) obtained in Reference Example 25 was dissolved in THF (1.09 mL) and cooled to 0°C. Then, a 1M potassium tert-butoxide THF solution (762 µL, 0.762 mmol) was added dropwise, and the temperature was raised to room temperature. After stirring at the same temperature for 30 minutes, Compound II-27 (100 mg, 0.109 mmol) obtained in [Production of Compound II-27] dissolved in THF (1.09 mL) was added at 0°C, and the temperature was raised to room temperature. After stirring at the same temperature for 3 hours, the temperature was raised to 60°C, and the mixture was stirred at the same temperature for 2 hours. After cooling the reaction solution to room temperature, a saturated aqueous solution of ammonium chloride was added to the reaction solution, and the mixture was extracted with ethyl acetate. The organic layer was washed with saturated brine and dried over anhydrous sodium sulfate. After the solvent was distilled off under reduced pressure, the residue was roughly purified by silica gel column chromatography (n-hexane/ethyl acetate system), and further repurified by high-performance liquid chromatography to obtain Compound II-96 (isolated yield 36.7 mg, percent yield 35%).
¹H-NMR (500 MHz, CDCl₃, δ): 7.53 (s, 1H), 6.48 (s, 1H), 5.84 (d, J = 10.3 Hz, 1H), 5.73-5.64 (m, 2H), 5.41-5.34 (m, 2H), 4.93 (br d, J = 10.9 Hz, 1H), 4.75 (d, J = 3.4 Hz, 1H), 4.62 (br s, 2H), 4.42 (s, 1H), 3.92 (br s, 1H), 3.85-3.78 (m, 1H), 3.78-3.72 (m, 1H), 3.69-3.57 (m, 2H), 3.51-3.45 (m, 1H), 3.43 (s, 3H), 3.41 (s, 3H), 3.26-3.18 (m, 2H), 3.15 (t, J = 9.2 Hz, 1H), 2.83-2.72 (m, 2H), 2.51-2.43 (m, 1H), 2.35-2.17 (m, 4H), 2.00-1.83 (m, 2H), 1.79-1.69 (m, 2H), 1.67-1.60 (m, 1H), 1.58-1.35 (1m, 5H), 1.28-1.22 (m, 7H), 1.13 (d, J = 6.9 Hz, 3H), 0.91 (t, J = 7.2 Hz, 3H), 0.85-0.80 (m, 3H), 0.80-0.73 (m, 4H); MSm/z 957.5502 [M+H]⁺.

### Reference Example 26

### Production of 3-bromo-4-((2-(trimethylsilyl)ethoxy)methyl)-4H-1,2,4-triazole

3-Bromo-4H-1,2,4-triazole (296 mg, 2.00 mmol) was dissolved in THF (12.0 mL) and then cooled in ice to 0°C. Then, sodium hydride (96 mg, 2.40 mmol) was added, and the temperature was raised to room temperature. After stirring at the same temperature for 30 minutes, (2-(chloromethoxy)ethyl)trimethylsilane (0.426 mL, 2.400 mmol) was added dropwise at 0°C, and the temperature was raised to room temperature. After stirring at the same temperature for 4 hours, water was added to the reaction solution, and the mixture was extracted with ethyl acetate. The organic layer was washed with saturated brine and dried over anhydrous sodium sulfate. After the solvent was distilled off under reduced pressure, the residue was purified by silica gel column chromatography (n-hexane/ethyl acetate system) to obtain isomers of 3-bromo-4-((2-(trimethylsilyl)ethoxy)methyl)-4H-1,2,4-triazole (isolated yield 207.9 mg, percent yield 37%), (isolated yield 109.6 mg, percent yield 20%) and (isolated yield 139.6 mg, percent yield 25%), respectively. The ¹H-NMR spectrum below is the analytical data of the main product.
¹H-NMR (500 MHz, CDCl₃, δ): 8.12 (s, 1H), 5.44 (s, 1H), 3.64 (t, J = 8.0 Hz, 2H), 0.92 (t, J = 8.0 Hz, 2H),-0.01 (s, 9H).

### Production of Compound II-97

Compound II-97 (isolated yield 31.4 mg, percent yield 49%) was obtained in a similar manner to [Production of Compound II-76], except that 3-bromo-4-((2-(trimethylsilyl)ethoxy)methyl)-4H-1,2,4-triazole obtained in Reference Example 26 was used in place of 3-iodo-1H-pyrazole-4-carbonitrile in [Production of Compound II-76].
¹H-NMR (500 MHz, CDCl₃, δ): 8.13 (s, 1H), 6.58 (d, J = 2.3 Hz, 1H), 5.84 (m, 1H), 5.75-5.66 (m, 2H), 5.42 (s, 2H), 5.40-5.34 (m, 2H), 4.95 (br d, J = 10.5 Hz, 1H), 4.76 (d, J = 10.5 Hz, 1H), 4.70 (d, J = 10.5 Hz, 2H), 4.57 (s, 1H), 3.93 (br s, 1H), 3.86-3.80 (m, 1H), 3.79-3.73 (m, 1H), 3.69-3.57 (m, 4H), 3.50-3.45 (m, 1H), 3.43 (s, 3H), 3.41 (s, 3H), 3.25-3.20 (m, 2H), 3.16 (t, J = 9.0 Hz, 1H), 2.92-2.88 (m, 1H), 2.72-2.65 (m, 1H), 2.57 (br s, 1H), 2.52-2.47 (m, 1H), 2.35-2.20 (m, 4H), 1.93-1.89 (m, 1H), 1.82-1.70 (m, 6H), 1.67-1.64 (m, 1H), 1.57-1.35 (m,20H), 1.27-1.22 (m,10H), 1.14 (d, J = 7.0 Hz, 3H), 0.92-0.89 (m, 5H), 0.83 (d, J = 7.0 Hz, 3H), 0.80-0.75 (m, 4H); MSm/z 1070.6346 [M+H]⁺.

### Production of Compound II-98

Compound II-97 (31.4 mg, 0.029 mmol) obtained in [Production of Compound II-97] was dissolved in THF (293 µL), and a 1M tetra-N-butylammonium fluoride THF solution (586 µL, 0.586 mmol) was added, followed by stirring at 60°C for 16 hours. A saturated aqueous solution of ammonium chloride was added to the reaction solution, and the mixture was extracted with diethyl ether. The organic layer was washed with a saturated aqueous solution of ammonium chloride and saturated brine sequentially, and dried over anhydrous sodium sulfate. After the solvent was distilled off under reduced pressure, the residue was roughly purified by silica gel column chromatography (n-hexane/ethyl acetate system) and further repurified by high-performance liquid chromatography to obtain Compound II-98 (isolated yield 14.6 mg, percent yield 53%).
¹H-NMR (500 MHz, CDCl₃, δ): 8.83 (d, J = 1.7 Hz, 1H), 8.71 (s, 1H), 6.70 (d, J = 2.3 Hz, 1H), 5.84-5.78 (m, 1H), 5.75-5.66 (m, 2H), 5.43-5.36 (m, 2H), 4.94 (d, J = 10.9 Hz, 1H), 4.79-4.61 (m, 5H), 3.93 (br s, 1H), 3.85-3.73 (m, 2H), 3.70-3.58 (m, 2H), 3.50-3.43 (m, 1H), 3.43 (s, 3H), 3.41 (s, 3H), 3.26-3.19 (m, 2H), 3.16 (t, J = 9.2 Hz, 1H), 2.93-2.87 (m, 1H), 2.79-2.69 (m, 1H), 2.54-2.44 (m, 1H), 2.35-2.18 (m, 4H), 1.92-1.70 (m, 4H), 1.68-1.64 (m, 1H), 1.58-1.36 (1m, 2H), 1.30-1.23 (m,10H), 1.14 (d, J = 6.9 Hz, 3H), 0.91 (t, J = 7.4 Hz, 3H), 0.85-0.81 (m, 3H), 0.79-0.74 (m, 4H); MSm/z 940.5521 [M+H]⁺.

### Reference Example 27

### Production of 5-bromo-1H-pyrazole-3-carboxamide

Ethyl 5-bromo-1H-pyrazole-3-carboxylate (0.200 g, 0.913 mmol) was dissolved in methanol (3.7 mL) and then stirred at 60°C for 1 hour. Then, aqueous ammonia (1.83 mL) was added at room temperature, and the mixture was stirred at 60°C for 10 hours. The solvent was distilled off under reduced pressure to obtain 5-bromo-1H-pyrazole-3-carboxamide (isolated yield 0.190 g, quantitative).
¹H-NMR (500 MHz, CD₃OD, δ): 6.82 (s, 1H).

### Production of Compound II-99

Compound II-99 (isolated yield 10.7 mg, percent yield 36%) was obtained in a similar manner to [Production of Compound II-76], except that 5-bromo-1H-pyrazole-3-carboxamide obtained in Reference Example 27 was used in place of 3-iodo-1H-pyrazole-4-carbonitrile in [Production of Compound II-76].
¹H-NMR (500 MHz, CD₃OD, δ): 6.81 (s, 1H), 6.54-6.51 (m, 1H), 5.93-5.86 (m, 1H), 5.82-5.78 (m, 1H), 5.67 (dd, J = 14.6, 10.0 Hz, 1H), 5.32 (d, J = 3.4 Hz, 1H), 5.19-5.08 (m, 2H), 4.80-4.73 (m, 2H), 4.65 (dd, J = 14.3, 2.3 Hz, 1H), 4.50 (br s, 1H), 3.97 (br s, 1H), 3.86 (dd, J = 9.2, 6.3 Hz, 1H), 3.73-3.60 (m, 3H), 3.40 (s, 3H), 3.43-3.39 (m, 1H), 3.38 (s, 3H), 3.26-3.23 (m, 1H), 3.17 (t, J = 8.9 Hz, 1H), 3.00 (t, J = 9.2 Hz, 1H), 2.83 (dd, J = 12.6, 3.4 Hz, 1H), 2.67-2.54 (m, 2H), 2.34-2.22 (m, 4H), 2.04-2.00 (m, 1H), 1.88-1.76 (m, 2H), 1.75-1.70 (m, 1H), 1.66-1.34 (1m, 3H), 1.29-1.19 (m,10H), 1.14 (d, J = 6.9 Hz, 3H), 0.94 (t, J = 7.4 Hz, 3H), 0.86 (d, J = 6.9 Hz, 3H), 0.84-0.76 (m, 4H); MSm/z 940.5521 [M+H]⁺.

### Reference Example 28

### Production of 3-iodo-5-methoxy-1H-pyrazole

5-Methoxy-1H-pyrazol-3-amine (0.200 g, 1.77 mmol) was dissolved in 0.5M hydrochloric acid (3.5 mL), and an aqueous solution (1.5 mL) of sodium nitrite (0.244 g, 3.54 mmol) was slowly added dropwise, followed by stirring at 0°C for 15 minutes. Then, an aqueous solution (1.5 mL) of sodium iodide (0.587 g, 3.54 mmol) was added, and the mixture was stirred at 0°C for 2 hours. A saturated aqueous sodium thiosulfate solution was added to the reaction solution, and the mixture was extracted with ethyl acetate. The organic layer was washed with saturated brine and dried over anhydrous magnesium sulfate. After the solvent was distilled off under reduced pressure, the residue was washed with chloroform to obtain 3-iodo-5-methoxy-1H-pyrazole (isolated yield 52.3 mg, percent yield 13%).
¹H-NMR (500 MHz, CDCl₃, δ): 5.86 (s, 2H), 3.88 (s, 3H).

### Production of Compound II-100

Compound II-100 (isolated yield 23.0 mg, percent yield 79%) was obtained in a similar manner to [Production of Compound II-76], except that 3-iodo-5-methoxy-1H-pyrazole obtained in Reference Example 28 was used in place of 3-iodo-1H-pyrazole-4-carbonitrile in [Production of Compound II-76].
¹H-NMR (500 MHz, CDCl₃, δ): 6.87 (s, 1H), 6.50-6.45 (m, 1H), 5.92-5.81 (m, 1H), 5.80-5.68 (m, 2H), 5.44-5.34 (m, 2H), 4.96 (br d, J = 9.2 Hz, 1H), 4.81-4.73 (m, 1H), 4.70-4.61 (m, 3H), 4.61-4.51 (m, 1H), 3.94 (br s, 1H), 3.86-3.80 (m, 1H), 3.80-3.72 (m, 1H), 3.69-3.60 (m, 2H), 3.51-3.45 (m, 1H), 3.43 (s, 3H), 3.40 (s, 3H), 3.27-3.19 (m, 2H), 3.16 (t, J = 9.2 Hz, 1H), 2.81 (dd, J = 11.5, 4.0 Hz, 1H), 2.61-2.45 (m, 3H), 2.36-2.18 (m, 5H), 1.96-1.74 (m, 4H), 1.69-1.64 (m, 1H), 1.59-1.38 (1m, 1H), 1.29-1.21 (m,10H), 1.17 (d, J = 6.9 Hz, 3H), 0.92 (t, J = 7.4 Hz, 3H), 0.84 (d, J = 6.9 Hz, 3H), 0.80-0.74 (m, 4H); MSm/z 1004.5463 [M+Na]⁺.

### Production of Compound II-101

Compound II-101 (isolated yield 7.8 mg, percent yield 14%) was obtained in a similar manner to [Production of Compound II-76], except that methyl 5-bromo-1H-imidazole-2-carboxylate was used in place of 3-iodo-1H-pyrazole-4-carbonitrile in [Production of Compound II-76].
¹H-NMR (500 MHz, CDCl₃, δ): 7.19 (s, 0.7H)*,* 7.17 (0.s, 3H), 6.70-6.67 (0.m,3H), 6.57-6.53 (m,0.7H), 5.88-5.8 (m, 1H), 5.73-5.66 (m, 2H), 5.44-5.34 (m, 2H), 5.27-5.20 (m, 1H), 4.95 (br d, J = 10.9 Hz, 1H), 4.81-4.74 (m, 2H), 4.70-4.59 (m, 2H), 4.45-4.38 (m, 2H), 3.93 (br s, 1H), 3.85-3.72 (m, 2H), 3.71-3.60 (m, 2H), 3.50-3.45 (m, 1H), 3.45-3.43 (m, 3H), 3.43-3.40 (m, 3H), 3.26-3.19 (m, 2H), 3.16 (t, J = 9.2 Hz, 1H), 2.85-2.76 (m, 1H), 2.63-2.46 (m, 3H), 2.36-2.18 (m, 4H), 1.92-1.85 (m, 1H), 1.79-1.61 (m, 4H), 1.58-1.37 (1m, 1H), 1.30-1.22 (m,10H), 1.17-1.10 (m, 5H), 0.92 (t, J = 7.4 Hz, 3H), 0.85-0.82 (m, 3H), 0.80-0.74 (m, 4H).

### Production of Compound II-102

Compound II-102 (isolated yield 2.0 mg, percent yield 27%) was obtained in a similar manner to [Production of Compound II-82], except that Compound II-101 obtained in [Production of Compound II-101] was used in place of Compound II-81 in [Production of Compound II-82].
¹H-NMR (500 MHz, CDCl₃, δ): 6.87 (s, 1H), 6.50-6.45 (m, 1H), 5.92-5.81 (m, 1H), 5.80-5.68 (m, 2H), 5.44-5.34 (m, 2H), 4.96 (br d, J = 9.2 Hz, 1H), 4.81-4.73 (m, 1H), 4.70-4.61 (m, 3H), 4.61-4.51 (m, 1H), 3.94 (br s, 1H), 3.86-3.80 (m, 1H), 3.80-3.72 (m, 1H), 3.69-3.60 (m, 2H), 3.51-3.45 (m, 1H), 3.43 (s, 3H), 3.40 (s, 3H), 3.27-3.19 (m, 2H), 3.16 (t, J = 9.2 Hz, 1H), 2.81 (dd, J = 11.5, 4.0 Hz, 1H), 2.61-2.45 (m, 3H), 2.36-2.18 (m, 5H), 1.96-1.74 (m, 4H), 1.69-1.64 (m, 1H), 1.59-1.38 (1m, 1H), 1.29-1.21 (m,10H), 1.17 (d, J = 6.9 Hz, 3H), 0.92 (t, J = 7.4 Hz, 3H), 0.84 (d, J = 6.9 Hz, 3H), 0.80-0.74 (m, 4H); MSm/z 969.5688 [M+H]⁺.

### Production of Compound II-103

Compound II-24 (30 mg, 0.031 mmol) obtained in [Production of Compound II-24] was dissolved in ethanol (311 µL), hydrazine monohydrate (7.57 µL, 0.156 mmol) was added, and the mixture was stirred at 70°C for 14 hours. Water was added to the reaction solution, and the mixture was extracted with ethyl acetate. The organic layer was washed with water and saturated brine sequentially, and dried over anhydrous sodium sulfate. After the solvent was distilled off under reduced pressure, the residue was purified by silica gel column chromatography (n-hexane/ethyl acetate system and chloroform/methanol system) to obtain Compound II-103 (isolated yield 23.5 mg, percent yield 80%).
¹H-NMR (500 MHz, CDCl₃, δ): 7.83 (s, 1H), 5.84-5.82 (m, 1H), 5.75-5.65 (m, 2H), 5.40-5.34 (m, 2H), 5.09 (d, J = 2.0 Hz, 1H), 4.94 (d, J = 10.5 Hz, 1H), 4.76 (d, J = 3.5 Hz, 1H), 4.67-4.59 (m, 2H), 4.47 (s, 1H), 3.93 (br s, 1H), 3.85-3.80 (m, 1H), 3.79-3.73 (m, 1H), 3.69-3.60 (m, 2H), 3.50-3.46 (m, 1H), 3.44 (s, 3H), 3.42 (s, 3H), 3.25-3.19 (m, 2H), 3.16 (t, J = 9.0 Hz, 1H), 2.79-2.73 (m, 1H), 2.73 (d, J = 13.0, 3.5 Hz, 1H), 2.63-2.53 (m, 1H), 2.52-2.46 (m, 1H), 2.35-2.20 (m, 4H), 1.97-1.87 (m, 2H), 1.82-1.73 (m, 6H), 1.66-1.64 (m, 1H), 1.58-1.31 (m,13H), 1.28-1.24 (m, 7H), 1.15 (d, J = 7.5 Hz, 3H), 0.91 (t, J = 7.5 Hz, 3H), 0.83 (d, J = 6.5 Hz, 3H), 0.80-0.73 (m, 4H); MSm/z 1070.6346 [M+H]⁺.

### Production of Compound II-104

Compound II-104 (isolated yield 28.8 mg, percent yield 48%) was obtained in a similar manner to [Production of Compound II-81], except that Compound II-103 obtained in [Production of Compound II-103] was used in place of Compound II-64 in [Production of Compound II-81].
¹H-NMR (500 MHz, CDCl₃, δ): 5.88-5.81 (m, 1H), 5.77-5.65 (m, 2H), 5.45-5.35 (m, 2H), 5.00-4.91 (m, 2H), 4.76 (d, J = 4.0 Hz, 1H), 4.70-4.60 (m, 2H), 4.52 (s, 1H), 4.06 (s, 3H), 3.93 (br s, 1H), 3.86-3.74 (m, 2H), 3.70-3.60 (m, 2H), 3.52-3.46 (m, 1H), 3.44 (s, 3H), 3.42 (s, 3H), 3.28-3.19 (m, 2H), 3.16 (t, J = 9.2 Hz, 1H), 2.96-2.87 (m, 1H), 2.79 (dd, J = 13.2, 3.4 Hz, 1H), 2.55-2.44 (m, 2H), 2.39-2.29 (m, 2H), 2.29-2.19 (m, 2H), 2.01 (d, J = 12.0 Hz, 1H), 1.84 (s, 2H), 1.78-1.72 (m, 1H), 1.66 (d, J = 12.6 Hz, 1H), 1.58-1.35 (1m, 1H), 1.30-1.23 (m, 9H), 1.15 (d, J = 6.9 Hz, 3H), 0.92 (t, J = 7.2 Hz, 3H), 0.87 (t, J = 7.2 Hz, 2H), 0.84 (d, J = 6.3 Hz, 3H), 0.80-0.74 (m, 4H); MSm/z 1034.5607 [M+NH₄]⁺.

### Production of Compound II-105

Compound II-104 (26.3 mg, 0.026 mmol) obtained in [Production of Compound II-104] was dissolved in THF (517 µL), 1M aqueous potassium hydroxide solution (103 µL, 0.103 mmol) was added, and the mixture was stirred at room temperature for 1 hour. Chloroform was added to the reaction solution to dilute it, 1M hydrochloric acid was added, and the mixture was extracted with chloroform. After the solvent was distilled off under reduced pressure, the residue was purified by high-performance liquid chromatography to obtain Compound II-105 (isolated yield 13.3 mg, percent yield 54%).
¹H-NMR (500 MHz, CDCl₃, δ): 8.44 (s, 1H), 5.85-5.81 (m, 1H), 5.75-5.65 (m, 2H), 5.42-5.35 (m, 2H), 4.94 (br d, J = 11.5 Hz, 1H), 4.88 (d, J = 2.3 Hz, 1H), 4.76 (d, J = 3.4 Hz, 1H), 4.69-4.62 (m, 2H), 4.53 (s, 1H), 3.93 (br s, 1H), 3.85-3.79 (m, 1H), 3.79-3.73 (m, 1H), 3.69-3.60 (m, 2H), 3.51-3.45 (m, 1H), 3.43 (s, 3H), 3.42 (s, 3H), 3.25-3.19 (m, 2H), 3.16 (t, J = 9.2 Hz, 1H), 2.90-2.83 (m, 1H), 2.81 (dd, J = 13.2, 3.4 Hz, 1H), 2.53-2.46 (m, 1H), 2.33 (dd, J = 12.6, 4.0 Hz, 2H), 2.30-2.19 (m, 2H), 2.05-1.92 (m, 1H), 1.92-1.88 (m, 1H), 1.79 (dt, J = 13.2, 3.7 Hz, 1H), 1.77-1.61 (m, 5H), 1.59-1.35 (1m, 2H), 1.29-1.22 (m, 7H), 1.15 (d, J = 6.9 Hz, 3H), 0.92 (t, J = 7.2 Hz, 3H), 0.83 (d, J = 6.3 Hz, 3H), 0.80-0.74 (m, 4H); MSm/z 976.5547 [M+NH₄]⁺.

### Production of Compound II-106

Compound II-106 (isolated yield 39.6 mg, percent yield 39%) was obtained in a similar manner to [Production of Compound II-48], except that Compound II-103 obtained in [Production of Compound II-103] was used in place of Compound II-43 in [Production of Compound II-48].
¹H-NMR (500 MHz, CDCl₃, δ): 5.85 (dt, J = 10.7, 2.4 Hz, 1H), 5.78-5.63 (m, 2H), 5.42-5.35 (m, 2H), 4.94 (br d, J = 10.9 Hz, 1H), 4.76 (d, J = 3.4 Hz, 1H), 4.63 (d, J = 1.7 Hz, 2H), 4.60 (d, J = 1.7 Hz, 1H), 3.92 (br s, 1H), 3.85-3.79 (m, 1H), 3.79-3.73 (m, 1H), 3.69-3.60 (m, 2H), 3.52-3.46 (m, 1H), 3.44 (s, 3H), 3.42 (s, 3H), 3.25-3.19 (m, 2H), 3.17 (t, J = 9.2 Hz, 1H), 2.83-2.73 (m, 2H), 2.52-2.44 (m, 1H), 2.36-2.30 (m, 2H), 2.30-2.19 (m, 3H), 2.01-1.94 (m, 1H), 1.93-1.87 (m, 1H), 1.80-1.69 (m, 2H), 1.68-1.63 (m, 1H), 1.58-1.46 (m,10H), 1.46-1.36 (m, 6H), 1.27 (d, J = 6.3 Hz, 3H), 1.24 (d, J = 6.3 Hz, 3H), 1.14 (d, J = 6.9 Hz, 3H), 0.92 (t, J = 7.4 Hz, 3H), 0.83 (d, J = 6.9 Hz, 3H), 0.80-0.73 (m, 4H); MSm/z 992.5488 [M+NH₄]⁺.

### Production of Compound II-107

Compound II-107 (isolated yield 11.1 mg, percent yield 45%) was obtained in a similar manner to [Production of Compound II-76], except that 6-bromo-1,2,4-triazin-3-amine was used in place of 3-iodo-1H-pyrazole-4-carbonitrile in [Production of Compound II-76].
¹H-NMR (500 MHz, CDCl₃,δ):(s, 1H), 6.73 (d, J = 2.3 Hz, 1H), 5.81 (dt, J = 10.3, 2.3 Hz, 1H), 5.72-5.67 (m, 2H), 5.47 (br s, 1H), 5.41-5.35 (m, 2H), 4.94 (br d, J = 9.5 Hz, 1H), 4.75 (br d, J = 2.9 Hz, 1H), 4.71-4.47 (m, 3H), 4.40 (s, 1H), 3.92 (br s, 1H), 3.85-3.73 (m, 2H), 3.68-3.59 (m, 2H), 3.50-3.43 (m, 2H), 3.43 (s, 3H), 3.41 (s, 3H), 3.25-3.19 (m, 2H), 3.16 (t, J = 9.2 Hz, 1H), 2.89-2.84 (m, 1H), 2.70-2.63 (m, 1H), 2.52-2.45 (m, 1H), 2.36-2.18 (m, 5H), 2.16-2.15 (m, 1H), 1.94-1.87 (m, 1H), 1.84-1.80 (m, 1H), 1.76-1.72 (m, 1H), 1.69-1.63 (m, 1H), 1.58-1.35 (m,16H), 1.29-1.15 (m,13H), 1.14 (d, J = 6.9 Hz, 3H), 0.91 (t, J = 7.4 Hz, 3H), 0.83 (d, J = 6.9 Hz, 3H), 0.79-0.75 (m, 4H); MSm/z 967.5648 [M+NH₄]⁺.

### Production of Compound II-108

Compound II-73 (22.4 mg, 0.023 mmol) obtained in [Production of Compound II-73] was dissolved in acetonitrile (468 µL), potassium carbonate (6.47 mg, 0.047 mmol) and methyl iodide (2.93 µL, 0.047 mmol) were added, and the mixture was stirred at 80°C for 2 hours. After cooling the reaction solution to room temperature, water was added, and the mixture was extracted with ethyl acetate. The organic layer was washed with saturated brine and dried over anhydrous sodium sulfate. After the solvent was distilled off under reduced pressure, the residue was purified by silica gel column chromatography (n-hexane/ethyl acetate system) to obtain Compound II-108 (isolated yield 18.4 mg, percent yield 81%).
¹H-NMR (500 MHz, CDCl₃, δ): 6.06 (d, J = 2.5 Hz, 1H), 5.85-5.83 (m, 1H), 5.77-5.68 (m, 2H), 5.425.36 (m, 2H), 5.06 (s, 1H), 4.95 (d, J = 10.5 Hz, 1H), 4.76 (d, J = 3.5 Hz, 1H), 4.74-4.67 (m, 2H), 4.60 (s, 1H), 3.93 (br s, 1H), 3.86-3.80 (m, 1H), 3.79-3.74 (m, 1H), 3.69-3.61 (m, 2H), 3.513.46 (m, 1H), 3.44 (s, 3H), 3.42 (s, 3H), 3.41 (s, 3H), 3.26-3.30 (m, 2H), 3.16 (t, J = 9.0 Hz, 1H), 2.83-2.85 (m, 1H), 2.71-2.64 (m, 1H), 2.56 (s, 1H), 2.54-2.48 (m, 1H), 2.35-2.32 (m, 2H), 2.30-2.20 (m, 2H), 1.90 (dd, J = 12, 3.5 Hz, 1H), 1.84-1.73 (m, 3H), 1.67 (s, 4H), 1.59-1.36 (m,12H), 1.28-1.24 (m, 7H), 1.19 (d, J = 7.0 Hz, 3H), 1.16 (d, J = 7.5 Hz, 3H), 0.92 (t, J = 7.5 Hz, 3H), 0.83 (d, J = 6.5 Hz, 3H), 0.80-0.76 (m, 4H); MSm/z 988.575 [M+NH₄]⁺.

### Production of Compound II-109

Compound II-109 (isolated yield 5.0 mg, percent yield 17%) was obtained in a similar manner to [Production of Compound II-76], except that 4-bromopyrimidin-2-amine was used in place of 3-iodo-1H-pyrazole-4-carbonitrile in [Production of Compound II-76] .
¹H-NMR (500 MHz, CDCl₃, δ): 8.19 (d, J = 4.6 Hz, 1H), 6.67 (d, J = 4.6 Hz, 1H), 6.45 (d, J = 1.7 Hz, 1H), 5.83 (dq, J = 8.2, 2.4 Hz, 1H), 5.74-5.66 (m, 2H), 5.40-5.33 (m, 2H), 5.03 (br s, 2H), 4.97-4.92 (m, 1H), 4.76 (d, J = 3.4 Hz, 1H), 4.74-4.62 (m, 4H), 3.92 (br s, 1H), 3.85-3.72 (m, 2H), 3.71-3.59 (m, 2H), 3.50-3.44 (m, 1H), 3.43 (s, 3H), 3.41 (s, 3H), 3.27-3.18 (m, 2H), 3.16 (t, J = 8.9 Hz, 1H), 2.89-2.83 (m, 1H), 2.69-2.58 (m, 2H), 2.52-2.45 (m, 1H), 2.35-2.30 (m, 2H), 2.30-2.19 (m, 2H), 1.95-1.88 (m, 1H), 1.86-1.72 (m, 3H), 1.70-1.61 (m, 2H), 1.58-1.36 (m,11H), 1.29-1.18 (m,10H), 1.14 (d, J = 6.9 Hz, 3H), 0.91 (t, J = 7.2 Hz, 3H), 0.83 (d, J = 6.9 Hz, 3H), 0.81-0.75 (m, 4H); MSm/z 966.5696 [M+H]⁺.

### Production of Compound II-110

Compound II-110 (isolated yield 9.9 mg, percent yield 38%) was obtained in a similar manner to [Production of Compound II-76], except that 4-bromo-2-methoxypyrimidine was used in place of 3-iodo-1H-pyrazole-4-carbonitrile in [Production of Compound II-76].
¹H-NMR (500 MHz, CDCl₃, δ): 8.42 (d, J = 5.2 Hz, 1H), 6.96 (d, J = 5.2 Hz, 1H), 6.55 (d, J = 1.7 Hz, 1H), 5.83-5.80 (m, 1H), 5.72-5.68 (m, 2H), 5.42-5.35 (m, 2H), 4.94 (br d, J = 10.9 Hz, 1H), 4.88 (s, 1H), 4.76 (d, J = 2.9 Hz, 1H), 4.74-4.69 (m, 1H), 4.66-4.61 (m, 2H), 3.98 (s, 3H), 3.92 (br s, 1H), 3.82 (dq, J = 9.2, 6.3 Hz, 1H), 3.76 (dq, J = 9.2, 6.3 Hz, 1H), 3.69-3.59 (m, 2H), 3.50-3.44 (m, 1H), 3.43 (s, 3H), 3.41 (s, 3H), 3.25-3.18 (m, 2H), 3.16 (t, J = 8.9 Hz, 1H), 2.90-2.85 (m, 1H), 2.71-2.65 (m, 1H), 2.57-2.45 (m, 2H), 2.35-2.28 (m, 2H), 2.28-2.18 (m, 2H), 1.93-1.88 (m, 1H), 1.86-1.64 (m, 9H), 1.58-1.37 (m,15H), 1.28-1.23 (m,10H), 1.14 (d, J = 6.9 Hz, 3H), 0.91 (t, J = 7.4 Hz, 3H), 0.83 (d, J = 6.9 Hz, 3H), 0.79-0.74 (m, 4H); MSm/z 981.5682 [M+H]⁺.

### Reference Example 29

### (1) Production of 3-iodo-4-methoxy-1H-pyrazole

4-Methoxy-1H-pyrazole (0.700 g, 7.14 mmol) was dissolved in DMF (7.93 mL), 1-iodopyrrolidine-2,5-dione (1.62 g, 7.21 mmol) was added, and the mixture was stirred at room temperature for 3 hours. The reaction solution was diluted with ethyl acetate, a saturated aqueous sodium thiosulfate solution was added, and the mixture was extracted with ethyl acetate. The organic layer was washed with saturated brine and dried over anhydrous sodium sulfate. After the solvent was distilled off under reduced pressure, the residue was purified by silica gel column chromatography (n-hexane/ethyl acetate system) to obtain 3-iodo-4-methoxy-1H-pyrazole (isolated yield 1.11 g, percent yield 69%).
¹H-NMR (500 MHz, CDCl₃, δ): 7.12 (s, 1H), 3.82 (s, 3H).

### (2) Production of 3-iodo-1H-pyrazol-4-ol

3-Iodo-4-methoxy-1H-pyrazole (0.700 g, 3.12 mmol) obtained in Reference Example 29-(1) was dissolved in dichloromethane (15.6 mL), a 1M tribromoborane dichloromethane solution (15.6 mL, 15.6 mmol) was added, and the mixture was stirred at room temperature for 2 hours. Water was added to the reaction solution, and the mixture was extracted with ethyl acetate. A 10% aqueous sodium hydroxide solution was added to the aqueous layer to adjust the pH to 7, and the mixture was extracted with ethyl acetate. The organic layer was dried over anhydrous sodium sulfate. After the solvent was distilled off under reduced pressure, the residue was purified by silica gel column chromatography (n-hexane/ethyl acetate system) to obtain 3-iodo-1H-pyrazol-4-ol (isolated yield 501 mg, percent yield 76%).
¹H-NMR (500 MHz, DMSO, δ): 8.68 (s, 1H), 7.13 (s, 1H).

### (3) Production of 4-((tert-butyldimethylsilyl)oxy)-3-iodo-1H-pyrazole

3-Iodo-1H-pyrazol-4-ol (100 mg, 0.476 mmol) obtained in Reference Example 29-(2) was dissolved in DMF (2.38 mL) and then ice-cooled. Imidazole (195 mg, 2.86 mmol) and tert-butylchlorodimethylsilane (215 mg, 1.43 mmol) were sequentially added, the temperature was raised to room temperature, and the mixture was stirred at the same temperature for 2 hours. Water was added to the reaction solution, and the mixture was extracted with diethyl ether. The organic layer was washed with water and saturated brine sequentially, and dried over anhydrous sodium sulfate. After the solvent was distilled off under reduced pressure, the residue was purified by silica gel column chromatography (n-hexane/ethyl acetate system) to obtain 4-((tertbutyldimethylsilyl)oxy)-3-iodo-1H-pyrazole (isolated yield 129 mg, percent yield 84%).
¹H-NMR (500 MHz, CDCl₃, δ): 7.13-7.12 (m, 1H), 1.02 (s, 9H), 0.20 (s, 6H).

### Production of Compound II-111

Compound II-111 (isolated yield 14.6 mg, percent yield 46%) was obtained in a similar manner to [Production of Compound II-76], except that 4-((tertbutyldimethylsilyl)oxy)-3-iodo-1H-pyrazole obtained in Reference Example 29-(3) was used in place of 3-iodo-1H-pyrazole-4-carbonitrile in [Production of Compound II-76].
¹H-NMR (500 MHz, CDCl₃, δ): 7.13 (s, 1H), 6.44 (d, J = 2.3 Hz, 1H), 5.87-5.83 (m, 1H), 5.73-5.69 (m, 2H), 5.43-5.36 (m, 2H), 4.94 (br d, J = 9.7 Hz, 1H), 4.76 (br d, J = 3.4 Hz, 1H), 4.74-4.66 (m, 2H), 4.58 (br s, 1H), 4.51 (br s, 1H), 3.93 (br s, 1H), 3.86-3.79 (m, 1H), 3.79-3.73 (m, 1H), 3.70-3.60 (m, 2H), 3.52-3.45 (m, 1H), 3.44 (s, 3H), 3.42 (s, 3H), 3.25-3.19 (m, 2H), 3.16 (t, J = 9.2 Hz, 1H), 2.79 (dd, J = 11.7, 3.7 Hz, 1H), 2.65-2.57 (m, 1H), 2.56-2.43 (m, 2H), 2.36-2.31 (m, 2H), 2.30-2.19 (m, 2H), 1.92-1.87 (m, 1H), 1.82-1.73 (m, 2H), 1.66 (d, J = 12.6 Hz, 1H), 1.58-1.37 (m,13H), 1.28-1.23 (m,10H), 1.15 (d, J = 7.4 Hz, 3H), 0.96-0.95 (m, 9H), 0.92 (t, J = 7.2 Hz, 4H), 0.83 (d, J = 6.9 Hz, 3H), 0.80-0.75 (m, 4H), 0.14-0.12 (m, 6H); MSm/z 1069.6395 [M+H]⁺.

### Production of Compound II-112

Compound II-111 (14.6 mg, 0.014 mmol) obtained in [Production of Compound II-111] was dissolved in THF (273 µL), a 1M tetra-N-butylammonium fluoride THF solution (68.3 µL, 0.068 mmol) was added, and the mixture was stirred at room temperature for 0.5 hours. A saturated aqueous solution of ammonium chloride was added to the reaction solution, and the mixture was extracted with diethyl ether. The organic layer was washed with a saturated aqueous solution of ammonium chloride and saturated brine sequentially, and dried over anhydrous sodium sulfate. After the solvent was distilled off under reduced pressure, the residue was purified by high-performance liquid chromatography to obtain Compound II-112 (isolated yield 11.5 mg, percent yield 88%).
¹H-NMR (500 MHz, CDCl₃, δ): 7.22 (s, 1H), 6.42 (d, J = 1.7 Hz, 1H), 5.84 (dt, J = 10.7, 2.4 Hz, 1H), 5.76-5.64 (m, 2H), 5.44-5.37 (m, 2H), 4.94 (br d, J = 10.9 Hz, 1H), 4.76 (d, J = 2.9 Hz, 1H), 4.73-4.60 (m, 2H), 4.34 (s, 1H), 3.93 (br s, 1H), 3.85-3.79 (m, 1H), 3.79-3.73 (m, 1H), 3.71-3.54 (m, 3H), 3.48 (ddd, J = 11.5, 9.2, 4.6 Hz, 1H), 3.43 (s, 3H), 3.42 (s, 3H), 3.26-3.19 (m, 2H), 3.16 (t, J = 9.2 Hz, 1H), 2.84-2.78 (m, 1H), 2.65-2.58 (m, 1H), 2.53-2.44 (m, 1H), 2.33 (dd, J = 12.0, 4.6 Hz, 2H), 2.30-2.18 (m, 2H), 1.94-1.86 (m, 1H), 1.84-1.70 (m, 3H), 1.66 (d, J = 12.6 Hz, 1H), 1.60-1.35 (m,13H), 1.29-1.22 (m, 9H), 1.14 (d, J = 6.9 Hz, 3H), 0.92 (t, J = 7.4 Hz, 3H), 0.83 (d, J = 6.9 Hz, 3H), 0.82-0.74 (m, 4H); MSm/z 955.5541 [M+H]⁺.

### Production of Compound II-113

Compound II-113 (isolated yield 17.7 mg, percent yield 42%) was obtained in a similar manner to [Production of Compound II-76], except that 3-bromo-1H-pyrazolo[3,4-d]pyrimidine was used in place of 3-iodo-1H-pyrazole-4-carbonitrile in [Production of Compound II-76].
¹H-NMR (500 MHz, CDCl₃, δ): 9.39 (s, 1H), 9.00 (s, 1H), 6.77 (d, J = 2.3 Hz, 1H), 5.84-5.76 (m, 1H), 5.71-5.64 (m, 2H), 5.43-5.34 (m, 2H), 4.96-4.91 (m, 1H), 4.77-4.71 (m, 3H), 4.68-4.61 (m, 1H), 3.91 (br s, 1H), 3.83-3.73 (m, 2H), 3.69-3.58 (m, 2H), 3.50-3.45 (m, 1H), 3.41 (s, 3H), 3.40 (s, 3H), 3.25-3.19 (m, 2H), 3.16 (t, J = 9.2 Hz, 1H), 2.97-2.91 (m, 1H), 2.81-2.72 (m, 1H), 2.51-2.43 (m, 1H), 2.35-2.29 (m, 2H), 2.29-2.17 (m, 2H), 1.95-1.84 (m, 3H), 1.77-1.71 (m, 1H), 1.67 (d, J = 12.0 Hz, 1H), 1.57-1.36 (m,14H), 1.35-1.32 (m, 3H), 1.28-1.21 (m, 8H), 1.11 (d, J = 6.9 Hz, 3H), 0.93-0.88 (m, 3H), 0.83 (d, J = 6.9 Hz, 3H), 0.75-0.81 (m, 4H); MSm/z 991.5651 [M+H]⁺.

### Production of Compound II-114

Compound II-114 (isolated yield 12.9 mg, percent yield 30%) was obtained in a similar manner to [Production of Compound II-76], except that 2-bromo-[1,2,4]triazolo[1,5-a]pyridine was used in place of 3-iodo-1H-pyrazole-4-carbonitrile in [Production of Compound II-76].
¹H-NMR (500 MHz, CDCl₃, δ): 8.50 (d, J = 6.4 Hz, 1H), 7.66 (d, J = 8.7 Hz, 1H), 7.45 (t, J = 7.7 Hz, 1H), 6.95 (td, J = 6.9, 1.1 Hz, 1H), 6.72 (d, J = 1.7 Hz, 1H), 5.88-5.85 (m, 1H), 5.79-5.78 (m, 1H), 5.76-5.69 (m, 2H), 5.42-5.35 (m, 2H), 4.96 (br d, J = 10.9 Hz, 1H), 4.79-4.70 (m, 3H), 3.93 (br s, 1H), 3.86-3.73 (m, 2H), 3.71-3.60 (m, 2H), 3.51-3.45 (m, 1H), 3.43 (s, 3H), 3.41 (s, 3H), 3.25-3.19 (m, 2H), 3.16 (t, J = 9.2 Hz, 1H), 2.96-2.91 (m, 1H), 2.79-2.71 (m, 1H), 2.54-2.47 (m, 1H), 2.36-2.29 (m, 3H), 2.29-2.19 (m, 3H), 1.94-1.89 (m, 1H), 1.87-1.82 (m, 2H), 1.80-1.73 (m, 1H), 1.66 (d, J = 12.6 Hz, 1H), 1.59-1.37 (m,11H), 1.30-1.23 (m,11H), 1.15 (d, J = 6.9 Hz, 3H), 0.91 (t, J = 7.4 Hz, 3H), 0.83 (d, J = 6.9 Hz, 3H), 0.82-0.76 (m, 4H); MSm/z 990.569 [M+H]⁺.

### Production of Compound II-115

Compound II-14 (100 mg, 0.109 mmol) obtained in [Production of Compound II-14] was dissolved in DMF (545 µL), di(1H-imidazol-1-yl)methanone (35.4 mg, 0.218 mmol) was added, and the mixture was stirred at room temperature for 1 hour. Guanidine hydrochloride (20.8 mg, 0.218 mmol) was dissolved in DMF (545 µL) and 1,4-dioxane (545 µL), potassium tert-butoxide (24.5 mg, 0.218 mmol) was added, the mixture was stirred at 50°C for 0.5 hours and filtered, and the filtrate was added, followed by stirring at room temperature overnight. A saturated aqueous sodium hydrogen carbonate solution was added to the reaction solution, and the mixture was extracted with diethyl ether. The organic layer was washed with water and saturated brine sequentially, and dried over anhydrous sodium sulfate. The solvent was distilled off under reduced pressure. The residue (55.3 mg) was dissolved in DMF (577 µL) and then ice-cooled. Then, 1,1'-(phenyl-13-iodanediyl)bis(ethan-1-one) (55.8 mg, 0.173 mmol) was added, and the temperature was raised to room temperature. The mixture was stirred at the same temperature for 3.5 hours. A saturated aqueous sodium hydrogen carbonate solution was added to the reaction solution, and the mixture was extracted with diethyl ether. The organic layer was washed with a saturated aqueous sodium hydrogen carbonate solution and saturated brine sequentially, and dried over anhydrous sodium sulfate. After the solvent was distilled off under reduced pressure, the residue was roughly purified by silica gel column chromatography (n-hexane/ethyl acetate system and chloroform/methanol system) and further purified by high-performance liquid chromatography to obtain Compound II-115 (isolated yield 10.9 mg, percent yield 20%).
¹H-NMR (500 MHz, CDCl₃, δ): 6.37 (d, J = 1.7 Hz, 1H), 5.87-5.83 (m, 1H), 5.74-5.70 (m, 2H), 5.48 (s, 1H), 5.42-5.36 (m, 2H), 4.95 (br d, J = 10.9 Hz, 1H), 4.76 (br d, J = 3.4 Hz, 1H), 4.73-4.69 (m, 2H), 4.61 (s, 1H), 4.33 (br s, 2H), 3.93 (br s, 1H), 3.86-3.80 (m, 1H), 3.80-3.74 (m, 1H), 3.70-3.60 (m, 2H), 3.52-3.45 (m, 1H), 3.44 (s, 3H), 3.42 (s, 3H), 3.26-3.19 (m, 2H), 3.16 (t, J = 9.2 Hz, 1H), 2.90 (dd, J = 11.5, 4.6 Hz, 1H), 2.76-2.71 (m, 1H), 2.55-2.48 (m, 2H), 2.34 (dd, J = 12.6, 4.0 Hz, 1H), 2.31-2.19 (m, 2H), 1.90 (dd, J = 12.3, 3.7 Hz, 1H), 1.88-1.81 (m, 1H), 1.78-1.73 (m, 1H), 1.68-1.35 (m,14H), 1.26 (dd, J = 10.9, 6.3 Hz, 8H), 1.22 (d, J = 6.3 Hz, 3H), 1.16 (d, J = 6.9 Hz, 3H), 0.92 (t, J = 7.2 Hz, 3H), 0.83 (d, J = 6.9 Hz, 3H), 0.80-0.76 (m, 4H); MSm/z 978.5316 [M+Na]⁺.

### Production of Compound II-116

Compound II-116 (isolated yield 10.6 mg, percent yield 31%) was obtained in a similar manner to [Production of Compound II-76], except that 3-bromo-4-fluoro-1H-pyrazole was used in place of 3-iodo-1H-pyrazole-4-carbonitrile in [Production of Compound II-76] .
¹H-NMR (500 MHz, CDCl₃, δ): 7.36 (d, J = 4.6 Hz, 1H), 6.44 (d, J = 2.3 Hz, 1H), 5.85 (dt, J = 10.3, 2.3 Hz, 1H), 5.76-5.66 (m, 2H), 5.44-5.37 (m, 2H), 4.95 (br d, J = 10.9 Hz, 1H), 4.76 (d, J = 3.4 Hz, 1H), 4.72 (br s, 1H), 4.69 (d, J = 1.7 Hz, 2H), 4.47 (s, 1H), 3.93 (br s, 1H), 3.85-3.72 (m, 2H), 3.71-3.60 (m, 2H), 3.51-3.44 (m, 1H), 3.44 (s, 3H), 3.41 (s, 3H), 3.25-3.19 (m, 2H), 3.16 (t, J = 9.2 Hz, 1H), 2.84-2.78 (m, 1H), 2.67-2.59 (m, 1H), 2.53-2.45 (m, 1H), 2.36-2.30 (m, 2H), 2.30-2.19 (m, 2H), 1.92-1.87 (m, 1H), 1.85-1.79 (m, 2H), 1.77-1.72 (m, 1H), 1.66 (d, J = 13.2 Hz, 1H), 1.58-1.47 (m,14H), 1.29-1.23 (m,10H), 1.15 (d, J = 6.9 Hz, 3H), 0.92 (t, J = 7.4 Hz, 3H), 0.83 (d, J = 6.9 Hz, 3H), 0.82-0.75 (m, 4H); MSm/z 957.5499 [M+H]⁺.

### Production of Compound II-117

Compound II-117 (isolated yield 3.9 mg, percent yield 8%) was obtained in a similar manner to [Production of Compound II-115], except that formamidine hydrochloride was used in place of guanidine hydrochloride in [Production of Compound II-115].
¹H-NMR (500 MHz, CDCl₃, δ): 8.37 (s, 1H), 6.58 (d, J = 1.7 Hz, 1H), 5.91-5.80 (m, 1H), 5.79-5.66 (m, 2H), 5.50 (s, 1H), 5.46-5.34 (m, 2H), 4.95 (br d, J = 10.9 Hz, 1H), 4.80-4.70 (m, 3H), 4.60 (s, 1H), 3.94 (br s, 1H), 3.86-3.80 (m, 1H), 3.80-3.74 (m, 1H), 3.70-3.60 (m, 2H), 3.52-3.45 (m, 1H), 3.44 (s, 3H), 3.42 (s, 3H), 3.29-3.19 (m, 2H), 3.16 (t, J = 9.2 Hz, 1H), 2.99-2.87 (m, 1H), 2.83-2.73 (m, 1H), 2.54-2.46 (m, 2H), 2.37-2.31 (m, 2H), 2.31-2.18 (m, 2H), 1.93-1.85 (m, 2H), 1.78-1.73 (m, 1H), 1.69-1.35 (m,13H), 1.29-1.23 (m,11H), 1.16 (d, J = 6.9 Hz, 3H), 0.92 (t, J = 7.4 Hz, 3H), 0.83 (d, J = 6.9 Hz, 3H), 0.82-0.75 (m, 4H); MSm/z 963.5208 [M+Na]⁺.

### Reference Example 30

### (1) Production of 4-methoxy-1H-pyrazole-3-carbonitrile

3-Iodo-4-methoxy-1H-pyrazole (100 mg, 0.446 mmol) was dissolved in DMF (1.12 mL), zinc cyanide (105 mg, 0.893 mmol) and tetrakis(triphenylphosphine)palladium (51.6 mg, 0.045 mmol) were added, and the mixture was stirred at 120°C for 2 hours using a microwave reactor. The reaction solution was diluted with ethyl acetate, filtered through Celite, the filtrate was distilled off under reduced pressure, and the residue was purified by silica gel column chromatography (n-hexane/ethyl acetate system) to obtain 4-methoxy-1H-pyrazole-3-carbonitrile (isolated yield 34.6 mg, percent yield 63%).
¹H-NMR (500 MHz, MeOD, δ): 7.55 (s, 1H), 3.84 (s, 3H); MSm/z 123.0425 (M+).

### (2) Production of 5-iodo-4-methoxy-1H-pyrazole-3-carbonitrile

4-Methoxy-1H-pyrazole-3-carbonitrile (26.3 mg, 0.214 mmol) obtained in Reference Example 30-(1) was dissolved in DMF (534 µL), 1-iodopyrrolidine-2,5-dione (57.7 mg, 0.256 mmol) was added, and the mixture was stirred at 60°C overnight. The reaction solution was diluted with ethyl acetate, a saturated aqueous sodium thiosulfate solution was added, and the mixture was extracted with ethyl acetate. The organic layer was washed with saturated brine and dried over anhydrous sodium sulfate. After the solvent was distilled off under reduced pressure, the residue was purified by silica gel column chromatography (n-hexane/ethyl acetate system) to obtain 5-iodo-4-methoxy-1H-pyrazole-3-carbonitrile (isolated yield 47.3 mg, percent yield 89%) .
¹H-NMR (500 MHz, CDCl₃, δ): 4.09 (s, 3H); MSm/z 248.9395 (M+).

### Production of Compound II-118

Compound II-118 (isolated yield 25.6 mg, percent yield 89%) was obtained in a similar manner to [Production of Compound II-76], except that 5-iodo-4-methoxy-1H-pyrazole-3-carbonitrile obtained in Reference Example 30-(2) was used in place of 3-iodo-1H-pyrazole-4-carbonitrile in [Production of Compound II-76].
¹H-NMR (500 MHz, CDCl₃, δ): 6.38 (d, J = 2.9 Hz, 1H), 5.86 (dt, J = 10.7, 2.4 Hz, 1H), 5.78-5.65 (m, 2H), 5.44-5.38 (m, 2H), 4.94 (br d, J = 10.9 Hz, 1H), 4.84 (s, 1H), 4.78-4.73 (m, 1H), 4.72-4.67 (m, 2H), 4.24 (s, 1H), 4.02 (s, 3H), 3.93 (br s, 1H), 3.85-3.73 (m, 2H), 3.70-3.60 (m, 2H), 3.51-3.45 (m, 1H), 3.44 (s, 3H), 3.41 (s, 3H), 3.26-3.19 (m, 2H), 3.16 (t, J = 9.2 Hz, 1H), 2.81-2.76 (m, 1H), 2.66-2.59 (m, 1H), 2.57 (br s, 1H), 2.55-2.47 (m, 1H), 2.36-2.30 (m, 2H), 2.30-2.18 (m, 2H), 1.91-1.87 (m, 1H), 1.84-1.78 (m, 2H), 1.76-1.71 (m, 1H), 1.68-1.64 (m, 2H), 1.59-1.37 (m,13H), 1.30-1.22 (m, 9H), 1.15 (d, J = 6.9 Hz, 3H), 0.92 (t, J = 7.2 Hz, 3H), 0.83 (d, J = 6.9 Hz, 3H), 0.81-0.75 (m, 4H); MSm/z 994.5648 [M+H]⁺.

### Reference Example 31

### Production of 4-iodo-3-methoxy-1-((2-(trimethylsilyl)ethoxy)methyl)-1H-pyrazole

4-Iodo-3-methoxy-1H-pyrazole (50.0 mg, 0.223 mmol) was dissolved in THF (2.23 mL), sodium hydride (13.4 mg, 0.335 mmol) was added, and the mixture was stirred at 0°C for 30 minutes. (2-(Chloromethoxy)ethyl)trimethylsilane (47.0 µL, 0.268 mmol) was added, and the mixture was stirred at room temperature for 1 hour. A saturated aqueous solution of ammonium chloride was added to the reaction solution, and the mixture was extracted with ethyl acetate. The organic layer was washed with saturated brine and dried over anhydrous magnesium sulfate. After the solvent was distilled off under reduced pressure, the residue was purified by silica gel column chromatography (n-hexane/ethyl acetate system) to obtain 4-iodo-3-methoxy-1-((2-(trimethylsilyl)ethoxy)methyl)-1H-pyrazole (isolated yield 73.0 mg, percent yield 92%). ¹H-NMR (500 MHz, CDCl₃, δ): 7.39 (s, 1H), 5.22 (s, 2H), 3.95 (s, 3H), 3.58-3.55 (m, 2H), 0.93-0.89 (m, 2H), - 0.01 (s, 9H).

### Production of Compound II-119

Compound II-119 (isolated yield 3.2 mg, percent yield 49%) was obtained in a similar manner to [Production of Compound II-76], except that 4-iodo-3-methoxy-1-((2-(trimethylsilyl)ethoxy)methyl)-1H-pyrazole obtained in Reference Example 31 was used in place of 3-iodo-1H-pyrazole-4-carbonitrile in [Production of Compound II-76].
¹H-NMR (500 MHz, CDCl₃, δ): 7.46 (s, 1H), 6.40 (d, J = 2.3 Hz, 1H), 5.89-5.80 (m, 1H), 5.78-5.69 (m, 2H), 5.41 (d, J = 3.4 Hz, 2H), 4.96 (d, J = 11.5 Hz, 1H), 4.78 (d, J = 3.4 Hz, 1H), 4.72-4.63 (m, 2H), 4.59 (s, 1H), 4.46 (s, 1H), 3.94 (s, 1H), 3.93 (s, 3H), 3.88-3.74 (m, 2H), 3.73-3.60 (m, 2H), 3.53-3.46 (m, 1H), 3.45 (s, 3H), 3.43 (s, 3H), 3.28-3.20 (m, 3H), 3.18 (t, J = 8.9 Hz, 1H), 2.83 (dd, J = 12.0, 4.0 Hz, 1H), 2.64-2.46 (m, 4H), 2.39-2.20 (m, 5H), 2.02 (d, J = 2.9 Hz, 1H), 1.95-1.89 (m, 1H), 1.83-1.61 (m, 6H), 1.60-1.37 (m, 6H), 1.36-1.22 (m, 6H), 1.16 (d, J = 6.9 Hz, 4H), 0.98 (t, J = 7.4 Hz, 1H), 0.95-0.89 (m, 5H), 0.88-0.76 (m, 8H); MSm/z 969.5689 [M+H]⁺.

### Production of Compound II-120

Compound II-120 (isolated yield 21.0 mg, percent yield 57%) was obtained in a similar manner to [Production of Compound II-76], except that 3-bromo-5-methoxy-4H-1,2,4-triazole was used in place of 3-iodo-1H-pyrazole-4-carbonitrile in [Production of Compound II-76].
¹H-NMR (500 MHz, CDCl₃, δ): 6.42 (d, J = 2.3 Hz, 1H), 5.90-5.80 (m, 1H), 5.76-5.66 (m, 2H), 5.43-5.34 (m, 2H), 4.95 (br d, J = 11.5 Hz, 1H), 4.86 (s, 1H), 4.76 (d, J = 3.4 Hz, 1H), 4.72 (s, 1H), 4.69 (d, J = 2.3 Hz, 2H), 3.97 (s, 3H), 3.93 (br s, 1H), 3.85-3.72 (m, 2H), 3.71-3.60 (m, 2H), 3.46 (d, J = 4.6 Hz, 1H), 3.43 (s, 3H), 3.41 (s, 3H), 3.26-3.18 (m, 2H), 3.15 (t, J = 9.2 Hz, 1H), 2.84-2.79 (m, 1H), 2.70-2.62 (m, 1H), 2.53-2.46 (m, 1H), 2.33 (dd, J = 12.3, 4.3 Hz, 2H), 2.29-2.18 (m, 2H), 1.93-1.88 (m, 1H), 1.84-1.78 (m, 2H), 1.78-1.72 (m, 1H), 1.66 (d, J = 12.6 Hz, 1H), 1.58-1.37 (m,13H), 1.30-1.20 (m, 10H) , 1.15 (d, J = 6.9 Hz, 3H), 0.91 (t, J = 7.4 Hz, 3H), 0.83 (d, J = 6.3 Hz, 3H), 0.80-0.74 (m, 4H); MSm/z 970.5646 [M+H]⁺.

### Production of Compound II-121

Compound II-121 (isolated yield 3.8 mg, percent yield 11%) was obtained in a similar manner to [Production of Compound II-76], except that 3-bromo-4-methyl-1H-pyrazole was used in place of 3-iodo-1H-pyrazole-4-carbonitrile in [Production of Compound II-76].
¹H-NMR (500 MHz, CDCl₃, δ): 7.33 (s, 1H), 6.43 (d, J = 2.3 Hz, 1H), 5.87-5.80 (m, 1H), 5.76-5.66 (m, 2H), 5.45-5.37 (m, 2H), 4.95 (br d, J = 9.7 Hz, 1H), 4.76 (d, J = 3.4 Hz, 1H), 4.69 (d, J = 2.3 Hz, 2H), 4.44 (s, 1H), 3.93 (br s, 1H), 3.86-3.72 (m, 2H), 3.71-3.61 (m, 2H), 3.51-3.45 (m, 1H), 3.44 (s, 3H), 3.42 (s, 3H), 3.26-3.19 (m, 2H), 3.16 (t, J = 9.2 Hz, 1H), 2.85-2.78 (m, 1H), 2.68-2.59 (m, 1H), 2.53-2.46 (m, 1H), 2.36-2.30 (m, 2H), 2.30-2.19 (m, 3H), 1.93-1.88 (m, 2H), 1.84-1.73 (m, 4H), 1.69-1.64 (m, 1H), 1.60-1.36 (m,14H), 1.31-1.21 (m,10H), 1.15 (d, J = 6.9 Hz, 3H), 0.92 (t, J = 7.4 Hz, 3H), 0.84 (d, J = 6.9 Hz, 3H), 0.82-0.75 (m, 4H); MSm/z 953.574 [M+H]⁺.

### Production of Compound II-122

Compound II-122 (isolated yield 1.7 mg, percent yield 3.4%) was obtained in a similar manner to [Production of Compound II-76], except that 6-bromo-N-methylpyridazin-3-amine was used in place of 3-iodo-1H-pyrazole-4-carbonitrile in [Production of Compound II-76] .
¹H-NMR (500 MHz, CDCl₃, δ): 7.48-7.41 (m, 1H), 6.85-6.78 (m, 1H), 6.67 (s, 1H), 5.87-5.78 (m, 1H), 5.75-5.65 (m, 2H), 5.42-5.32 (m, 2H), 4.98-4.91 (m, 1H), 4.80-4.73 (m, 1H), 4.72-4.54 (m, 3H), 4.54-4.47 (m, 1H), 4.32-4.24 (m, 1H), 3.97-3.90 (m, 1H), 3.84-3.79 (m, 1H), 3.79-3.72 (m, 1H), 3.70-3.59 (m, 3H), 3.50-3.44 (m, 1H), 3.43 (s, 3H), 3.41 (s, 3H), 3.26-3.19 (m, 2H), 3.16 (t, J = 8.9 Hz, 1H), 3.01 (d, J = 4.6 Hz, 3H), 2.91-2.84 (m, 1H), 2.71-2.61 (m, 1H), 2.53-2.44 (m, 1H), 2.36-2.19 (m, 5H), 1.95-1.90 (m, 1H), 1.84-1.80 (m, 1H), 1.76-1.60 (m, 1H), 1.58-1.48 (m,12H), 1.33-1.17 (m, 10H) 1.16-1.12 (m, 3H), 0.94-0.78 (m,11H); MSm/z 980.6030 [M+H]⁺.

### Reference Example 32

### Production of 4-iodoisoxazole

Isoxazole (750 mg, 0.694 mmol) was dissolved in trifluoroacetic acid (3.62 mL), 1-iodopyrrolidine-2,5-dione (3.18 g, 14.1 mmol) was added, and the mixture was stirred at 120°C for 20 minutes using a microwave reactor. The reaction solution was added to an ice-cooled saturated aqueous sodium bicarbonate solution to stop the reaction. After extraction with diethyl ether, the organic layer was washed with a 10% aqueous sodium thiosulfate solution and saturated brine sequentially, and dried over anhydrous sodium sulfate. After the solvent was distilled off under reduced pressure, the residue was purified by silica gel column chromatography (n-hexane/ethyl acetate system) and neutral alumina sequentially to obtain 4-iodoisoxazole (isolated yield 800 mg, percent yield 38%).
¹H-NMR (500 MHz, CDCl₃, δ): 8.44 (s, 1H), 8.29 (s, 1H); MSm/z 194.9172 [M]⁺.

### Production of Compound II-123

Compound II-123 (isolated yield 18.9 mg, percent yield 50%) was obtained in a similar manner to [Production of Compound II-76], except that 4-iodoisoxazole obtained in Reference Example 32 was used in place of 3-iodo-1H-pyrazole-4-carbonitrile in [Production of Compound II-76].
¹H-NMR (500 MHz, CDCl₃, δ): 8.42 (s, 1H), 8.27 (s, 1H), 6.36 (s, 1H), 5.87-5.78 (m, 1H), 5.76-5.66 (m, 2H), 5.44-5.37 (m, 2H), 4.98-4.92 (m, 1H), 4.76 (d, J = 3.4 Hz, 1H), 4.74-4.64 (m, 2H), 4.63 (d, J = 1.1 Hz, 1H), 4.62-4.61 (m, 1H), 4.28 (s, 1H), 3.93 (br s, 1H), 3.85-3.73 (m, 2H), 3.70-3.59 (m, 2H), 3.49-3.44 (m, 1H), 3.44-3.42 (m, 3H), 3.42-3.40 (m, 3H), 3.27-3.18 (m, 2H), 3.16 (t, J = 8.9 Hz, 1H), 2.85-2.79 (m, 1H), 2.61-2.54 (m, 1H), 2.54-2.45 (m, 2H), 2.38-2.18 (m, 4H), 1.89 (dd, J = 4.6,12.0 Hz, 1H), 1.81-1.71 (m, 2H), 1.69-1.60 (m, 2H), 1.58-1.38 (m,12H), 1.29-1.21 (m, 9H), 1.15 (d, J = 6.9 Hz, 3H), 0.92 (t, J = 7.2 Hz, 3H), 0.83 (d, J = 6.3 Hz, 3H), 0.81-0.73 (m, 4H); MSm/z 962.5242 [M+Na]⁺.

### Production of Compound II-124

Compound II-84 (14.5 mg, 0.015 mmol) obtained in [Production of Compound II-84] was dissolved in dichloromethane (0.30 mL), manganese dioxide (52.0 mg, 0.598 mmol) was added, and the mixture was stirred at room temperature for 16 hours. After filtering the reaction solution using Celite, the solvent was distilled off under reduced pressure to obtain Compound II-124 (isolated yield 3.3 mg, percent yield 23%).
¹H-NMR (500 MHz, CDCl₃, δ): 9.94 (s, 1H), 7.98 (s, 1H), 6.84 (d, J = 2.3 Hz, 1H), 5.86 (td, J = 2.4,10.7 Hz, 1H), 5.79-5.65 (m, 2H), 5.47-5.34 (m, 2H), 4.95 (br d, J = 10.9 Hz, 1H), 4.97-4.92 (m, 1H), 4.85 (s, 1H), 4.76 (br d, J = 3.4 Hz, 1H), 4.74-4.67 (m, 2H), 4.34 (s, 1H), 3.93 (br s, 1H), 3.85-3.72 (m, 2H), 3.71-3.60 (m, 2H), 3.51-3.45 (m, 1H), 3.43 (s, 3H), 3.41 (s, 3H), 3.28-3.19 (m, 2H), 3.16 (t, J = 9.2 Hz, 1H), 2.86-2.78 (m, 1H), 2.74-2.62 (m, 1H), 2.61-2.44 (m, 2H), 2.37-2.18 (m, 4H), 1.92-1.80 (m, 2H), 1.77-1.71 (m, 1H), 1.69-1.61 (m, 1H), 1.60-1.36 (m, 13H) , 1.31 (d, J = 6.3 Hz, 3H), 1.27 (d, J = 6.3 Hz, 3H), 1.26-1.22 (m, 4H), 1.15 (d, J = 6.9 Hz, 3H), 0.92 (t, J = 7.4 Hz, 3H), 0.84 (d, J = 6.9 Hz, 3H), 0.82-0.74 (m, 4H).

### Production of Compound II-125

Compound II-83 (8.6 mg, 8.5 µmol) obtained in [Production of Compound II-83] was dissolved in THF (0.42 mL), a 1M aqueous potassium hydroxide solution (85 µL, 0.085 mmol) was added at room temperature, and the mixture was stirred at 120°C for 1 hour and at 150°C for 5 hours using a microwave reactor. An aqueous solution of ammonium chloride was added to the reaction solution, the mixture was extracted with ethyl acetate, and the organic layer was dried over anhydrous magnesium sulfate. After the solvent was distilled off under reduced pressure, the residue was purified by preparative thin-layer chromatography (ethyl acetate) to obtain Compound II-125 (isolated yield 1.4 mg, percent yield 17%).
¹H-NMR (500 MHz, CDCl₃, δ): 8.00 (s, 1H), 6.93 (d, J = 1.7 Hz, 1H), 5.91-5.85 (m, 1H), 5.80-5.67 (m, 2H), 5.48-5.38 (m, 2H), 5.01-4.92 (m, 1H), 4.85-4.80 (m, 1H), 4.78 (d, J = 4.6 Hz, 1H), 4.73 (d, J = 2.3 Hz, 2H), 4.35 (s, 1H), 3.98-3.91 (m, 1H), 3.88-3.74 (m, 2H), 3.71-3.62 (m, 2H), 3.52-3.47 (m, 1H), 3.46 (s, 3H), 3.43 (s, 3H), 3.29-3.21 (m, 2H), 3.18 (t, J = 9.2 Hz, 1H), 2.85-2.80 (m, 1H), 2.73-2.64 (m, 1H), 2.54-2.46 (m, 1H), 2.40-2.20 (m, 4H), 1.96-1.89 (m, 1H), 1.88-1.82 (m, 2H), 1.80-1.71 (m, 2H), 1.70-1.37 (m,12H), 1.34-1.19 (m, 8H), 1.18-1.13 (m, 4H), 0.96-0.75 (m,14H); MSm/z 983.5493 [M+Na]⁺.

### Production of Compound III-1

Benzohydrazide (37.7 µL, 0.189 mmol) was dissolved in ethanol (0.6 mL) and cooled to 0°C. Then, 5-oxo-ivermectin B1a (50 mg, 0.057 mmol) and cesium chloride heptahydrate (2.1 mg, 5.73 µmol) were added, and the mixture was stirred at 60°C overnight. A saturated aqueous solution of ammonium chloride was added to the reaction solution, the temperature was raised to room temperature, and the mixture was extracted with ethyl acetate. The organic layer was washed with saturated brine and dried over anhydrous magnesium sulfate. After the solvent was distilled off under reduced pressure, the residue was purified by silica gel column chromatography (n-hexane/ethyl acetate system) to obtain Compound III-1 (isolated yield 28.9 mg, percent yield 51%).
¹H-NMR (500 MHz, CDCl₃, δ): 10.21 (br s, 1H), 7.92-7.80 (m, 2H), 7.59-7.50 (m, 1H), 7.49-7.41 (m, 2H), 6.03-5.97 (m, 1H), 5.96-5.89 (m, 1H), 5.84-5.77 (m, 1H), 5.77-5.68 (m, 1H), 5.48-5.41 (m, 1H), 5.39 (d, J = 4.0 Hz, 1H), 4.98 (br d, J = 10.9 Hz, 1H), 4.77 (d, J = 3.4 Hz, 2H), 4.75 (br s, 1H), 4.35 (br s, 1H), 4.14-4.07 (m, 1H), 3.95 (br s, 1H), 3.86-3.80 (m, 1H), 3.79-3.73 (m, 1H), 3.71-3.65 (m, 1H), 3.64-3.59 (m, 1H), 3.51-3.45 (m, 1H), 3.43 (s, 3H), 3.41 (s, 3H), 3.36-3.32 (m, 1H), 3.27-3.20 (m, 2H), 3.16 (t, J = 8.9 Hz, 1H), 2.57-2.49 (m, 2H), 2.37-2.27 (m, 3H), 2.24-2.19 (m, 1H), 1.99-1.93 (m, 1H), 1.80-1.74 (m, 1H), 1.65 (br s, 3H), 1.59-1.38 (m,13H), 1.26 (dd, J = 6.0, 8.9 Hz, 7H), 1.19-1.14 (m, 3H), 0.93 (t, J = 7.4 Hz, 3H), 0.86 (d, J = 6.9 Hz, 3H), 0.84-0.77 (m, 4H); MSm/z 1003.5359 [M+Na]⁺.

### Production of Compound III-2

Compound III-2 (isolated yield 33.8 mg, percent yield 59%) was obtained in a similar manner to [Production of Compound III-1] except that 2-phenylacetohydrazide was used in place of benzohydrazide.
¹H-NMR (500 MHz, CDCl₃, δ): 9.25 (s, 1H), 7.34-7.28 (m, 4H), 7.25-7.21 (m, 1H), 5.97-5.92 (m, 1H), 5.90-5.87 (m, 1H), 5.81-5.75 (m, 1H), 5.74-5.67 (m, 1H), 5.47-5.40 (m, 1H), 5.39 (d, J = 3.4 Hz, 1H), 5.00-4.94 (m, 1H), 4.77 (d, J = 2.9 Hz, 1H), 4.72-4.64 (m, 2H), 4.16 (s, 1H), 4.12 (s, 1H), 4.06-3.98 (m, 2H), 3.96-3.93 (m, 1H), 3.86-3.80 (m, 1H), 3.79-3.74 (m, 1H), 3.70-3.58 (m, 2H), 3.51-3.45 (m, 1H), 3.43 (s, 3H), 3.42 (s, 3H), 3.30 (t, J = 2.6 Hz, 1H), 3.24 (s, 2H), 3.18-3.14 (m, 1H), 2.57-2.49 (m, 2H), 2.37-2.30 (m, 2H), 2.29-2.18 (m, 2H), 1.99-1.93 (m, 1H), 1.79-1.73 (m, 1H), 1.64 (br s, 3H), 1.59-1.36 (m,13H), 1.29-1.23 (m, 7H), 1.17 (d, J = 6.9 Hz, 3H), 0.93 (t, J = 7.4 Hz, 3H), 0.87-0.84 (m, 3H), 0.82-0.75 (m, 4H); MSm/z 1027.5522 [M+Na]⁺.

### Production of Compound III-3

Compound III-3 (isolated yield 24.6 mg, percent yield 38%) was obtained in a similar manner to [Production of Compound III-1] except that 2-hydroxyacetohydrazide was used in place of benzohydrazide.
¹H-NMR (500 MHz, CDCl₃, δ): 9.36 (s, 1H), 5.99-5.94 (m, 1H), 5.92-5.89 (m, 1H), 5.83-5.76 (m, 1H), 5.75-5.67 (m, 1H), 5.47-5.40 (m, 1H), 5.41-5.37 (m, 1H), 4.97 (br d, J = 11.5 Hz, 1H), 4.83-4.76 (m, 1H), 4.74-4.68 (m, 2H), 4.61-4.55 (m, 1H), 4.49-4.43 (m, 1H), 4.18 (s, 1H), 4.17-4.11 (m, 1H), 3.98-3.93 (m, 1H), 3.89-3.79 (m, 1H), 3.80-3.72 (m, 1H), 3.71-3.65 (m, 1H), 3.64-3.52 (m, 1H), 3.48 (s, 2H), 3.43 (s, 3H), 3.41 (s, 3H), 3.31-3.28 (m, 1H), 3.26-3.19 (m, 2H), 3.16 (t, J = 9.2 Hz, 1H), 3.05 (br s, 1H), 2.59-2.50 (m, 2H), 2.37-2.29 (m, 2H), 2.27-2.18 (m, 2H), 1.97-1.95 (m, 1H), 1.94 (s, 3H), 1.79-1.74 (m, 1H), 1.68-1.63 (m, 1H), 1.60-1.35 (m,10H), 1.29-1.23 (m, 6H), 1.17 (t, J = 6.9 Hz, 3H), 0.93 (t, J = 7.4 Hz, 3H), 0.85 (d, J = 6.9 Hz, 3H), 0.84-0.83 (m, 1H), 0.82-0.77 (m, 4H); MSm/z 945.5262 [M+H]⁺.

### Production of Compound III-4

Compound III-4 (isolated yield 18.3 mg, percent yield 28%) was obtained as a mixture of isomers that were difficult to separate in a similar manner to [Production of Compound III-1] except that 2-methoxyacetohydrazide was used in place of benzohydrazide.
¹H-NMR (500 MHz, CDCl₃, δ): 10.29 (s, 1H), 9.25-9.21 (m,0.5H), 6.02-5.94 (m,0.5H), 5.94-5.87 (m, 1H), 5.84-5.76 (m, 1H), 5.75-5.68 (m, 1H), 5.48-5.41 (m, 1H), 5.39 (br s, 1H), 4.97 (brd, J = 11.5 Hz, 1H), 4.82-4.74 (m, 2H), 4.69 (d, J = 2.3 Hz, 1H), 4.55-4.48 (m,0.5H), 4.44-4.36 (m,0.5H), 4.27 (s, 0.5H), 4.16 (s, 0.5H), 4.14 (s, 0.5H), 4.07 (s, 1H), 4.06-4.05 (m,0.5H), 3.95 (br s, 1H), 3.86-3.80 (m, 1H), 3.78-3.72 (m, 1H), 3.71-3.59 (m, 2H), 3.50 (s, 2H), 3.49-3.46 (m, 1H), 3.45 (s, 2H), 3.43 (s, 1.5H), 3.43 (s, 1.5H), 3.41 (s, 3H), 3.35-3.31 (m,0.5H), 3.31-3.28 (m,0.5H), 3.26-3.20 (m, 2H), 3.16 (t, J = 9.2 Hz, 1H), 2.58-2.47 (m, 2H), 2.33 (d, J = 7.4 Hz, 2H), 2.29-2.19 (m, 2H), 2.08 (s, 2H), 1.98-1.92 (m, 3H), 1.79-1.73 (m, 1H), 1.68-1.63 (m, 1H), 1.60-1.35 (m,13H), 1.29-1.23 (m, 7H), 1.17 (t, J = 7.2 Hz, 3H), 0.93 (t, J = 7.2 Hz, 3H), 0.88-0.83 (m, 4H), 0.82-0.76 (m, 4H)); MSm/z 959.5420 [M+H]⁺.

### Production of Compound III-5

Compound III-5 (isolated yield 22.1 mg, percent yield 32%) was obtained in a similar manner to [Production of Compound III-1] except that tetrahydro-2H-pyran-4-carbohydrazide was used in place of benzohydrazide.
¹H-NMR (500 MHz, CDCl₃, δ): 9.18-9.11 (m, 1H), 5.98-5.93 (m, 1H), 5.90-5.87 (m, 1H), 5.82-5.76 (m, 1H), 5.75-5.67 (m, 1H), 5.48-5.41 (m, 1H), 5.39 (d, J = 3.4 Hz, 1H), 4.97 (brd, J = 10.9 Hz, 1H), 4.77 (d, J = 3.4 Hz, 1H), 4.74-4.65 (m, 2H), 4.18 (s, 1H), 4.14 (s, 1H), 4.06-3.99 (m, 2H), 3.95 (br s, 1H), 3.85-3.79 (m, 1H), 3.78-3.73 (m, 1H), 3.71-3.59 (m, 2H), 3.51-3.44 (m, 4H), 3.43 (s, 3H), 3.42 (s, 3H), 3.37-3.32 (m, 1H), 3.31-3.29 (m, 1H), 3.26-3.20 (m, 2H), 3.16 (t, J = 9.2 Hz, 1H), 2.57-2.49 (m, 2H), 2.37-2.31 (m, 2H), 2.31-2.18 (m, 3H), 1.96 (s, 3H), 1.95-1.92 (m, 1H), 1.92-1.85 (m, 2H), 1.82-1.70 (m, 2H), 1.67-1.63 (m, 1H), 1.60-1.35 (m, 9H), 1.28-1.24 (m, 7H), 1.17 (d, J = 6.9 Hz, 3H), 1.16-1.13 (m, 1H), 0.93 (t, J = 7.4 Hz, 3H), 0.87-0.83 (m, 4H), 0.82-0.77 (m, 4H); MSm/z 999.5692 [M+H]⁺.

The numbers (Comp.) and structural formulas of the compounds obtained above are shown in Tables 1 to 33 below. In the table, "TBS" in the structural formula represents a tert-butyldimethylsilyl group which is a protecting group for a hydroxy group, and the group represented by -O-TBS represents a group in which a tert-butyldimethylsilyl group is substituted for a hydroxy group (-OH). In addition, in the table, the structure containing a wavy line (~) in the structural formula indicates that it is either the (E) form structure or the (Z) form structure when geometric isomers exist, and in this case, the compound obtained in the example is either or a mixture of these isomers. Furthermore, in the table, a single bond represented by a straight line (-) in the structural formula indicates that it is either a "wedge-shaped bond" or a "dashed bond" when stereoisomers exist, and in this case, the compound obtained in the example is either or a mixture of these isomers, but when an asymmetric carbon bonded to a single bond represented by a straight line (-) is marked with "*," the compound obtained in the example is one of these isomers.

**[Table 1]**

| Comp. | Structural formula | Comp. | Structural formula |
|---|---|---|---|
| I-1 | 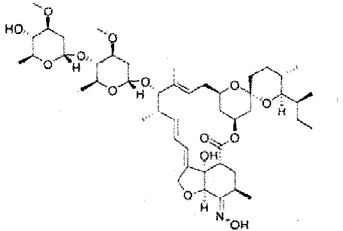 | I-6 | 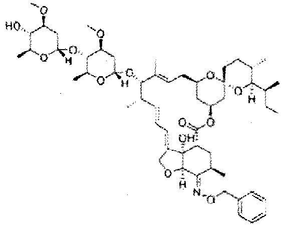 |
| I-2 | 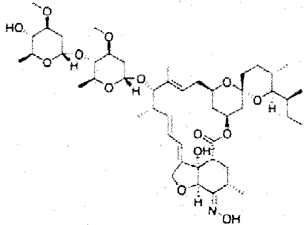 | I-7 | 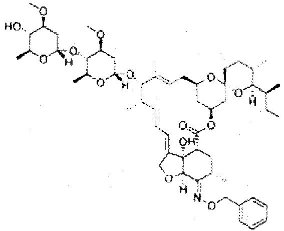 |
| I-3 | 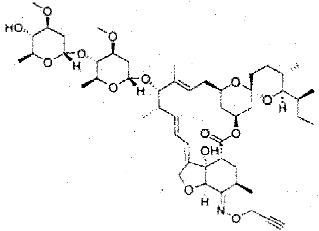 | I-8 | 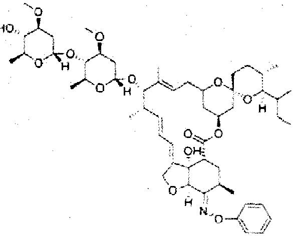 |
| I-4 | 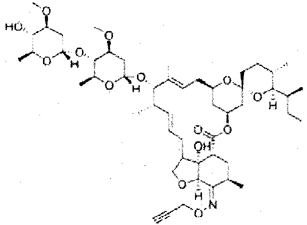 | I-9 | 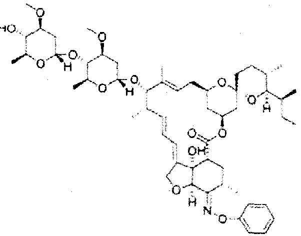 |
| I-5 | 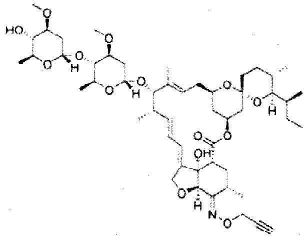 | I-10 | 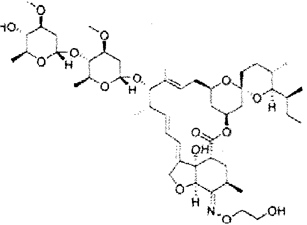 |

**[Table 2]**

| Comp. | Structural formula | Comp. | Structural formula |
|---|---|---|---|
| I-11 | 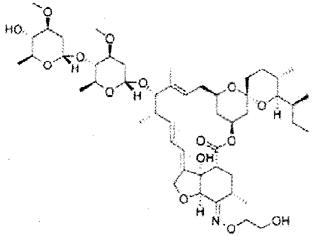 | I-16 | 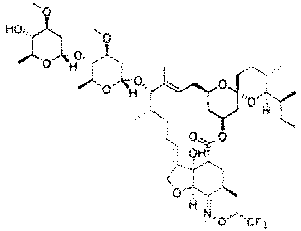 |
| I-12 | 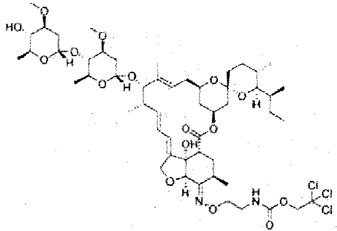 | I-17 | 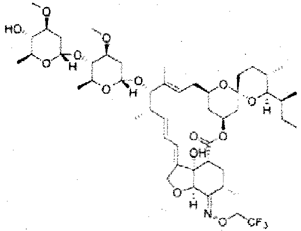 |
| I-13 | 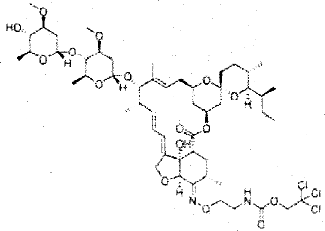 | I-18 | 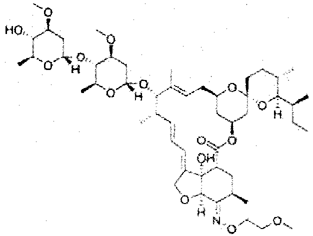 |
| I-14 | 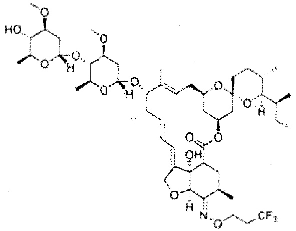 | I-19 | 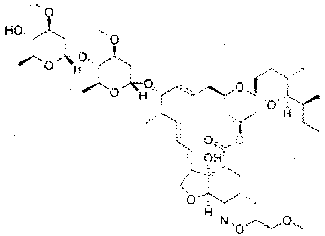 |
| I-15 | 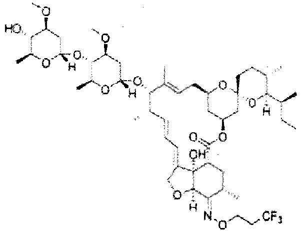 | I-20 | 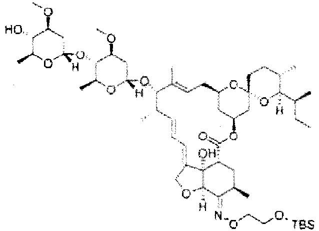 |

**[Table 3]**

| Comp. | Structural formula | Comp. | Structural formula |
|---|---|---|---|
| I-21 | 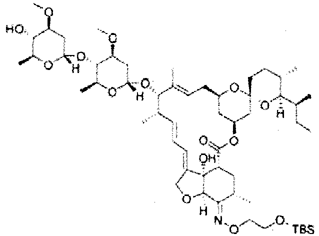 | I-26 | 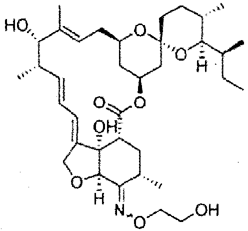 |
| I-22 | 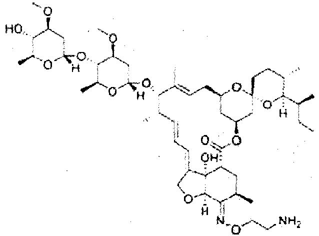 | I-27 | 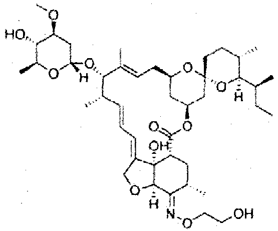 |
| I-23 | 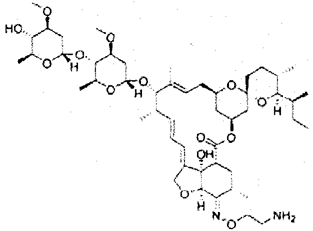 | I-28 | 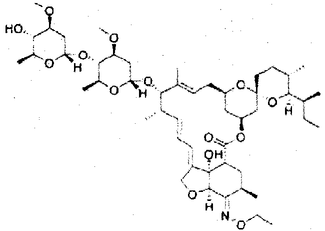 |
| I-24 | 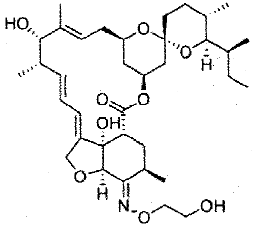 | I-29 | 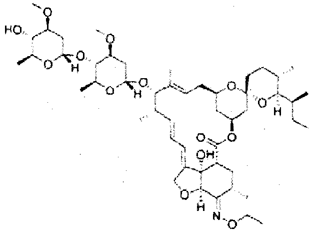 |
| I-25 | 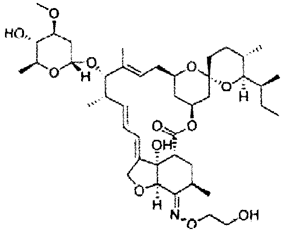 | I-30 | 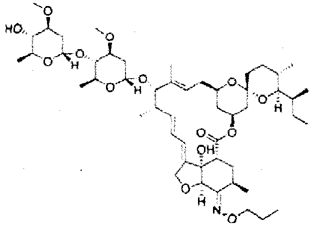 |

**[Table 4]**

| Comp. | Structural formula | Comp. | Structural formula |
|---|---|---|---|
| I-31 | 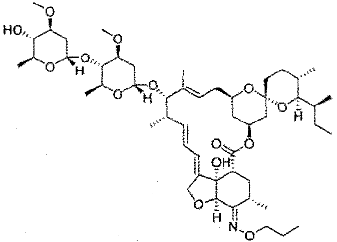 | I-36 | 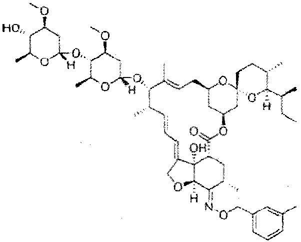 |
| I-32 | 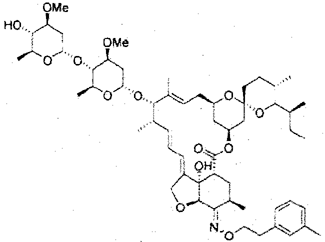 | I-37 | 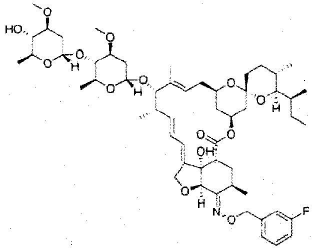 |
| I-33 | 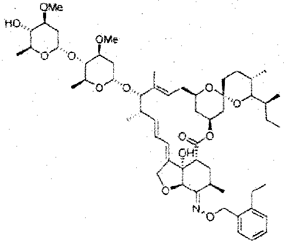 | I-38 | 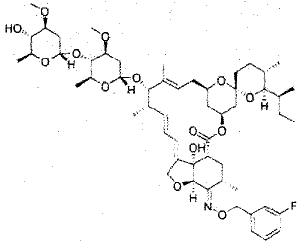 |
| I-34 | 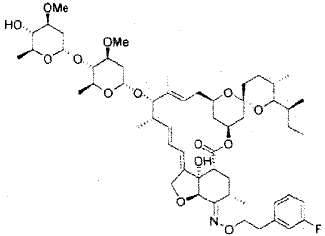 | I-39 | 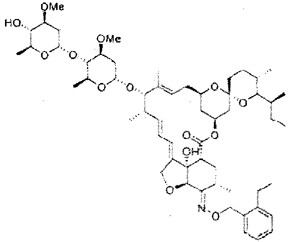 |
| I-35 | 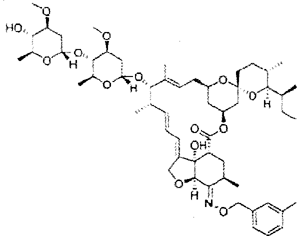 | I-40 | 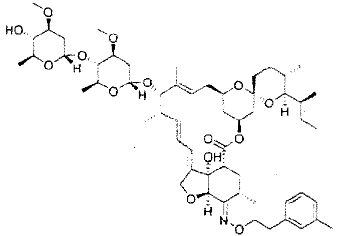 |

**[Table 5]**

| Comp. | Structural formula | Comp. | Structural formula |
|---|---|---|---|
| I-41 | 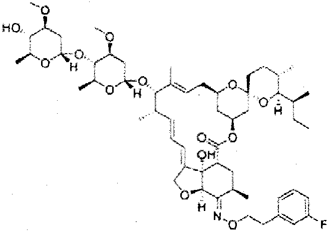 | I-46 | 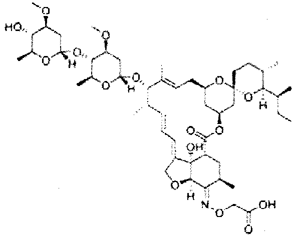 |
| I-42 | 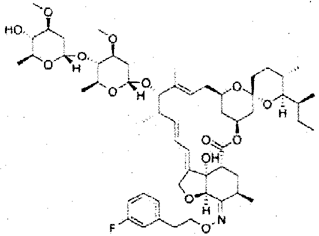 | I-47 | 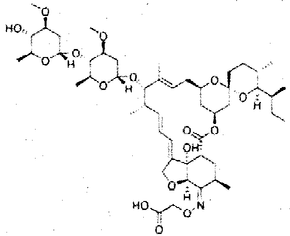 |
| I-43 | 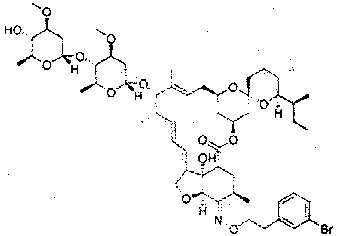 | I-48 | 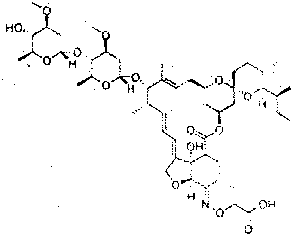 |
| I-44 | 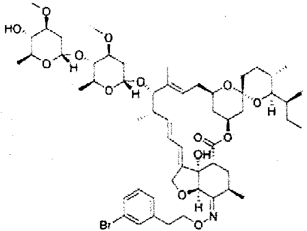 | I-49 | 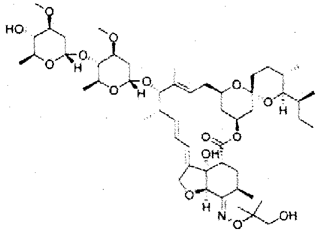 |
| I-45 | 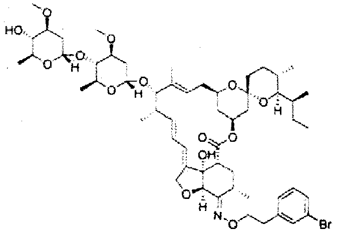 | I-50 | 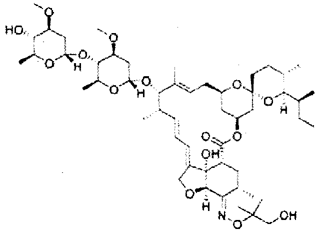 |

**[Table 6]**

| Comp. | Structural formula | Comp. | Structural formula |
|---|---|---|---|
| I-51 | 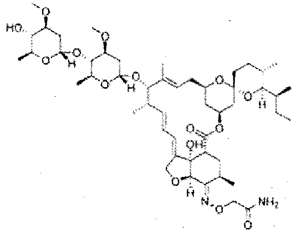 | I-56 | 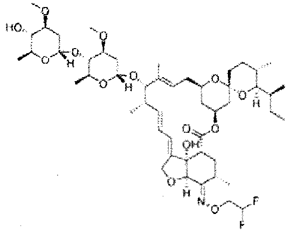 |
| I-52 | 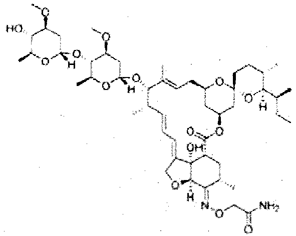 | I-57 | 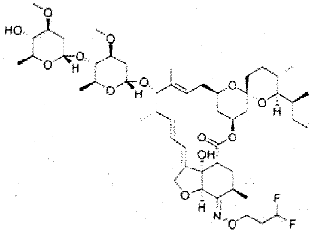 |
| I-53 | 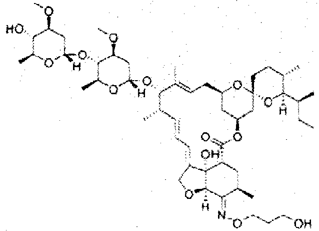 | I-58 | 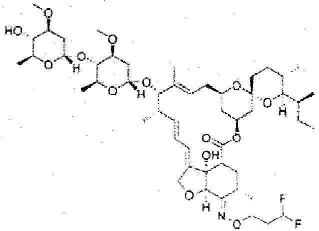 |
| I-54 | 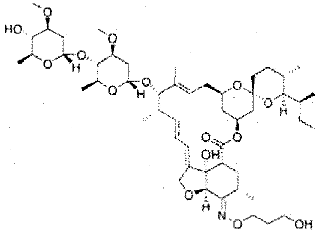 | I-59 | 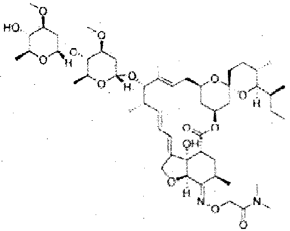 |
| I-55 | 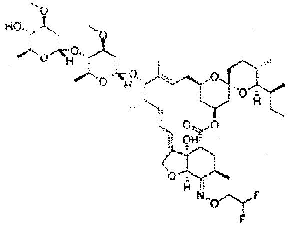 | I-60 | 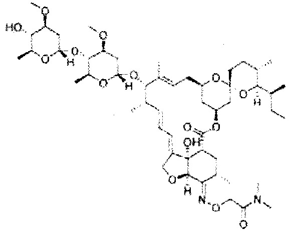 |

**[Table 7]**

| Comp. | Structural formula | Comp. | Structural formula |
|---|---|---|---|
| I-61 | 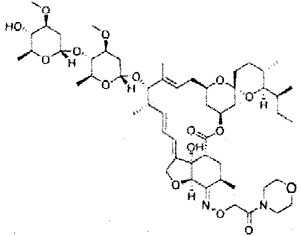 | I-66 | 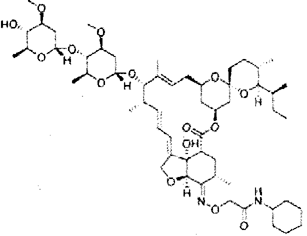 |
| I-62 | 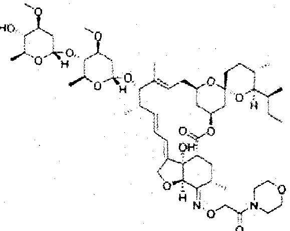 | I-67 | 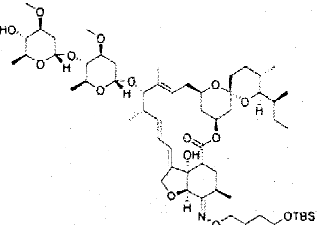 |
| I-63 | 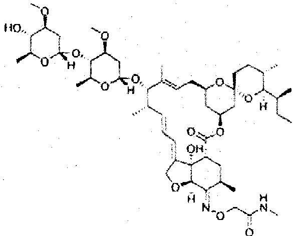 | I-68 | 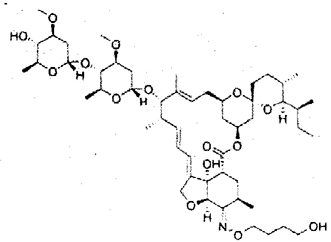 |
| I-64 | 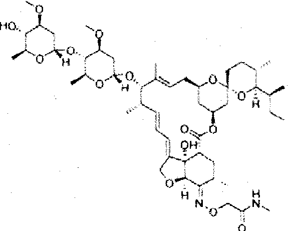 | I-69 | 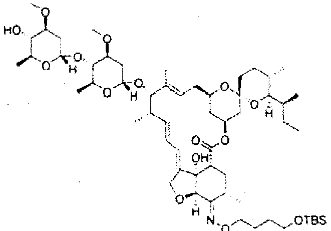 |
| I-65 | 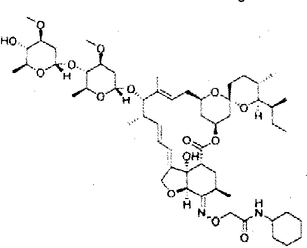 | I-70 | 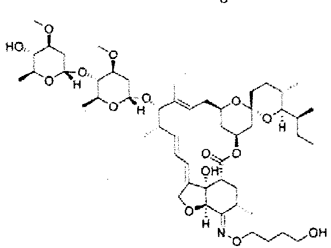 |

**[Table 8]**

| Comp. | Structural formula | Comp. | Structural formula |
|---|---|---|---|
| I-71 | 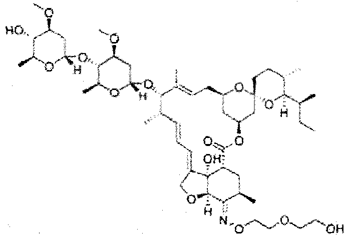 | I-76 | 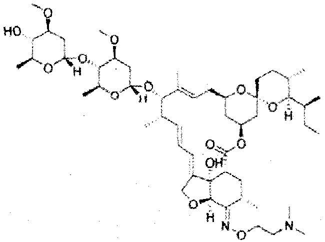 |
| I-72 | 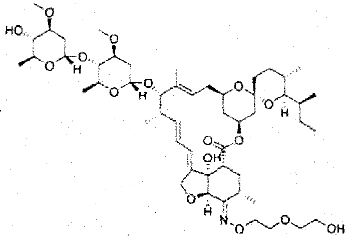 | I-77 | 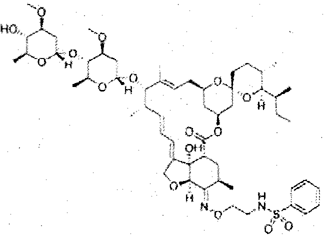 |
| I-73 | 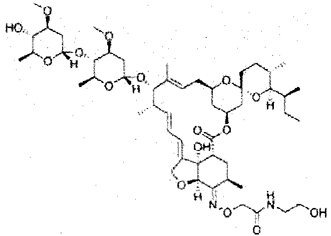 | I-78 | 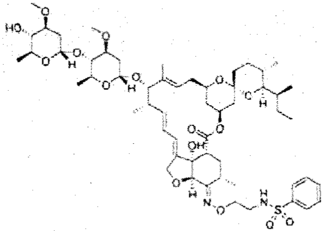 |
| I-74 | 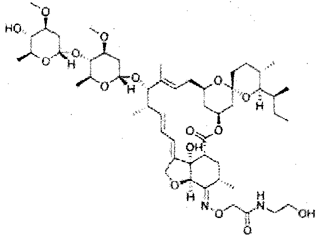 | I-79 | 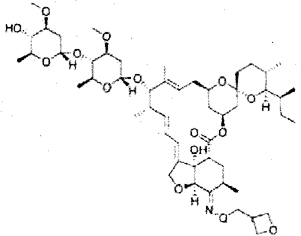 |
| I-75 | 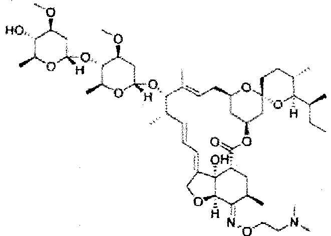 | I-80 | 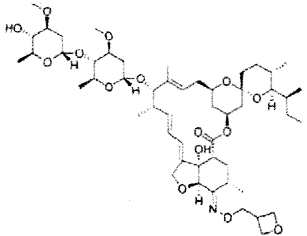 |

**[Table 9]**

| Comp. | Structural formula | Comp. | Structural formula |
|---|---|---|---|
| I**-**81 | 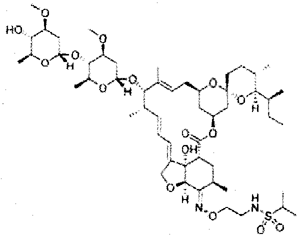 | I-86 | 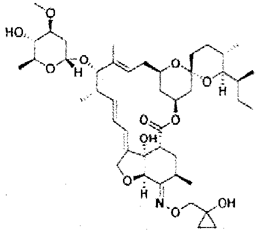 |
| I-82 | 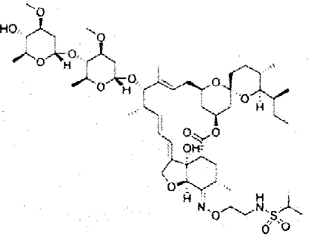 | I-87 | 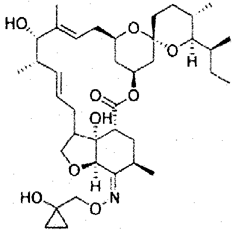 |
| I-83 | 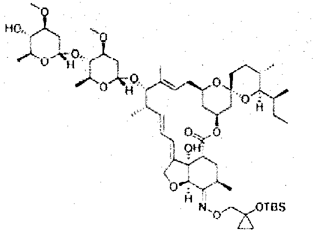 | I-88 | 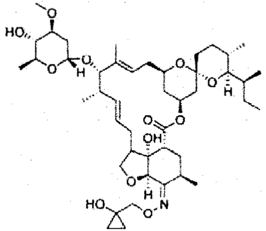 |
| I-84 | 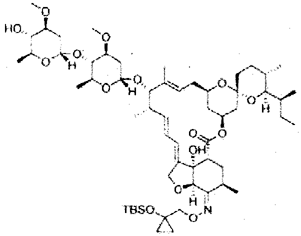 | I-89 | 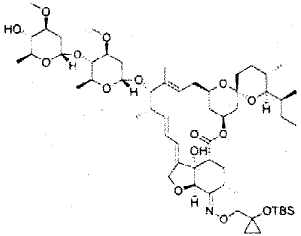 |
| I-85 | 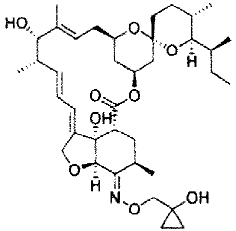 | I-90 | 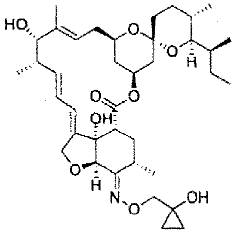 |

**[Table 10]**

| Comp. | Structural formula | Comp. | Structural formula |
|---|---|---|---|
| I-91 | 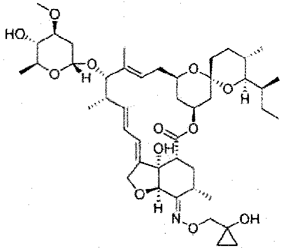 | I-96 | 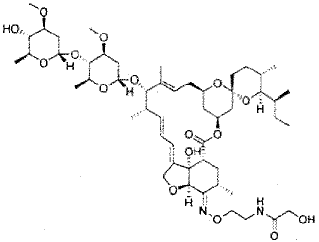 |
| I-92 | 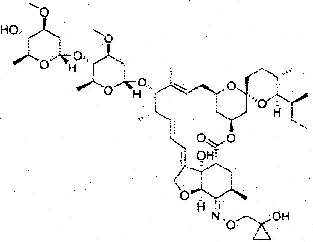 | I-97 | 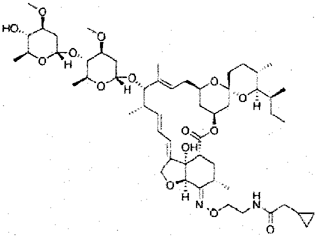 |
| I-93 | 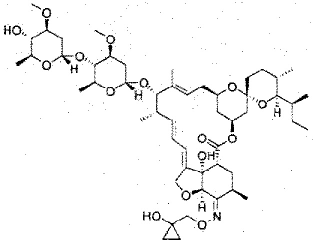 | I-98 | 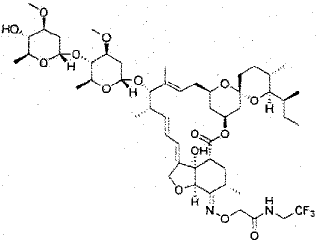 |
| I-94 | 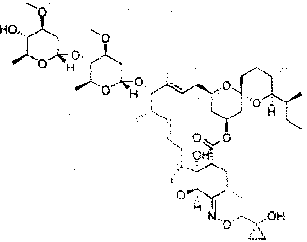 | I-99 | 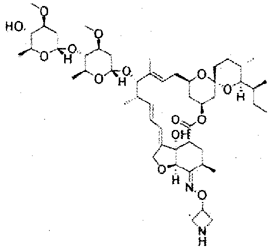 |
| I-95 | 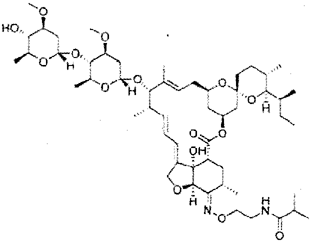 | I-100 | 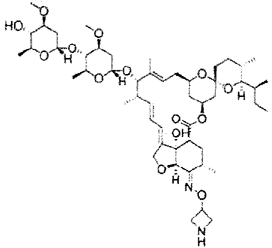 |

**[Table 11]**

| Comp. | Structural formula | Comp. | Structural formula |
|---|---|---|---|
| I-101 | 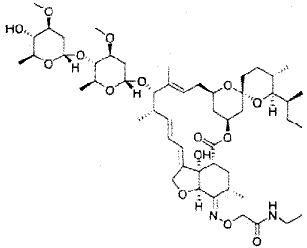 | I-106 | 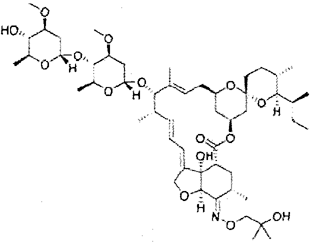 |
| I-102 | 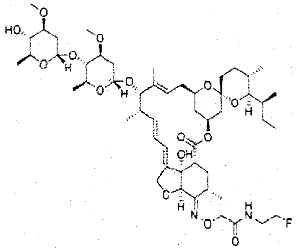 | I-107 | 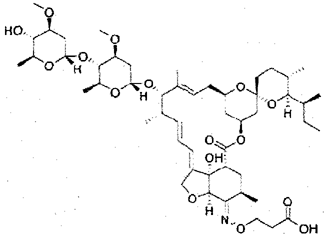 |
| I-103 | 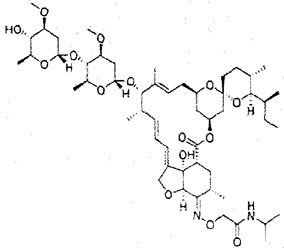 | I-108 | 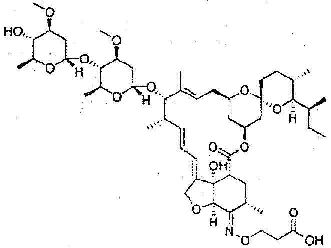 |
| I-104 | 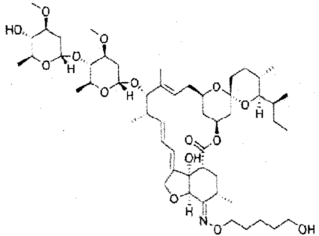 | I-109 | 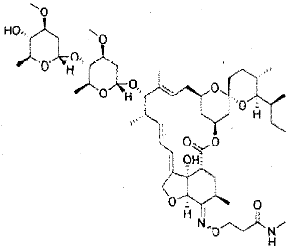 |
| I-105 | 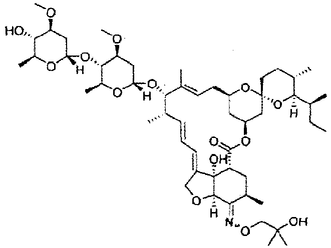 | I-110 | 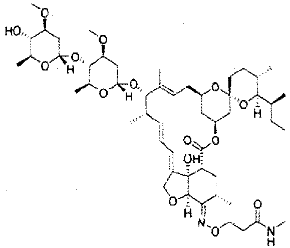 |

**[Table 12]**

| Comp. | Structural formula | Comp. | Structural formula |
|---|---|---|---|
| I-111 | 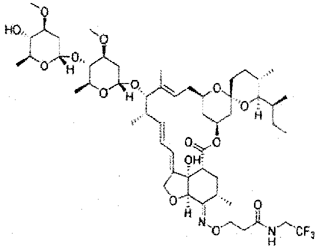 | I**-**116 | 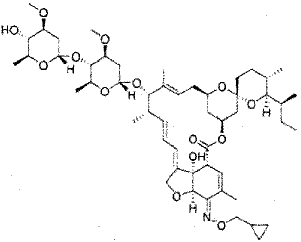 |
| I-112 | 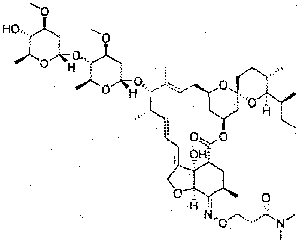 | I-117 | 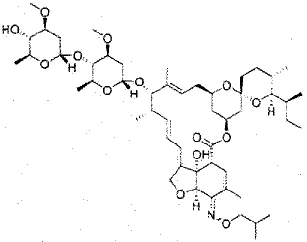 |
| I-113 | 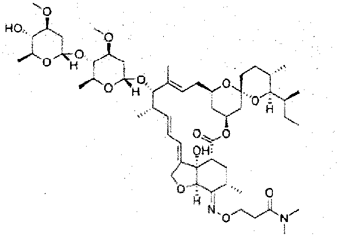 | I-118 | 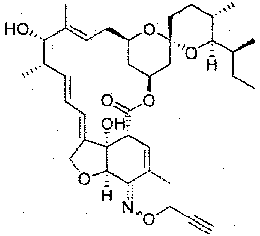 |
| I-114 | 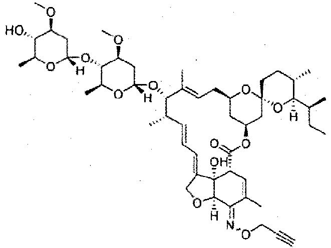 | I-119 | 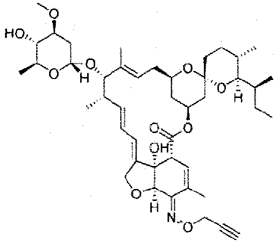 |
| I-115 | 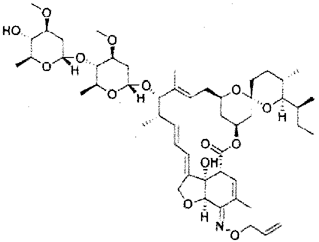 | I-120 | 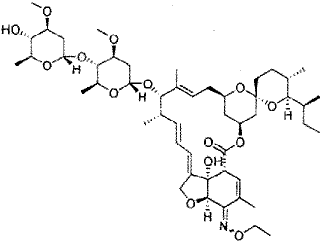 |

**[Table 13]**

| Comp. | Structural formula | Comp. | Structural formula |
|---|---|---|---|
| I-121 | 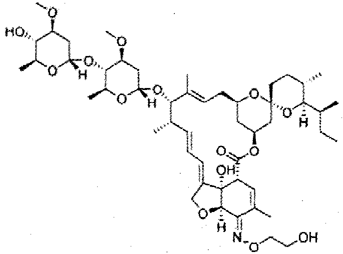 | I-125 | 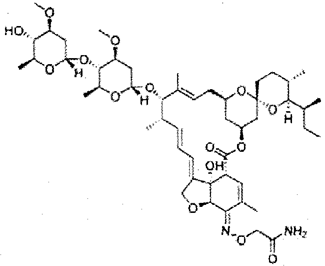 |
| I-122 | 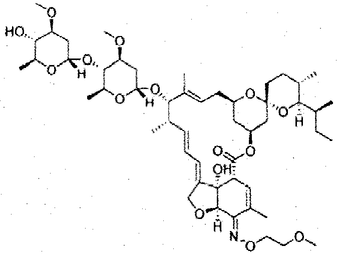 | I-126 | 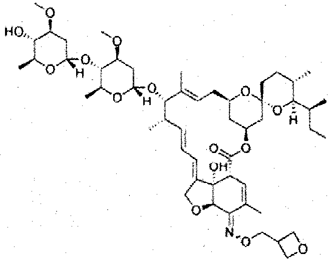 |
| I-123 | 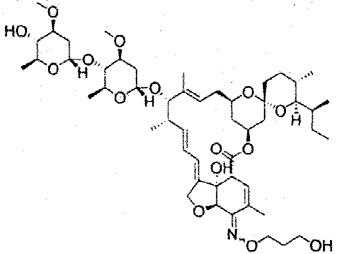 | I-127 | 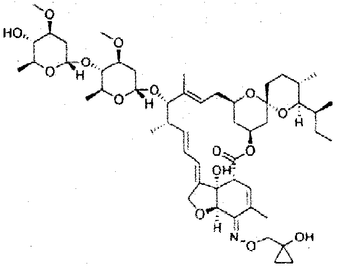 |
| I-124 | 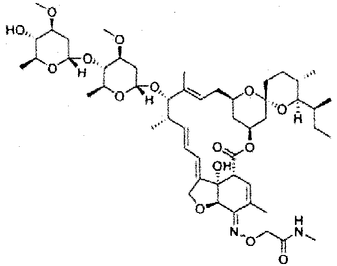 | I-128 | 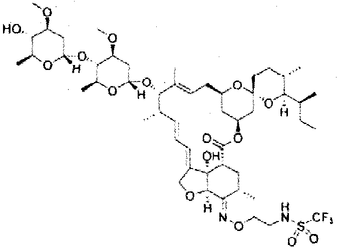 |

**[Table 14]**

| Comp. | Structural formula | Comp. | Structural formula |
|---|---|---|---|
| I-129 | 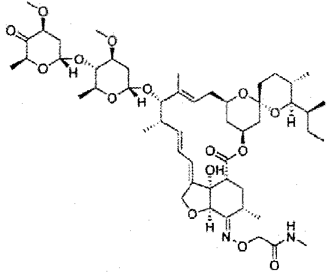 | I-133 | 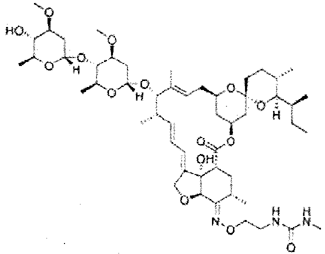 |
| I-130 | 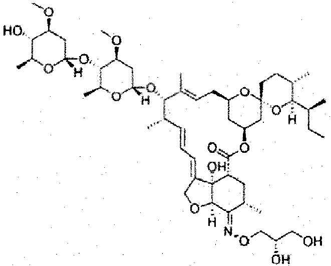 | I-134 | 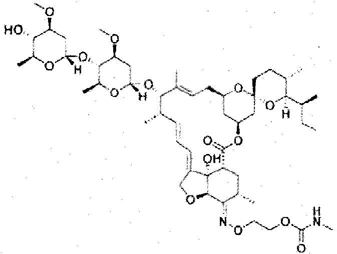 |
| I-131 | 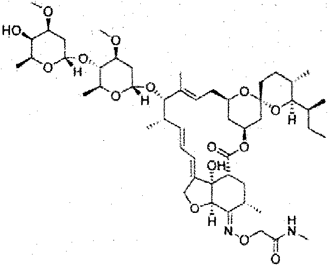 | I-135 | 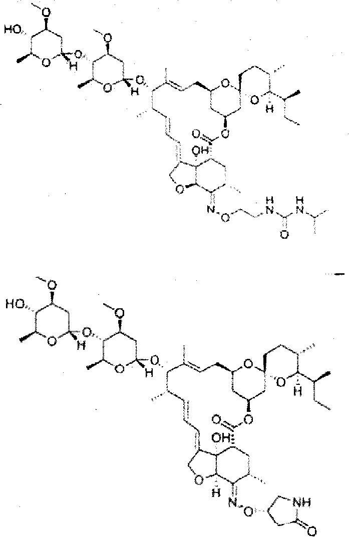 |
| I-132 | 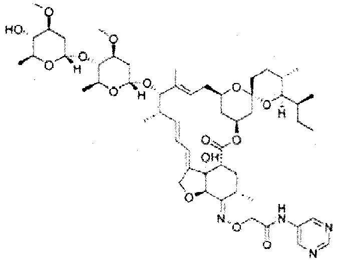 | I-136 | 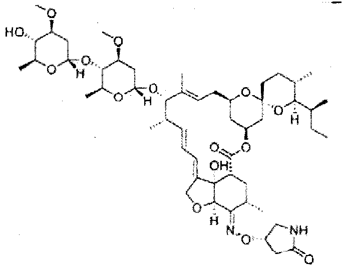 |

**[Table 15]**

| Comp. | Structural formula | Comp. | Structural formula |
|---|---|---|---|
| I-137 | 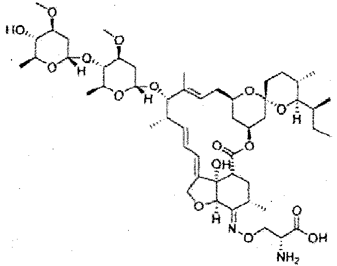 | I-141 | 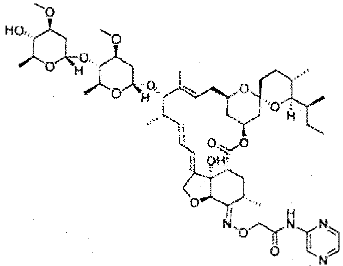 |
| I-138 | 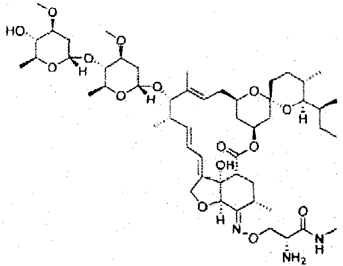 | I-142 | 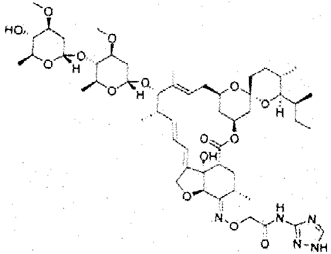 |
| I-139 | 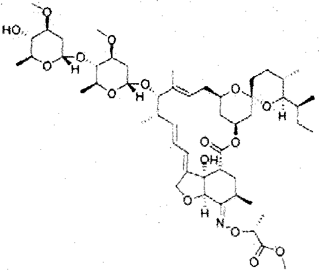 | I-143 | 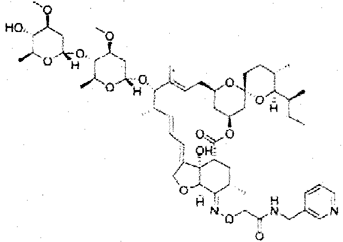 |
| I-140 | 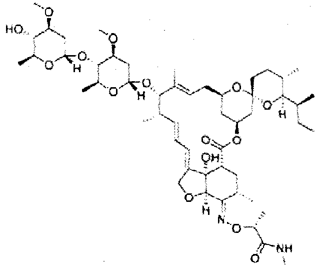 | I-144 | 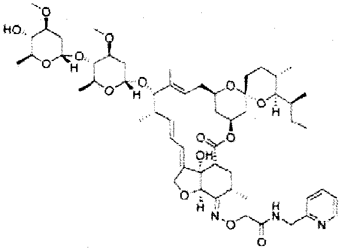 |

**[Table 16]**

| Comp. | Structural formula | Comp. | Structural formula |
|---|---|---|---|
| I-145 | 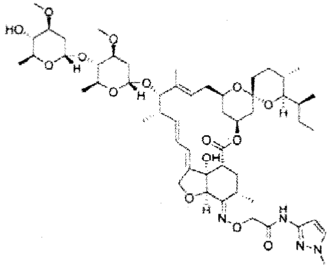 | I-149 | 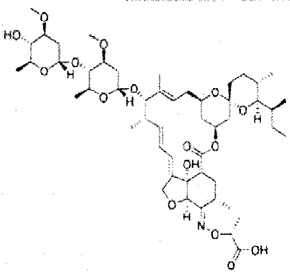 |
| I-146 | 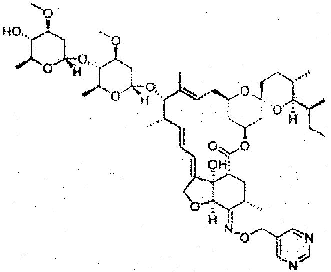 | I-150 | 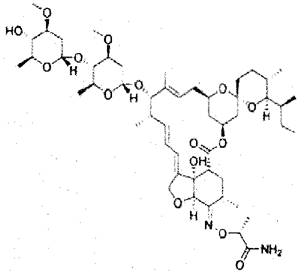 |
| I*-*147 | 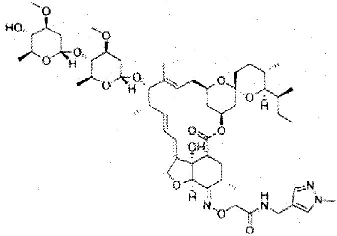 | I-151 | 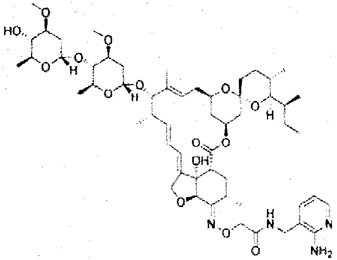 |
| I-148 | 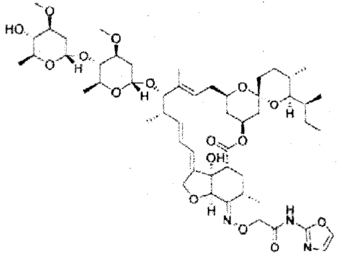 | I-152 | 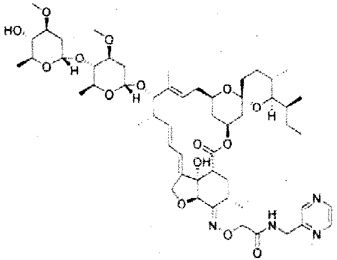 |

**[Table 17]**

| Comp. | Structural formula | Comp. | Structural formula |
|---|---|---|---|
| I-153 | 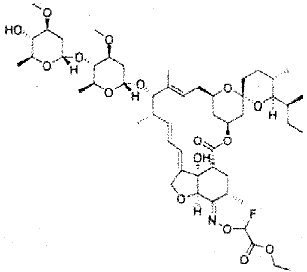 | I-157 | 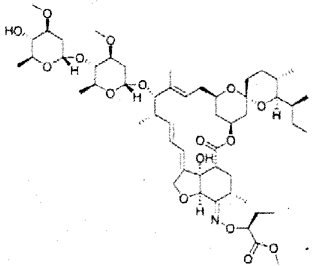 |
| I-154 | 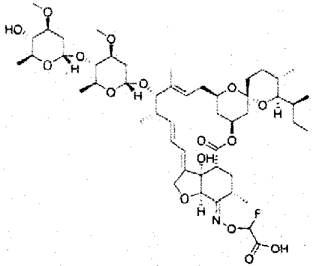 | I-158 | 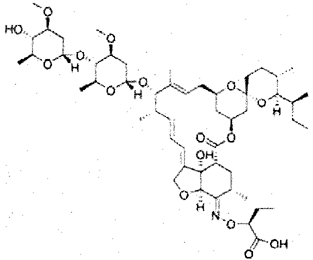 |
| I-155 | 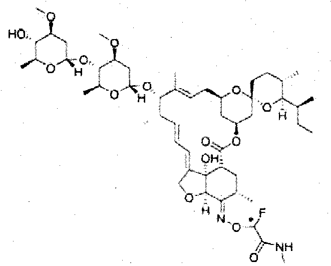 | I-159 | 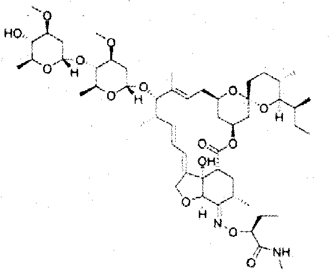 |
| I-156 | 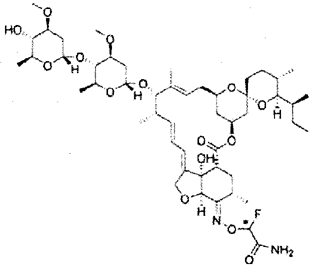 | I-160 | 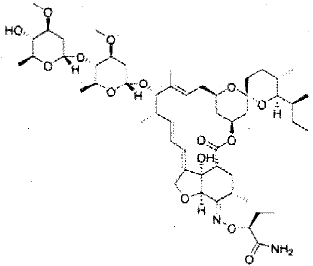 |

**[Table 18]**

| Comp. | Structural formula | Comp. | Structural formula |
|---|---|---|---|
| I-161 | 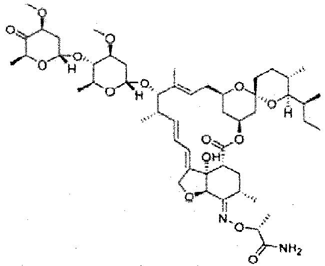 | I-165 | 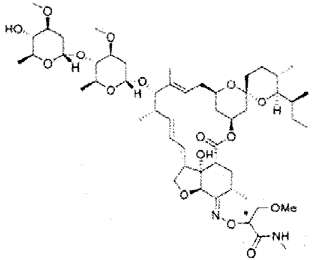 |
| I-162 | 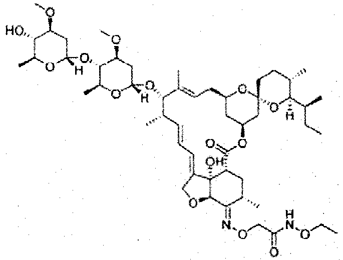 | I-166 | 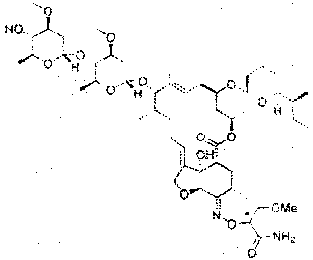 |
| I-163 | 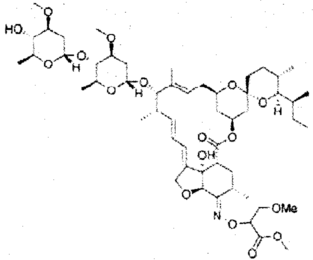 | I-167 | 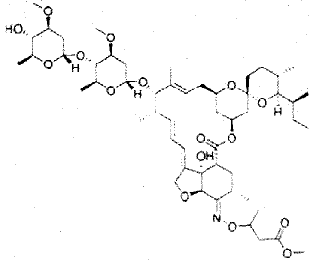 |
| I-164 | 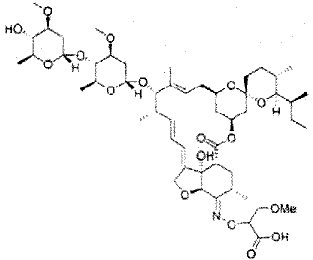 | I-168 | 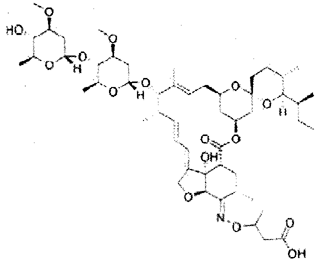 |

**[Table 19]**

| Comp. | Structural formula | Comp. | Structural formula |
|---|---|---|---|
| I-169 | 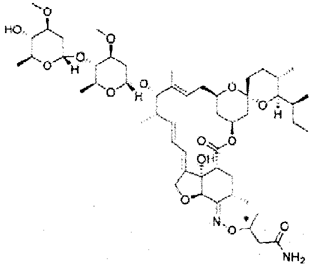 | I-173 | 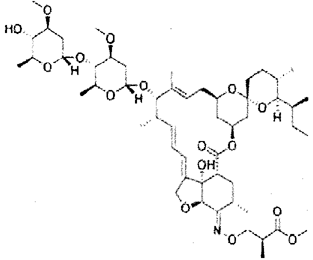 |
| I-170 | 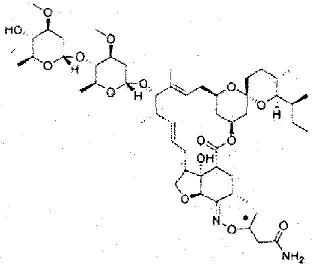 | I-174 | 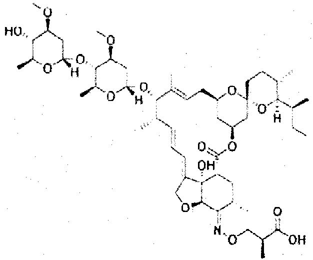 |
| I-171 | 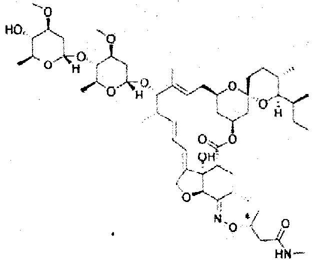 | I-175 | 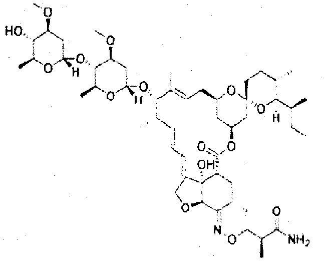 |
| I-172 | 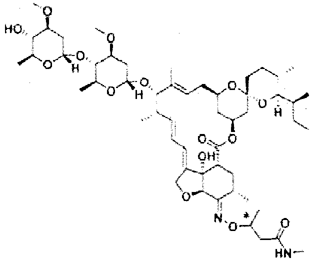 | I-176 | 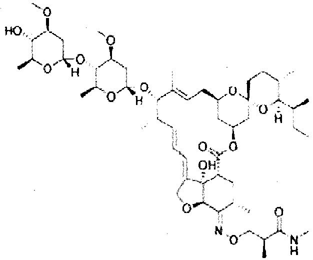 |

**[Table 20]**

| Comp. | Structural formula | Comp. | Structural formula |
|---|---|---|---|
| II -1 | 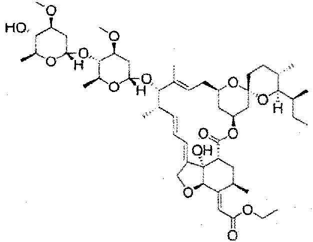 | II -6 | 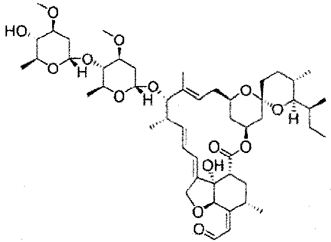 |
| II -2 | 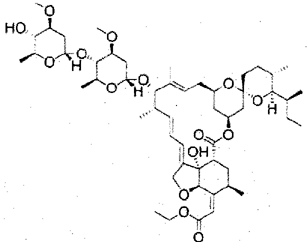 | II -7 | 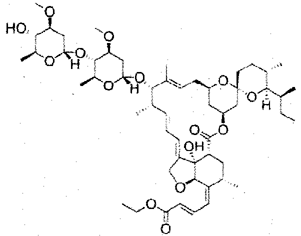 |
| II -3 | 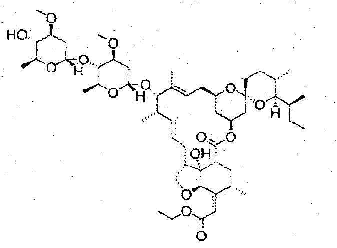 | II -8 | 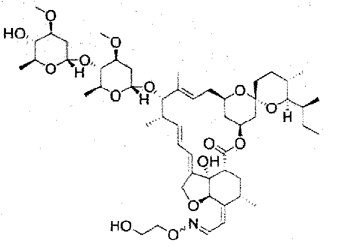 |
| II -4 | 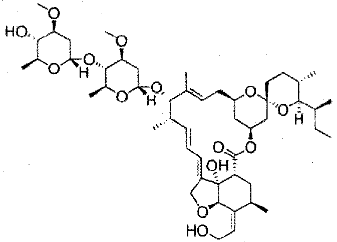 | II -9 | 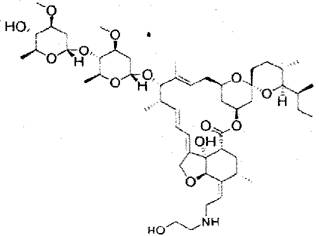 |
| II -5 | 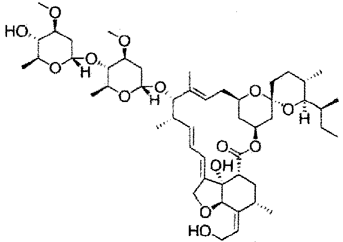 | II -10 | 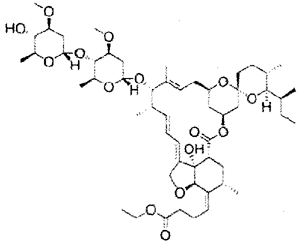 |

**[Table 21]**

| Comp. | Structural formula | Comp. | Structural formula |
|---|---|---|---|
| II -11 | 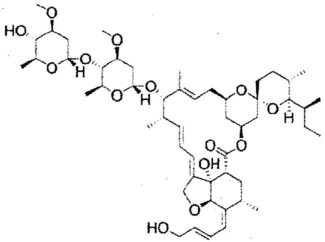 | II -16 | 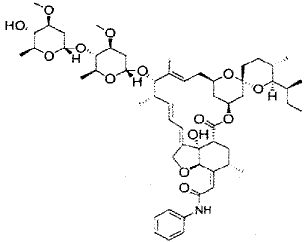 |
| II -12 | 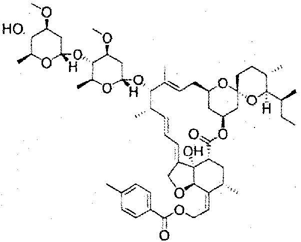 | II -17 | 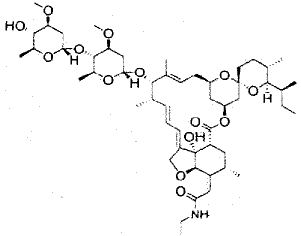 |
| II -13 | 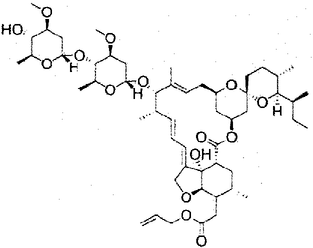 | II -18 | 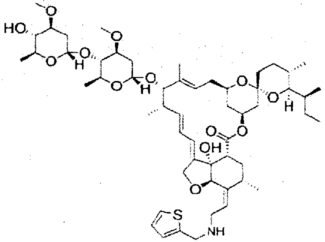 |
| II -14 | 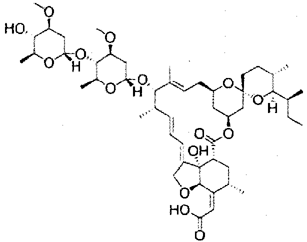 | II -19 | 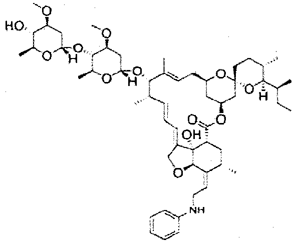 |
| II -15 | 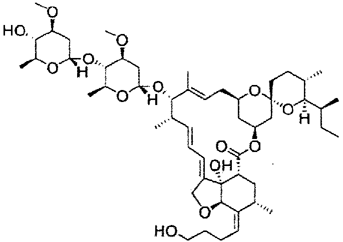 | II -20 | 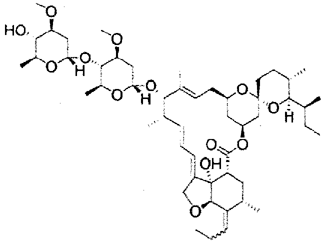 |

**[Table 22]**

| Comp. | Structural formula | Comp. | Structural formula |
|---|---|---|---|
| II -21 | 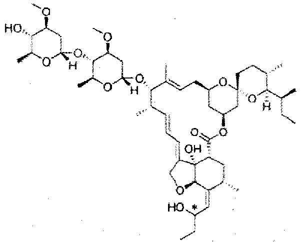 | II -26 | 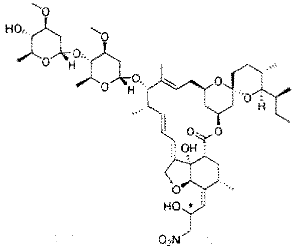 |
| II -22 | 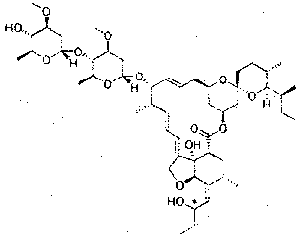 | II -27 | 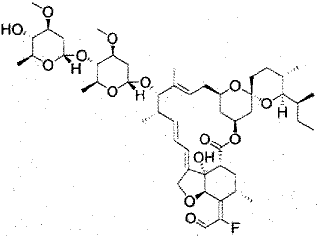 |
| II -23 | 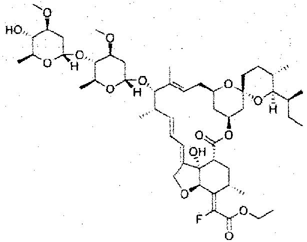 | II -28 | 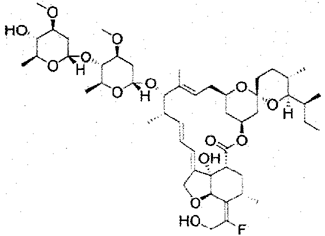 |
| II -24 | 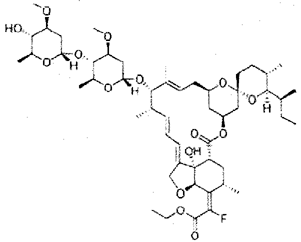 | II -29 | 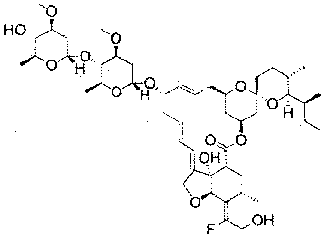 |
| II -25 | 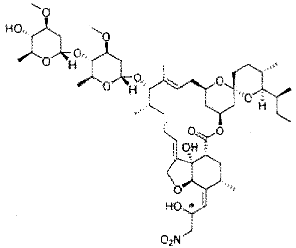 | II -30 | 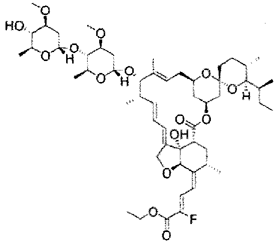 |

**[Table 23]**

| Comp. | Structural formula | Comp. | Structural formula |
|---|---|---|---|
| II -31 | 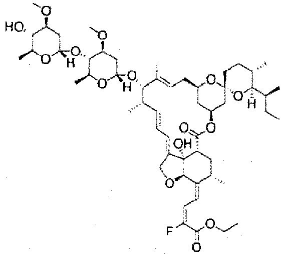 | II -36 | 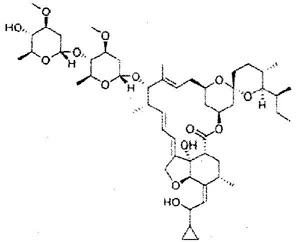 |
| II -32 | 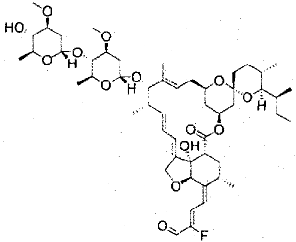 | II -37 | 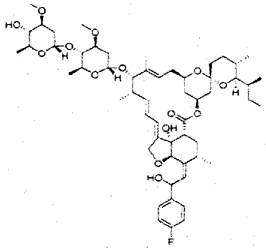 |
| II -33 | 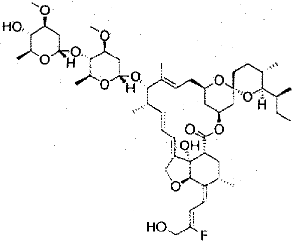 | II -38 | 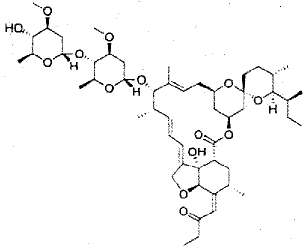 |
| II -34 | 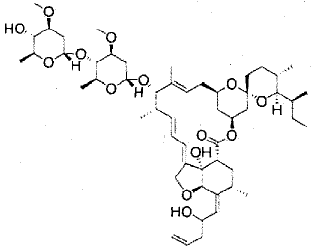 | II -39 | 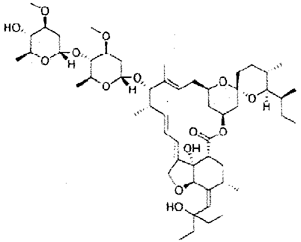 |
| II -35 | 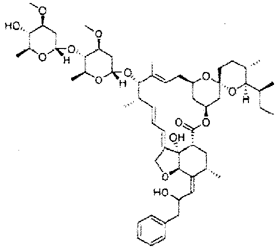 | II -40 | 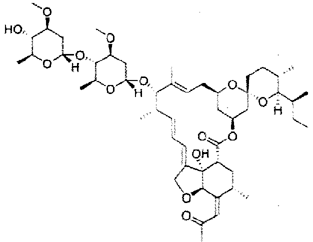 |

**[Table 24]**

| Comp. | Structural formula | Comp. | Structural formula |
|---|---|---|---|
| II -41 | 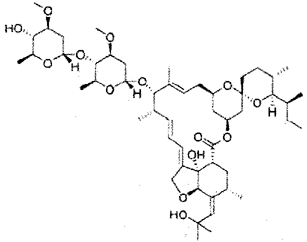 | II -46 | 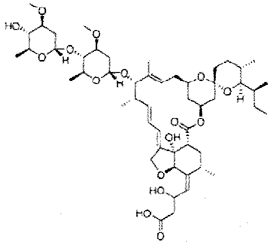 |
| II -42 | 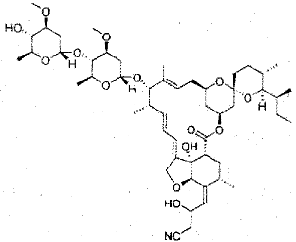 | II -47 | 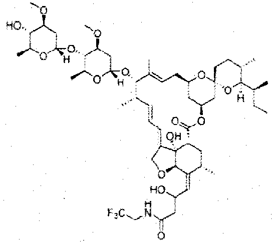 |
| II -43 | 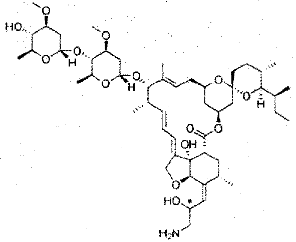 | II -48 | 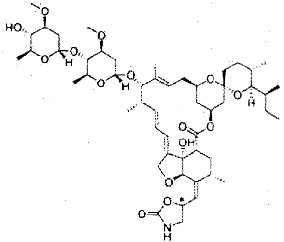 |
| II -44 | 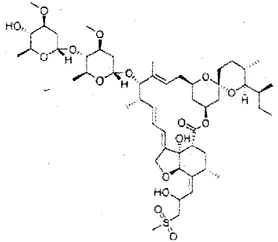 | II -49 | 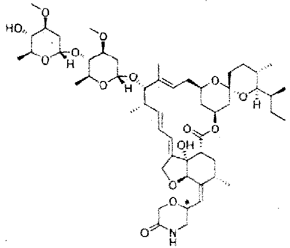 |
| II -45 | 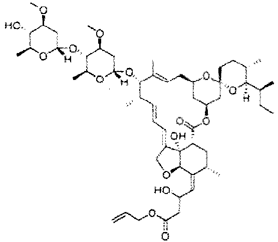 | II -50 | 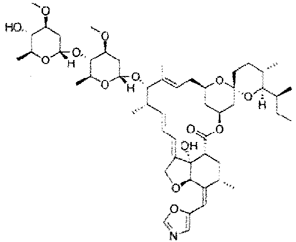 |

**[Table 25]**

| Comp. | Structural formula | Comp. | Structural formula |
|---|---|---|---|
| II -51 | 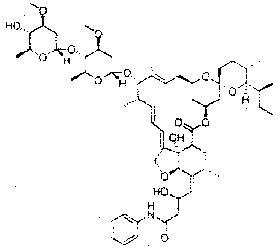 | II -56 | 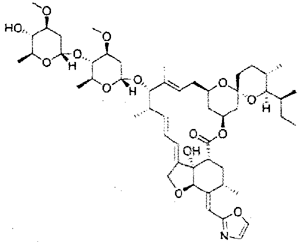 |
| II -52 | 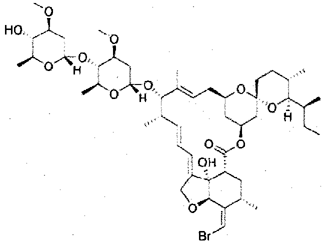 | II -57 | 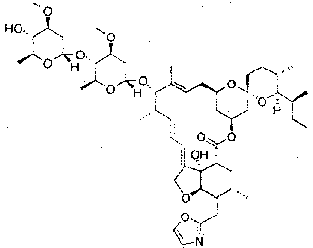 |
| II -53 | 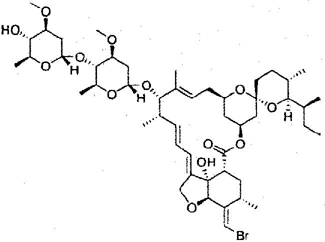 | II -58 | 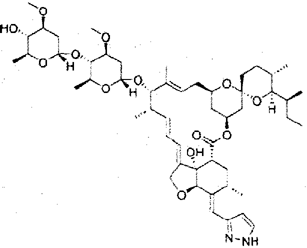 |
| II -54 | 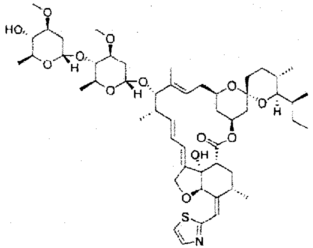 | II -59 | 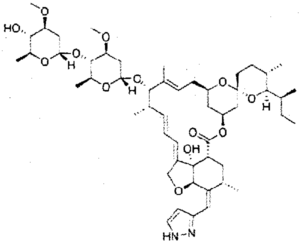 |
| II -55 | 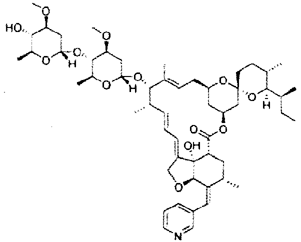 | II -60 | 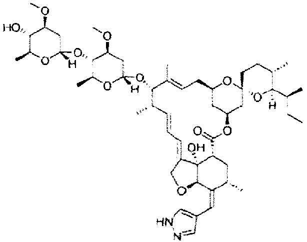 |

**[Table 26]**

| Comp. | Structural formula | Comp. | Structural formula |
|---|---|---|---|
| II -61 | 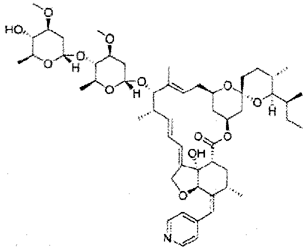 | II -66 | 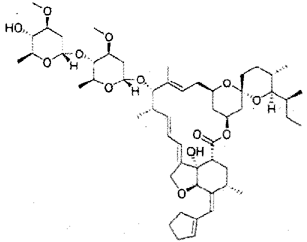 |
| II -62 | 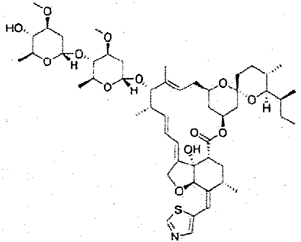 | II -67 | 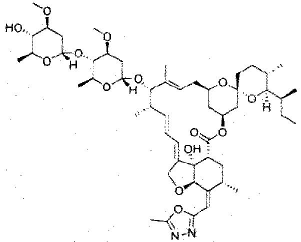 |
| II -63 | 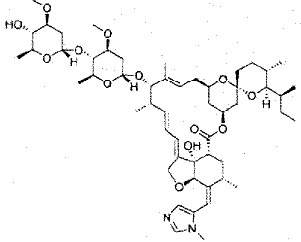 | II -68 | 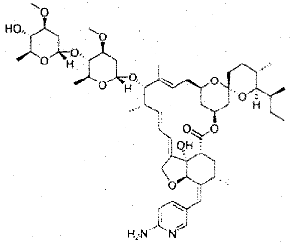 |
| II -64 | 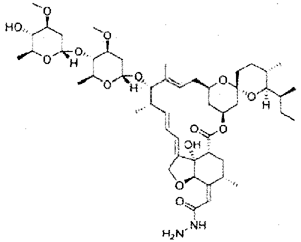 | II -69 | 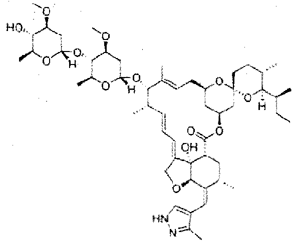 |
| II -65 | 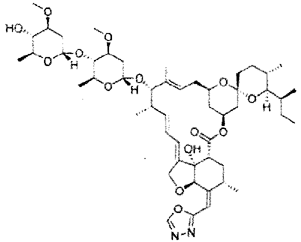 | II -70 | 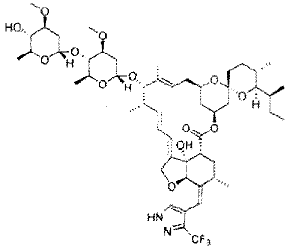 |

**[Table 27]**

| Comp. | Structural formula | Comp. | Structural formula |
|---|---|---|---|
| II -71 | 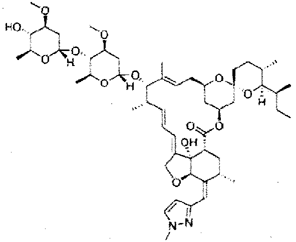 | II -76 | 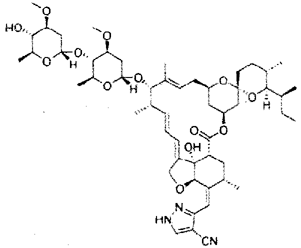 |
| II -72 | 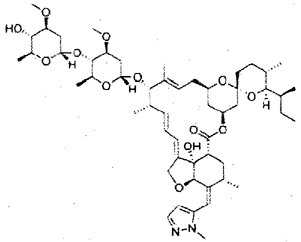 | II -77 | 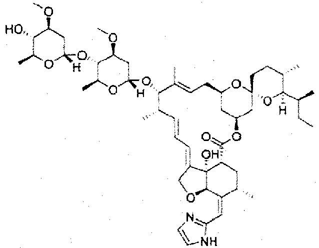 |
| II -73 | 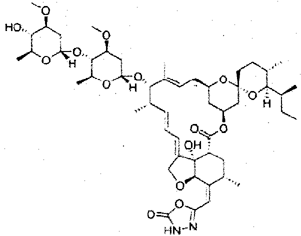 | II -78 | 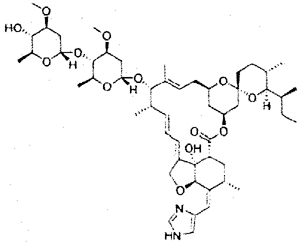 |
| II -74 | 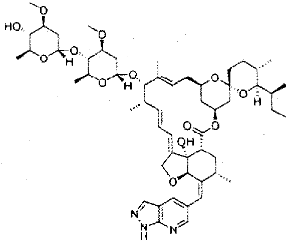 | II -79 | 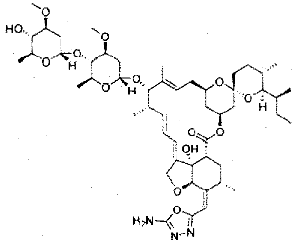 |
| II -75 | 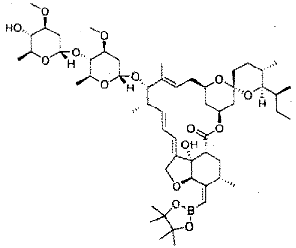 | II -80 | 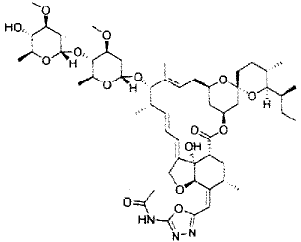 |

**[Table 28]**

| Comp. | Structural formula | Comp. | Structural formula |
|---|---|---|---|
| II -81 | 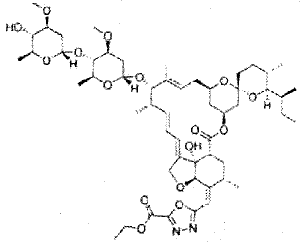 | I -86 | 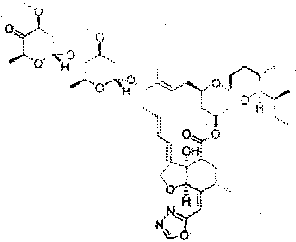 |
| II -82 | 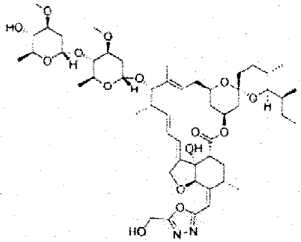 | II -87 | 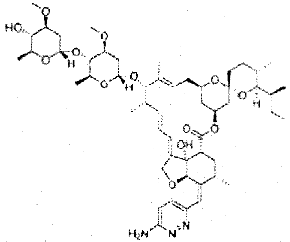 |
| II -83 | 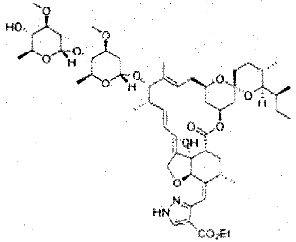 | II -88 | 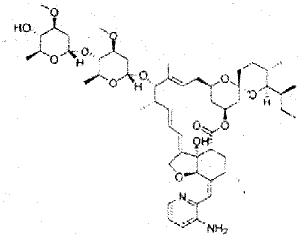 |
| II -84 | 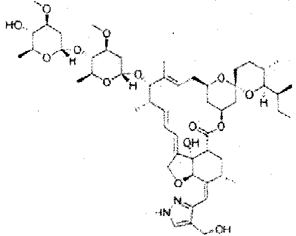 | II -89 | 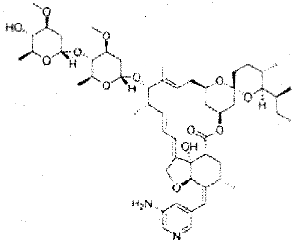 |
| II -85 | 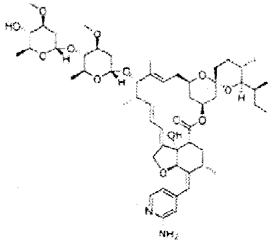 | II -90 | 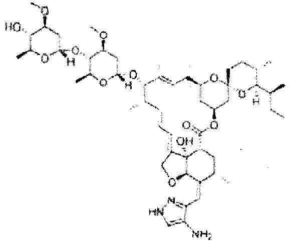 |

**[Table 29]**

| Comp. | Structural formula | Comp. | Structural formula |
|---|---|---|---|
| II -91 | 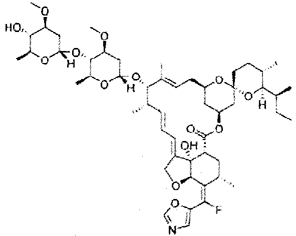 | II -96 | 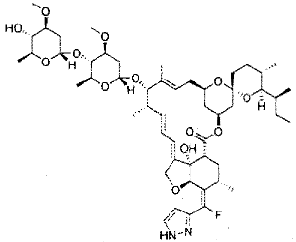 |
| II -92 | 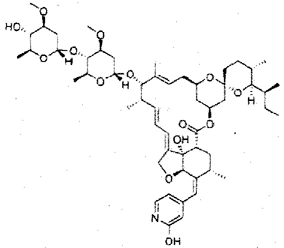 | II -97 | 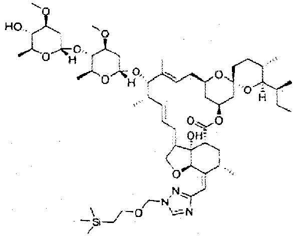 |
| II -93 | 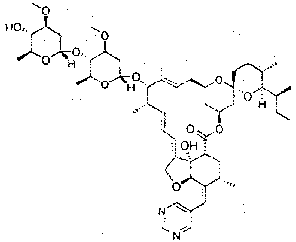 | II -98 | 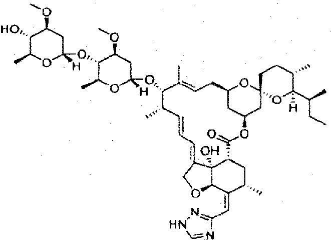 |
| II -94 | 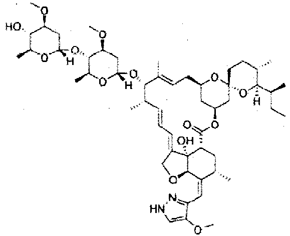 | II -99 | 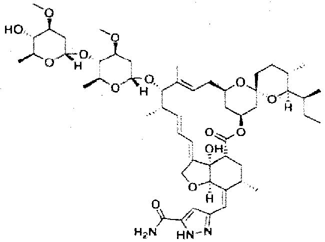 |
| II -95 | 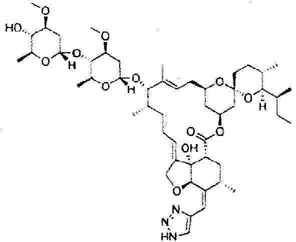 | II -100 | 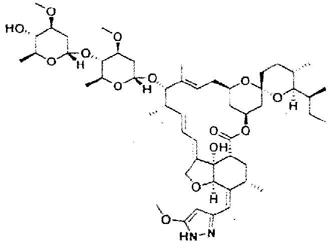 |

**[Table 30]**

| Comp. | Structural formula | Comp. | Structural formula |
|---|---|---|---|
| II -101 | 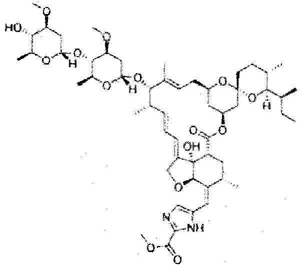 | II -105 | 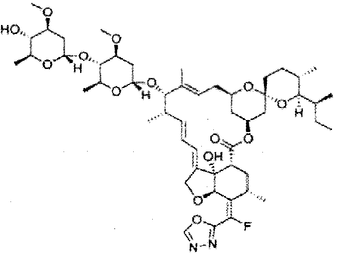 |
| II -102 | 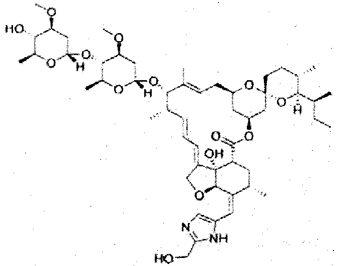 | II -106 | 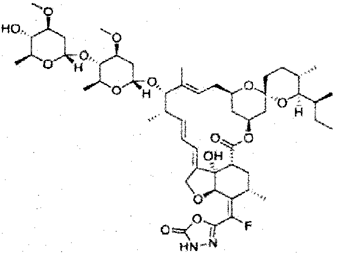 |
| II -103 | 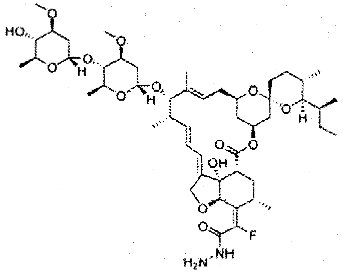 | II -107 | 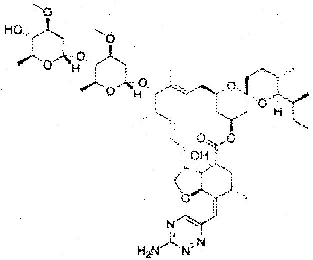 |
| II -104 | 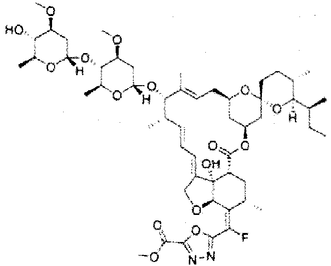 | II -108 | 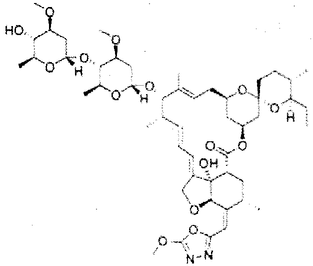 |

**[Table 31]**

| Comp. | Structural formula | Comp. | Structural formula |
|---|---|---|---|
| II-109 | 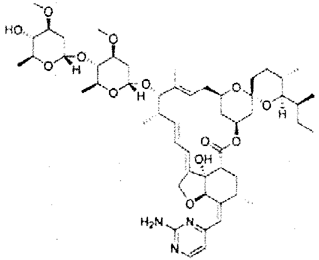 | II-113 | 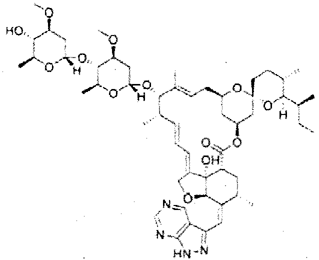 |
| II-110 | 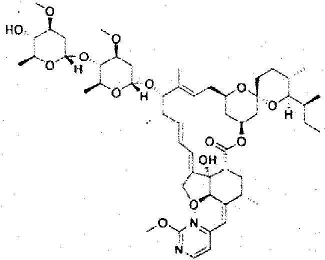 | II-114 | 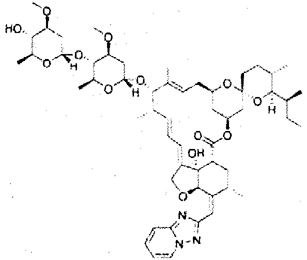 |
| II-111 | 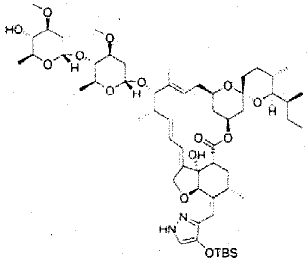 | II-115 | 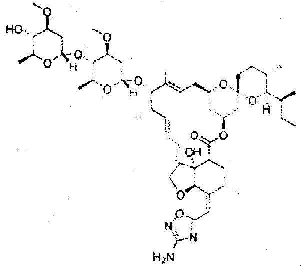 |
| II-112 | 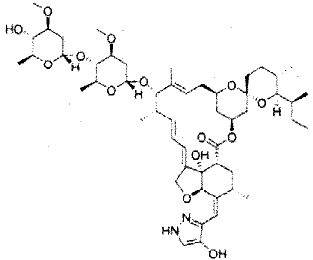 | II-116 | 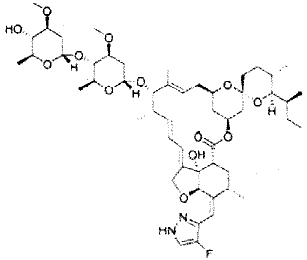 |

**[Table 32]**

| Comp. | Structural formula | Comp. | Structural formula |
|---|---|---|---|
| II-117 | 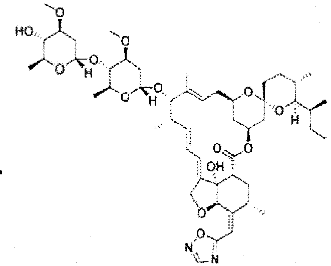 | II-121 | 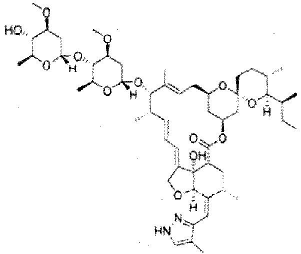 |
| II-118 | 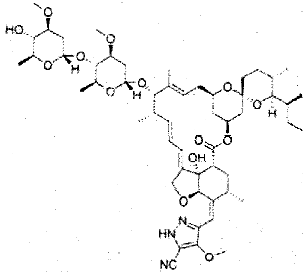 | II-122 | 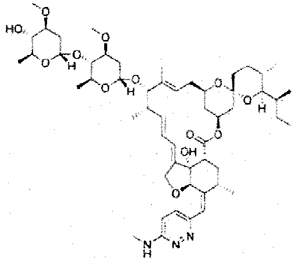 |
| II-119 | 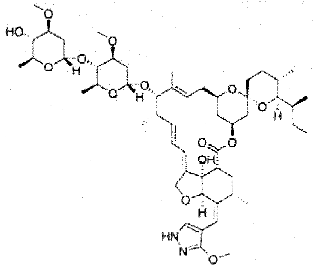 | II-123 | 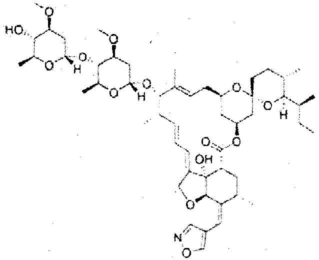 |
| II-120 | 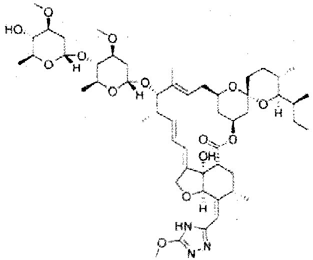 | II-124 | 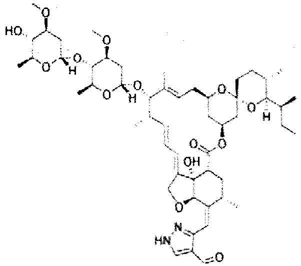 |

**[Table 33]**

| Comp. | Structural formula | Comp. | Structural formula |
|---|---|---|---|
| II-125 | 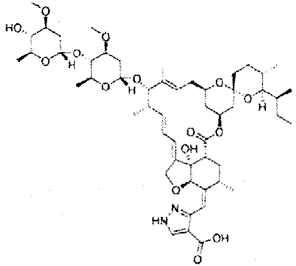 | III-3 | 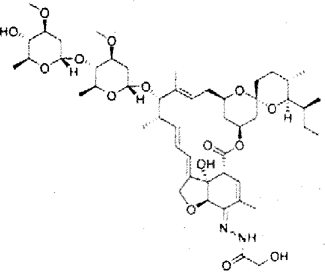 |
| III-1 | 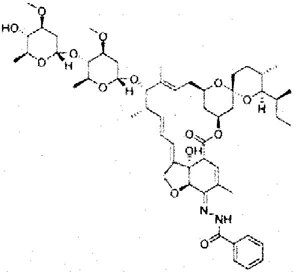 | III-4 | 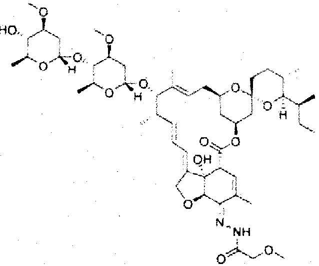 |
| III-2 | 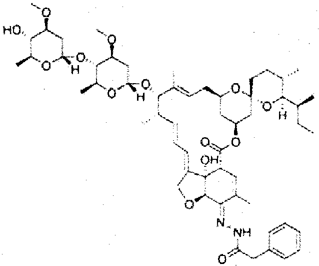 | III-5 | 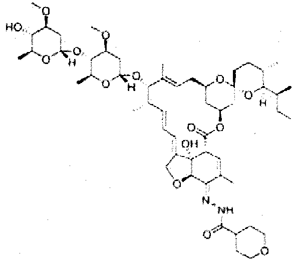 |

### <Evaluation of virus growth inhibitory activity>

### (Test Example 1) Anti-SARS-CoV-2 activity evaluation test

VeroE6 cells (5 × 10⁴ cells/well) derived from African green monkey kidney, which had been cultured in advance in a 96-well plate with a medium supplemented with 10% fetal bovine serum (FBS) (MEM containing penicillin-streptomycin), were used to evaluate the anti-SARS-CoV-2 activity of each compound obtained above (hereinafter, test compound).

First, the medium was removed from the 96-well plate in which VeroE6 cells were cultured, and a virus solution (2-3 × 10⁴ TCID₅₀/well) in which SARS-CoV-2 was added to a medium supplemented with 2% FBS (MEM containing penicillin-streptomycin) was dispensed. Next, after culturing the 96-well plate in a CO₂ incubator for 1 hour, the virus solution was removed. Next, a sample solution prepared by dissolving the test compound in DMSO and appropriately serially diluting it with a medium supplemented with 2% FBS (MEM containing penicillin-streptomycin) was dispensed into the 96-well plate. After culturing the 96-well plate in a CO₂ incubator for 1 day, viral RNA in the culture supernatant was quantified by real-time PCR method.

The results of real-time PCR were analyzed with statistical software, and the concentration of the test compound that reduced the replication of SARS-CoV-2 to 50% compared to the control that was similarly tested except that the test compound was not added was defined as the IC₅₀ value, and the following criteria were used:
A: IC₅₀ value is less than 1 µM
B: IC₅₀ value is 1 µM or more and less than 5 µM
C: IC₅₀ value is 5 µM or more and less than 20 µM
D: IC₅₀ value is 20 µM or more
to evaluate the antiviral activity. It can be evaluated that the antiviral activity is sufficient against coronavirus if the evaluation is A to C. The evaluation results of antiviral activity when each compound obtained above was used as each test compound are shown in Tables 34 to 36 below.

**[Table 34]**

| Comp. | Antiviral activity | Comp. | Antiviral activity | Comp. | Antiviral activity |
|---|---|---|---|---|---|
| I-1 | A | I-57 | A | I-101 | A |
| I-2 | A | I-58 | B | I-102 | A |
| I-3 | A | I-59 | A | I-103 | A |
| I-4 | A | I-60 | A | I-104 | A |
| I-5 | A | I-61 | B | I-105 | A |
| I-6 | B | I-62 | A | I-106 | A |
| I-10 | A | I-63 | A | I-109 | A |
| I-11 | A | I-64 | A | I-110 | A |
| I-14 | B | I-66 | A | I-111 | A |
| I-15 | C | I-68 | A | I-112 | A |
| I-16 | B | I-70 | A | I-113 | A |
| I-18 | A | I-71 | B | I-114 | A |
| I-19 | A | I-72 | A | I-115 | A |
| I-20 | C | I-74 | A | I-116 | B |
| I-21 | C | I-75 | B | I-117 | C |
| I-22 | B | I-76 | C | I-118 | B |
| I-23 | B | I-79 | A | I-119 | B |
| I-24 | C | I-80 | A | I-120 | A |
| I-25 | B | I-81 | A | I-121 | A |
| I-26 | B | I-82 | A | I-122 | A |
| I-27 | B | I-85 | C | I-123 | A |
| I-28 | A | I-86 | B | I-124 | A |
| I-29 | B | I-87 | B | I-125 | A |
| I-30 | B | I-88 | B | I-126 | A |
| I-46 | C | I-90 | B | I-127 | A |
| I-47 | C | I-91 | C | I-128 | B |
| I-48 | B | I-92 | A | I-129 | A |
| I-49 | A | I-93 | A | I-130 | A |
| I-50 | A | I-94 | A | I-131 | A |
| I-51 | A | I-95 | A | I-132 | A |
| I-52 | A | I-96 | A | I-133 | A |
| I-53 | A | I-97 | A | I-134 | A |
| I-54 | A | I-98 | A | I-135 | A |
| I-55 | A | I-99 | B | I-136 | A |
| I-56 | B | I-100 | B | I-137 | C |

**[Table 35]**

| Comp. | Antiviral activity | Comp. | Antiviral activity | Comp. | Antiviral activity |
|---|---|---|---|---|---|
| I-138 | B | II-1 | B | II-39 | A |
| I-139 | A | II-2 | A | II-40 | A |
| I-140 | A | II-3 | A | II-41 | A |
| I-141 | A | II-4 | A | II-42 | B |
| I-142 | A | II-5 | A | II-43 | A |
| I-143 | A | II-6 | B | II-44 | B |
| I-144 | A | II-7 | C | II-45 | B |
| I-145 | A | II-8 | B | II-46 | B |
| I-146 | A | II-9 | B | II-47 | A |
| I-147 | A | II-10 | C | II-48 | A |
| I-148 | A | II-11 | A | II-49 | B |
| I-150 | A | II-12 | C | II-50 | A |
| I-151 | A | II-13 | B | II-51 | B |
| I-152 | A | II-14 | B | II-54 | A |
| I-155 | A | II-15 | A | II-55 | A |
| I-156 | A | II-17 | B | II-57 | A |
| I-159 | A | II-18 | C | II-59 | A |
| I-160 | A | II-19 | C | II-60 | A |
| I-161 | A | II-20 | C | II-61 | A |
| I-162 | A | II-22 | A | II-62 | A |
| I-165 | A | II-23 | A | II-63 | A |
| I-166 | A | II-24 | B | II-65 | A |
| I-169 | A | II-25 | A | II-66 | C |
| I-170 | A | II-26 | A | II-67 | C |
| I-172 | A | II-27 | B | II-68 | A |
| I-175 | A | II-28 | A | II-69 | A |
| I-176 | A | II-29 | B | II-70 | B |
| | | II-30 | C | II-71 | A |
| | | II-31 | C | II-72 | A |
| | | II-32 | C | II-73 | A |
| | | II-33 | B | II-74 | A |
| | | II-34 | B | II-76 | A |
| | | II-36 | B | II-77 | A |
| | | II-37 | C | II-78 | A |
| | | II-38 | A | II-79 | A |

**[Table 36]**

| Comp. | Antiviral activity | Comp. | Antiviral activity | Comp. | Antiviral activity |
|---|---|---|---|---|---|
| II-80 | A | II-104 | A | III-1 | C |
| II-81 | B | II-105 | A | III-2 | A |
| II-82 | B | II-106 | A | III-3 | B |
| II-83 | B | II-107 | A | III-4 | B |
| II-84 | A | II-108 | B | III-5 | B |
| II-85 | A | II-109 | A | | |
| II-86 | A | II-110 | A | | |
| II-87 | A | II-112 | A | | |
| II-88 | A | II-113 | A | | |
| II-89 | A | II-114 | B | | |
| II-90 | A | II-115 | A | | |
| II-91 | A | II-116 | A | | |
| II-92 | A | II-117 | A | | |
| II-93 | B | II-118 | B | | |
| II-94 | A | II-119 | A | | |
| II-95 | A | II-120 | A | | |
| II-96 | A | II-121 | A | | |
| II-98 | A | II-122 | A | | |
| II-99 | A | II-123 | A | | |
| II-100 | B | II-124 | A | | |
| II-102 | A | II-125 | B | | |

### [Industrial Applicability]

As described above, according to the present invention, it is possible to provide a novel compound having excellent antiviral action against coronavirus and a pharmaceutically acceptable salt thereof, and a composition comprising the same as an active ingredient for treating or preventing a disease or condition involving coronavirus.

## Claims

1. A compound represented by the following general formula (I), or a pharmaceutically acceptable salt thereof: 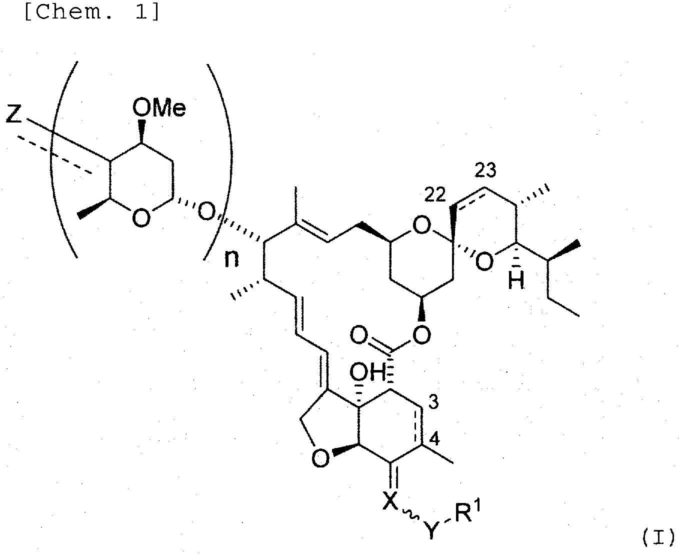 [In the above formula (I),
n represents an integer of 0, 1, or 2,
X represents CH, CF, CCl, N, CHCH=N, CHCH=CH, or CHCH=CF,
Y represents O, NH, or a single bond,
Z represents a hydroxy group or an oxo group,
R¹ represents a hydrogen atom; a halogen atom; a C1-C6 alkyl group; a 3- to 6-membered cycloalkyl group; a 4- to 8-membered heterocycloalkyl group; a 6- to 10-membered aryl group; a 5- to 10-membered heteroaryl group; a C2-C6 alkenyl group; a C2-C6 alkynyl group; a group represented by -CO-R²; a group represented by - COO-R²; a group represented by -CONH-R²; or a group represented by -B(OR⁴)OR⁵, wherein,
when R¹ is other than a hydrogen atom and a C1-C6 alkyl group, the R¹ may be substituted with one or more substituents selected from a halogen atom, a hydroxy group, an amino group, a cyano group, a nitro group, a formyl group, a sulfanyl group, an oxo group, a C1-C3 alkyl group, a C1-C3 hydroxyalkyl group, a C1-C6 alkoxy group, a C1-C3 aminoalkyl group, a C1-C3 haloalkyl group, a group represented by -N-R²R³, a group represented by -COO-R², a group represented by -CONH-R², a group represented by -NHCO-R², a group represented by -NHSO₂-R², and a group represented by -NHSO₂NH-R²,
when R¹ is a C1-C6 alkyl group, the C1-C6 alkyl group may be substituted with one or more substituents selected from a halogen atom; a hydroxy group; an amino group; a nitro group; a cyano group; an oxo group; a 3-to 6-membered cycloalkyl group which may be substituted with a hydroxy group, an amino group, a group represented by -CONH-R², a group represented by -NHCO-R², or a group represented by -NHSO₂-R²; a 4- to 8-membered heterocycloalkyl group which may be substituted with an oxo group; a 6- to 10-membered aryl group which may be substituted with a halogen atom or a C1-C3 alkyl group; a 5- to 10-membered heteroaryl group; a group represented by -O-R²; a group represented by -O-CO-R²; a group represented by -O-CONH-R²; a group represented by -N-R²R³; a group represented by -S-R²; a group represented by -CONH-R²; a group represented by - NHCO-R²; a group represented by -COO-R²; a group represented by -NHCOO-R²; a group represented by - NHCONH-R²; a group represented by -NHSO₂-R²; a group represented by -CO-R²; a group represented by -SO₂-R²; and a group represented by -NHSO₂NH-R², wherein
R² represents a hydrogen atom, a C1-C6 alkyl group, a C2-C6 alkenyl group, a C2-C6 alkynyl group, a C1-C3 haloalkyl group, a hydroxy group, a C1-C3 hydroxyalkyl group, a C1-C6 alkoxy group, a C1-C3 haloalkoxy group, a 3- to 6-membered cycloalkyl group, a 3- to 6-membered cycloalkyl C1-C3 alkyl group, a 4-to 8-membered heterocycloalkyl group, a 6- to 10-membered aryl group, a 6- to 10-membered aryl C1-C3 alkyl group, a 5- to 10-membered heteroaryl group, a 5-to 10-membered heteroaryl C1-C3 alkyl group, a C1-C3 alkoxy C1-C3 alkyl group, an amino group, a C1-C6 alkylamino group, a C1-C6 dialkylamino group, a sulfanyl group, or a C1-C6 trialkylsilyl group, wherein the 5- to 10-membered heteroaryl group represented by R² may be further substituted with one or more substituents selected from a halogen atom, a hydroxy group, an amino group, a cyano group, a C1-C3 alkyl group, a C1-C3 hydroxyalkyl group, a C1-C6 alkoxy group, a C1-C3 aminoalkyl group, a C1-C3 haloalkyl group, a group represented by -N-R²R³, and a group represented by -CONH-R², and the heteroaryl group of the 5- to 10-membered heteroaryl C1-C3 alkyl group represented by R² may be further substituted with an amino group or a C1-C3 alkyl group,
R³ represents a hydrogen atom or a C1-C6 alkyl group, and
R⁴ and R⁵ each represent a hydrogen atom, or independently represent a C1-C10 alkyl group which may be bonded to each other to form a ring structure, and
numbers 3, 4, 22, and 23 indicate position numbers of carbon atoms, and a bond between positions 3 and 4 and a bond between positions 22 and 23 may each independently be a single bond or a double bond.].

2. The compound or pharmaceutically acceptable salt thereof according to claim 1, wherein in the formula (I), X is N, Y is O, and the bond between positions 3 and 4 is a single bond.

3. The compound or pharmaceutically acceptable salt thereof according to claim 2, wherein R¹ in the formula (I) is a C1-C6 alkyl group which may be substituted.

4. The compound or pharmaceutically acceptable salt thereof according to claim 3, wherein R¹ in the formula (I) is a C1-C6 alkyl group substituted with a 5- to 10-membered heteroaryl group, a group represented by - CONH-R², or a group represented by -CO-R².

5. The compound or pharmaceutically acceptable salt thereof according to claim 1, wherein in the formula (I), X is N, Y is O, and the bond between positions 3 and 4 is a double bond.

6. The compound or pharmaceutically acceptable salt thereof according to claim 1, wherein in the formula (I), X is N, Y is O, and R¹ is a hydrogen atom; a C2-C6 alkenyl group; a C2-C6 alkynyl group; a halogen atom; a 3- to 6-membered cycloalkyl group which may be substituted with a hydroxy group; a 4- to 8-membered heterocycloalkyl group; or a C1-C6 alkyl group substituted with one or more substituents selected from a group represented by -O-R², a group represented by - O-CONH-R², a group represented by -COO-R², a group represented by -CONH-R², a group represented by -NHCONH-R², a group represented by -CO-R², and a group represented by -NHSO₂-R².

7. The compound or pharmaceutically acceptable salt thereof according to claim 1, wherein the compound represented by the formula (I) is selected from compounds represented by the following formulas (1) to (94) :

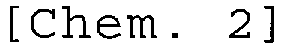

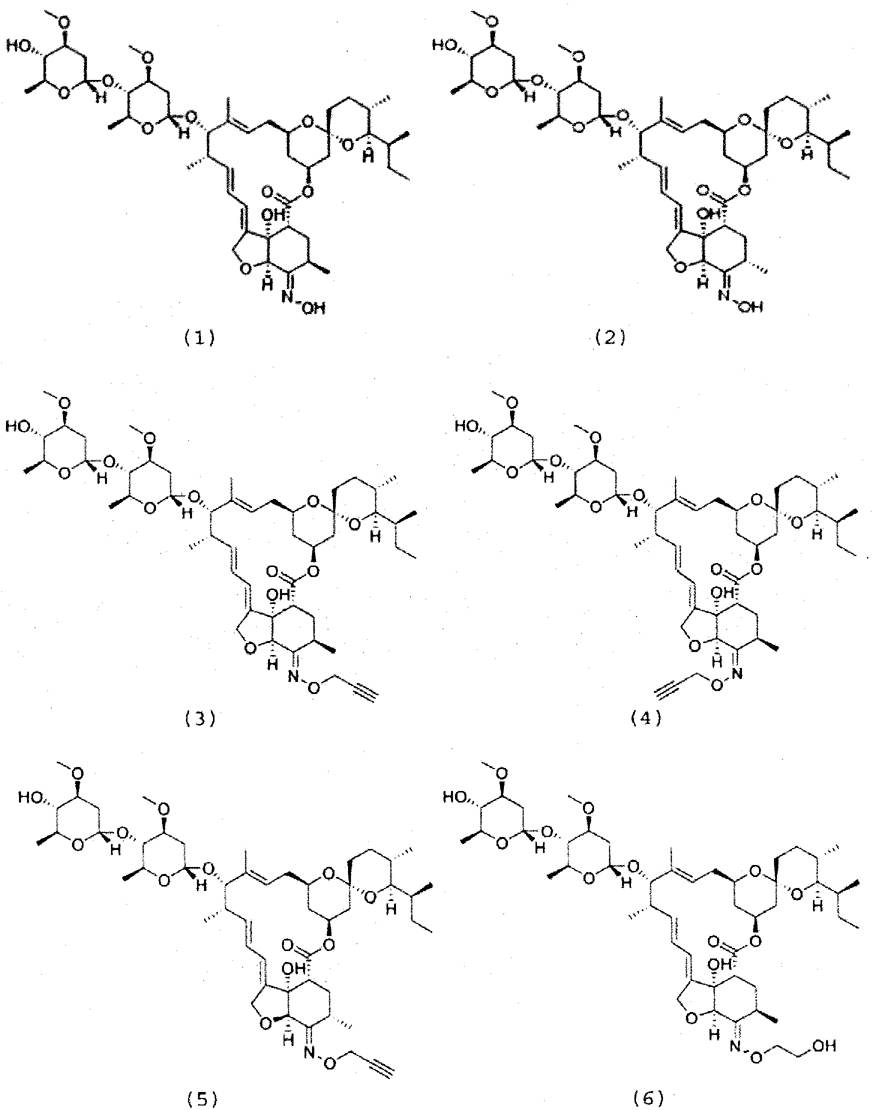

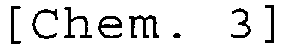

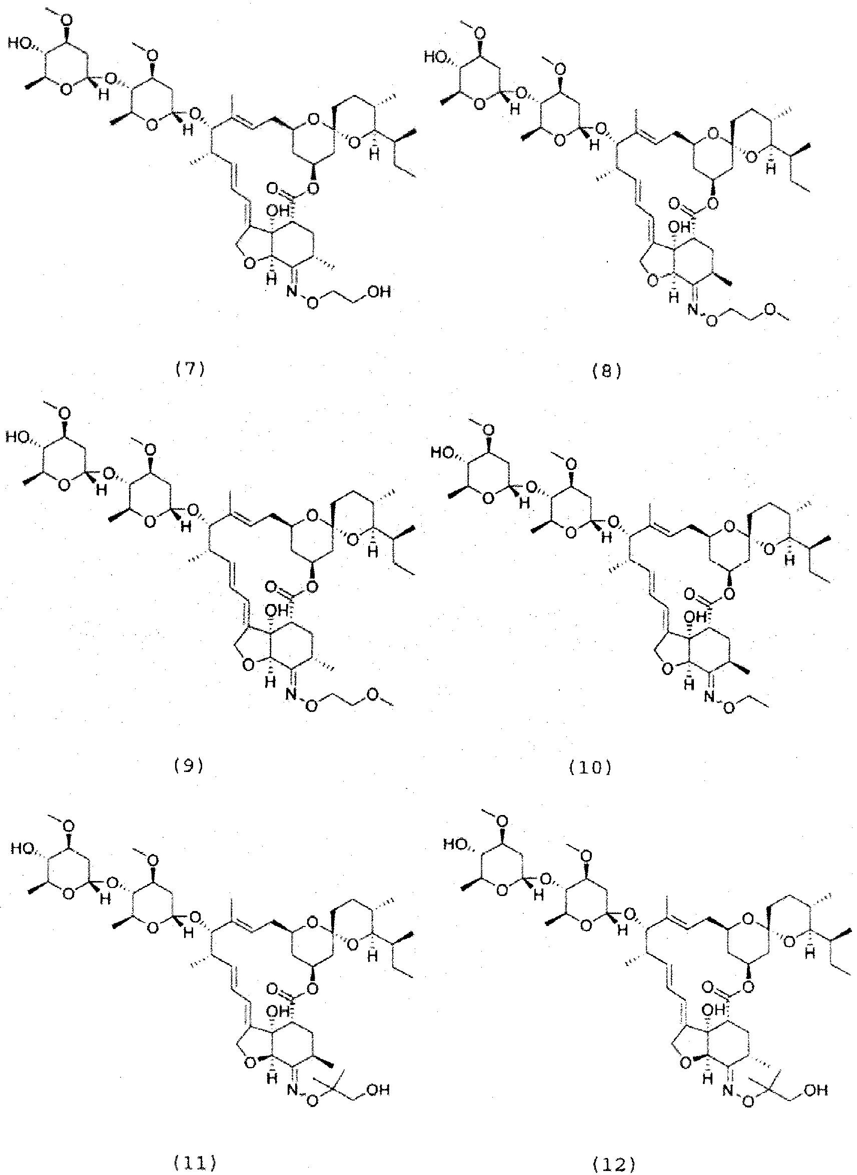

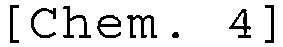

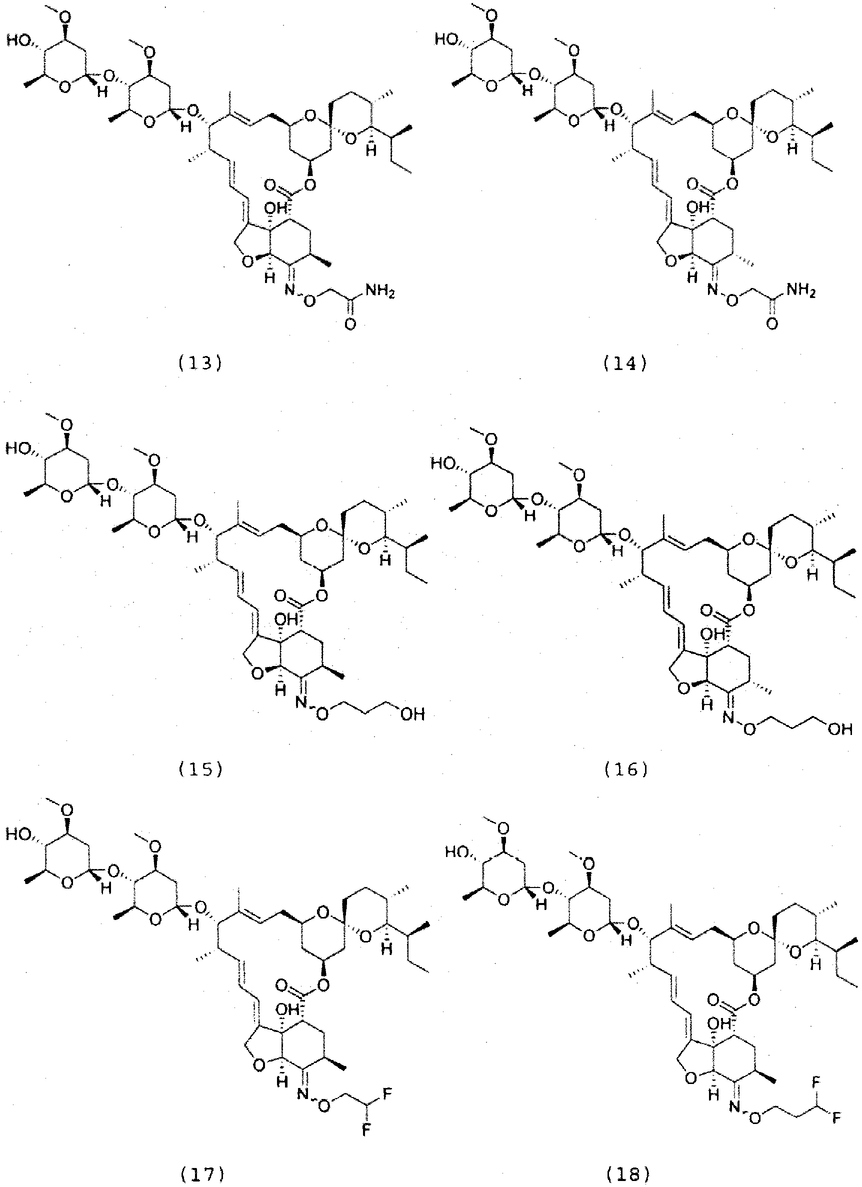

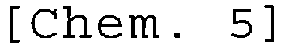

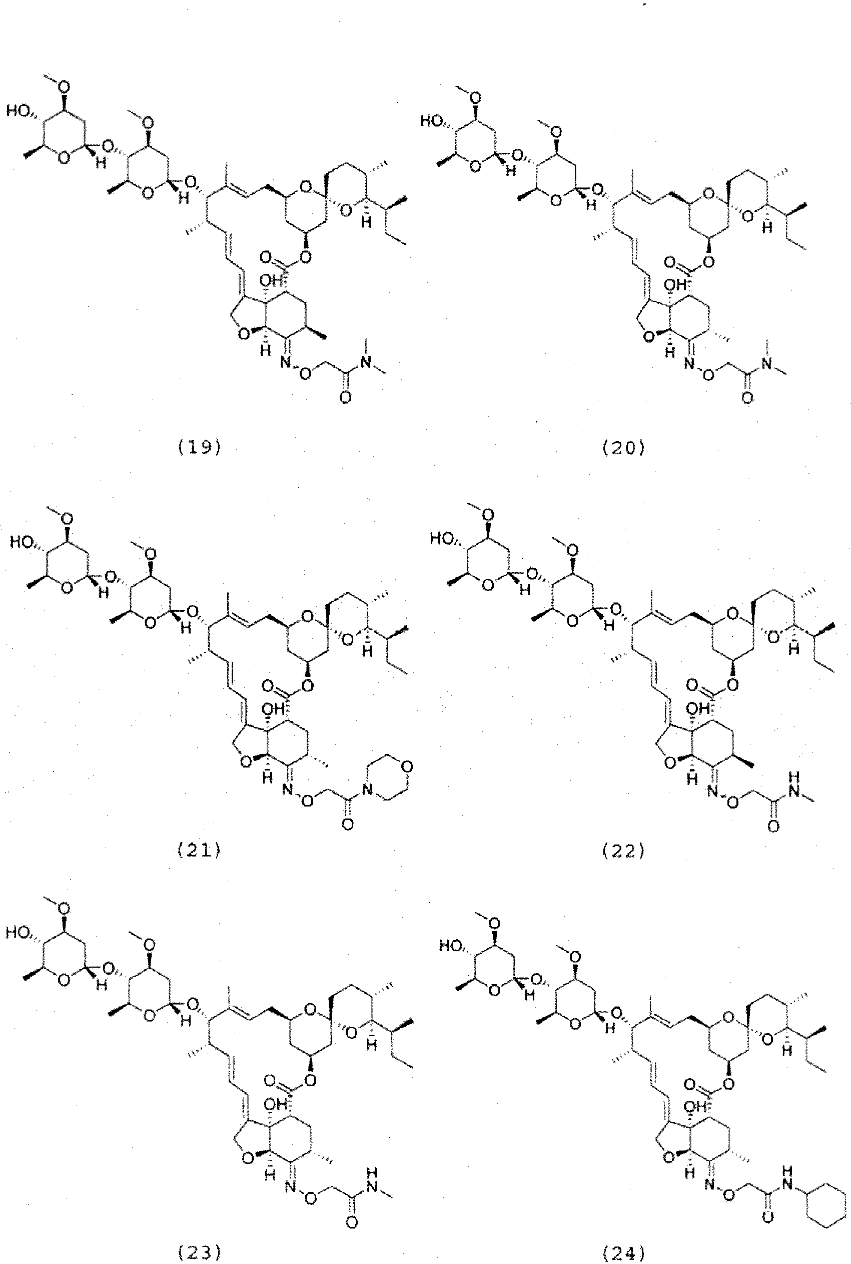

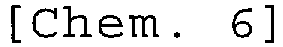

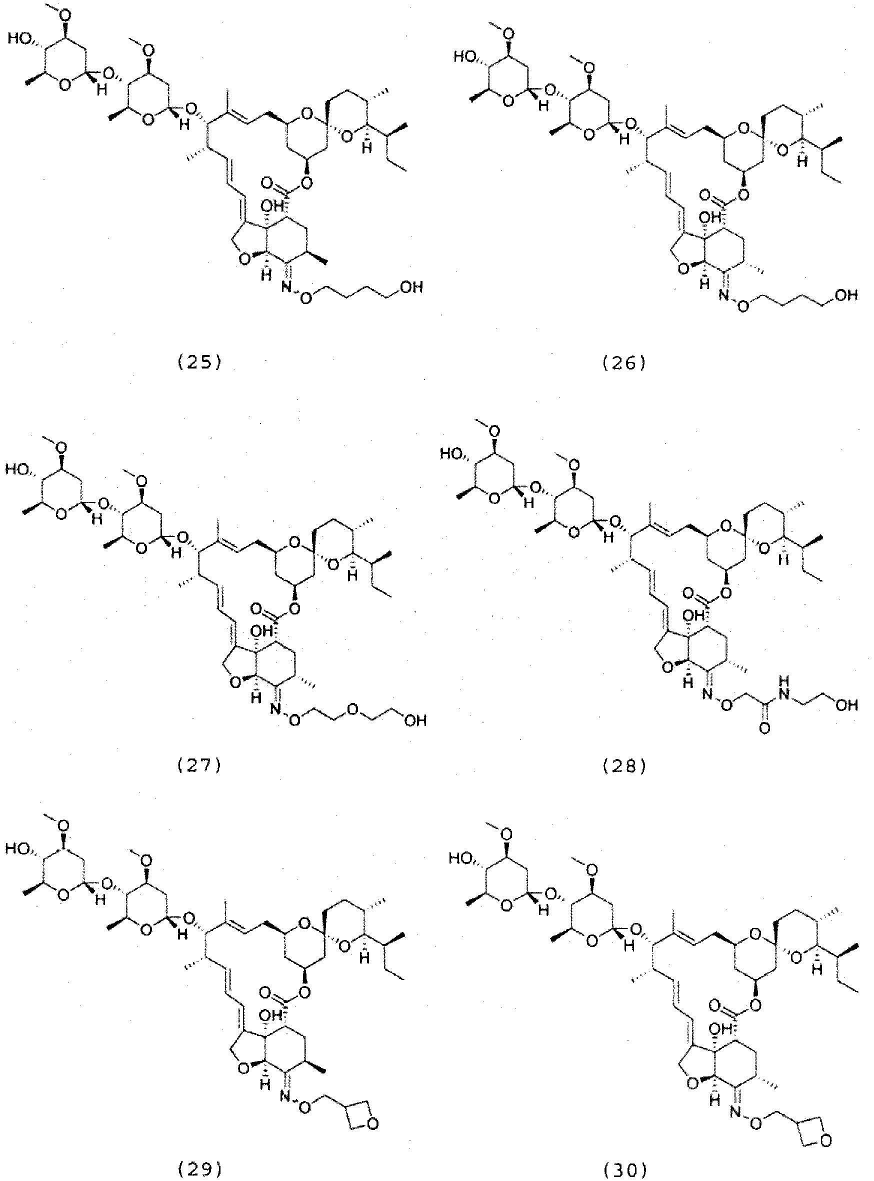

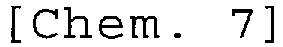

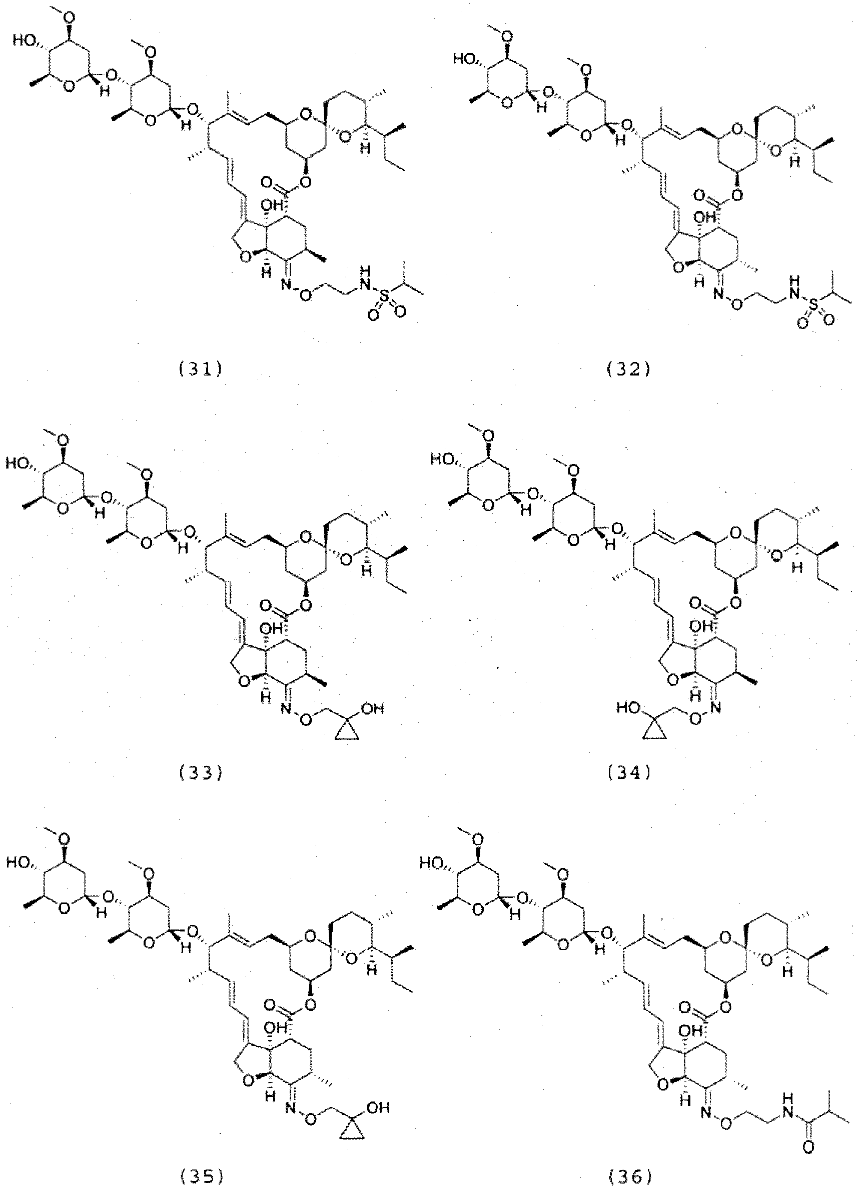

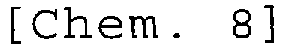

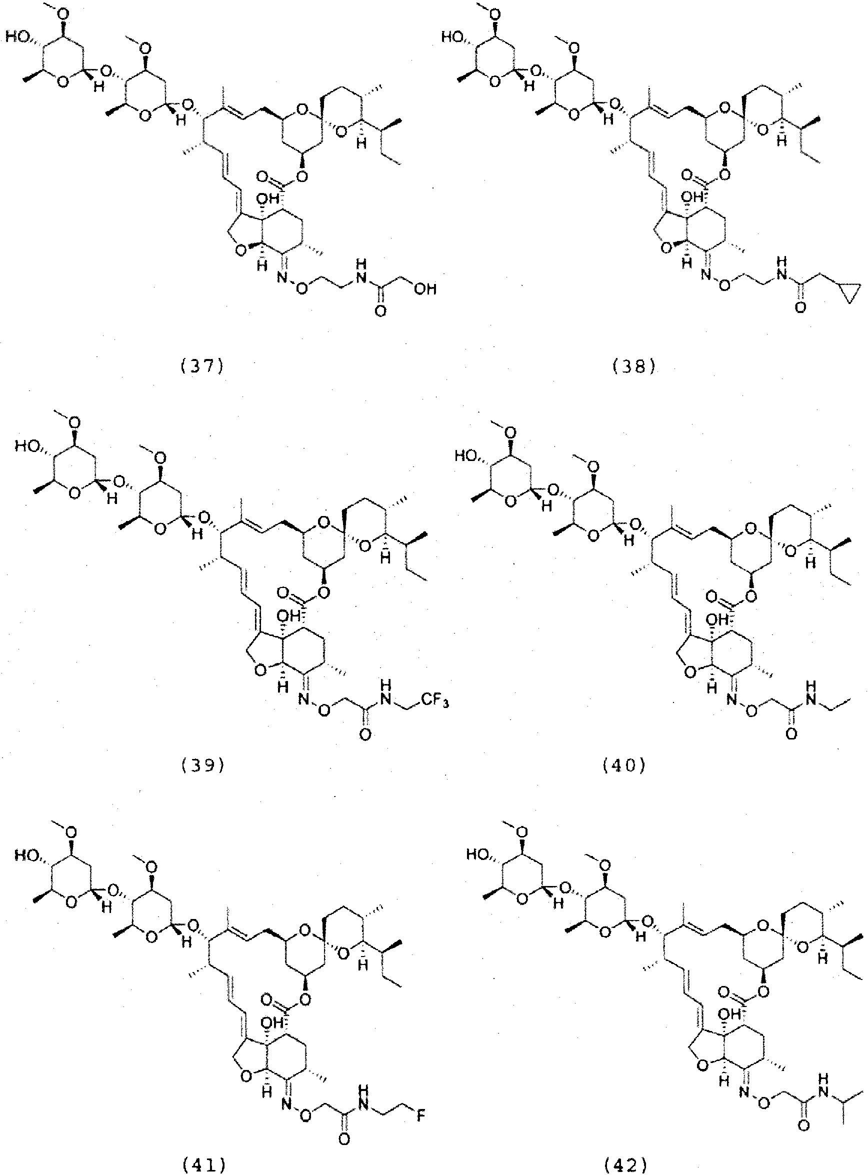

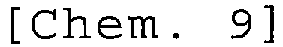

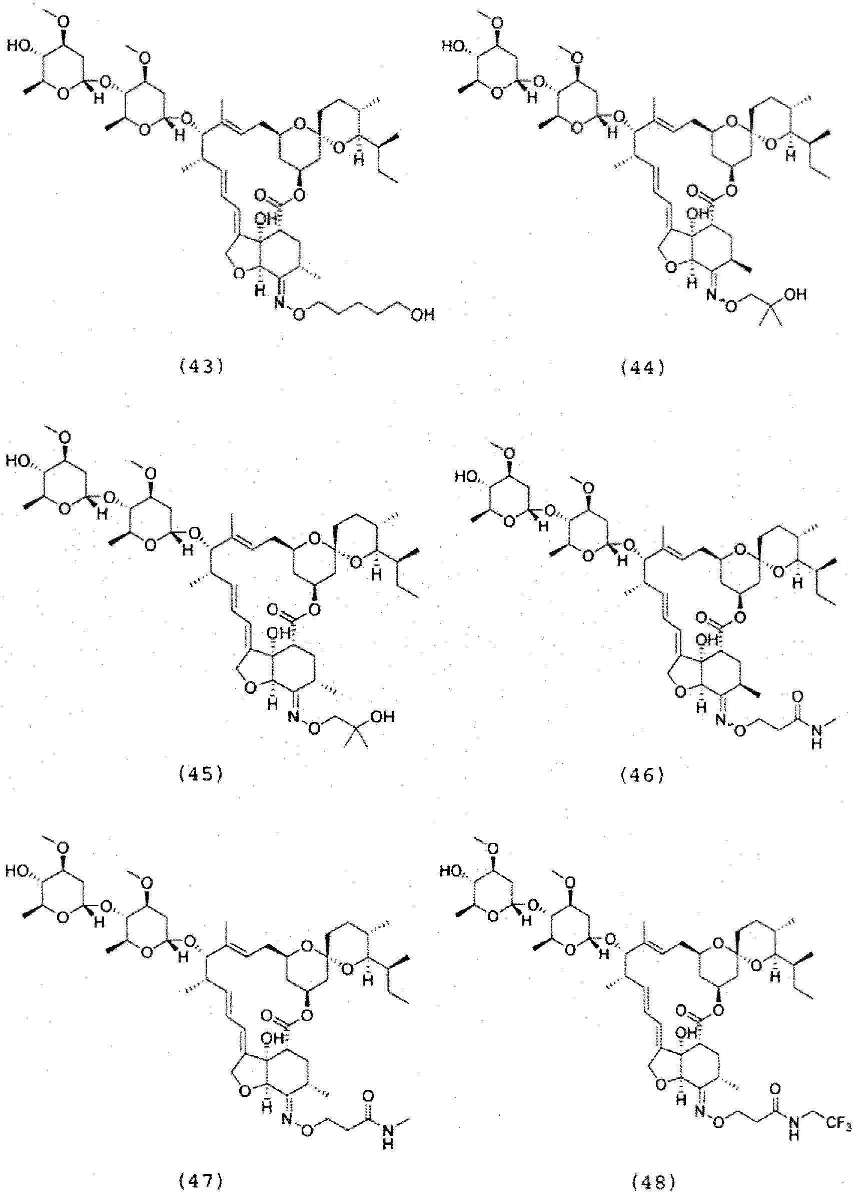

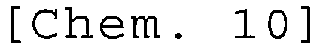

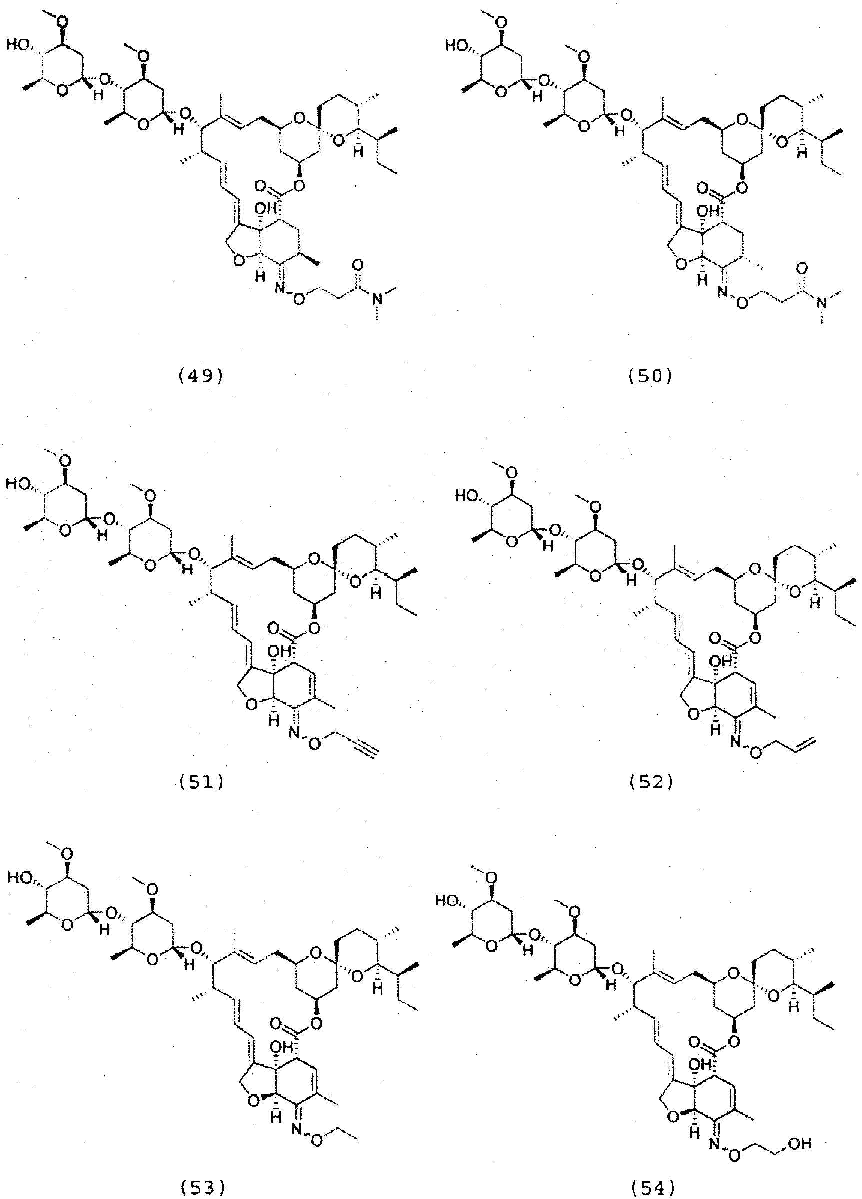

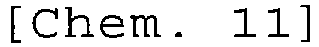

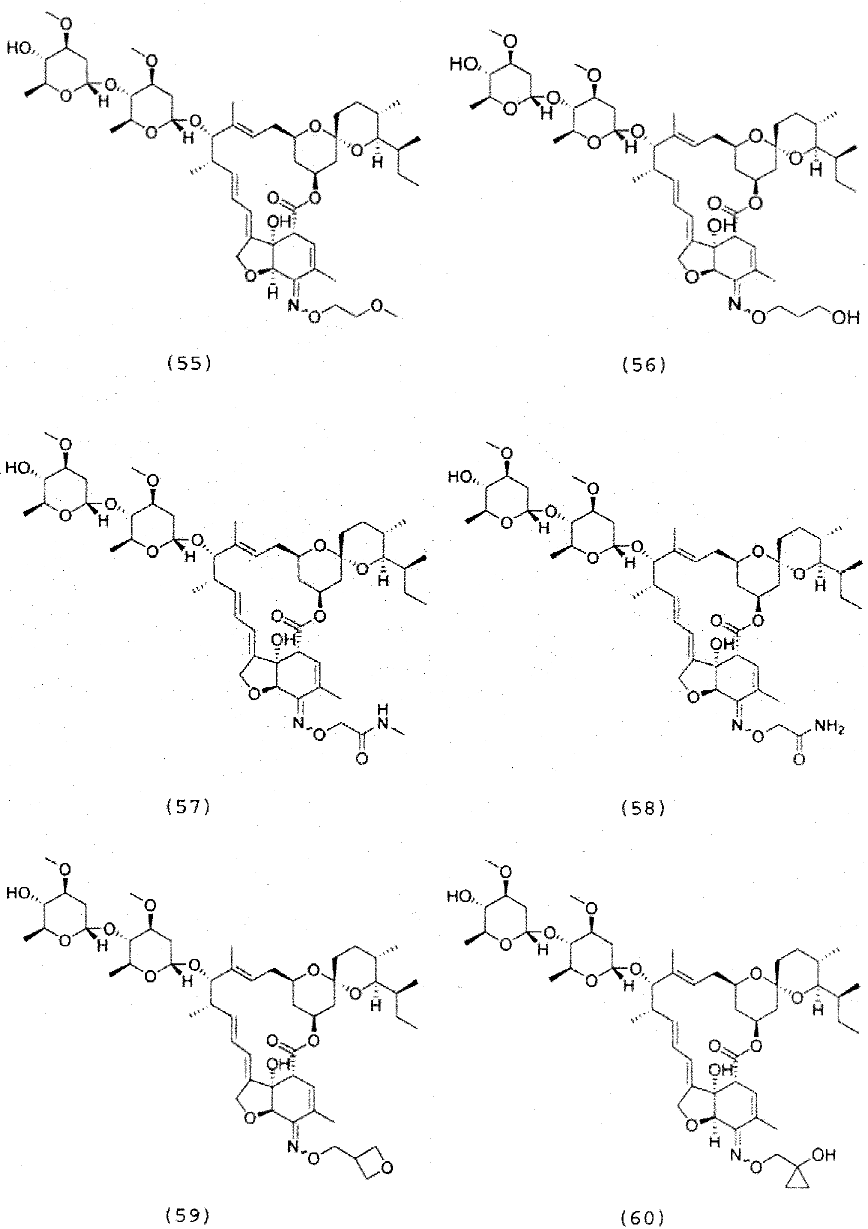

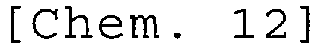

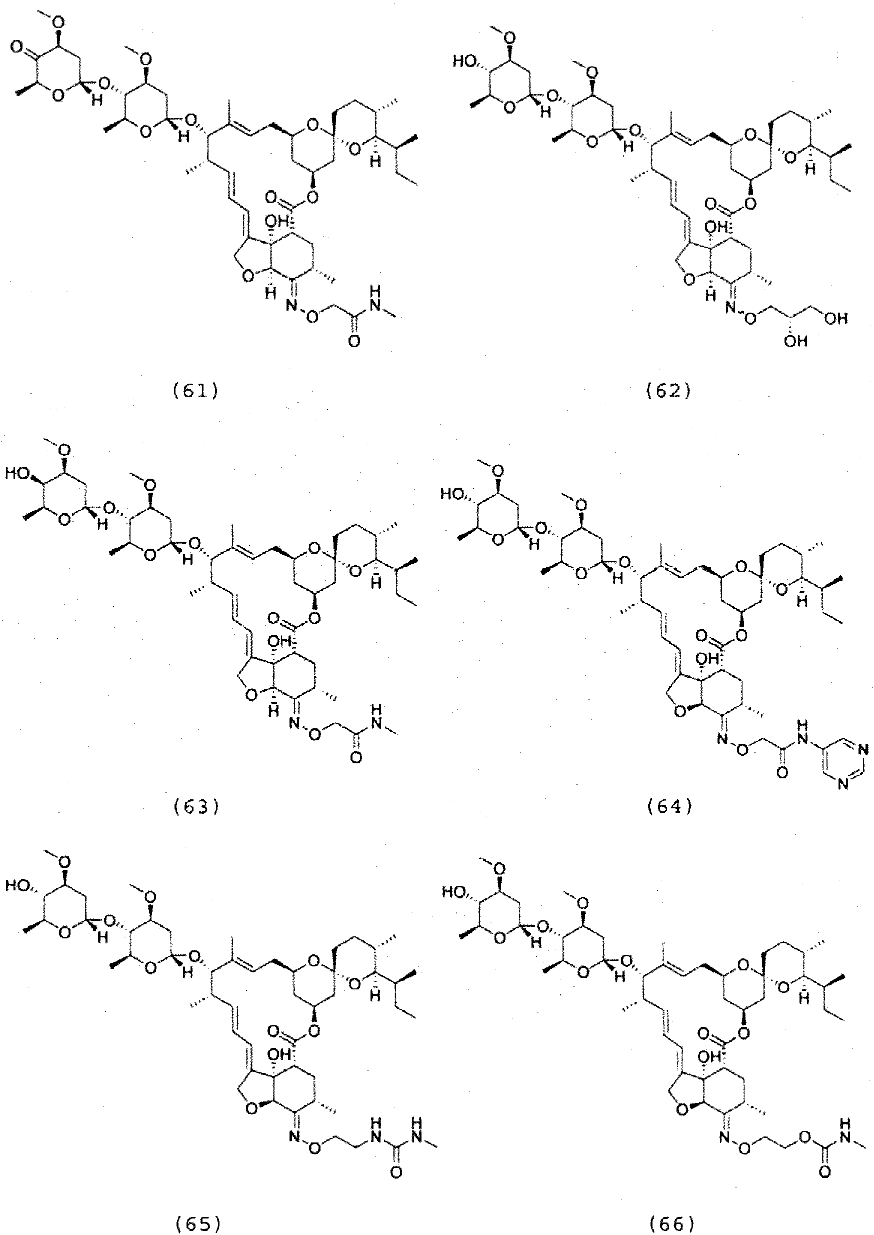

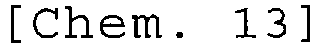

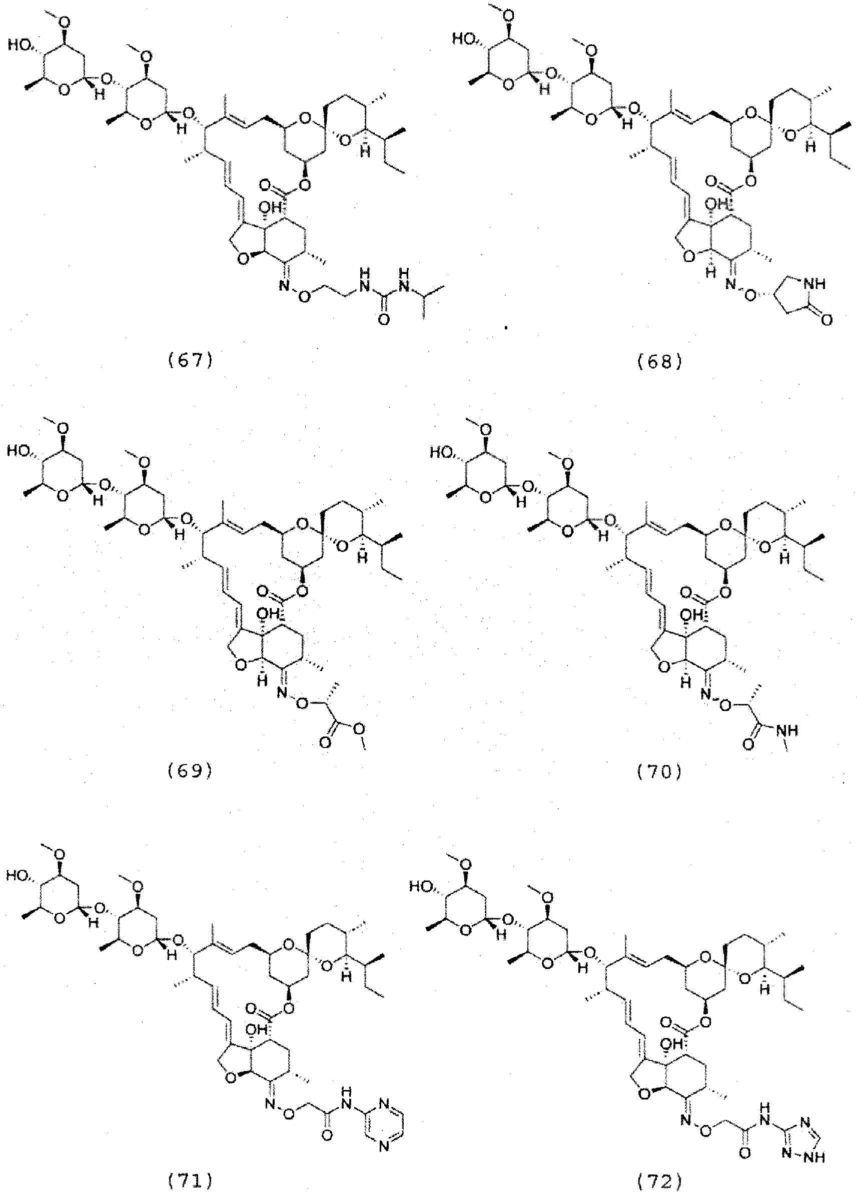

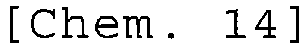

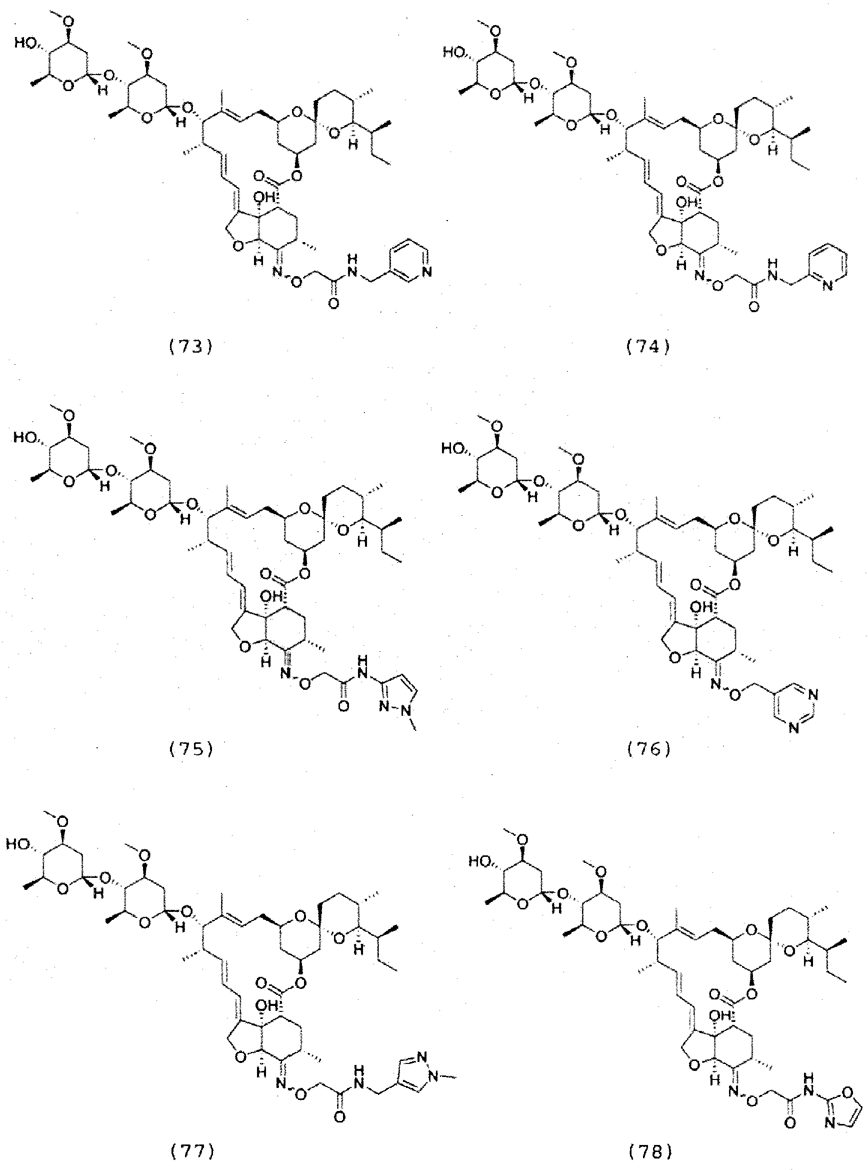

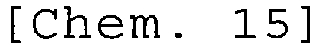

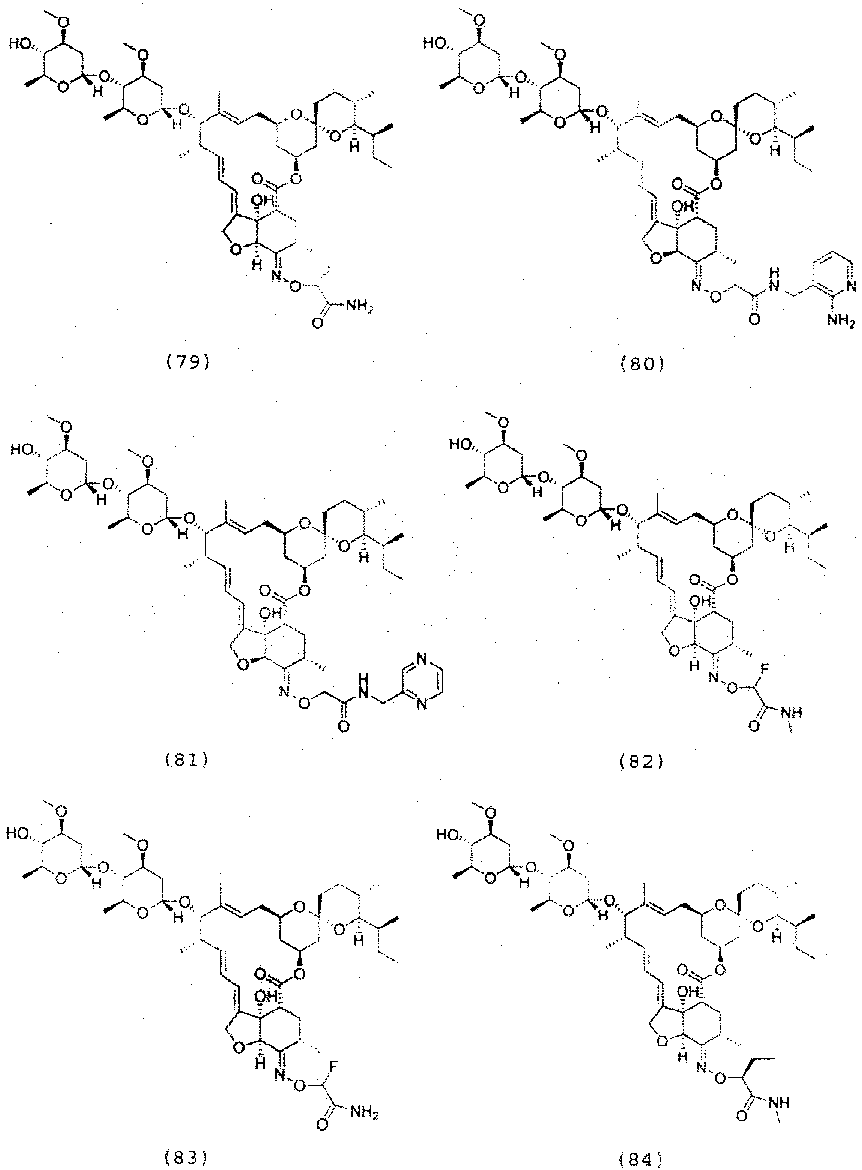

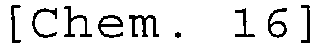

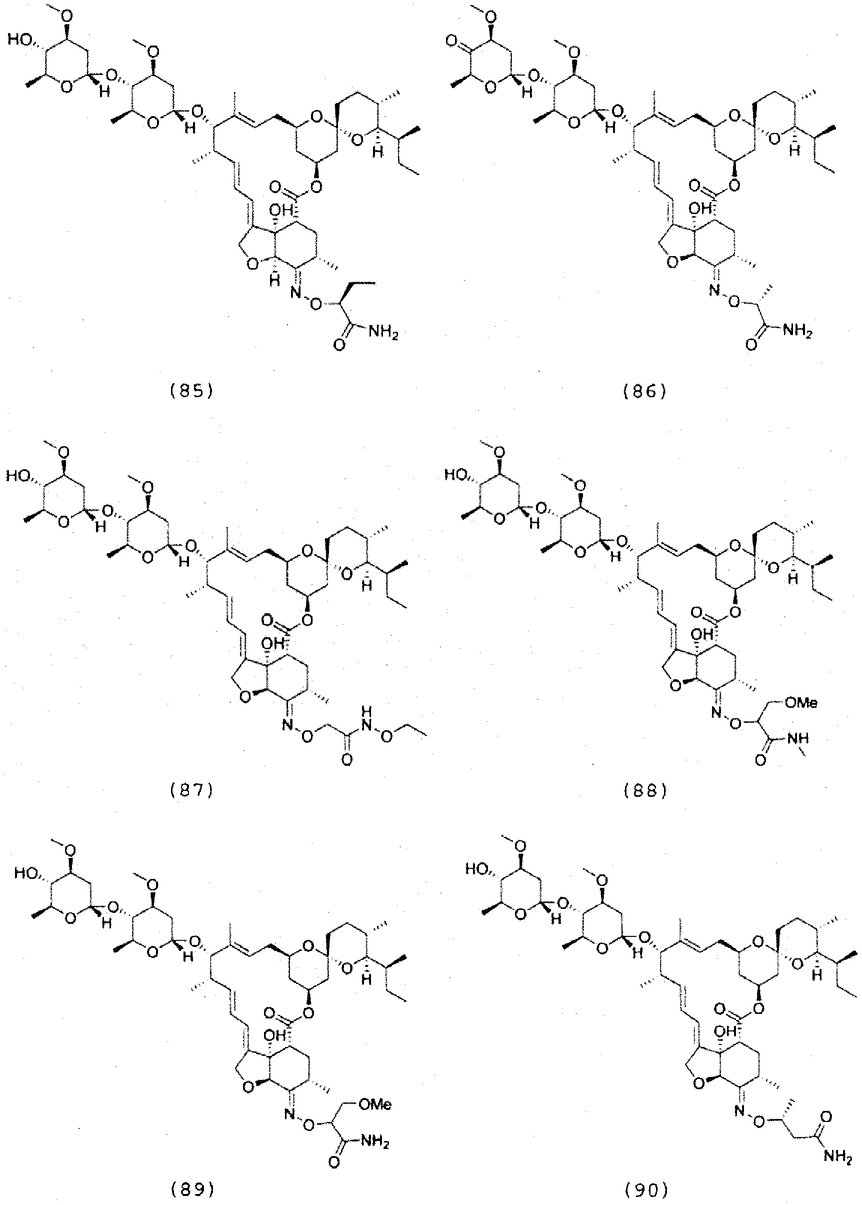

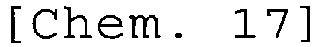

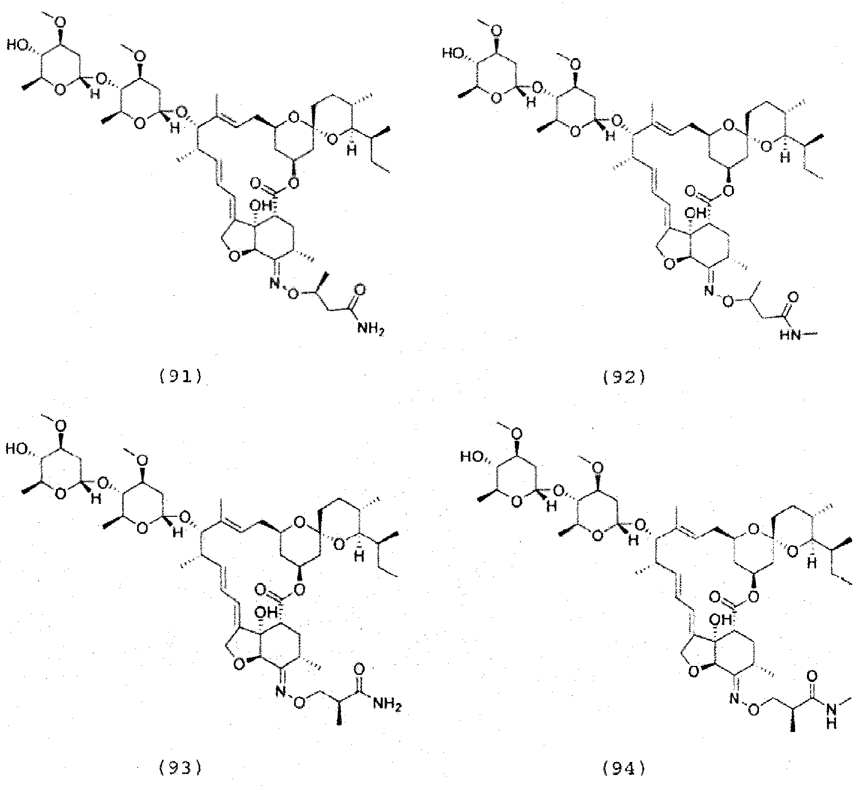

8. The compound or pharmaceutically acceptable salt thereof according to claim 1, wherein X in the formula (I) is CH, CF, or CHCH=CH, and the bond between positions 3 and 4 is a single bond.

9. The compound or pharmaceutically acceptable salt thereof according to claim 1, wherein X in the formula (I) is CH, CF, or CHCH=CH, and the bond between positions 3 and 4 is a double bond.

10. The compound or pharmaceutically acceptable salt thereof according to claim 1, wherein in the formula (I), X is CH, CF, or CHCH=CH, Y is a single bond, and
R¹ is a 4- to 8-membered heterocycloalkyl group which may be substituted with an oxo group; a group represented by -COO-R²; a 5- to 10-membered heteroaryl group which may be substituted with one or more substituents selected from a halogen atom, a hydroxy group, an amino group, a cyano group, a formyl group, an oxo group, a C1-C3 alkyl group, a C1-C3 hydroxyalkyl group, a C1-C6 alkoxy group, a C1-C3 haloalkyl group, a group represented by -N-R²R³, a group represented by -NHCO-R², and a group represented by -CONH-R²; or a C1-C6 alkyl group which may be substituted with one or more substituents selected from a hydroxy group, an amino group, a nitro group, an oxo group, a group represented by -CONH-R², and a group represented by - COO-R².

11. The compound or pharmaceutically acceptable salt thereof according to claim 1, wherein the compound represented by the formula (I) is selected from compounds represented by the following formulas (201) to (271):

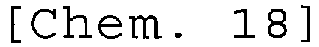

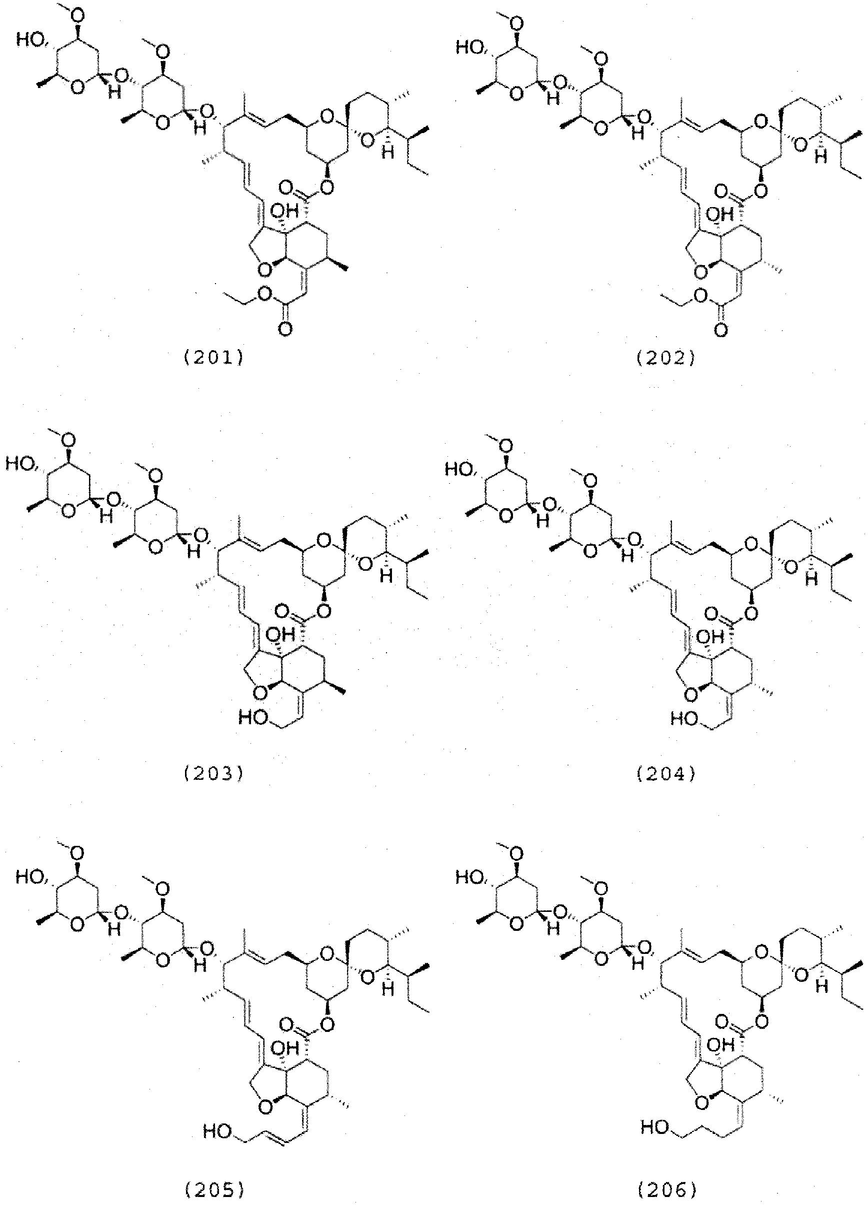

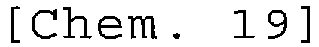

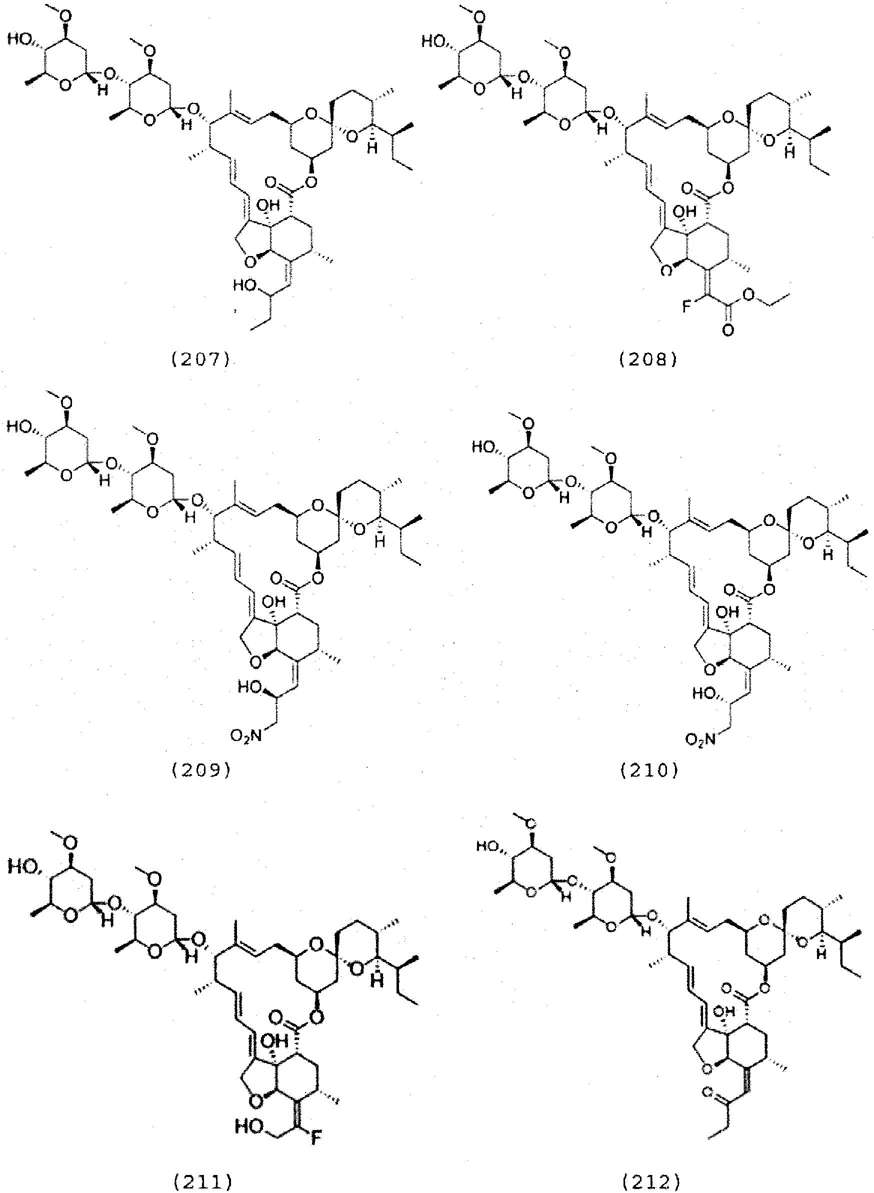

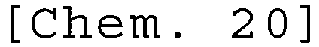

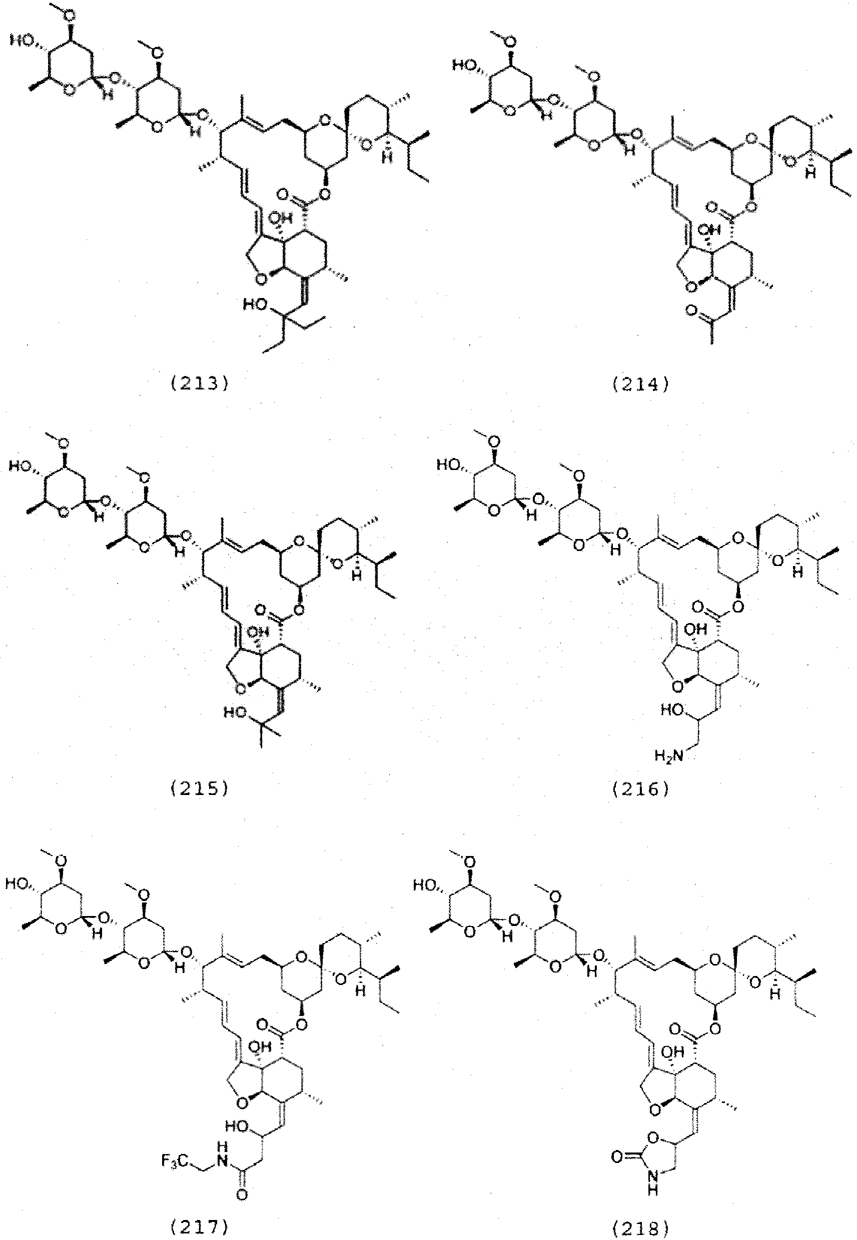

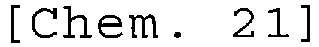

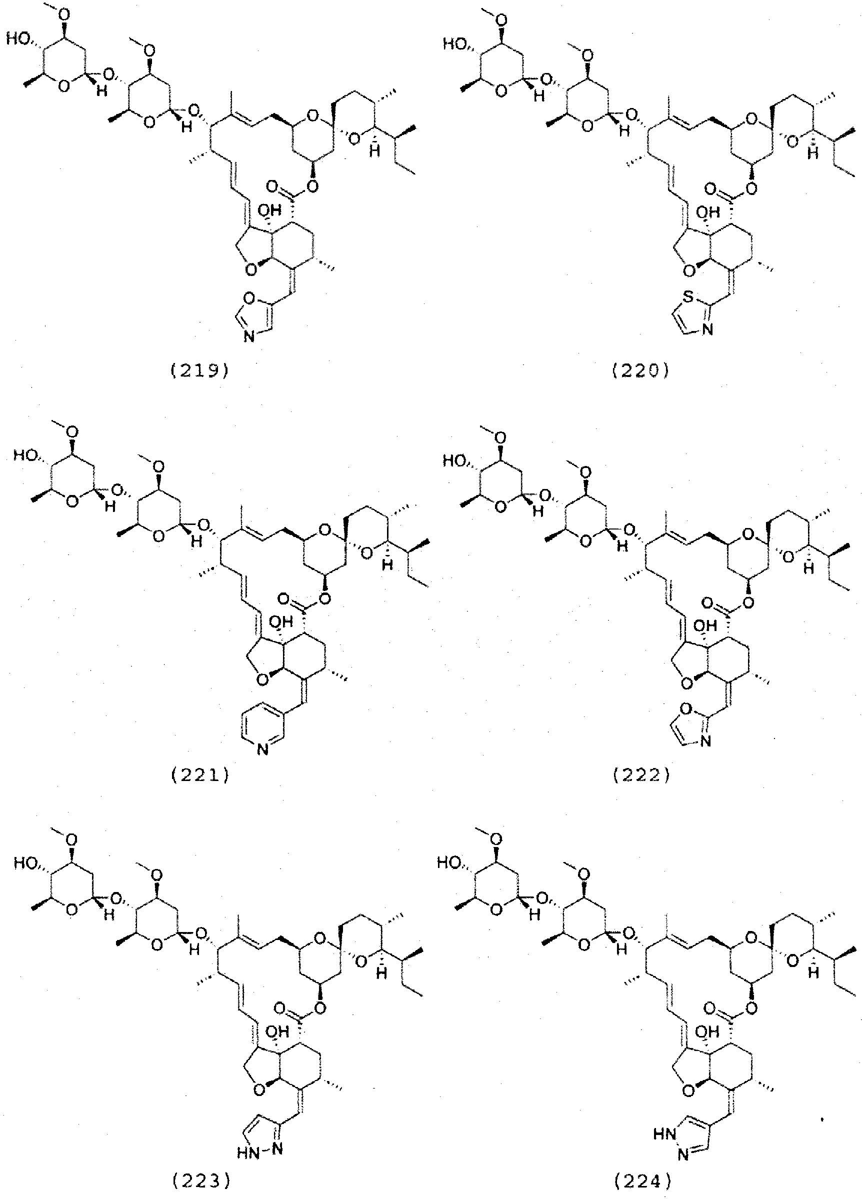

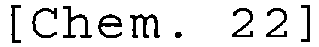

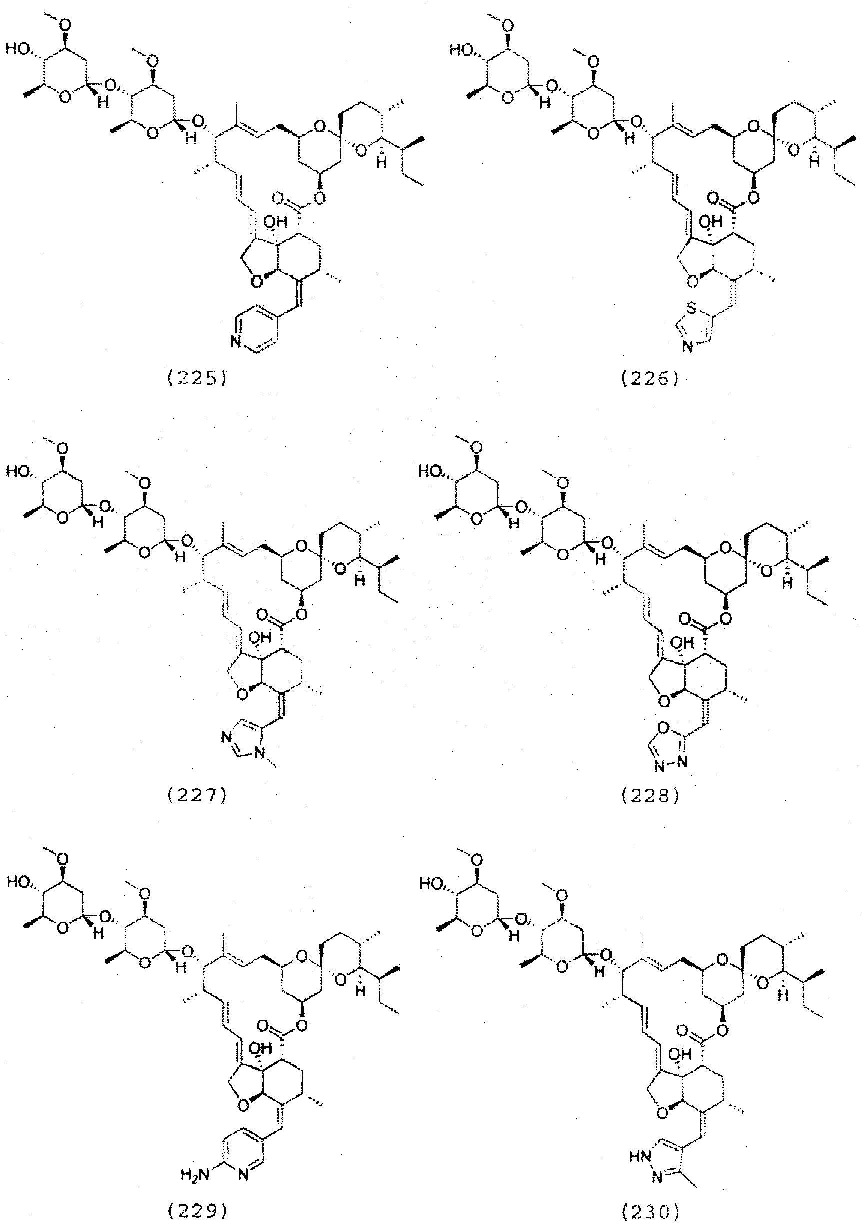

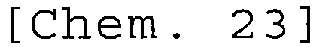

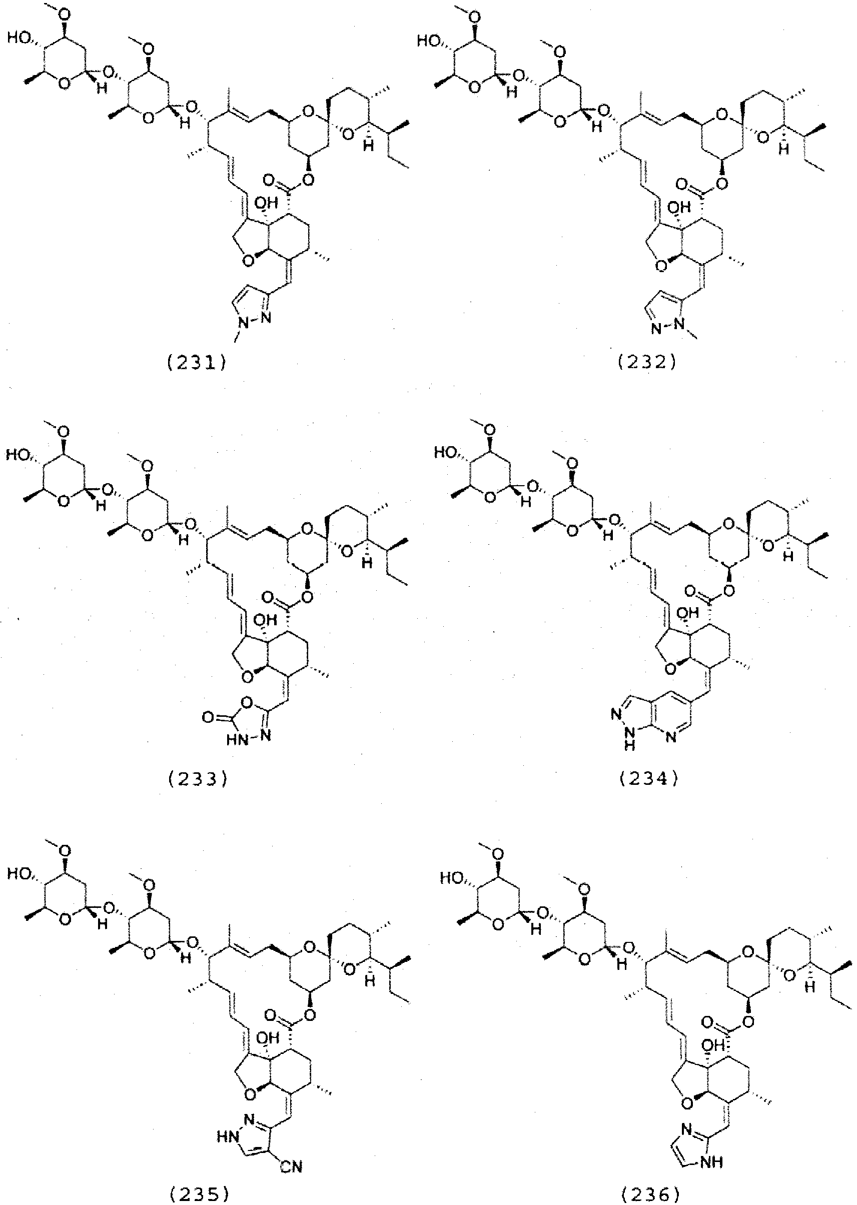

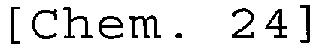

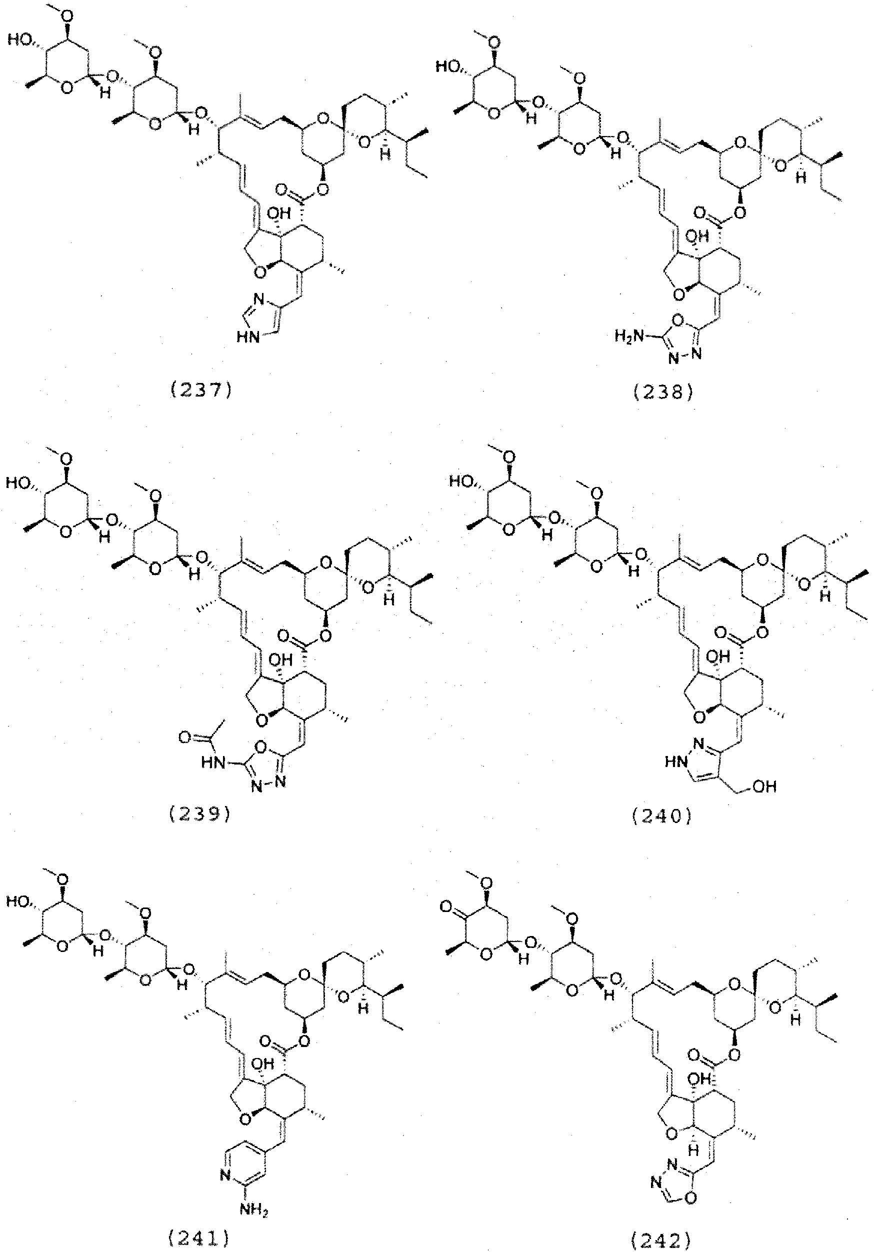

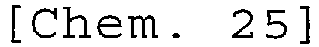

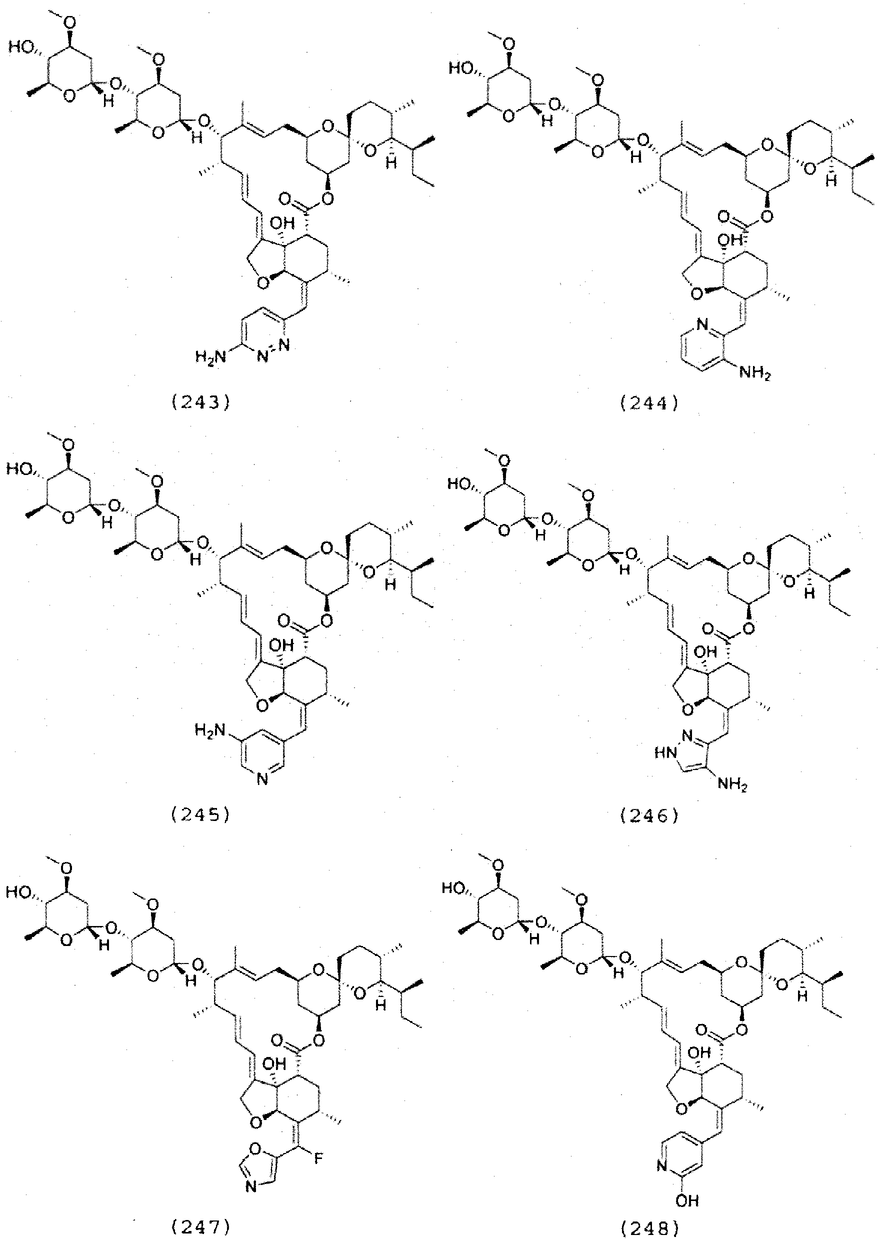

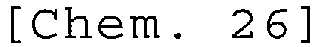

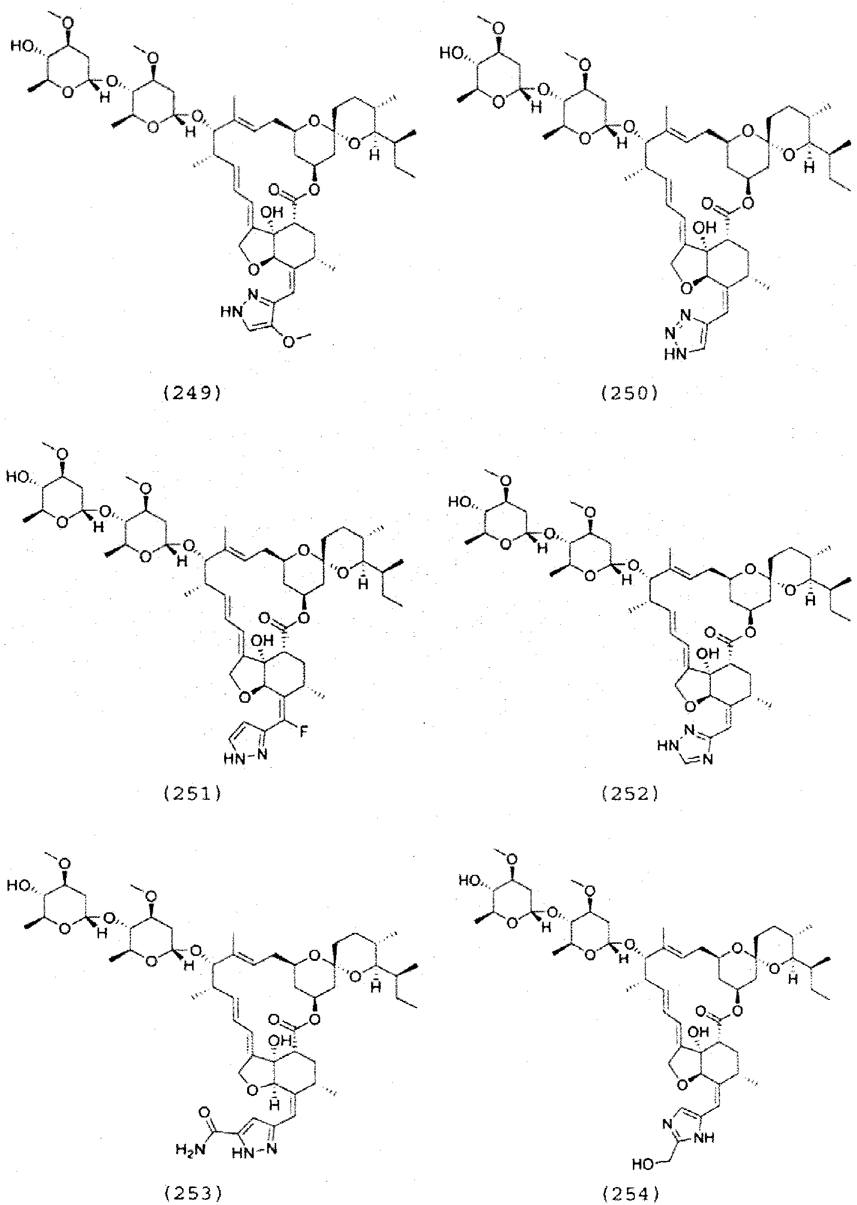

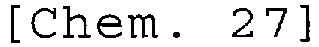

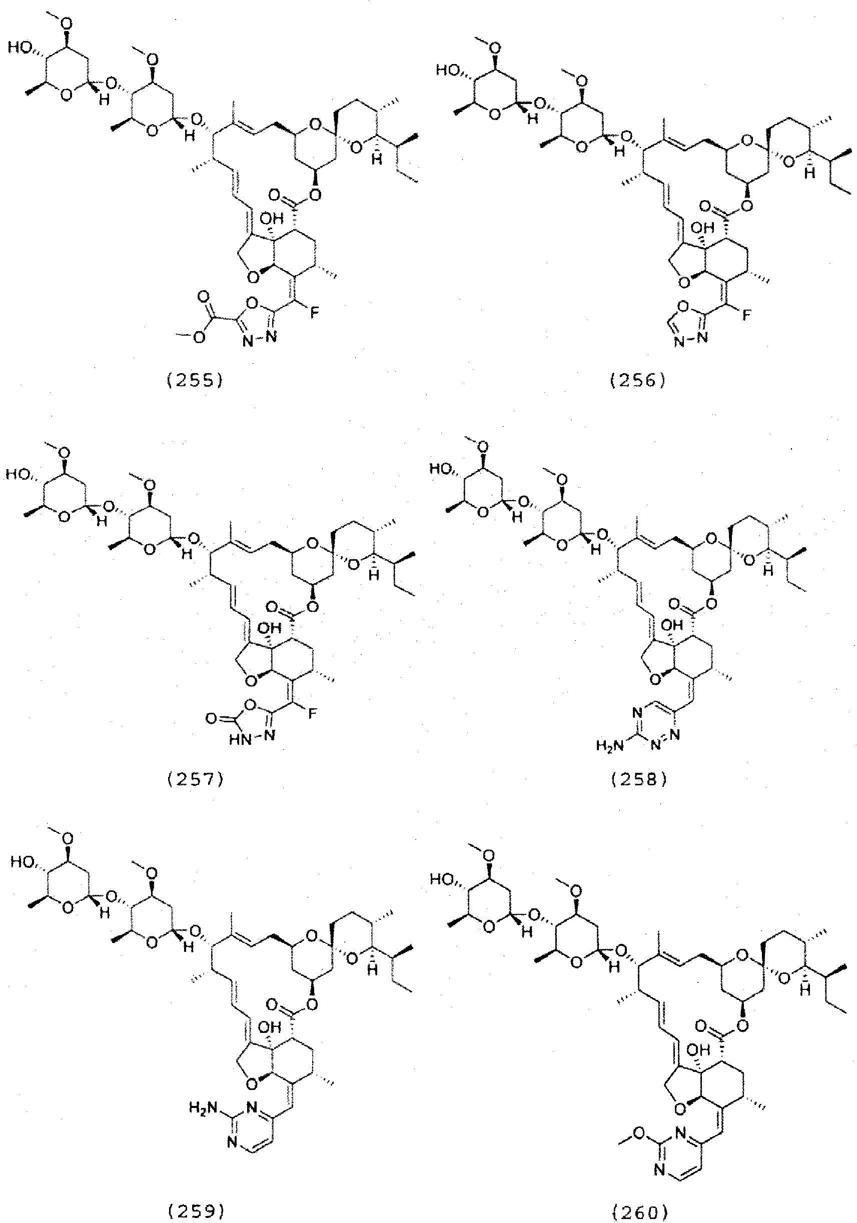

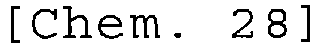

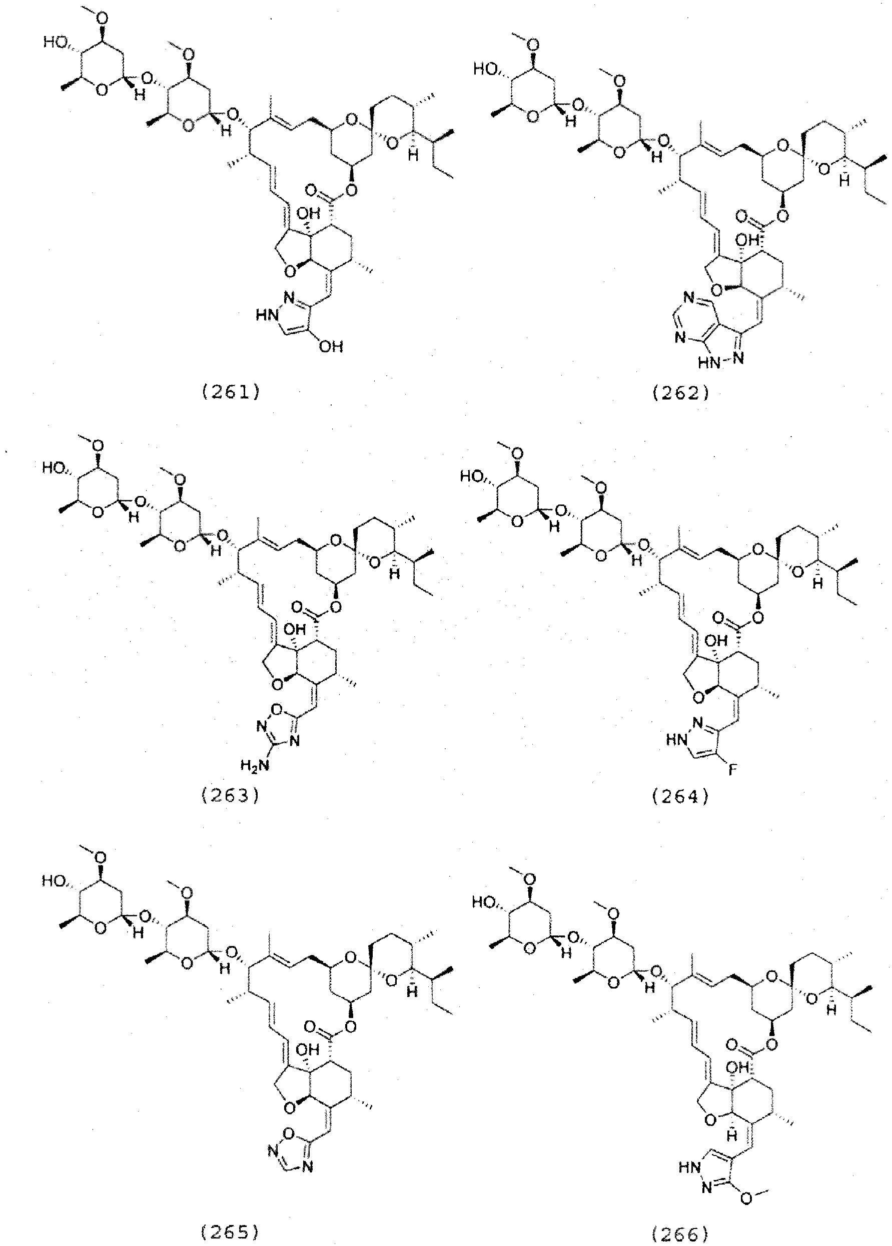

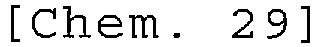

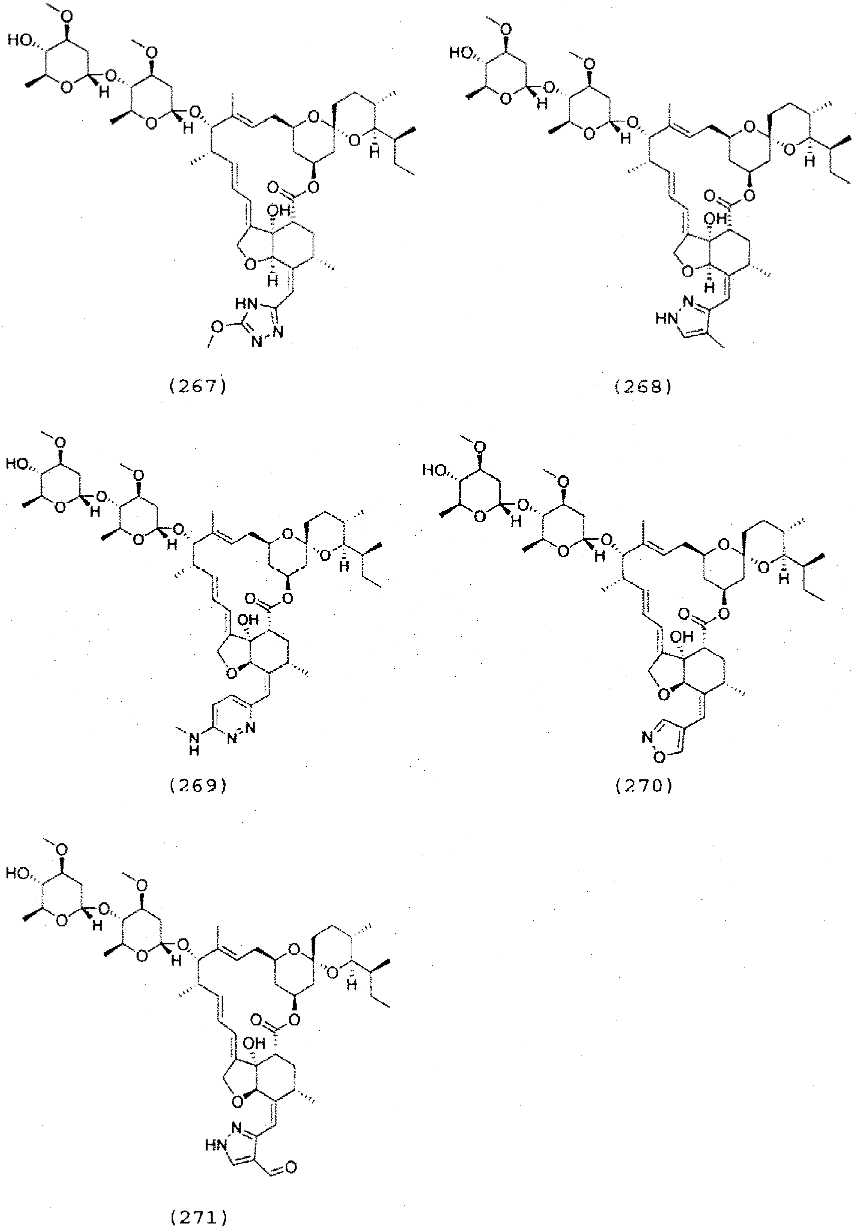

12. The compound or pharmaceutically acceptable salt thereof according to claim 1, wherein in the formula (I), X is N, Y is NH, and R¹ is a group represented by -CO-R².

13. The compound or pharmaceutically acceptable salt thereof according to any one of claims 1 to 12, which has antiviral activity against coronaviruses.

14. The compound or pharmaceutically acceptable salt thereof according to claim 13, wherein the coronavirus is a coronavirus classified in the genus Betacoronavirus.

15. The compound or pharmaceutically acceptable salt thereof according to claim 13, wherein the coronavirus is at least one selected from the group consisting of human coronavirus (HCoV), severe acute respiratory syndrome coronavirus (SARS-CoV), novel coronavirus (SARS-CoV-2), and Middle East respiratory syndrome coronavirus (MERS-CoV).

16. A composition for the treatment or prevention of a disease or condition involving a coronavirus, comprising the compound or pharmaceutically acceptable salt thereof according to any one of claims 1 to 12 as an active ingredient.

17. The composition according to claim 16, wherein the coronavirus is a coronavirus classified in the genus Betacoronavirus.

18. The composition according to claim 16, wherein the coronavirus is at least one selected from the group consisting of human coronavirus (HCoV), severe acute respiratory syndrome coronavirus (SARS-CoV), novel coronavirus (SARS-CoV-2), and Middle East respiratory syndrome coronavirus (MERS-CoV).

19. A method for treating or preventing a disease or condition involving a coronavirus, comprising the step of administering to a target the compound or pharmaceutically acceptable salt thereof according to any one of claims 1 to 12.

20. Use of the compound or pharmaceutically acceptable salt thereof according to any one of claims 1 to 12 for the manufacture of a composition for the treatment or prevention of a disease or condition involving a coronavirus.
